# EUROPEAN PATENT SPECIFICATION

(11) **EP 2 635 675 B1**
(45) Date of publication and mention of the grant of the patent: **06.01.2016**
(21) Application number: 11776434.0
(22) Date of filing: 28.10.2011
(51) Int. Cl.: C12N 9/10, G06F 19/16

(54) **CRYSTAL STRUCTURE OF ISOGLUTAMINYL CYCLASE**
KRISTALLSTRUKTUR EINER ISOGLUTAMINYLCYCLASE
STRUCTURE CRISTALLINE D'ISOGLUTAMINYLE CYCLASE

(30) Priority: 02.11.2010 US 409200 P
(43) Date of publication of application: 11.09.2013
(73) Proprietor: Probiodrug AG, 06120 Halle/Saale (DE)
(72) Inventor: SCHILLING, Stephan, 06130 Halle / Saale (DE); RAHFELD, Jens-Ulrich, 06317 Gemeinde Seegebiet Mansfelder Land (DE); WERMANN, Michael, 06118 Halle / Saale (DE); STEPHAN, Anett, 06114 Halle / Saale (DE); RUIZ-CARILLO, David, Singapore 610357 (SG); PARTHIER, Christoph, 06114 Halle / Saale (DE); STUBBS, Milton, 06114 Halle / Saale (DE); DEMUTH, Hans-Ulrich, 06120 Halle / Saale (DE)
(74) Representative: Maikowski & Ninnemann
(86) International application number: PCT/EP2011/068934
(87) International publication number: WO 2012/059413

(56) References cited:
- WO-A1-2010/026209
- US-A1- 2009 203 045
- ANETT STEPHAN ET AL: "Mammalian glutaminyl cyclases and their isoenzymes have identical enzymatic characteristics", FEBS JOURNAL, vol. 276, no. 22, 1 November 2009 (2009-11-01), pages 6522-6536, XP55013009, ISSN: 1742-464X, DOI: 10.1111/j.1742-4658.2009.07337.x
- K.-F. HUANG ET AL: "Structures of Human Golgi-resident Glutaminyl Cyclase and Its Complexes with Inhibitors Reveal a Large Loop Movement upon Inhibitor Binding", JOURNAL OF BIOLOGICAL CHEMISTRY, vol. 286, no. 14, 8 April 2011 (2011-04-08), pages 12439-12449, XP55015913, ISSN: 0021-9258, DOI: 10.1074/jbc.M110.208595
- DAVID RUIZ-CARRILLO ET AL: "Structures of Glycosylated Mammalian Glutaminyl Cyclases Reveal Conformational Variability near the Active Center", BIOCHEMISTRY, vol. 50, no. 28, 19 July 2011 (2011-07-19) , pages 6280-6288, XP55013004, ISSN: 0006-2960, DOI: 10.1021/bi200249h

### SEQUENCE LISTING

<110> PROBIODRUG AG
<120> CRYSTAL STRUCTURE OF ISOGLUTAMINYL CYCLASE
<130> PBD00085/WO
<160> 19
<170> PatentIn version 3.5
<210> 1
   <211> 382
   <212> PRT
   <213> Human
<400> 1
   Met Arg Ser Gly Gly Arg Gly Arg Pro Arg Leu Arg Leu Gly Glu Arg
   1               5                   10                  15
   Gly Leu Met Glu Pro Leu Leu Pro Pro Lys Arg Arg Leu Leu Pro Arg
               20                  25                  30
   Val Arg Leu Leu Pro Leu Leu Leu Ala Leu Ala Val Gly Ser Ala Phe
           35                  40                  45
   Tyr Thr Ile Trp Ser Gly Trp His Arg Arg Thr Glu Glu Leu Pro Leu
       50                  55                  60
   Gly Arg Glu Leu Arg Val Pro Leu Ile Gly Ser Leu Pro Glu Ala Arg
   65                  70                  75                  80
   Leu Arg Arg Val Val Gly Gln Leu Asp Pro Gln Arg Leu Trp Ser Thr
                   85                  90                  95
   Tyr Leu Arg Pro Leu Leu Val Val Arg Thr Pro Gly Ser Pro Gly Asn
               100                 105                 110
   Leu Gln Val Arg Lys Phe Leu Glu Ala Thr Leu Arg Ser Leu Thr Ala
           115                 120                 125
   Gly Trp His Val Glu Leu Asp Pro Phe Thr Ala Ser Thr Pro Leu Gly
       130                 135                 140
   Pro Val Asp Phe Gly Asn Val Val Ala Thr Leu Asp Pro Arg Ala Ala
   145                 150                 155                 160
   Arg His Leu Thr Leu Ala Cys His Tyr Asp Ser Lys Leu Phe Pro Pro
                   165                 170                 175
   Gly Ser Thr Pro Phe Val Gly Ala Thr Asp Ser Ala Val Pro Cys Ala
               180                 185                 190
   Leu Leu Leu Glu Leu Ala Gln Ala Leu Asp Leu Glu Leu Ser Arg Ala
           195                 200                 205
   Lys Lys Gln Ala Ala Pro Val Thr Leu Gln Leu Leu Phe Leu Asp Gly
       210                 215                 220
   Glu Glu Ala Leu Lys Glu Trp Gly Pro Lys Asp Ser Leu Tyr Gly Ser
   225                 230                 235                 240
   Arg His Leu Ala Gln Leu Met Glu Ser Ile Pro His Ser Pro Gly Pro
                   245                 250                 255
   Thr Arg Ile Gln Ala Ile Glu Leu Phe Met Leu Leu Asp Leu Leu Gly
               260                 265                 270
   Ala Pro Asn Pro Thr Phe Tyr Ser His Phe Pro Arg Thr Val Arg Trp
           275                 280                 285
   Phe His Arg Leu Arg Ser Ile Glu Lys Arg Leu His Arg Leu Asn Leu
       290                 295                 300
   Leu Gln Ser His Pro Gln Glu Val Met Tyr Phe Gln Pro Gly Glu Pro
   305                 310                 315                 320
   Phe Gly Ser Val Glu Asp Asp His Ile Pro Phe Leu Arg Arg Gly Val
                   325                 330                 335
   Pro Val Leu His Leu Ile Ser Thr Pro Phe Pro Ala Val Trp His Thr
               340                 345                 350
   Pro Ala Asp Thr Glu Val Asn Leu His Pro Pro Thr Val His Asn Leu
           355                 360                 365
   Cys Arg Ile Leu Ala Val Phe Leu Ala Glu Tyr Leu Gly Leu
       370                 375                 380
<210> 2
   <211> 309
   <212> PRT
   <213> Human
<400> 2
   Gly Ser Leu Pro Glu Ala Arg Leu Arg Arg Val Val Gly Gln Leu Asp
   1               5                   10                  15
   Pro Gln Arg Leu Trp Ser Thr Tyr Leu Arg Pro Leu Leu Val Val Arg
               20                  25                  30
   Thr Pro Gly Ser Pro Gly Asn Leu Gln Val Arg Lys Phe Leu Glu Ala
           35                  40                  45
   Thr Leu Arg Ser Leu Thr Ala Gly Trp His Val Glu Leu Asp Pro Phe
       50                  55                  60
   Thr Ala Ser Thr Pro Leu Gly Pro Val Asp Phe Gly Asn Val Val Ala
   65                  70                  75                  80
   Thr Leu Asp Pro Arg Ala Ala Arg His Leu Thr Leu Ala Cys His Tyr
                   85                  90                  95
   Asp Ser Lys Leu Phe Pro Pro Gly Ser Thr Pro Phe Val Gly Ala Thr
               100                 105                 110
   Asp Ser Ala Val Pro Cys Ala Leu Leu Leu Glu Leu Ala Gln Ala Leu
           115                 120                 125
   Asp Leu Glu Leu Ser Arg Ala Lys Lys Gln Ala Ala Pro Val Thr Leu
       130                 135                 140
   Gln Leu Leu Phe Leu Asp Gly Glu Glu Ala Leu Lys Glu Trp Gly Pro
   145                 150                 155                 160
   Lys Asp Ser Leu Tyr Gly Ser Arg His Leu Ala Gln Leu Met Glu Ser
                   165                 170                 175
   Ile Pro His Ser Pro Gly Pro Thr Arg Ile Gln Ala Ile Glu Leu Phe
               180                 185                 190
   Met Leu Leu Asp Leu Leu Gly Ala Pro Asn Pro Thr Phe Tyr Ser His
           195                 200                 205
   Phe Pro Arg Thr Val Arg Trp Phe His Arg Leu Arg Ser Ile Glu Lys
       210                 215                 220
   Arg Leu His Arg Leu Asn Leu Leu Gln Ser His Pro Gln Glu Val Met
   225                 230                 235                 240
   Tyr Phe Gln Pro Gly Glu Pro Phe Gly Ser Val Glu Asp Asp His Ile
                   245                 250                 255
   Pro Phe Leu Arg Arg Gly Val Pro Val Leu His Leu Ile Ser Thr Pro
               260                 265                 270
   Phe Pro Ala Val Trp His Thr Pro Ala Asp Thr Glu Val Asn Leu His
           275                 280                 285
   Pro Pro Thr Val His Asn Leu Ala Arg Ile Leu Ala Val Phe Leu Ala
       290                 295                 300
   Glu Tyr Leu Gly Leu
   305
<210> 3
   <211> 307
   <212> PRT
   <213> Human
<400> 3
   Gly Ser Leu Pro Glu Ala Arg Leu Arg Arg Val Val Gly Gln Leu Asp
   1               5                   10                  15
   Pro Gln Arg Leu Trp Ser Thr Tyr Leu Arg Pro Leu Leu Val Val Arg
               20                  25                  30
   Thr Pro Gly Ser Pro Gly Asn Leu Gln Val Arg Lys Phe Leu Glu Ala
           35                  40                  45
   Thr Leu Arg Ser Leu Thr Ala Gly Trp His Val Glu Leu Asp Pro Phe
       50                  55                  60
   Thr Ala Ser Thr Pro Leu Gly Pro Val Asp Phe Gly Asn Val Val Ala
   65                  70                  75                  80
   Thr Leu Asp Pro Arg Ala Ala Arg His Leu Thr Leu Ala Cys His Tyr
                   85                  90                  95
   Asp Ser Lys Leu Phe Pro Pro Gly Ser Thr Pro Phe Val Gly Ala Thr
               100                 105                 110
   Asp Ser Ala Val Pro Cys Ala Leu Leu Leu Glu Leu Ala Gln Ala Leu
           115                 120                 125
   Asp Leu Glu Leu Ser Arg Ala Lys Lys Gln Ala Val Thr Leu Gln Leu
       130                 135                 140
   Leu Phe Leu Asp Gly Glu Glu Ala Leu Lys Glu Trp Gly Pro Lys Asp
   145                 150                 155                 160
   Ser Leu Tyr Gly Ser Arg His Leu Ala Gln Leu Met Glu Ser Ile Pro
                   165                 170                 175
   His Ser Pro Gly Pro Thr Arg Ile Gln Ala Ile Glu Leu Phe Met Leu
               180                 185                 190
   Leu Asp Leu Leu Gly Ala Pro Asn Pro Thr Phe Tyr Ser His Phe Pro
           195                 200                 205
   Arg Thr Val Arg Trp Phe His Arg Leu Arg Ser Ile Glu Lys Arg Leu
       210                 215                 220
   His Arg Leu Asn Leu Leu Gln Ser His Pro Gln Glu Val Met Tyr Phe
   225                 230                 235                 240
   Gln Pro Gly Glu Pro Phe Gly Ser Val Glu Asp Asp His Ile Pro Phe
                   245                 250                 255
   Leu Arg Arg Gly Val Pro Val Leu His Leu Ile Ser Thr Pro Phe Pro
               260                 265                 270
   Ala Val Trp His Thr Pro Ala Asp Thr Glu Val Asn Leu His Pro Pro
           275                 280                 285
   Thr Val His Asn Leu Ala Arg Ile Leu Ala Val Phe Leu Ala Glu Tyr
       290                 295                 300
   Leu Gly Leu
   305
<210> 4
   <211> 309
   <212> PRT
   <213> Human
<400> 4
   Gly Ser Leu Pro Glu Ala Arg Leu Arg Arg Val Val Gly Gln Leu Asp
   1               5                   10                  15
   Pro Gln Arg Leu Trp Ser Thr Tyr Leu Arg Pro Leu Leu Val Val Arg
               20                  25                  30
   Thr Pro Gly Ser Pro Gly Asn Leu Gln Val Arg Lys Phe Leu Glu Ala
           35                  40                  45
   Thr Leu Arg Ser Leu Thr Ala Gly Trp His Val Glu Leu Asp Pro Phe
       50                  55                  60
   Thr Ala Ser Thr Pro Leu Gly Pro Val Asp Phe Gly Asn Val Val Ala
   65                  70                  75                  80
   Thr Leu Asp Pro Arg Ala Ala Arg His Leu Thr Leu Ala Cys His Tyr
                   85                  90                  95
   Asp Ser Lys Leu Phe Pro Pro Gly Ser Thr Pro Phe Val Gly Ala Thr
               100                 105                 110
   Asp Ser Ala Val Pro Cys Ala Leu Leu Leu Glu Leu Ala Gln Ala Leu
           115                 120                 125
   Asp Leu Glu Leu Ser Arg Ala Lys Lys Gln Ala Ala Pro Val Thr Leu
       130                 135                 140
   Gln Leu Leu Phe Leu Asp Gly Glu Glu Ala Leu Lys Glu Trp Gly Pro
   145                 150                 155                 160
   Lys Asp Ser Leu Tyr Gly Ser Arg His Leu Ala Gln Leu Met Glu Ser
                   165                 170                 175
   Ile Pro His Ser Pro Gly Pro Thr Arg Ile Gln Ala Ile Glu Leu Phe
               180                 185                 190
   Met Leu Leu Asp Leu Leu Gly Ala Pro Asn Pro Thr Phe Tyr Ser His
   195 200 205
   Phe Pro Arg Thr Val Arg Trp Phe His Arg Leu Arg Ser Ile Glu Lys
   210 215 220
   Arg Leu His Arg Leu Asn Leu Leu Gln Ser His Pro Gln Glu Val Met
   225 230 235 240
   Tyr Phe Gln Pro Gly Glu Pro Phe Gly Ser Val Glu Asp Asp His Ile
   245 250 255
   Pro Phe Leu Arg Arg Gly Val Pro Val Leu His Leu Ile Ser Thr Pro
   260 265 270
   Phe Pro Ala Val Trp His Thr Pro Ala Asp Thr Glu Val Asn Leu His
   275 280 285
   Pro Pro Thr Val His Asn Leu Ala Arg Ile Leu Ala Val Phe Leu Ala
   290 295 300
   Glu Tyr Leu Gly Leu
   305
<210> 5
   <211> 308
   <212> PRT
   <213> Human
<400> 5
   Ser Leu Pro Glu Ala Arg Leu Arg Arg Val Val Gly Gln Leu Asp Pro
   1               5                   10                  15
   Gln Arg Leu Trp Ser Thr Tyr Leu Arg Pro Leu Leu Val Val Arg Thr
               20                  25                  30
   Pro Gly Ser Pro Gly Asn Leu Gln Val Arg Lys Phe Leu Glu Ala Thr
           35                  40                  45
   Leu Arg Ser Leu Thr Ala Gly Trp His Val Glu Leu Asp Pro Phe Thr
       50                  55                  60
   Ala Ser Thr Pro Leu Gly Pro Val Asp Phe Gly Asn Val Val Ala Thr
   65                  70                  75                  80
   Leu Asp Pro Arg Ala Ala Arg His Leu Thr Leu Ala Cys His Tyr Asp
                   85                  90                  95
   Ser Lys Leu Phe Pro Pro Gly Ser Thr Pro Phe Val Gly Ala Thr Asp
               100                 105                 110
   Ser Ala Val Pro Cys Ala Leu Leu Leu Glu Leu Ala Gln Ala Leu Asp
           115                 120                 125
   Leu Glu Leu Ser Arg Ala Lys Lys Gln Ala Ala Pro Val Thr Leu Gln
       130                 135                 140
   Leu Leu Phe Leu Asp Gly Glu Glu Ala Leu Lys Glu Trp Gly Pro Lys
   145                 150                 155                 160
   Asp Ser Leu Tyr Gly Ser Arg His Leu Ala Gln Leu Met Glu Ser Ile
                   165                 170                 175
   Pro His Ser Pro Gly Pro Thr Arg Ile Gln Ala Ile Glu Leu Phe Met
               180                 185                 190
   Leu Leu Asp Leu Leu Gly Ala Pro Asn Pro Thr Phe Tyr Ser His Phe
           195                 200                 205
   Pro Arg Thr Val Arg Trp Phe His Arg Leu Arg Ser Ile Glu Lys Arg
       210                 215                 220
   Leu His Arg Leu Asn Leu Leu Gln Ser His Pro Gln Glu Val Met Tyr
   225                 230                 235                 240
   Phe Gln Pro Gly Glu Pro Phe Gly Ser Val Glu Asp Asp His Ile Pro
                   245                 250                 255
   Phe Leu Arg Arg Gly Val Pro Val Leu His Leu Ile Ser Thr Pro Phe
               260                 265                 270
   Pro Ala Val Trp His Thr Pro Ala Asp Thr Glu Val Asn Leu His Pro
           275                 280                 285
   Pro Thr Val His Asn Leu Ala Arg Ile Leu Ala Val Phe Leu Ala Glu
       290                 295                 300
   Tyr Leu Gly Leu
   305
<210> 6
   <211> 310
   <212> PRT
   <213> Human
<400> 6
   Asn Gly Ser Leu Pro Glu Ala Arg Leu Arg Arg Val Val Gly Gln Leu
   1               5                   10                  15
   Asp Pro Gln Arg Leu Trp Ser Thr Tyr Leu Arg Pro Leu Leu Val Val
               20                  25                  30
   Arg Thr Pro Gly Ser Pro Gly Asn Leu Gln Val Arg Lys Phe Leu Glu
           35                  40                  45
   Ala Thr Leu Arg Ser Leu Thr Ala Gly Trp His Val Glu Leu Asp Pro
           50                  55                  60
   Phe Thr Ala Ser Thr Pro Leu Gly Pro Val Asp Phe Gly Asn Val Val
   65                  70                  75                  80
   Ala Thr Leu Asp Pro Arg Ala Ala Arg His Leu Thr Leu Ala Cys His
                   85                  90                  95
   Tyr Asp Ser Lys Leu Phe Pro Pro Gly Ser Thr Pro Phe Val Gly Ala
               100                 105                 110
   Thr Asp Ser Ala Val Pro Cys Ala Leu Leu Leu Glu Leu Ala Gln Ala
           115                 120                 125
   Leu Asp Leu Glu Leu Ser Arg Ala Lys Lys Gln Ala Ala Pro Val Thr
       130                 135                 140
   Leu Gln Leu Leu Phe Leu Asp Gly Glu Glu Ala Leu Lys Glu Trp Gly
   145                 150                 155                 160
   Pro Lys Asp Ser Leu Tyr Gly Ser Arg His Leu Ala Gln Leu Met Glu
                   165                 170                 175
   Ser Ile Pro His Ser Pro Gly Pro Thr Arg Ile Gln Ala Ile Glu Leu
               180                 185                 190
   Phe Met Leu Leu Asp Leu Leu Gly Ala Pro Asn Pro Thr Phe Tyr Ser
           195                 200                 205
   His Phe Pro Arg Thr Val Arg Trp Phe His Arg Leu Arg Ser Ile Glu
       210                 215                 220
   Lys Arg Leu His Arg Leu Asn Leu Leu Gln Ser His Pro Gln Glu Val
   225                 230                 235                 240
   Met Tyr Phe Gln Pro Gly Glu Pro Phe Gly Ser Val Glu Asp Asp His
                   245                 250                 255
   Ile Pro Phe Leu Arg Arg Gly Val Pro Val Leu His Leu Ile Ser Thr
               260                 265                 270
   Pro Phe Pro Ala Val Trp His Thr Pro Ala Asp Thr Glu Val Asn Leu
           275                 280                 285
   His Pro Pro Thr Val His Asn Leu Ala Arg Ile Leu Ala Val Phe Leu
       290                 295                 300
   Ala Glu Tyr Leu Gly Leu
   305                 310
<210> 7
   <211> 308
   <212> PRT
   <213> Human
<400> 7
   Ser Leu Pro Glu Ala Arg Leu Arg Arg Val Val Gly Gln Leu Asp Pro
   1               5                   10                  15
   Gln Arg Leu Trp Ser Thr Tyr Leu Arg Pro Leu Leu Val Val Arg Thr
               20                  25                  30
   Pro Gly Ser Pro Gly Asn Leu Gln Val Arg Lys Phe Leu Glu Ala Thr
           35                  40                  45
   Leu Arg Ser Leu Thr Ala Gly Trp His Val Glu Leu Asp Pro Phe Thr
       50                  55                  60
   Ala Ser Thr Pro Leu Gly Pro Val Asp Phe Gly Asn Val Val Ala Thr
   65                  70                  75                  80
   Leu Asp Pro Arg Ala Ala Arg His Leu Thr Leu Ala Cys His Tyr Asp
                   85                  90                  95
   Ser Lys Leu Phe Pro Pro Gly Ser Thr Pro Phe Val Gly Ala Thr Asp
               100                 105                 110
   Ser Ala Val Pro Cys Ala Leu Leu Leu Glu Leu Ala Gln Ala Leu Asp
           115                 120                 125
   Leu Glu Leu Ser Arg Ala Lys Lys Gln Ala Ala Pro Val Thr Leu Gln
       130                 135                 140
   Leu Leu Phe Leu Asp Gly Glu Glu Ala Leu Lys Glu Trp Gly Pro Lys
   145                 150                 155                 160
   Asp Ser Leu Tyr Gly Ser Arg His Leu Ala Gln Leu Met Glu Ser Ile
                   165                 170                 175
   Pro His Ser Pro Gly Pro Thr Arg Ile Gln Ala Ile Glu Leu Phe Met
               180                 185                 190
   Leu Leu Asp Leu Leu Gly Ala Pro Asn Pro Thr Phe Tyr Ser His Phe
           195                 200                 205
   Pro Arg Thr Val Arg Trp Phe His Arg Leu Arg Ser Ile Glu Lys Arg
       210                 215                 220
   Leu His Arg Leu Asn Leu Leu Gln Ser His Pro Gln Glu Val Met Tyr
   225                 230                 235                 240
   Phe Gln Pro Gly Glu Pro Phe Gly Ser Val Glu Asp Asp His Ile Pro
                   245                 250                 255
   Phe Leu Arg Arg Gly Val Pro Val Leu His Leu Ile Ser Thr Pro Phe
               260                 265                 270
   Pro Ala Val Trp His Thr Pro Ala Asp Thr Glu Val Asn Leu His Pro
           275                 280                 285
   Pro Thr Val His Asn Leu Ala Arg Ile Leu Ala Val Phe Leu Ala Glu
       290                 295                 300
   Tyr Leu Gly Leu
   305
<210> 8
   <211> 303
   <212> PRT
   <213> Human
<400> 8
   Ser Leu Pro Glu Ala Arg Leu Arg Arg Val Val Gly Gln Leu Asp Pro
   1               5                   10                  15
   Gln Arg Leu Trp Ser Thr Tyr Leu Arg Pro Leu Leu Val Val Arg Thr
               20                  25                  30
   Pro Gly Ser Pro Gly Asn Leu Gln Val Arg Lys Phe Leu Glu Ala Thr
           35                  40                  45
   Leu Arg Ser Leu Thr Ala Gly Trp His Val Glu Leu Asp Pro Phe Thr
       50                  55                  60
   Ala Ser Thr Pro Leu Gly Pro Val Asp Phe Gly Asn Val Val Ala Thr
   65                  70                  75                  80
   Leu Asp Pro Arg Ala Ala Arg His Leu Thr Leu Ala Cys His Tyr Asp
                   85                  90                  95
   Ser Lys Leu Phe Pro Ser Thr Pro Phe Val Gly Ala Thr Asp Ser Ala
               100                 105                 110
   Val Pro Cys Ala Leu Leu Leu Glu Leu Ala Gln Ala Leu Asp Leu Glu
           115                 120                 125
   Leu Ser Arg Ala Lys Lys Pro Val Thr Leu Gln Leu Leu Phe Leu Asp
       130                 135                 140
   Gly Glu Glu Ala Leu Lys Glu Trp Gly Pro Lys Asp Ser Leu Tyr Gly
   145                 150                 155                 160
   Ser Arg His Leu Ala Gln Leu Met Glu Ser Ile Pro His Ser Pro Gly
                   165                 170                 175
   Pro Thr Arg Ile Gln Ala Ile Glu Leu Phe Met Leu Leu Asp Leu Leu
               180                 185                 190
   Gly Ala Pro Asn Pro Thr Phe Tyr Ser His Phe Pro Arg Thr Val Arg
           195                 200                 205
   Trp Phe His Arg Leu Arg Ser Ile Glu Lys Arg Leu His Arg Leu Asn
       210                 215                 220
   Leu Leu Gln Ser His Pro Gln Glu Val Met Tyr Phe Gln Pro Gly Glu
   225                 230                 235                 240
   Pro Phe Gly Ser Val Glu Asp Asp His Ile Pro Phe Leu Arg Arg Gly
                   245                 250                 255
   Val Pro Val Leu His Leu Ile Ser Thr Pro Phe Pro Ala Val Trp His
               260                 265                 270
   Thr Pro Ala Asp Thr Glu Val Asn Leu His Pro Pro Thr Val His Asn
           275                 280                 285
   Leu Ala Arg Ile Leu Ala Val Phe Leu Ala Glu Tyr Leu Gly Leu
       290                 295                 300
<210> 9
   <211> 302
   <212> PRT
   <213> Human
<400> 9
   Leu Pro Glu Ala Arg Leu Arg Arg Val Val Gly Gln Leu Asp Pro Gln
   1               5                   10                  15
   Arg Leu Trp Ser Thr Tyr Leu Arg Pro Leu Leu Val Val Arg Thr Pro
               20                  25                  30
   Gly Ser Pro Gly Asn Leu Gln Val Arg Lys Phe Leu Glu Ala Thr Leu
           35                  40                  45
   Arg Ser Leu Thr Ala Gly Trp His Val Glu Leu Asp Pro Phe Thr Ala
       50                  55                  60
   Ser Thr Pro Leu Gly Pro Val Asp Phe Gly Asn Val Val Ala Thr Leu
   65                  70                  75                  80
   Asp Pro Arg Ala Ala Arg His Leu Thr Leu Ala Cys His Tyr Asp Ser
                   85                  90                  95
   Lys Leu Phe Pro Pro Gly Ser Thr Pro Phe Val Gly Ala Thr Asp Ser
               100                 105                 110
   Ala Val Pro Cys Ala Leu Leu Leu Glu Leu Ala Gln Ala Leu Asp Leu
           115                 120                 125
   Glu Leu Ser Arg Ala Lys Val Thr Leu Gln Leu Leu Phe Leu Asp Gly
       130                 135                 140
   Glu Glu Ala Leu Lys Glu Trp Gly Pro Lys Asp Ser Leu Tyr Gly Ser
   145                 150                 155                 160
   Arg His Leu Ala Gln Leu Met Glu Ser Ile Pro His Ser Pro Gly Pro
                   165                 170                 175
   Thr Arg Ile Gln Ala Ile Glu Leu Phe Met Leu Leu Asp Leu Leu Gly
               180                 185                 190
   Ala Pro Asn Pro Thr Phe Tyr Ser His Phe Pro Arg Thr Val Arg Trp
           195                 200                 205
   Phe His Arg Leu Arg Ser Ile Glu Lys Arg Leu His Arg Leu Asn Leu
       210                 215                 220
   Leu Gln Ser His Pro Gln Glu Val Met Tyr Phe Gln Pro Gly Glu Pro
   225                 230                 235                 240
   Phe Gly Ser Val Glu Asp Asp His Ile Pro Phe Leu Arg Arg Gly Val
                   245                 250                 255
   Pro Val Leu His Leu Ile Ser Thr Pro Phe Pro Ala Val Trp His Thr
               260                 265                 270
   Pro Ala Asp Thr Glu Val Asn Leu His Pro Pro Thr Val His Asn Leu
           275                 280                 285
   Ala Arg Ile Leu Ala Val Phe Leu Ala Glu Tyr Leu Gly Leu
       290                 295                 300
<210> 10
   <211> 300
   <212> PRT
   <213> Human
<400> 10
   Ser Leu Pro Glu Ala Arg Leu Arg Arg Val Val Gly Gln Leu Asp Pro
   1               5                   10                  15
   Gln Arg Leu Trp Ser Thr Tyr Leu Arg Pro Leu Leu Val Val Arg Thr
               20                  25                  30
   Pro Gly Ser Pro Gly Asn Leu Gln Val Arg Lys Phe Leu Glu Ala Thr
           35                  40                  45
   Leu Arg Ser Leu Thr Ala Gly Trp His Val Glu Leu Asp Pro Phe Thr
       50                  55                  60
   Ala Ser Thr Pro Leu Gly Pro Val Asp Phe Gly Asn Val Val Ala Thr
   65                  70                  75                  80
   Leu Asp Pro Arg Ala Ala Arg His Leu Thr Leu Ala Cys His Tyr Asp
   85 90 95
   Ser Lys Leu Phe Ser Thr Pro Phe Val Gly Ala Thr Asp Ser Ala Val
   100 105 110
   Pro Cys Ala Leu Leu Leu Glu Leu Ala Gln Ala Leu Asp Leu Glu Leu
   115 120 125
   Ser Arg Ala Lys Val Thr Leu Gln Leu Leu Phe Leu Asp Gly Glu Glu
   130 135 140
   Ala Leu Lys Glu Trp Gly Pro Lys Asp Ser Leu Tyr Gly Ser Arg His
   145 150 155 160
   Leu Ala Gln Leu Met Glu Ser Ile Pro His Ser Pro Gly Pro Thr Arg
   165 170 175
   Ile Gln Ala Ile Glu Leu Phe Met Leu Leu Asp Leu Leu Gly Ala Pro
   180 185 190
   Asn Pro Thr Phe Tyr Ser His Phe Pro Arg Thr Val Arg Trp Phe His
   195 200 205
   Arg Leu Arg Ser Ile Glu Lys Arg Leu His Arg Leu Asn Leu Leu Gln
   210 215 220
   Ser His Pro Gln Glu Val Met Tyr Phe Gln Pro Gly Glu Pro Phe Gly
   225 230 235 240
   Ser Val Glu Asp Asp His Ile Pro Phe Leu Arg Arg Gly Val Pro Val
   245 250 255
   Leu His Leu Ile Ser Thr Pro Phe Pro Ala Val Trp His Thr Pro Ala
   260 265 270
   Asp Thr Glu Val Asn Leu His Pro Pro Thr Val His Asn Leu Ala Arg
   275 280 285
   Ile Leu Ala Val Phe Leu Ala Glu Tyr Leu Gly Leu
   290 295 300
<210> 11
   <211> 302
   <212> PRT
   <213> Human
<400> 11
   Leu Pro Glu Ala Arg Leu Arg Arg Val Val Gly Gln Leu Asp Pro Gln
   1 5 10 15
   Arg Leu Trp Ser Thr Tyr Leu Arg Pro Leu Leu Val Val Arg Thr Pro
               20                  25                  30
   Gly Ser Pro Gly Asn Leu Gln Val Arg Lys Phe Leu Glu Ala Thr Leu
           35                  40                  45
   Arg Ser Leu Thr Ala Gly Trp His Val Glu Leu Asp Pro Phe Thr Ala
       50                  55                  60
   Ser Thr Pro Leu Gly Pro Val Asp Phe Gly Asn Val Val Ala Thr Leu
   65                  70                  75                  80
   Asp Pro Arg Ala Ala Arg His Leu Thr Leu Ala Cys His Tyr Asp Ser
                   85                  90                  95
   Lys Leu Phe Pro Pro Gly Ser Thr Pro Phe Val Gly Ala Thr Asp Ser
               100                 105                 110
   Ala Val Pro Cys Ala Leu Leu Leu Glu Leu Ala Gln Ala Leu Asp Leu
           115                 120                 125
   Glu Leu Ser Arg Ala Lys Val Thr Leu Gln Leu Leu Phe Leu Asp Gly
       130                 135                 140
   Glu Glu Ala Leu Lys Glu Trp Gly Pro Lys Asp Ser Leu Tyr Gly Ser
   145                 150                 155                 160
   Arg His Leu Ala Gln Leu Met Glu Ser Ile Pro His Ser Pro Gly Pro
                   165                 170                 175
   Thr Arg Ile Gln Ala Ile Glu Leu Phe Met Leu Leu Asp Leu Leu Gly
               180                 185                 190
   Ala Pro Asn Pro Thr Phe Tyr Ser His Phe Pro Arg Thr Val Arg Trp
           195                 200                 205
   Phe His Arg Leu Arg Ser Ile Glu Lys Arg Leu His Arg Leu Asn Leu
       210                 215                 220
   Leu Gln Ser His Pro Gln Glu Val Met Tyr Phe Gln Pro Gly Glu Pro
   225                 230                 235                 240
   Phe Gly Ser Val Glu Asp Asp His Ile Pro Phe Leu Arg Arg Gly Val
                   245                 250                 255
   Pro Val Leu His Leu Ile Ser Thr Pro Phe Pro Ala Val Trp His Thr
               260                 265                 270
   Pro Ala Asp Thr Glu Val Asn Leu His Pro Pro Thr Val His Asn Leu
           275                 280                 285
   Ala Arg Ile Leu Ala Val Phe Leu Ala Glu Tyr Leu Gly Leu
   290 295 300
<210> 12
   <211> 298
   <212> PRT
   <213> Human
<400> 12
   Pro Glu Ala Arg Leu Arg Arg Val Val Gly Gln Leu Asp Pro Gln Arg
   1               5                   10                  15
   Leu Trp Ser Thr Tyr Leu Arg Pro Leu Leu Val Val Arg Thr Pro Gly
               20                  25                  30
   Ser Pro Gly Asn Leu Gln Val Arg Lys Phe Leu Glu Ala Thr Leu Arg
           35                  40                  45
   Ser Leu Thr Ala Gly Trp His Val Glu Leu Asp Pro Phe Thr Ala Ser
       50                  55                  60
   Thr Pro Leu Gly Pro Val Asp Phe Gly Asn Val Val Ala Thr Leu Asp
   65                  70                  75                  80
   Pro Arg Ala Ala Arg His Leu Thr Leu Ala Cys His Tyr Asp Ser Lys
                   85                  90                  95
   Leu Phe Ser Thr Pro Phe Val Gly Ala Thr Asp Ser Ala Val Pro Cys
               100                 105                 110
   Ala Leu Leu Leu Glu Leu Ala Gln Ala Leu Asp Leu Glu Leu Ser Arg
           115                 120                 125
   Ala Lys Val Thr Leu Gln Leu Leu Phe Leu Asp Gly Glu Glu Ala Leu
       130                 135                 140
   Lys Glu Trp Gly Pro Lys Asp Ser Leu Tyr Gly Ser Arg His Leu Ala
   145                 150                 155                 160
   Gln Leu Met Glu Ser Ile Pro His Ser Pro Gly Pro Thr Arg Ile Gln
                   165                 170                 175
   Ala Ile Glu Leu Phe Met Leu Leu Asp Leu Leu Gly Ala Pro Asn Pro
               180                 185                 190
   Thr Phe Tyr Ser His Phe Pro Arg Thr Val Arg Trp Phe His Arg Leu
           195                 200                 205
   Arg Ser Ile Glu Lys Arg Leu His Arg Leu Asn Leu Leu Gln Ser His
       210                 215                 220
   Pro Gln Glu Val Met Tyr Phe Gln Pro Gly Glu Pro Phe Gly Ser Val
   225                 230                 235                 240
   Glu Asp Asp His Ile Pro Phe Leu Arg Arg Gly Val Pro Val Leu His
                   245                 250                 255
   Leu Ile Ser Thr Pro Phe Pro Ala Val Trp His Thr Pro Ala Asp Thr
               260                 265                 270
   Glu Val Asn Leu His Pro Pro Thr Val His Asn Leu Ala Arg Ile Leu
           275                 280                 285
   Ala Val Phe Leu Ala Glu Tyr Leu Gly Leu
       290                 295
<210> 13
   <211> 48
   <212> DNA
   <213> Artificial
<220>
   <223> Synthetic Primer
<400> 13
   tatgaattcc atcaccatca ccatcacgag gagctgccgc tgggccgg 48
<210> 14
   <211> 35
   <212> DNA
   <213> Artificial
<220>
   <223> Synthetic Primer
<400> 14
   atatatgcgg ccgcctagag ccccaggtat tcagc 35
<210> 15
   <211> 33
   <212> DNA
   <213> Artificial
<220>
   <223> Synthetic Primer
<400> 15
   ctgcgggtcc cattgaacgg aagcctcccc gaa 33
<210> 16
   <211> 33
   <212> DNA
   <213> Artificial
<220>
   <223> Synthetic Primer
<400> 16
   ttcggggagg cttccgttca atgggacccg cag 33
<210> 17
   <211> 33
   <212> DNA
   <213> Artificial
<220>
   <223> Synthetic Primer
<400> 17
   acggtacaca acttggcccg cattctcgct gtg 33
<210> 18
   <211> 33
   <212> DNA
   <213> Artificial
<220>
   <223> Synthetic Primer
<400> 18
   cacagcgaga atgcgggcca agttgtgtac cgt 33
<210> 19
   <211> 331
   <212> PRT
   <213> Human
<400> 19
   Glu Phe His His His His His His Glu Glu Leu Pro Leu Gly Arg Glu
   1               5                   10                  15
   Leu Arg Val Pro Leu Asn Gly Ser Leu Pro Glu Ala Arg Leu Arg Arg
               20                  25                  30
   Val Val Gly Gln Leu Asp Pro Gln Arg Leu Trp Ser Thr Tyr Leu Arg
           35                  40                  45
   Pro Leu Leu Val Val Arg Thr Pro Gly Ser Pro Gly Asn Leu Gln Val
       50                  55                  60
   Arg Lys Phe Leu Glu Ala Thr Leu Arg Ser Leu Thr Ala Gly Trp His
   65                  70                  75                  80
   Val Glu Leu Asp Pro Phe Thr Ala Ser Thr Pro Leu Gly Pro Val Asp
                   85                  90                  95
   Phe Gly Asn Val Val Ala Thr Leu Asp Pro Arg Ala Ala Arg His Leu
               100                 105                 110
   Thr Leu Ala Cys His Tyr Asp Ser Lys Leu Phe Pro Pro Gly Ser Thr
           115                 120                 125
   Pro Phe Val Gly Ala Thr Asp Ser Ala Val Pro Cys Ala Leu Leu Leu
       130                 135                 140
   Glu Leu Ala Gln Ala Leu Asp Leu Glu Leu Ser Arg Ala Lys Lys Gln
   145                 150                 155                 160
   Ala Ala Pro Val Thr Leu Gln Leu Leu Phe Leu Asp Gly Glu Glu Ala
                   165                 170                 175
   Leu Lys Glu Trp Gly Pro Lys Asp Ser Leu Tyr Gly Ser Arg His Leu
               180                 185                 190
   Ala Gln Leu Met Glu Ser Ile Pro His Ser Pro Gly Pro Thr Arg Ile
           195                 200                 205
   Gln Ala Ile Glu Leu Phe Met Leu Leu Asp Leu Leu Gly Ala Pro Asn
       210                 215                 220
   Pro Thr Phe Tyr Ser His Phe Pro Arg Thr Val Arg Trp Phe His Arg
   225                 230                 235                 240
   Leu Arg Ser Ile Glu Lys Arg Leu His Arg Leu Asn Leu Leu Gln Ser
                   245                 250                 255
   His Pro Gln Glu Val Met Tyr Phe Gln Pro Gly Glu Pro Phe Gly Ser
               260                 265                 270
   Val Glu Asp Asp His Ile Pro Phe Leu Arg Arg Gly Val Pro Val Leu
           275                 280                 285
   His Leu Ile Ser Thr Pro Phe Pro Ala Val Trp His Thr Pro Ala Asp
       290                 295                 300
   Thr Glu Val Asn Leu His Pro Pro Thr Val His Asn Leu Ala Arg Ile
   305                 310                 315                 320
   Leu Ala Val Phe Leu Ala Glu Tyr Leu Gly Leu
                   325                 330

## Description

### FIELD OF THE INVENTION

The invention relates to the novel crystal structure of isoglutaminyl cyclase (isoQC). QC catalyzes the intramolecular cyclization of N-terminal glutamine residues into pyroglutamic acid (5-oxo-prolyl, pGlu*) under liberation of ammonia and the intramolecular cyclization of N-terminal glutamate residues into pyroglutamic acid under liberation of water.

### BACKGROUND OF THE INVENTION

Glutaminyl cyclase (QC, EC 2.3.2.5; Qpct; glutaminyl peptide cyclotransferase) catalyzes the intramolecular cyclization of N-terminal glutamine residues into pyroglutamic acid (5-oxo-proline, pGlu*) under liberation of ammonia and the intramolecular cyclization of N-terminal glutamate residues into pyroglutamic acid under liberation of water.

A QC was first isolated by Messer from the Latex of the tropical plant Carica papaya in 1963 (Messer, M. 1963 Nature 4874, 1299). 24 years later, a corresponding enzymatic activity was discovered in animal pituitary (Busby, W. H. J. et al. 1987 J Biol Chem 262, 8532-8536; Fischer, W. H. and Spiess, J. 1987 Proc Natl Acad Sci U S A 84, 3628-3632). For the mammalian QC, the conversion of Gin into pGlu by QC could be shown for the precursors of TRH and GnRH (Busby, W. H. J. et al. 1987 J Biol Chem 262, 8532-8536; Fischer, W. H. and Spiess, J. 1987 Proc Natl Acad Sci U S A 84, 3628-3632). In addition, initial localization experiments of QC revealed a co-localization with its putative products of catalysis in bovine pituitary, further improving the suggested function in peptide hormone synthesis (Bockers, T. M. et al. 1995 J Neuroendocrinol 7, 445-453). In contrast, the physiological function of the plant QC is less clear. In the case of the enzyme from *C*. *papaya,* a role in the plant defense against pathogenic microorganisms was suggested (El Moussaoui, A. et al. 2001 Cell Mol Life Sci 58, 556-570). Putative QCs from other plants were identified by sequence comparisons recently (Dahl, S. W. et al. 2000 Protein Expr Purif 20, 27-36). The physiological function of these enzymes, however, is still ambiguous.

The QCs known from plants and animals show a strict specificity for L-glutamine in the N-terminal position of the substrates and their kinetic behavior was found to obey the Michaelis-Menten equation (Pohl, T. et al. 1991 Proc Natl Acad Sci U S A 88, 10059-10063; Consalvo, A. P. et al. 1988 Anal Biochem 175, 131-138; Gololobov, M. Y. et al. 1996 Biol Chem Hoppe Seyler 377, 395-398). A comparison of the primary structures of the QCs from *C*. *papaya* and that of the highly conserved QC from mammals, however, did not reveal any sequence homology (Dahl, S. W. et al. 2000 Protein Expr Purif 20, 27-36). Whereas the plant QCs appear to belong to a new enzyme family (Dahl, S. W. et al. 2000 Protein Expr Purif 20, 27-36), the mammalian QCs were found to have a pronounced sequence homology to bacterial aminopeptidases (Bateman, R. C. et al. 2001 Biochemistry 40, 11246-11250), leading to the conclusion that the QCs from plants and animals have different evolutionary origins.

Recently, it was shown that recombinant human QC as well as QC-activity from brain extracts catalyze both, the N-terminal glutaminyl as well as glutamate cyclization. Most striking is the finding, that cyclase-catalyzed Glu₁-conversion is favored around pH 6.0 while Gln₁-conversion to pGlu-derivatives occurs with a pH-optimum of around 8.0. Since the formation of pGlu-Aβ-related peptides can be suppressed by inhibition of recombinant human QC and QC-activity from pig pituitary extracts, the enzyme QC is a target in drug development for treatment of Alzheimer's disease.

Isoenzymes of QC (i.e. isoglutaminyl peptide cyclotransferase; isoQC; QPCTL) have been described in WO 2008/034891, WO 2008/087197 and WO 2010/026209 (each in the name of Probiodrug AG).

US 7,572,614 (Wang et al) and Huang et al (2005) PNAS 102(37), 13117-13122 both describe one example of the crystal structure of soluble glutaminyl cyclase. The crystal structure disclosed in Wang *et al* and Huang *et al* was generated using a protein expressed in *E. coli,* which results in a lack of glycosylation. It is well known that all mammalian QC contain at least one glycosylation site (Pohl, T. et al. (1991) Proc Natl Acad Sci U S A 88, 10059-10063; Song, I. et al. (1994) J Mol Endocrinol 13, 77-86), which is glycosylated in the isoQC crystallized according to the invention by virtue of being expressed in eukaryotic hosts, which can be observed in the crystal structures presented herein. In addition, all mammalian QCs contain two conserved cysteine residues close to the active site, which form a disulfide bond. In the crystal structure of Wang *et al* and Huang *et al,* the disulfide bond is lacking. The expression of mammalian secretory proteins in bacteria frequently results in the absence of disulfide formation (Hannig, G. and Makrides, S. C. (1998) Trends Biotechnol 16, 54-60). The disulfide bond is clearly present in the human isoQC crystal structure presented herein. Notably, mutational analyses conducted by the inventors with human QC have suggested an important stabilizing function of the disulfide bond upon the overall structure. Furthermore, in the structure of Wang *et al* and Huang *et al,* a segment of residues (L205-H206-W207) close to the active site appears in two different conformations. Due to the orientations, the binding mode of substrates is affected and reliable mechanistic conclusions could not be drawn (Huang *et al.,* 2005).

The expression of human isoQC in an eukaryotic host, as described in the present invention, allows the crystallization and structural refinement of a native mammalian isoQC and, importantly, unambiguous determination of the binding modes of inhibitors.

### SUMMARY OF THE INVENTION

According to a first aspect of the invention there is provided a crystal comprising human isoglutaminyl cyclase having a characterised space group of P1211 and unit cell dimensions of +/- 5% of a = 126.51 Å, b = 109.68 Å, c = 159.53 Å, α = 90.0°, β = 104.9° and γ = 90.0°.

According to a second aspect of the invention there is provided a method of preparing the crystal of human isoglutaminyl cyclase as described herein, which comprises the steps of:
(a) providing a solution of human isoglutaminyl cyclase, optionally in the presence of a known isoglutaminyl cyclase inhibitor, in a suitable buffer such as 25mM Bis-Tris pH6.8 / 100mM NaCl buffer;
(b) mixing the solution with a crystallization solution comprising 0.1 M sodium citrate, 0.1 M ammonium sulfate and 13% (w/v) 35000 PEG; and
(c) incubating the mixture under conditions to promote hanging drop vapor diffusion for a time sufficient to produce the crystal of human isoglutaminyl cyclase.

According to a third aspect there is provided a crystal comprising human isoglutaminyl cyclase obtainable by the crystallisation method as defined herein.

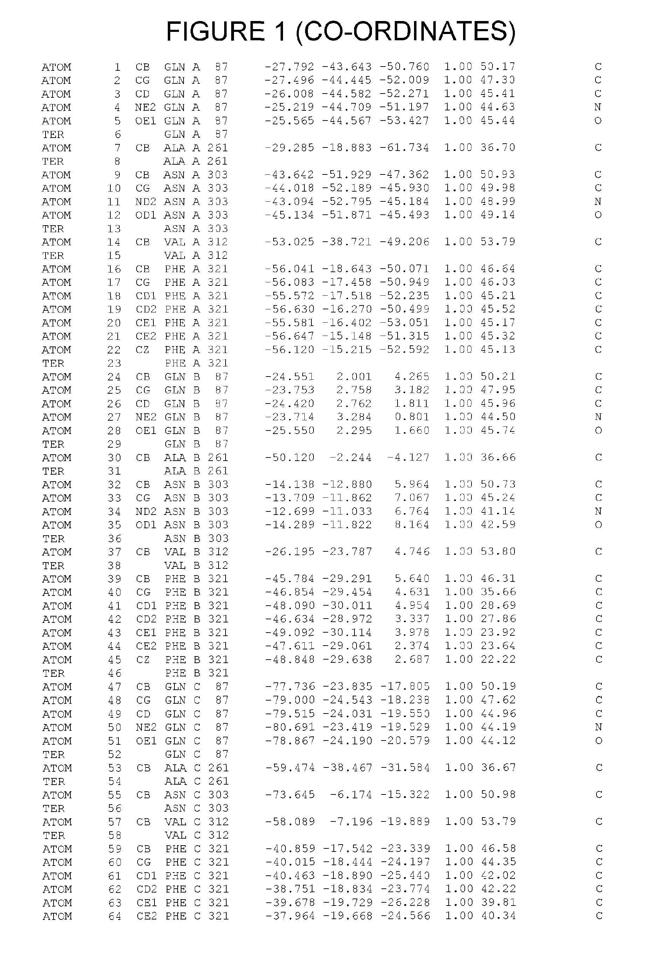

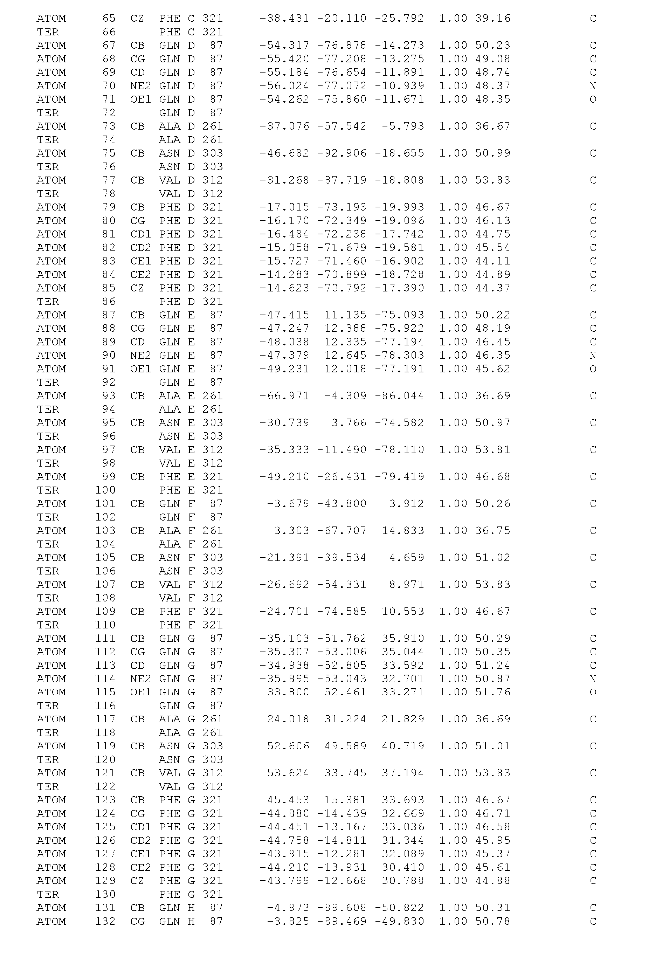

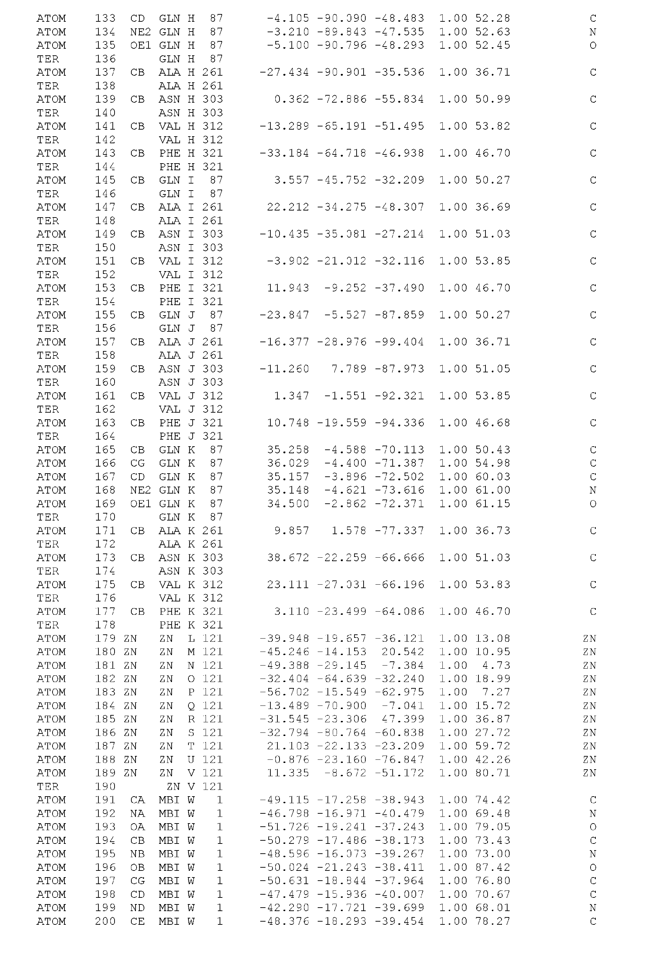

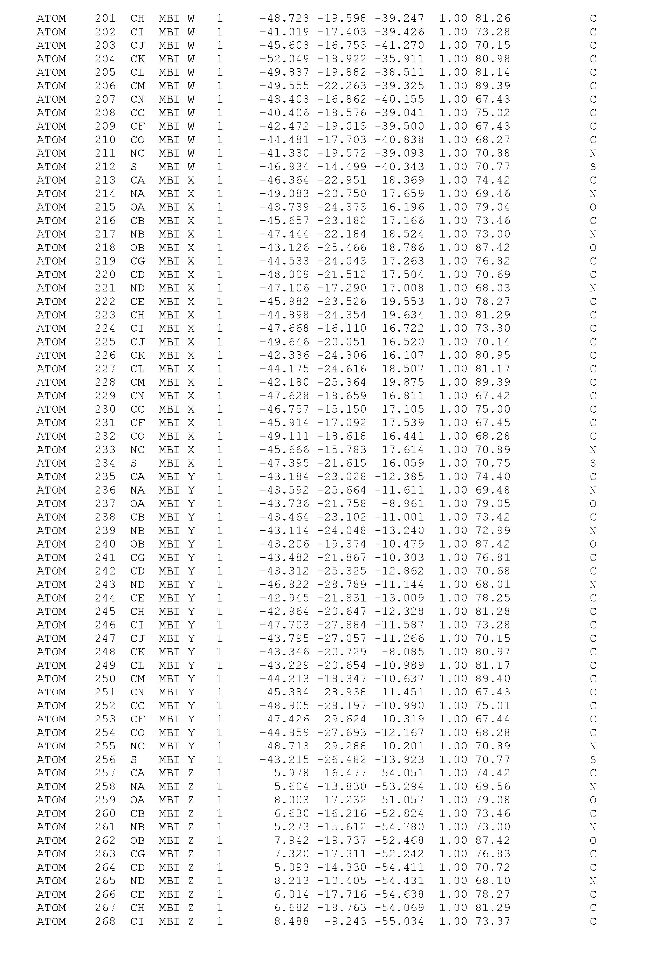

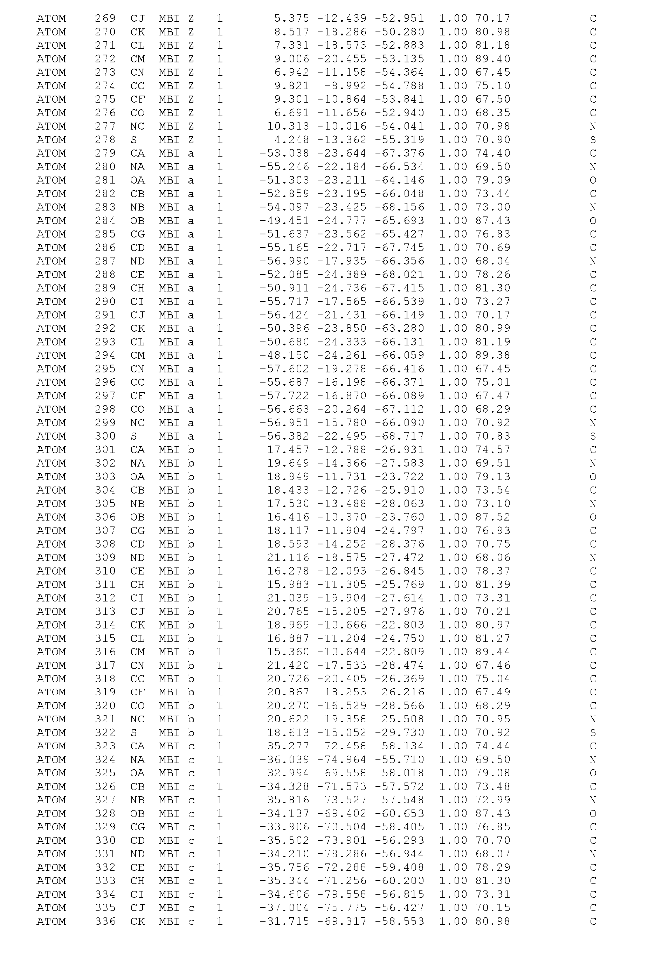

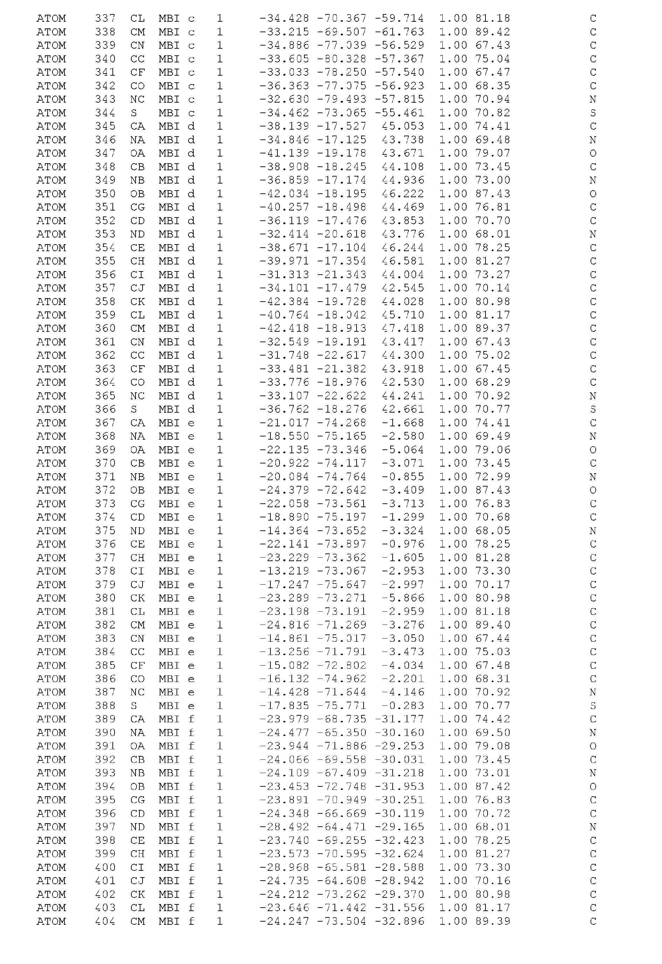

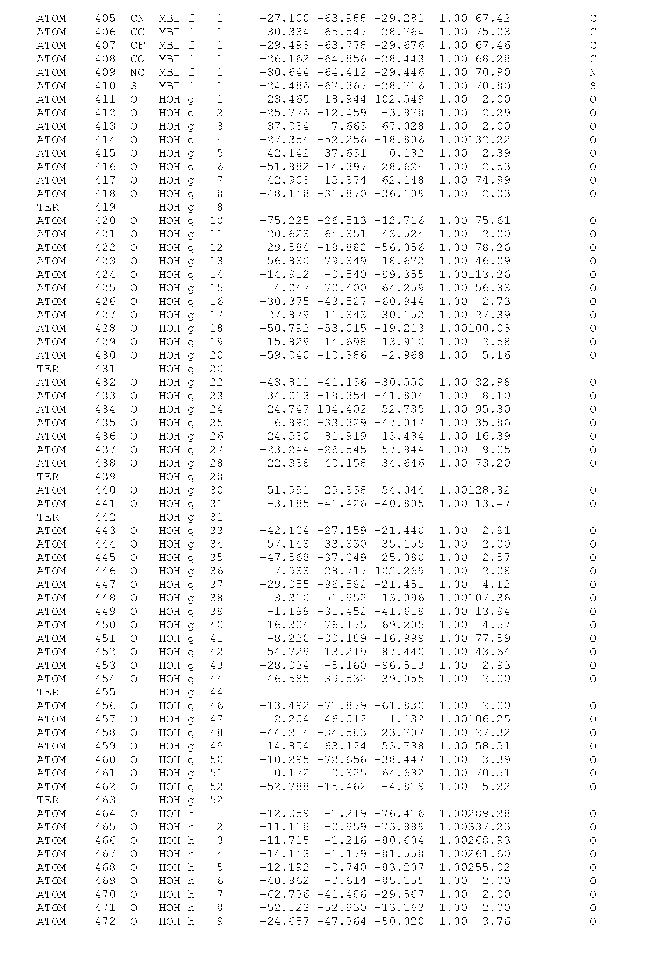

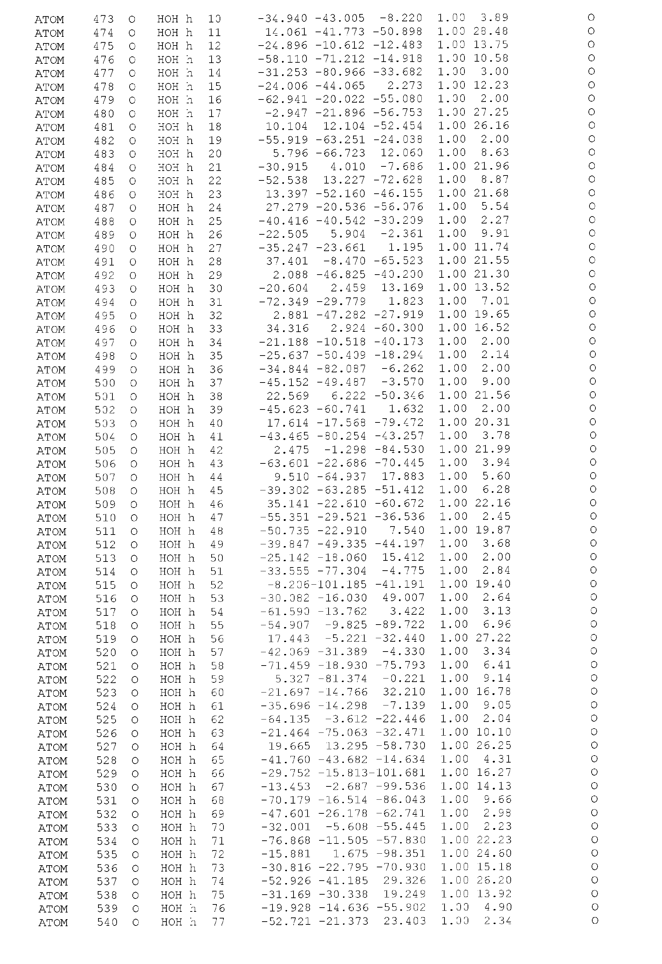

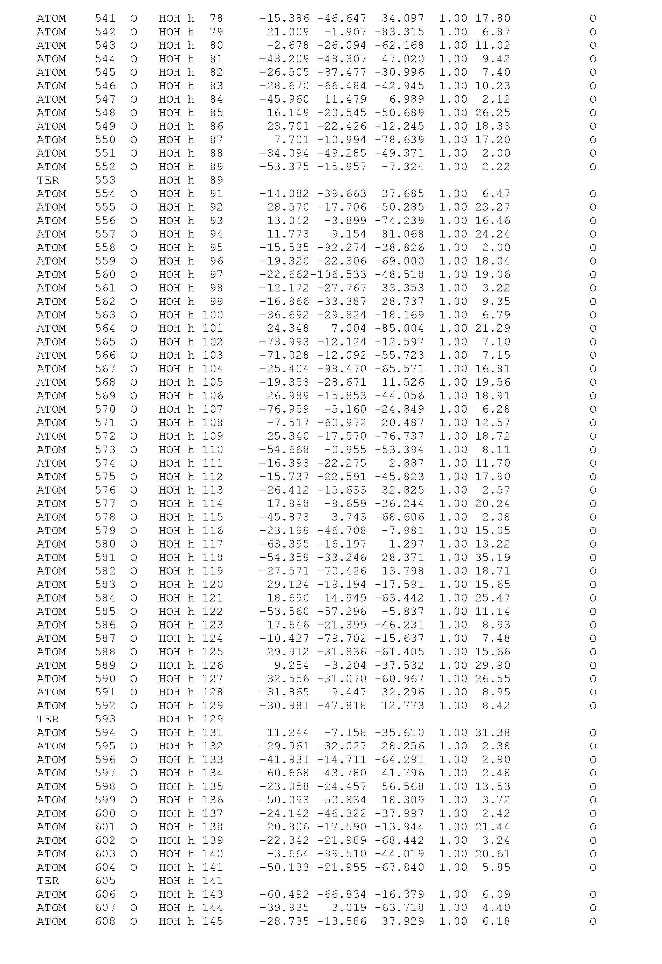

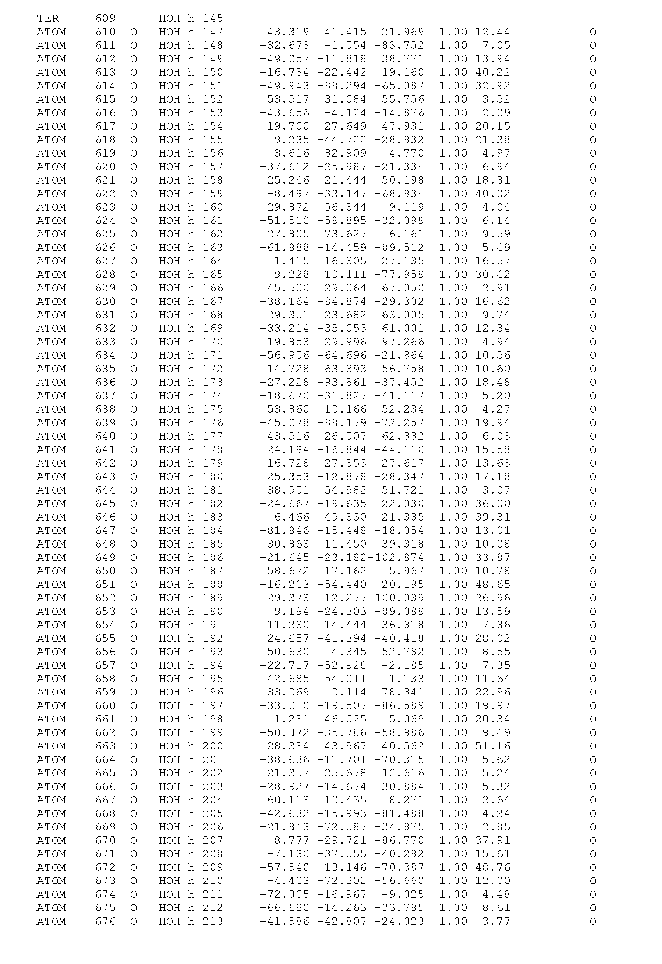

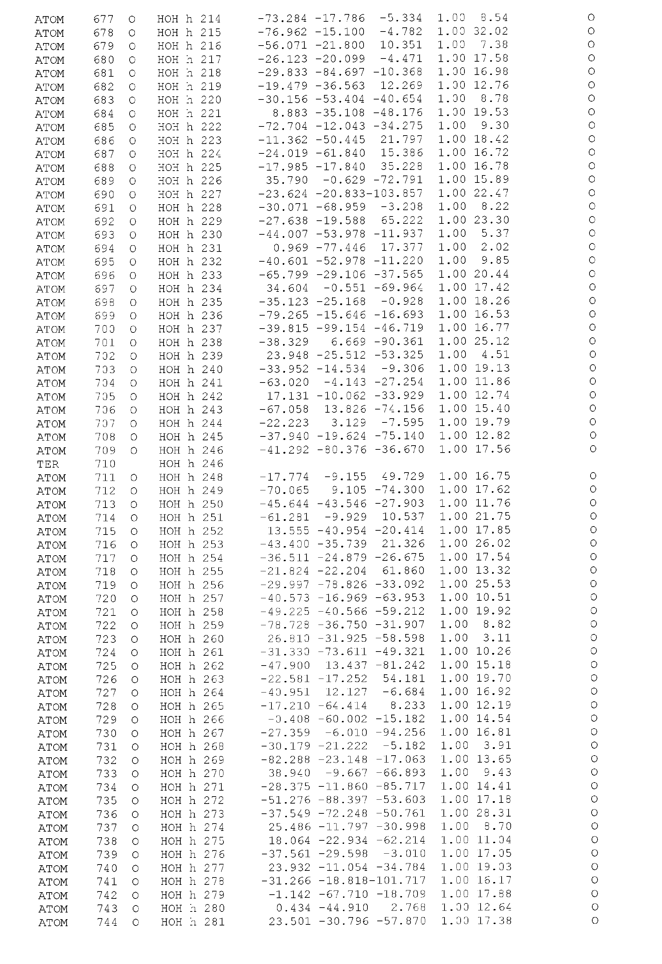

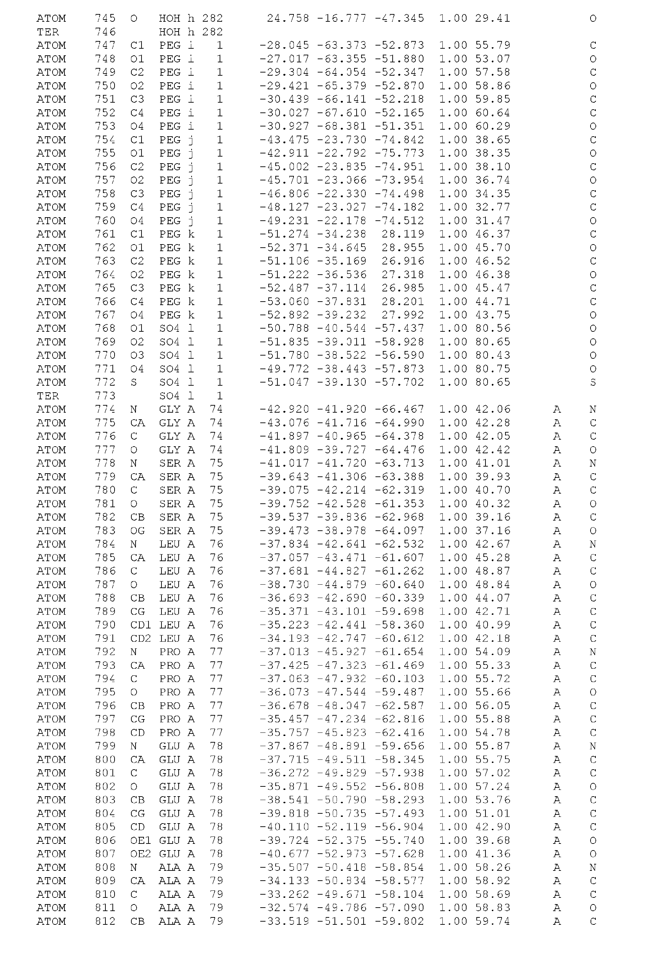

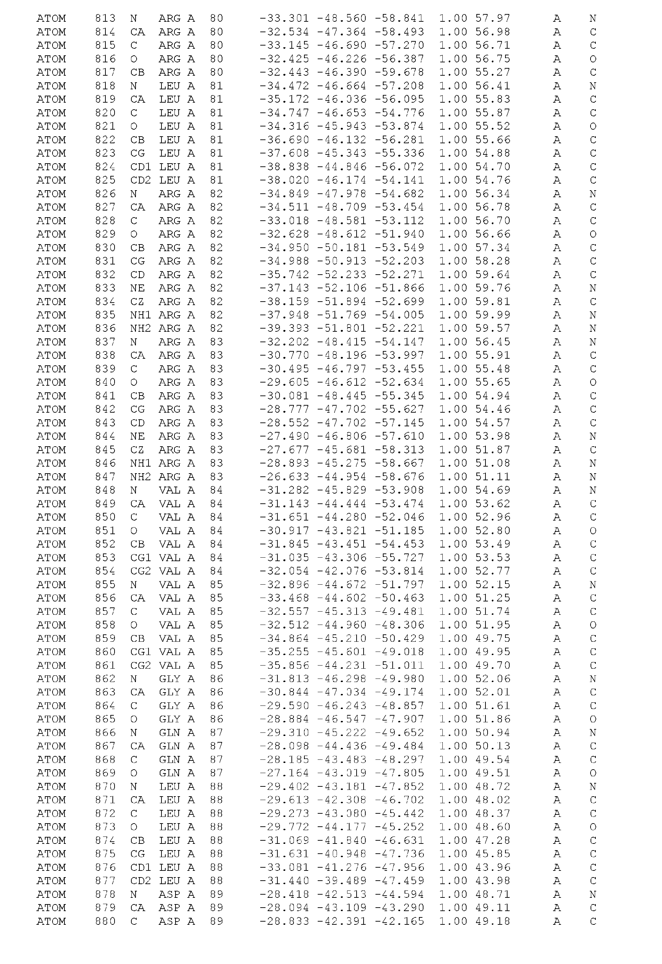

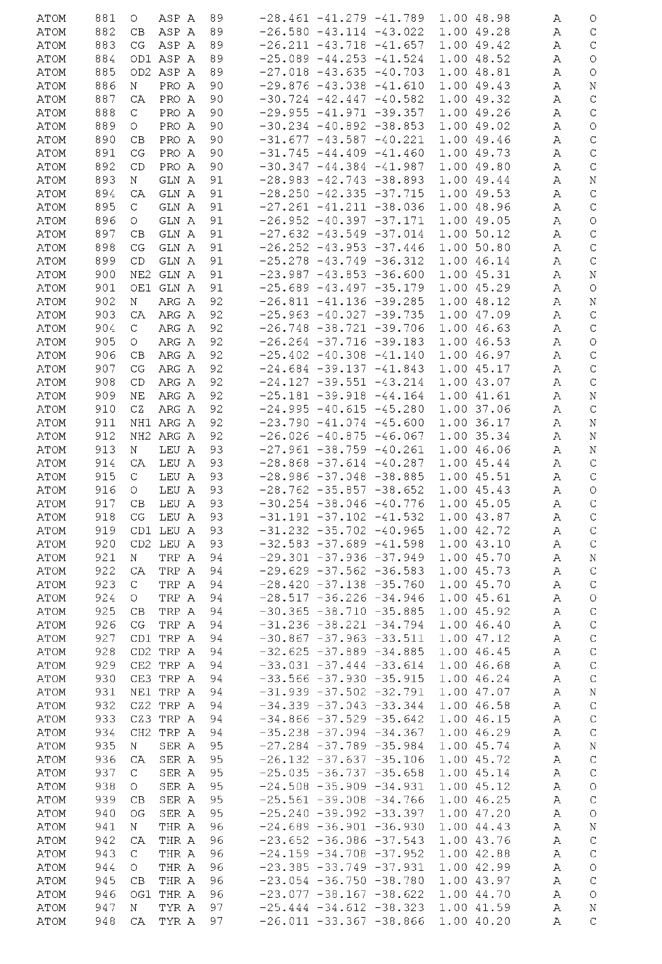

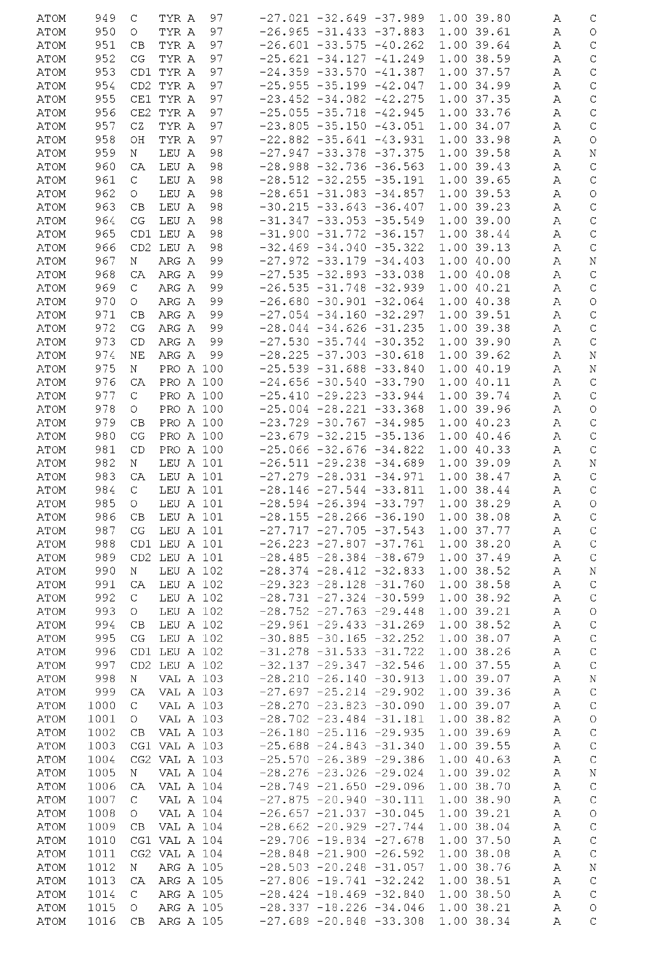

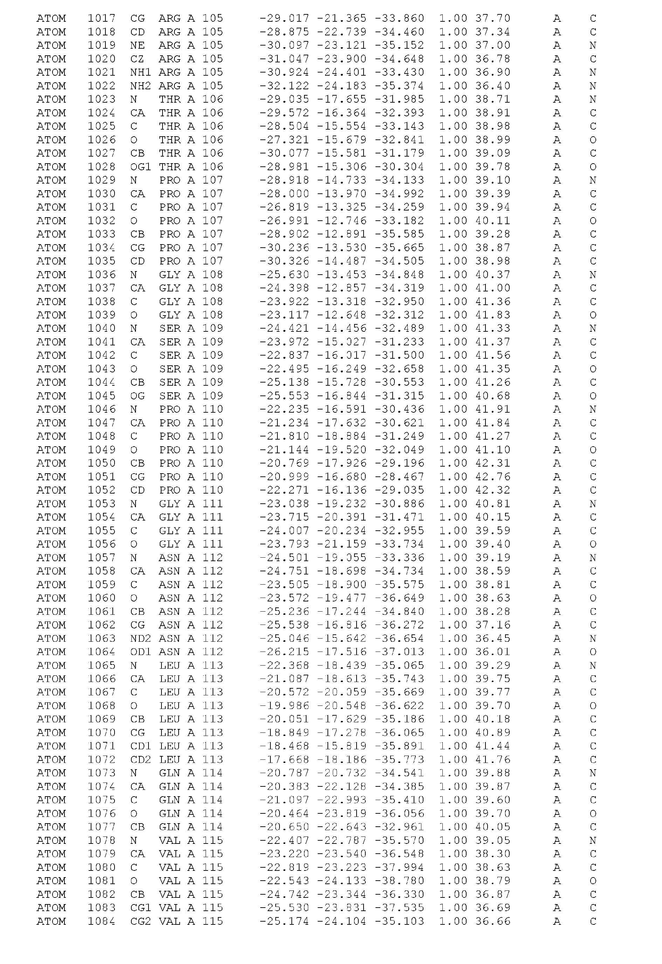

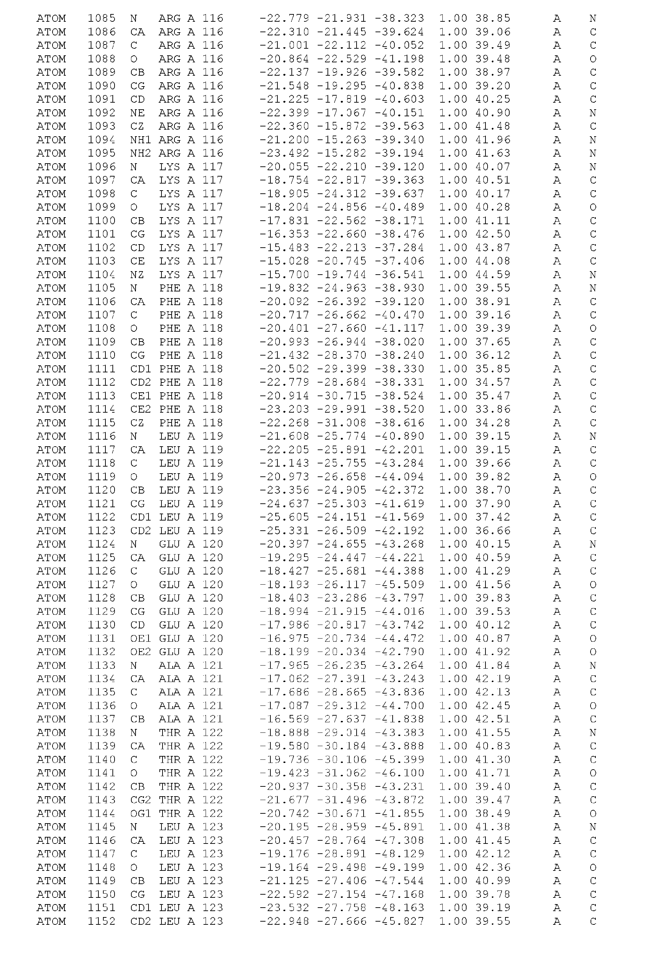

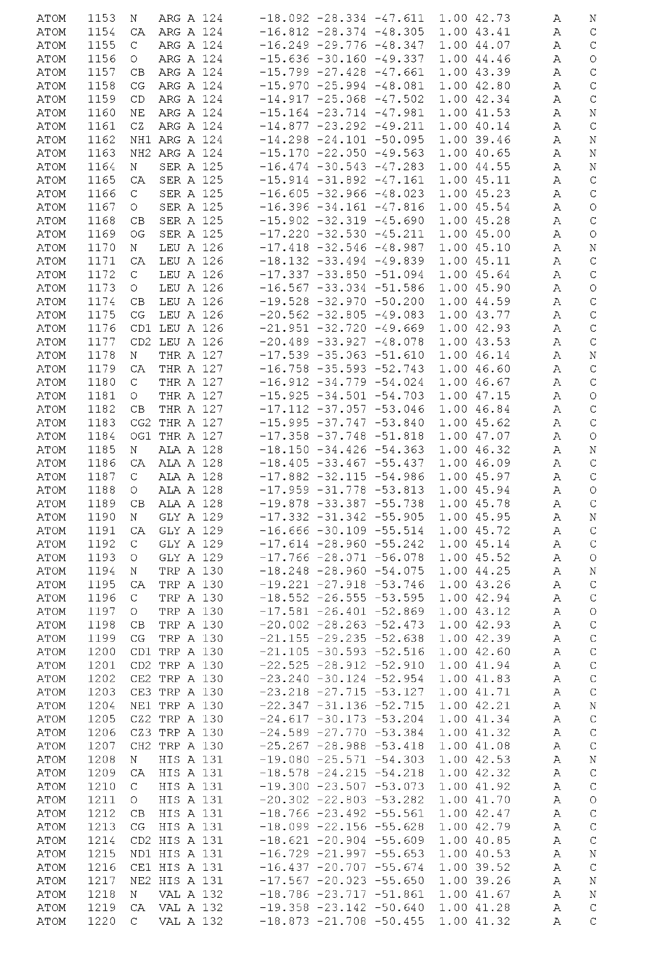

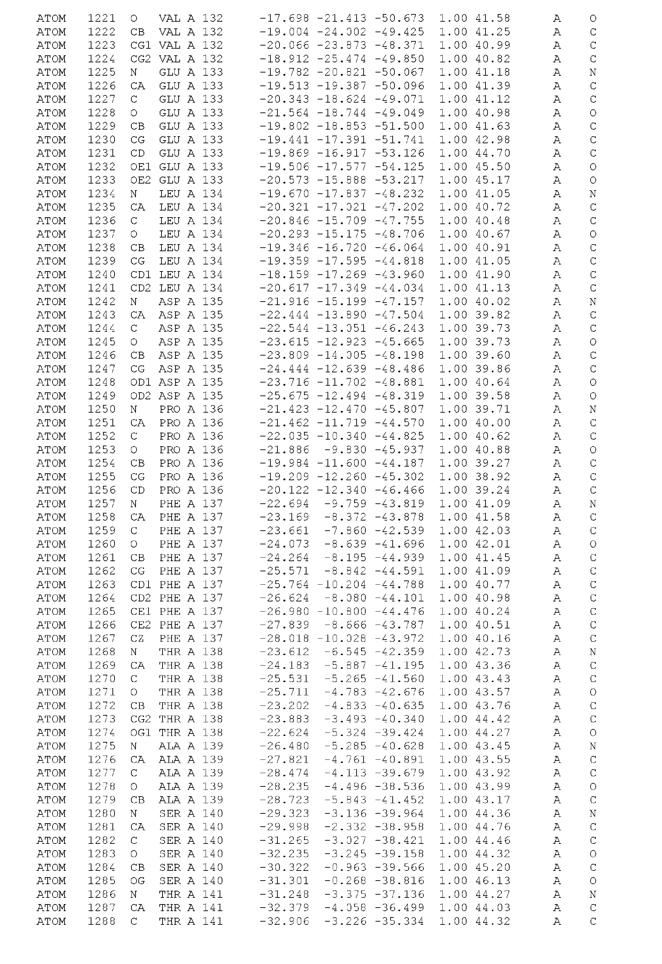

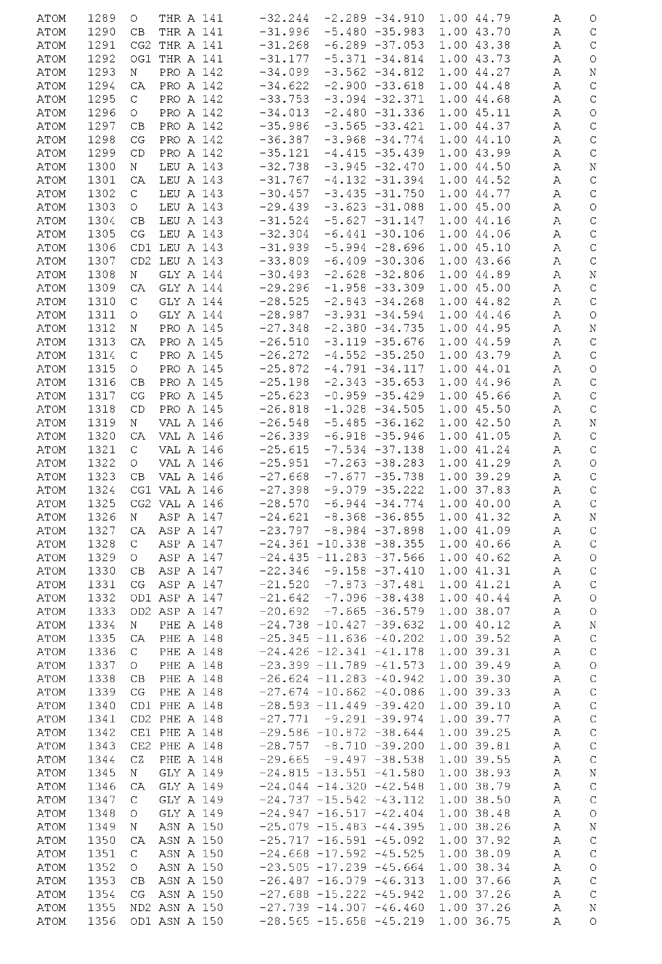

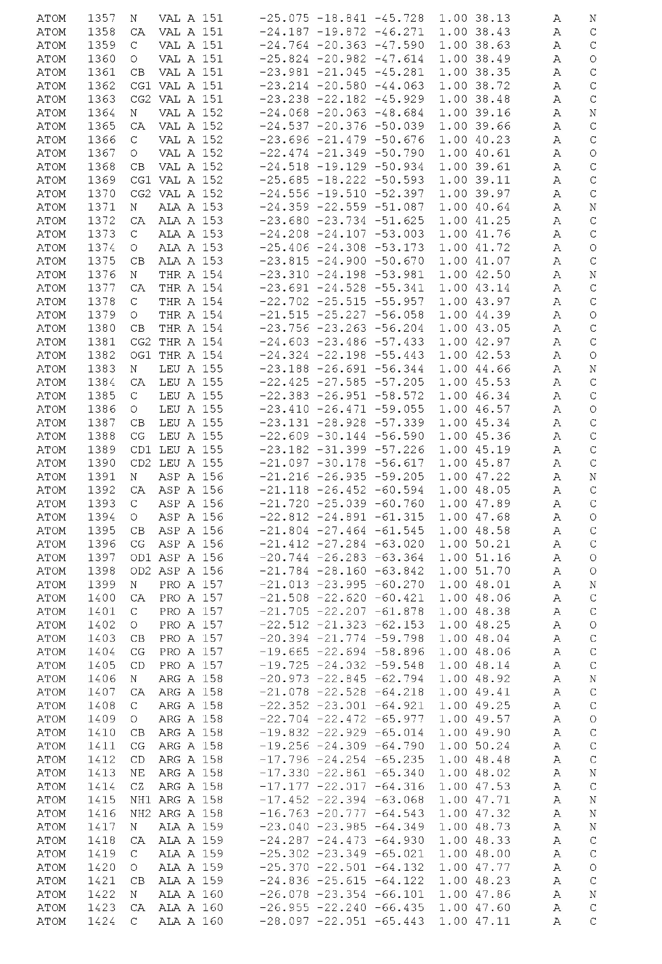

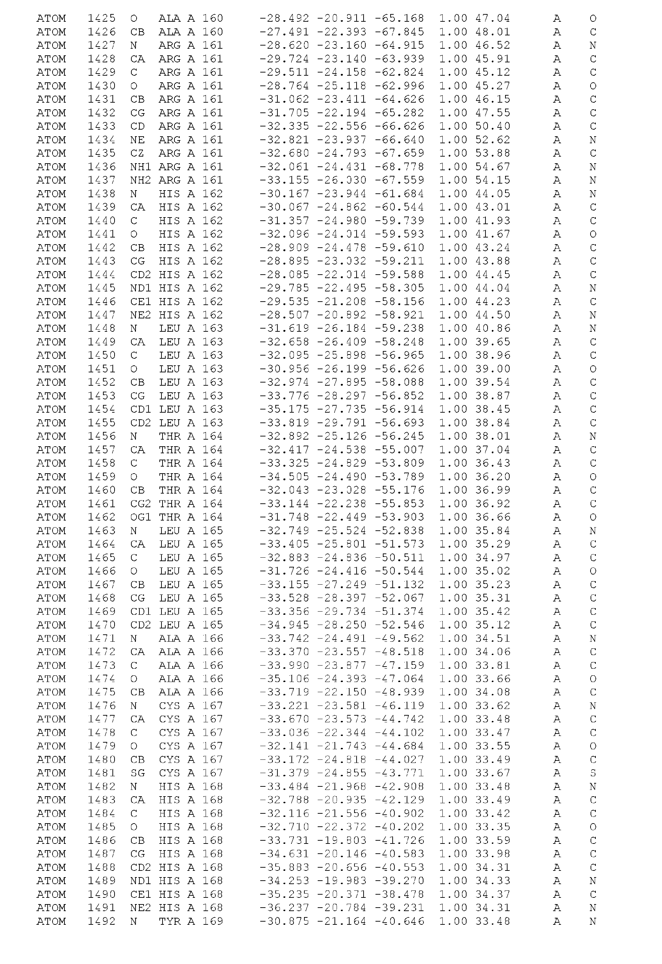

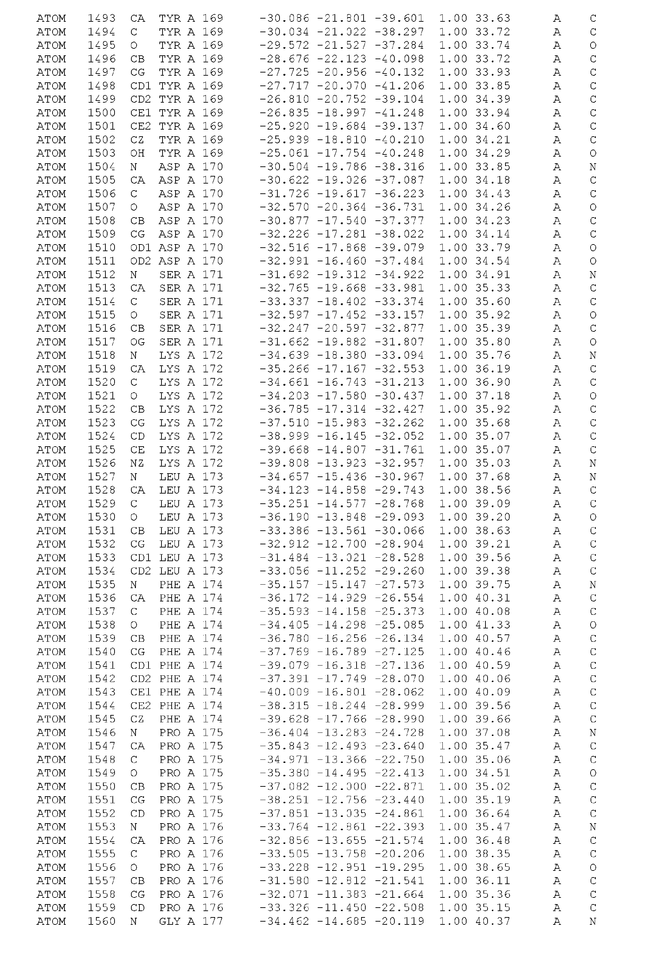

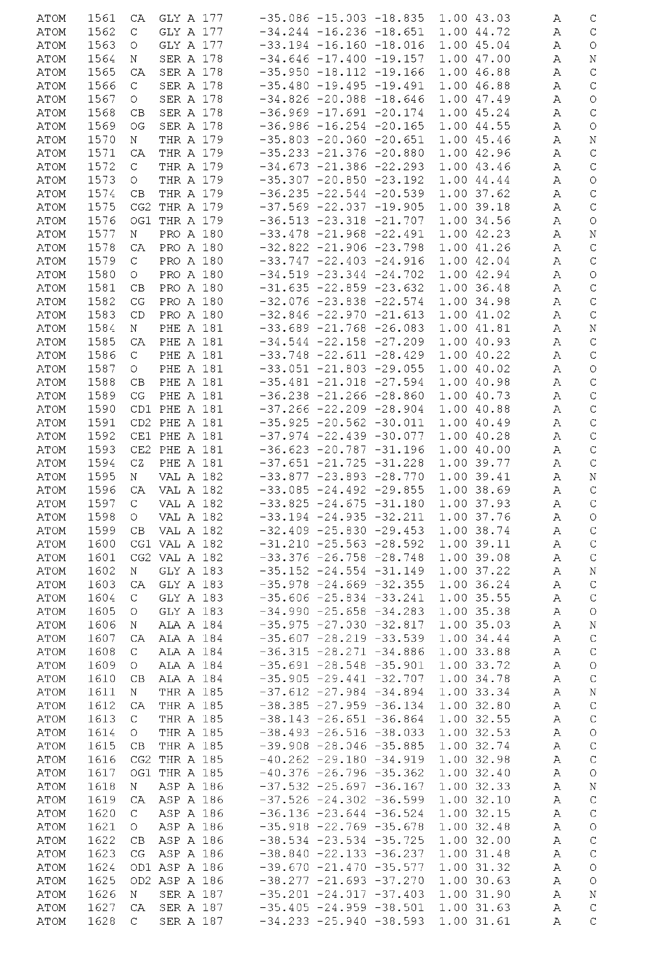

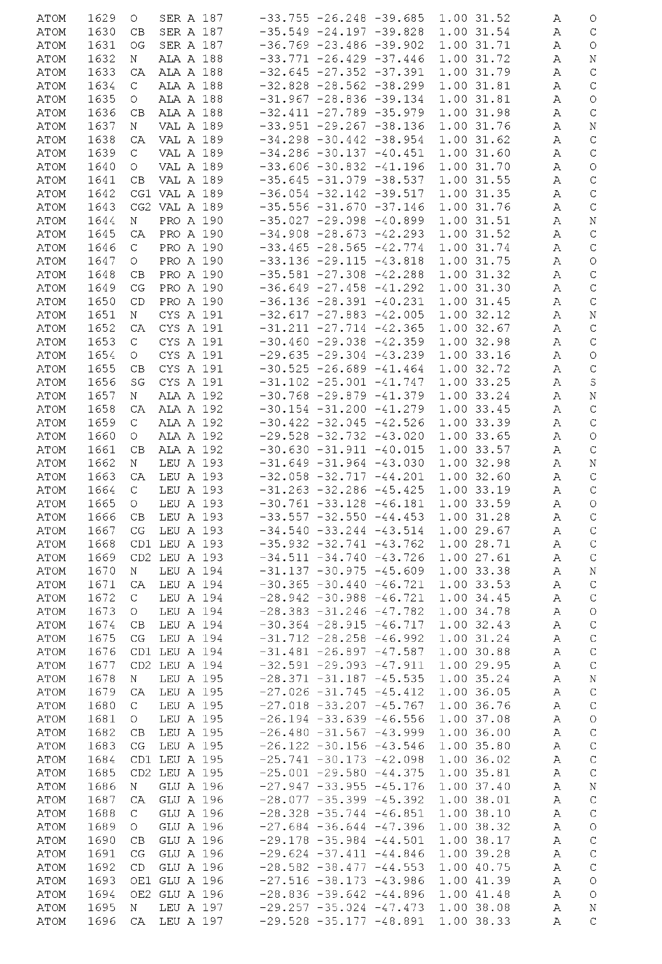

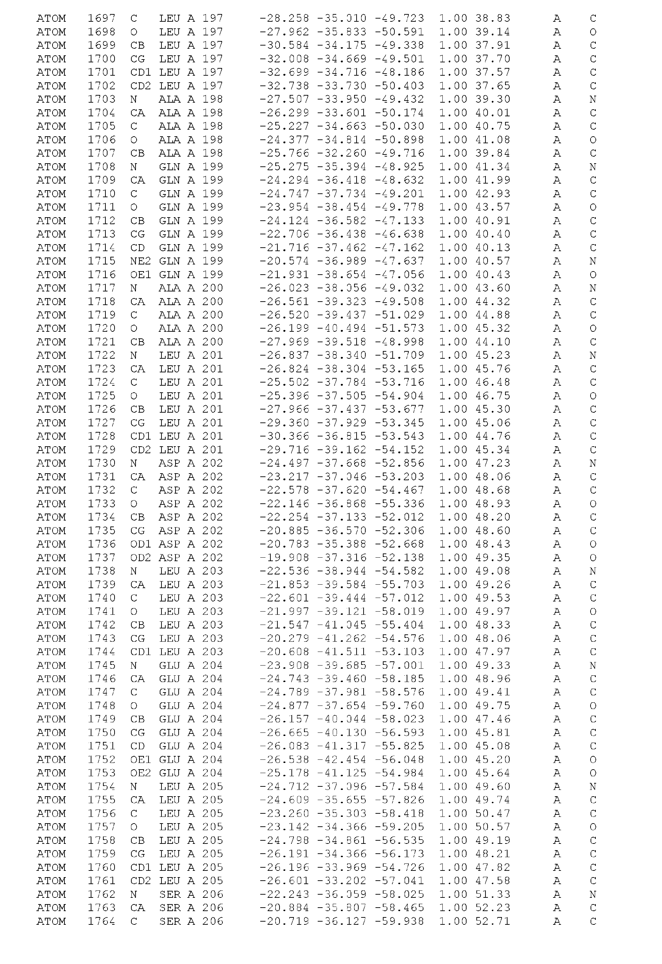

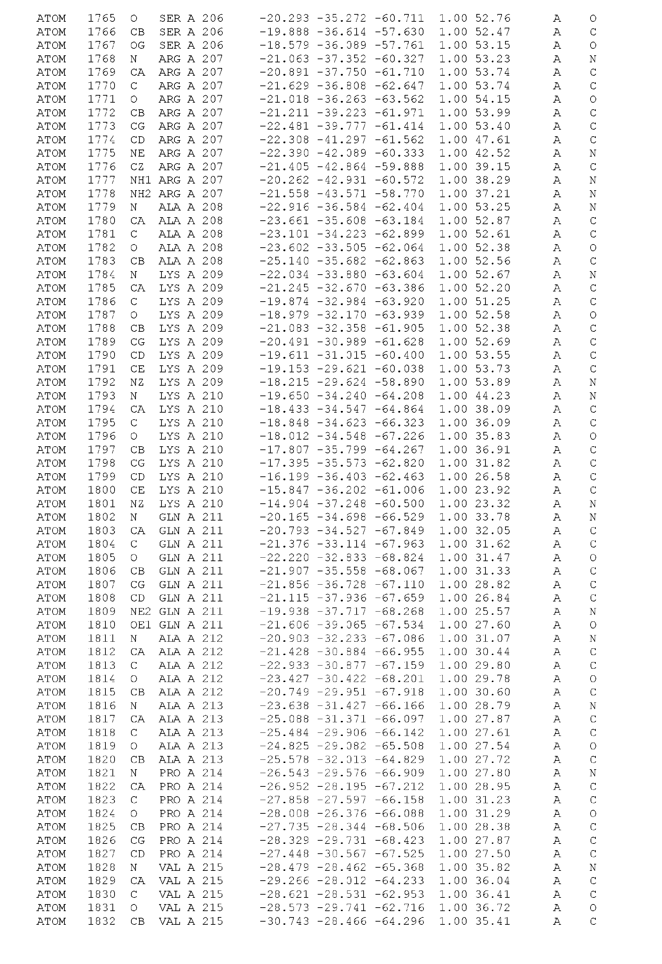

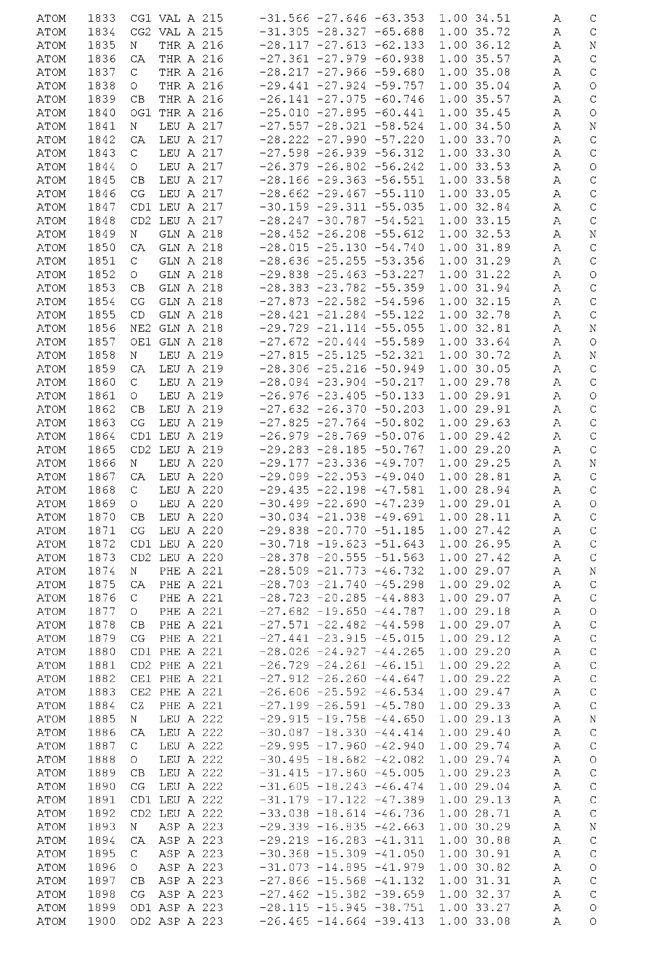

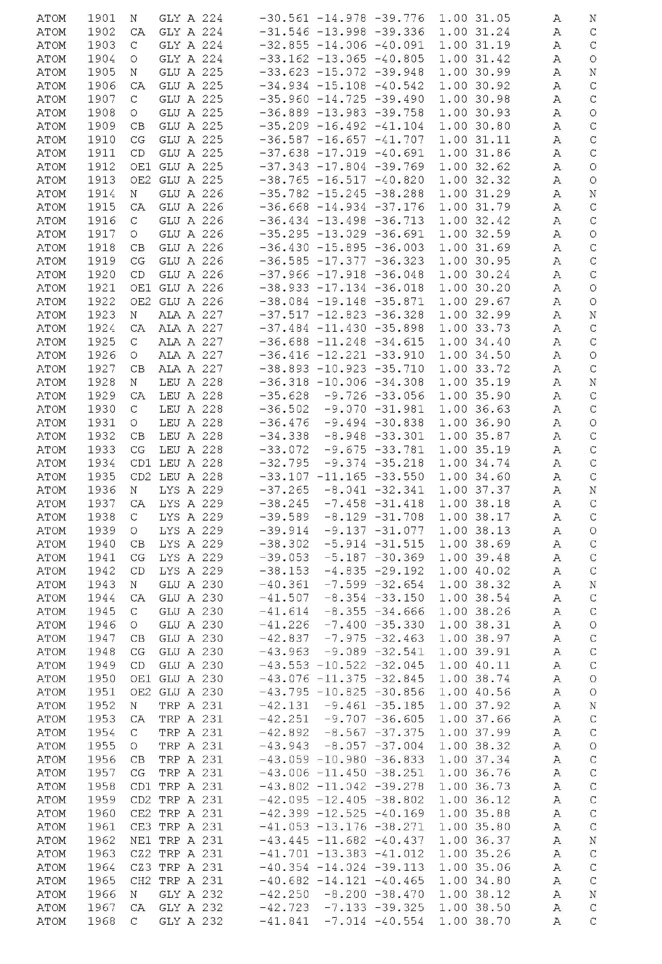

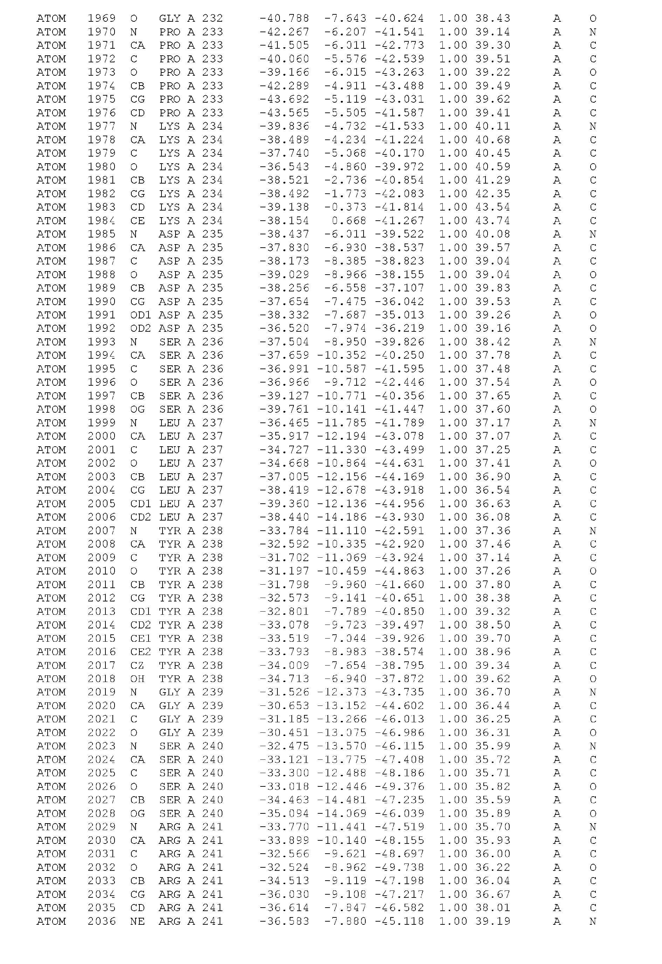

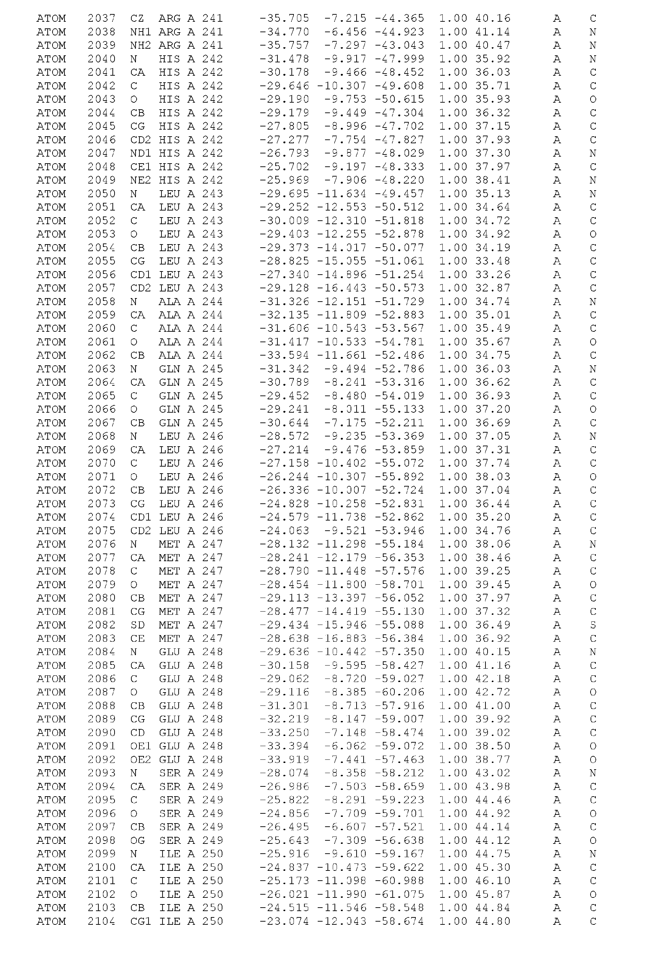

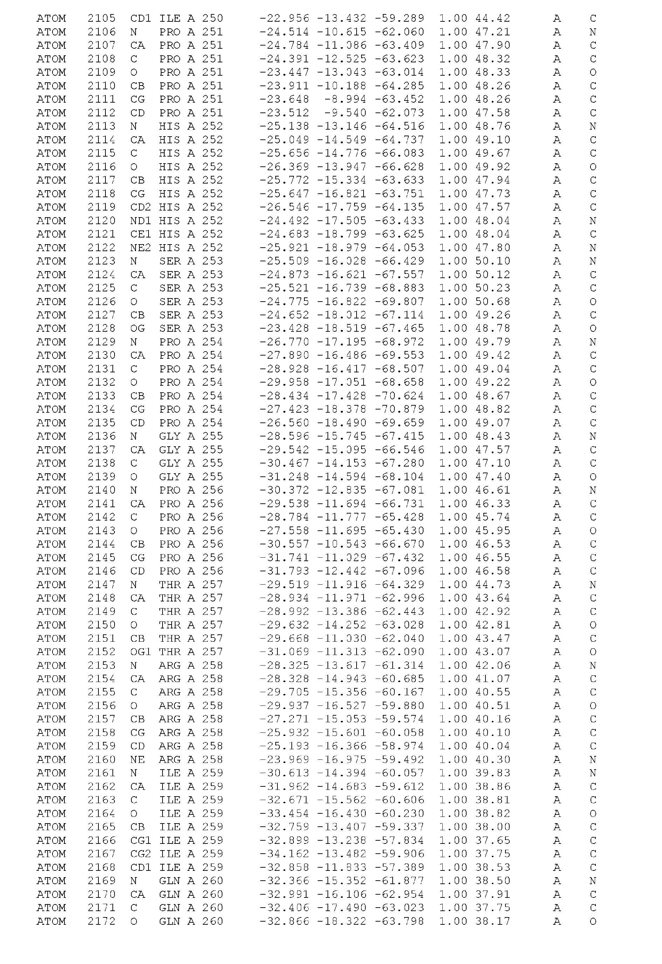

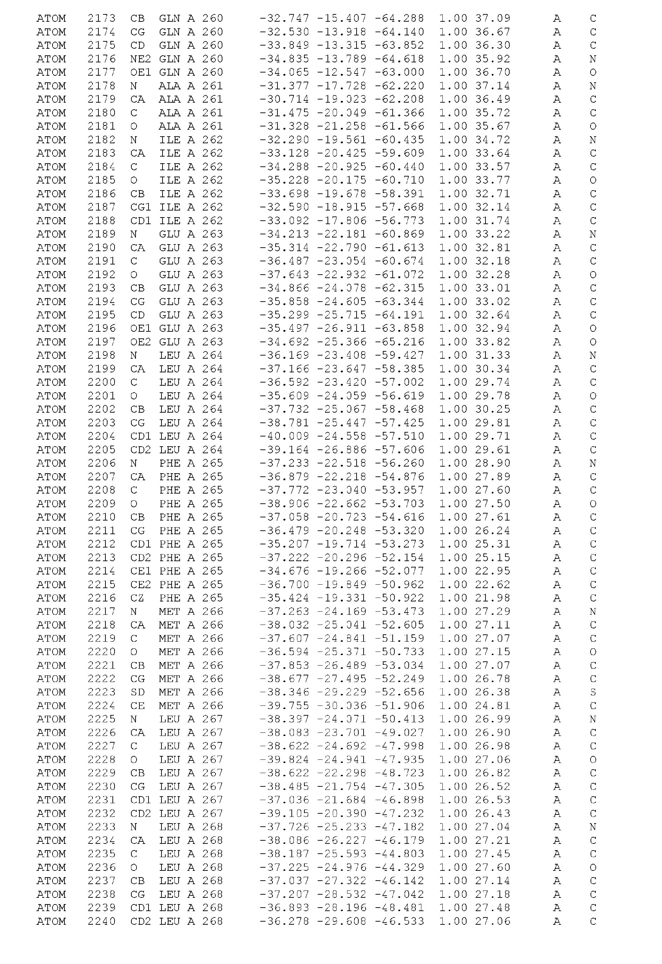

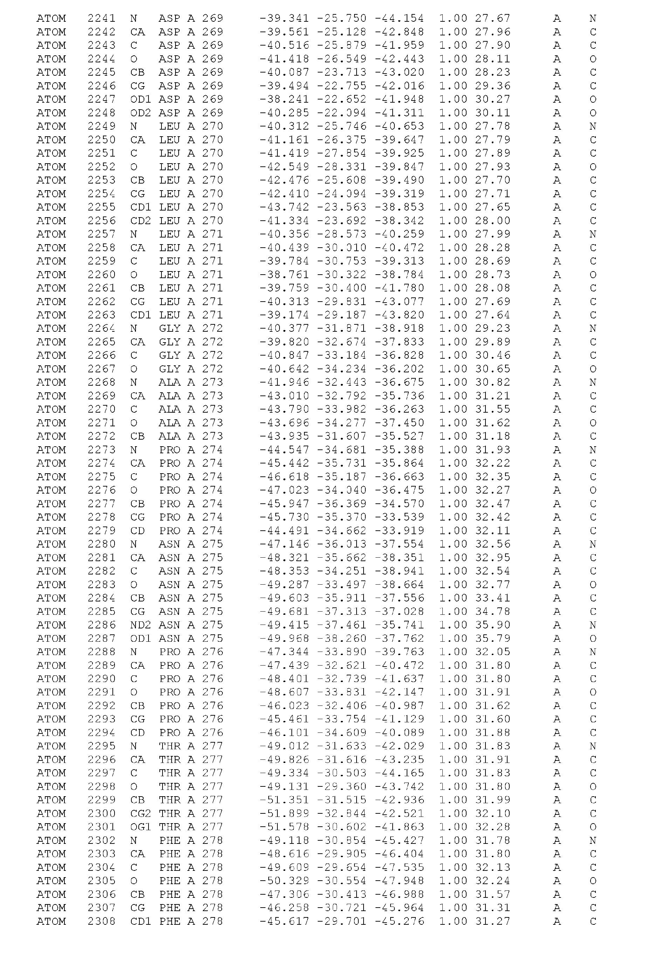

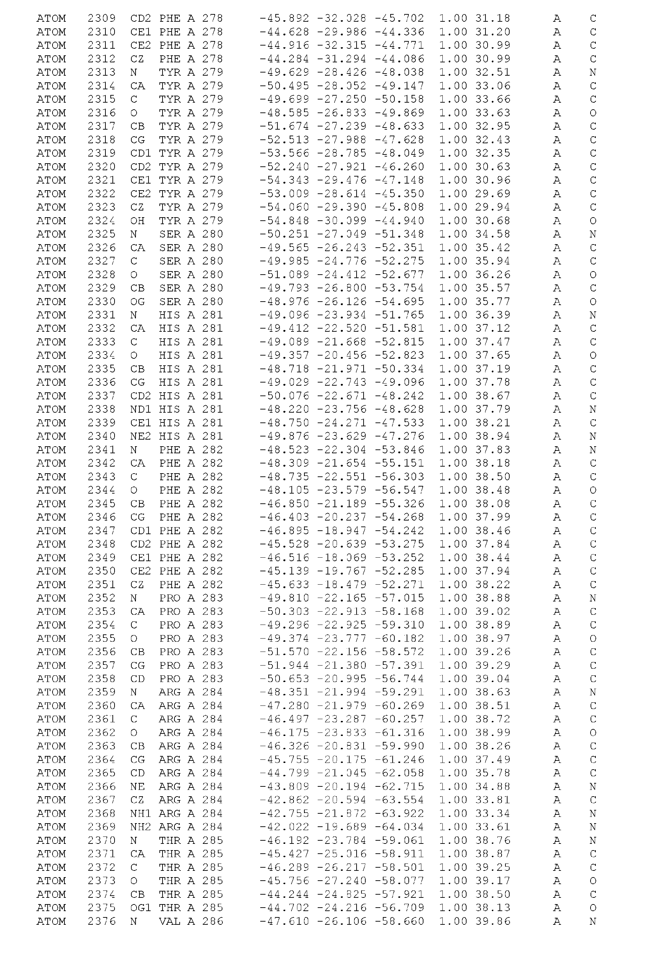

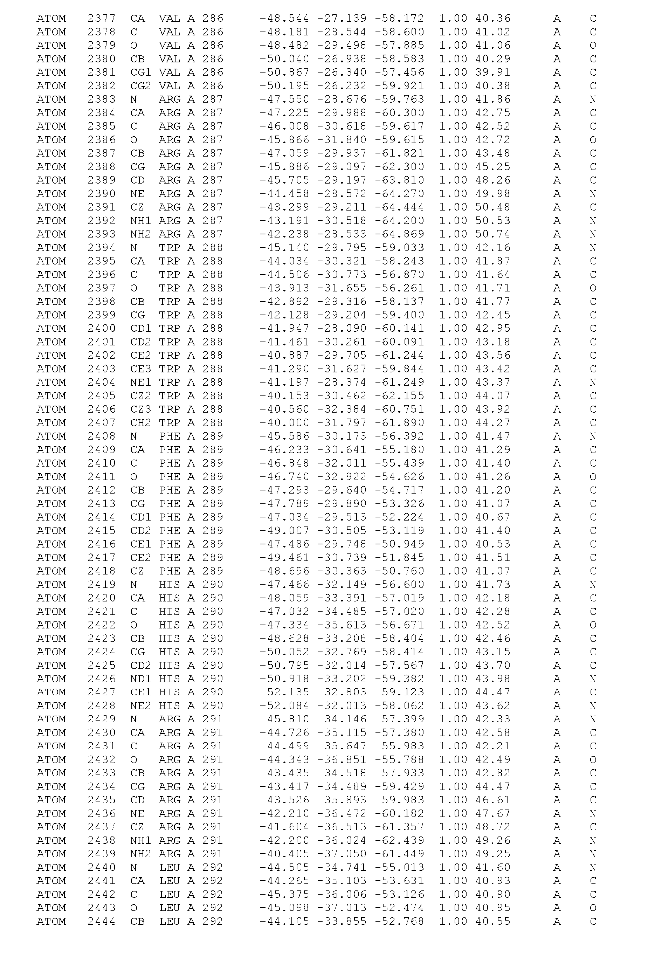

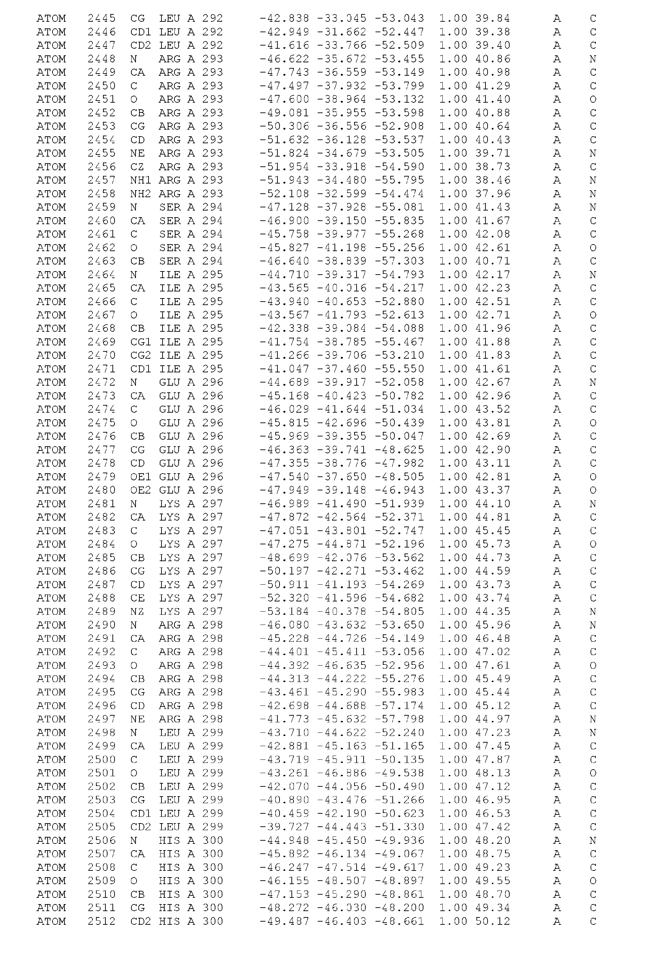

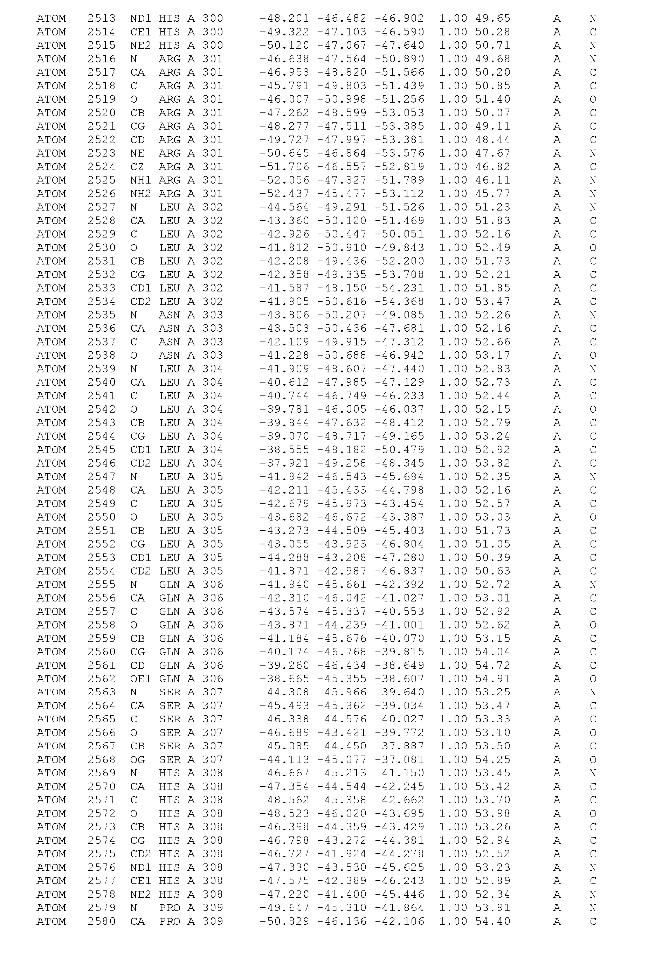

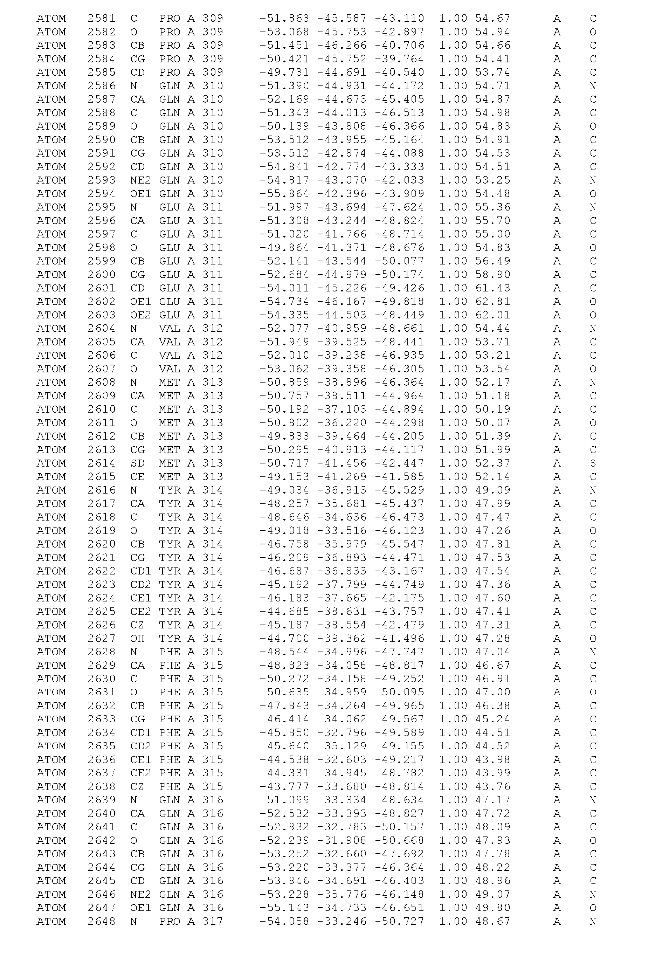

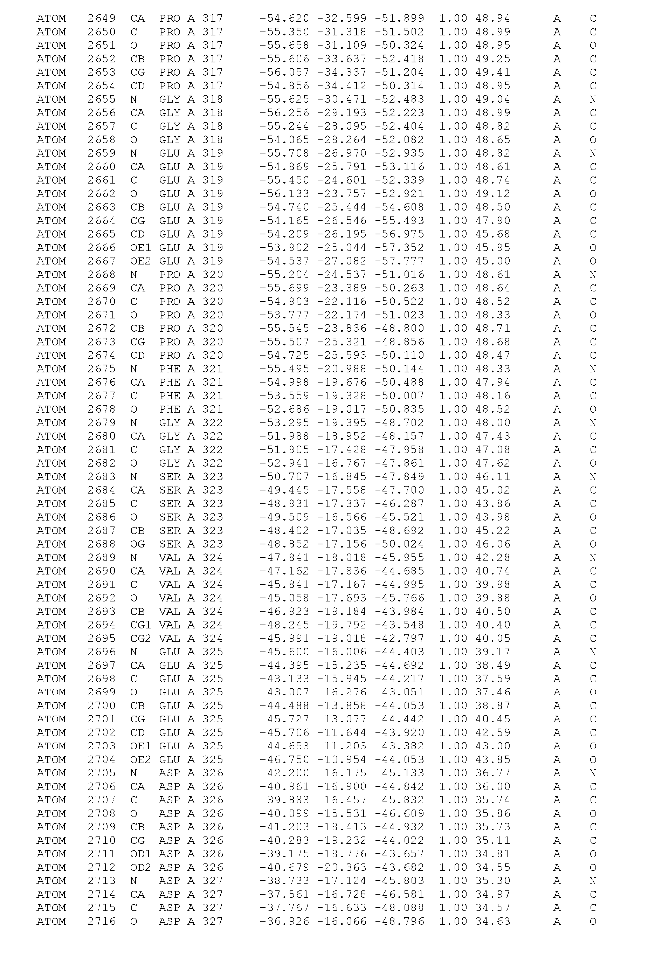

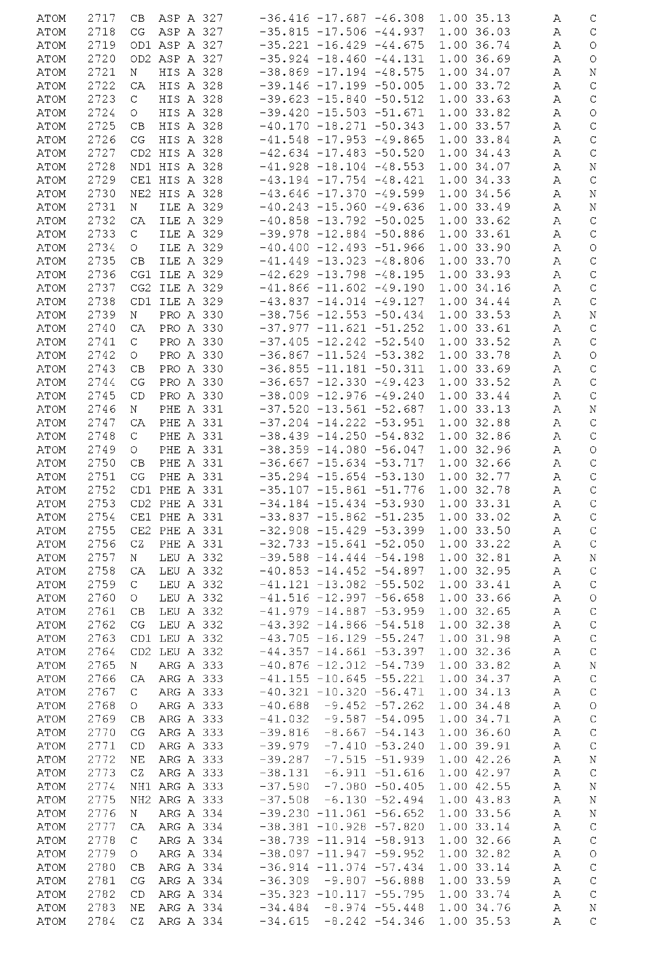

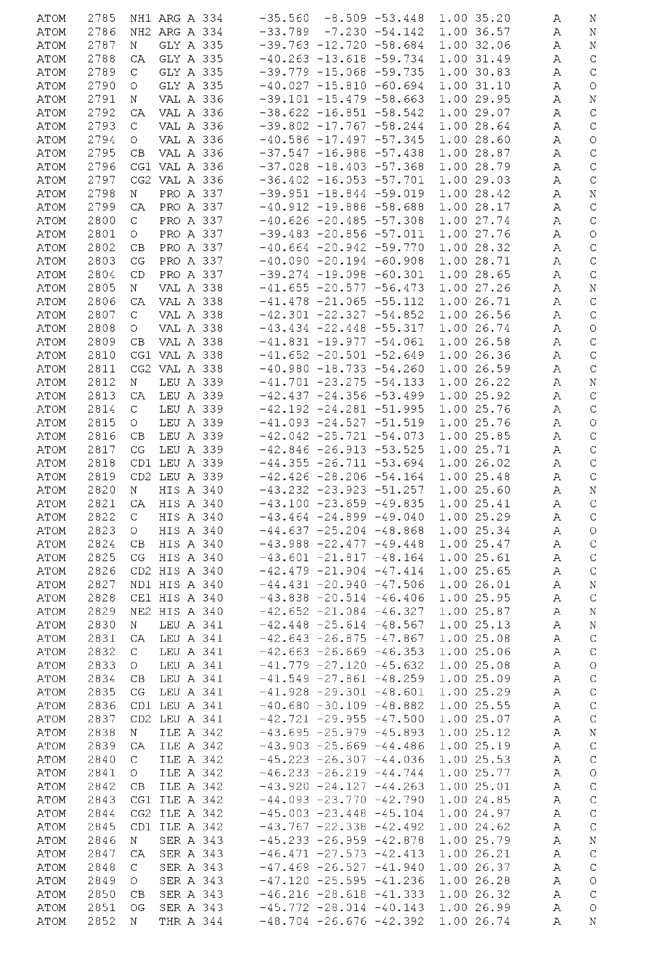

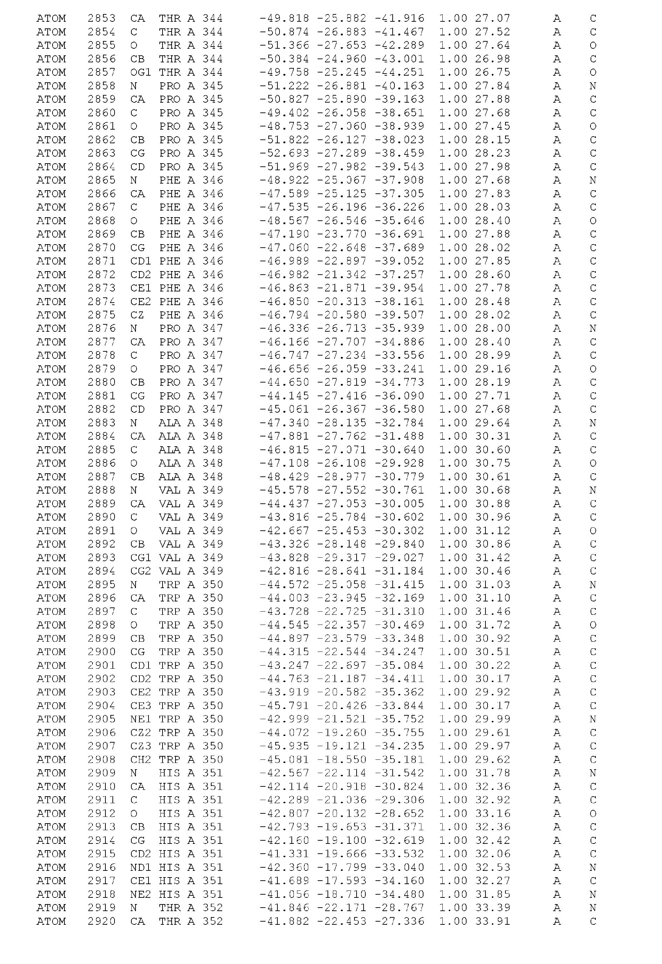

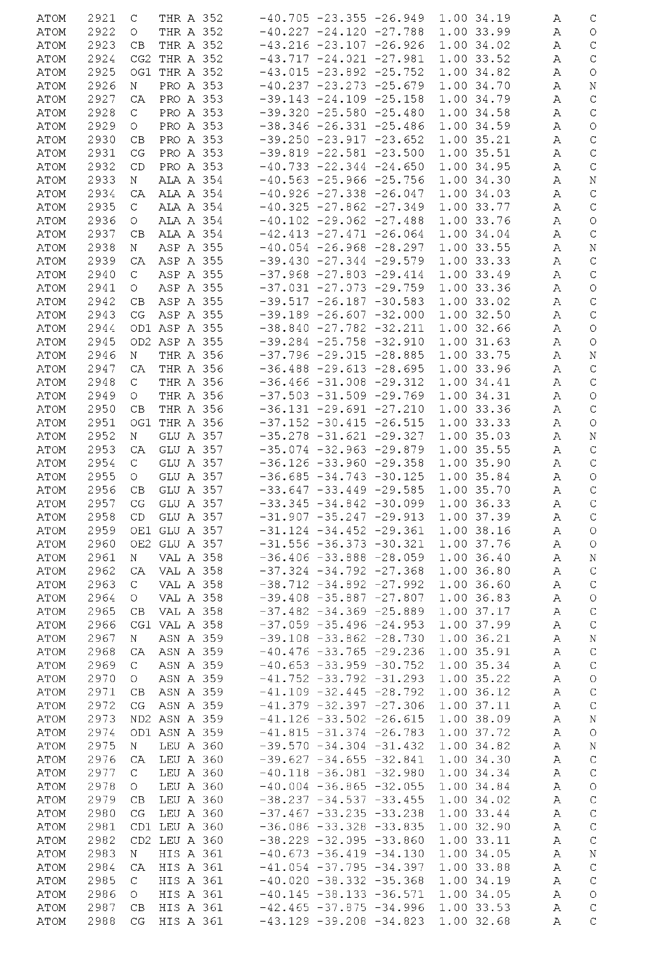

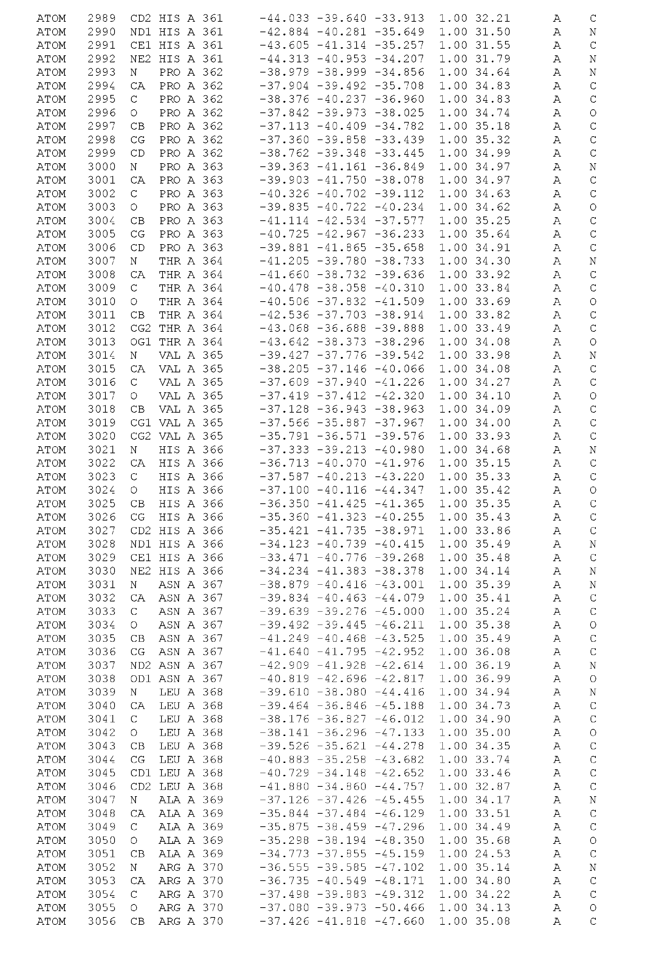

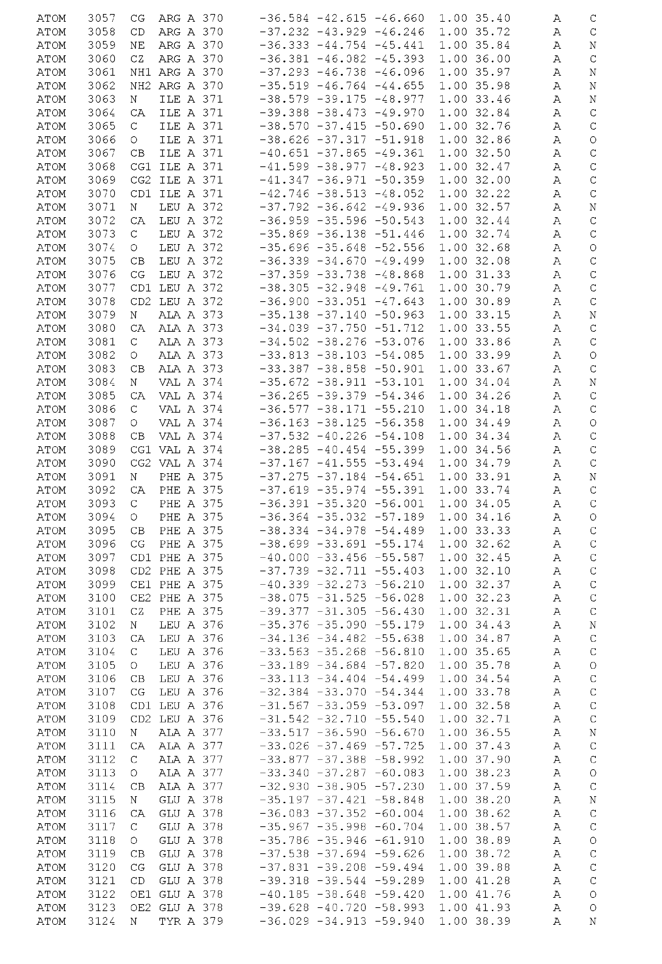

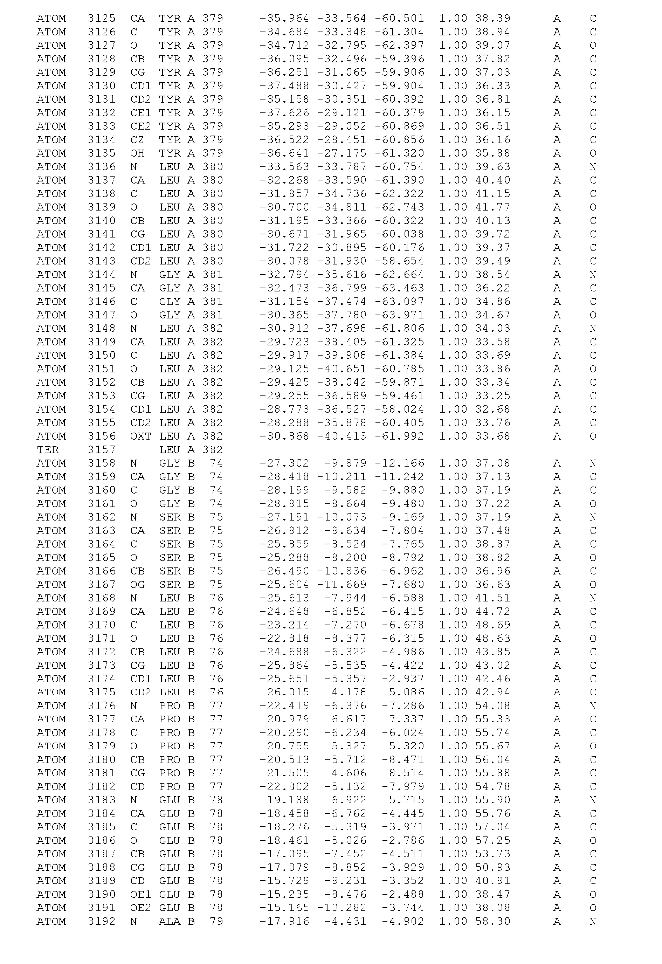

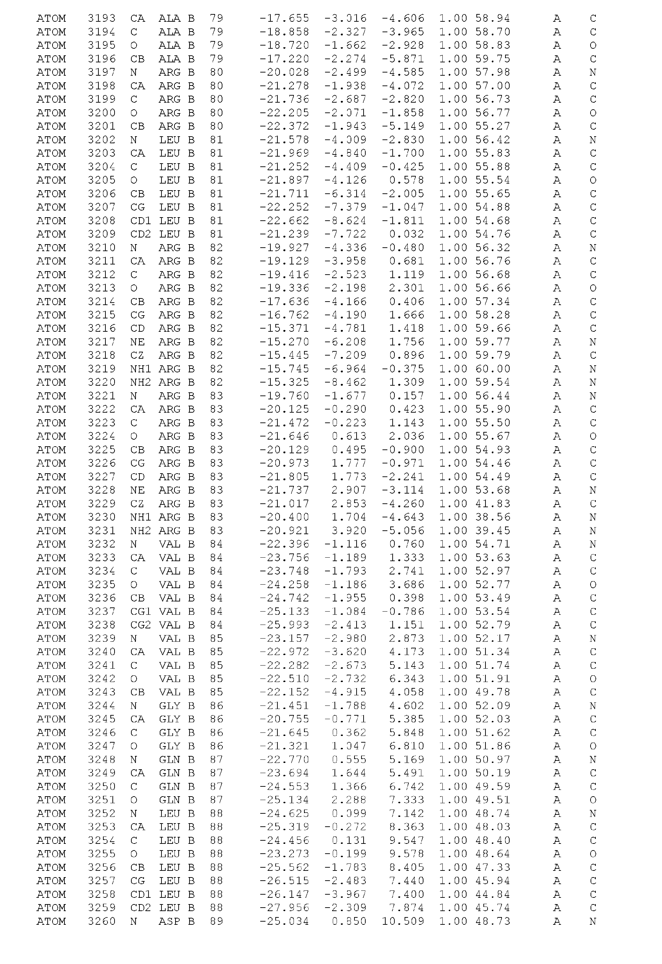

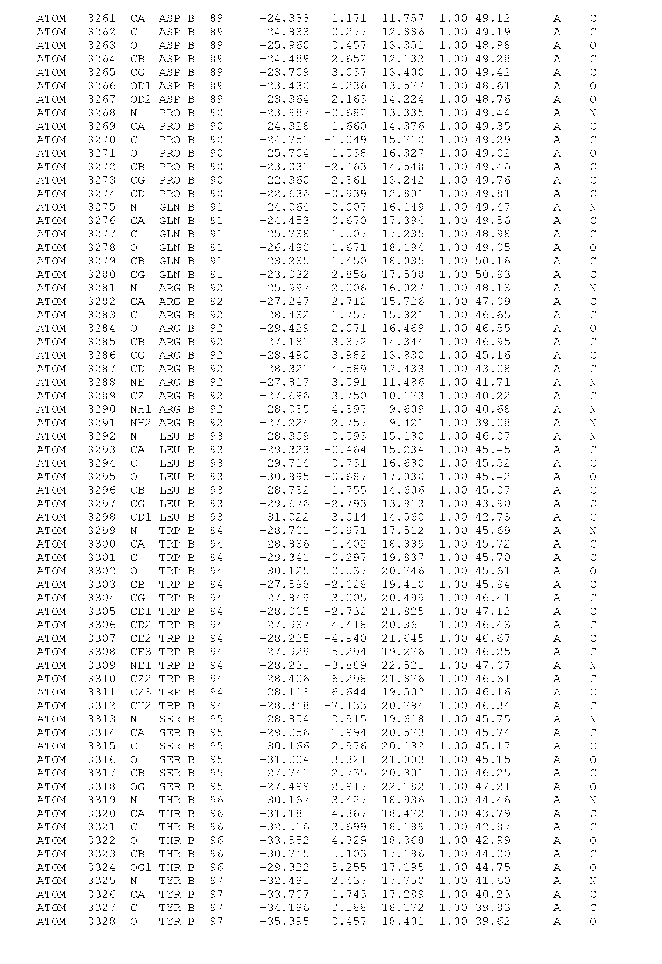

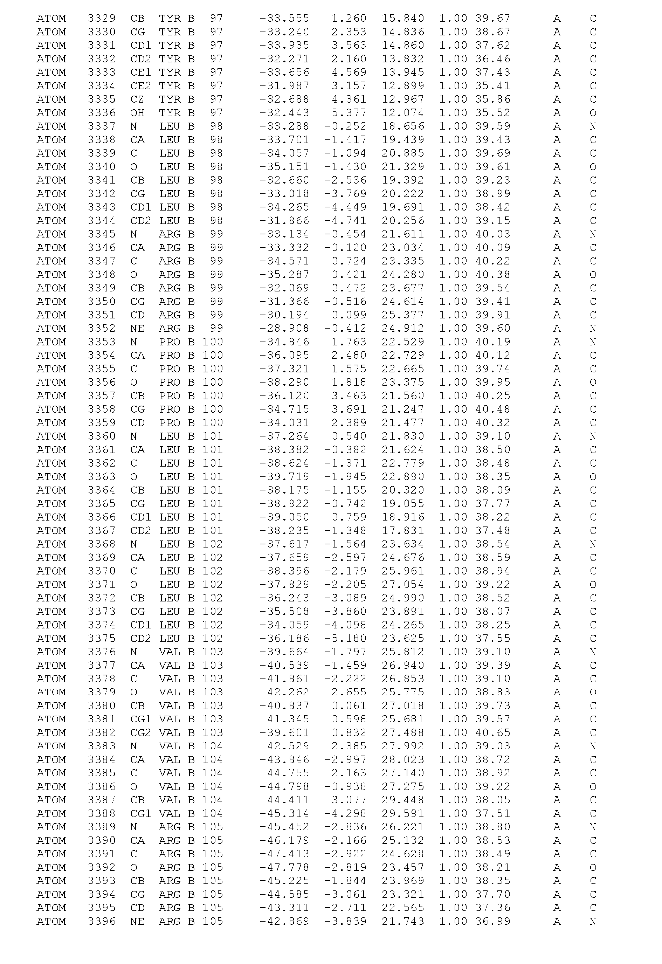

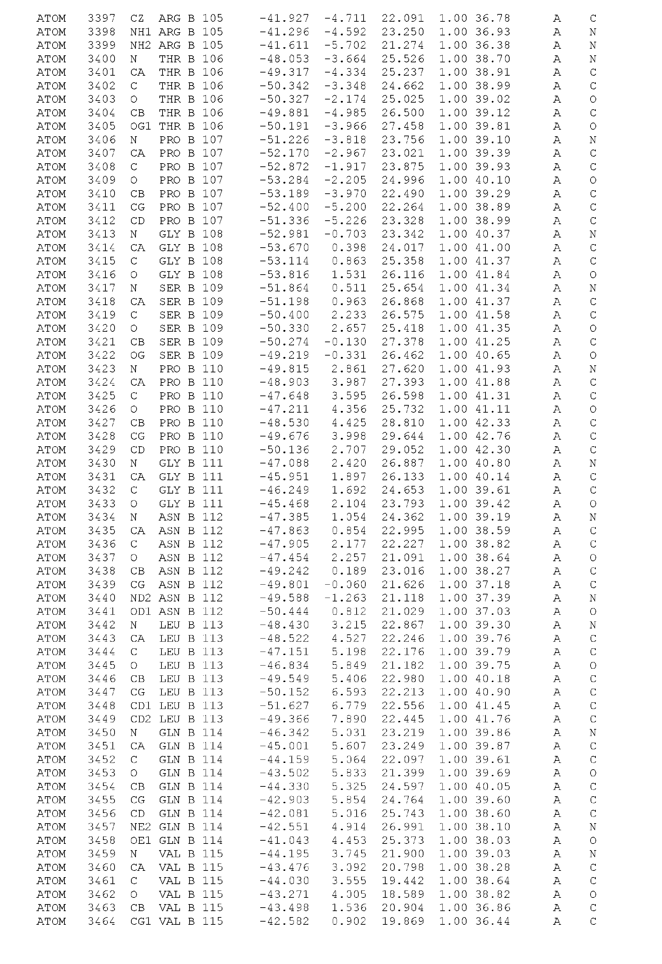

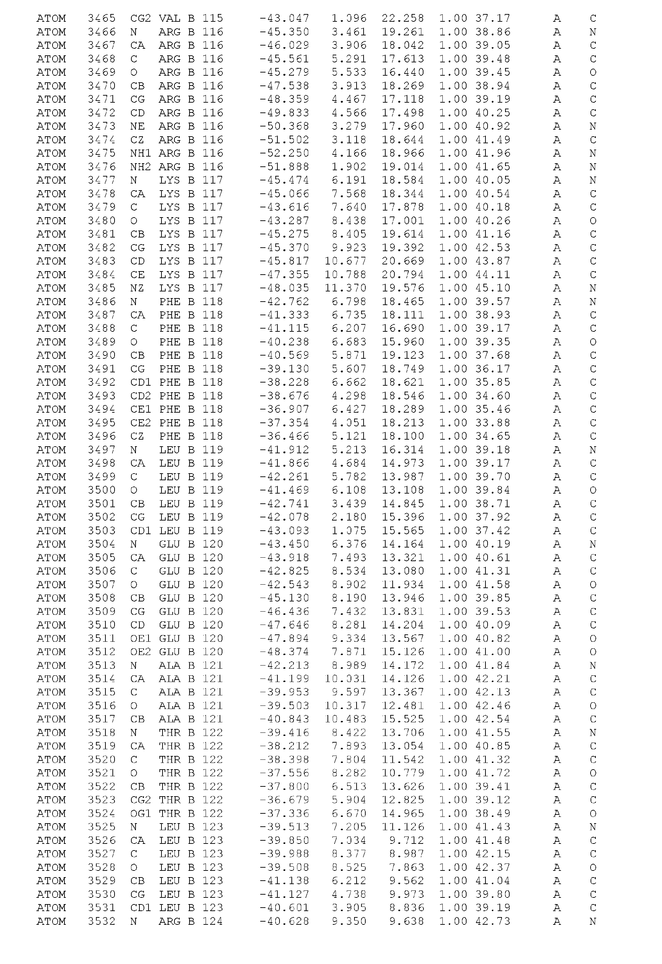

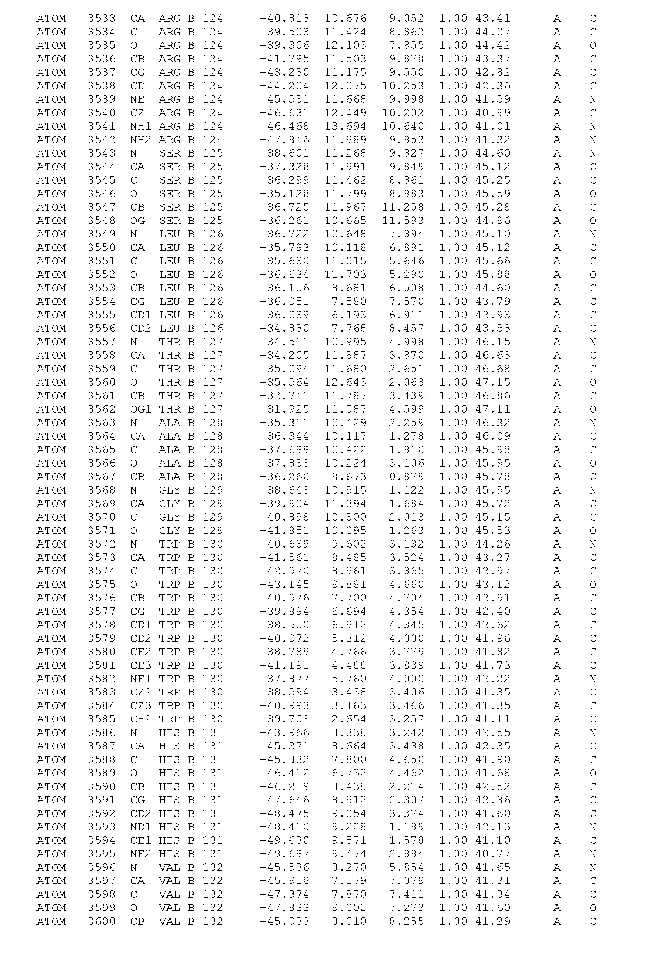

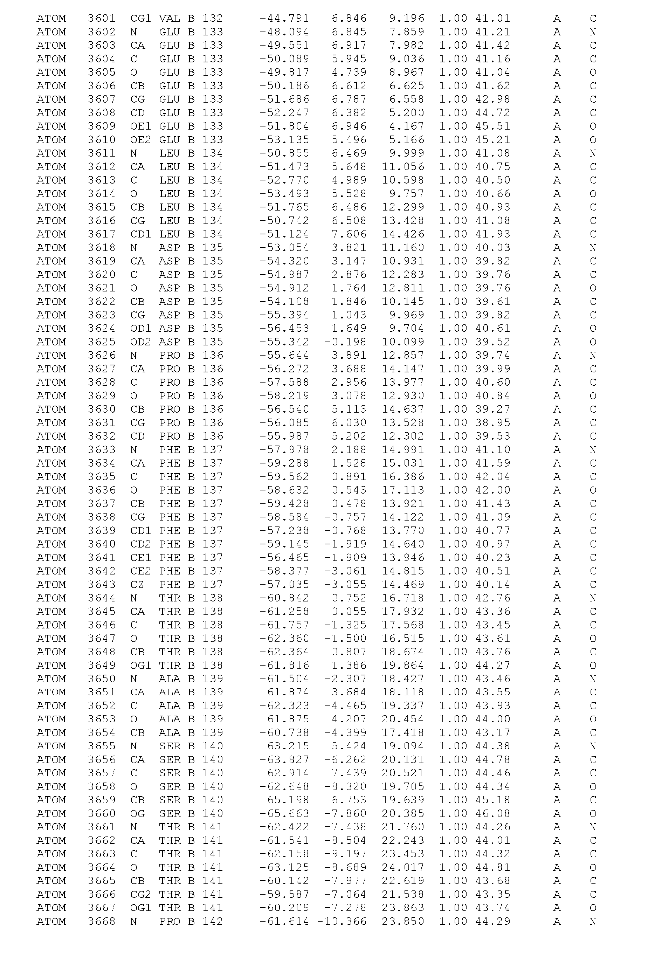

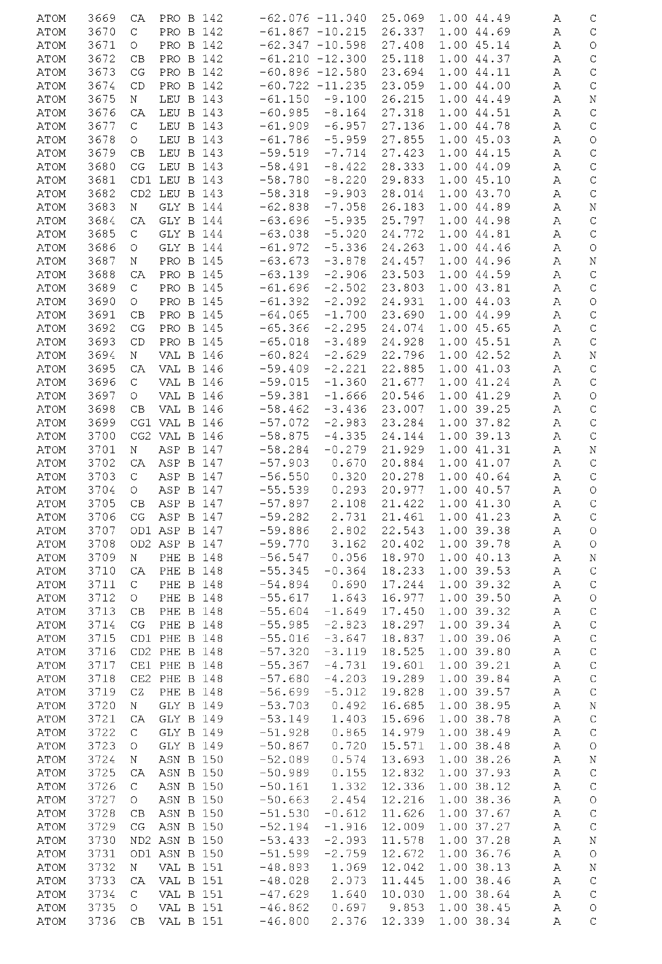

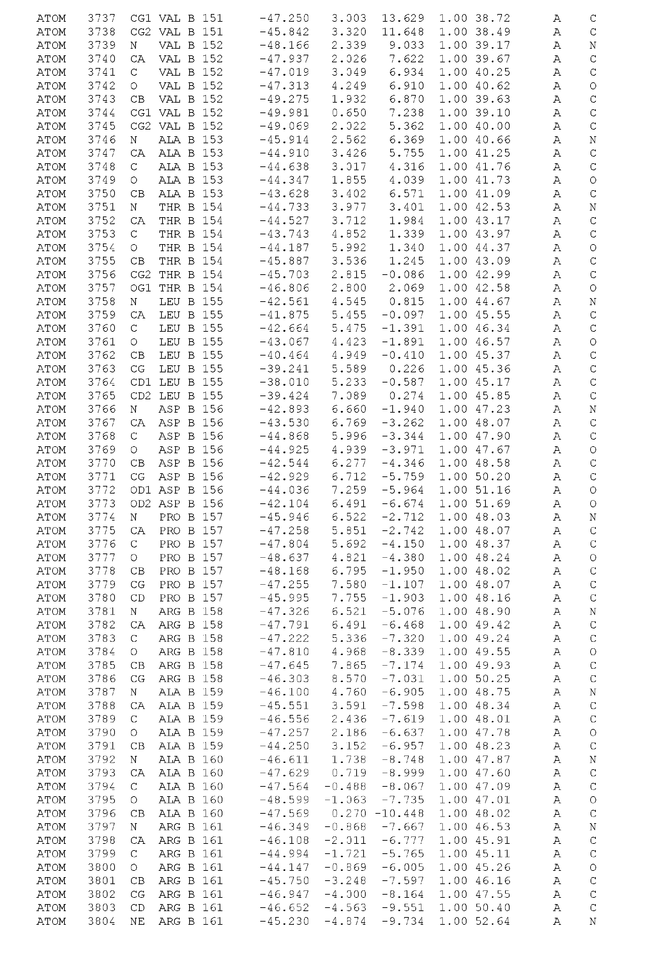

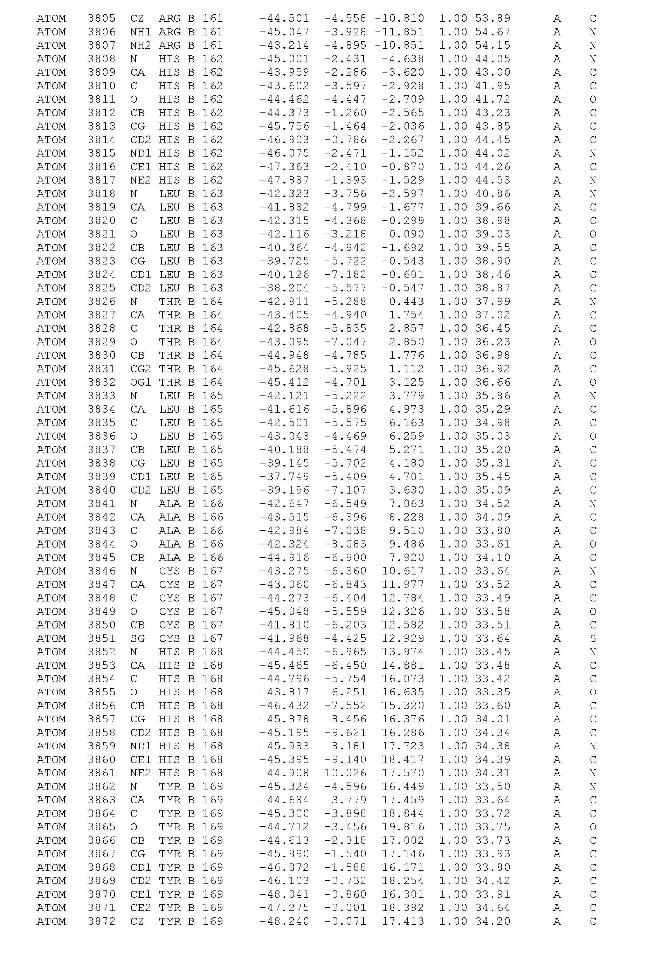

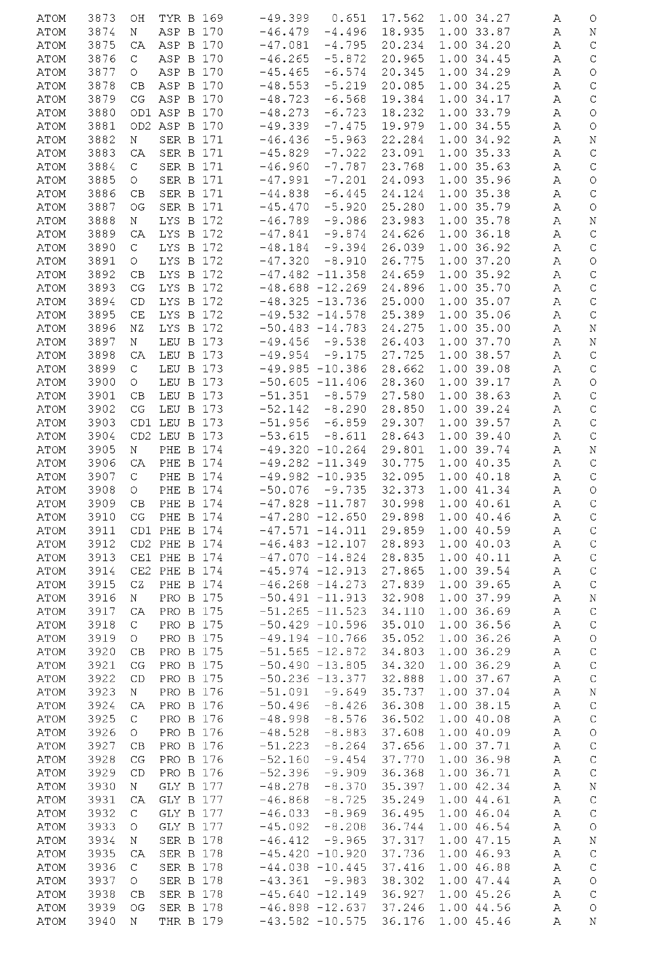

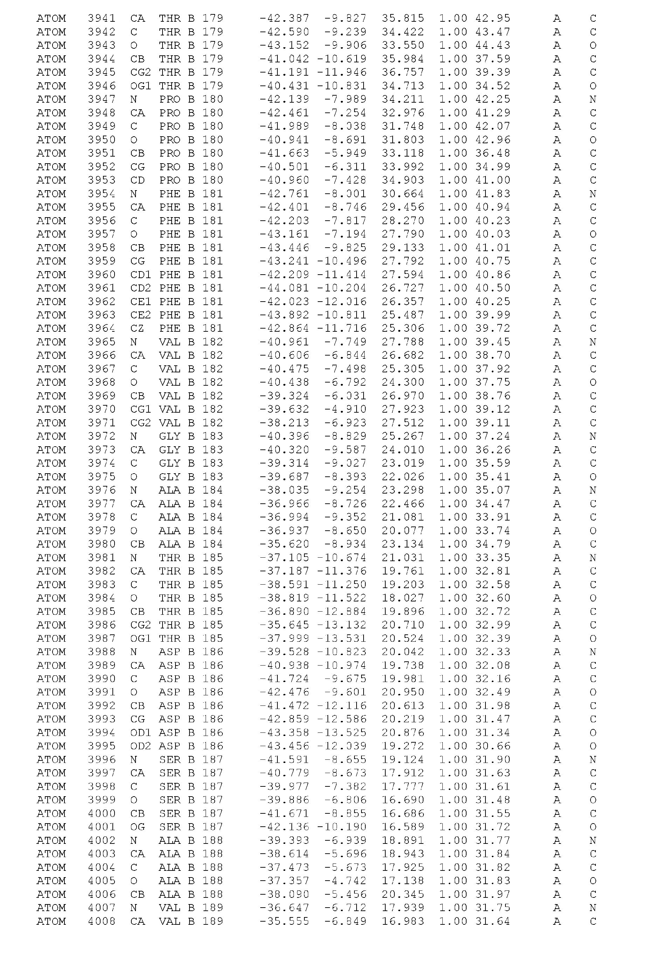

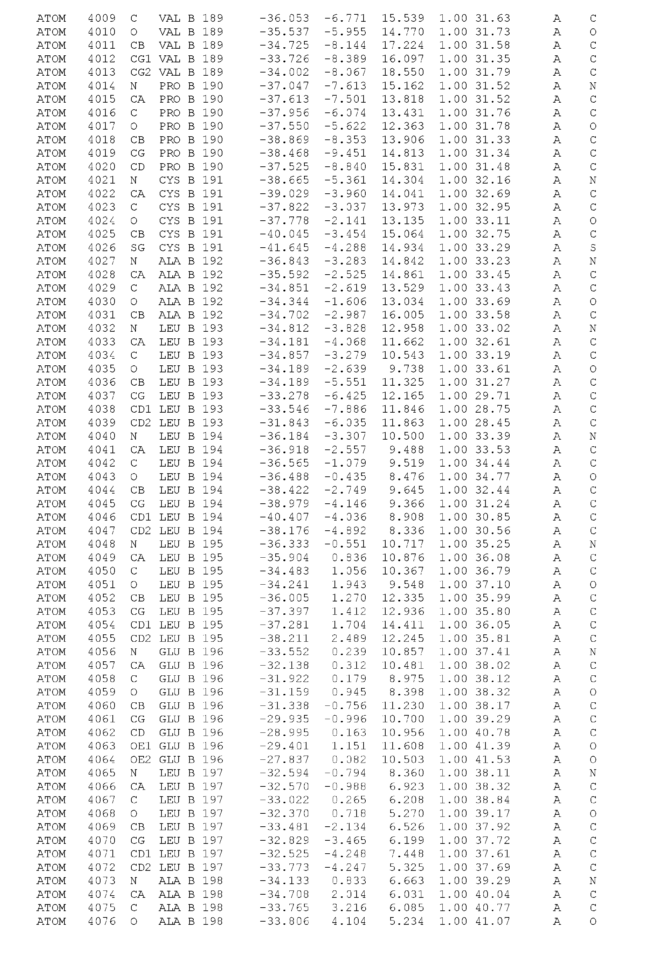

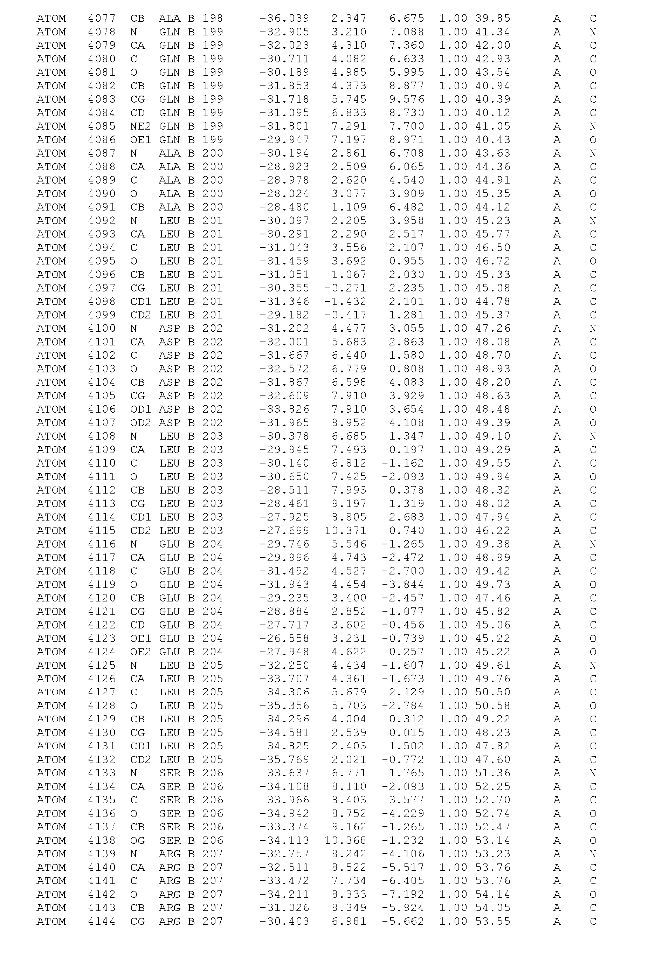

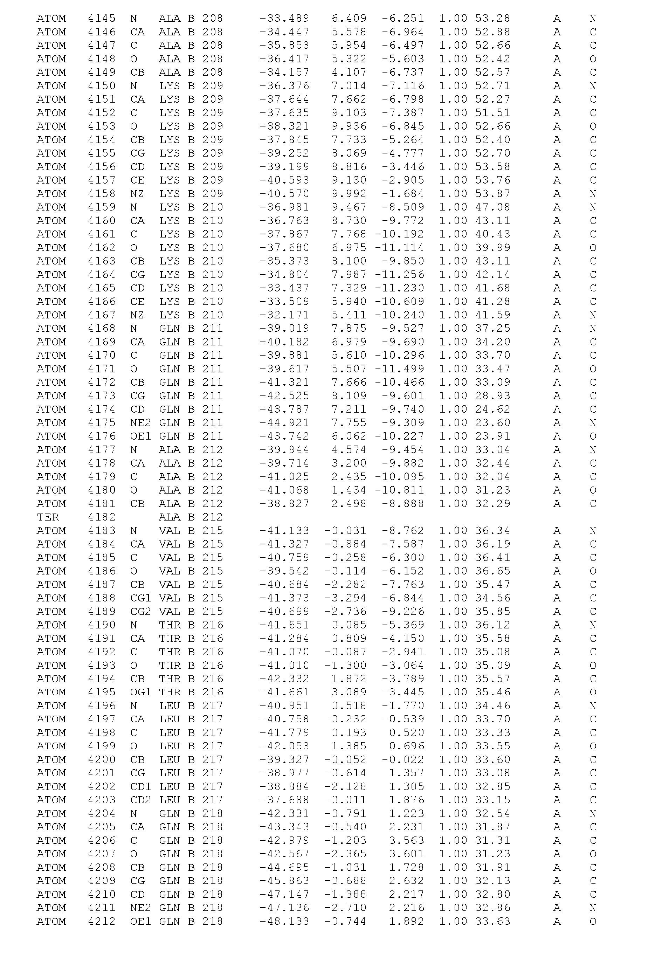

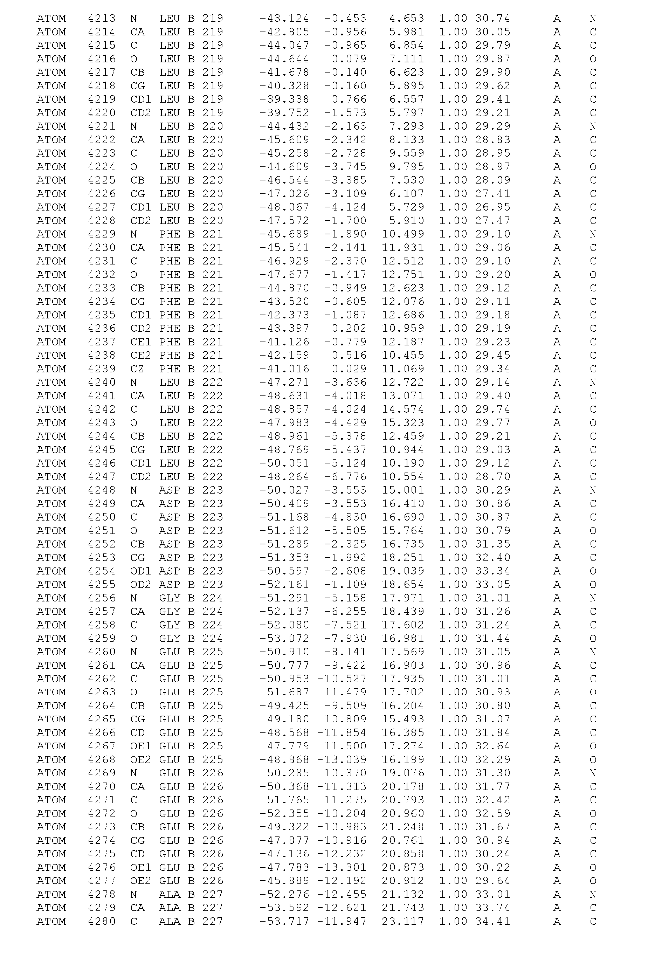

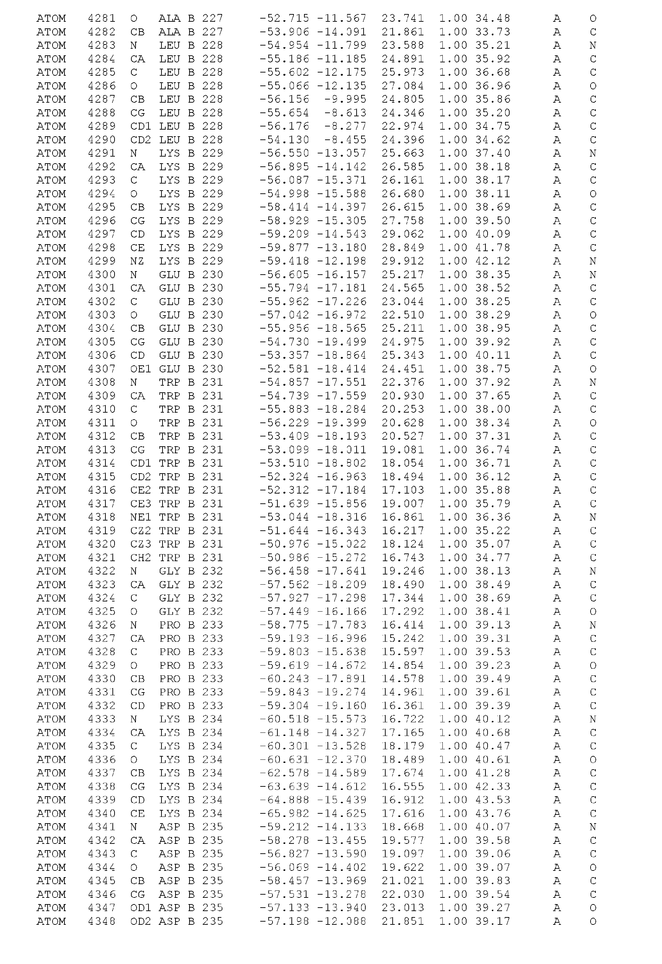

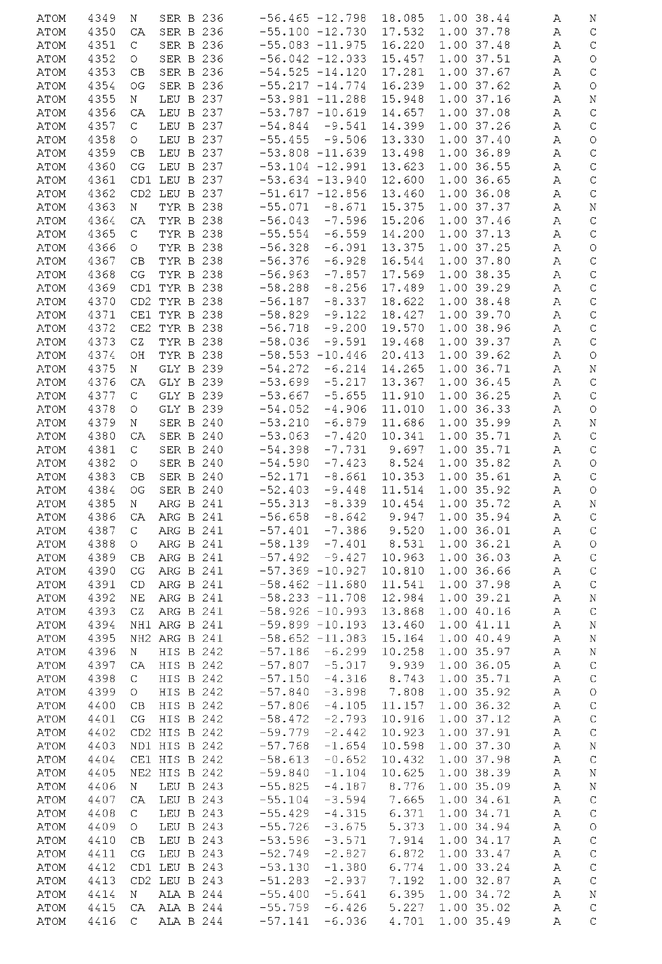

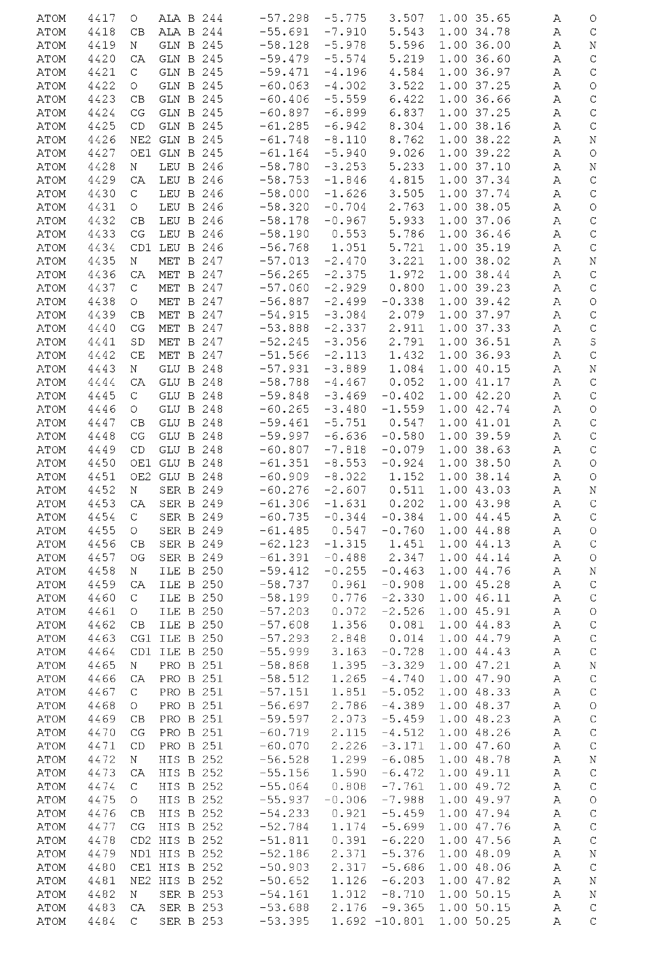

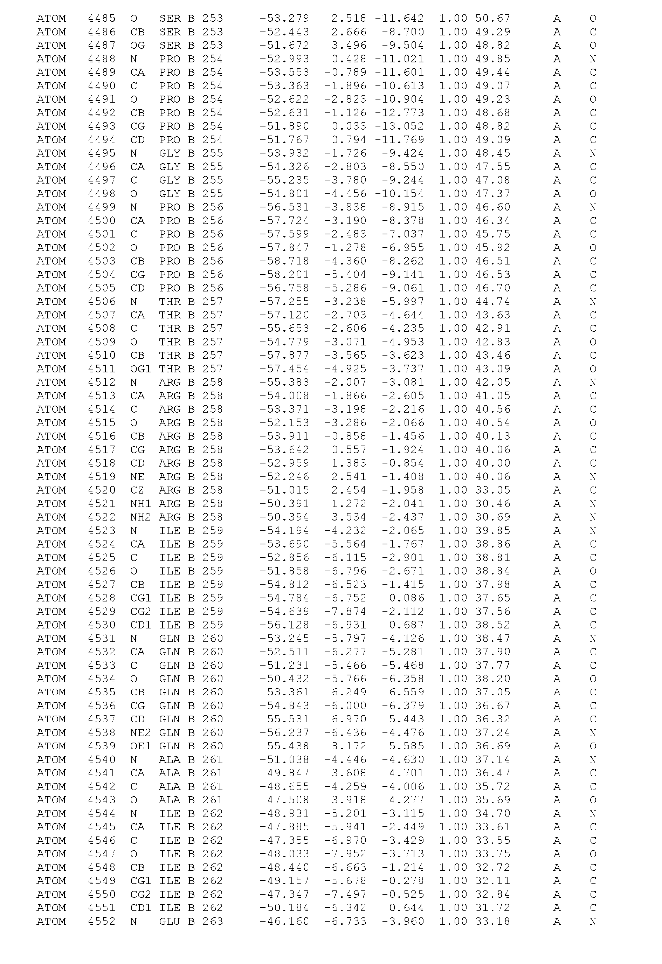

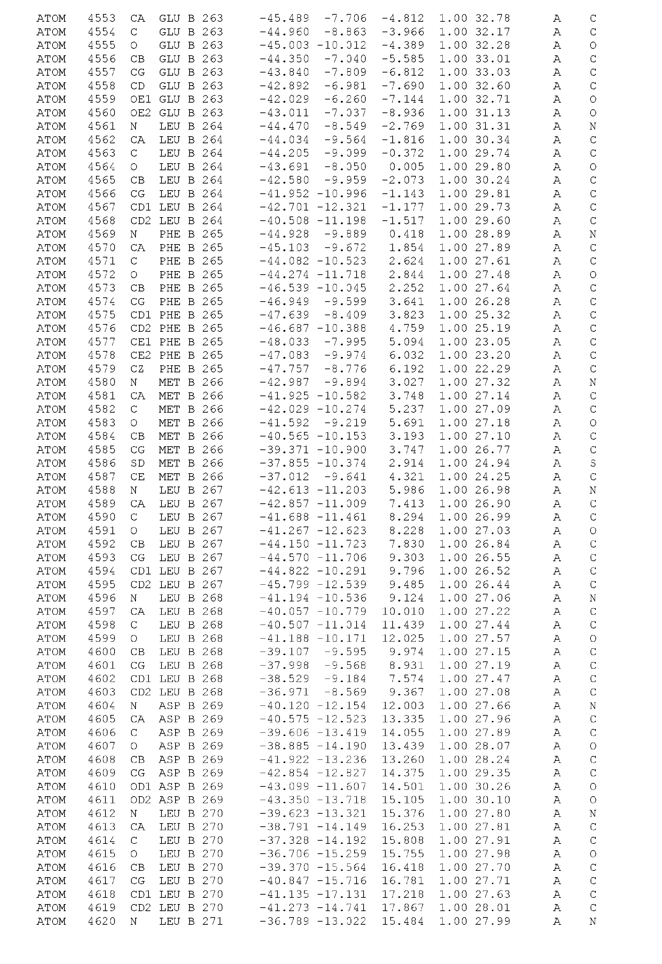

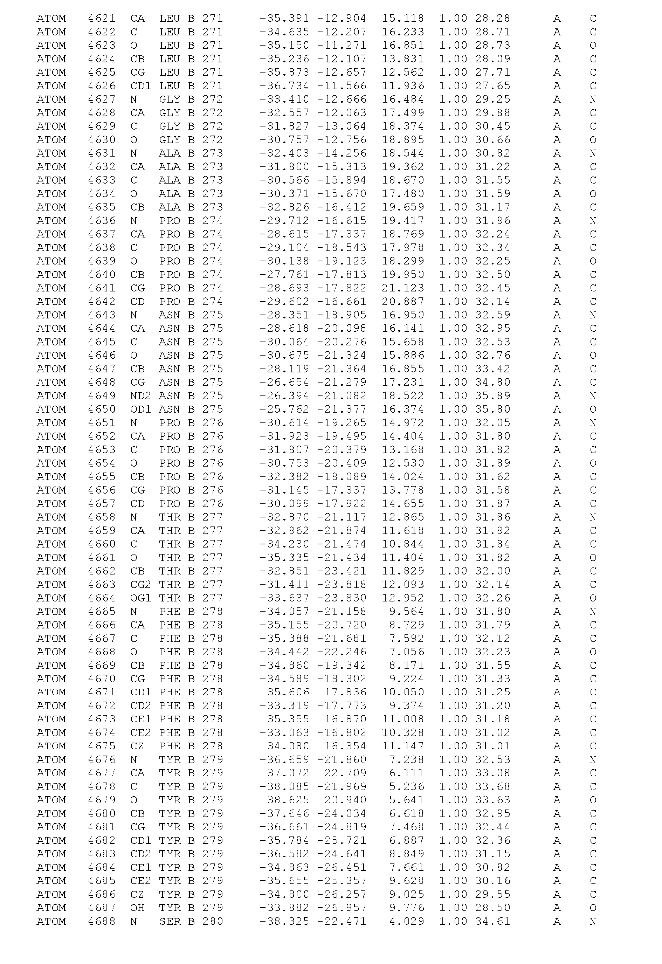

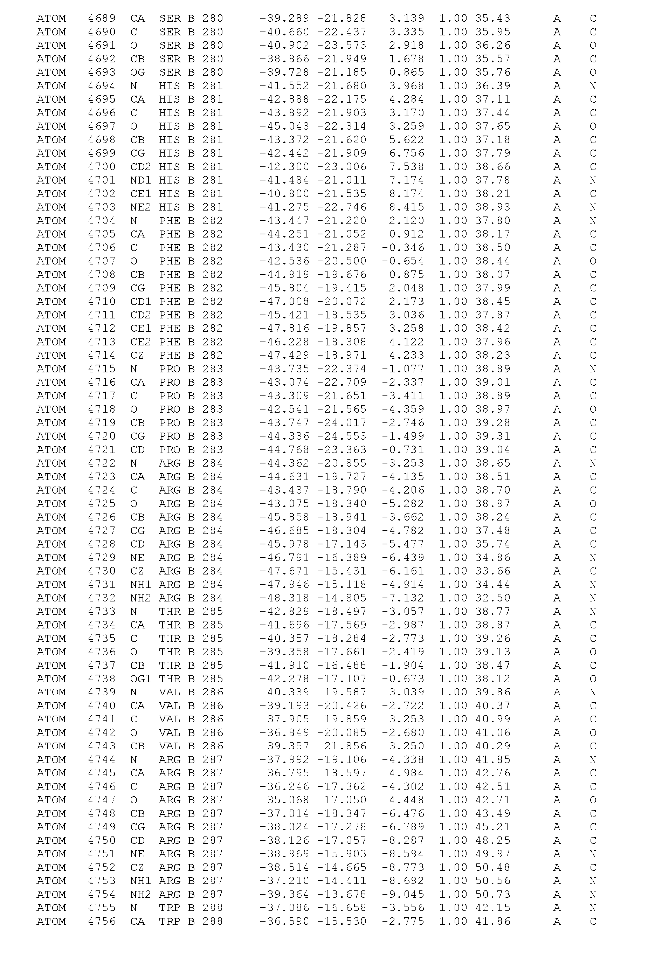

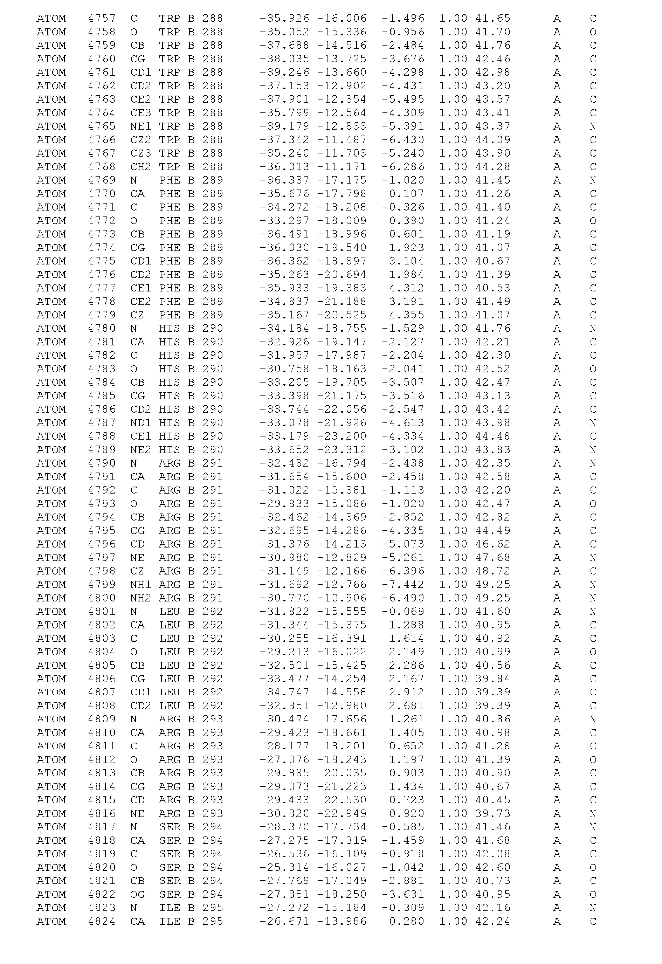

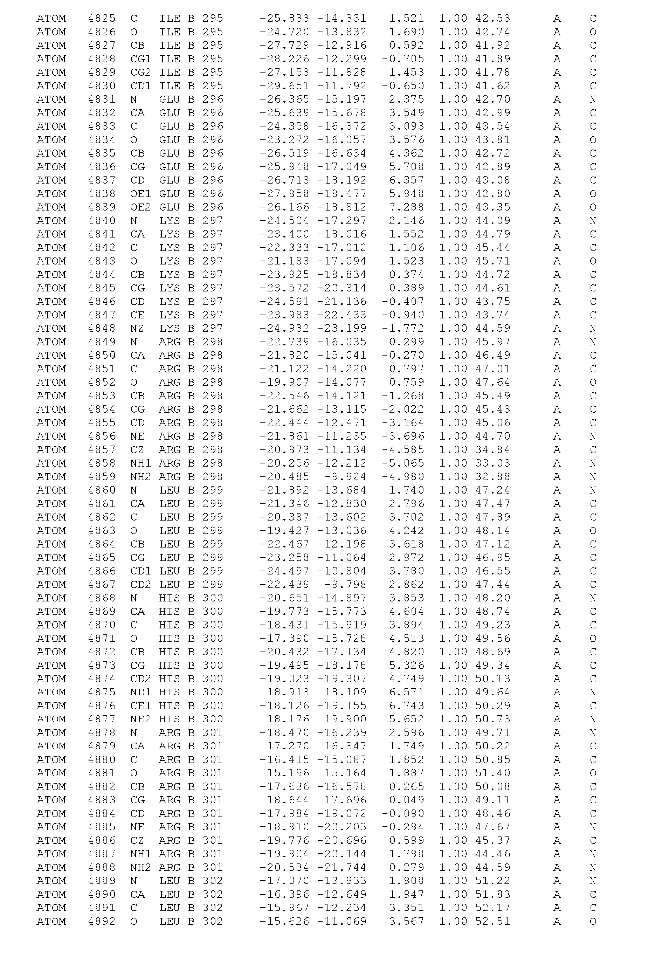

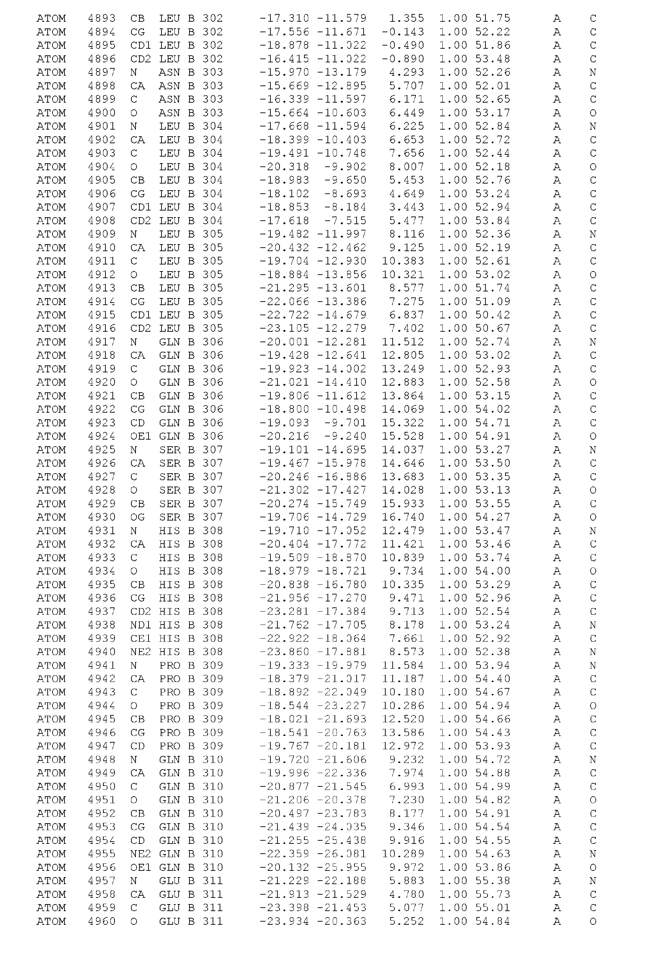

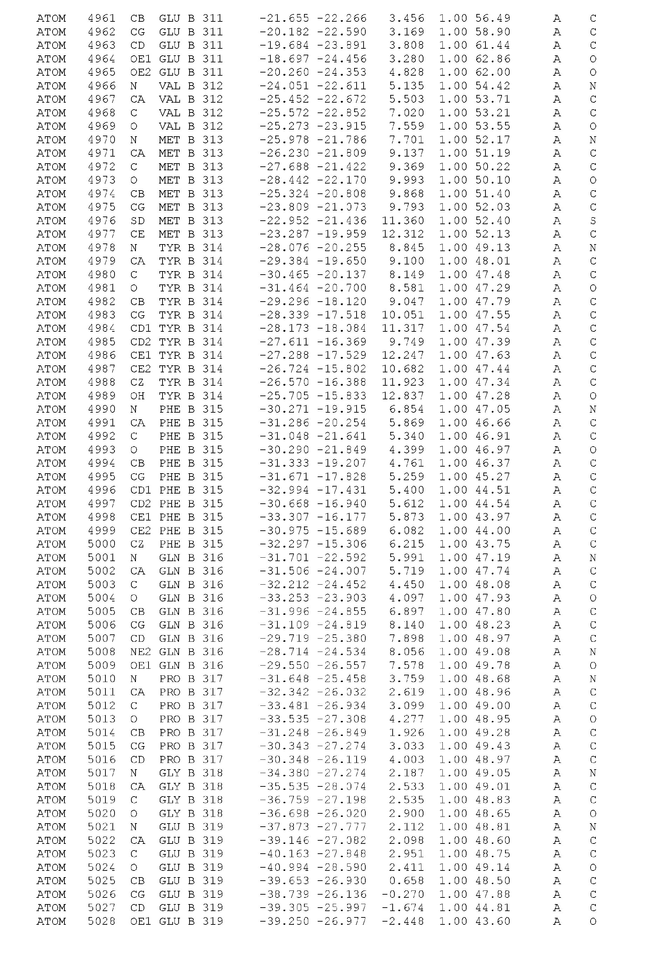

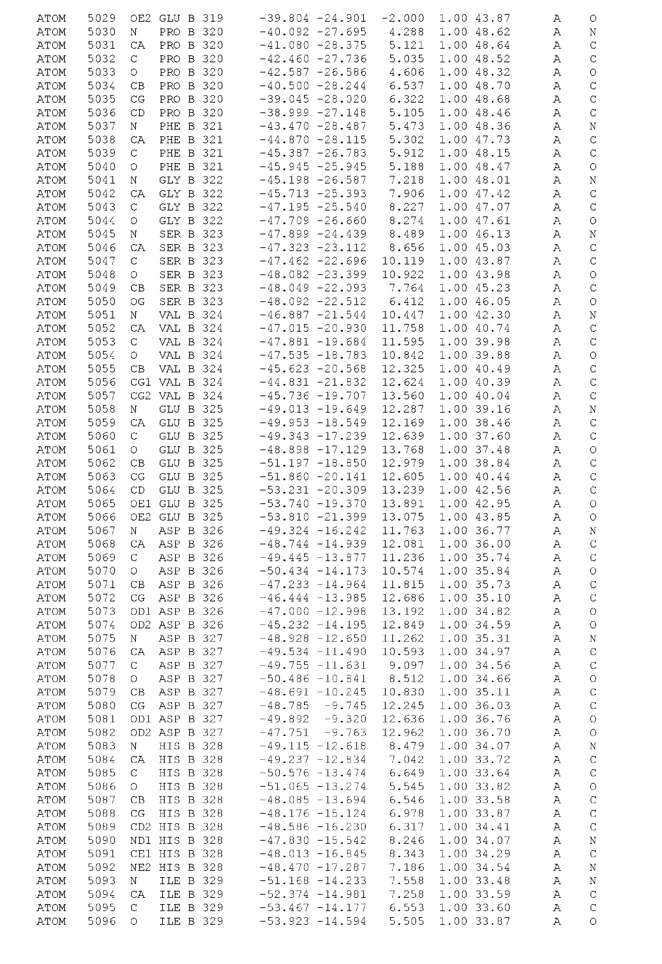

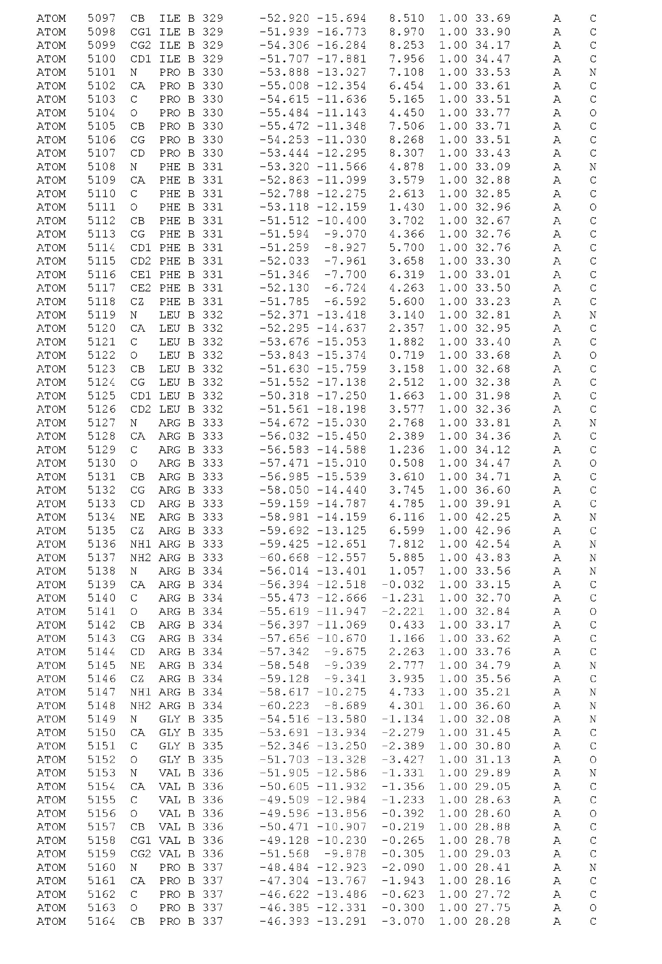

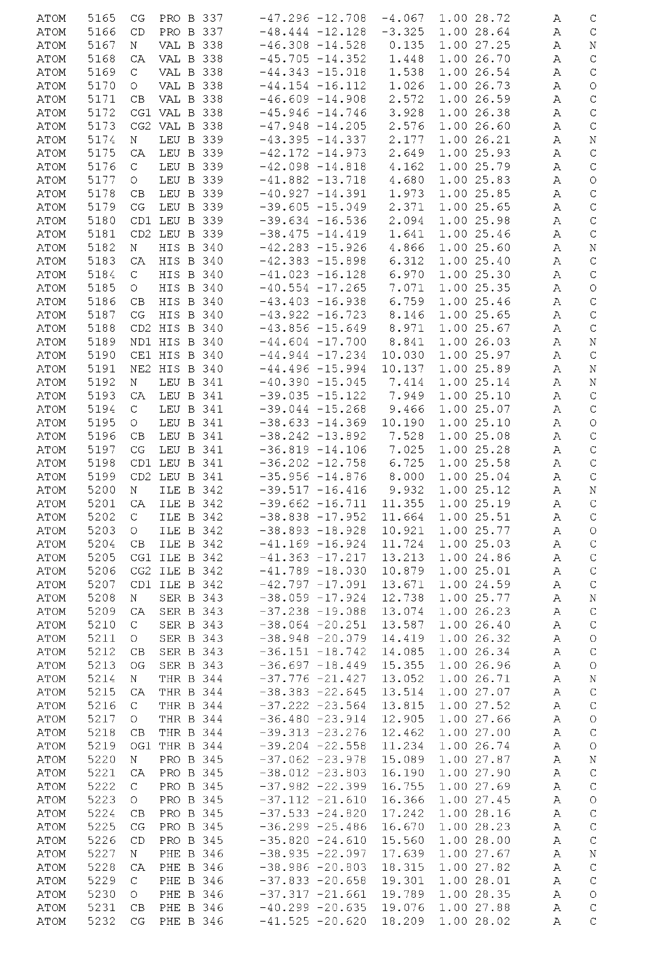

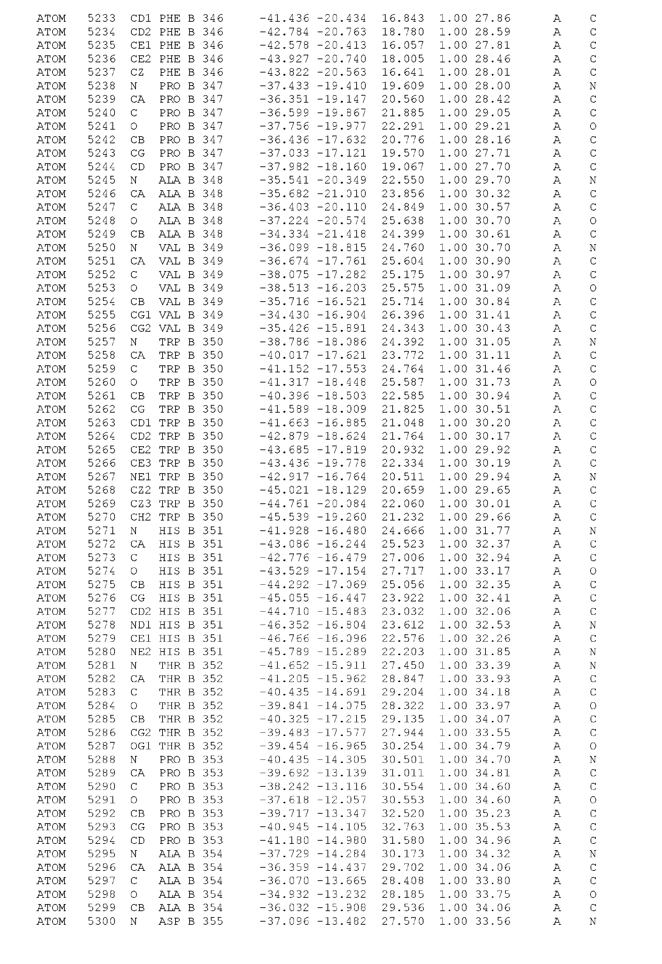

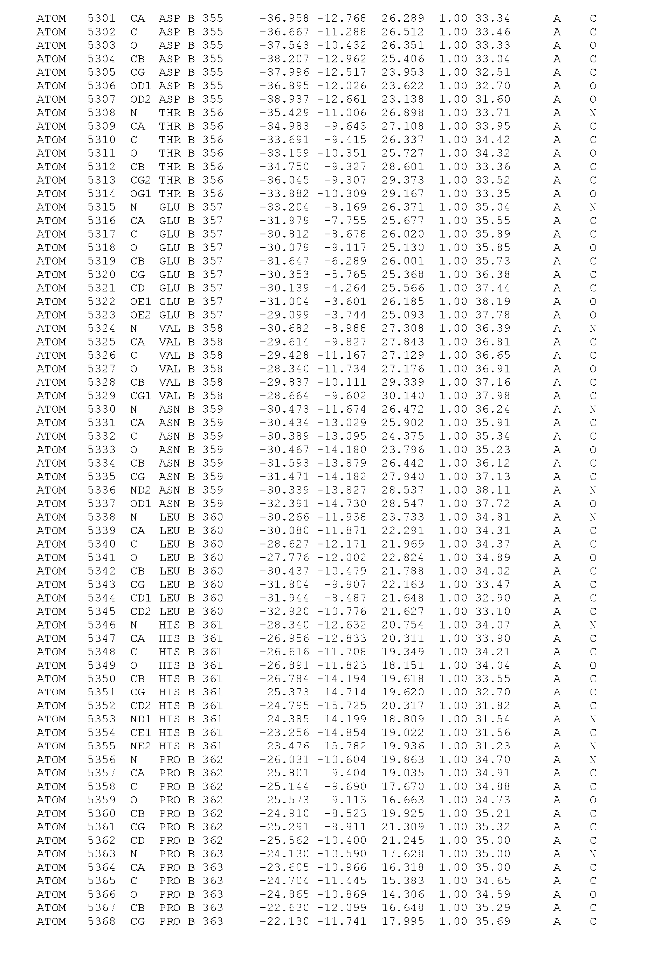

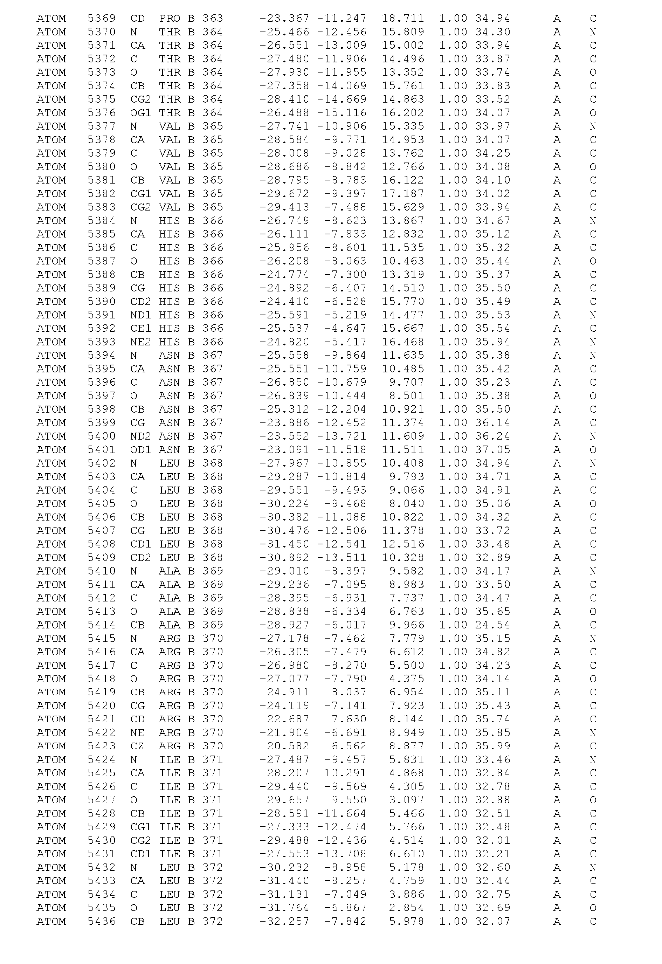

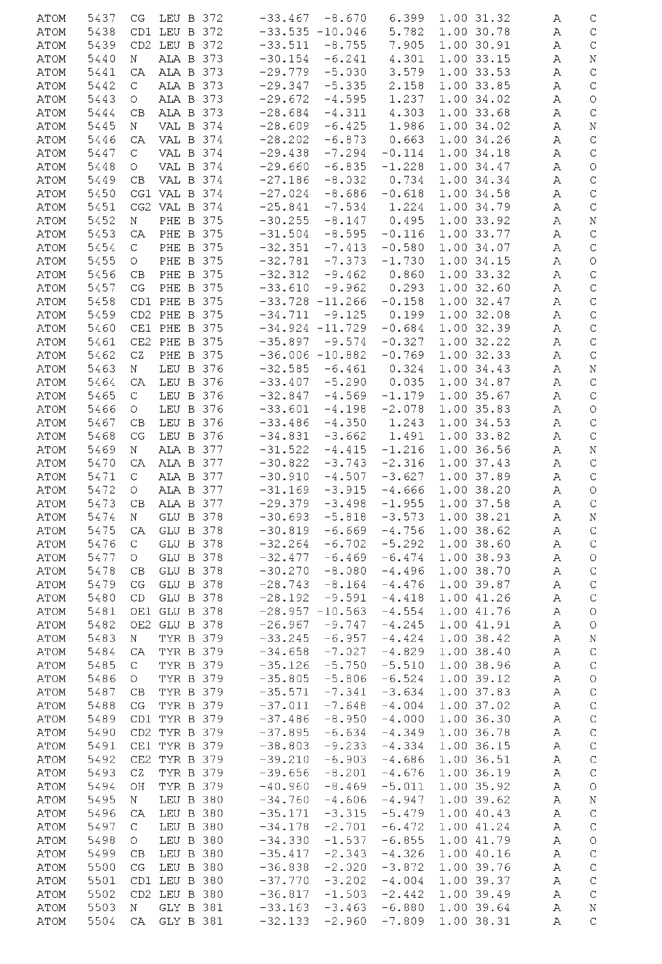

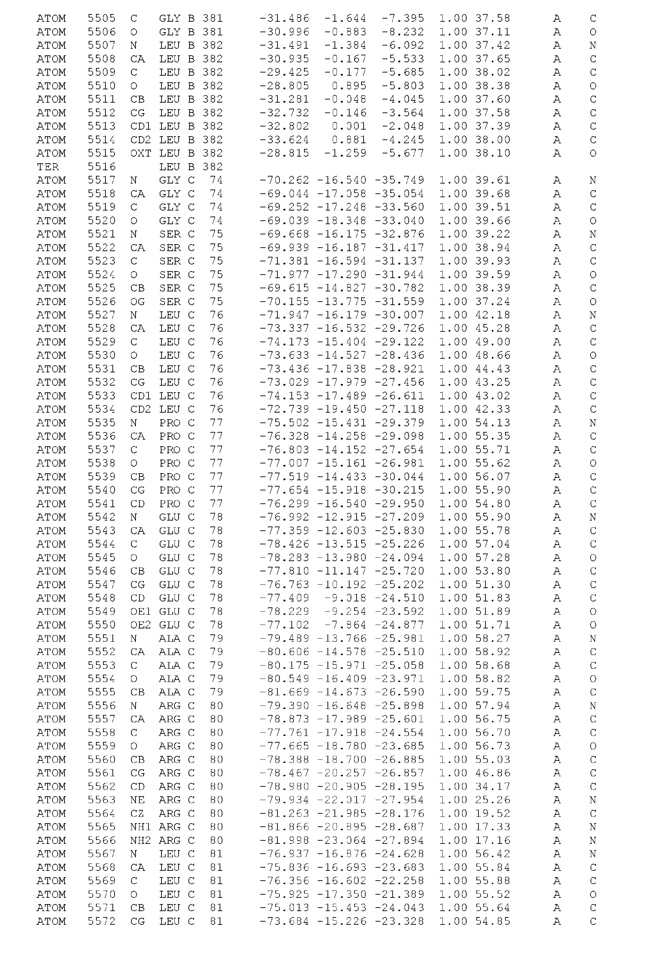

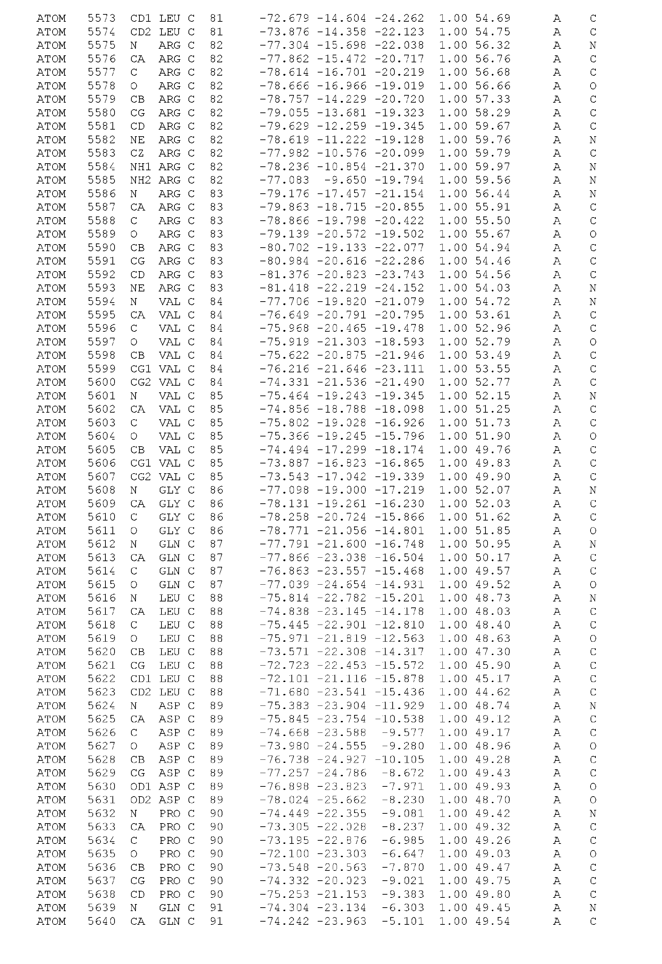

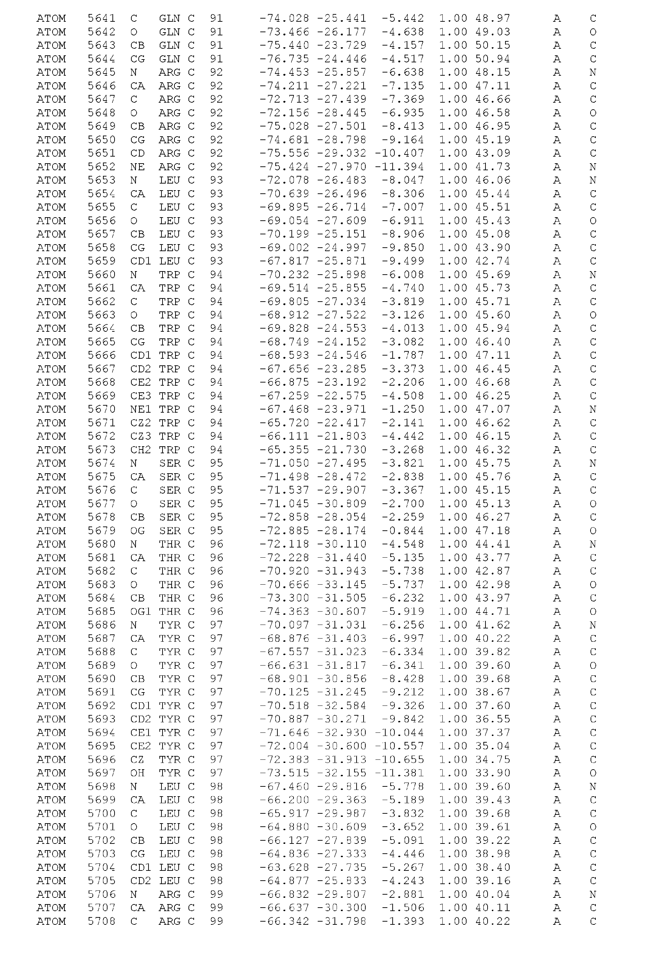

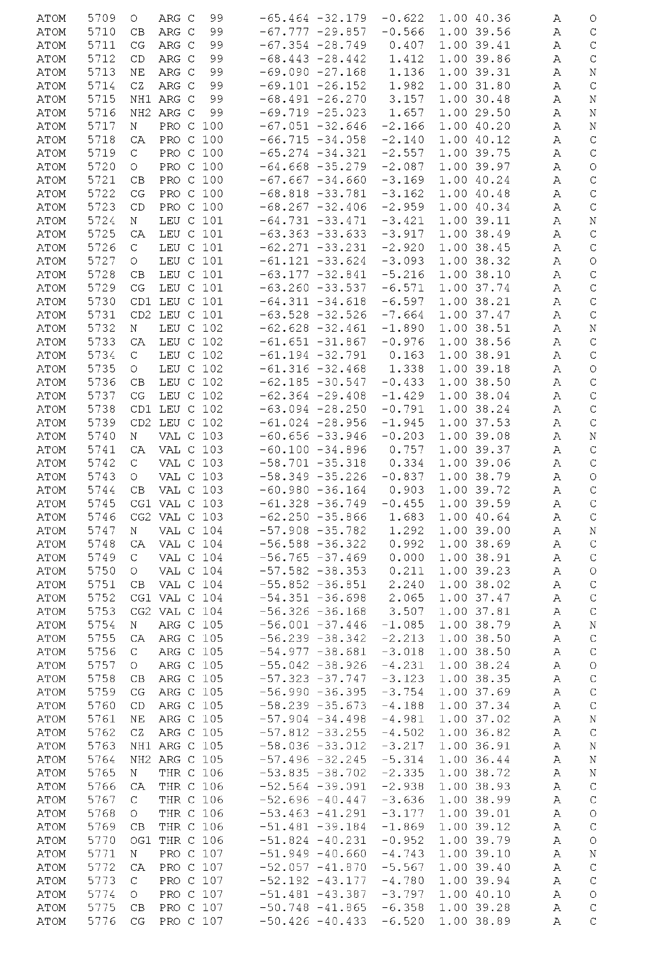

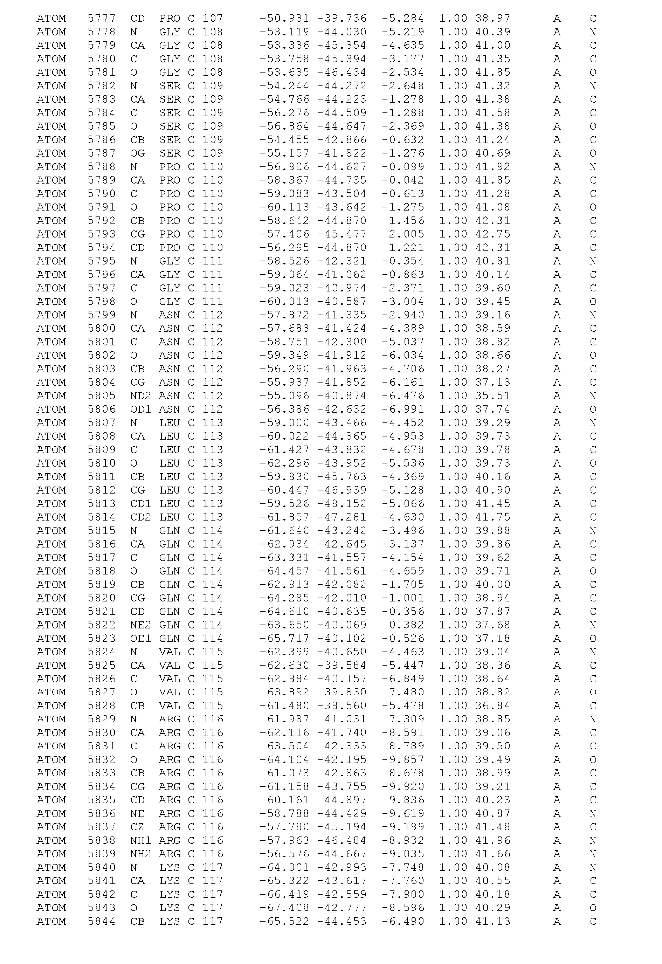

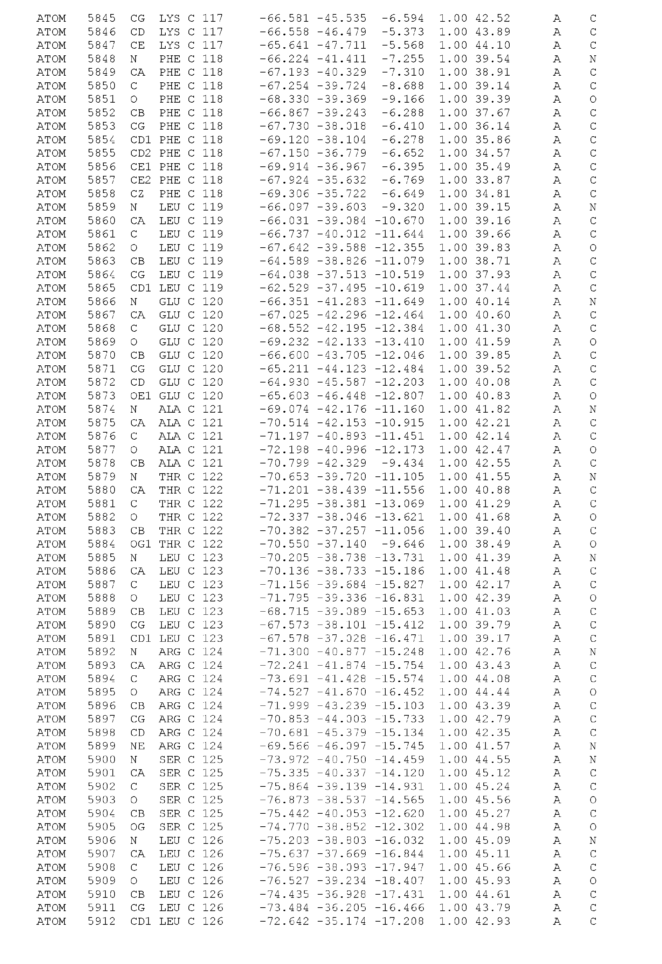

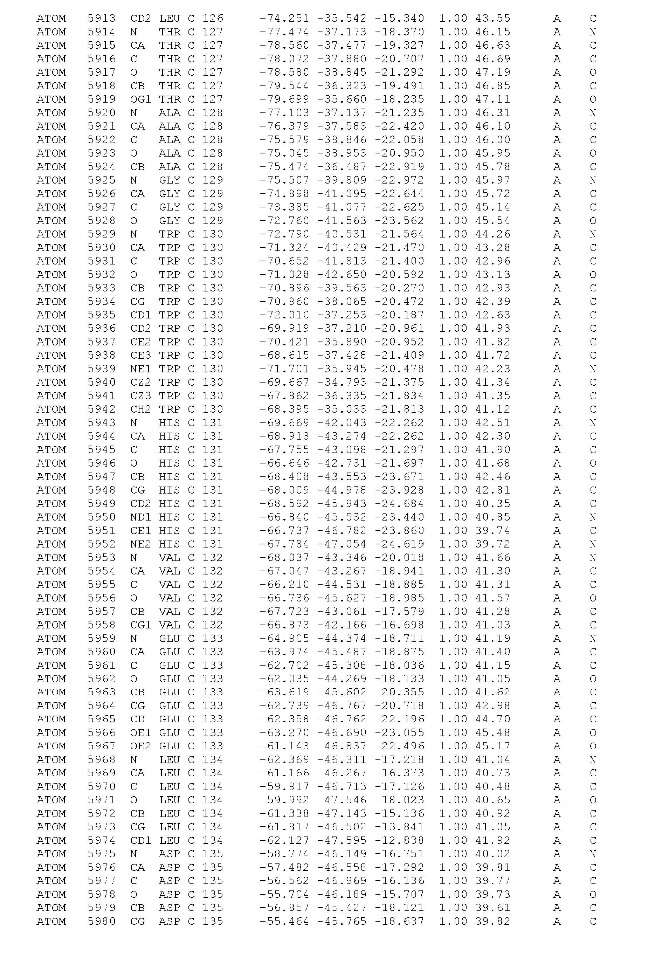

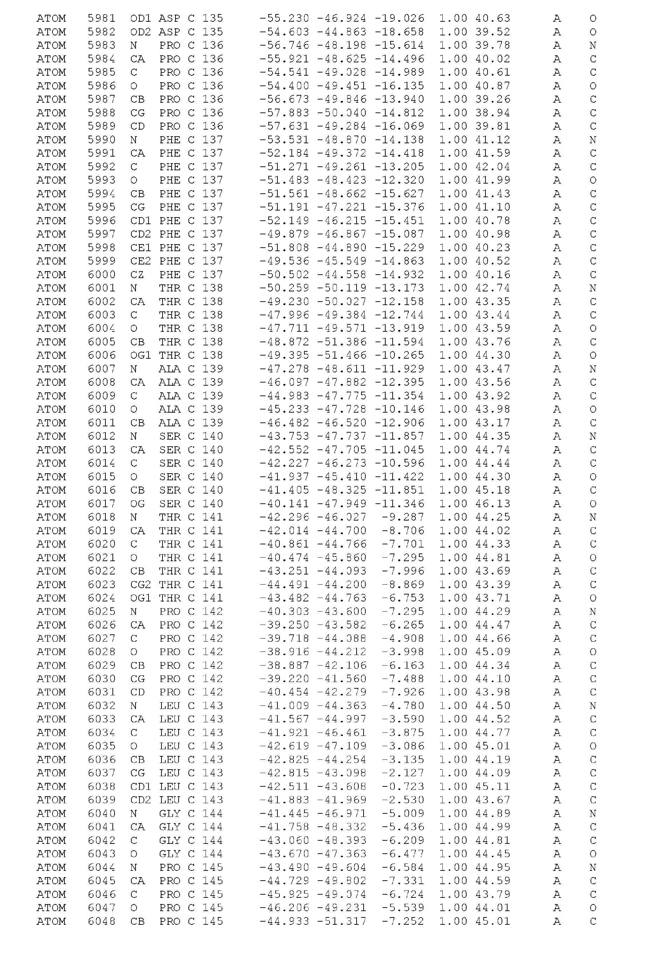

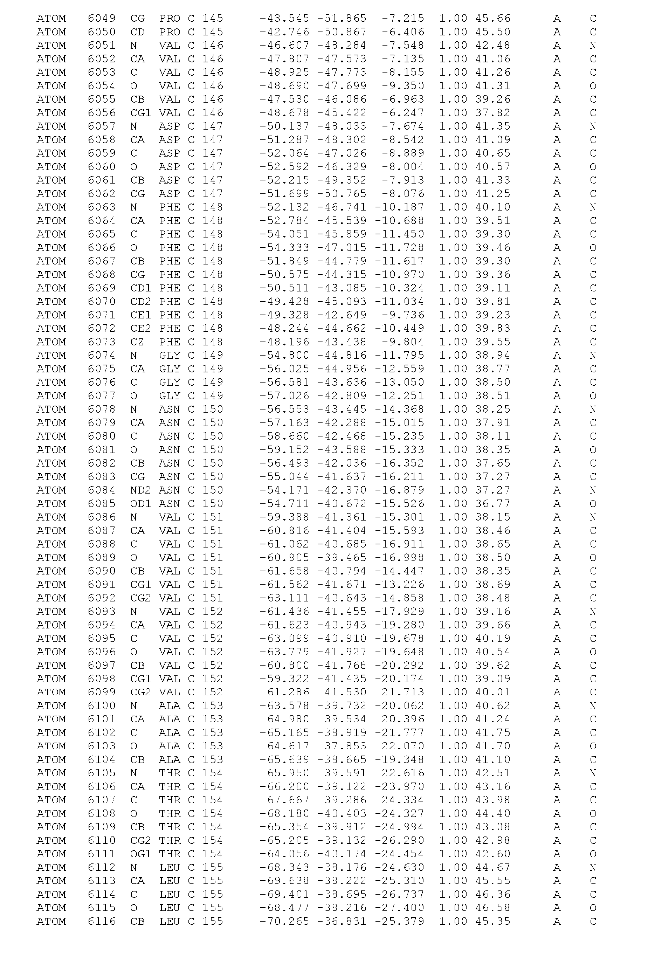

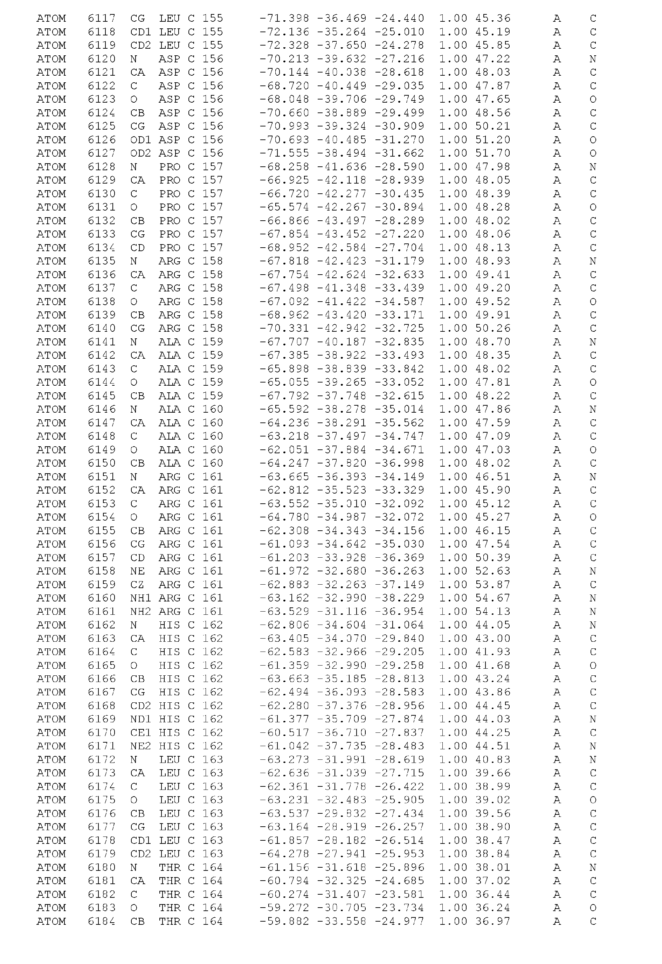

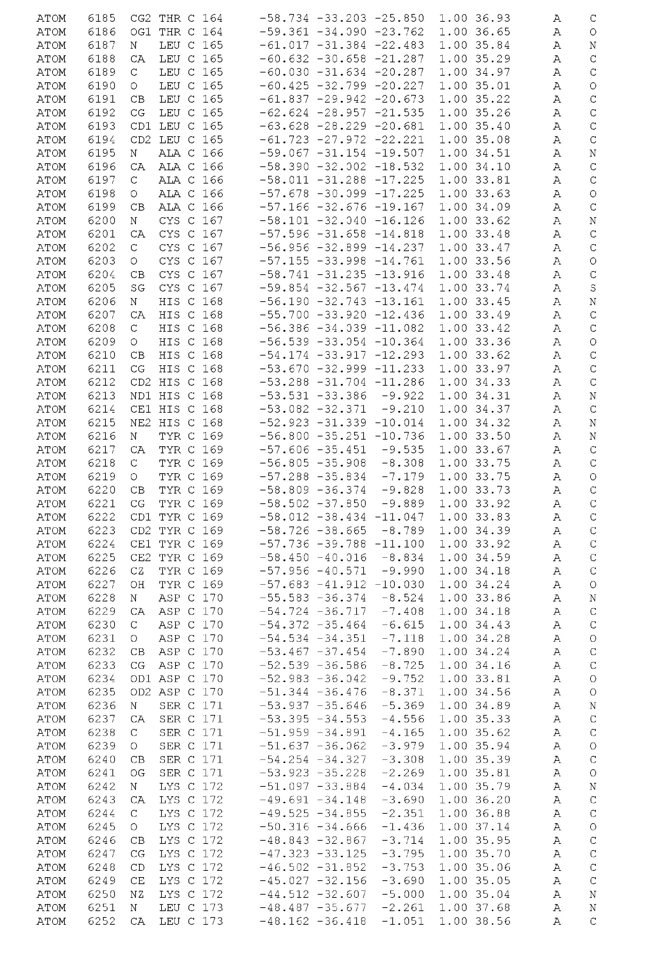

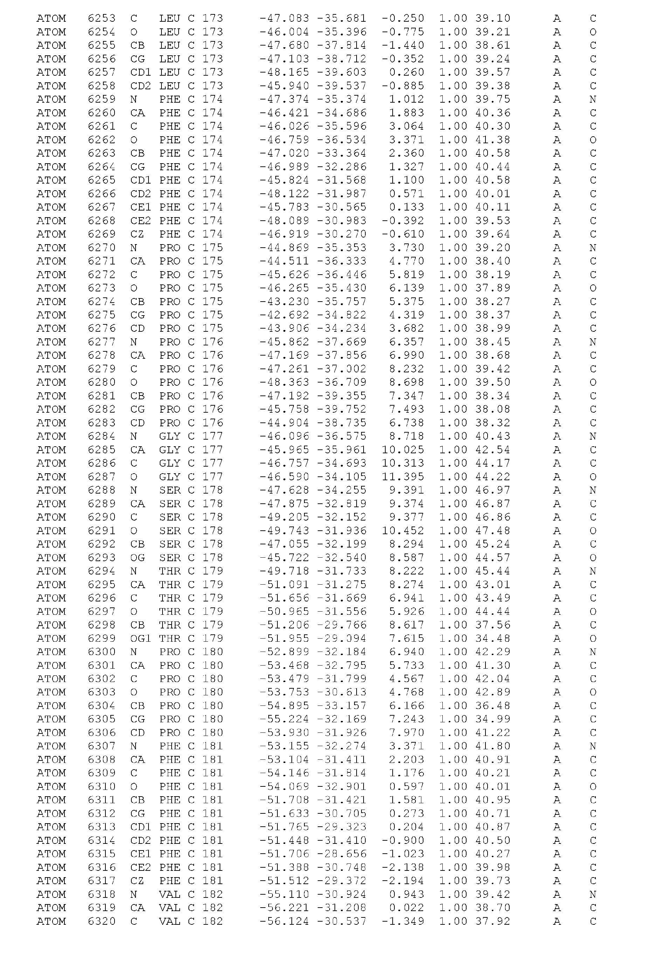

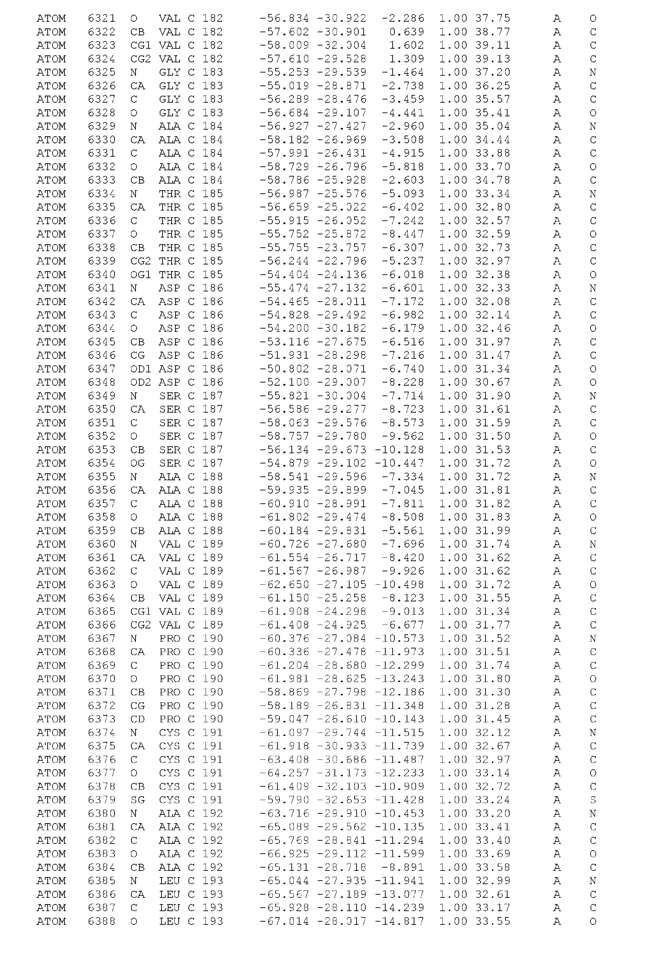

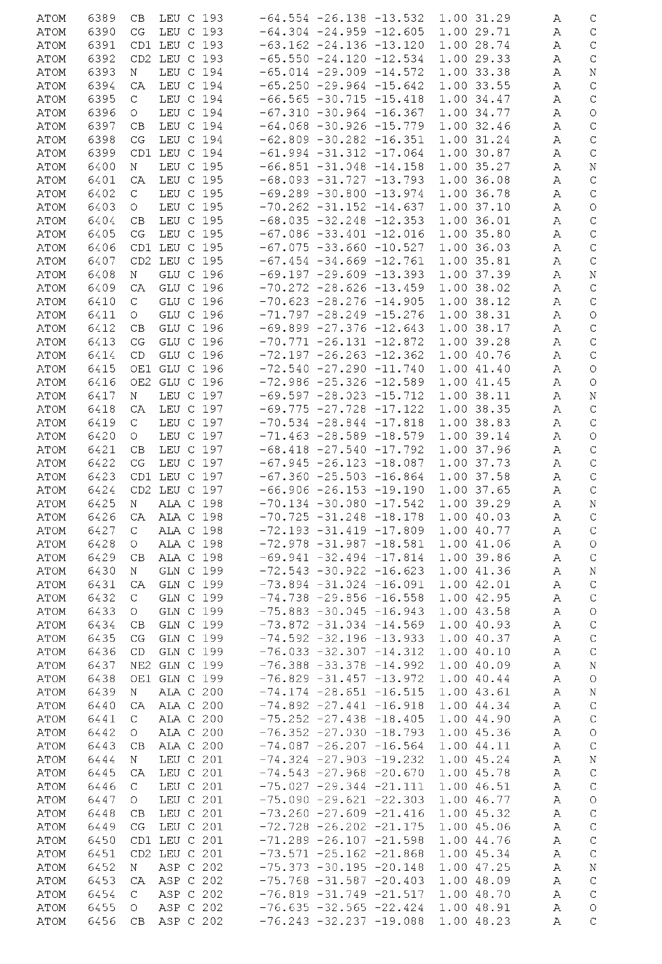

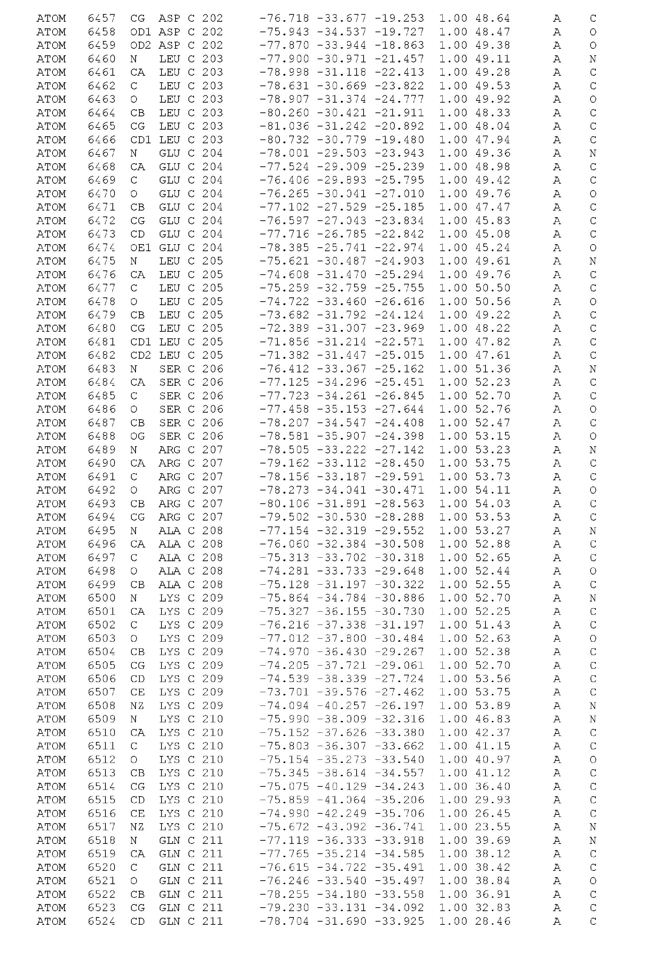

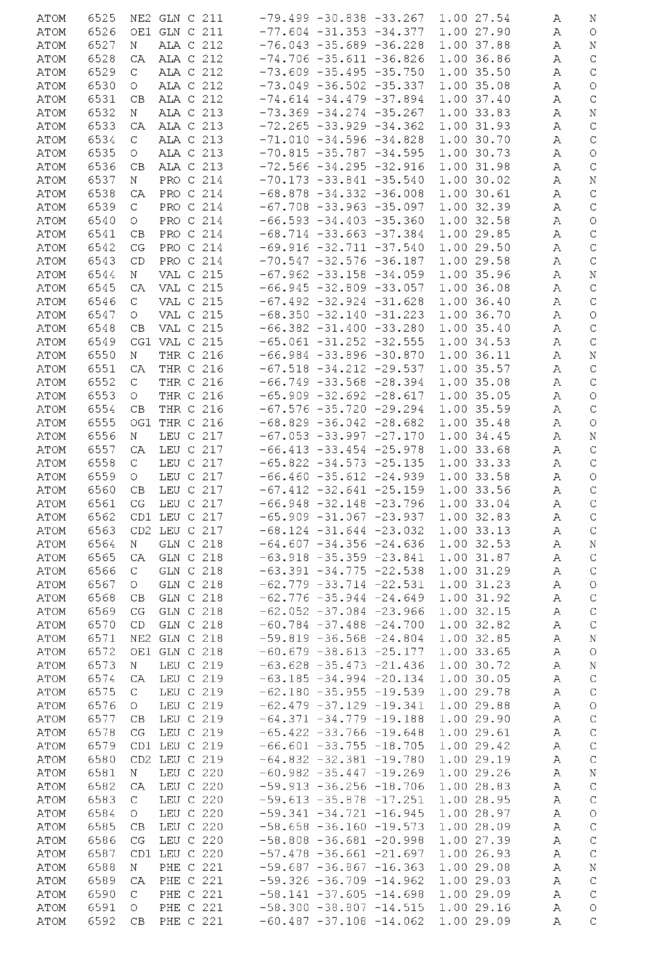

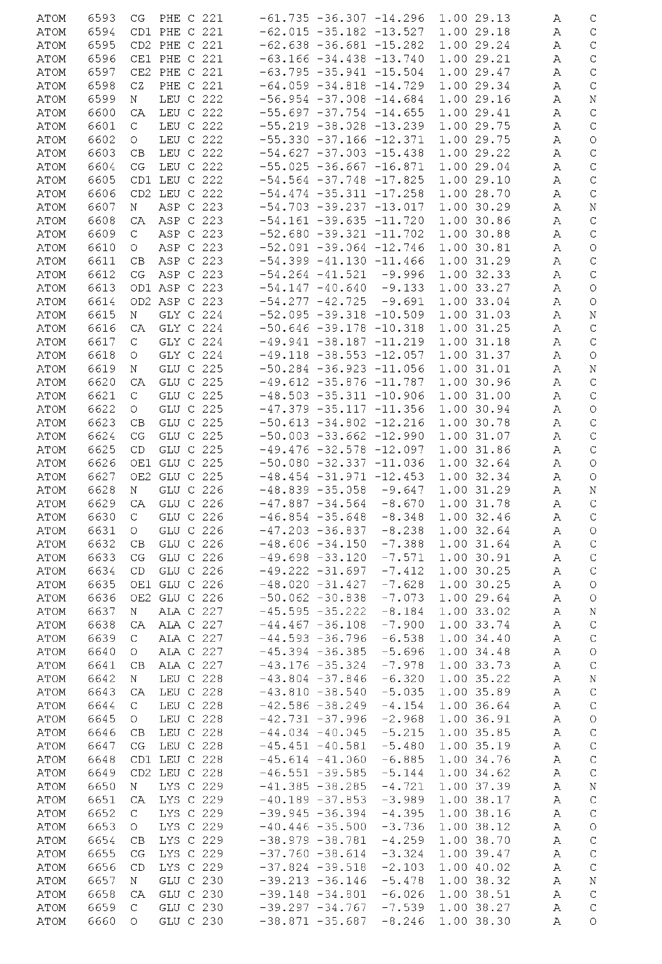

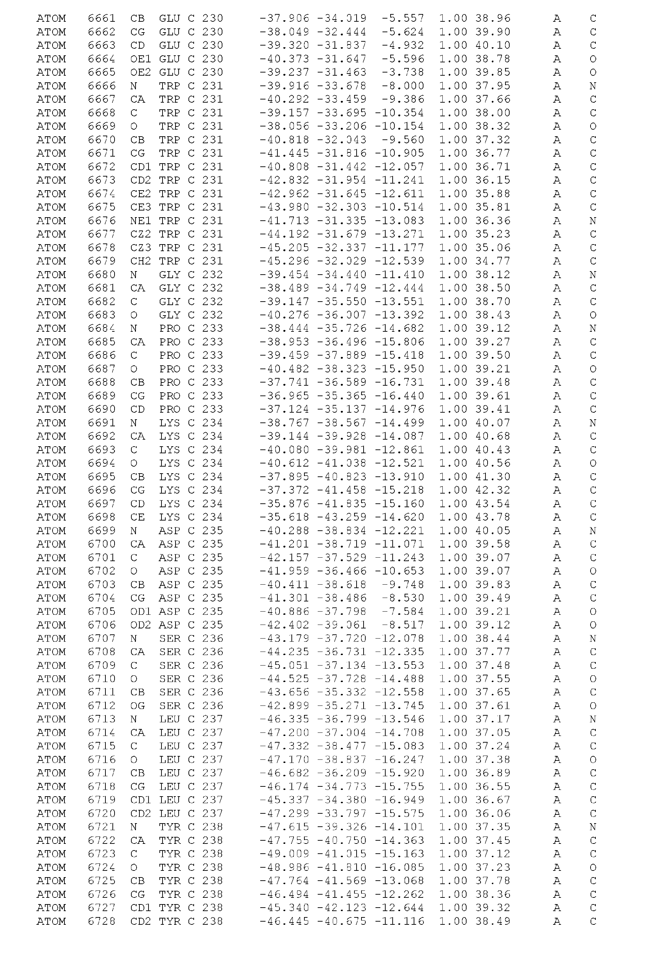

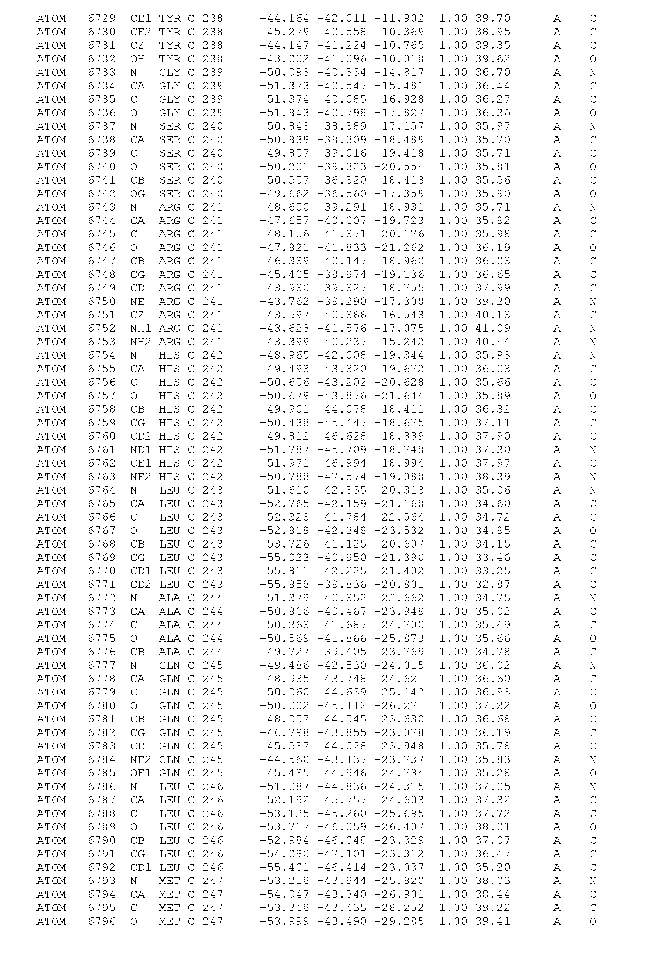

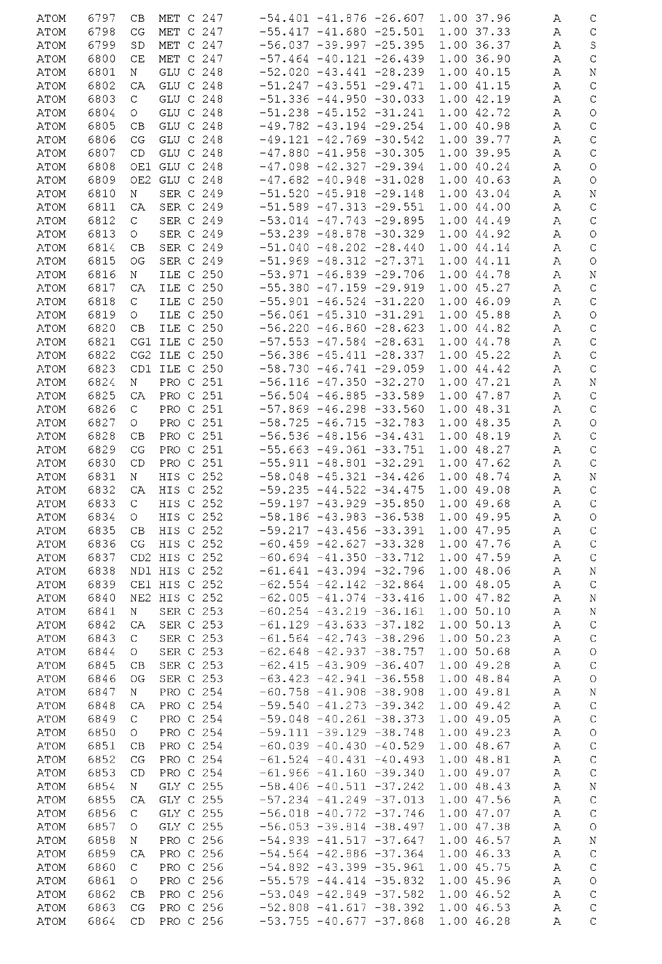

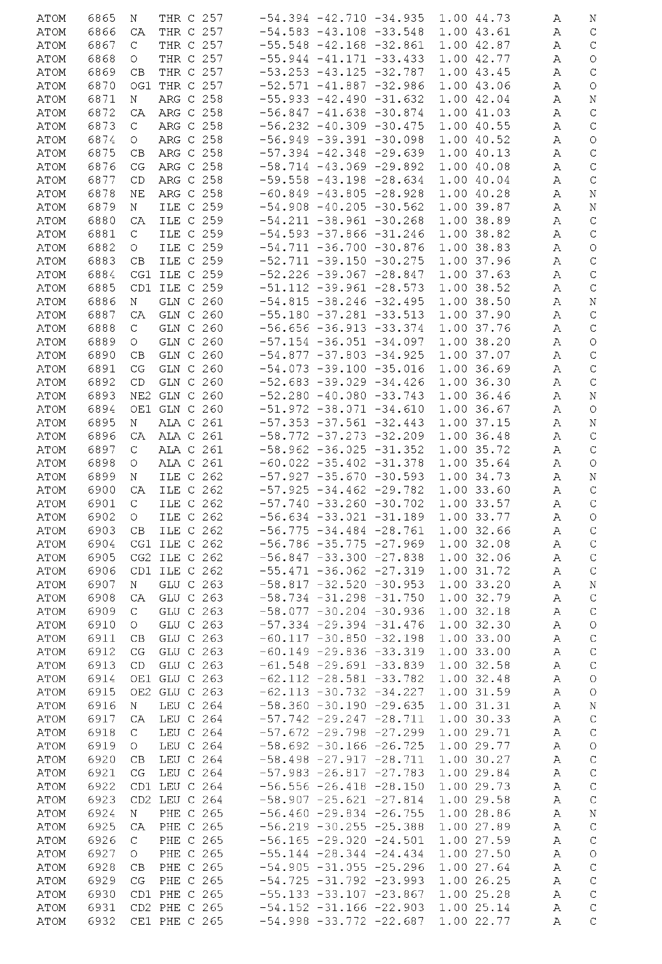

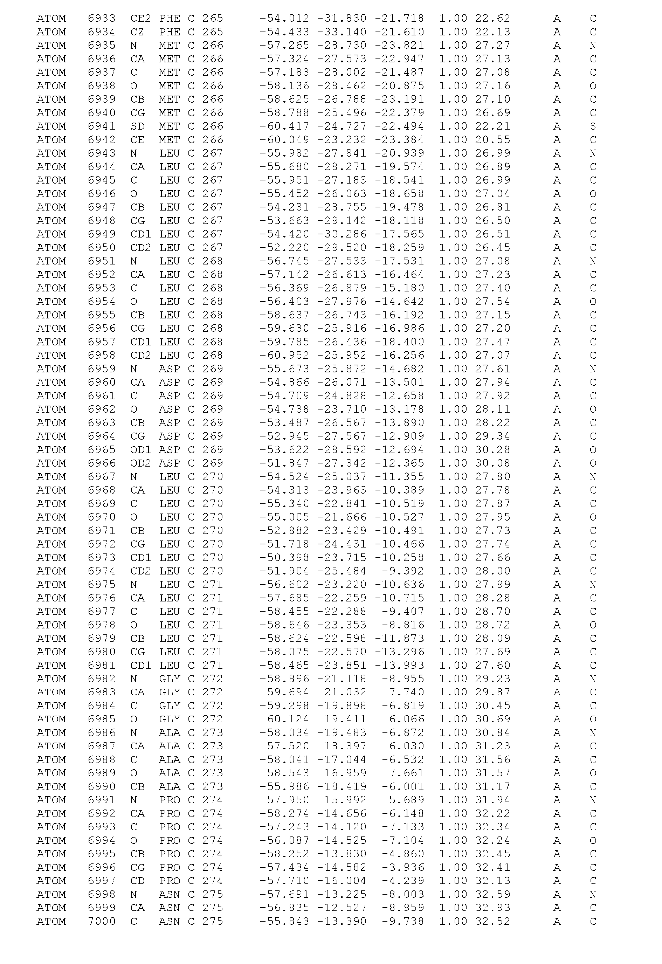

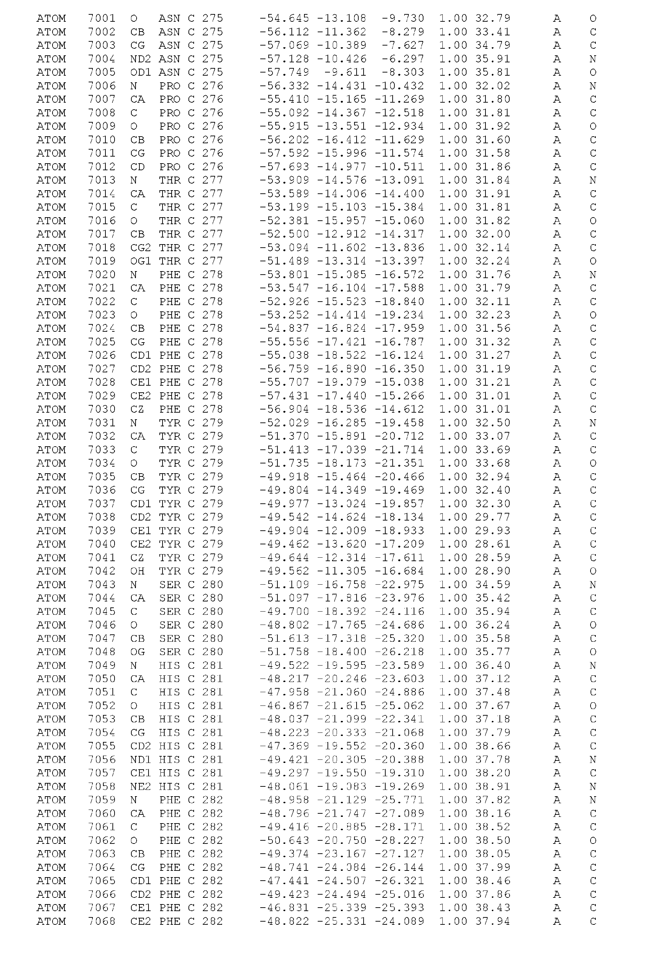

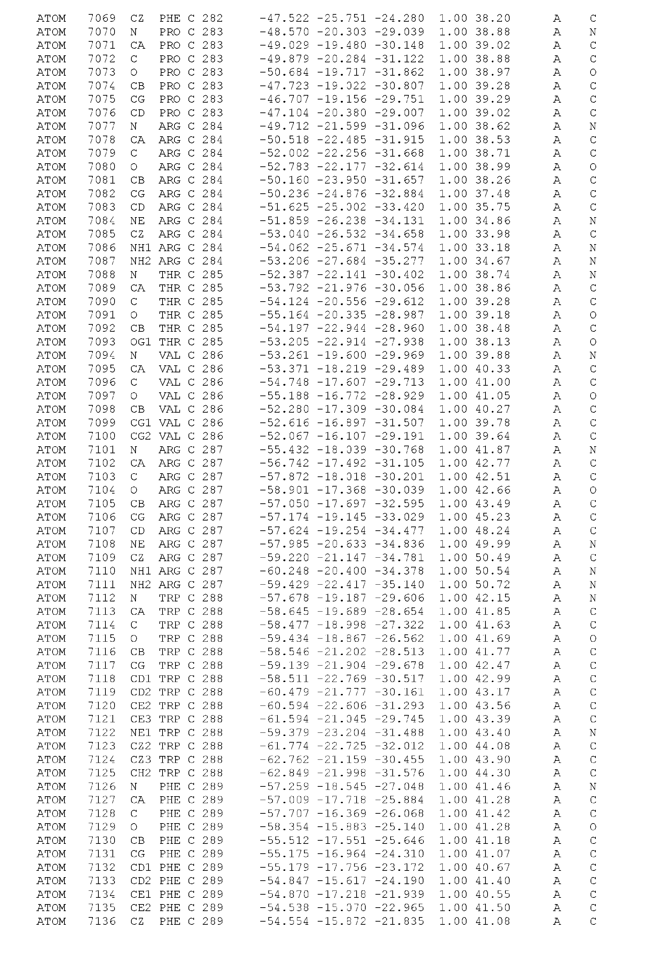

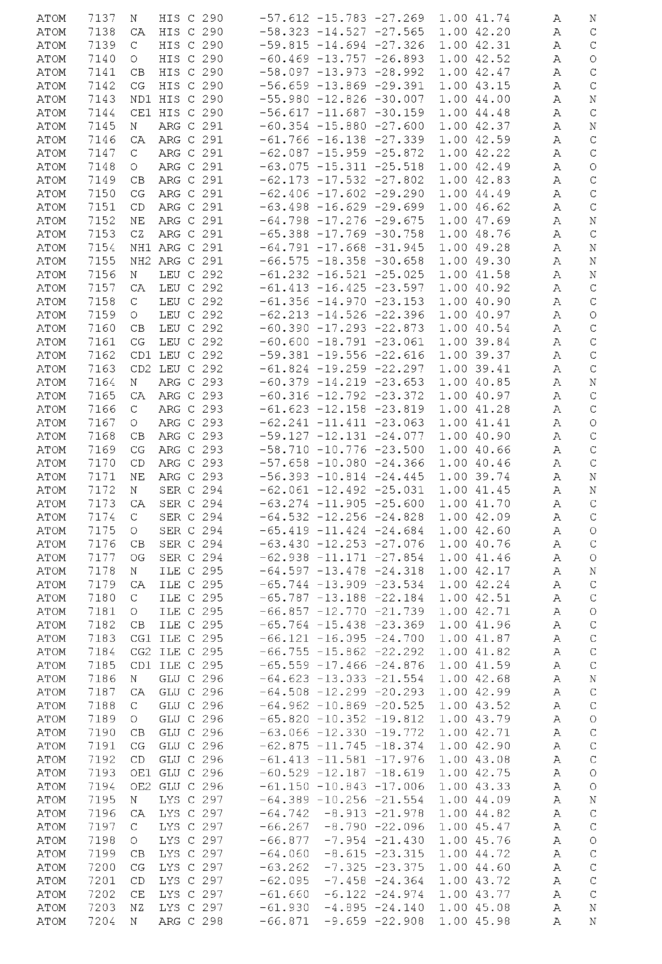

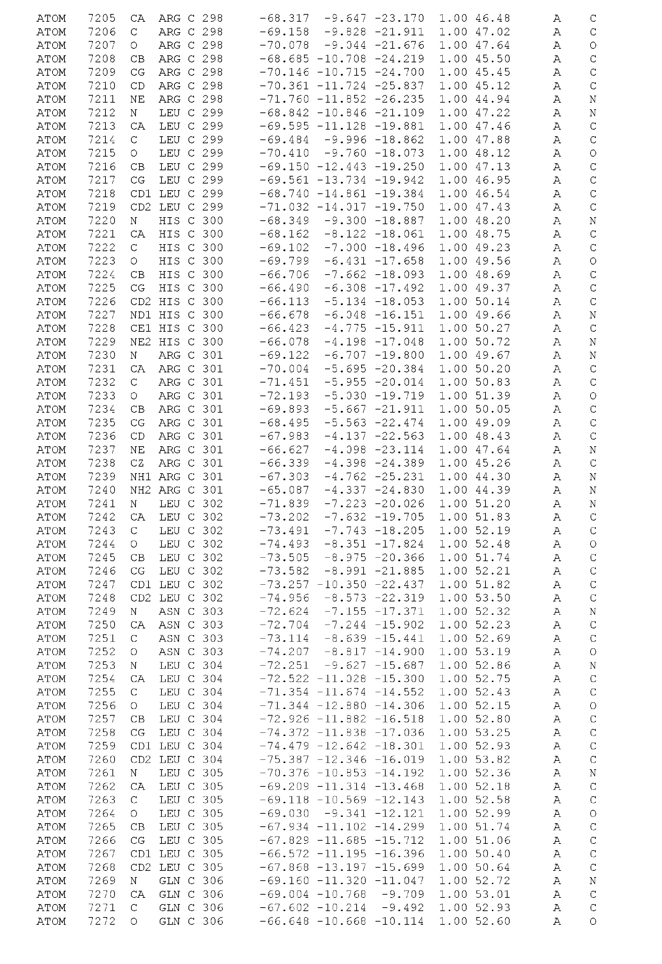

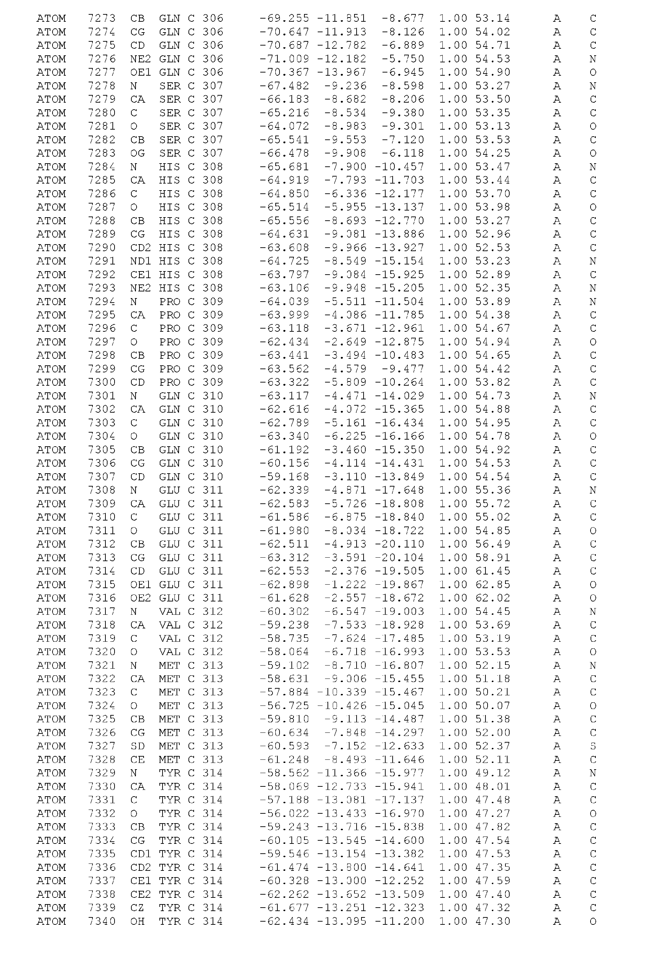

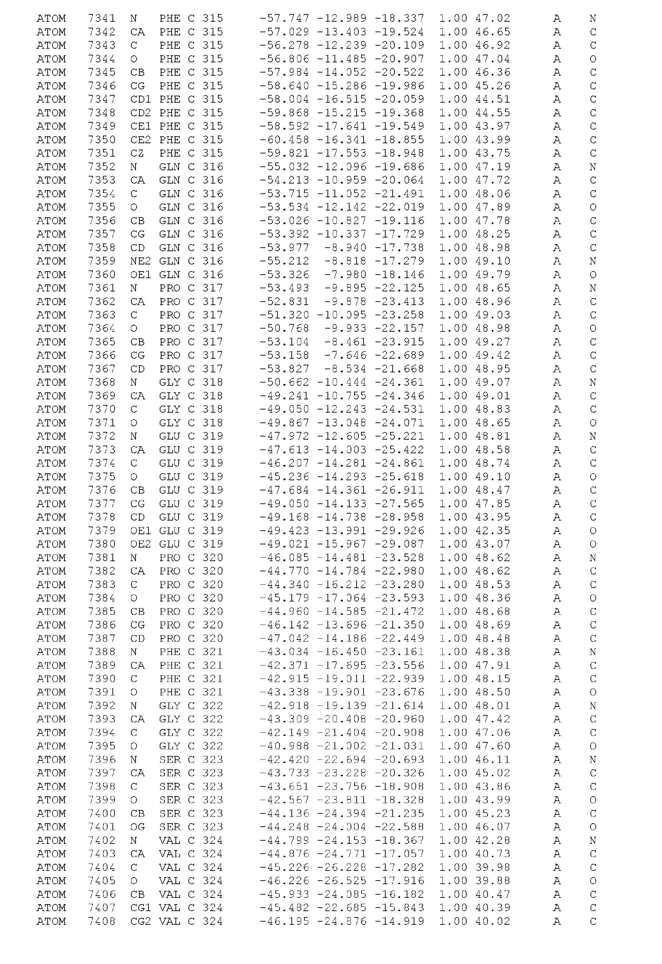

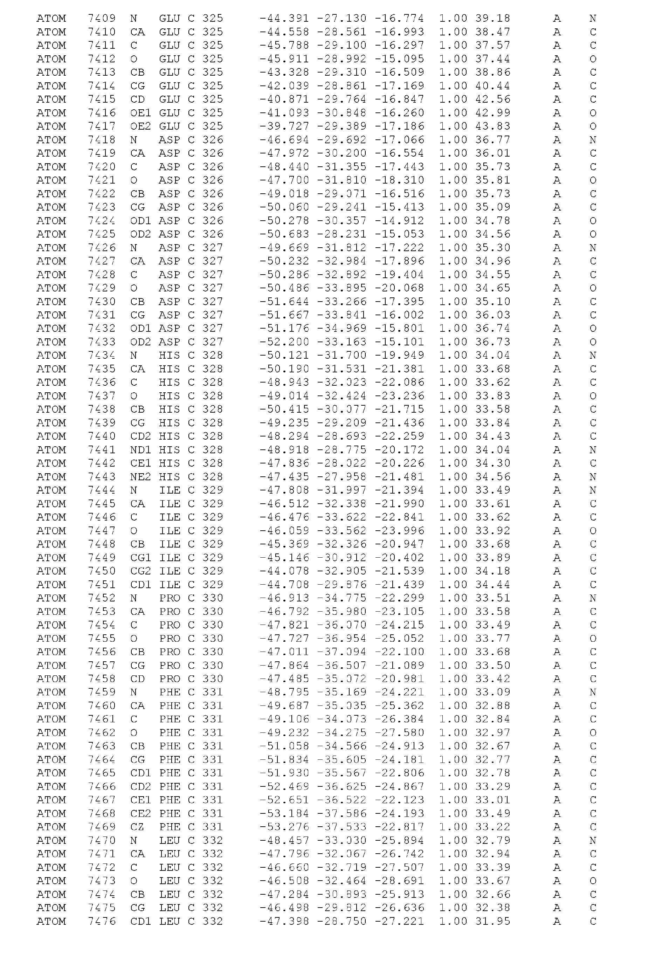

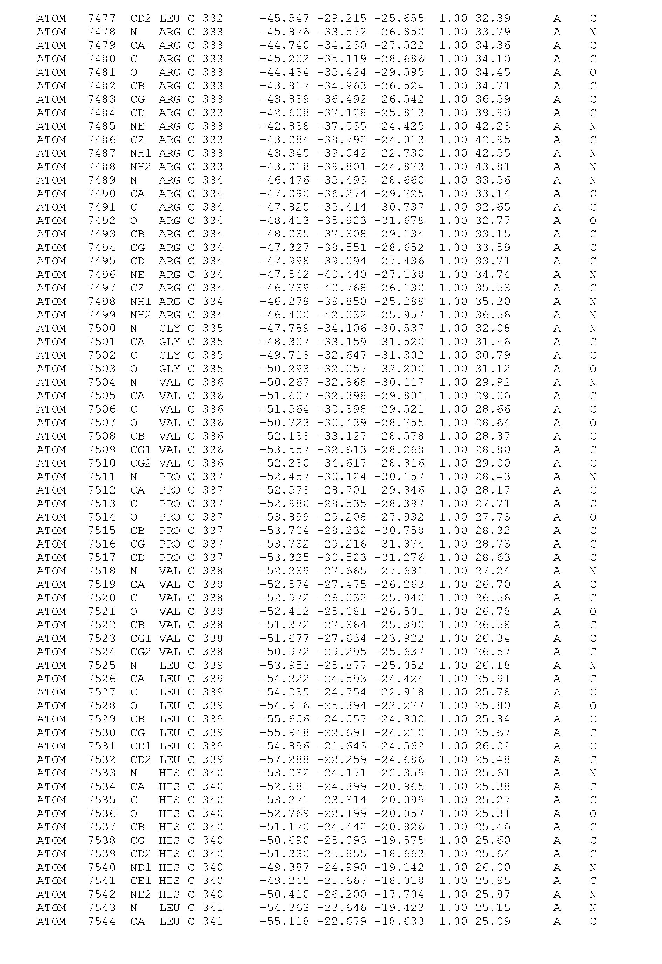

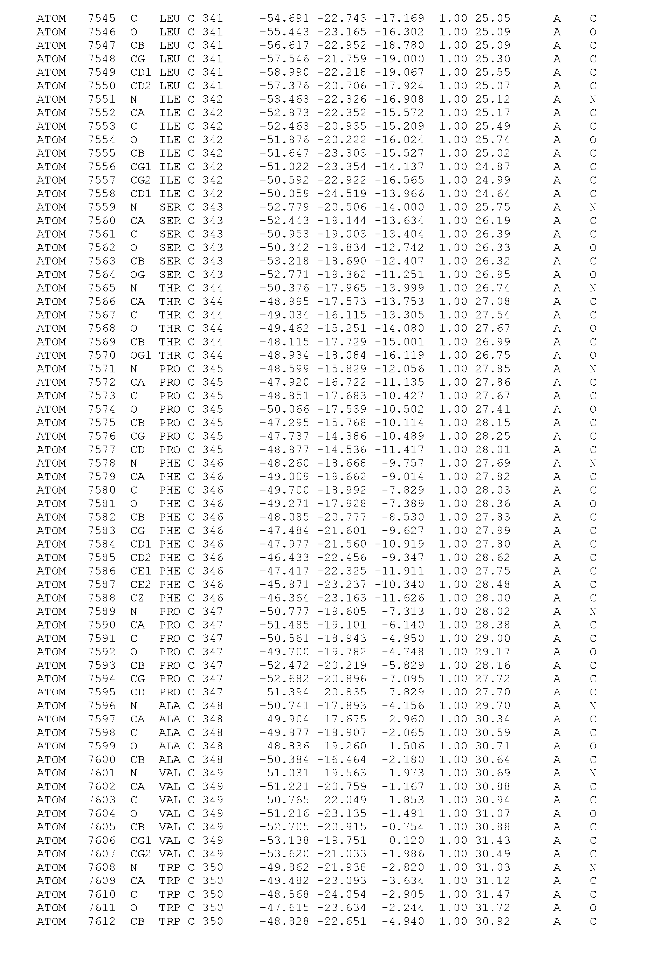

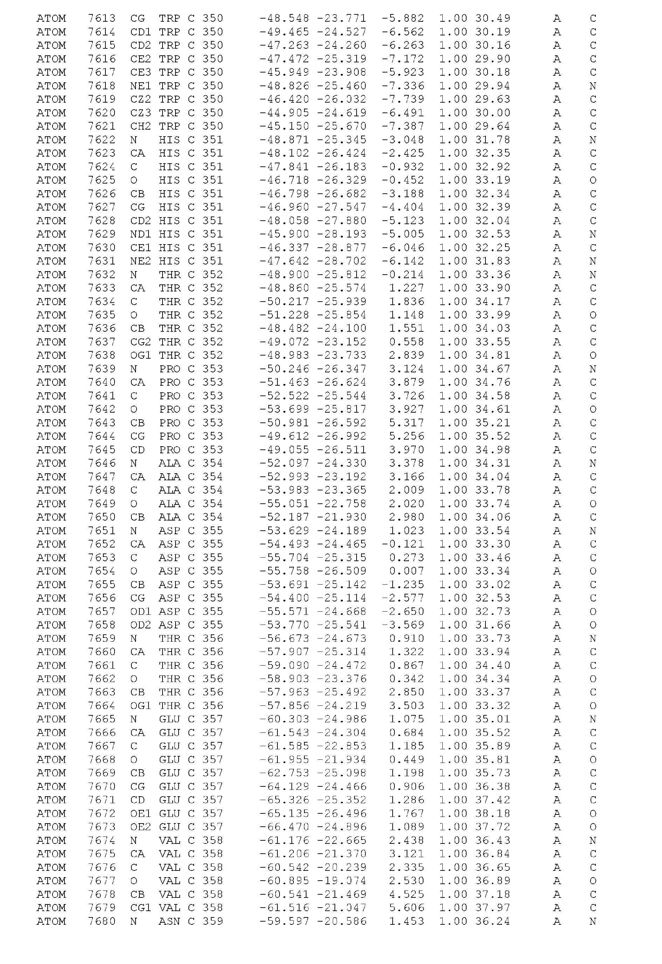

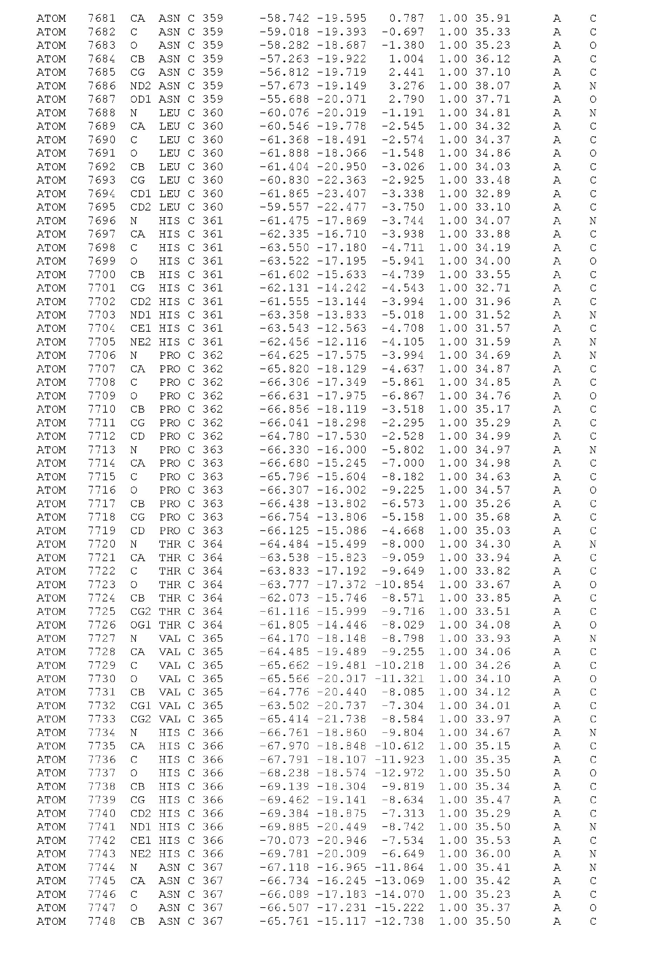

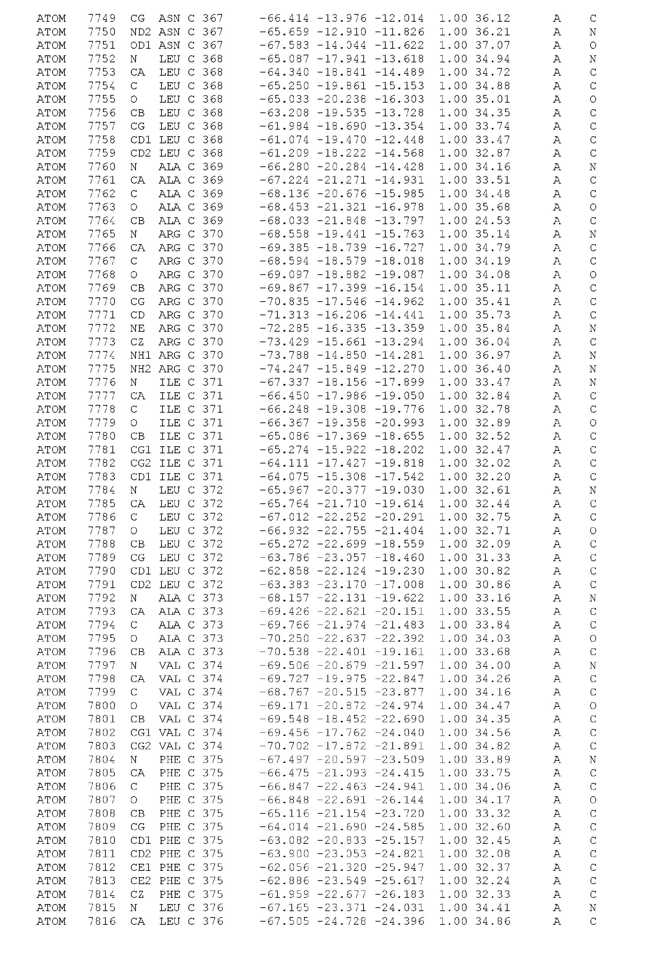

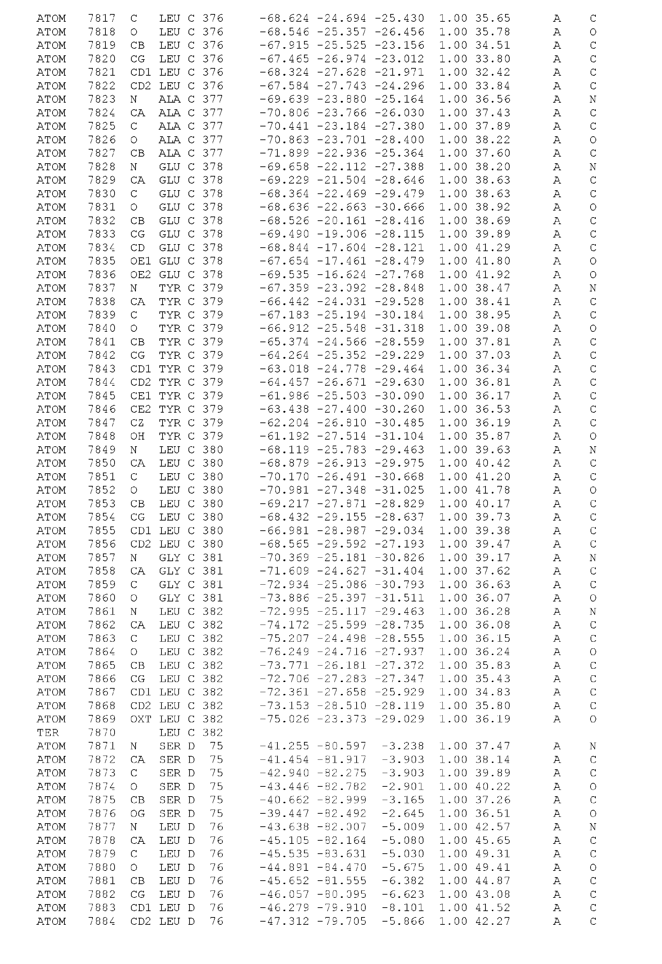

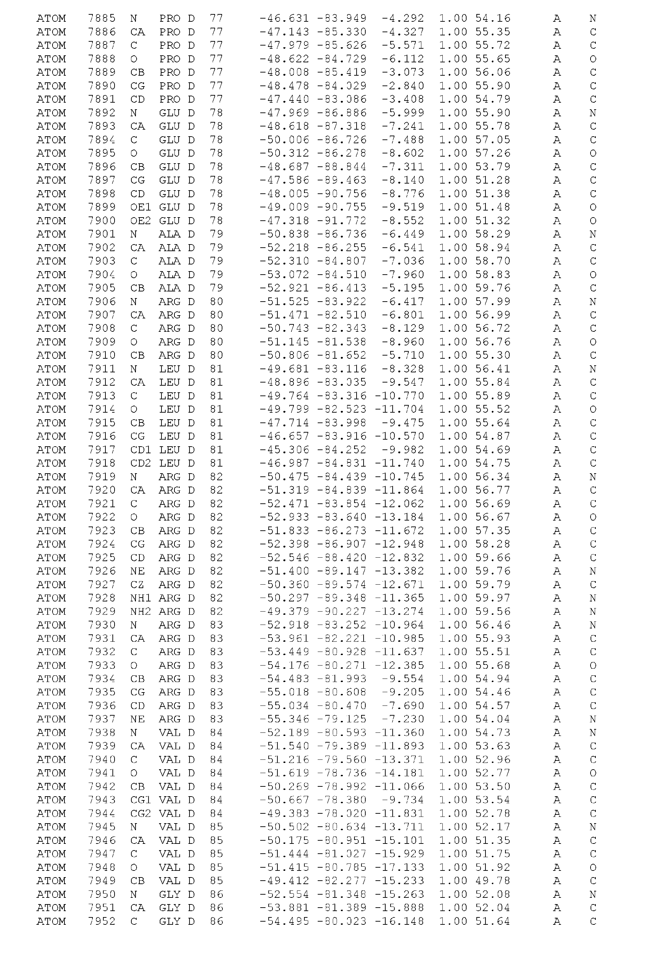

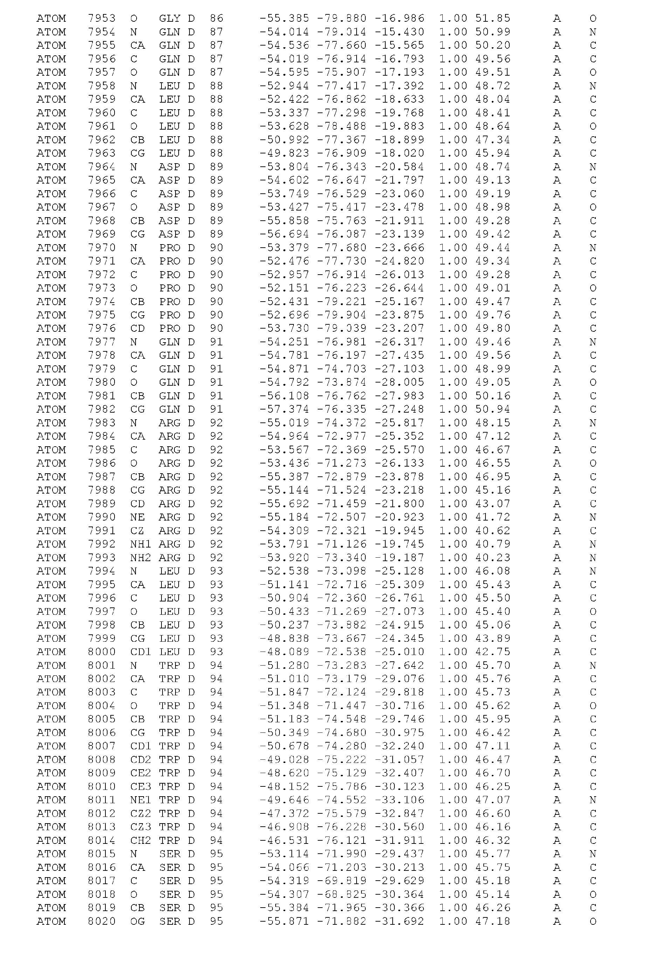

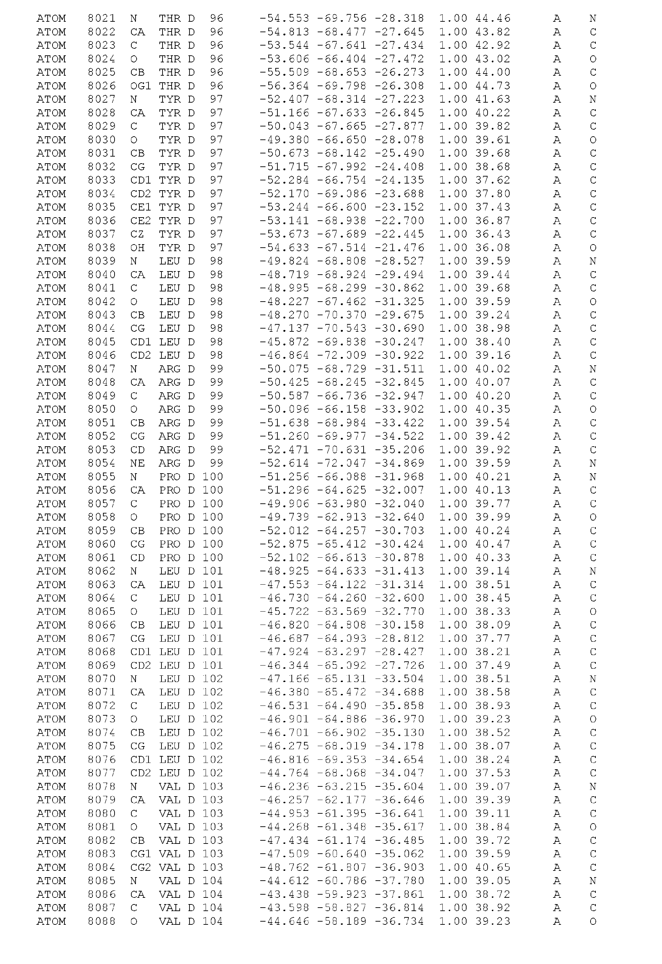

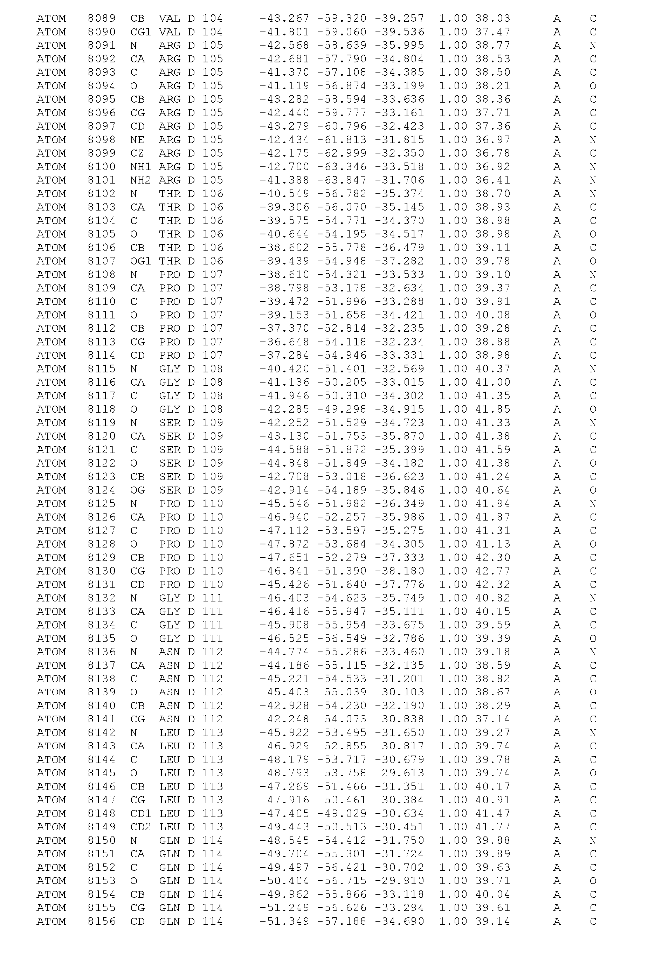

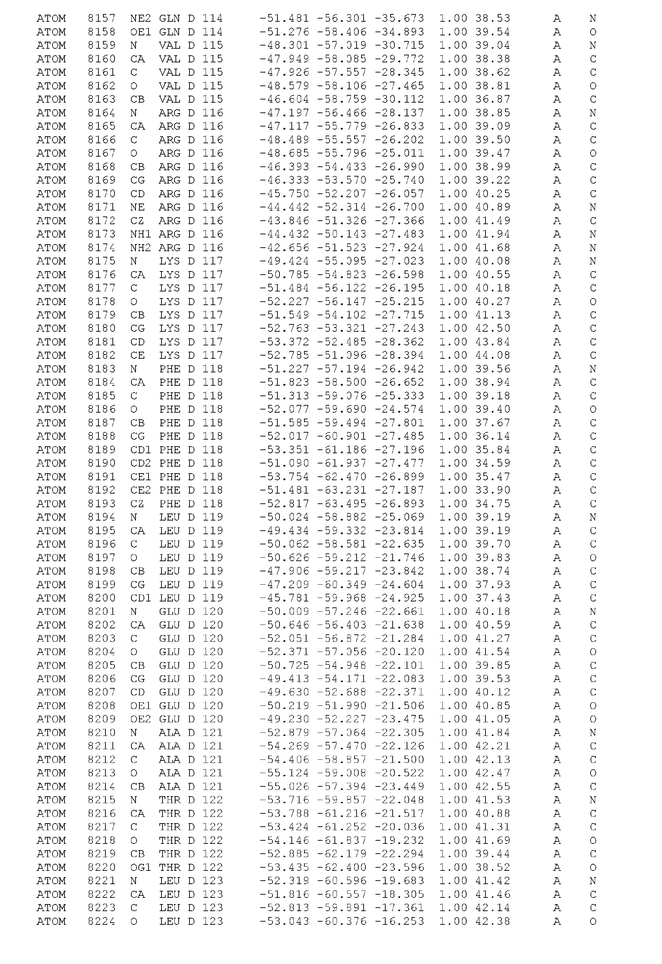

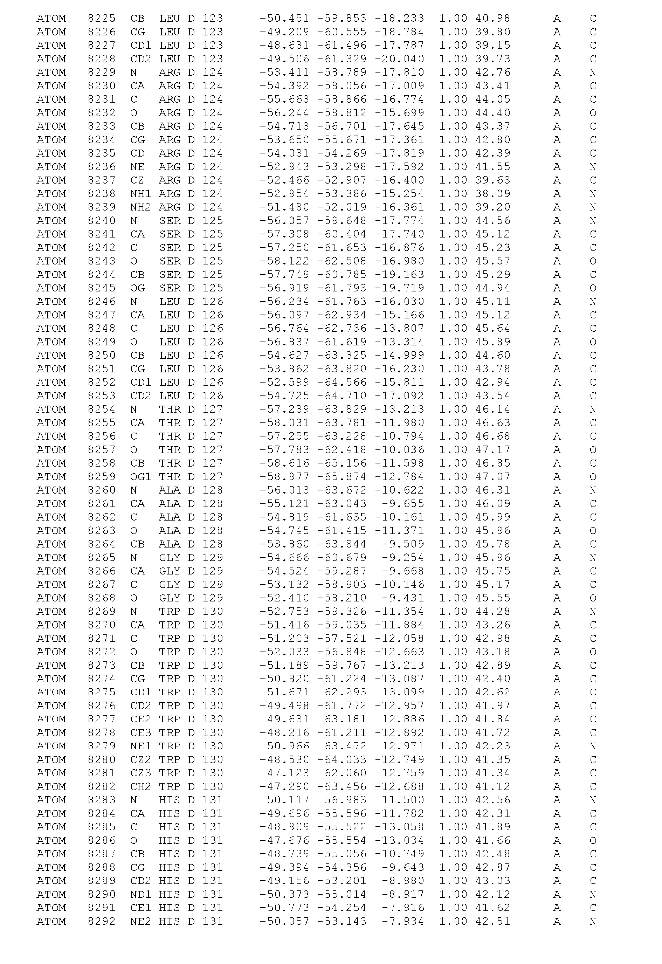

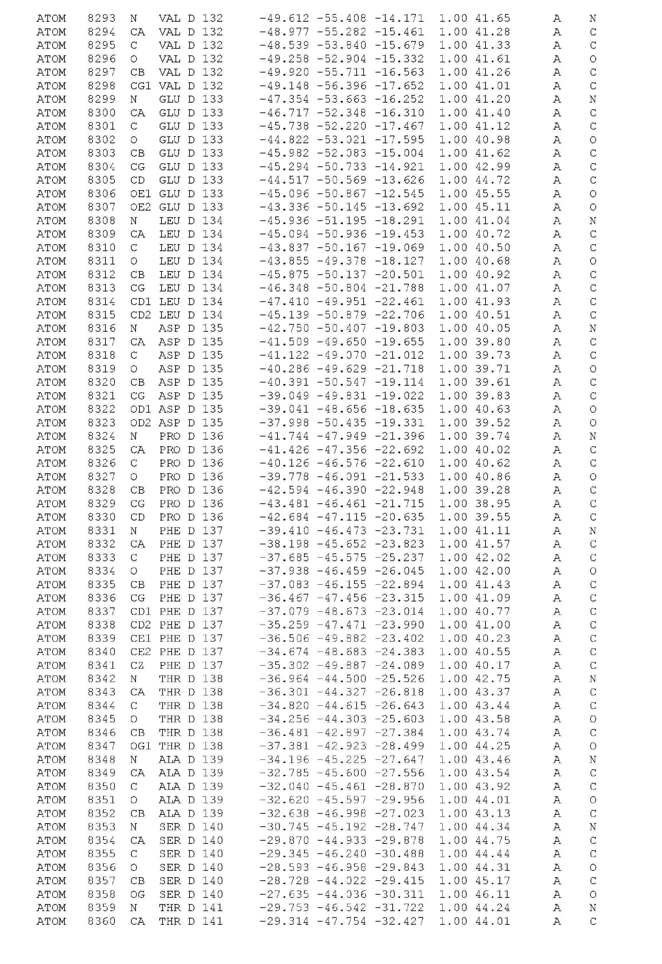

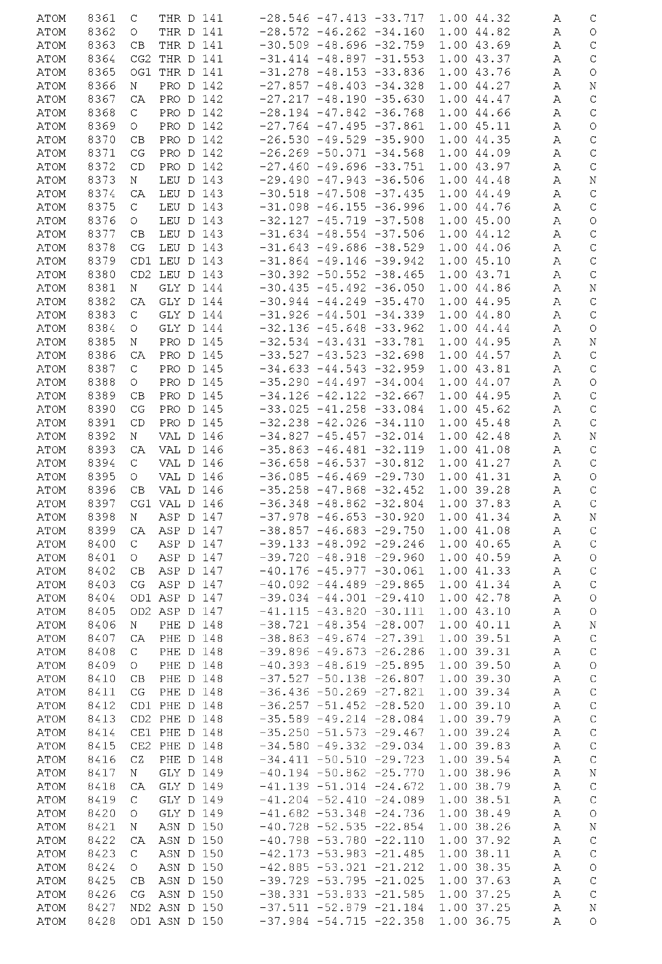

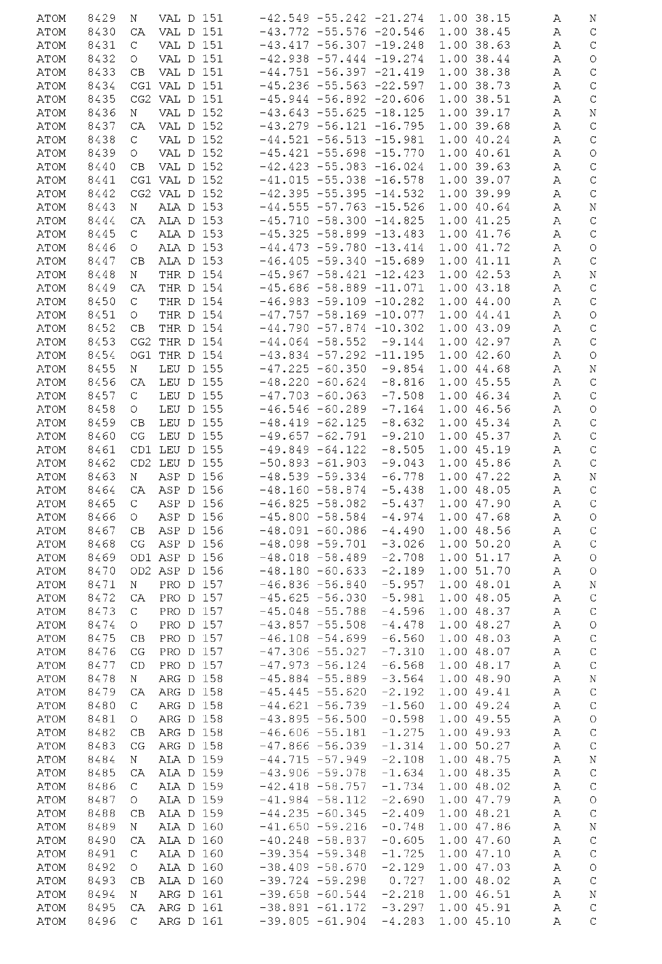

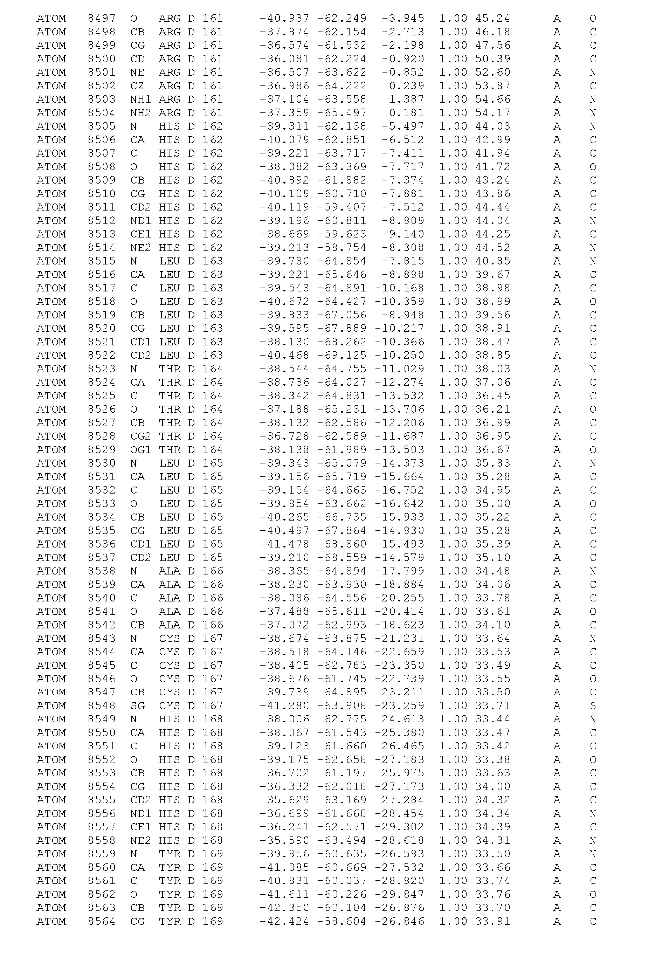

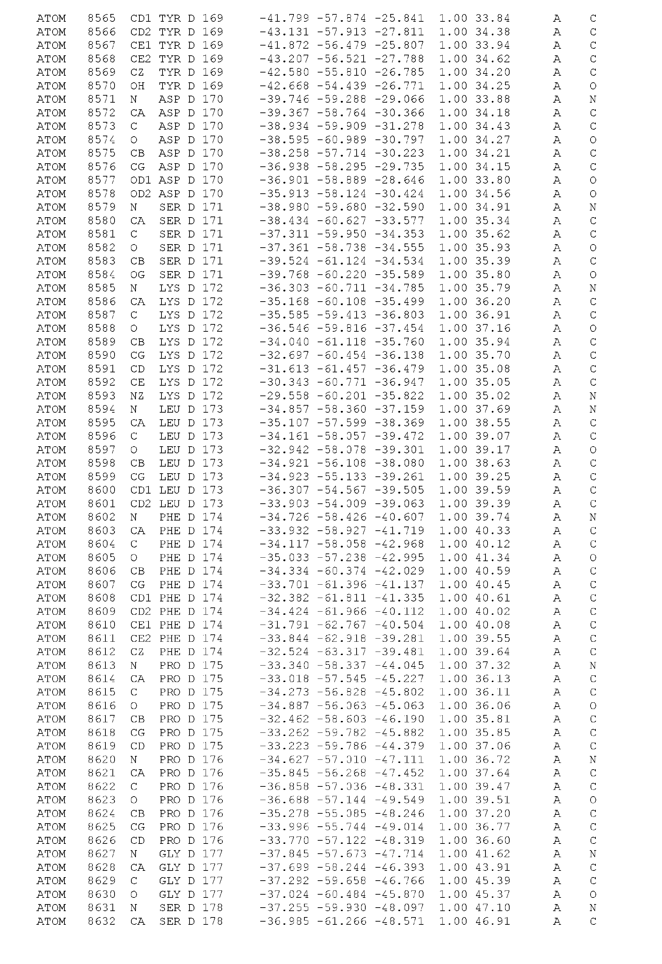

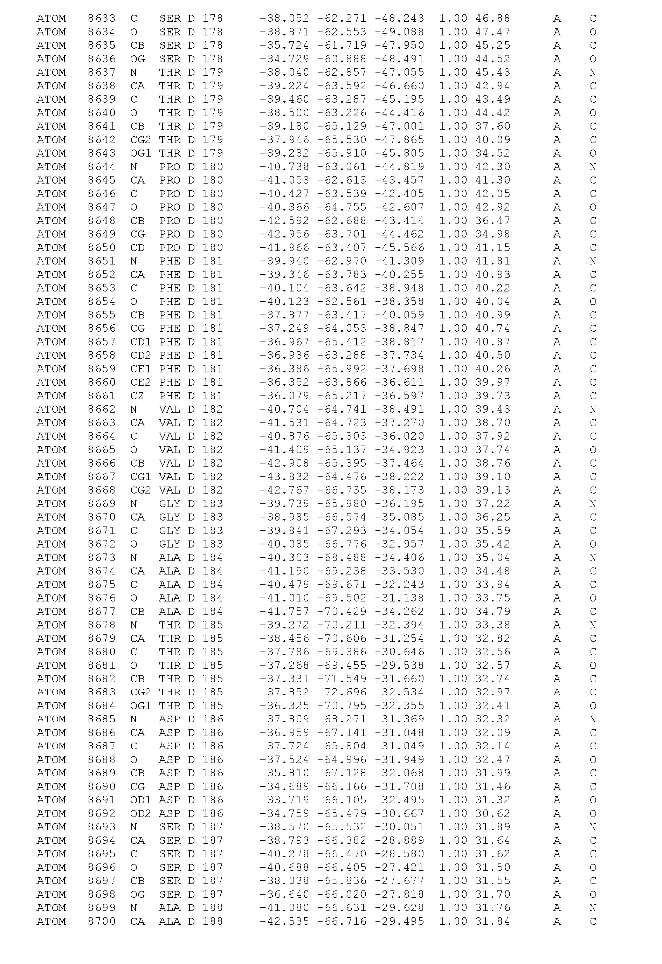

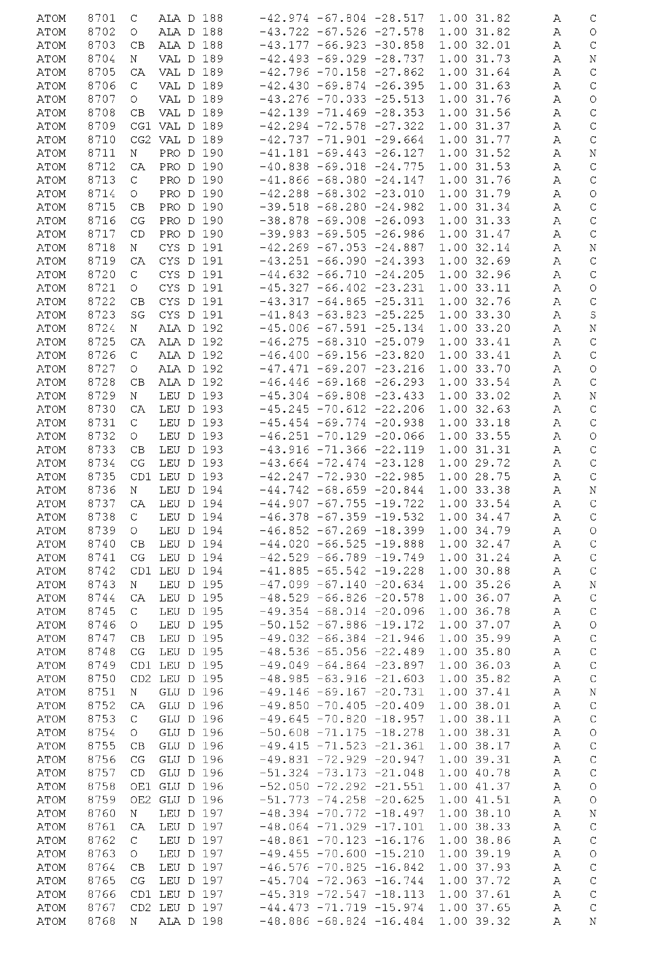

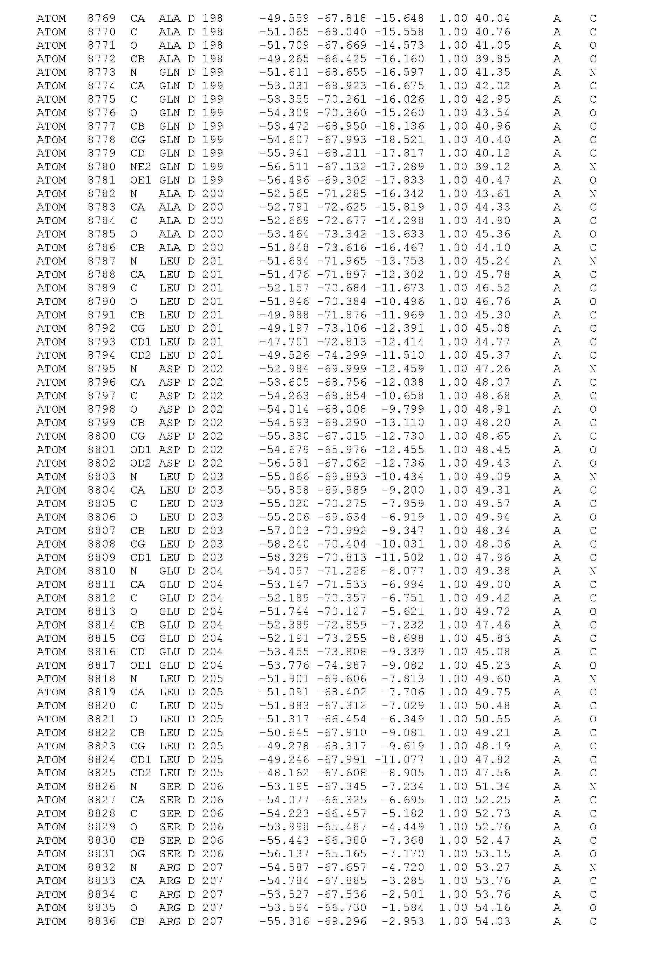

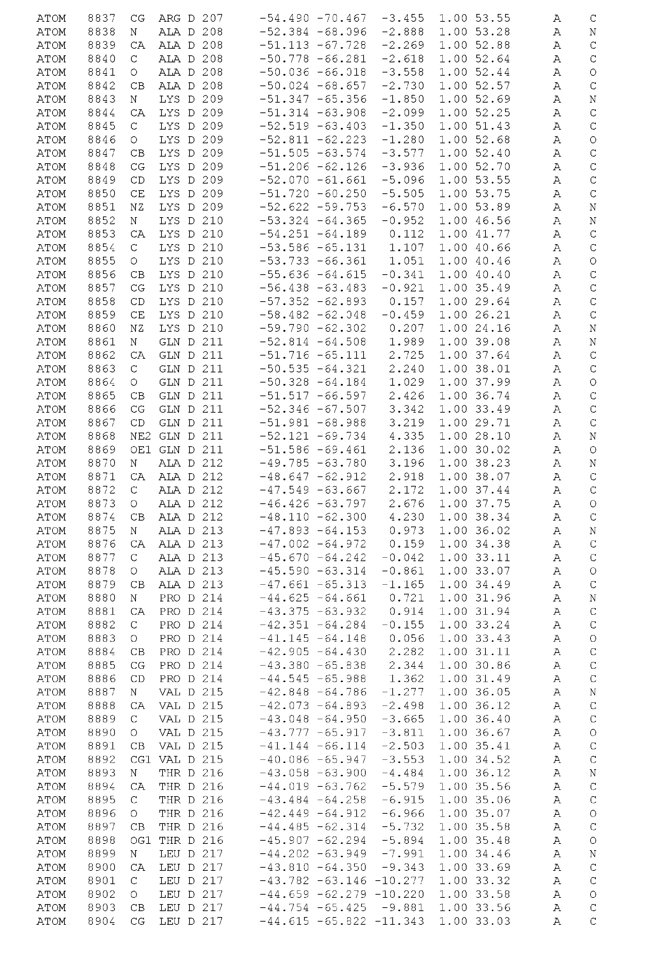

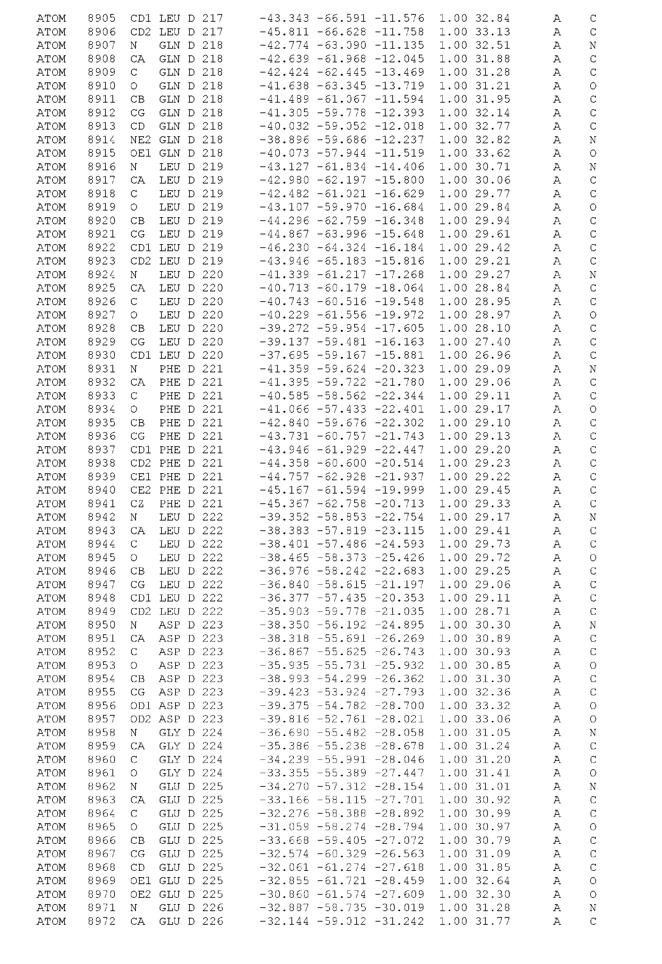

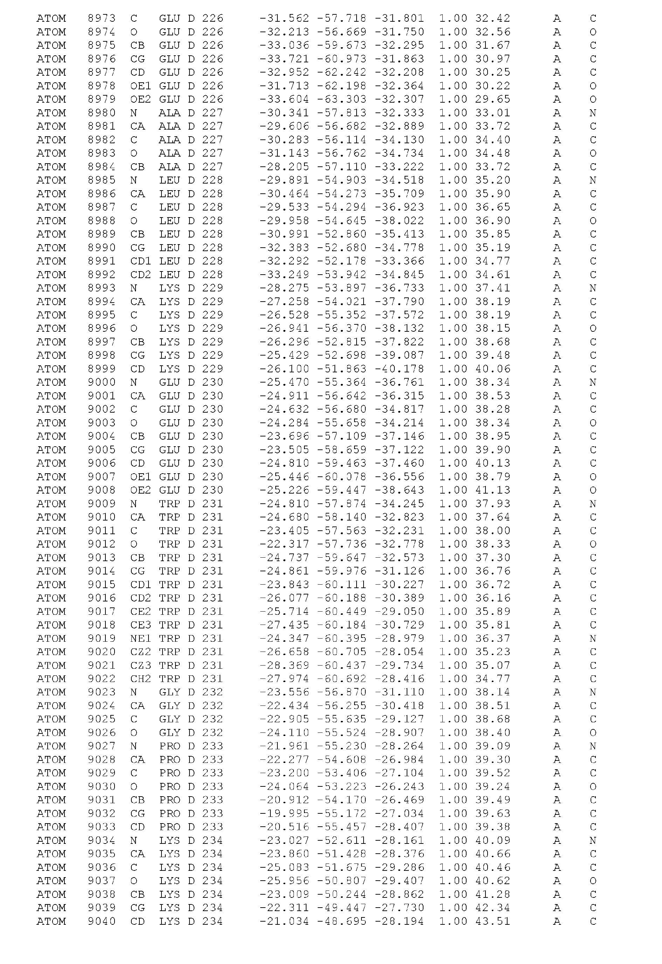

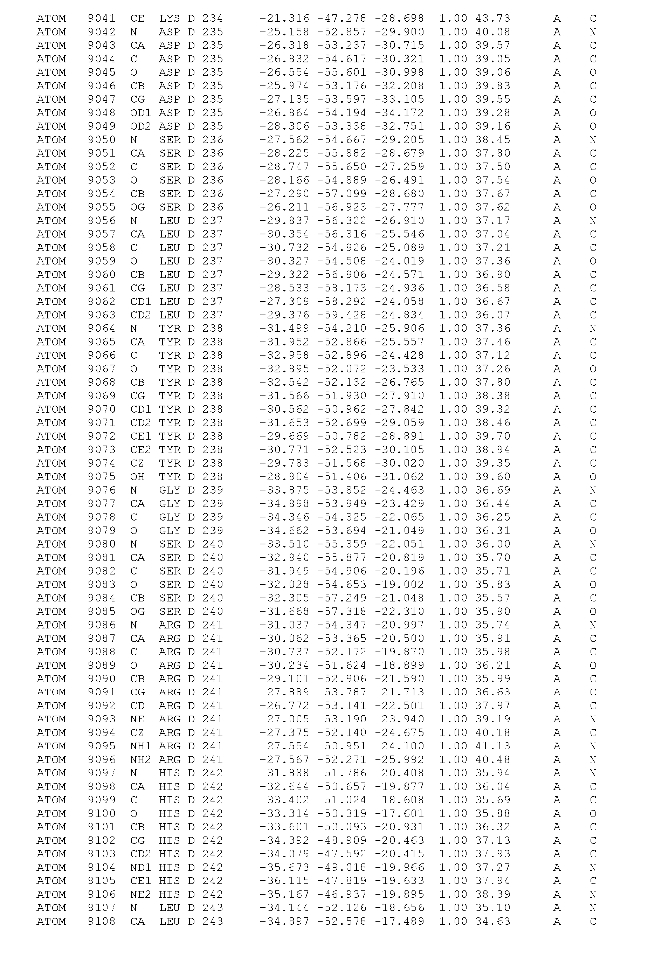

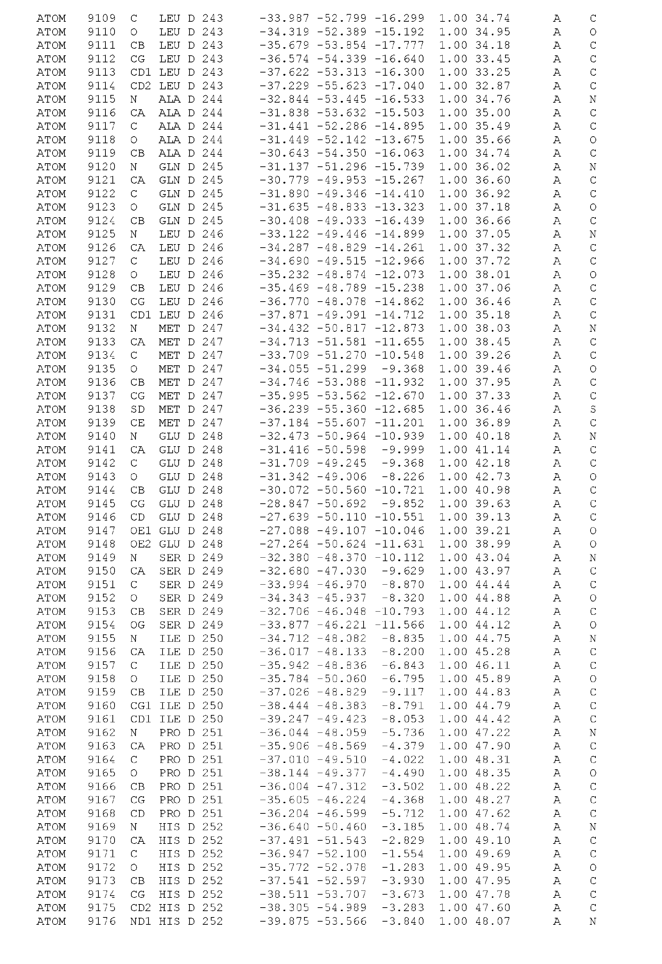

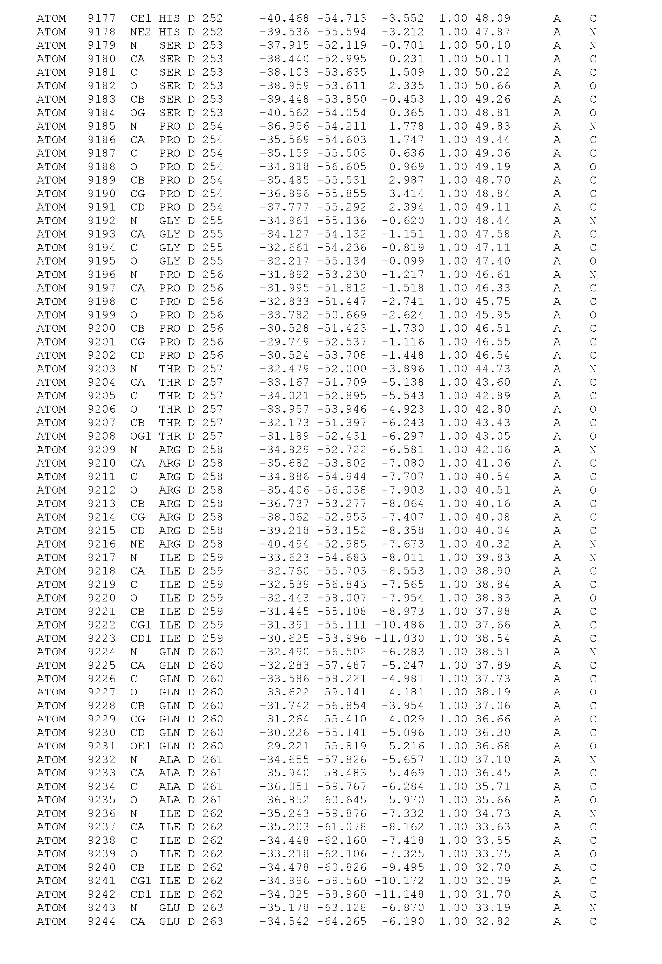

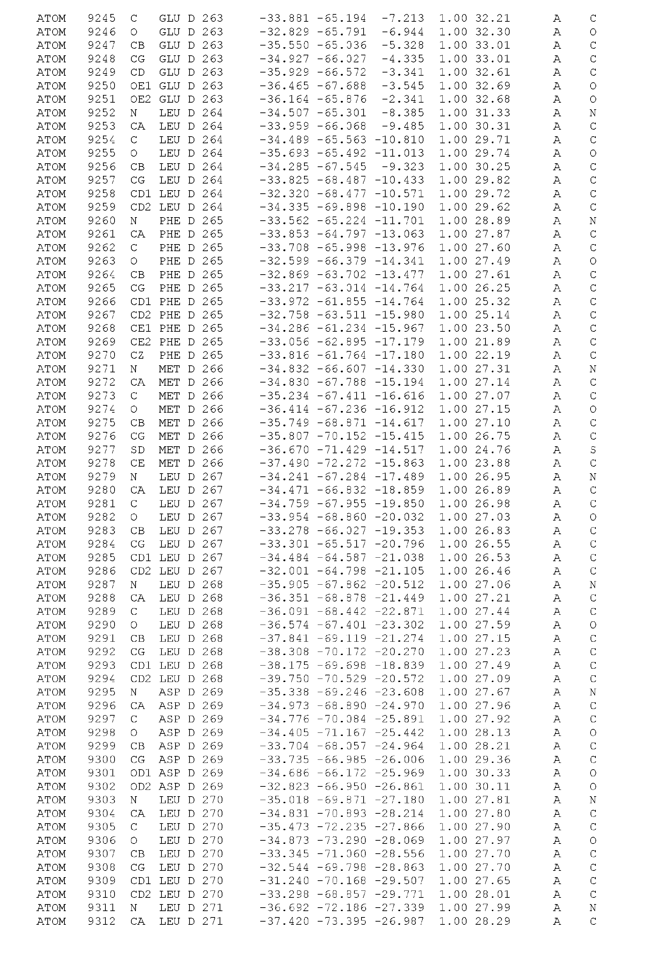

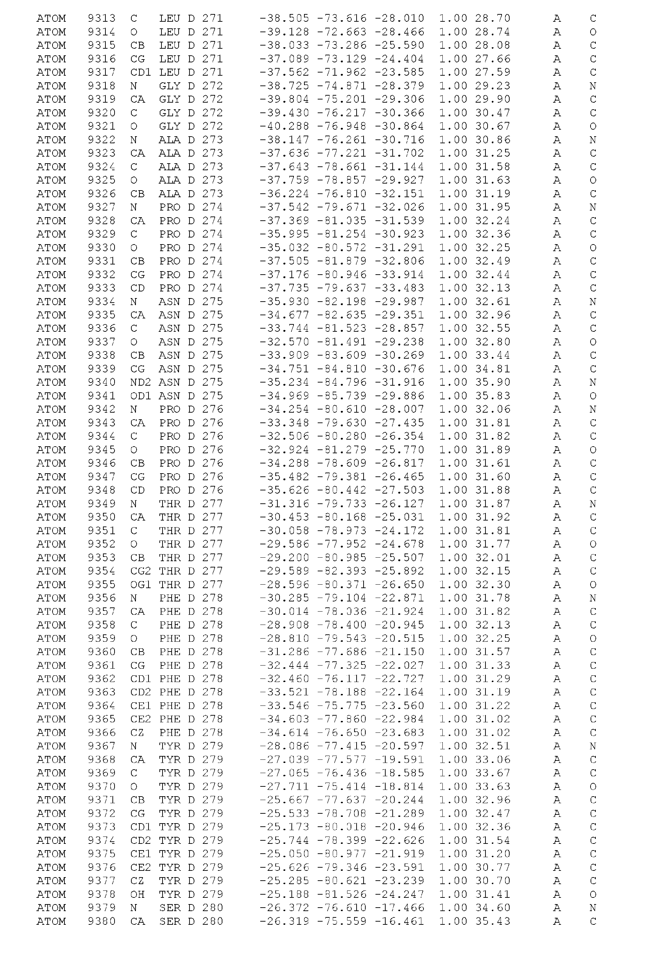

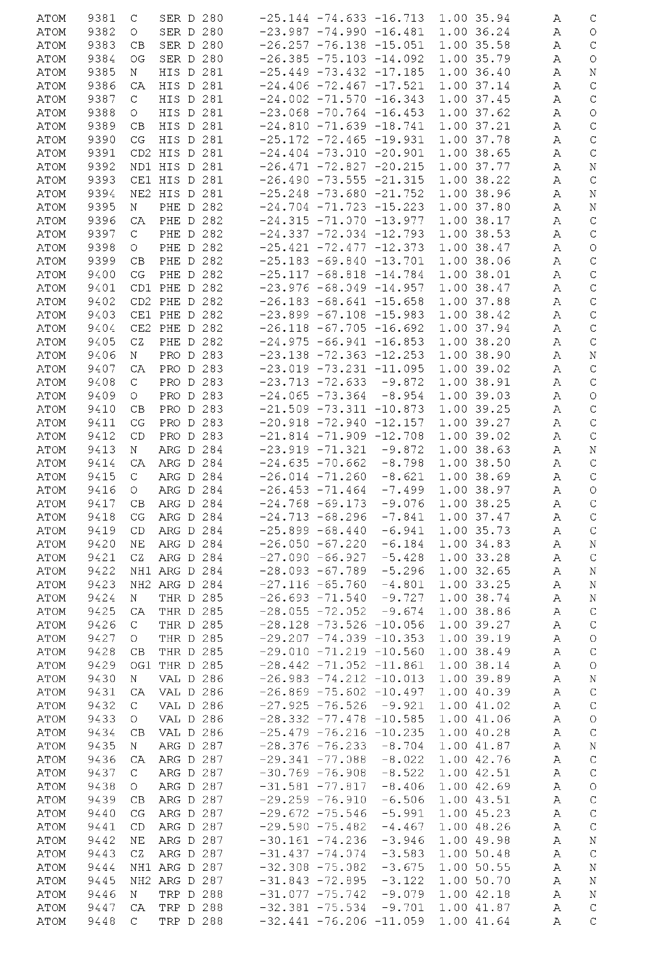

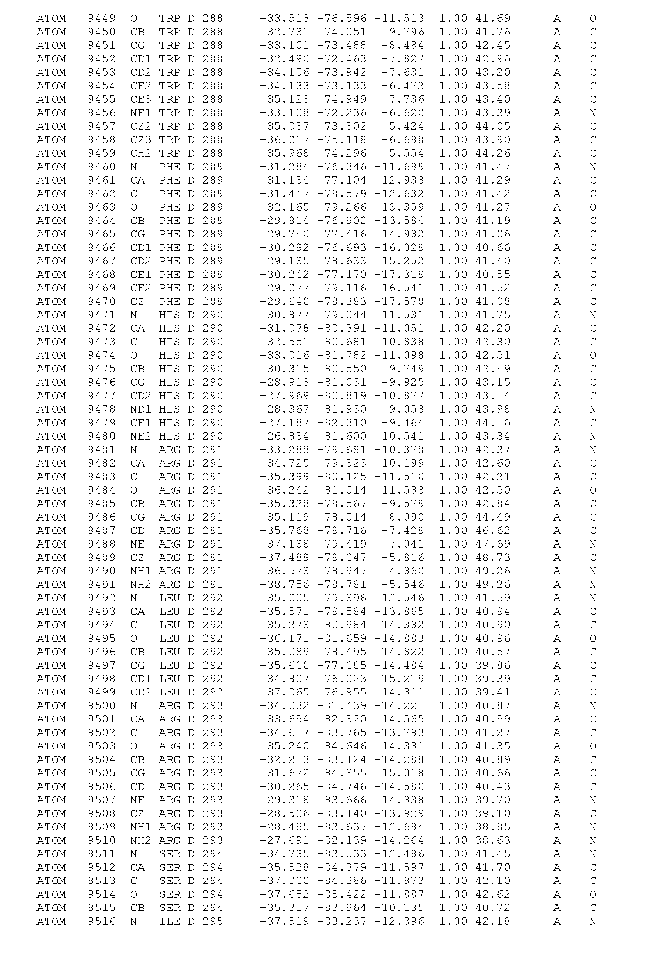

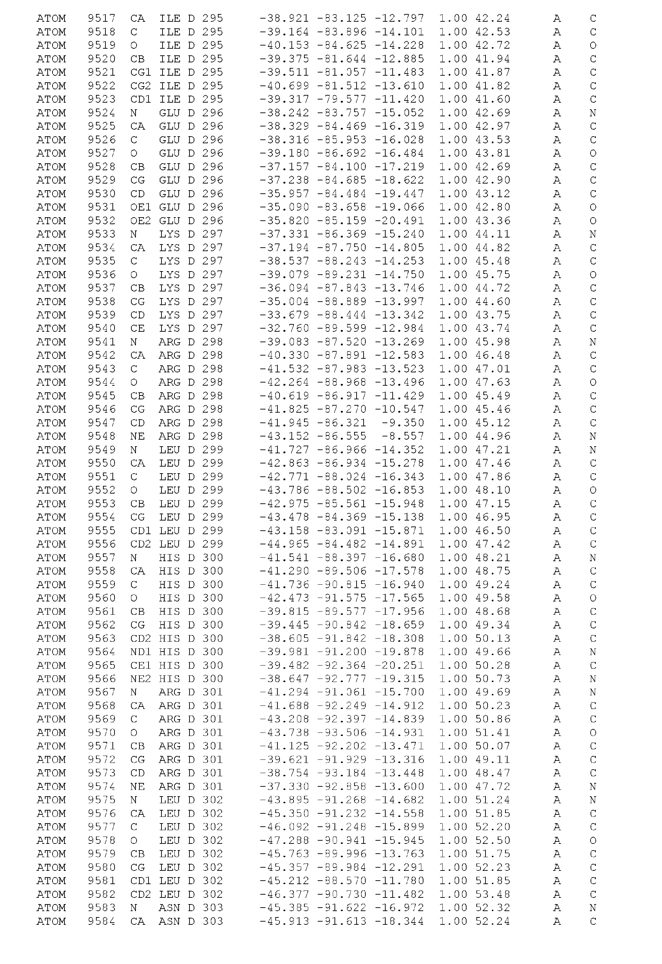

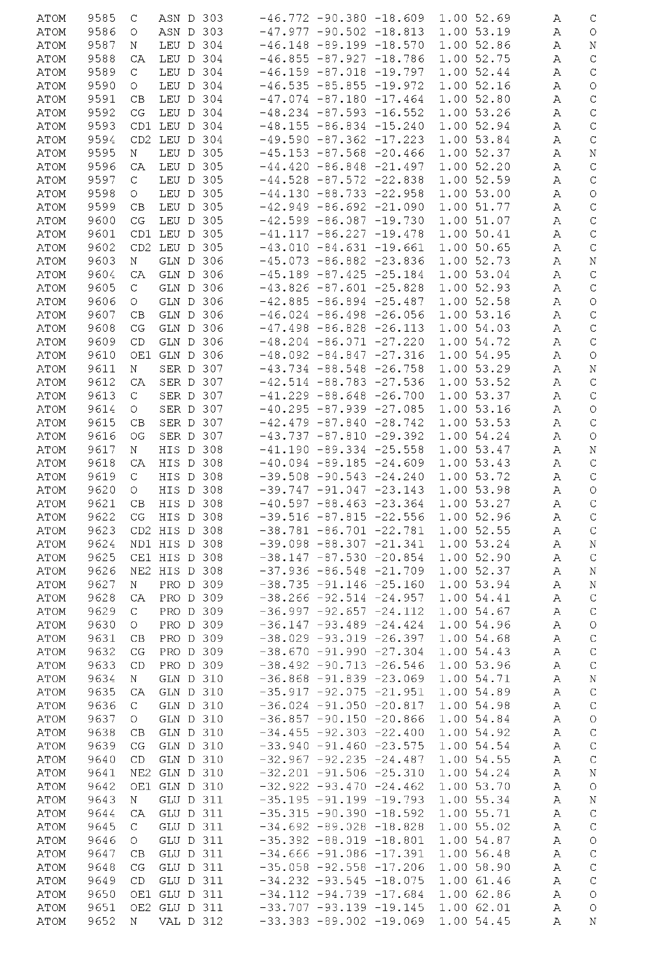

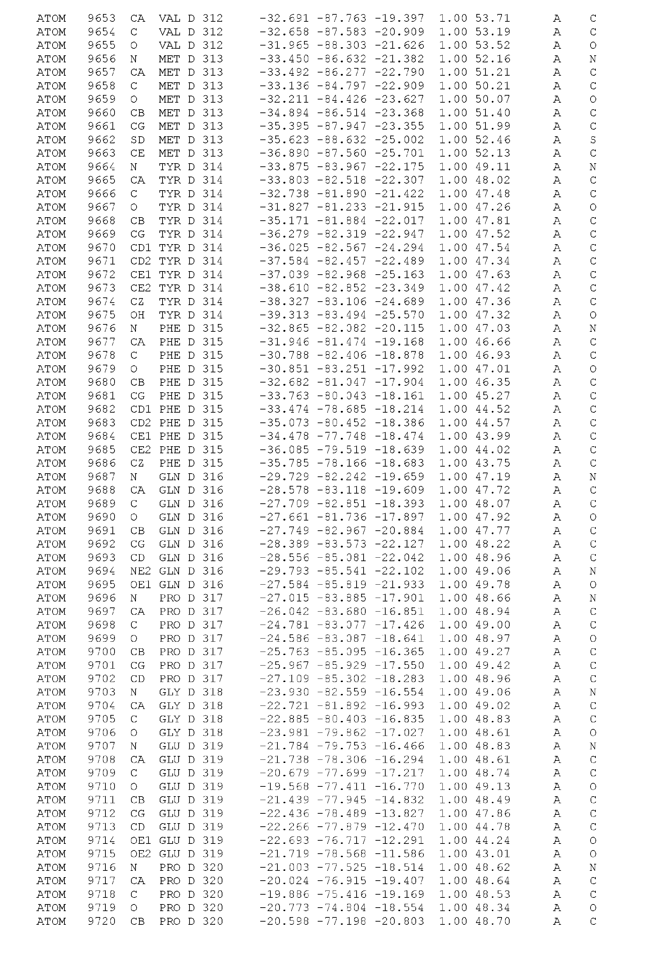

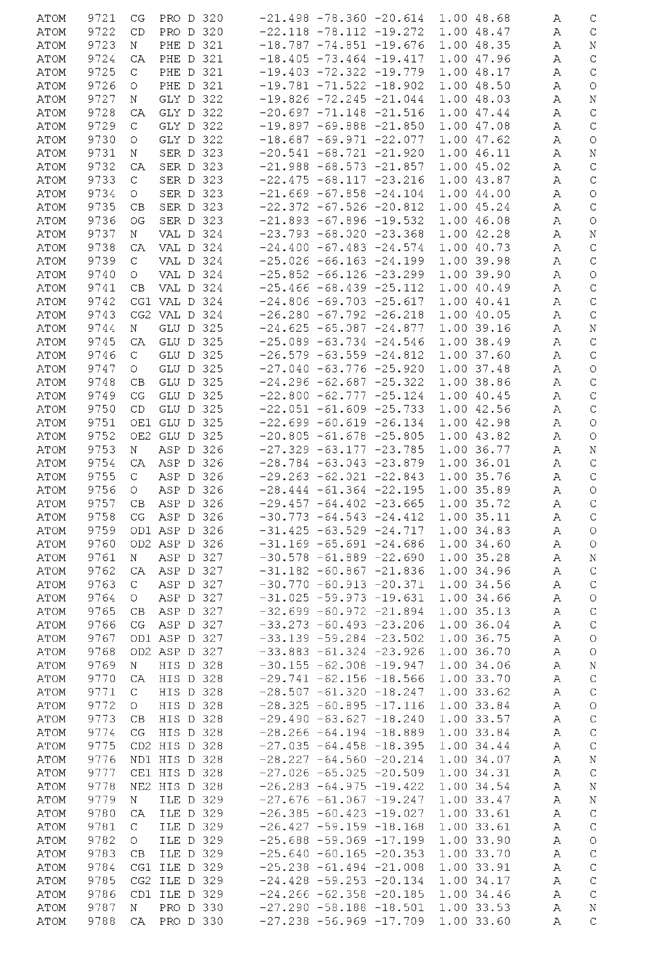

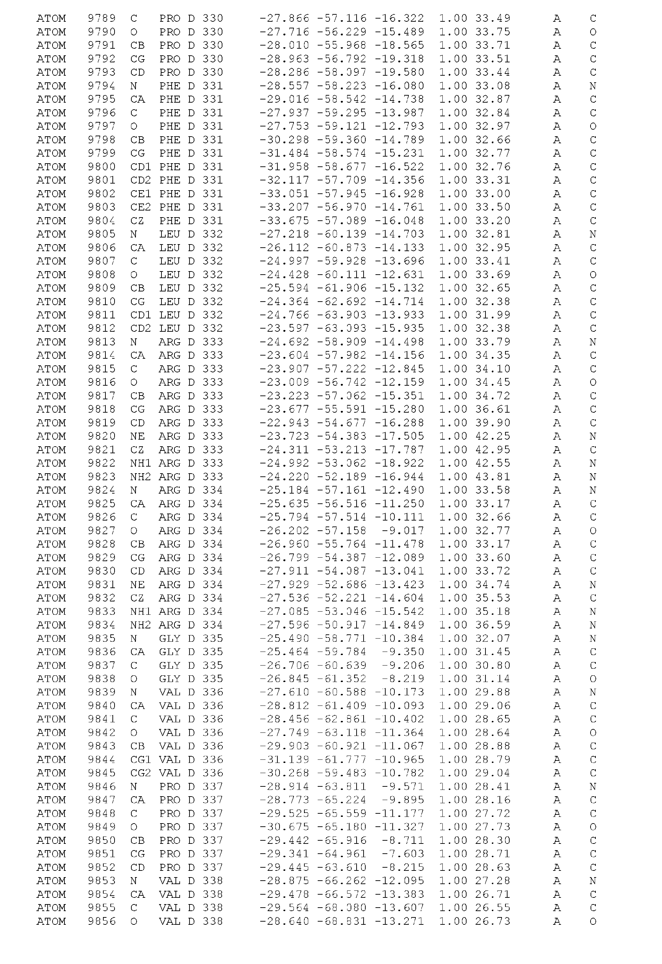

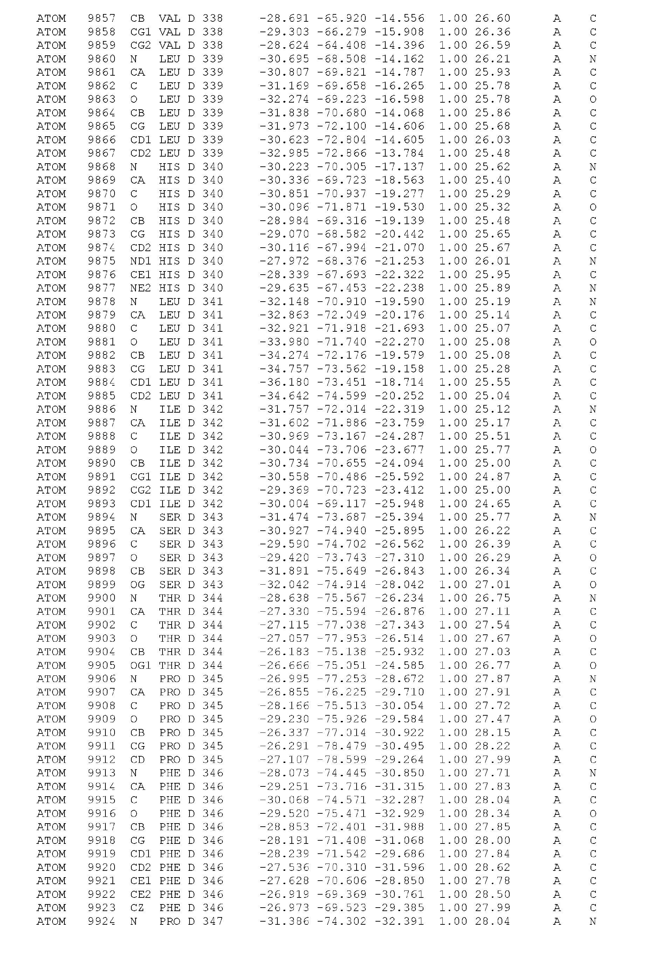

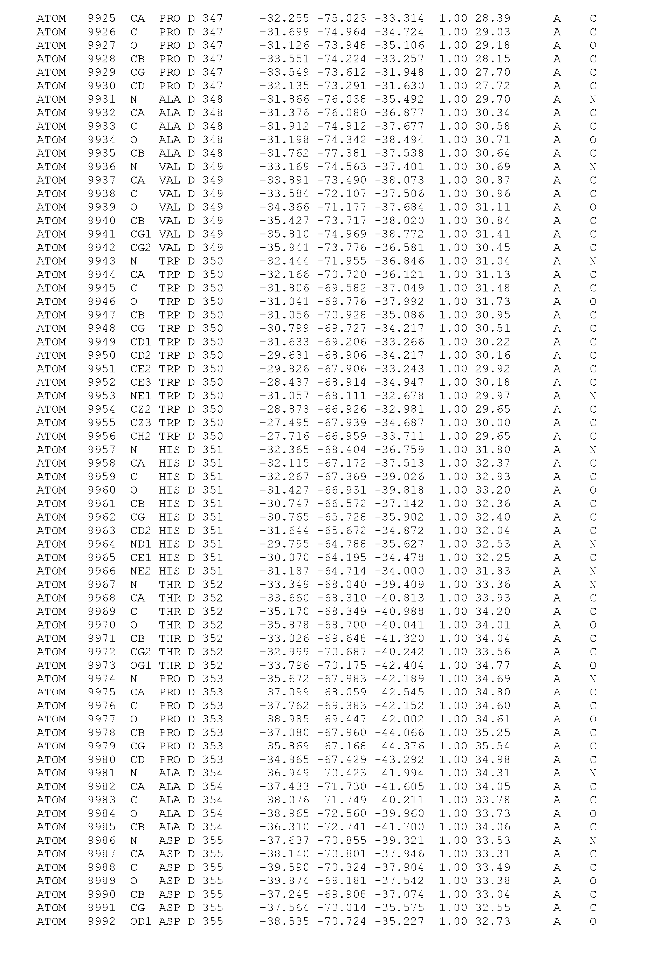

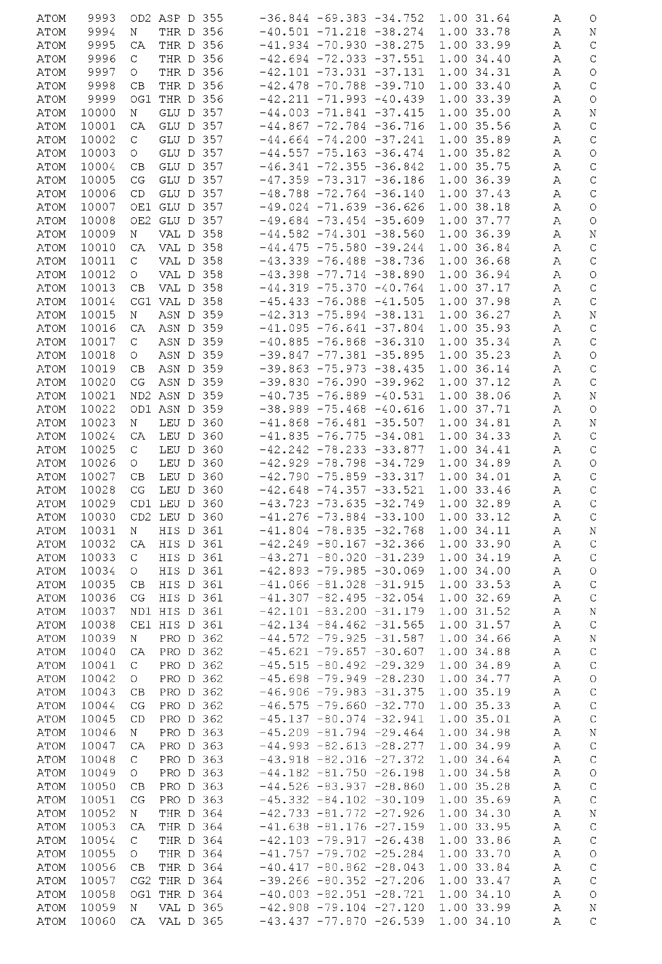

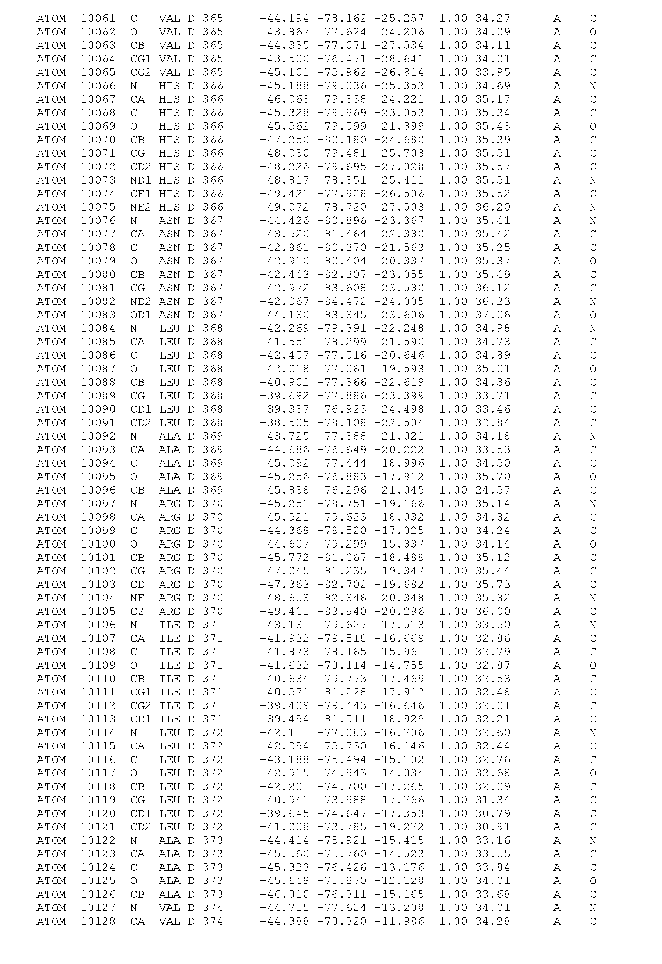

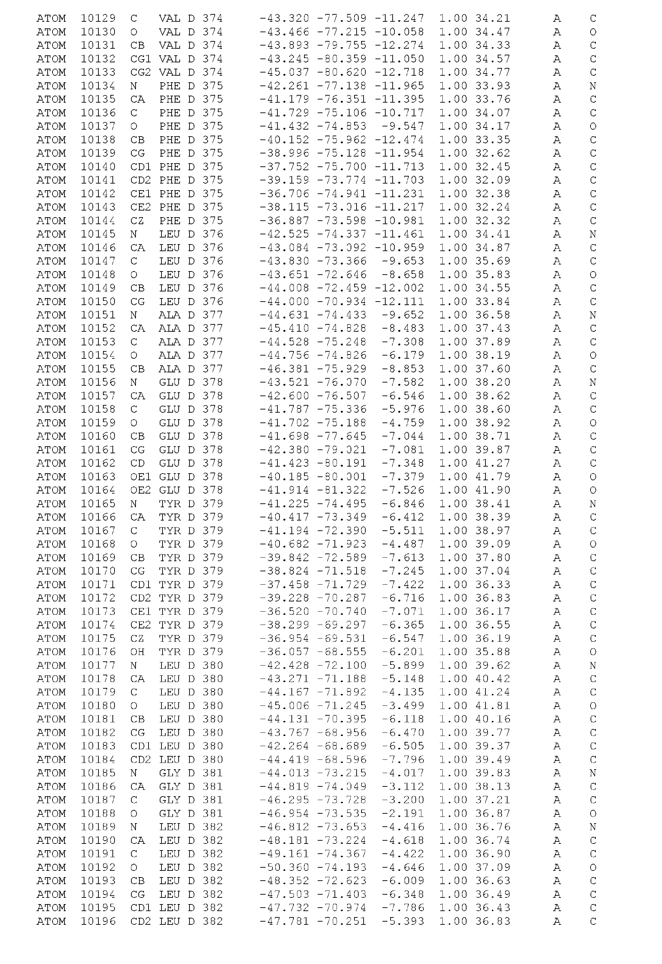

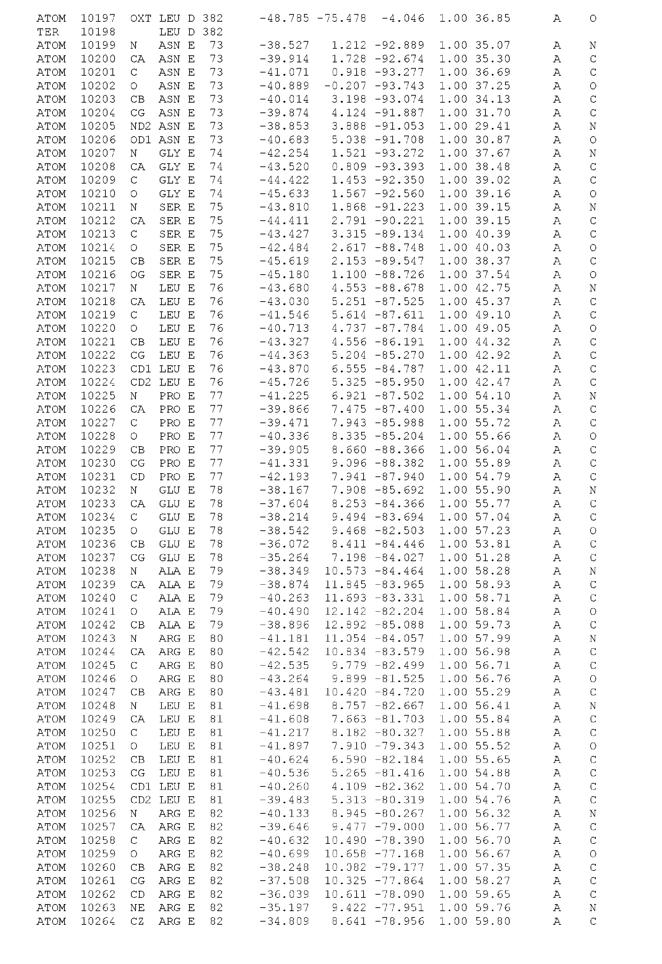

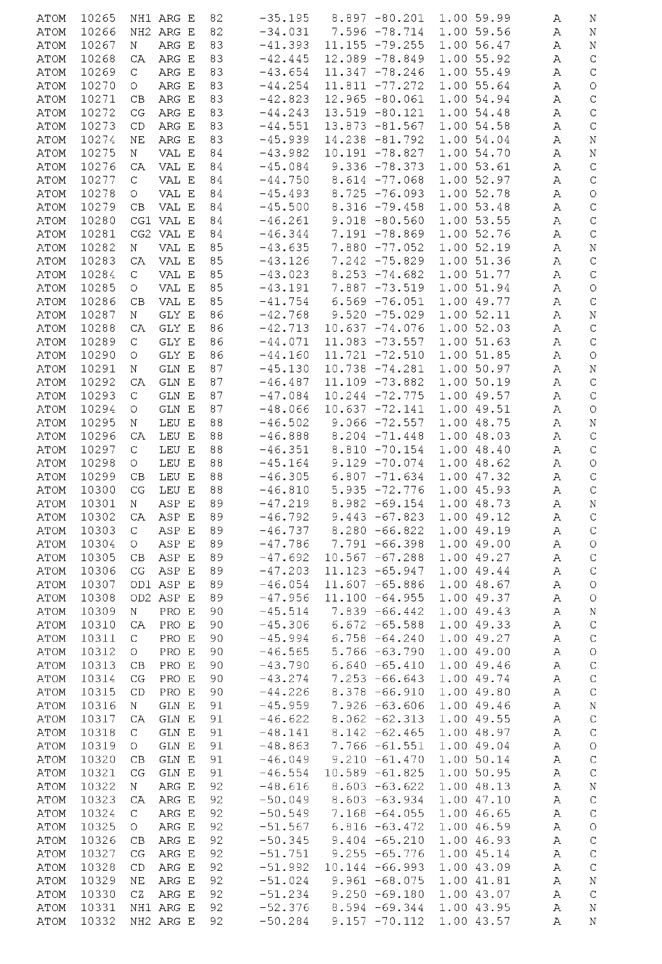

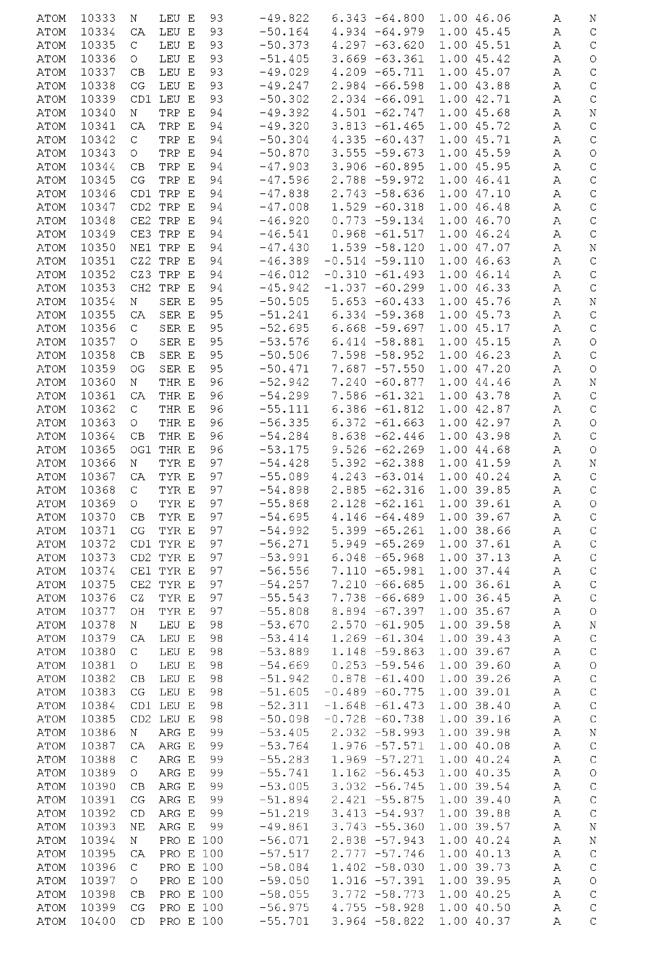

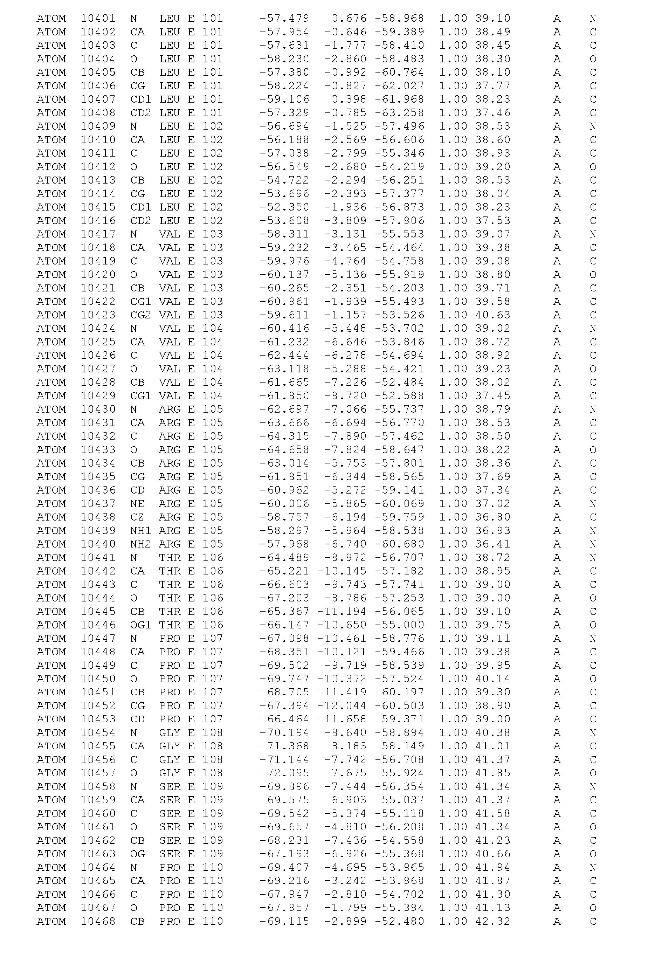

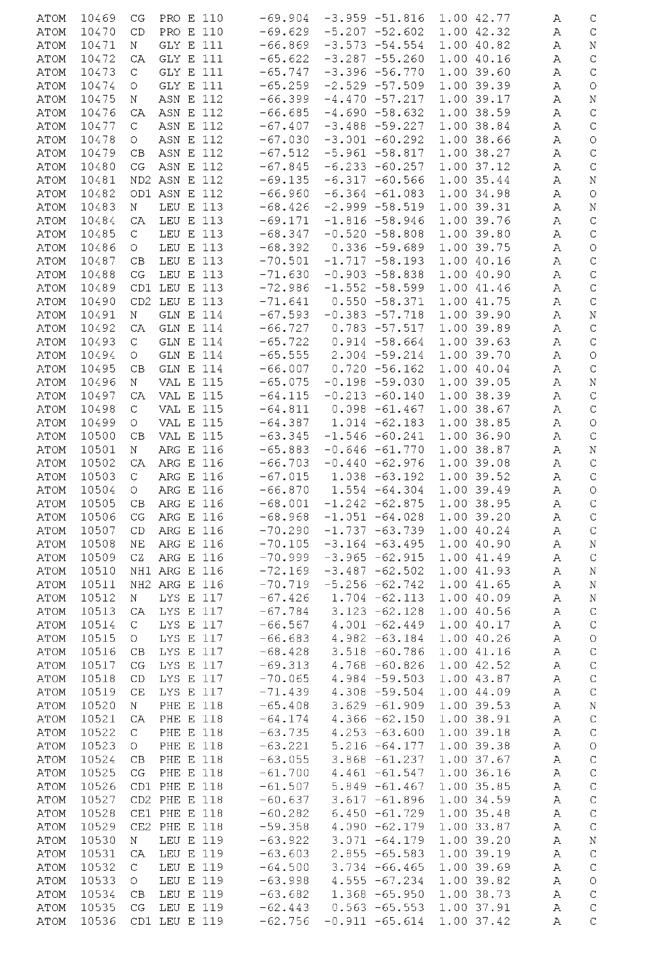

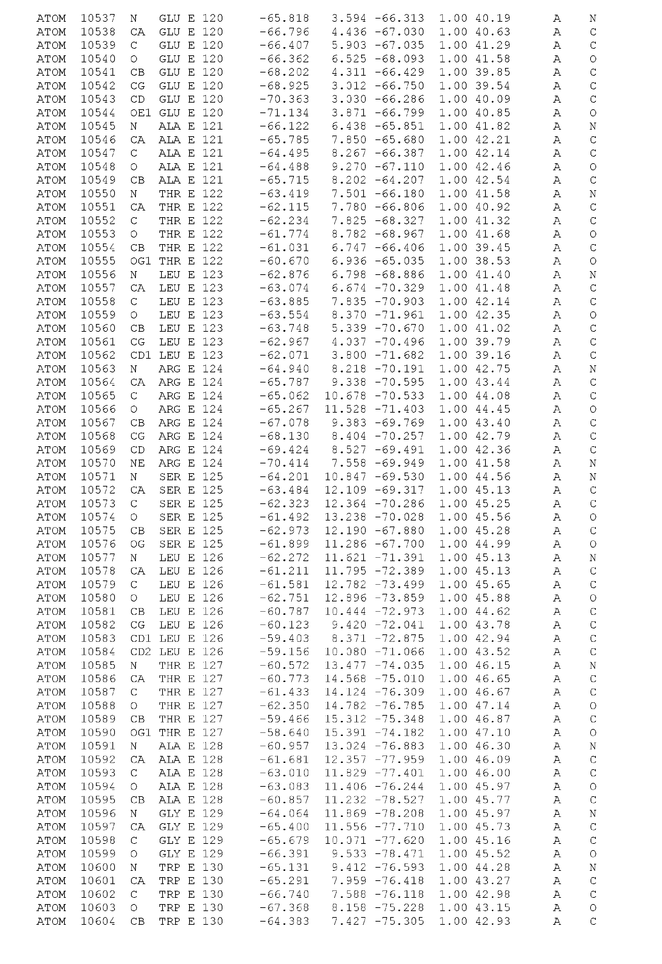

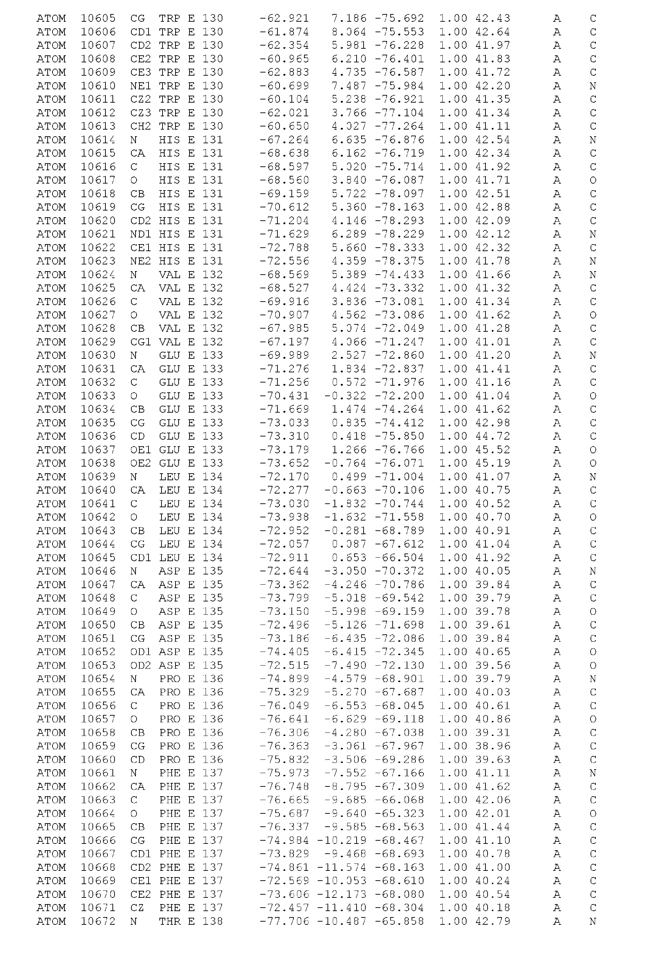

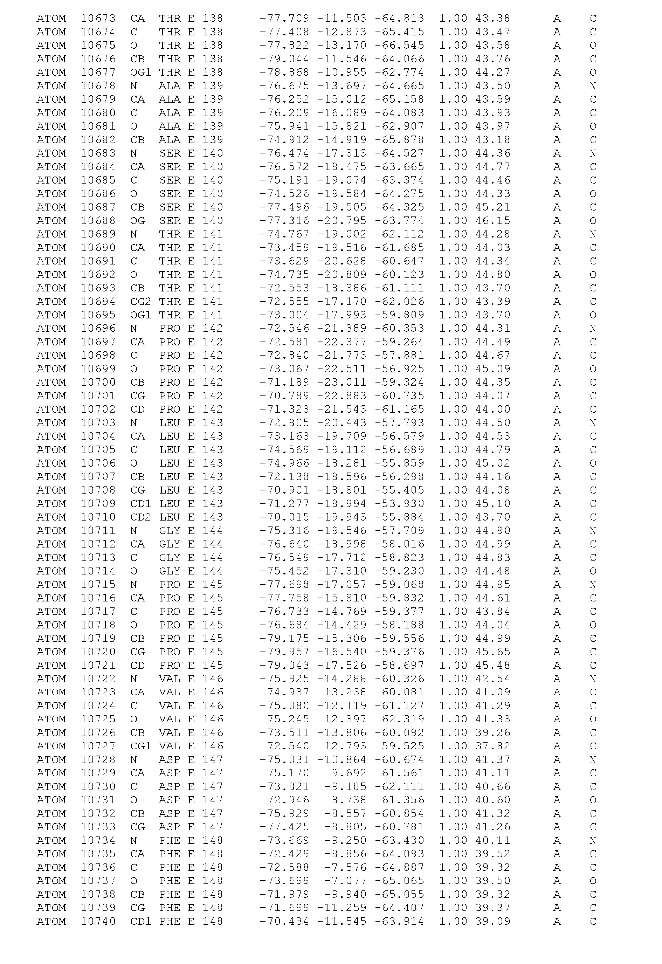

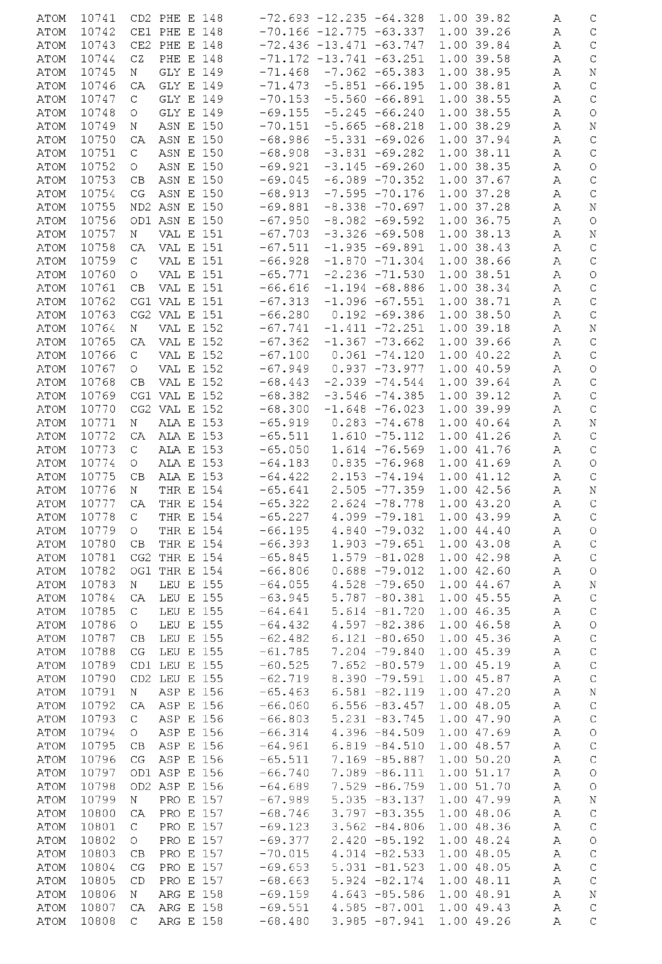

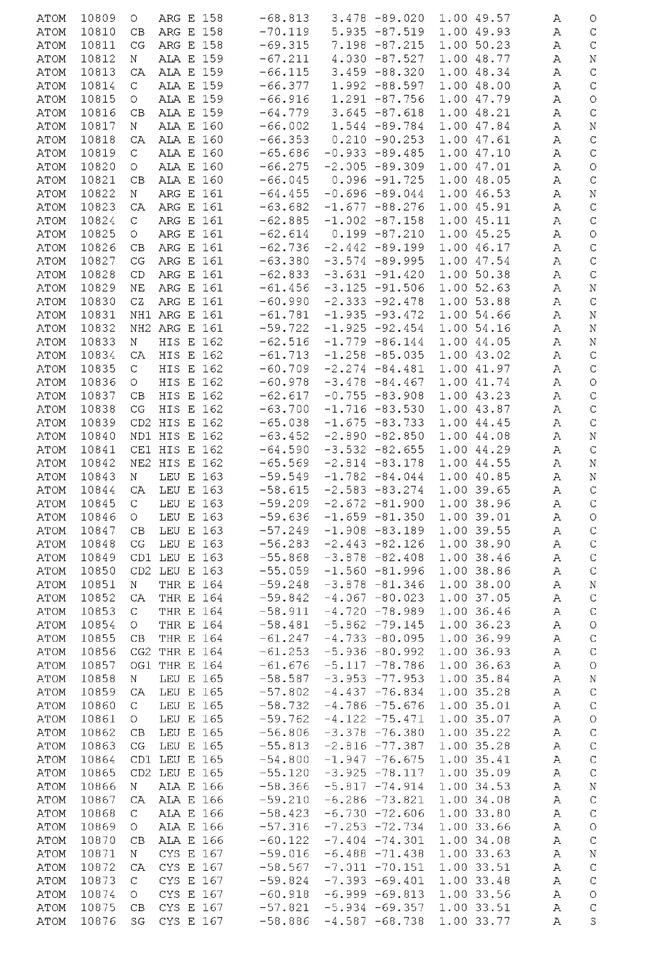

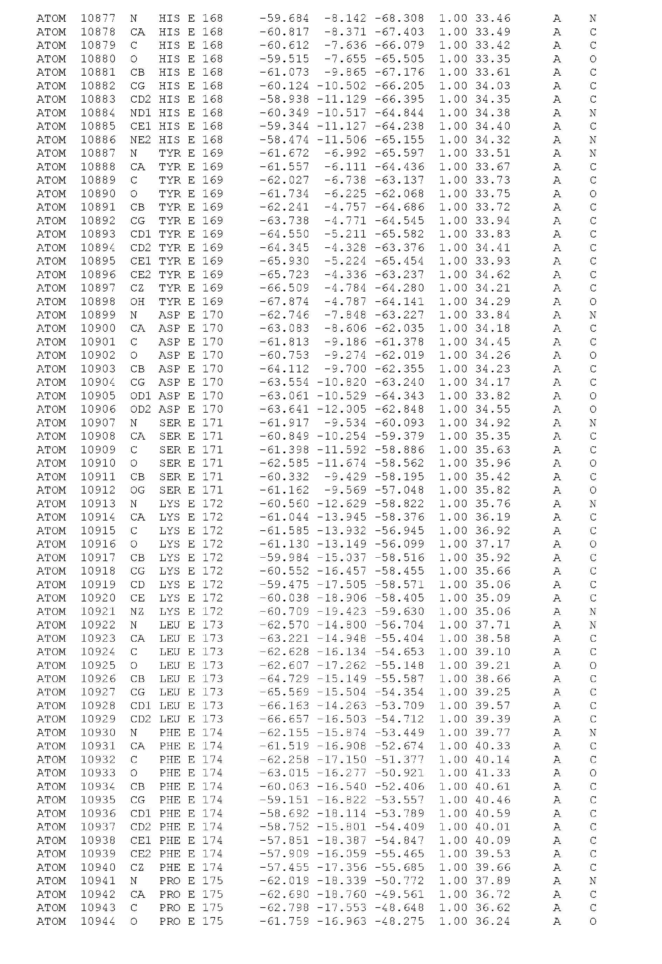

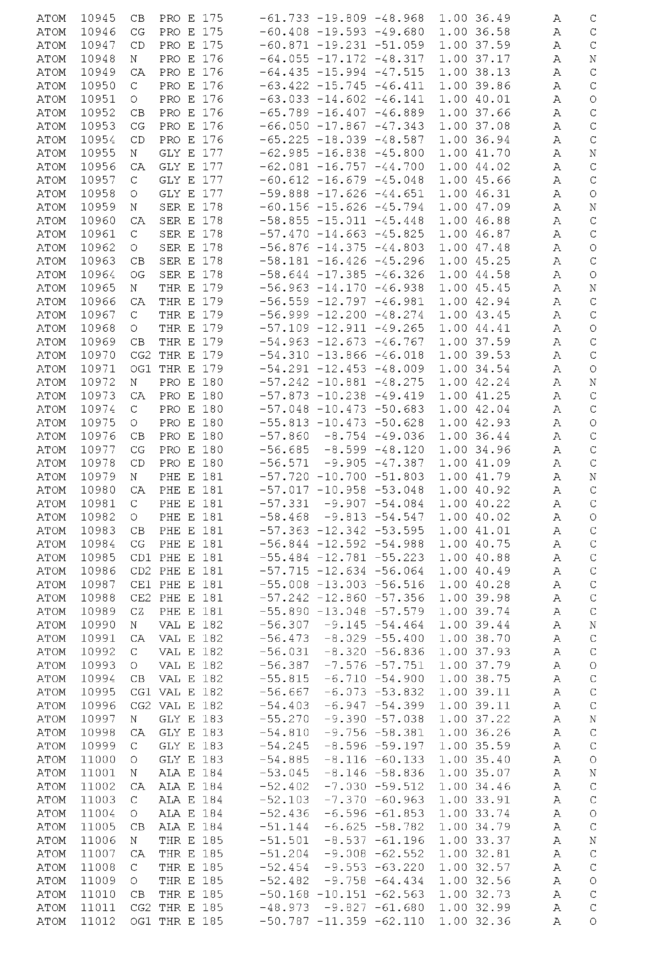

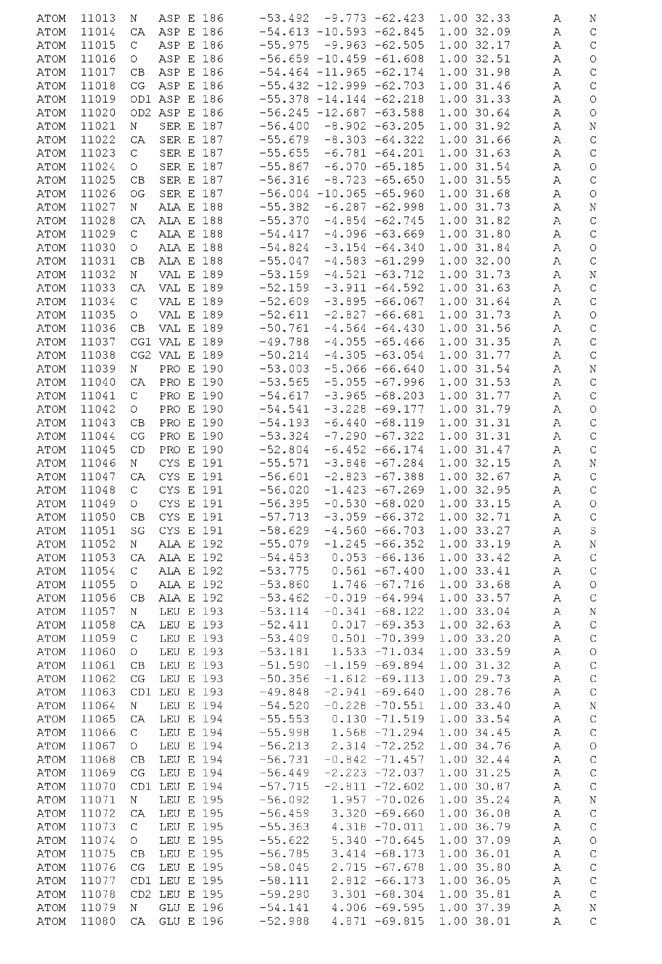

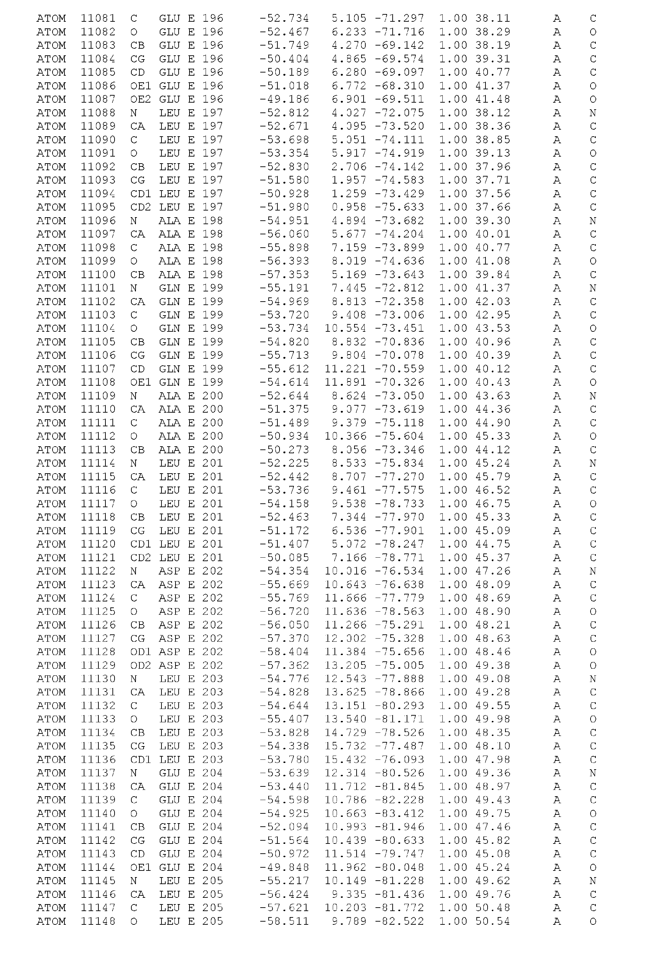

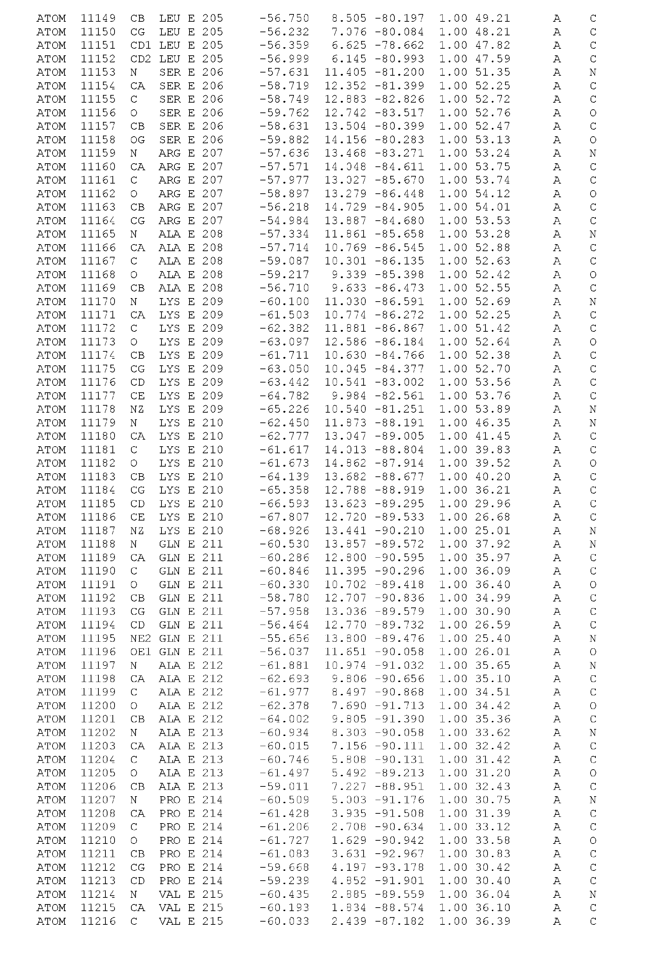

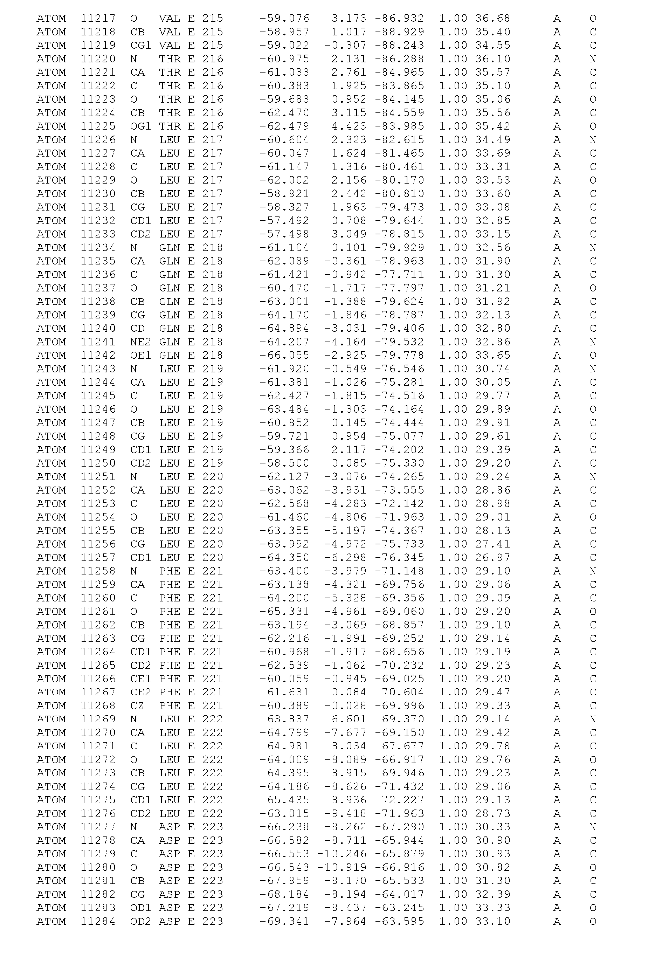

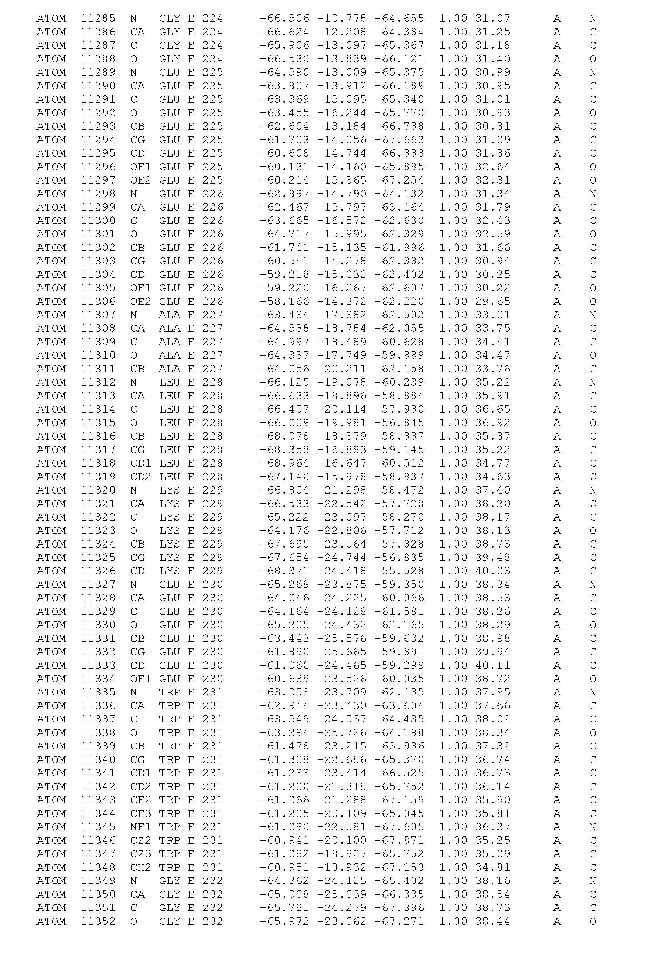

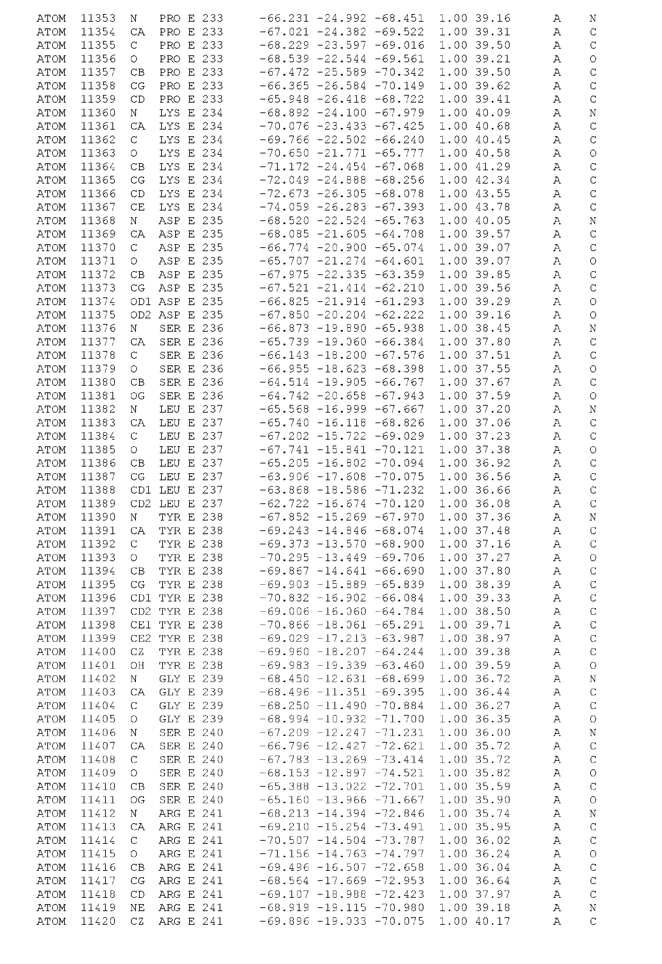

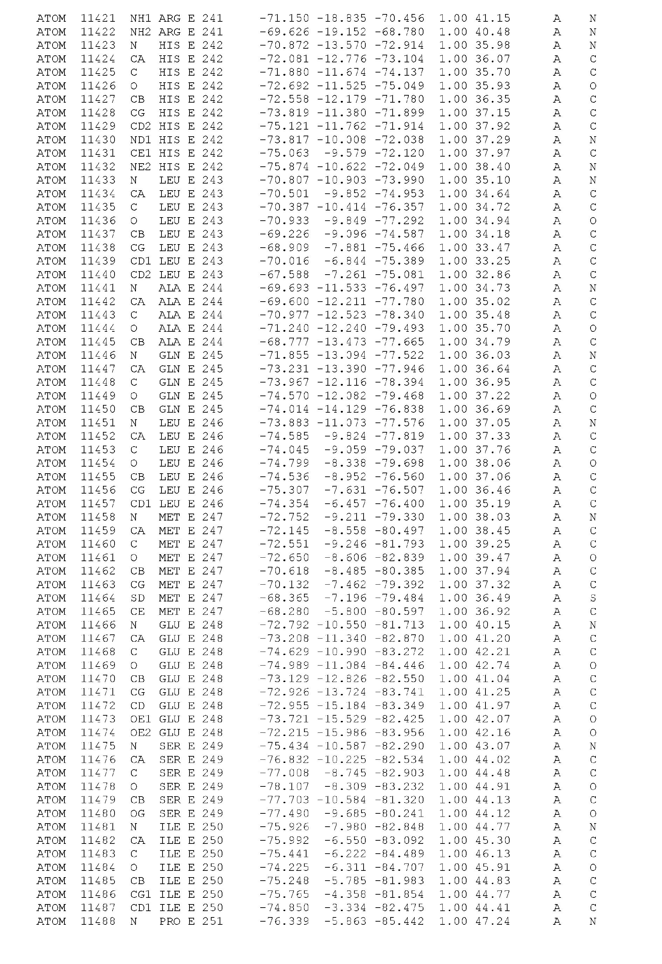

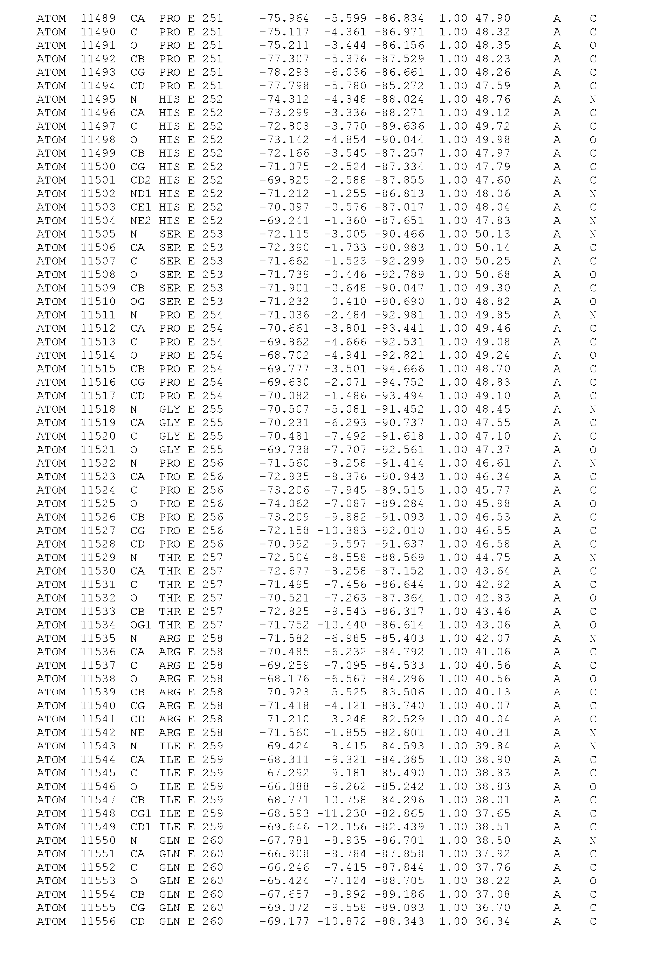

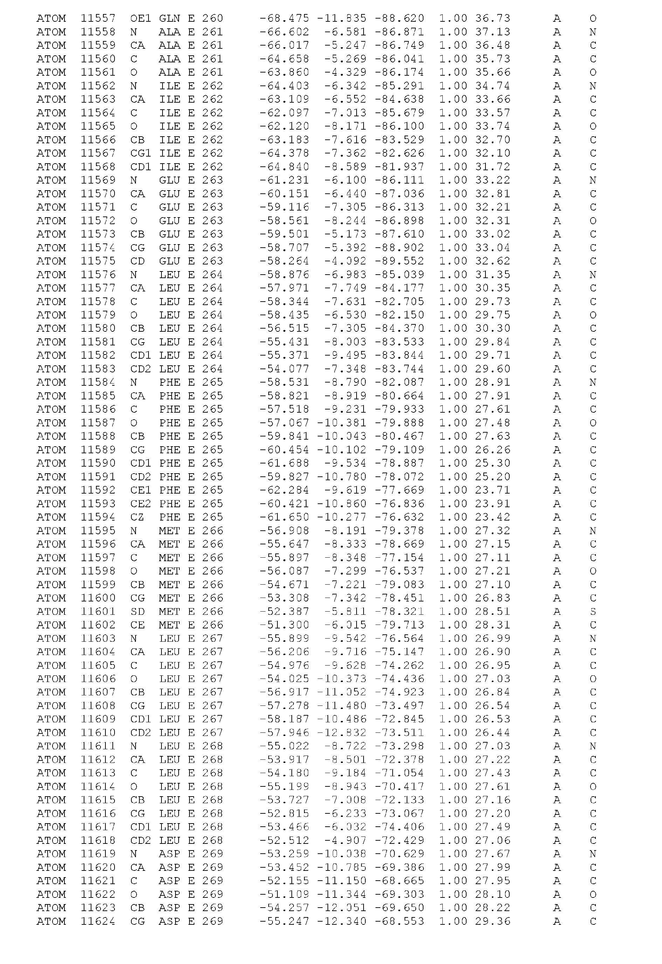

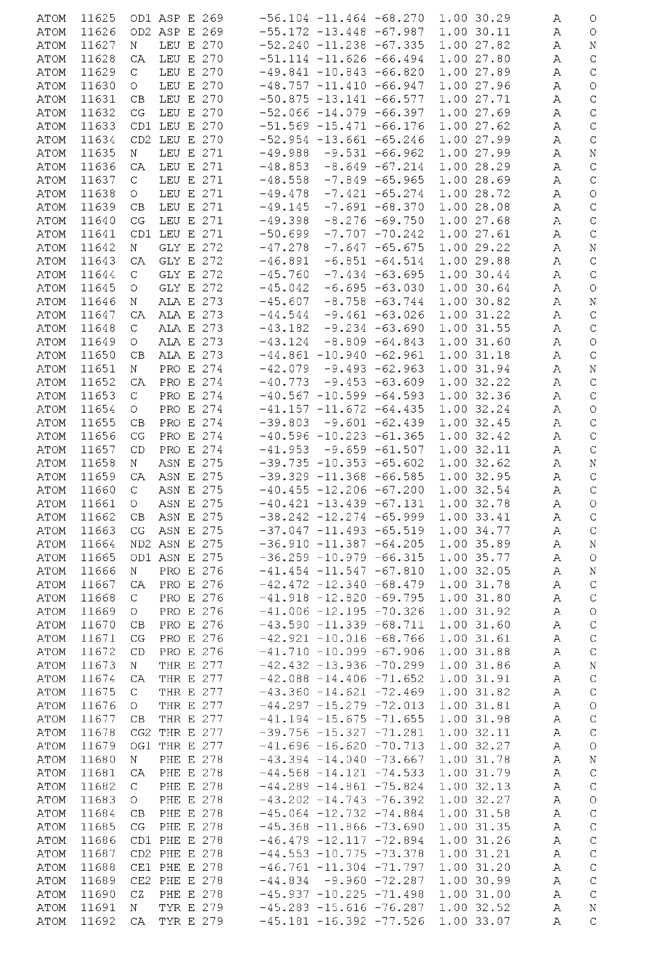

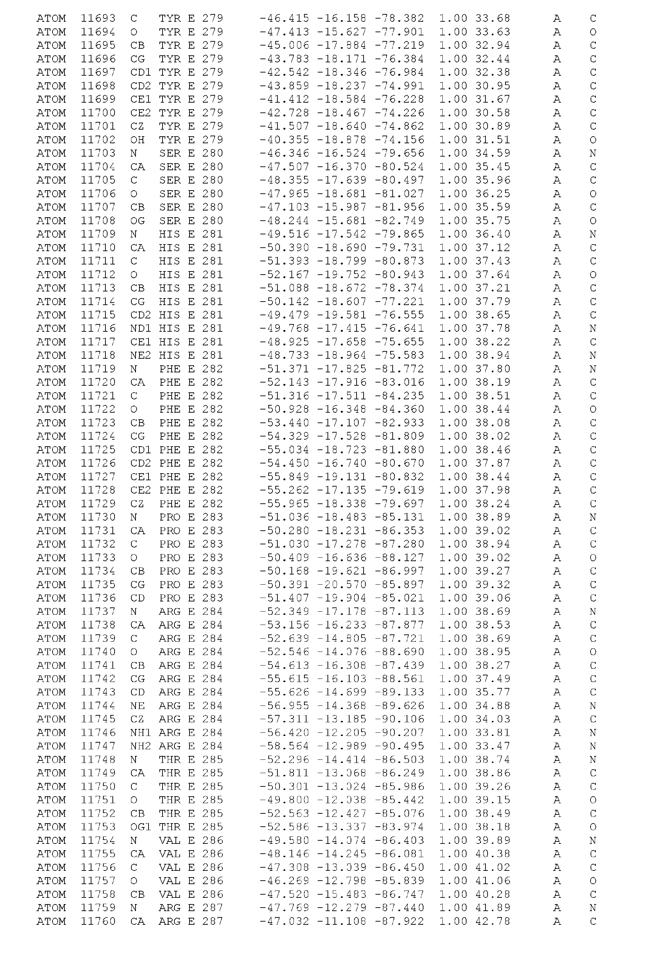

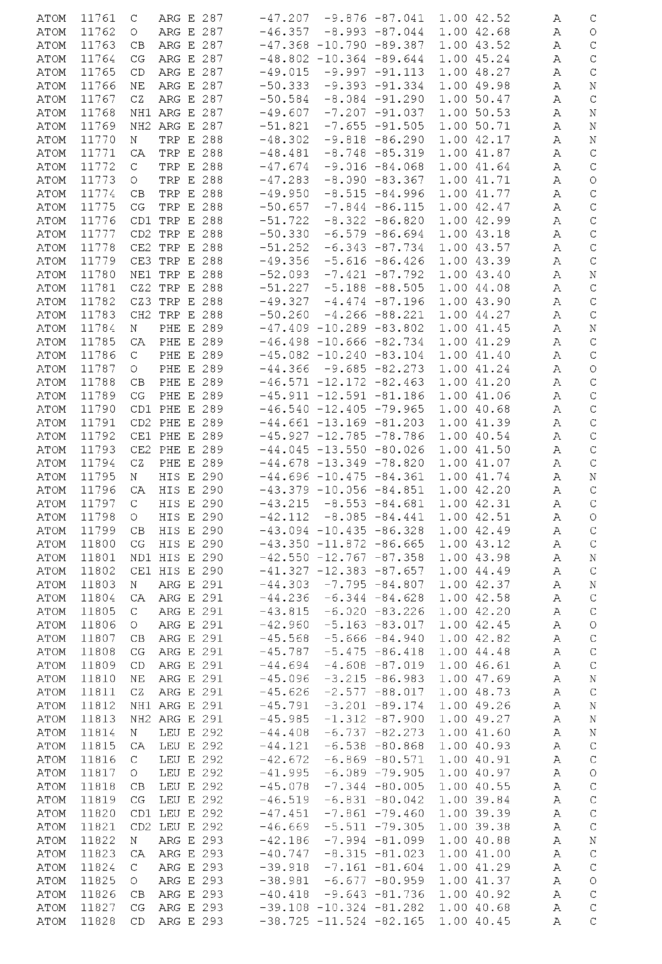

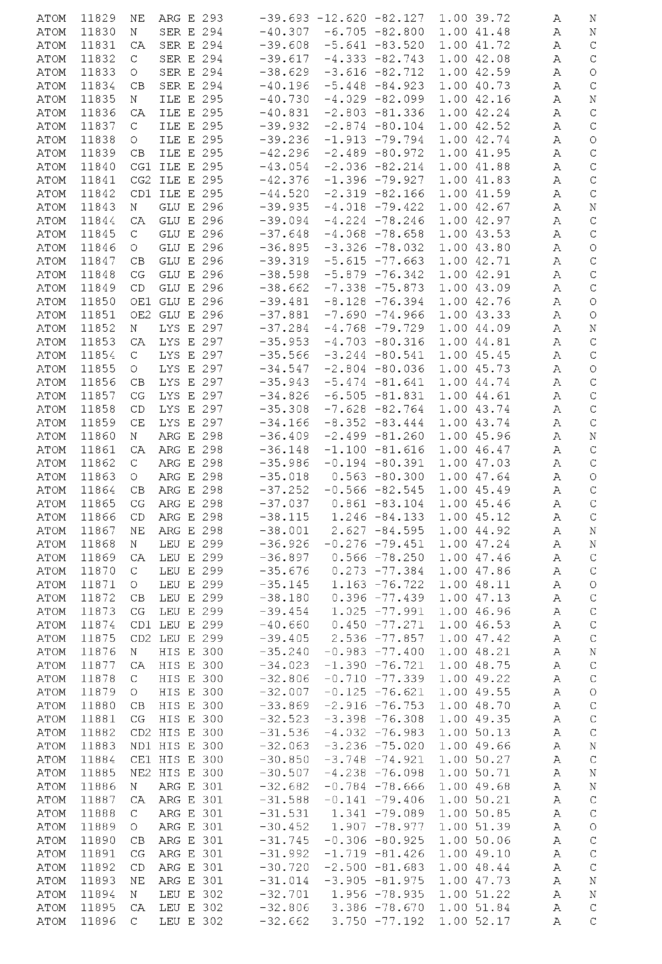

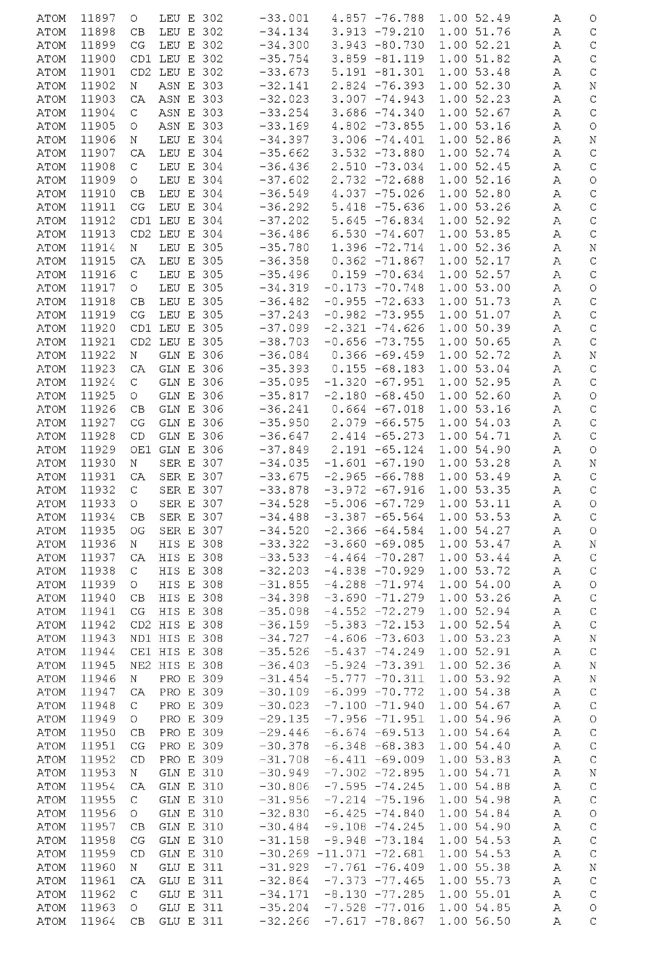

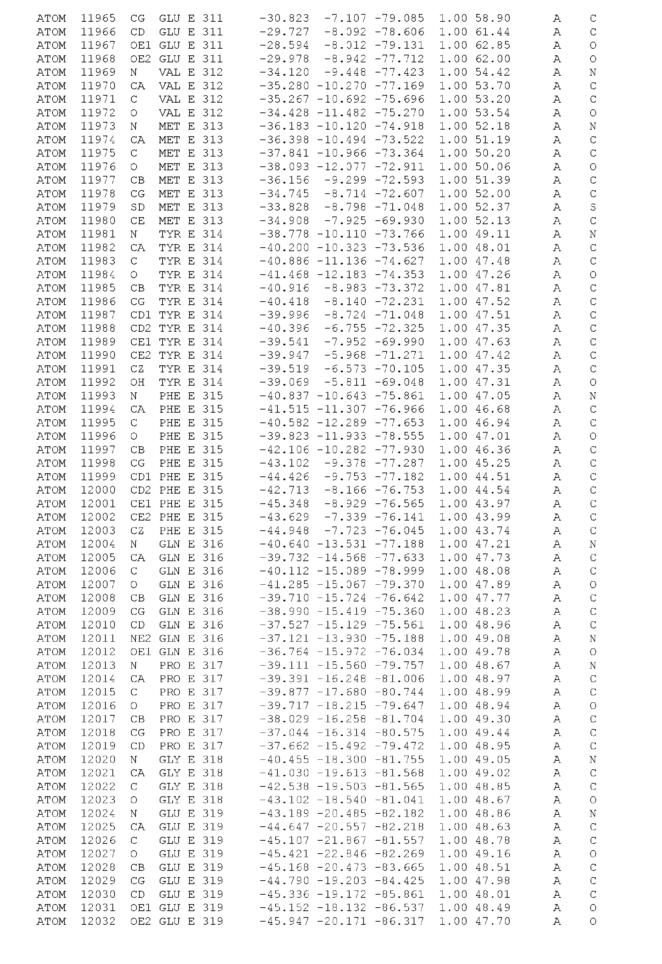

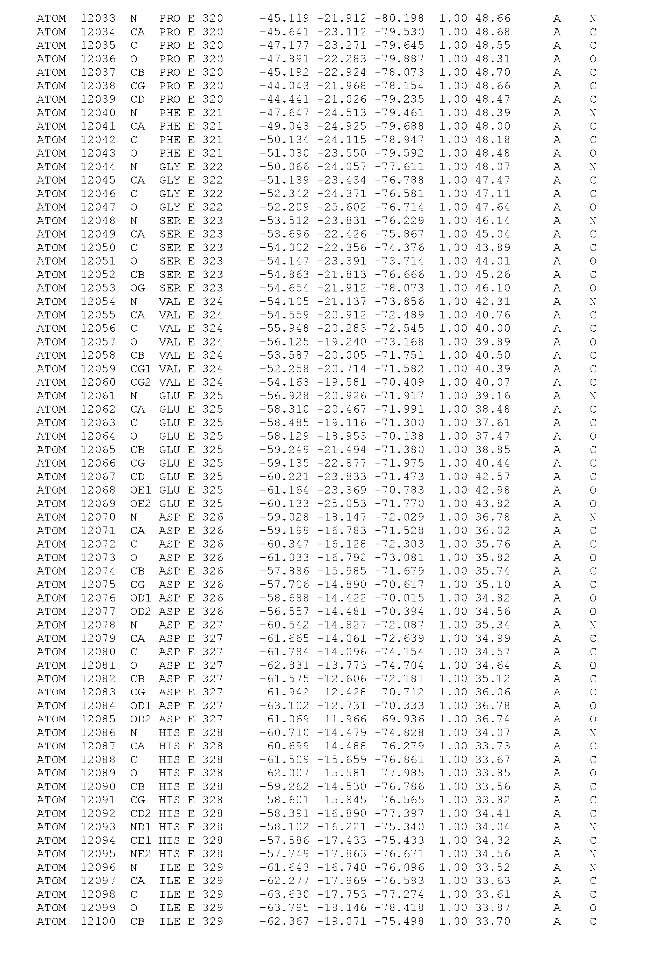

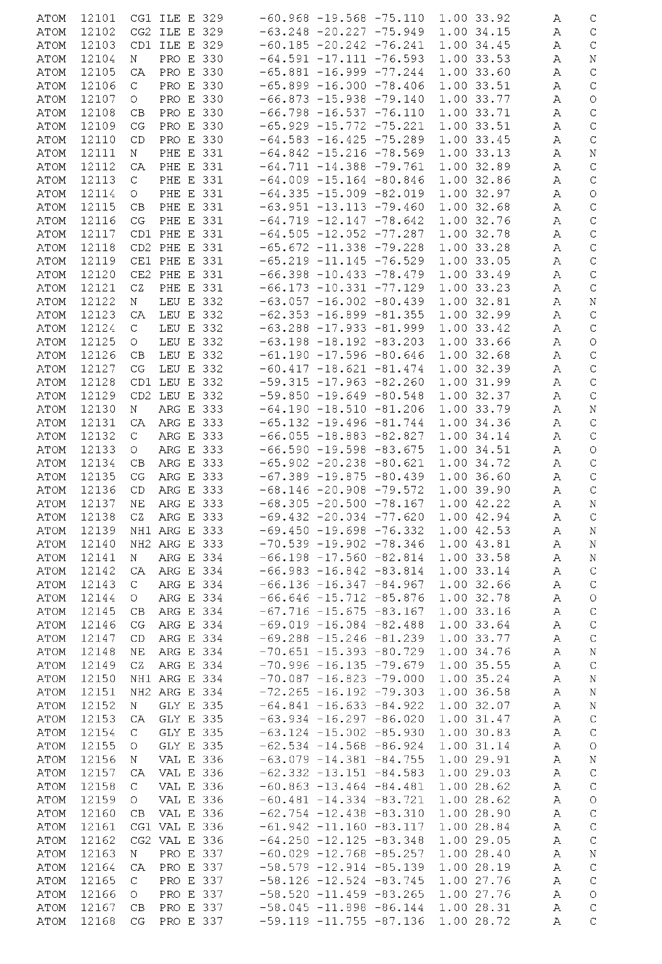

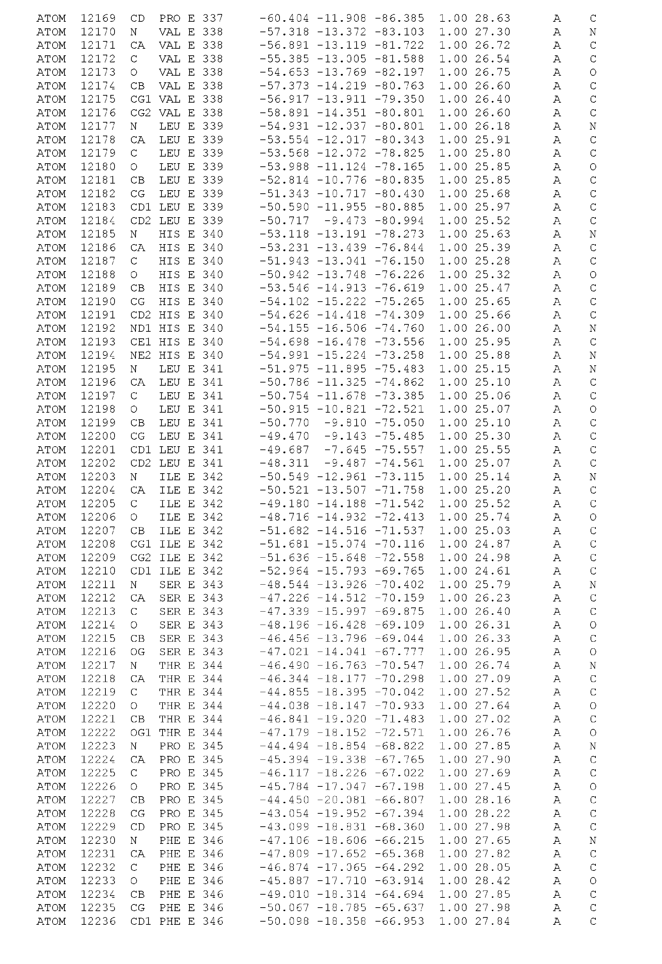

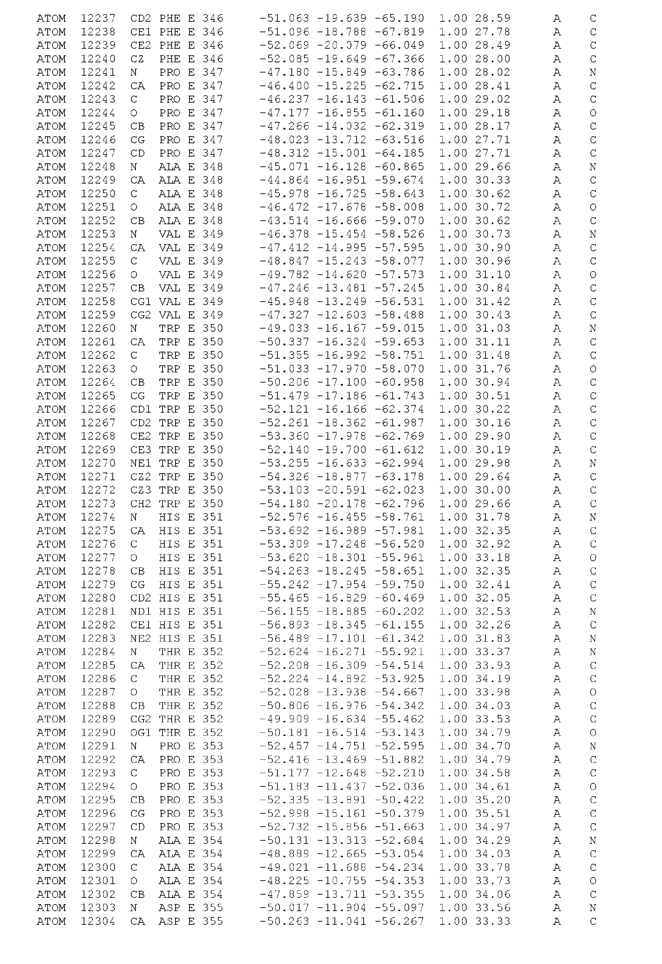

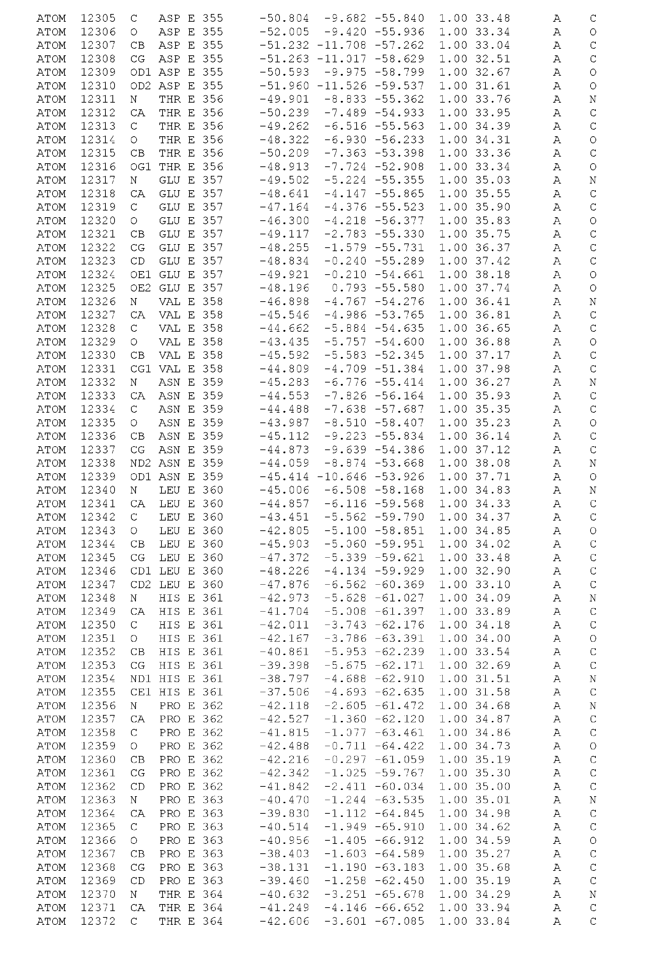

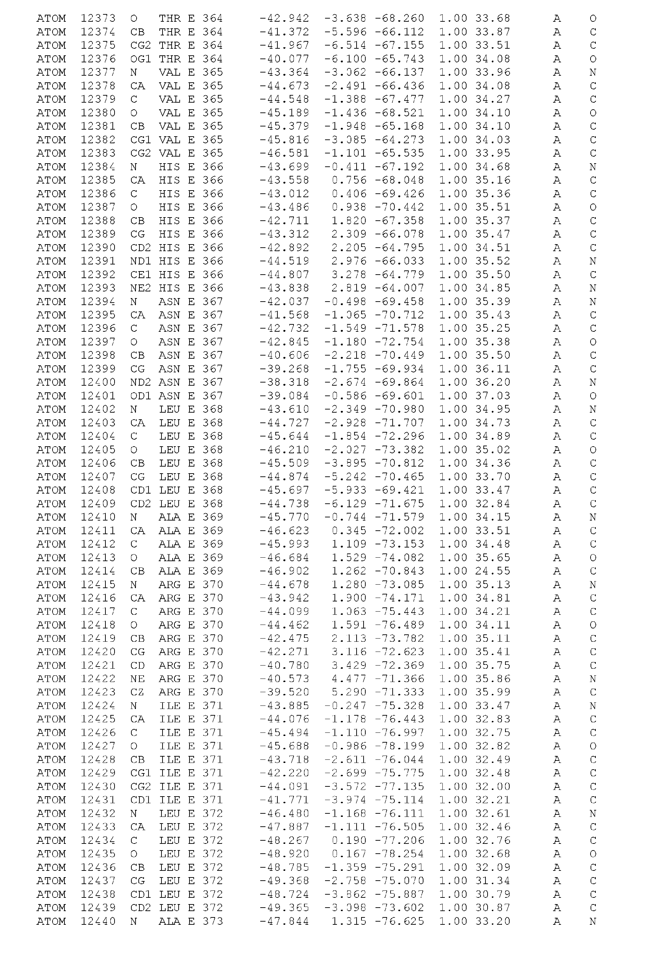

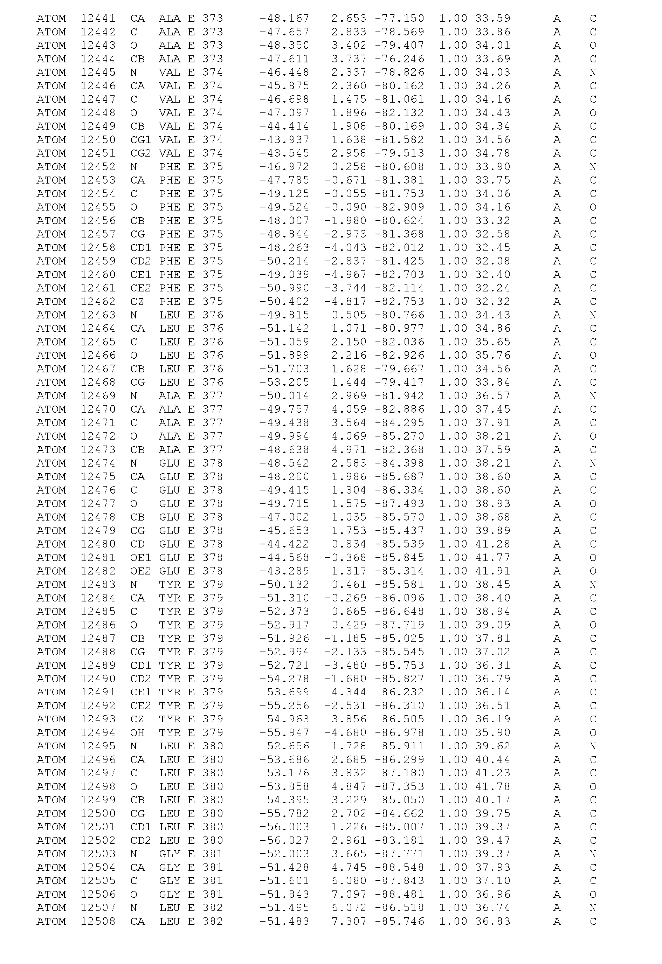

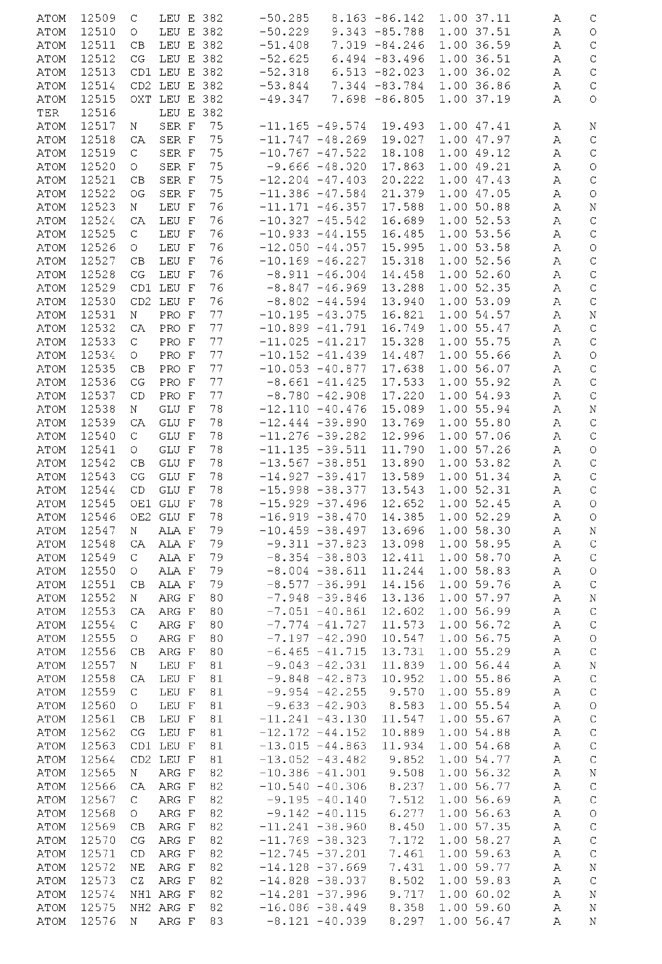

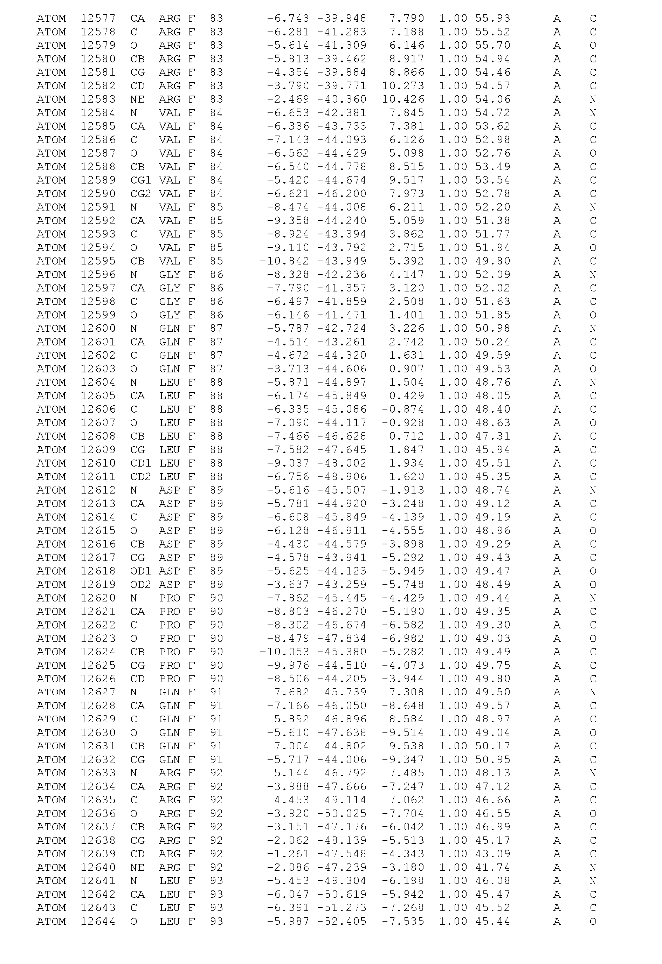

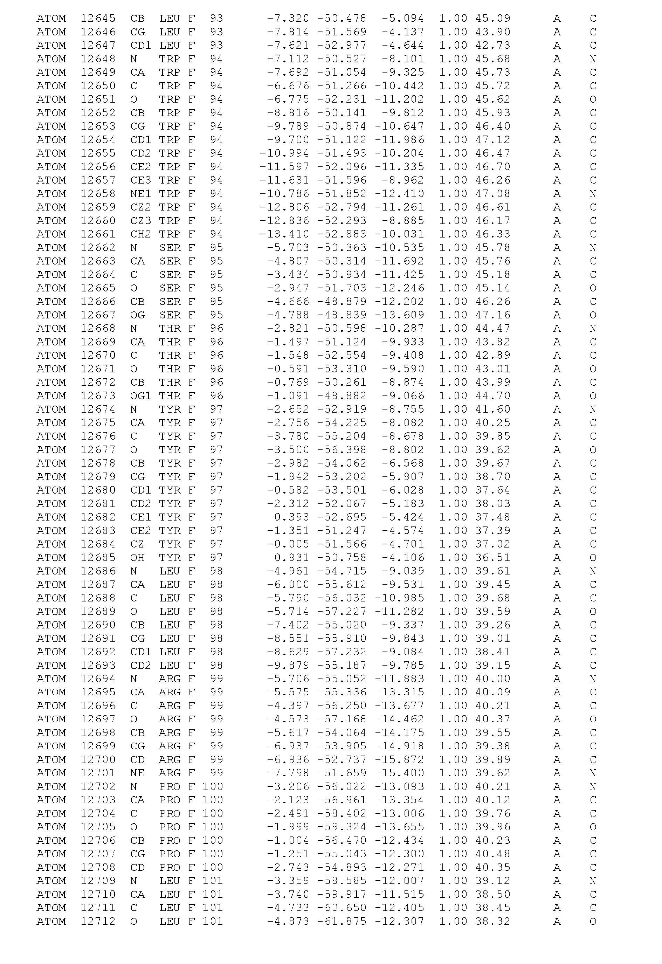

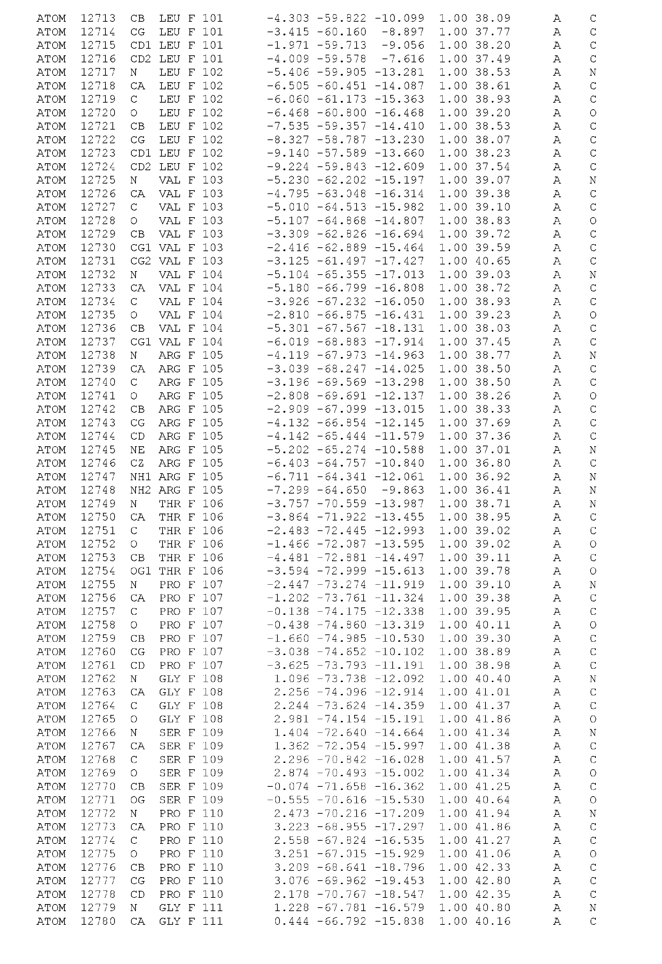

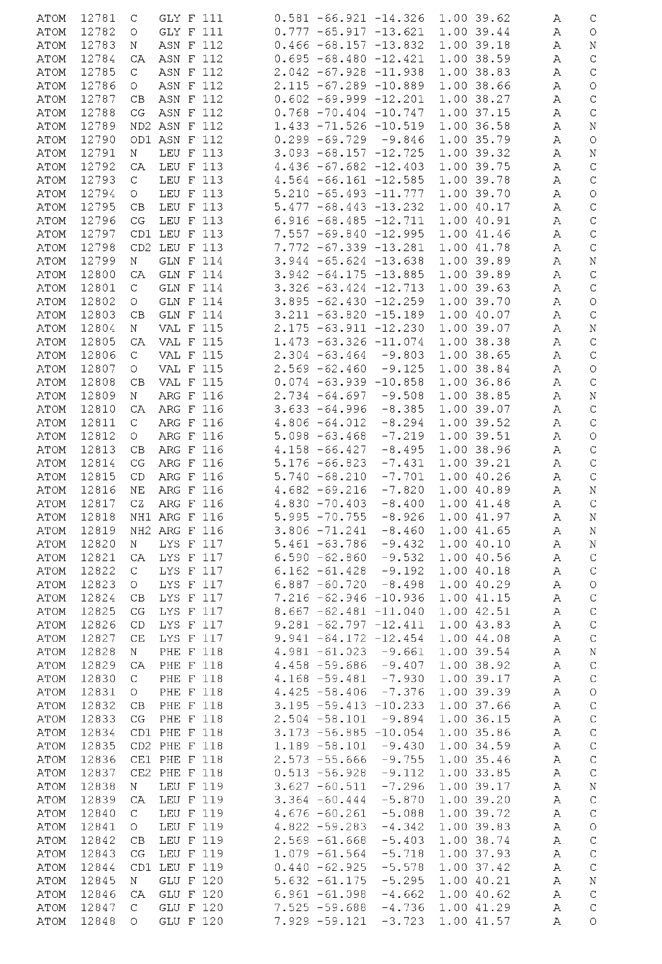

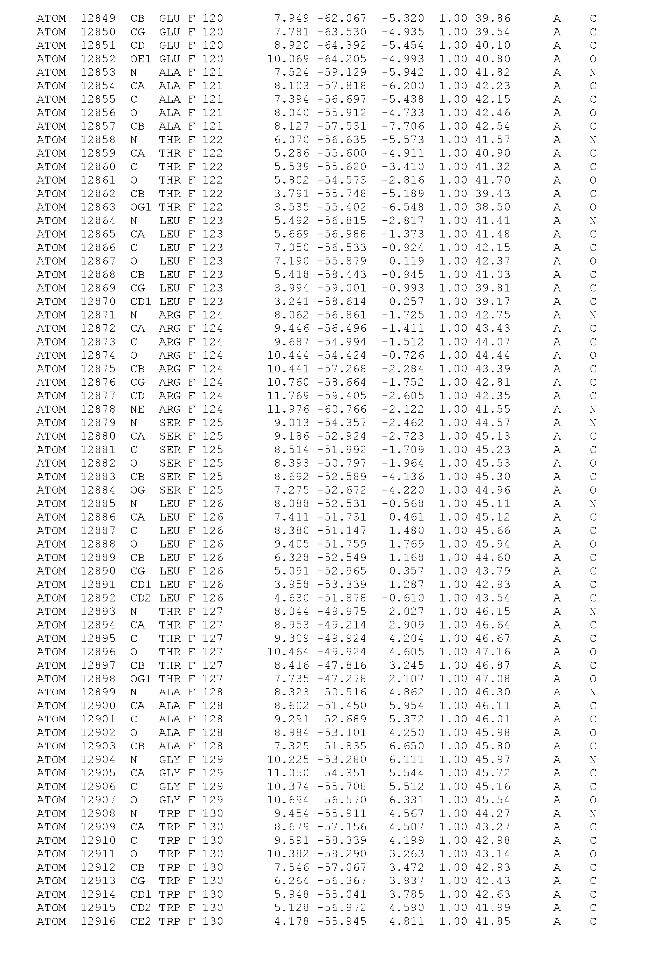

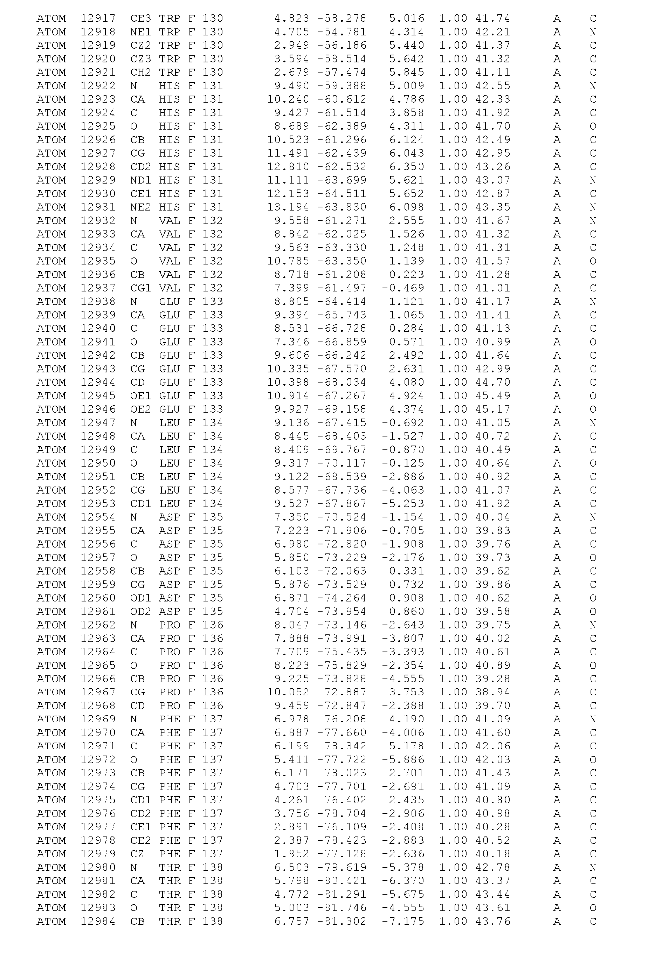

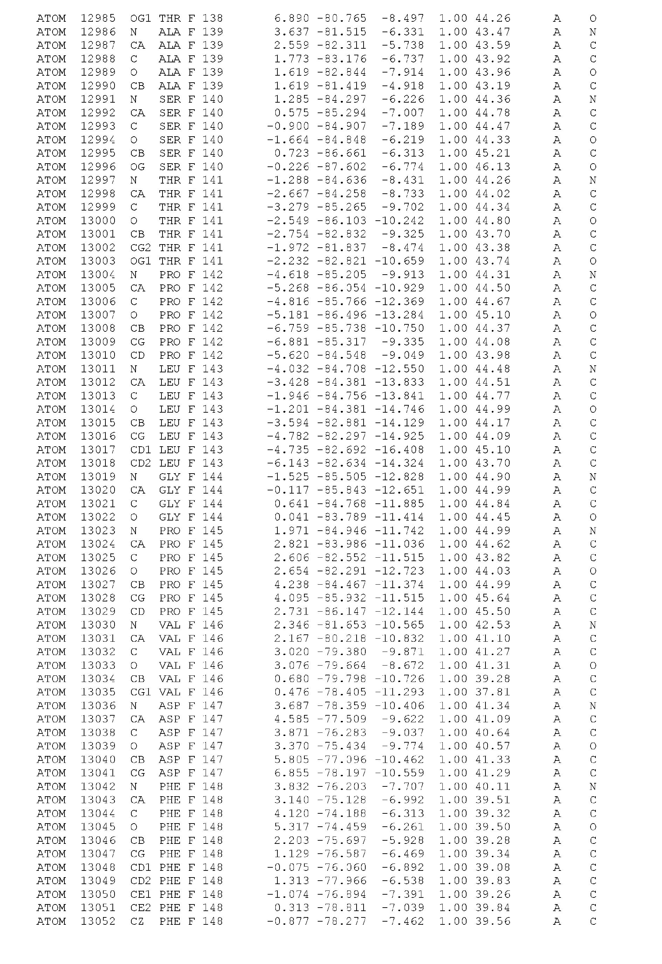

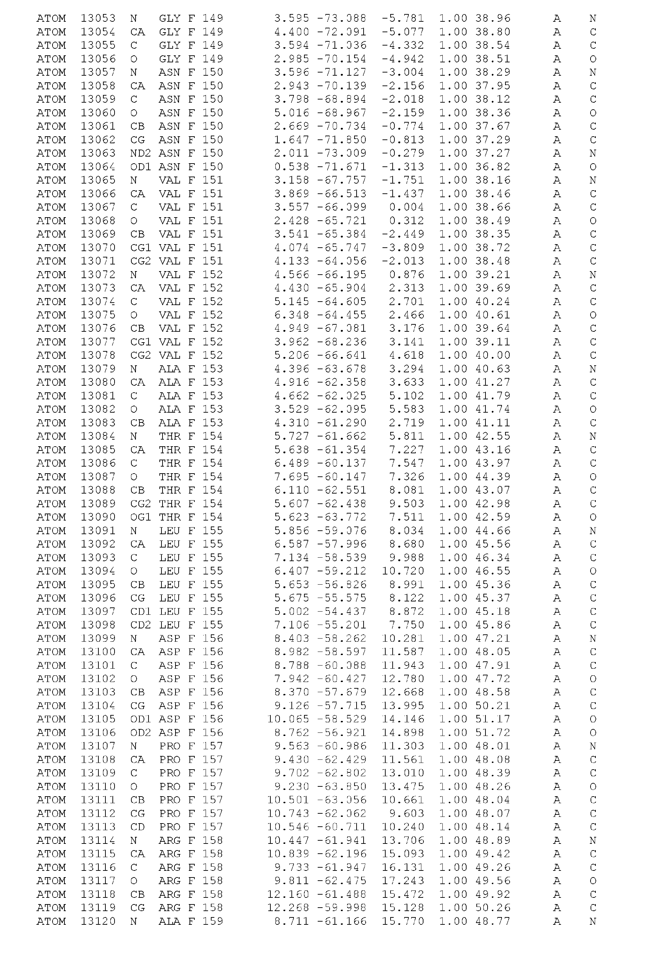

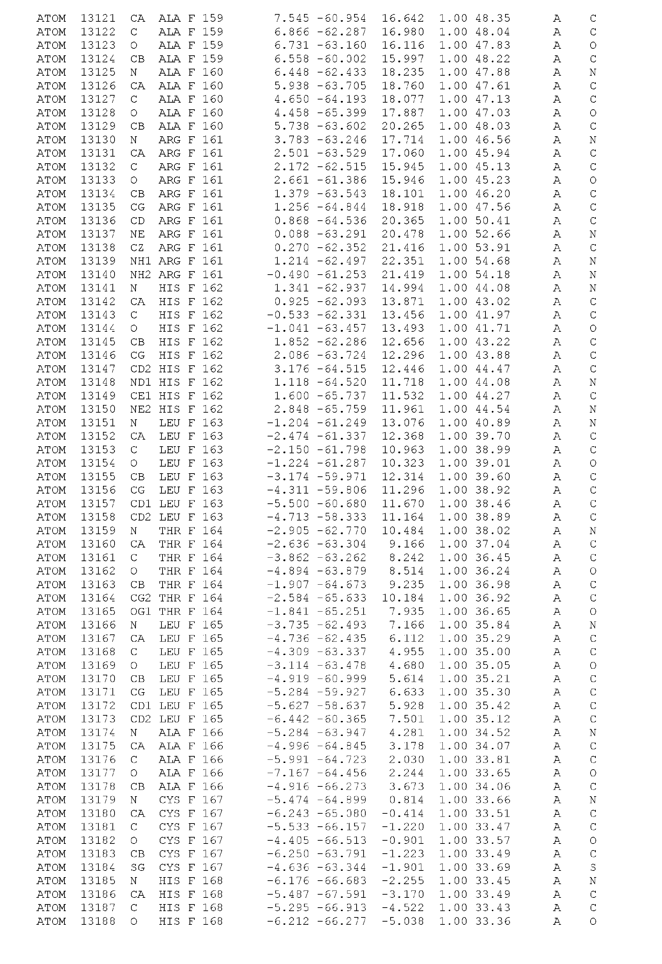

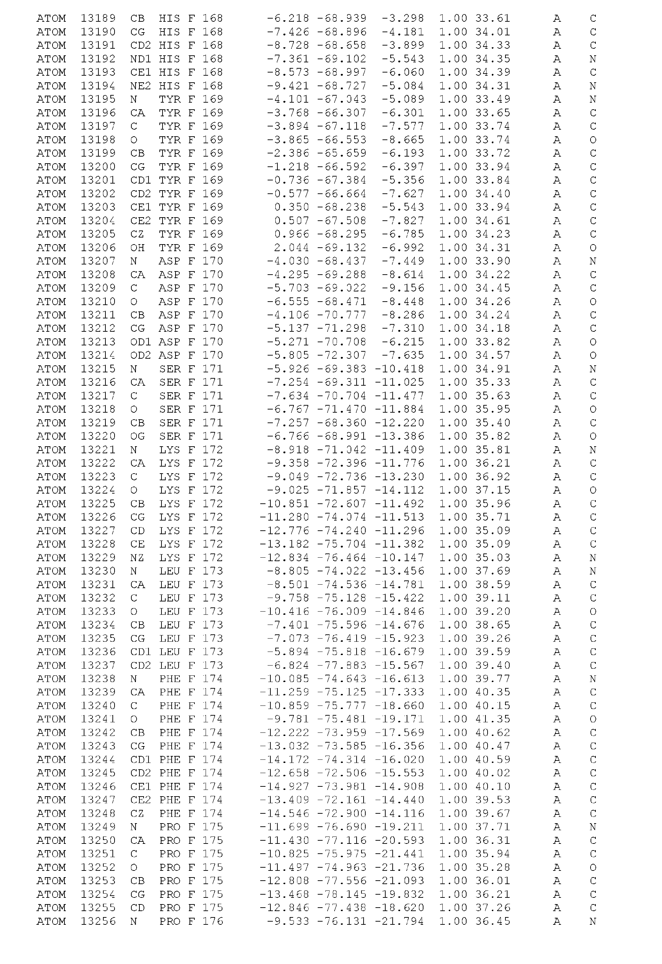

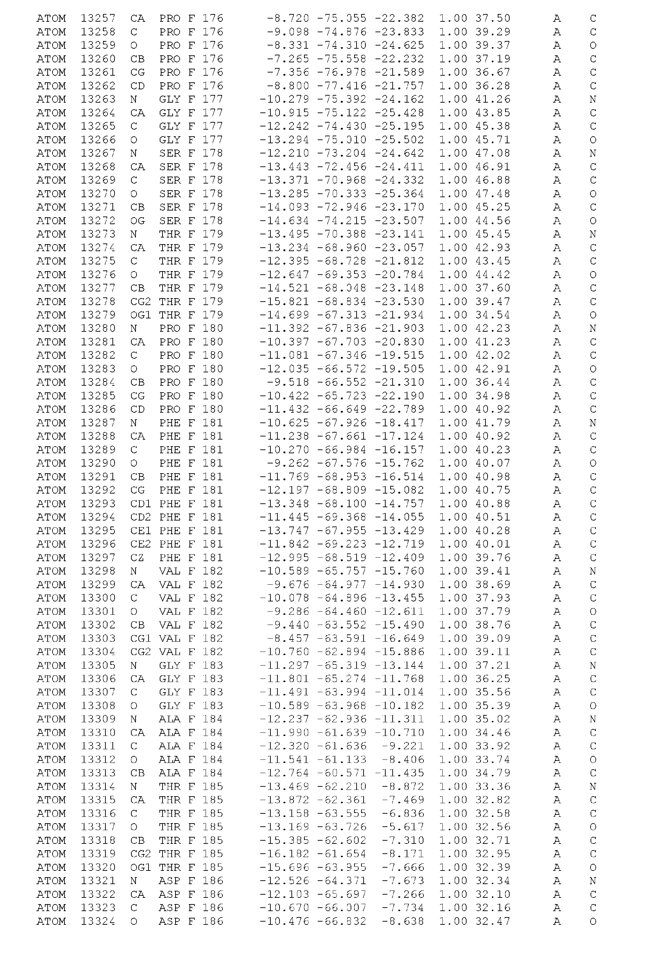

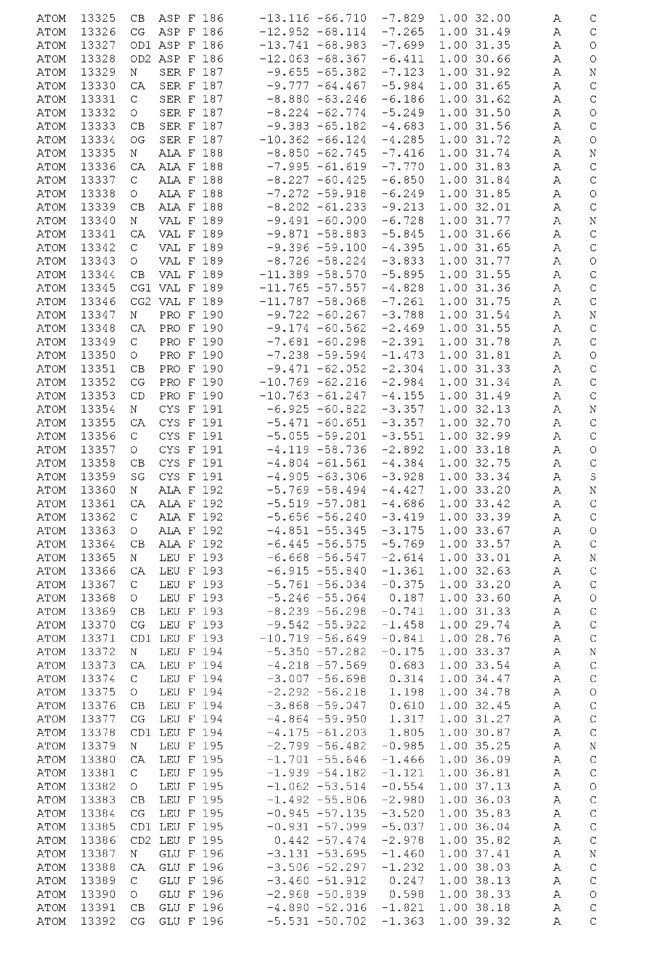

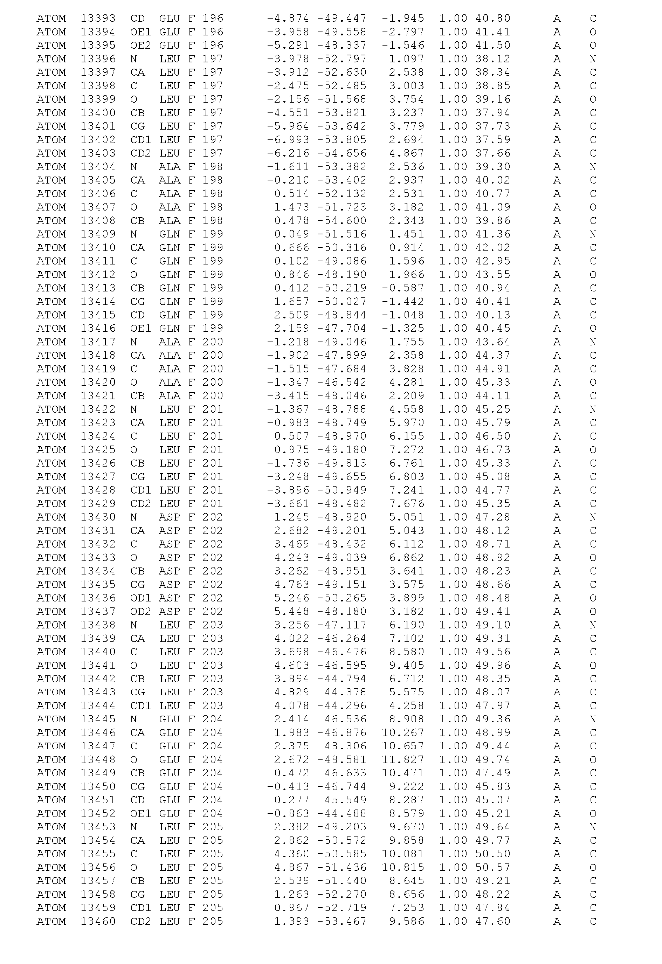

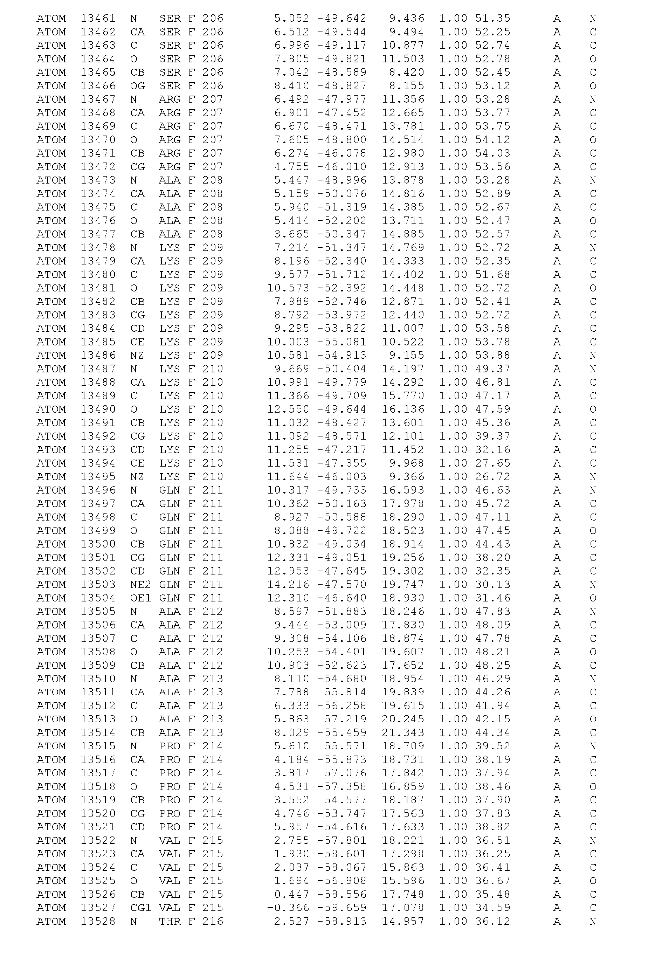

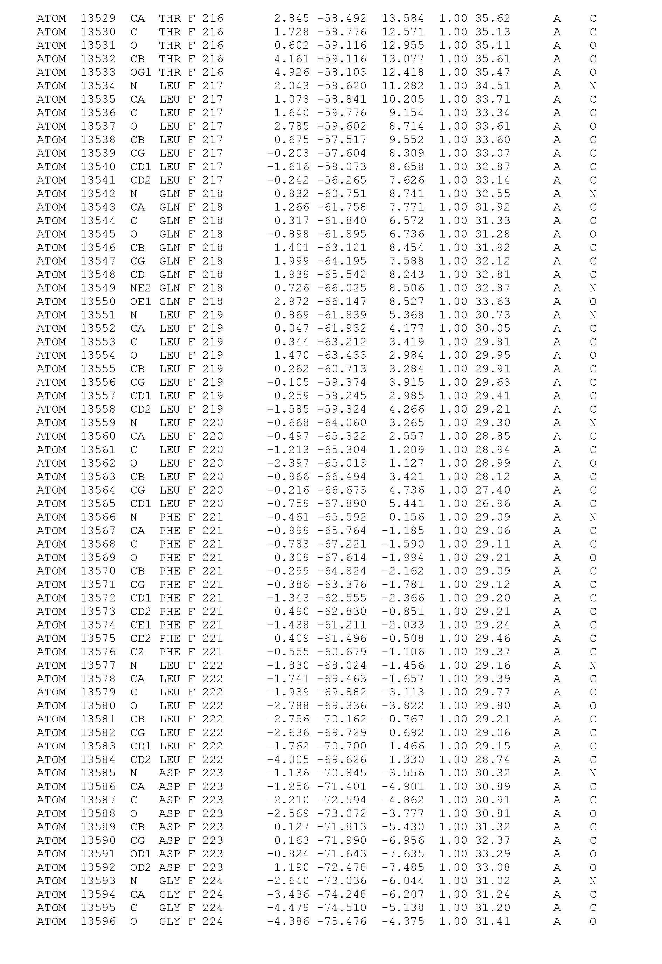

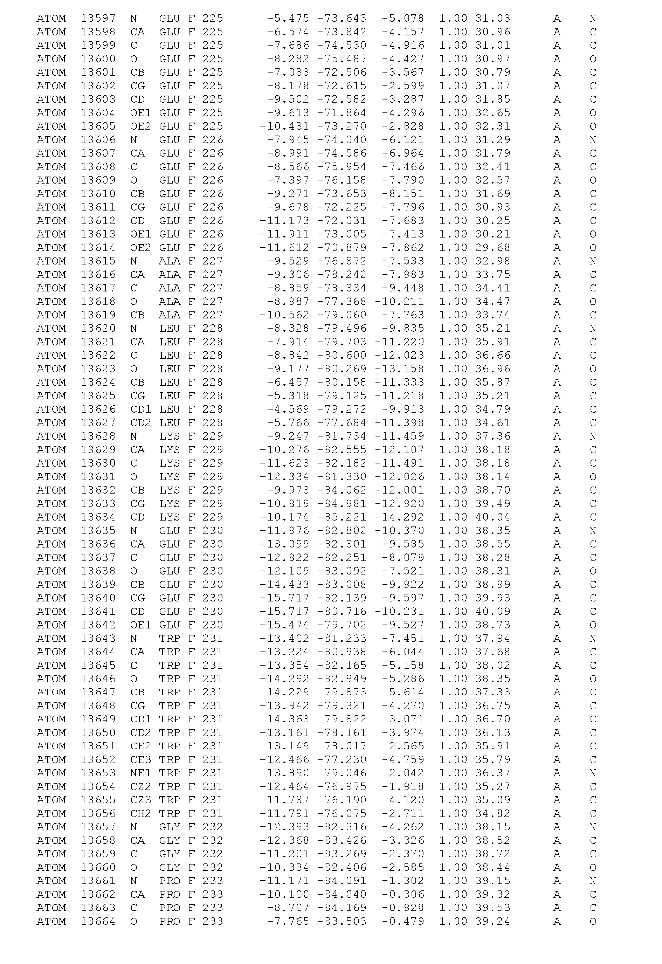

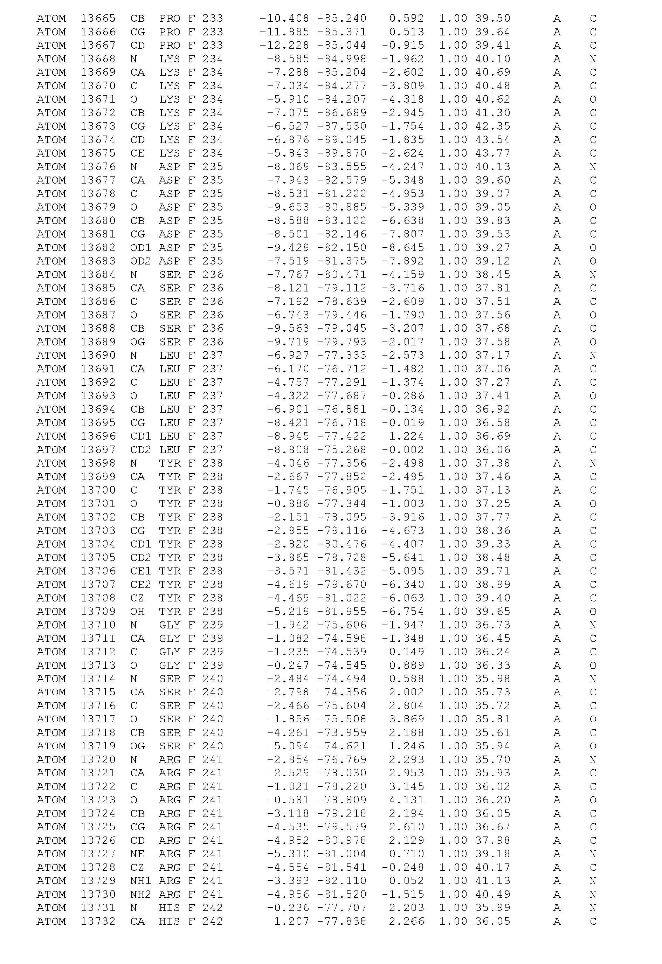

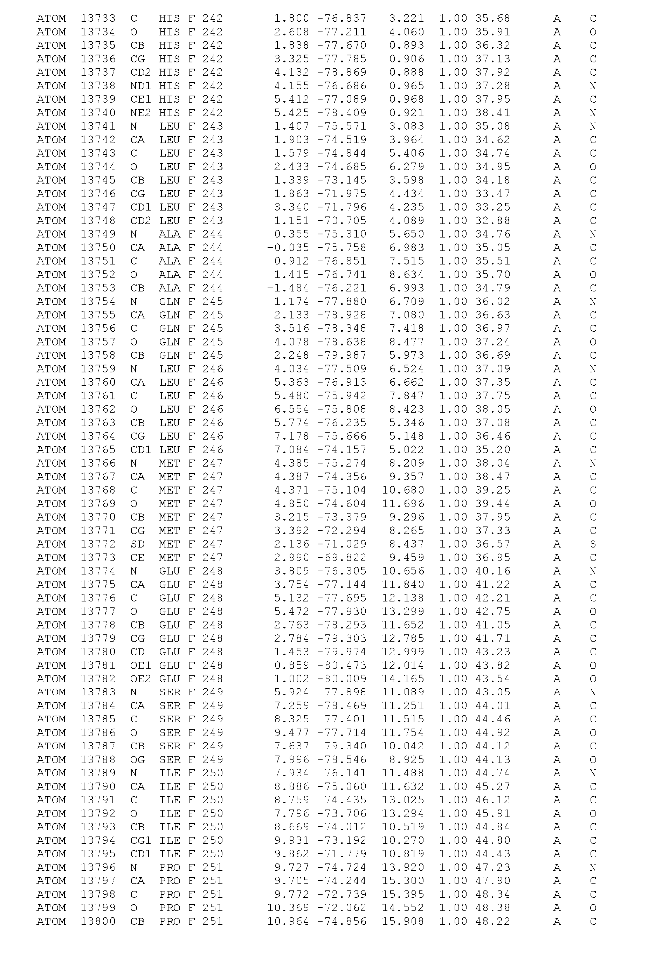

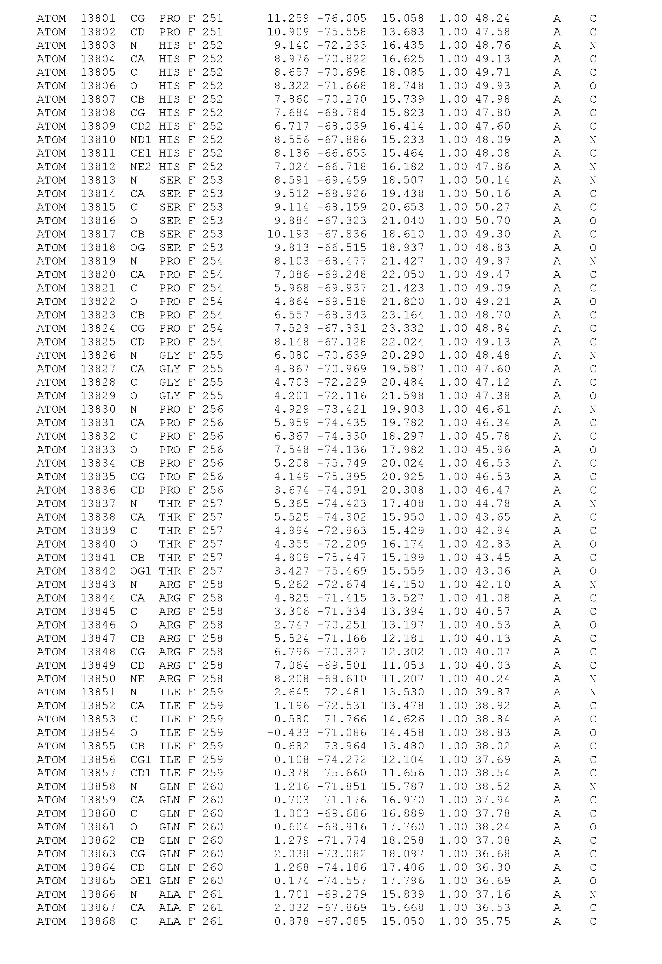

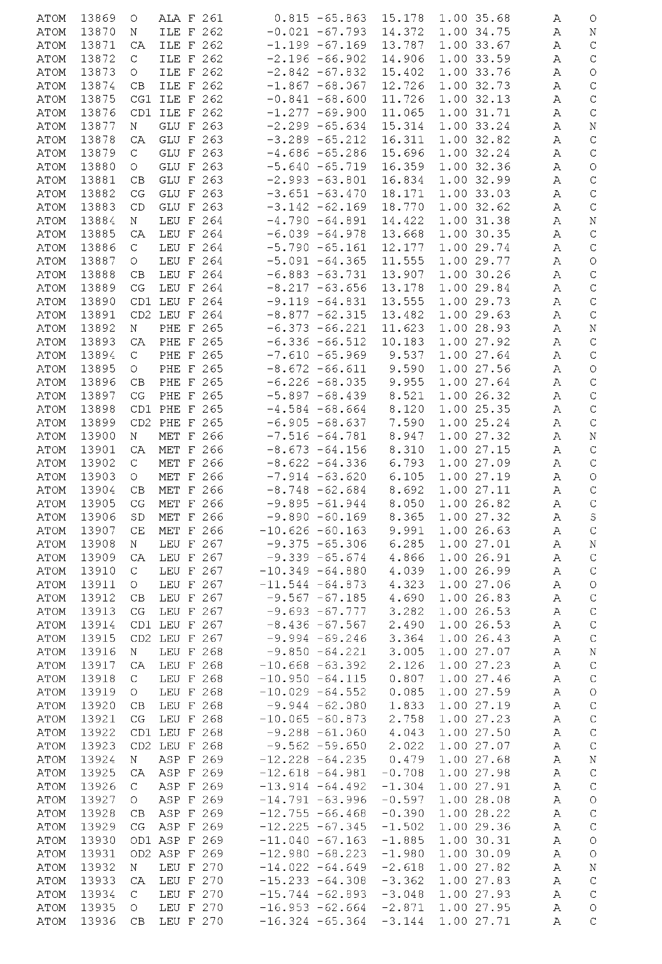

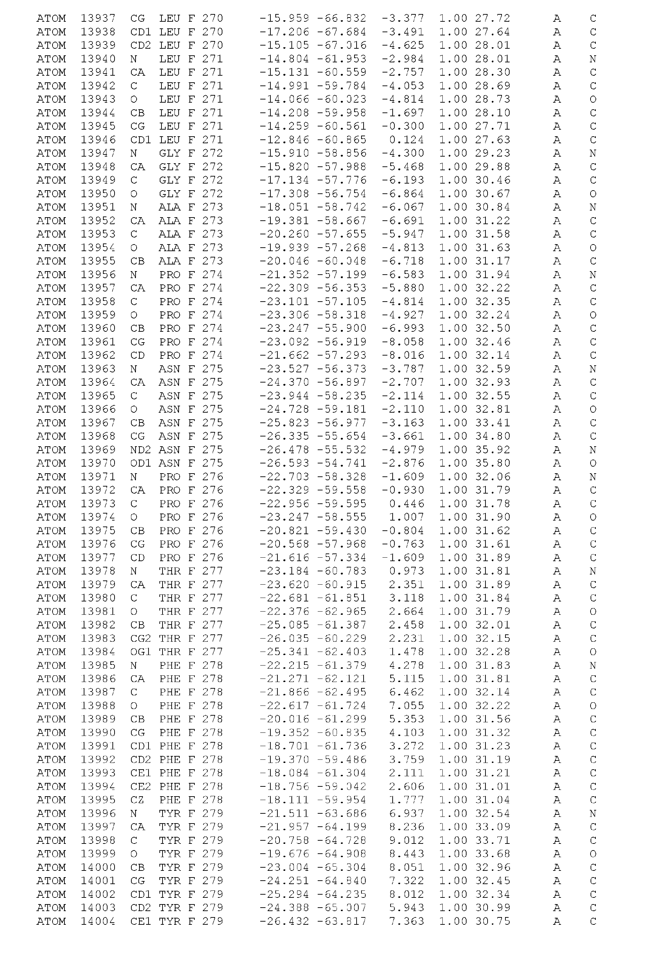

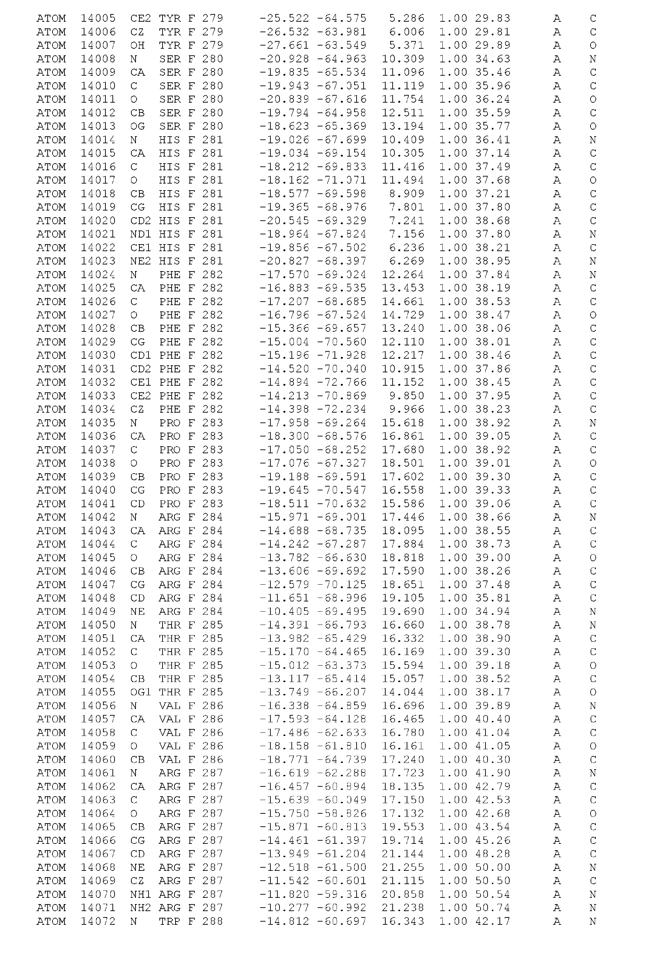

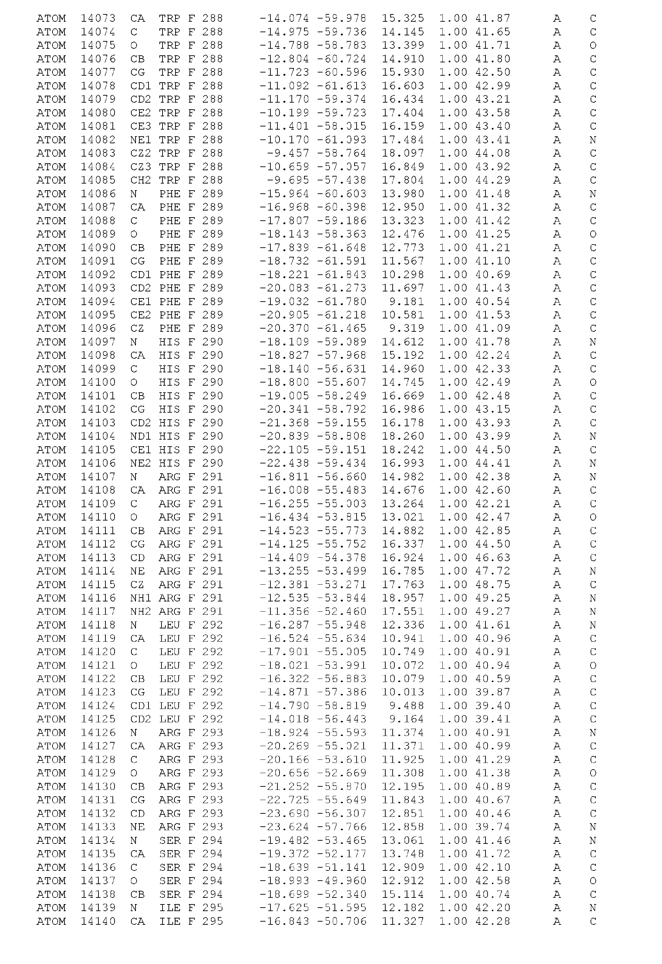

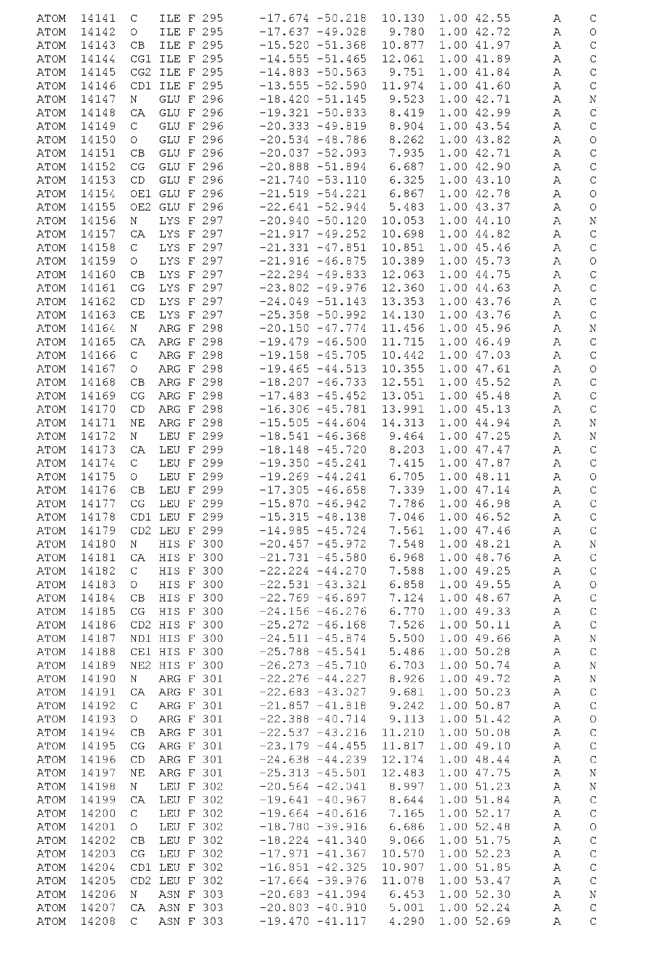

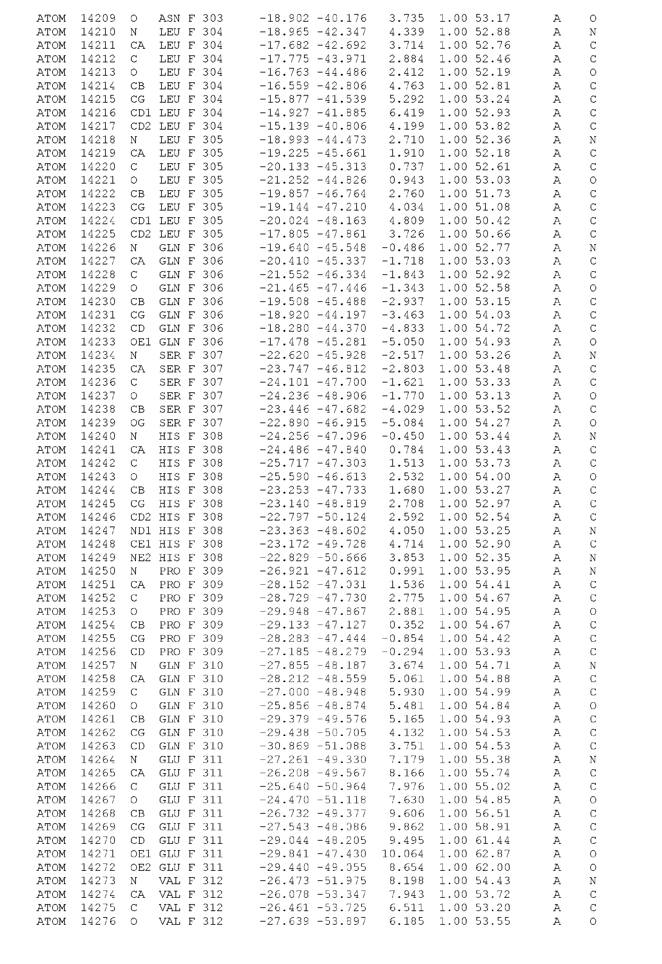

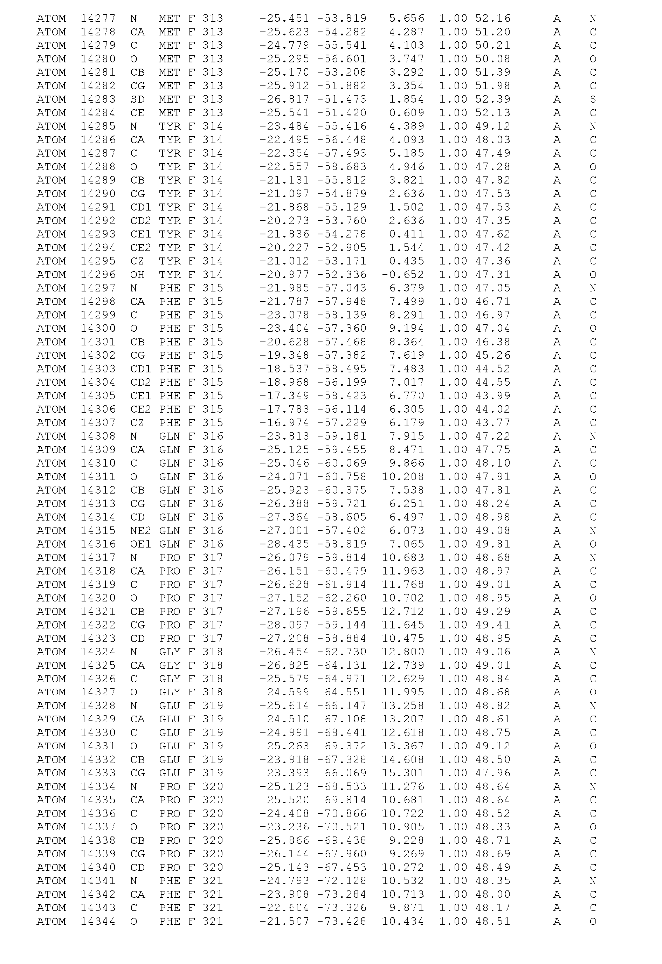

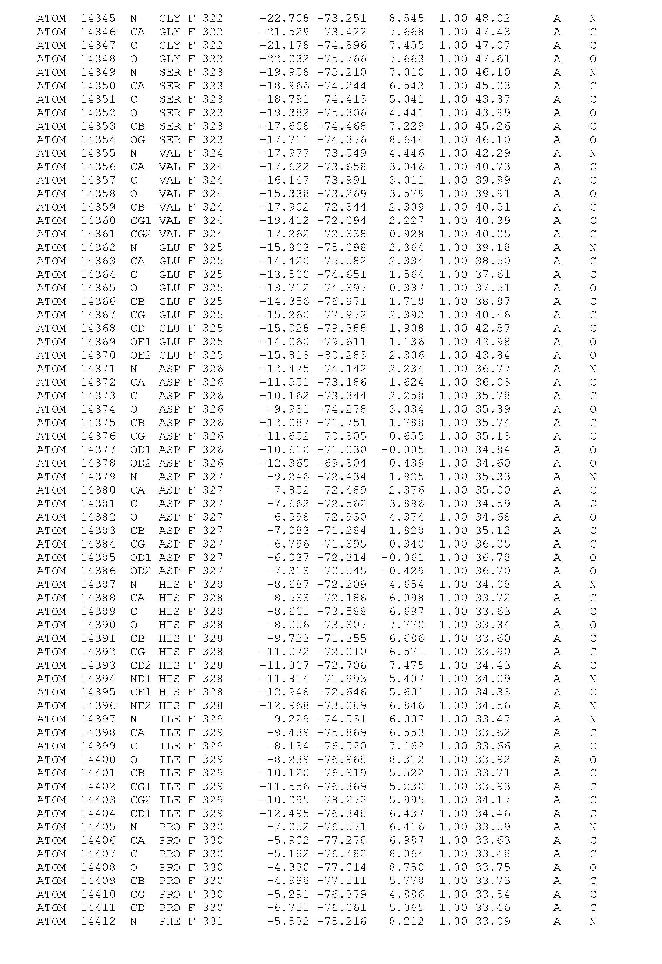

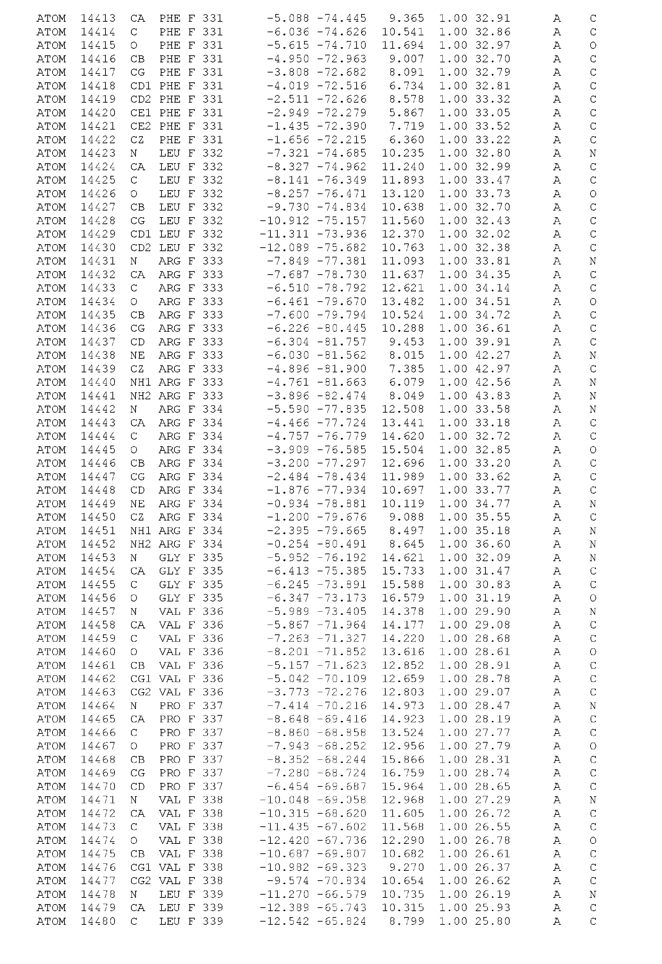

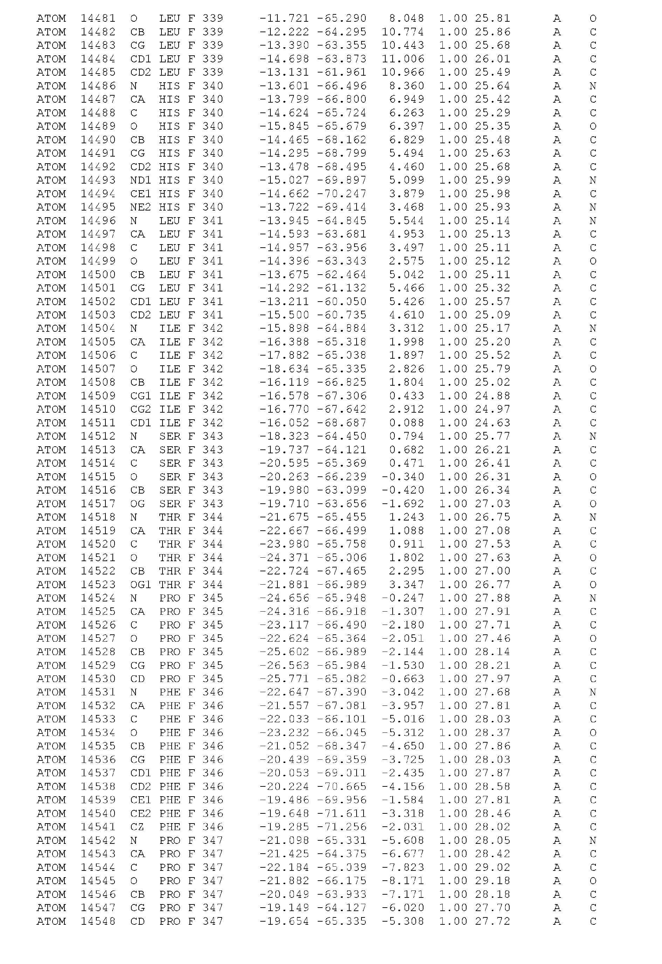

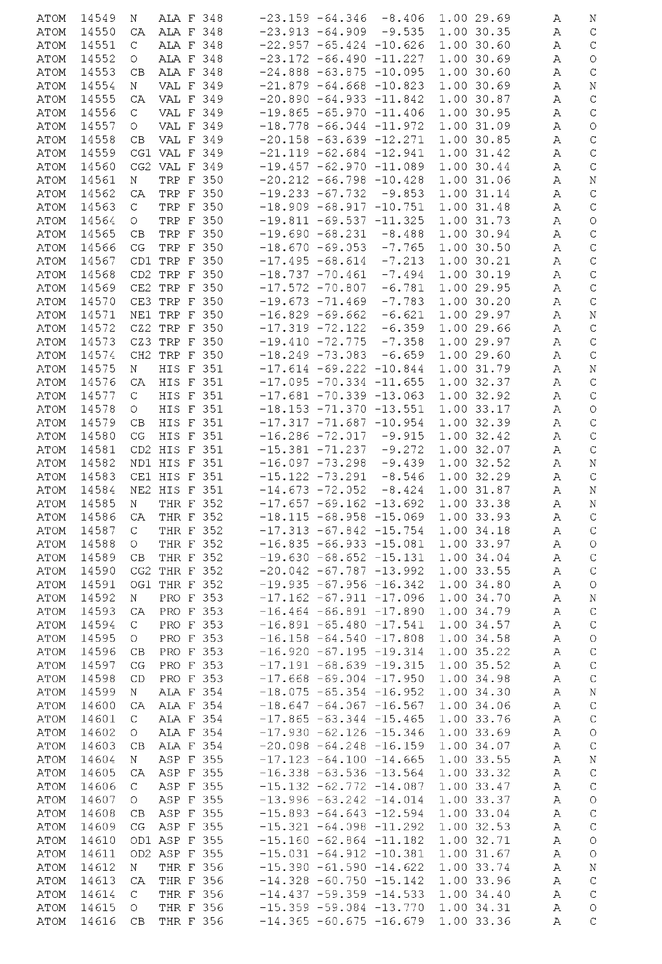

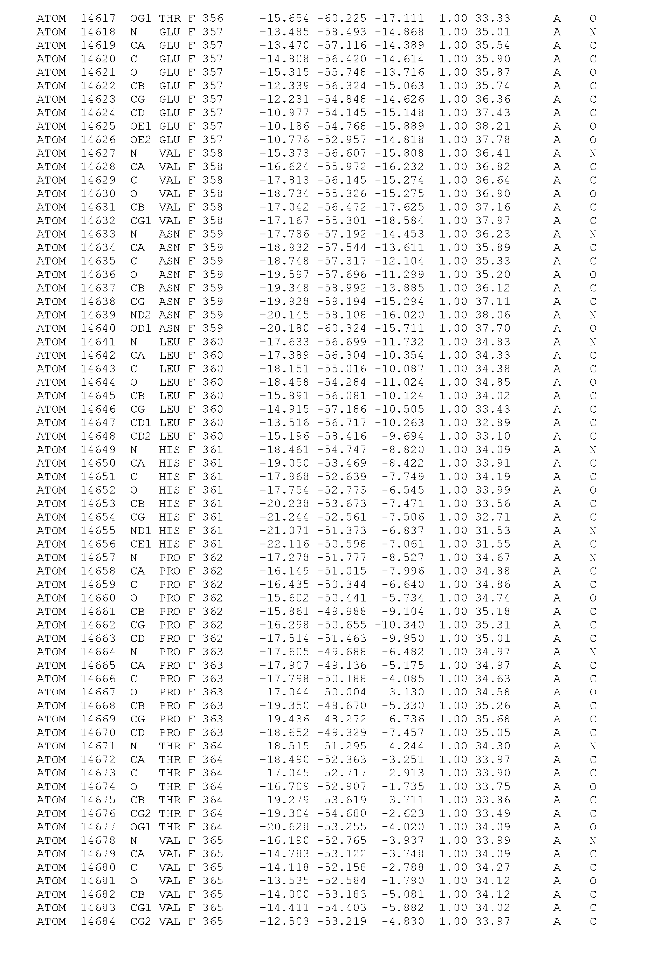

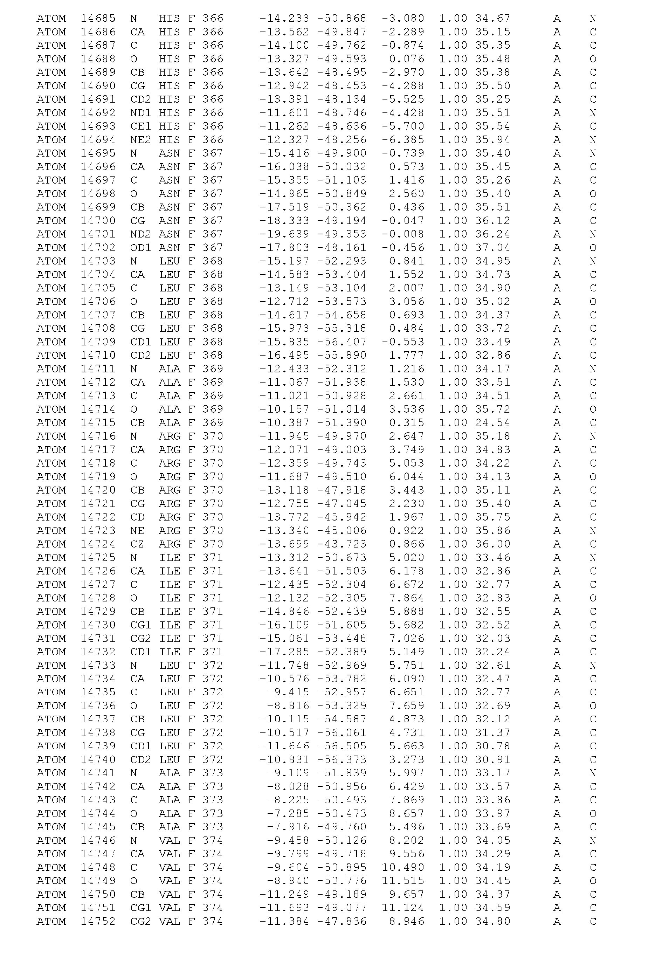

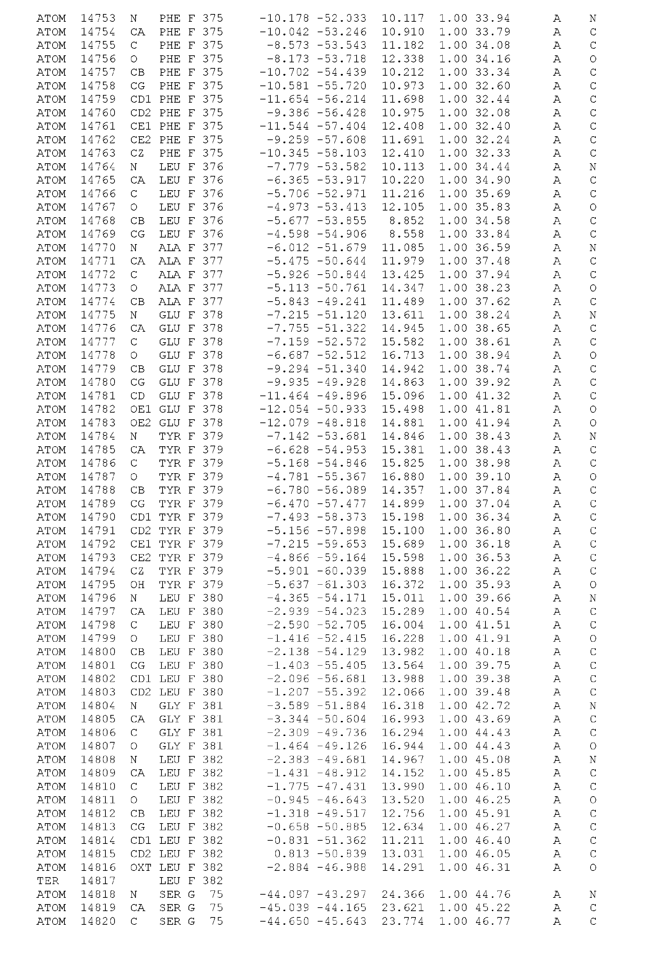

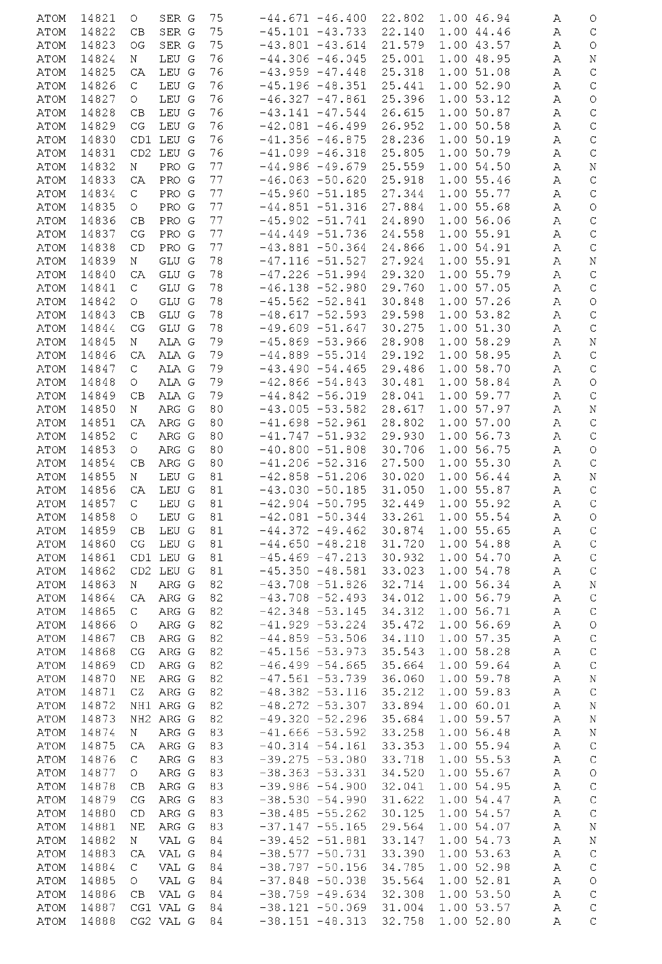

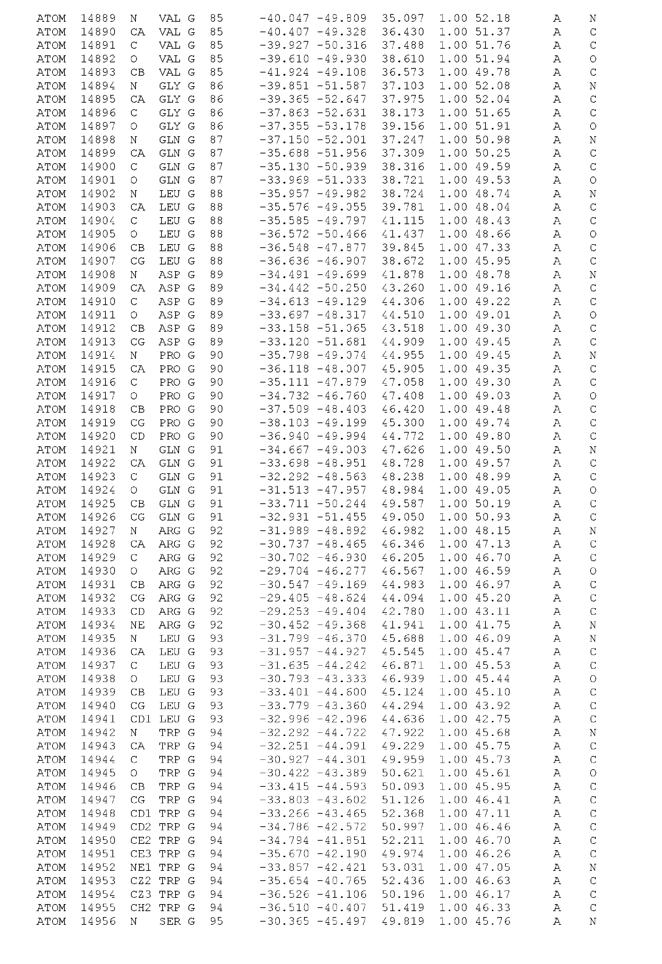

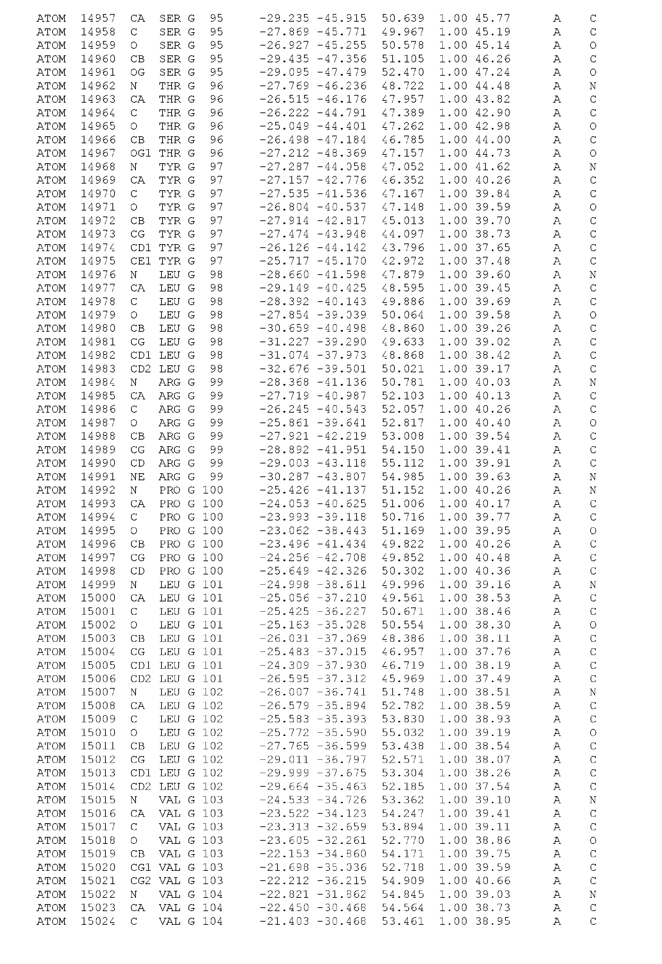

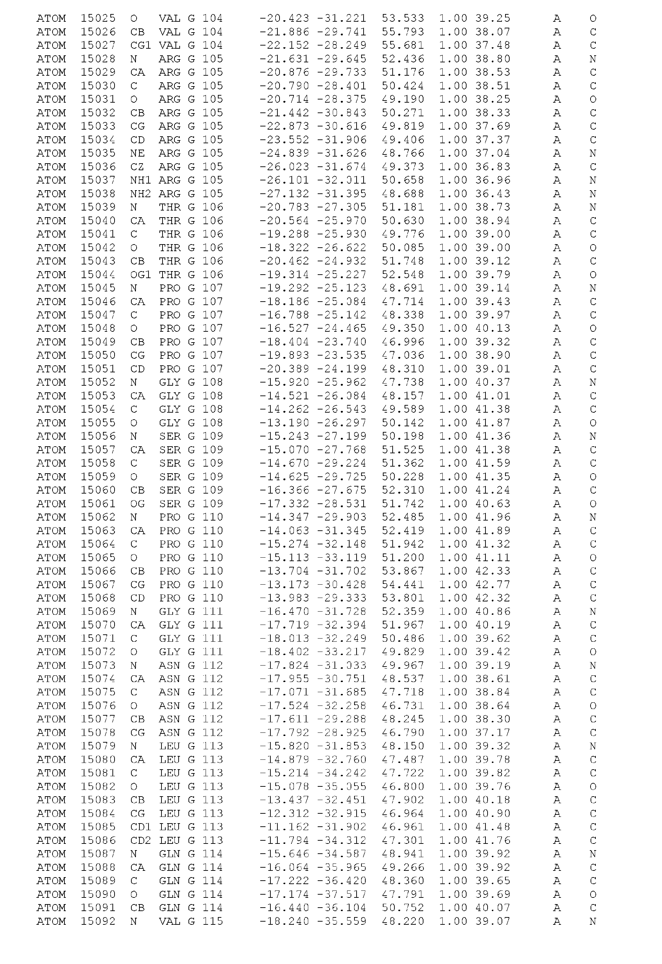

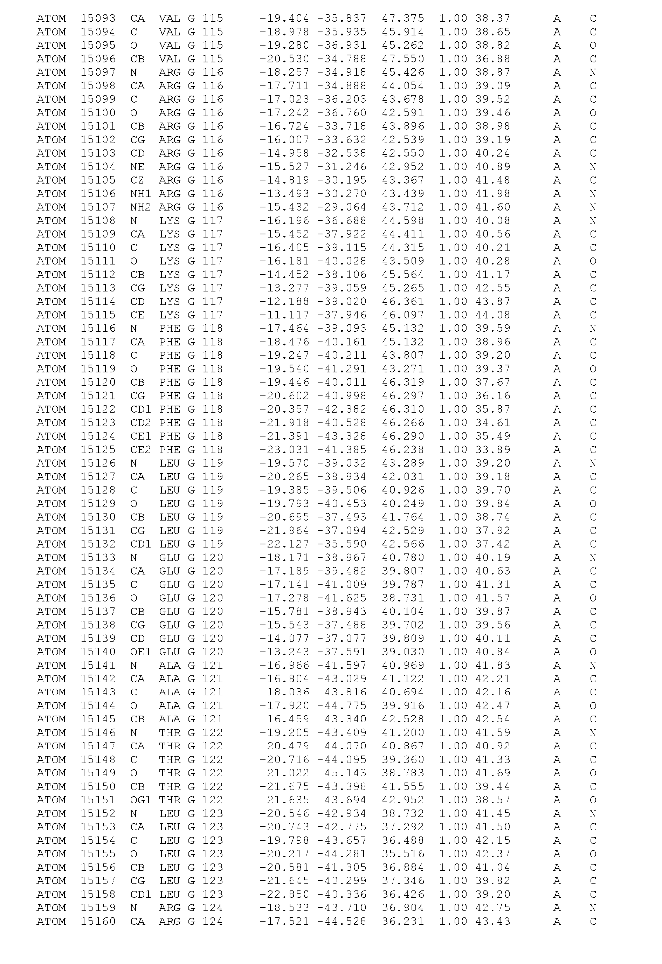

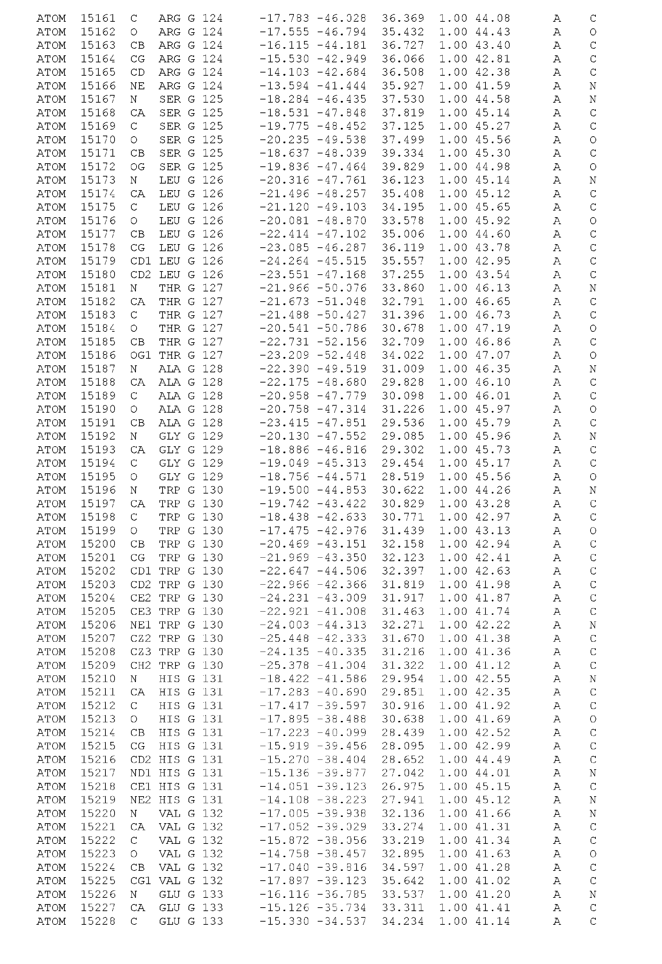

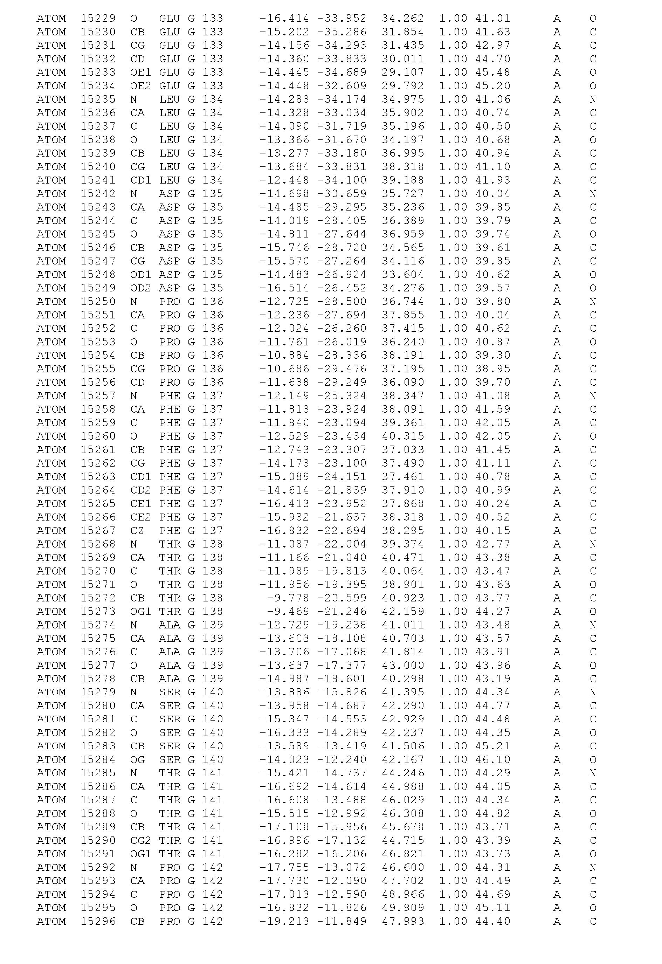

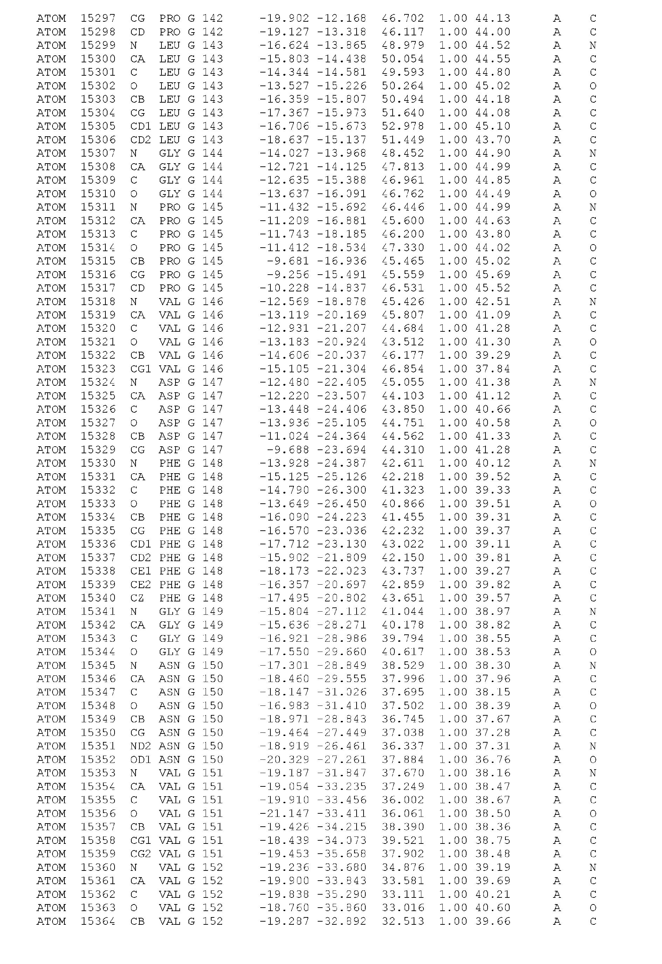

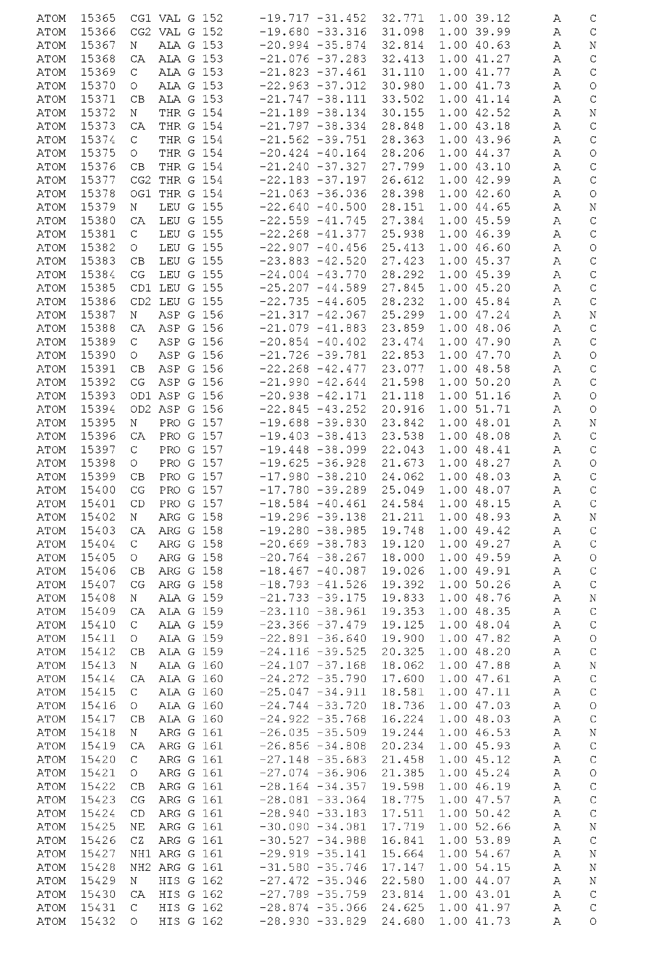

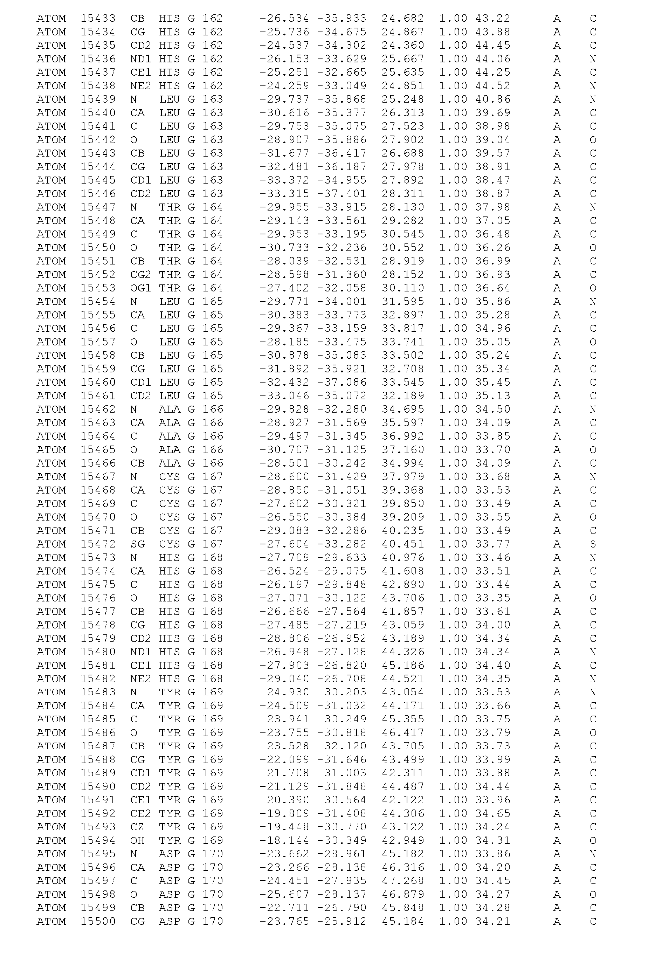

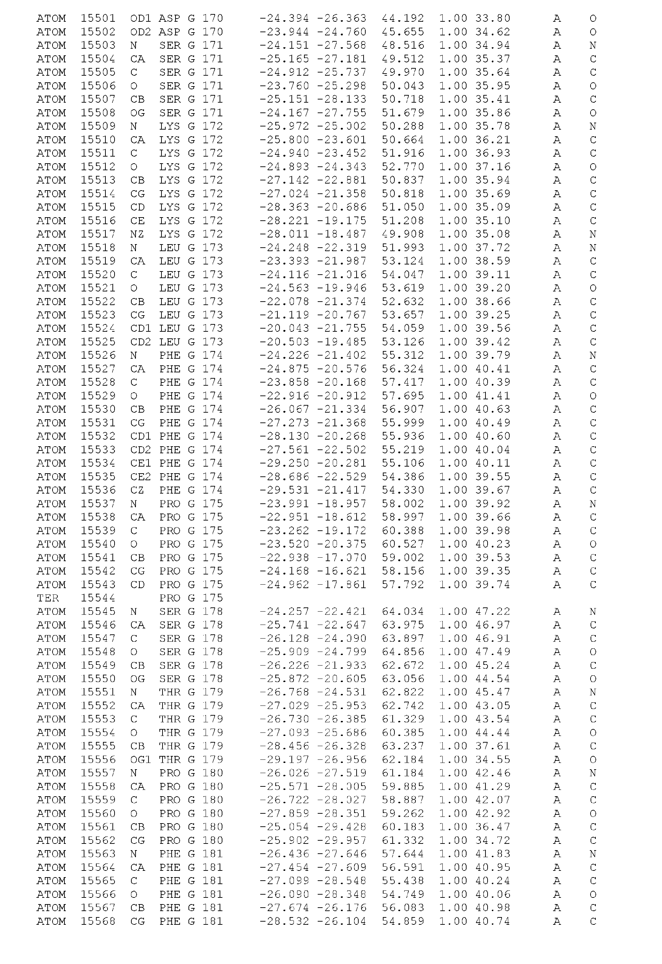

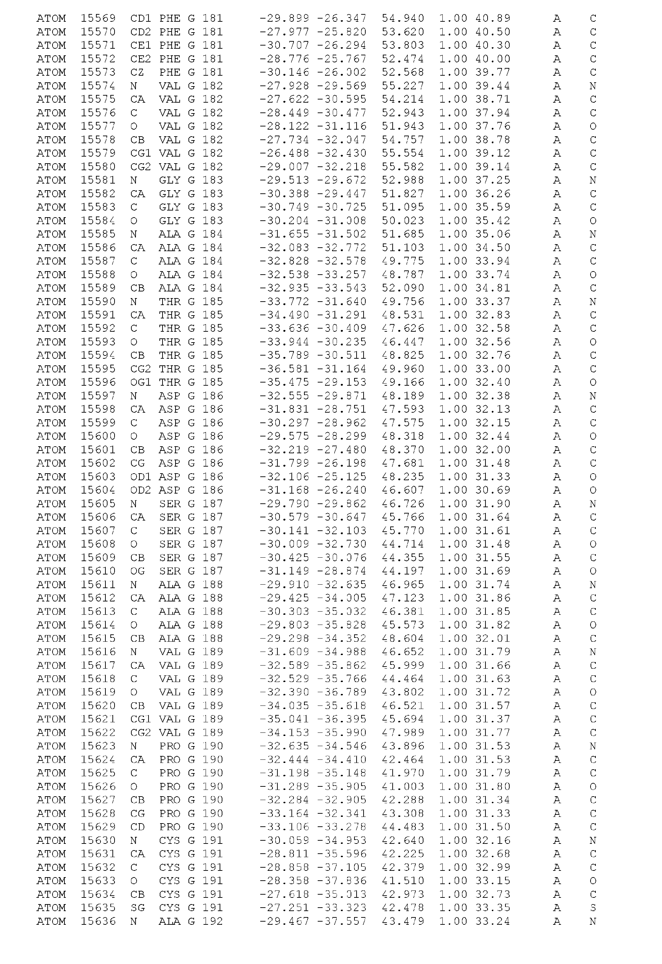

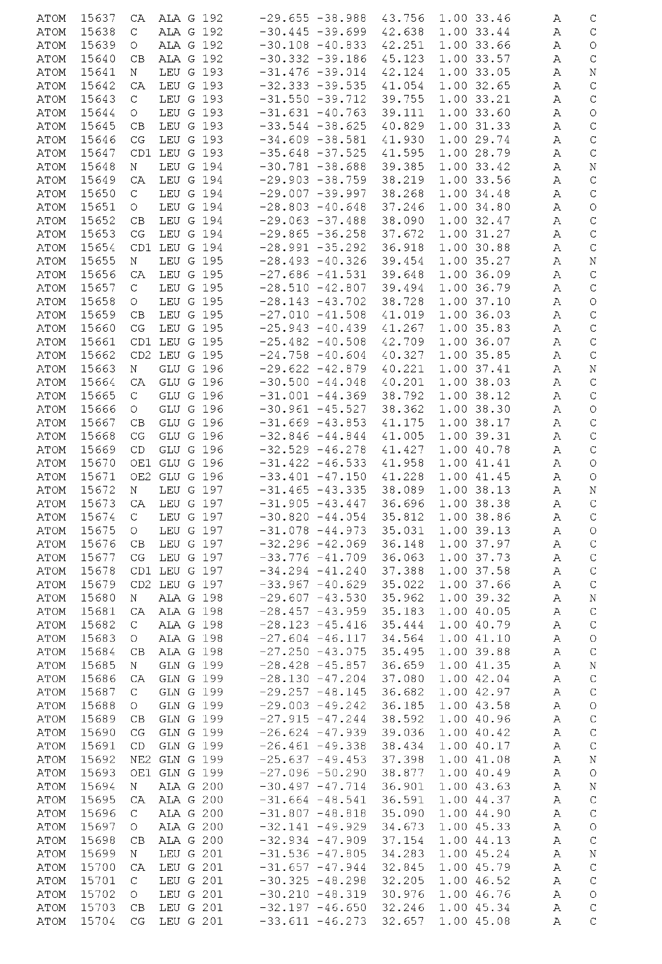

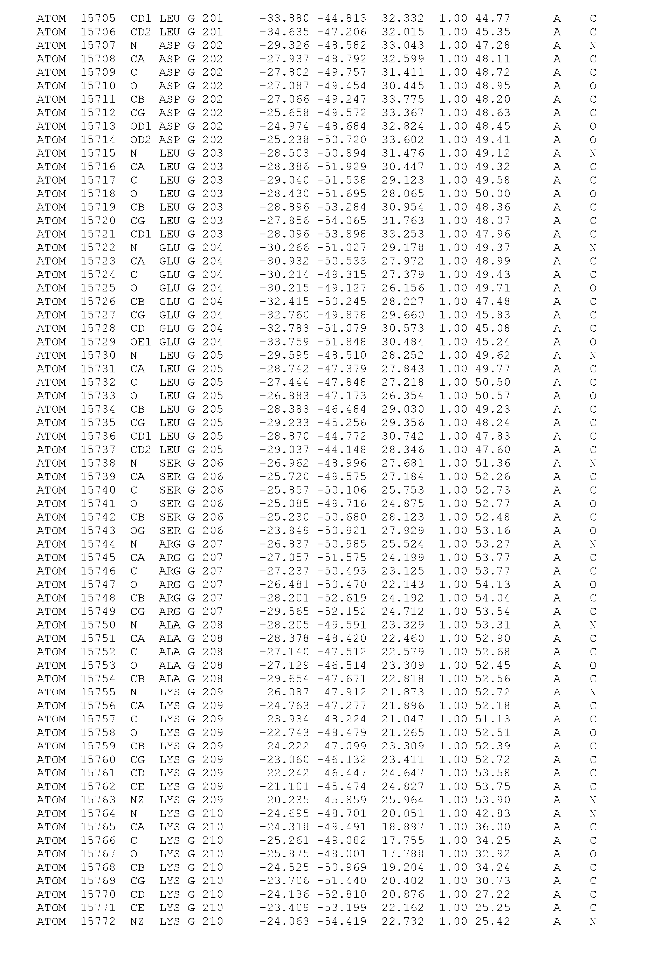

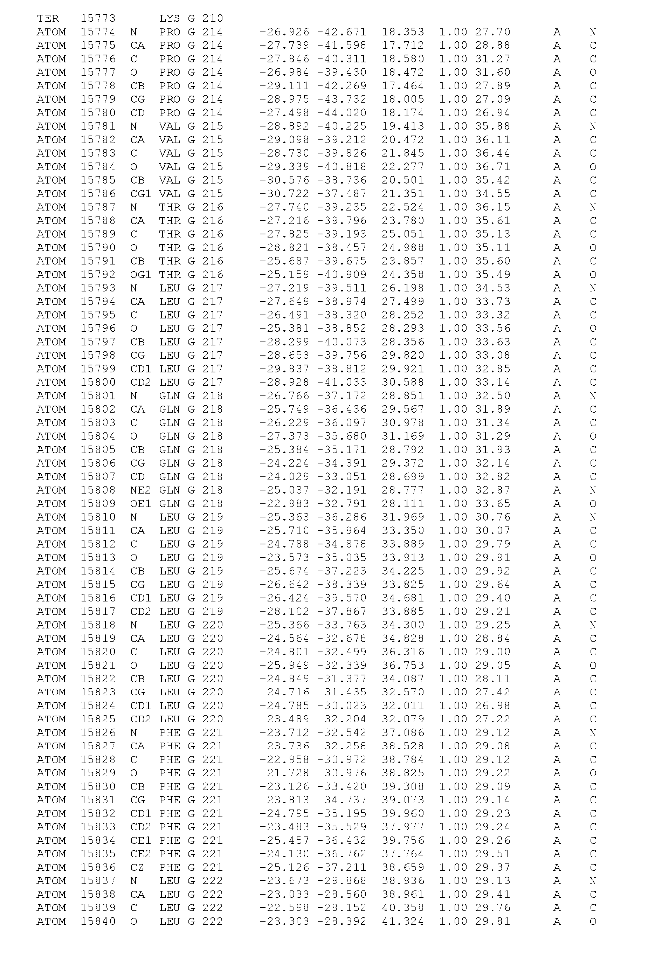

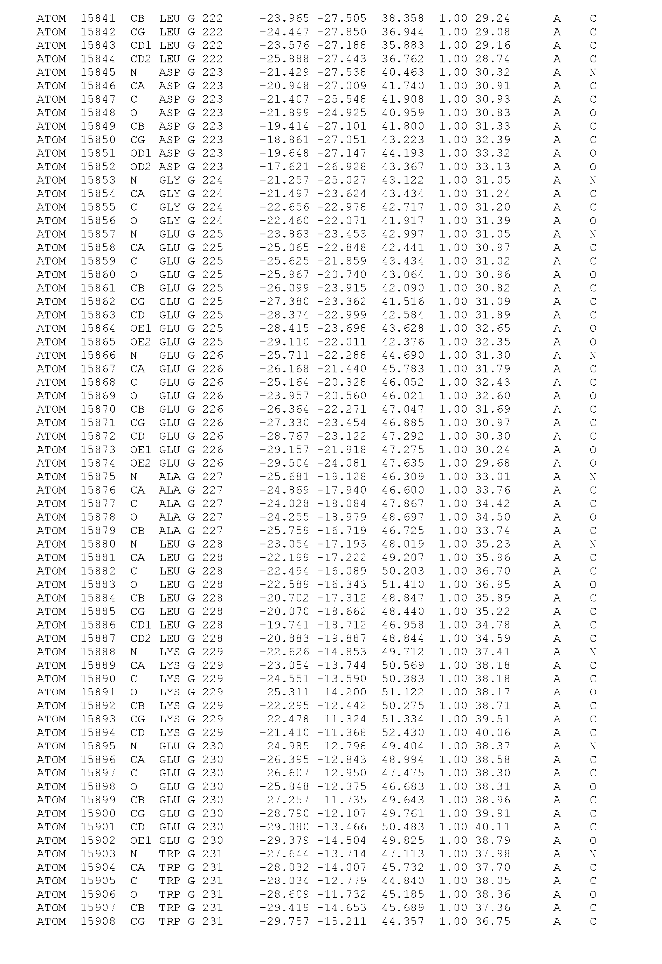

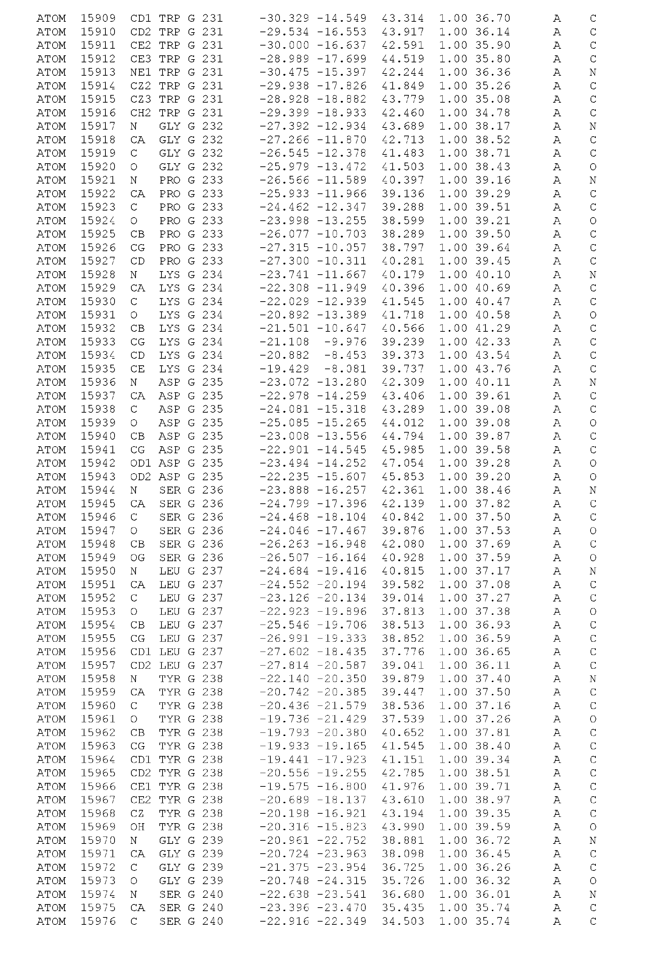

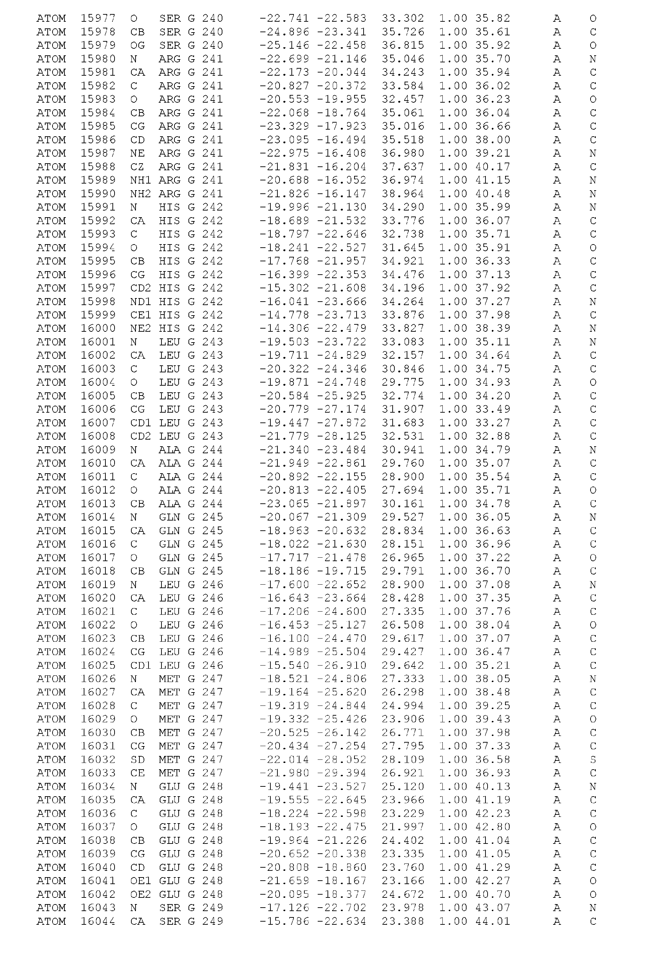

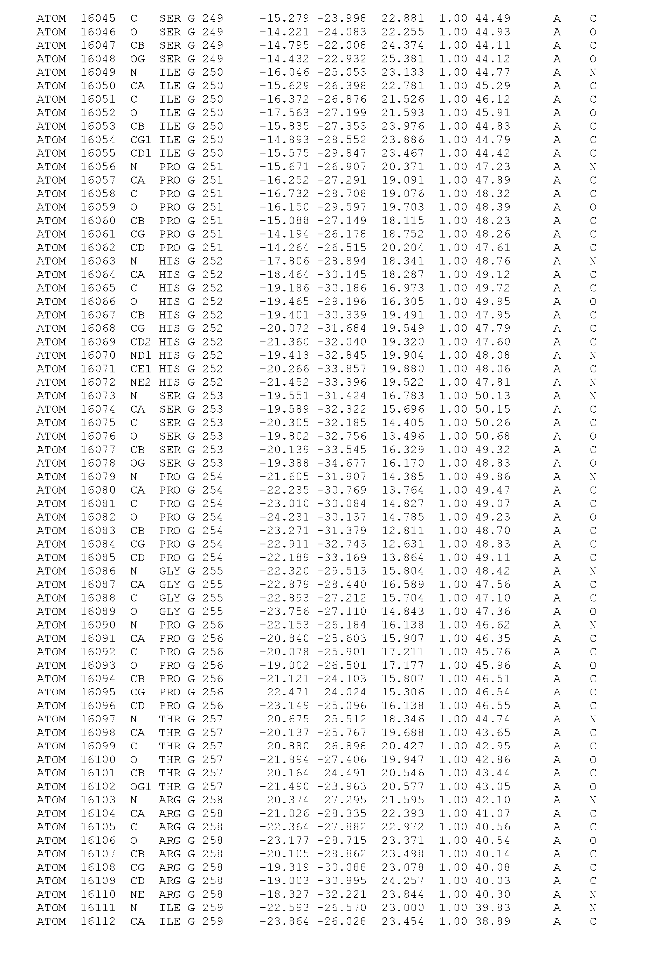

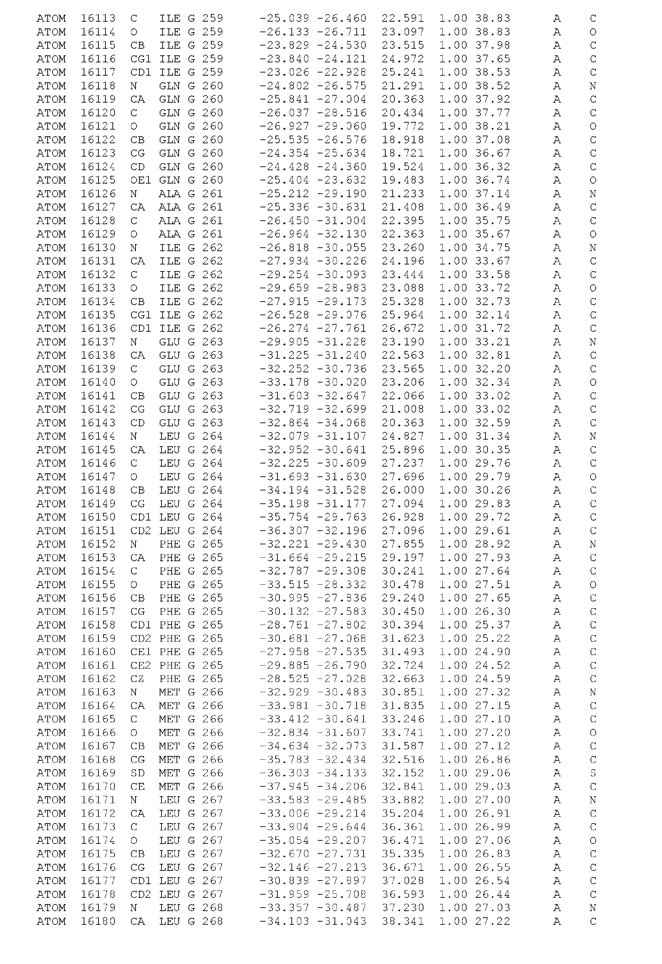

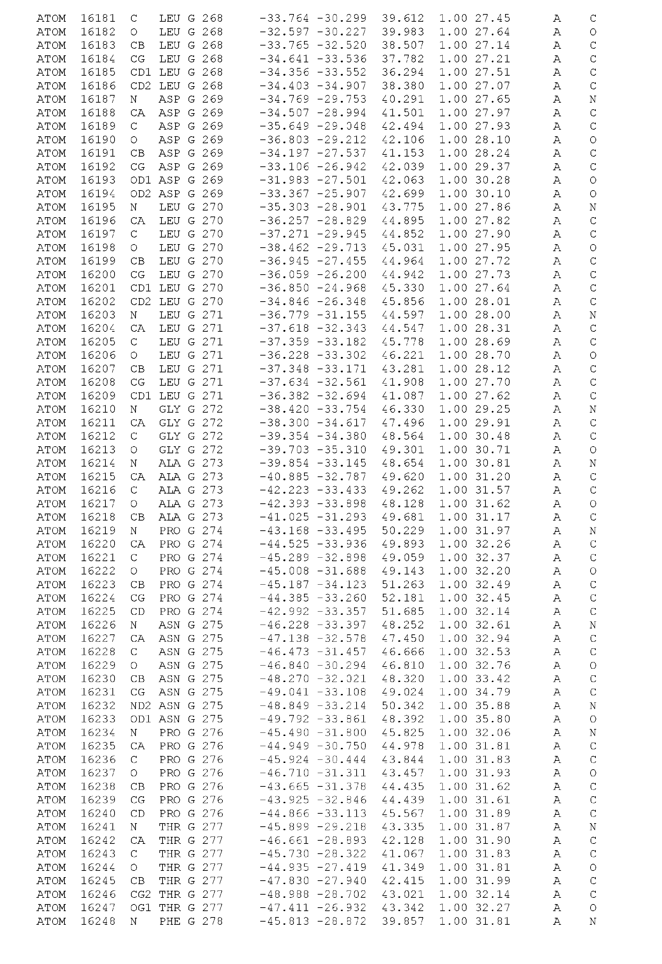

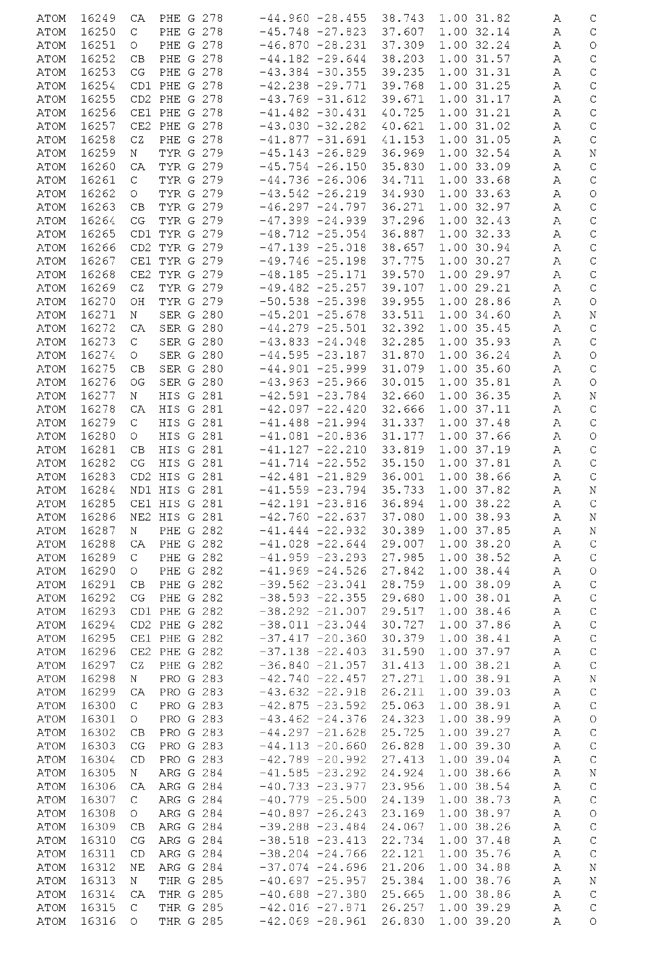

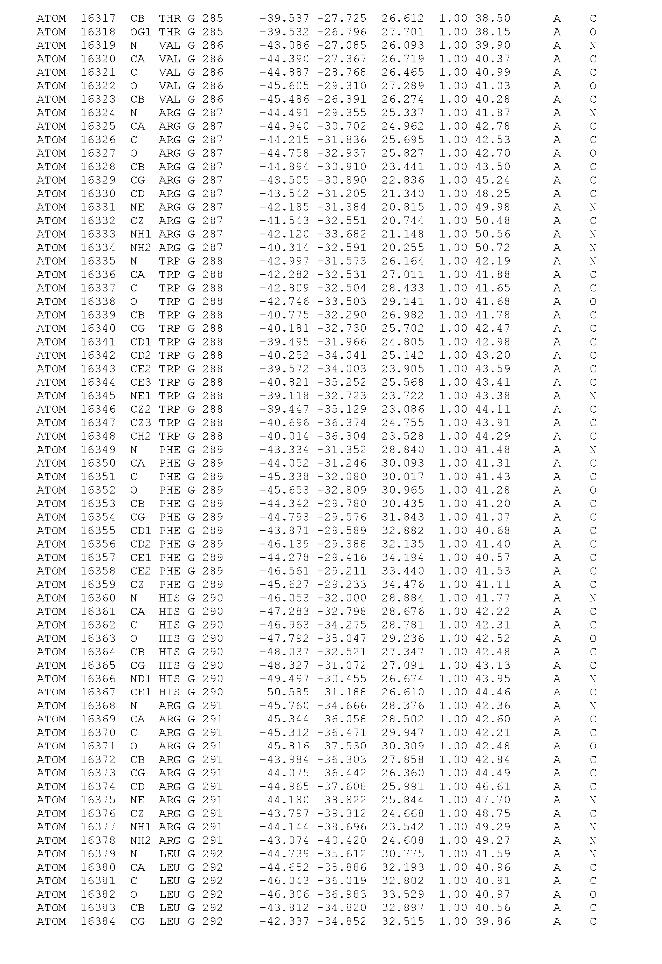

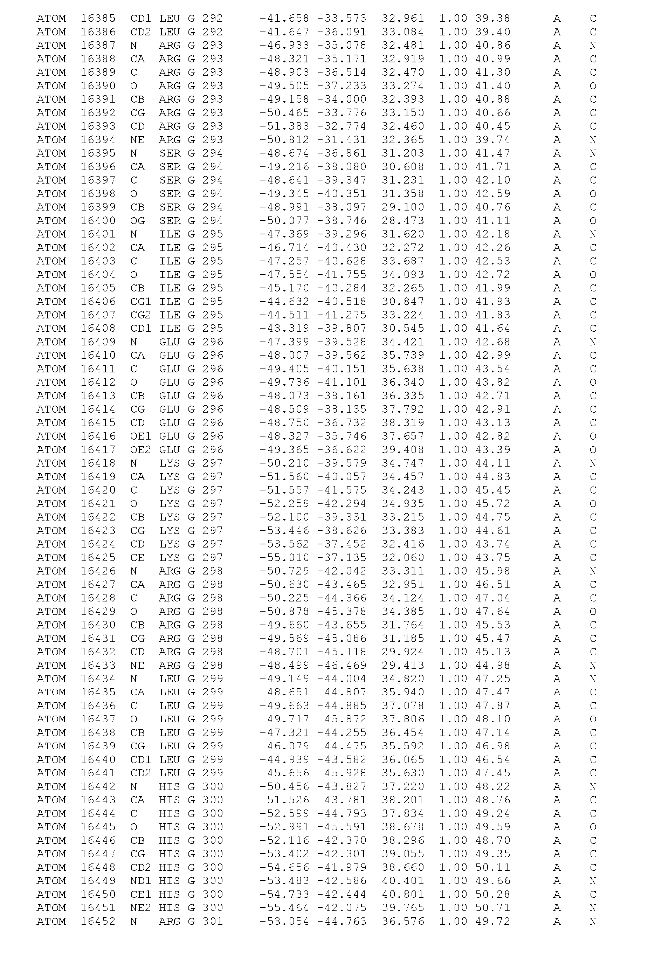

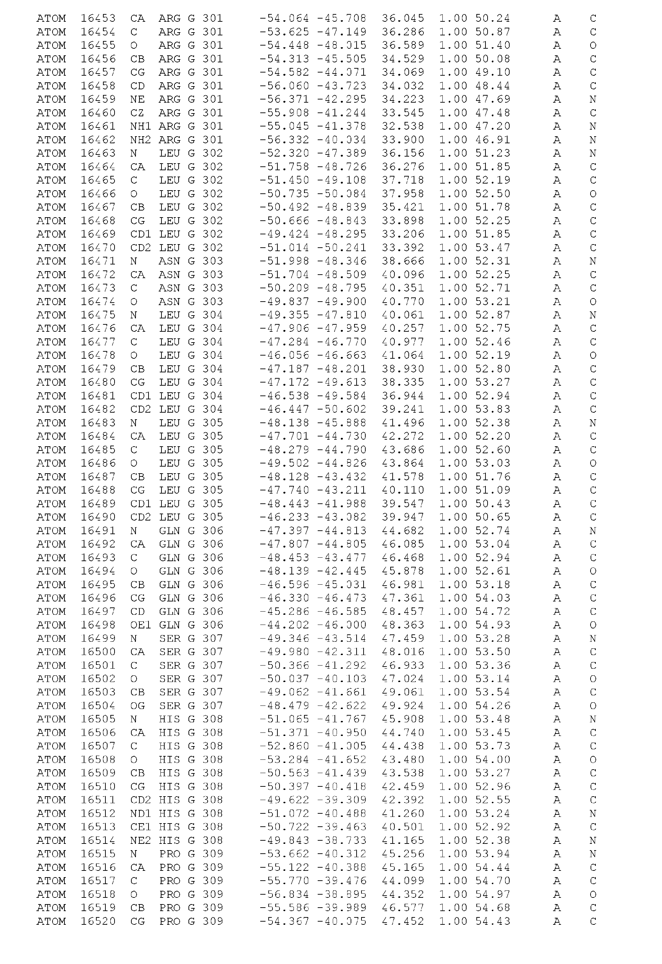

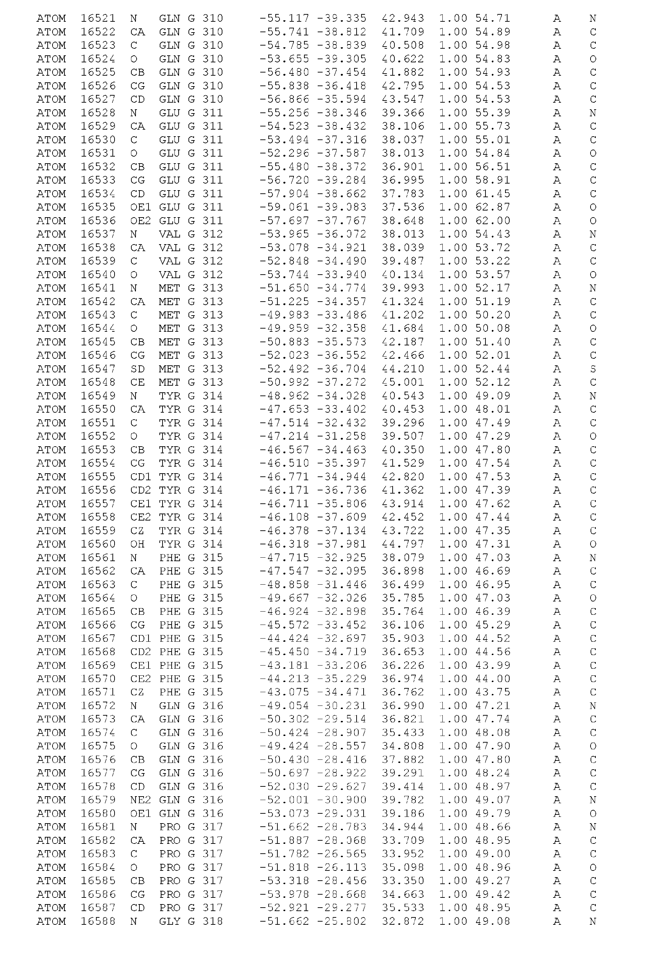

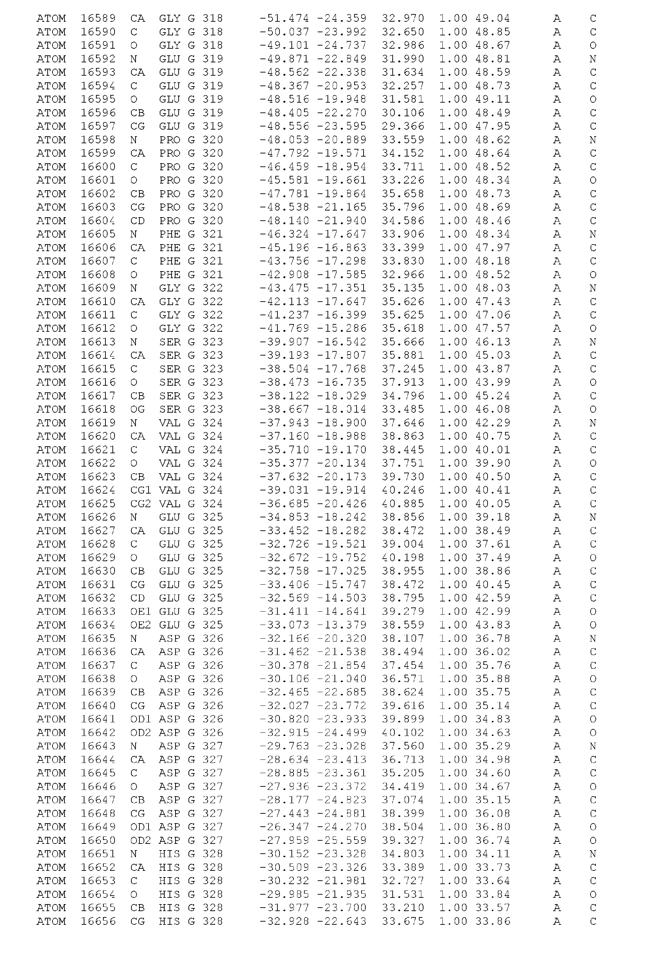

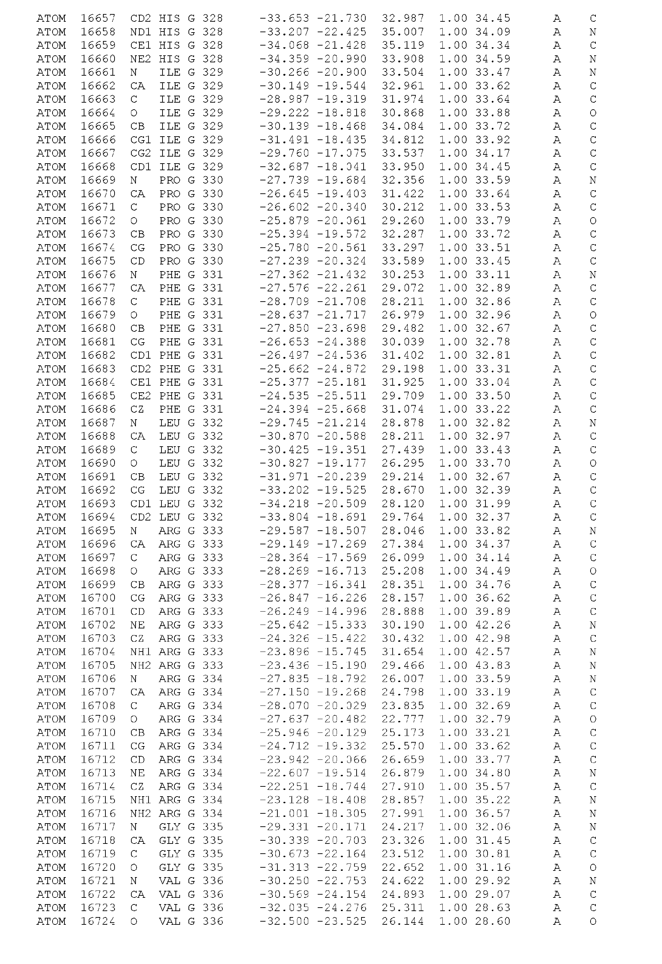

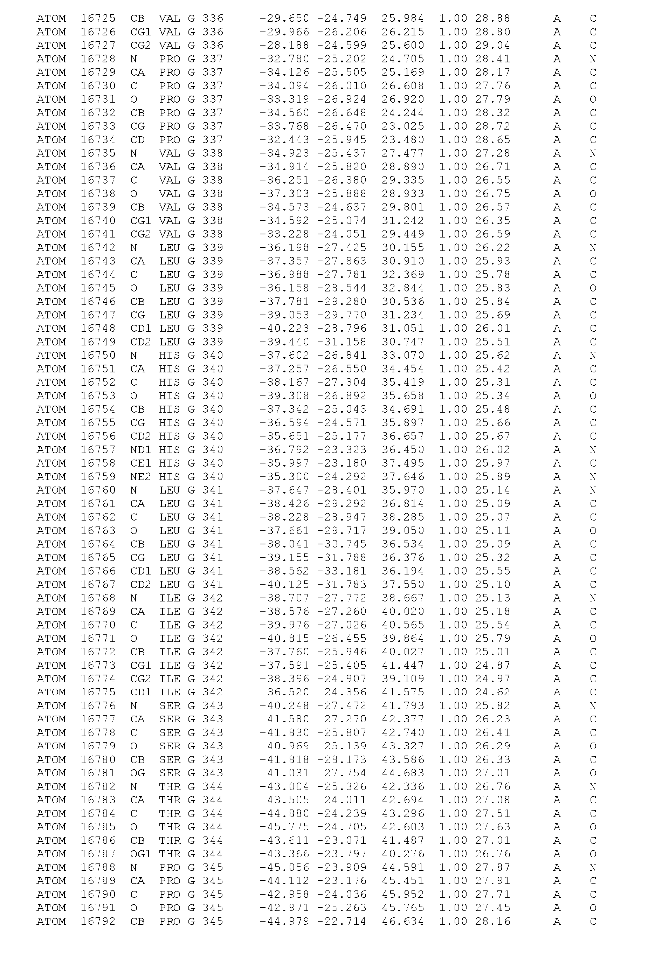

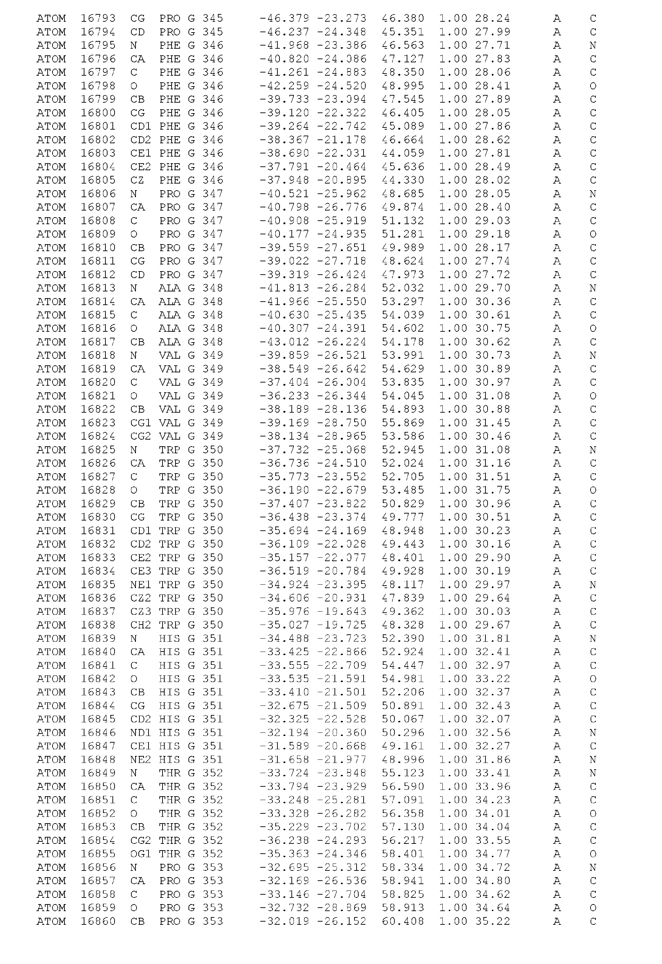

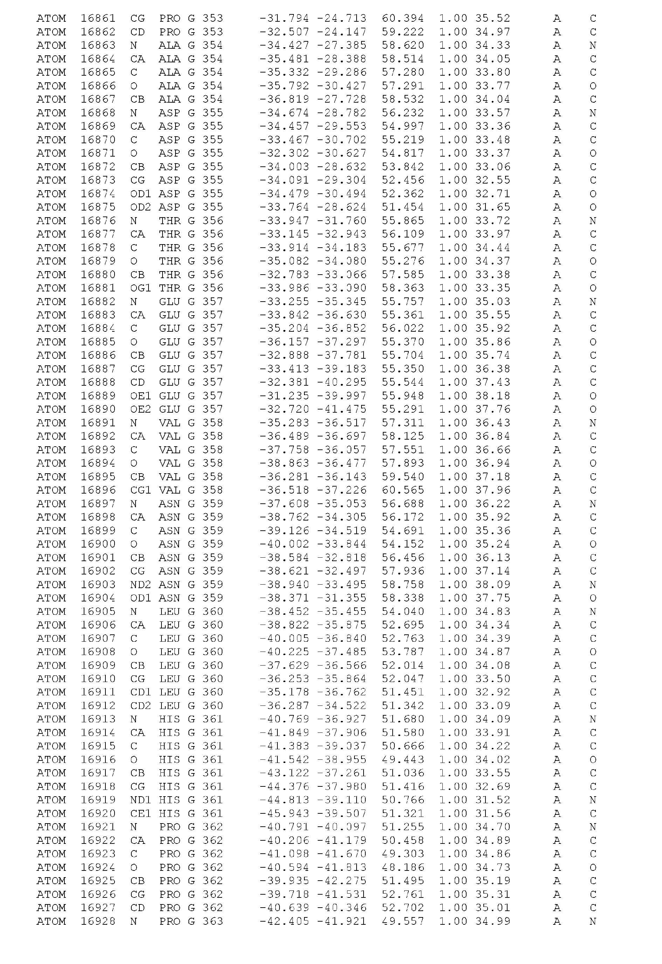

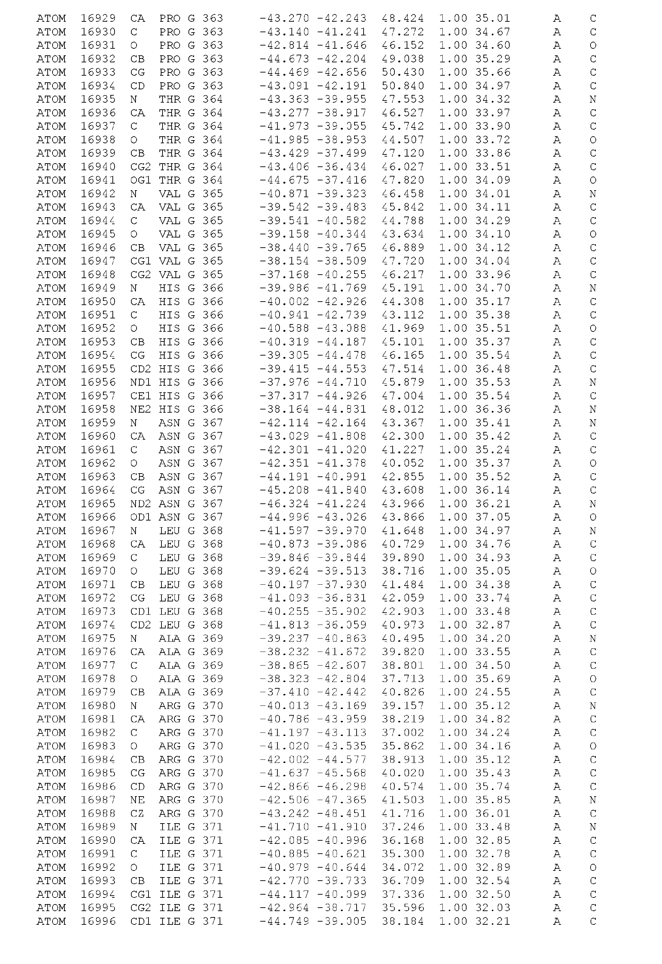

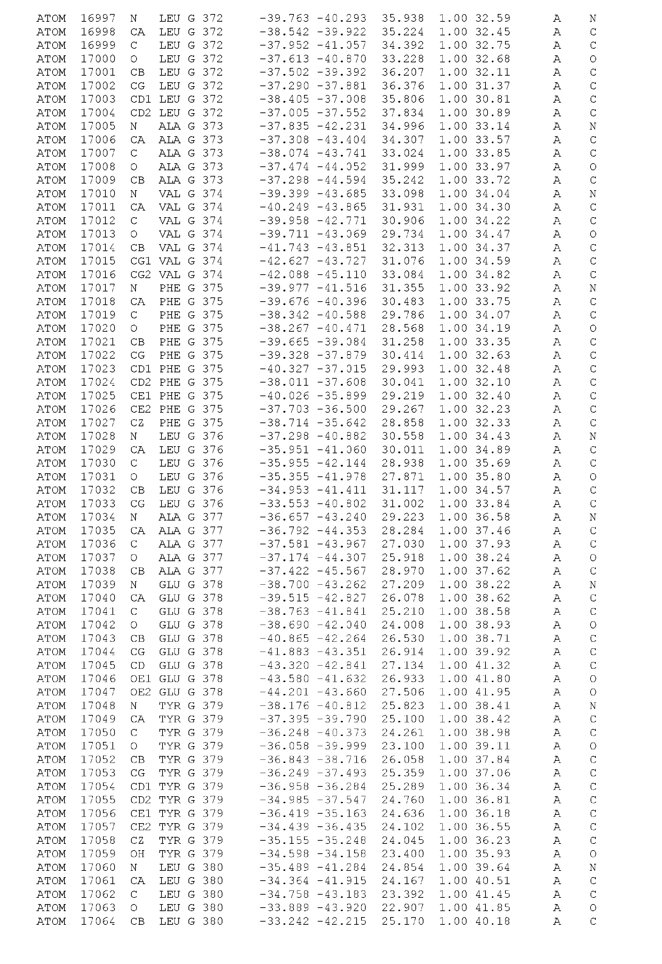

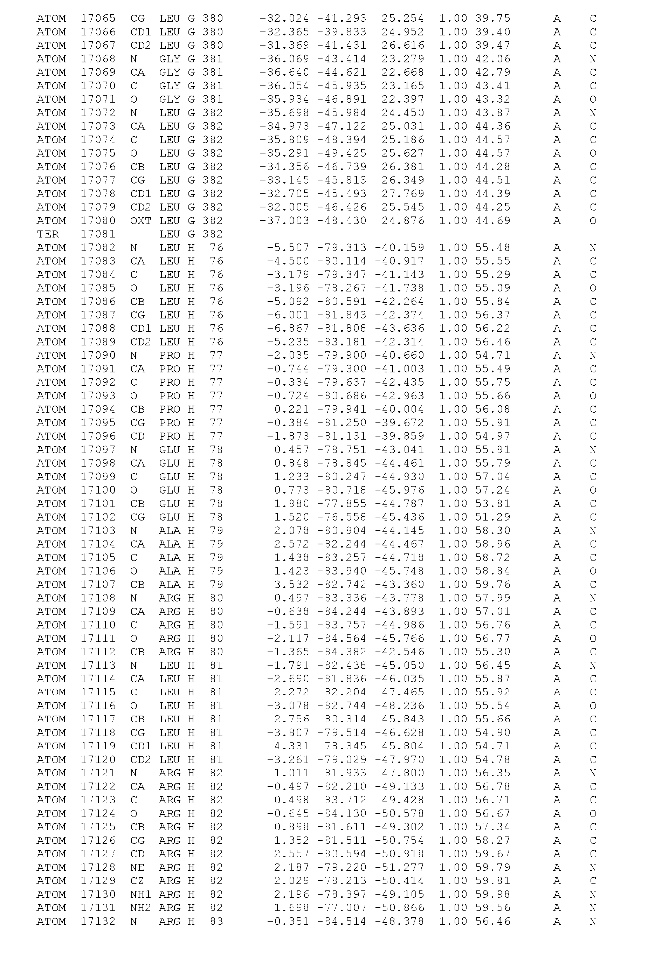

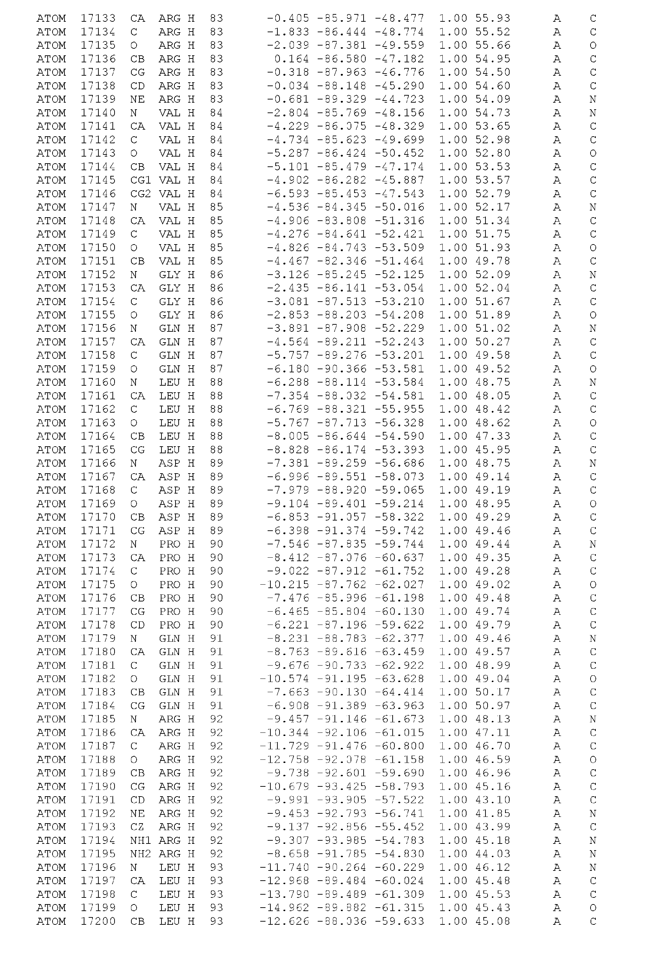

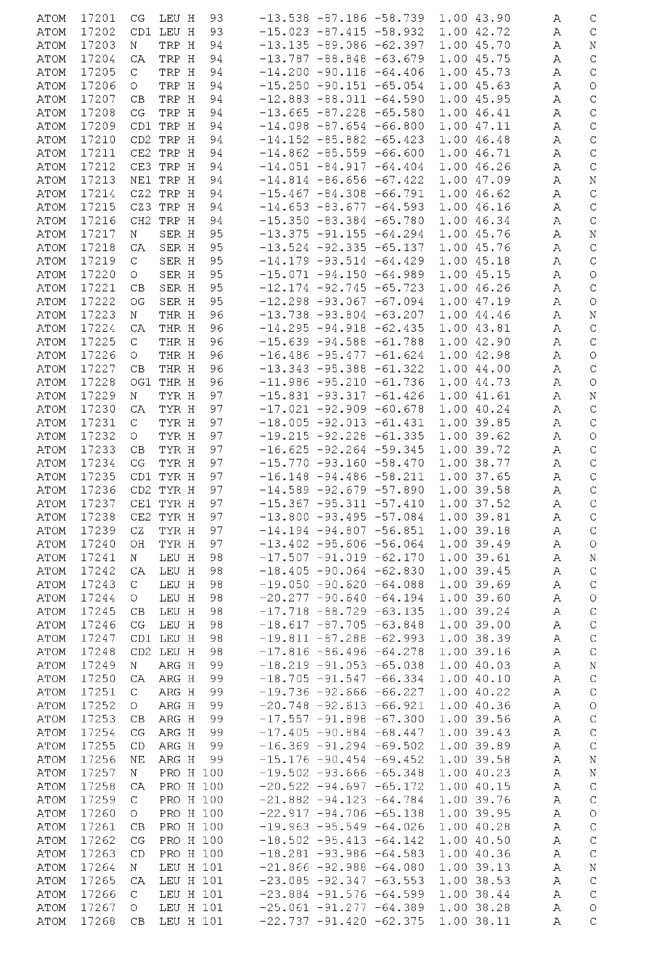

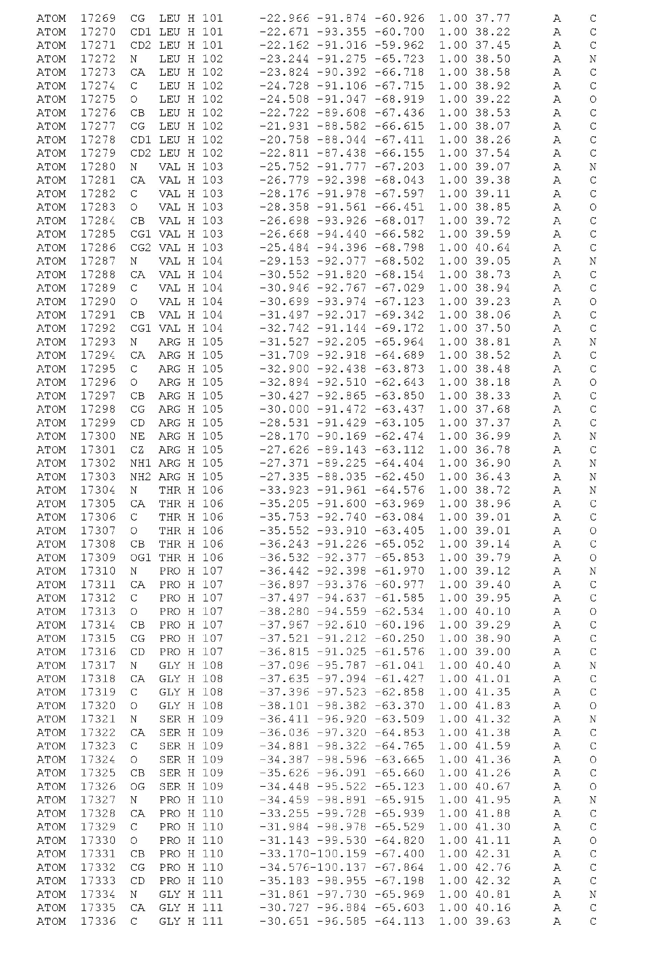

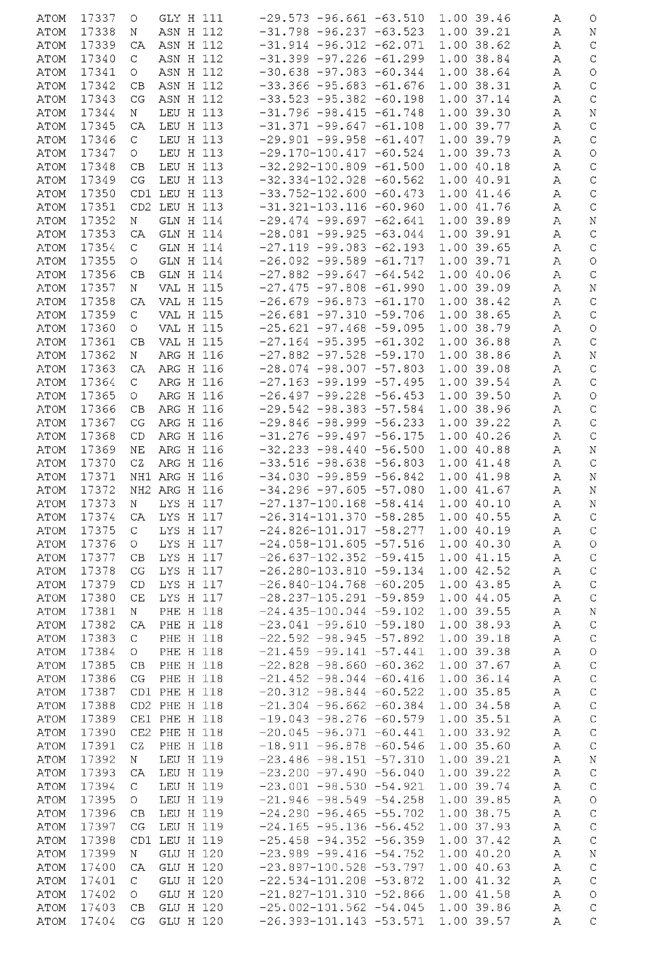

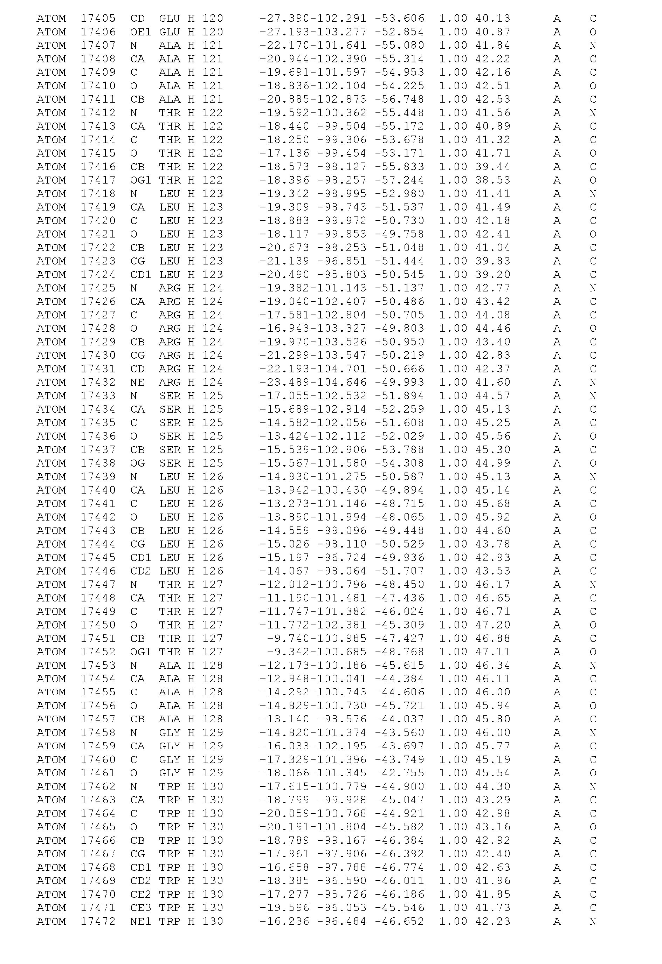

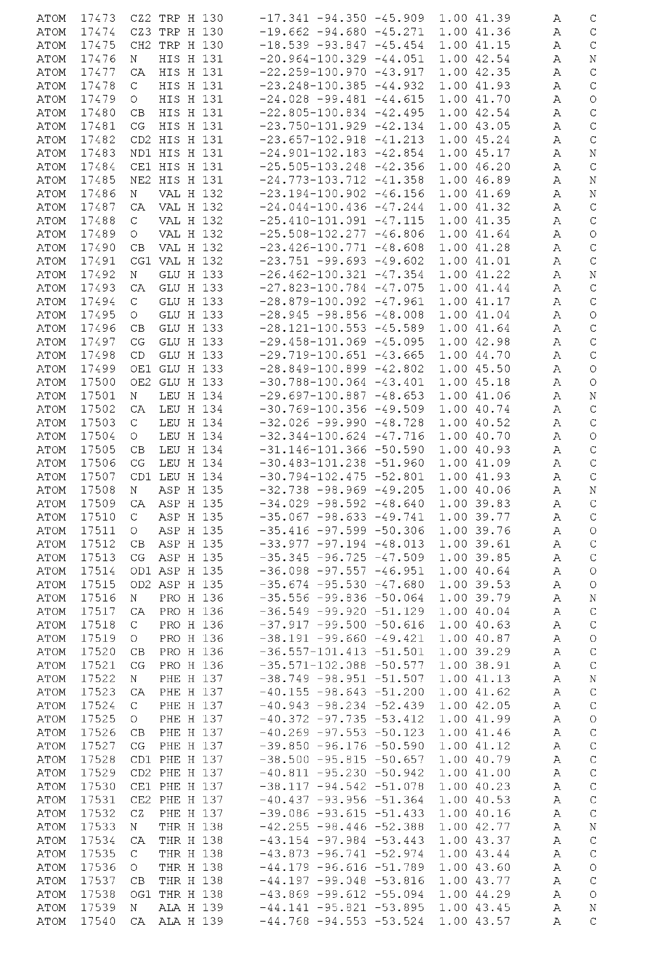

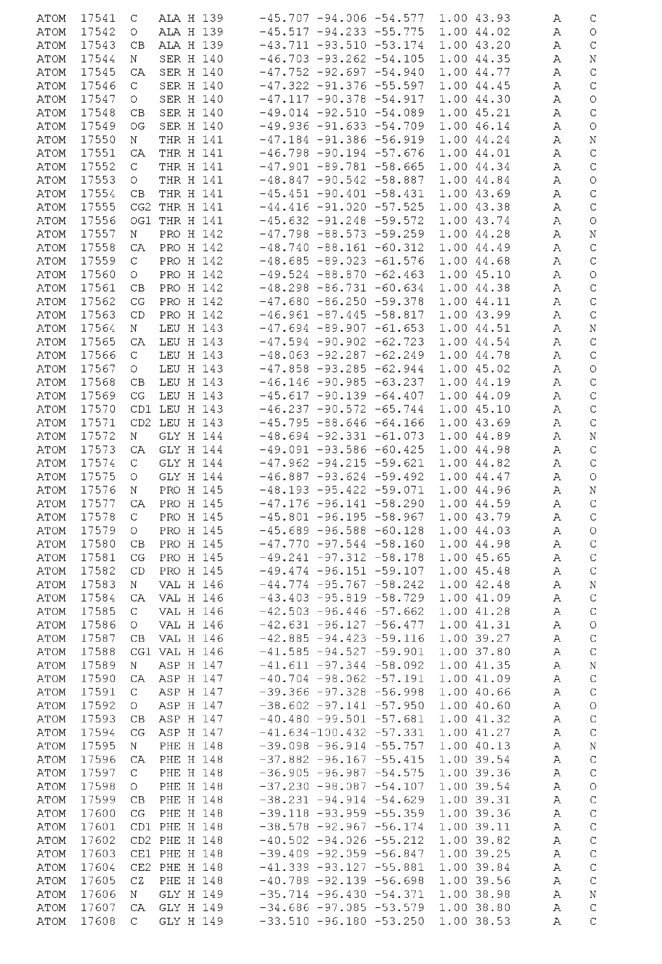

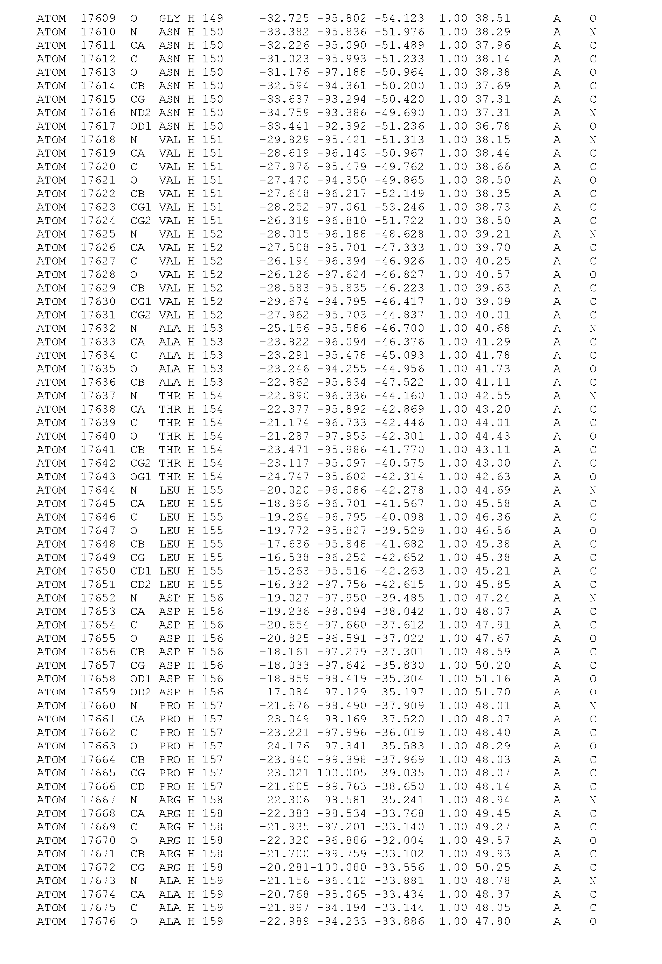

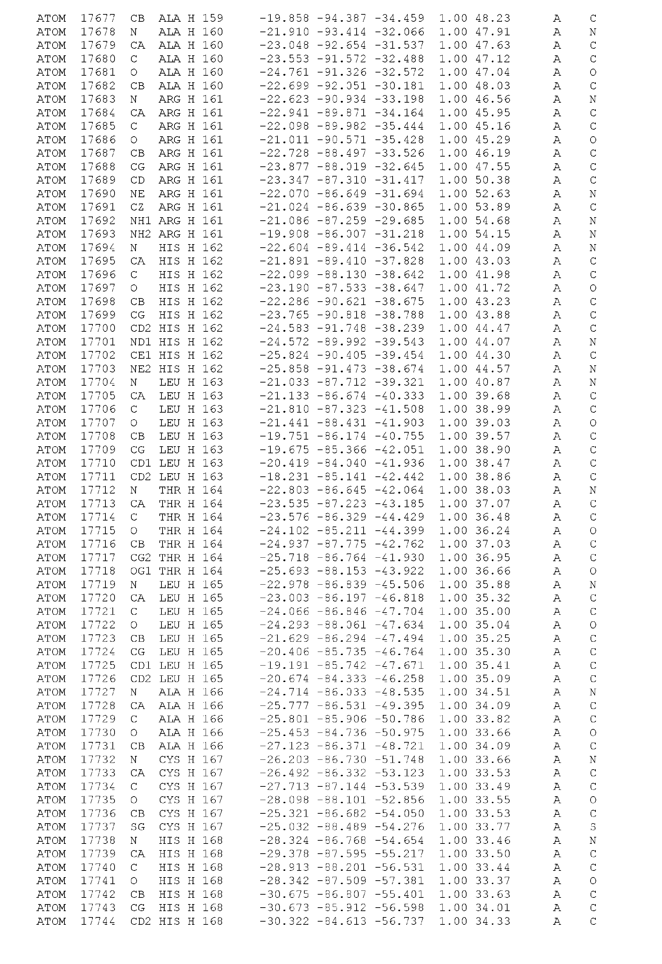

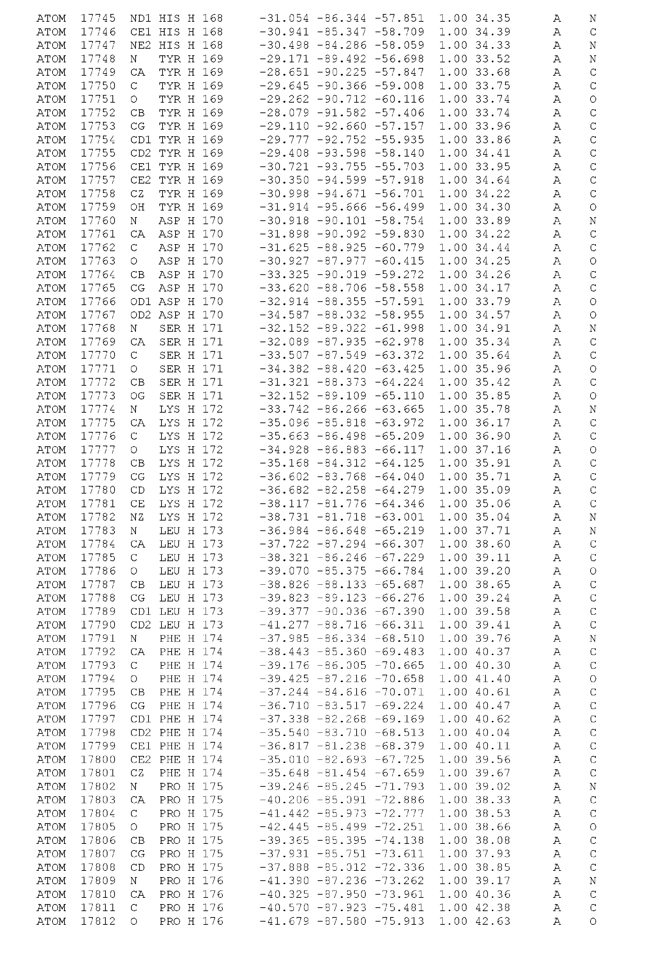

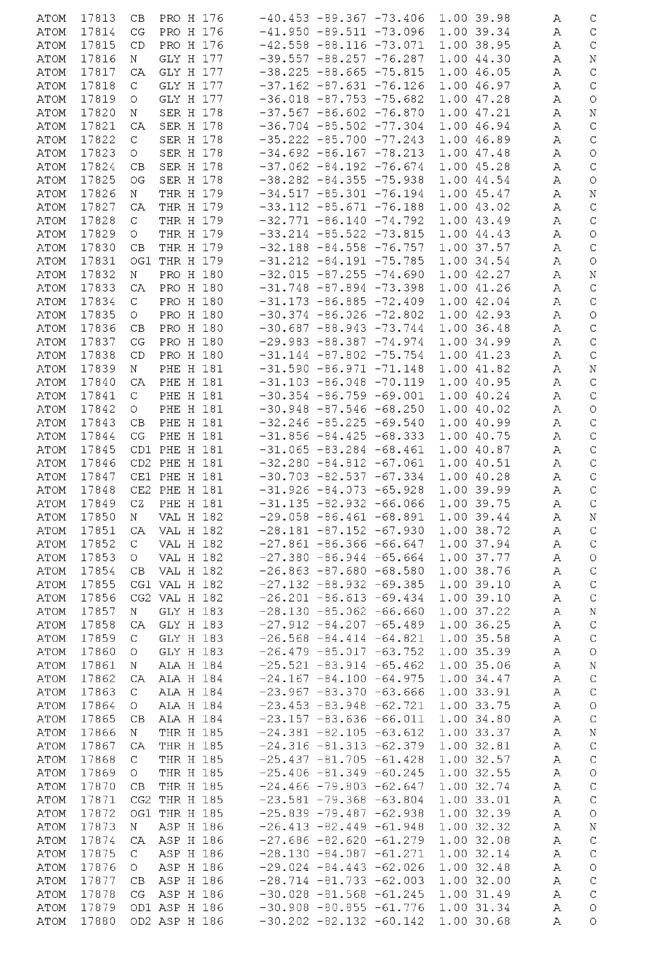

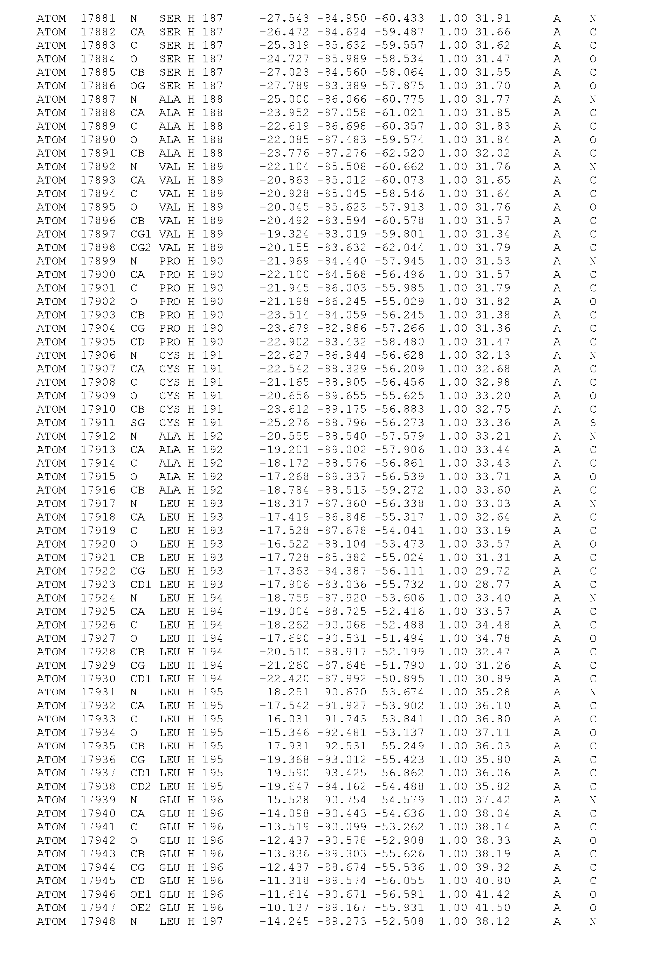

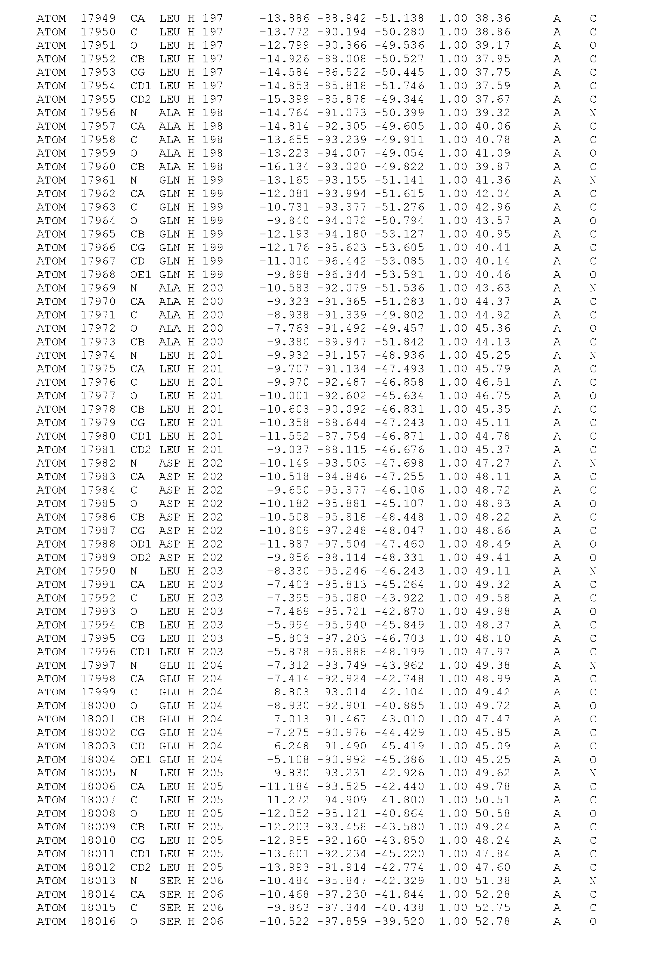

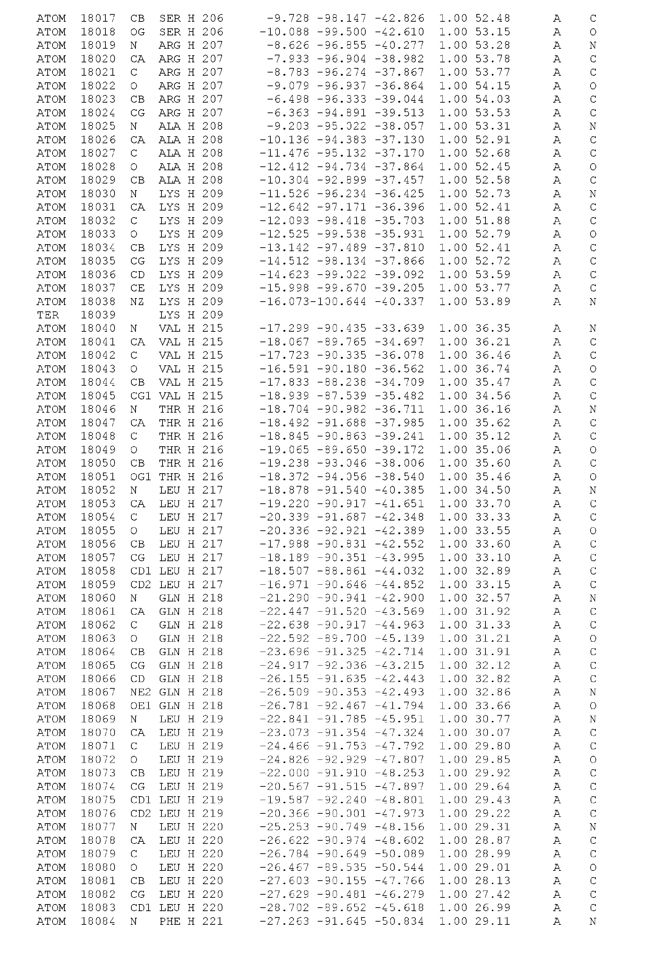

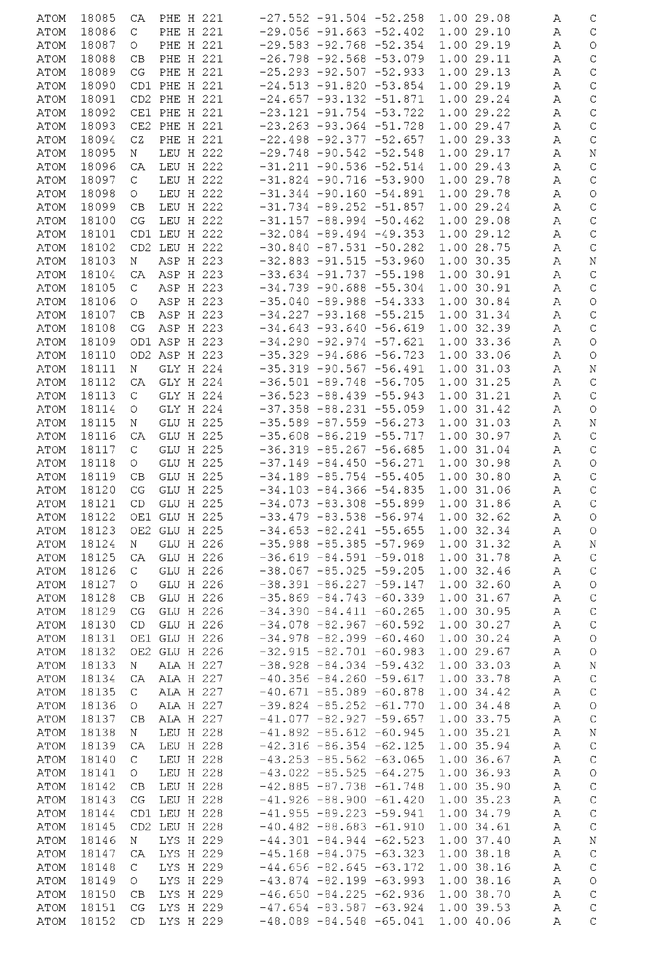

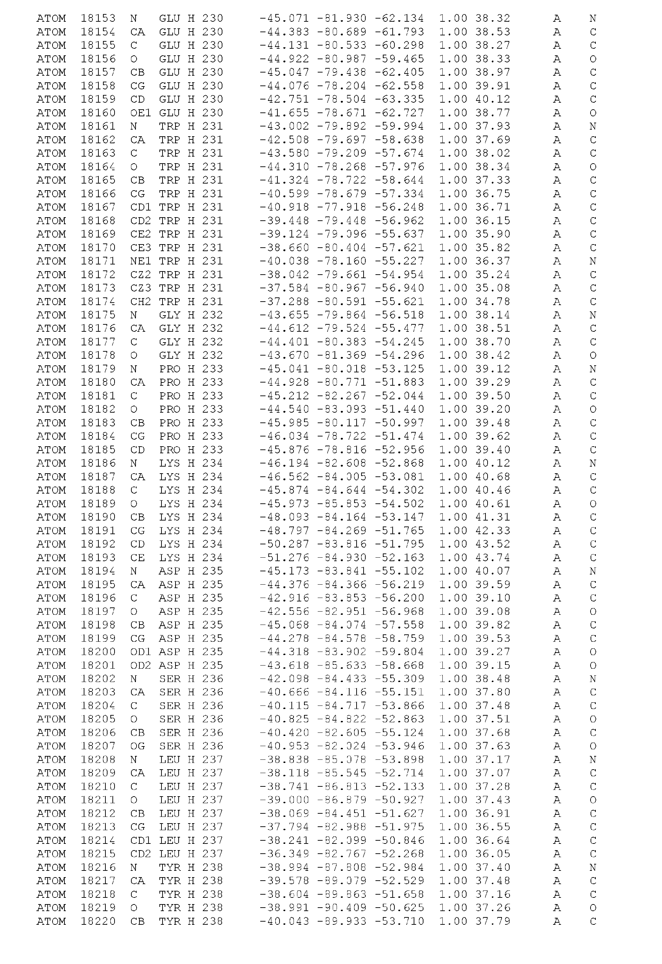

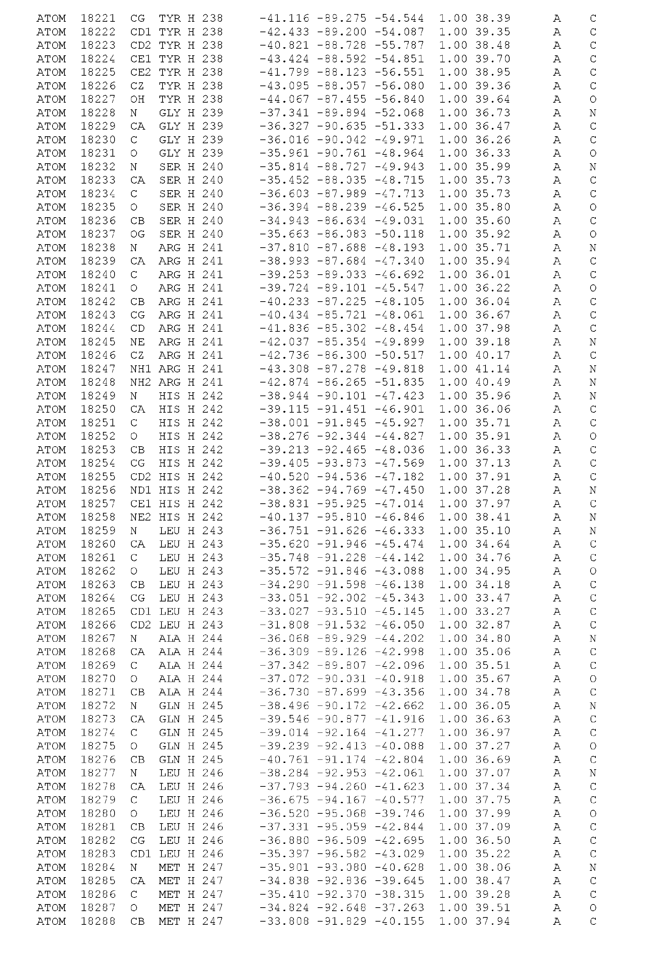

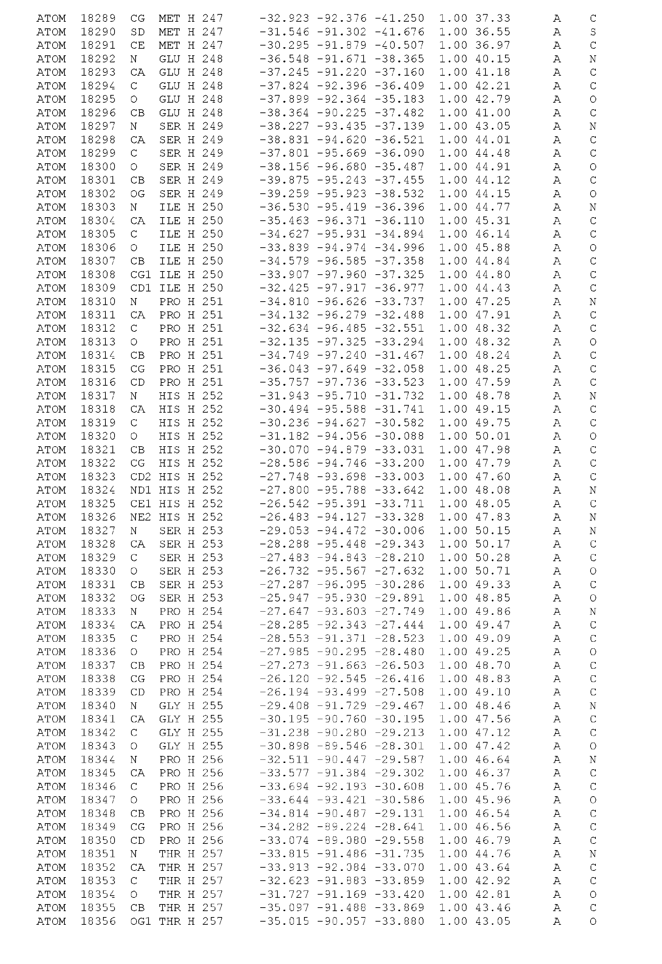

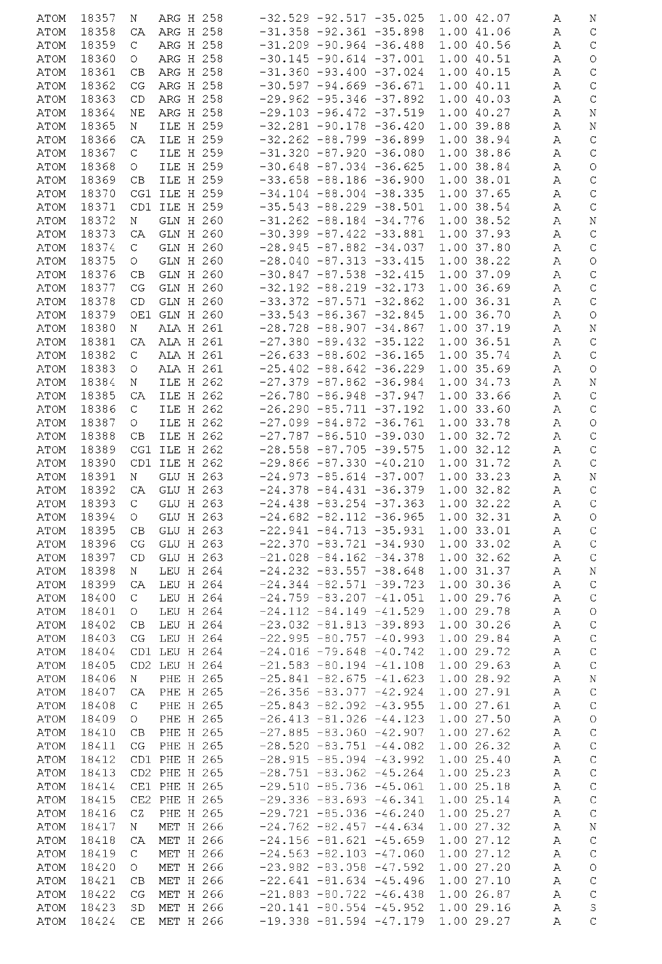

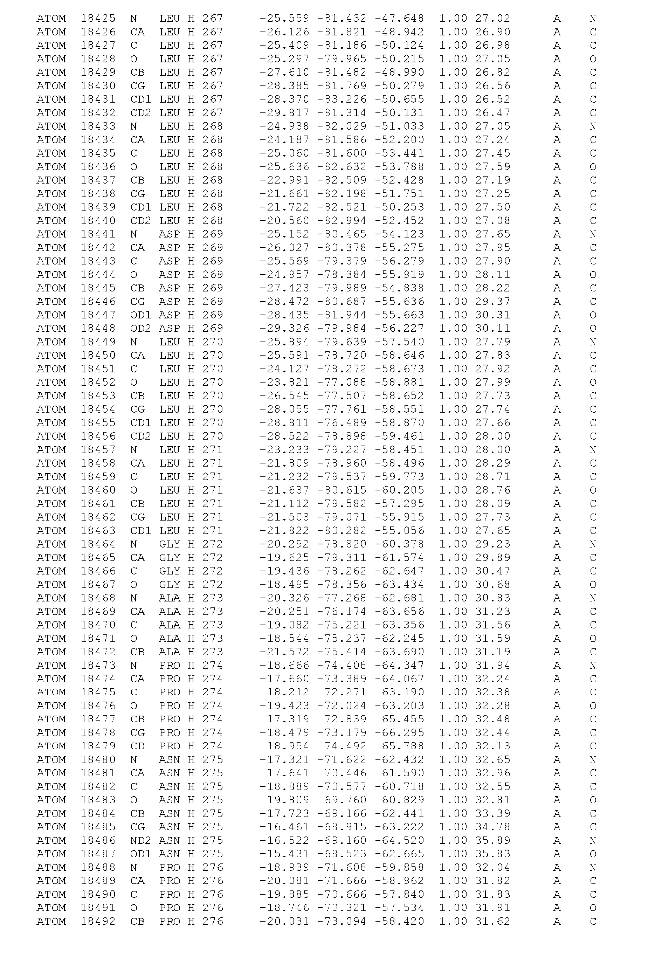

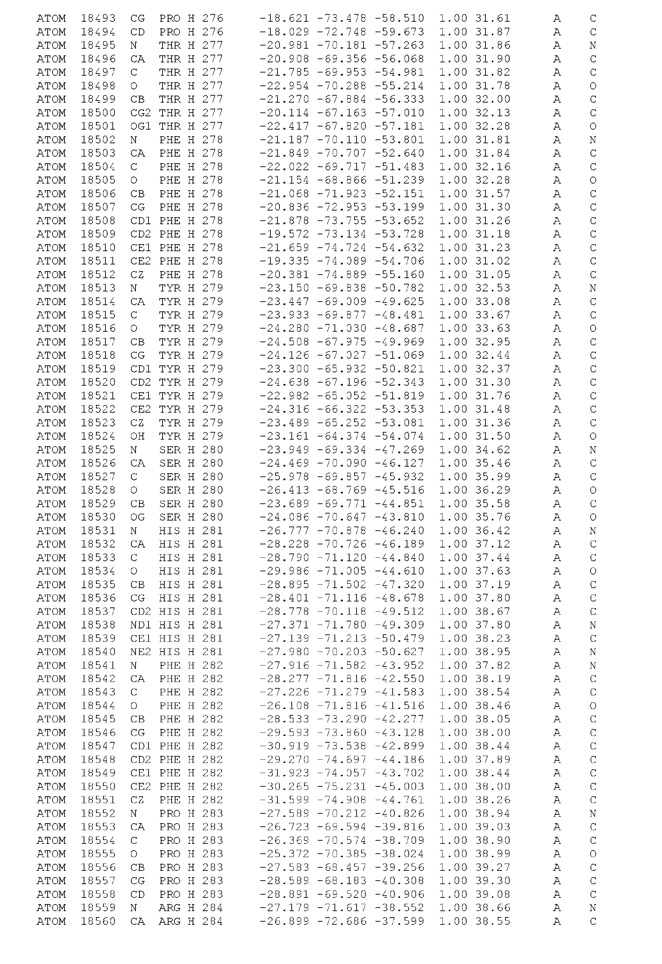

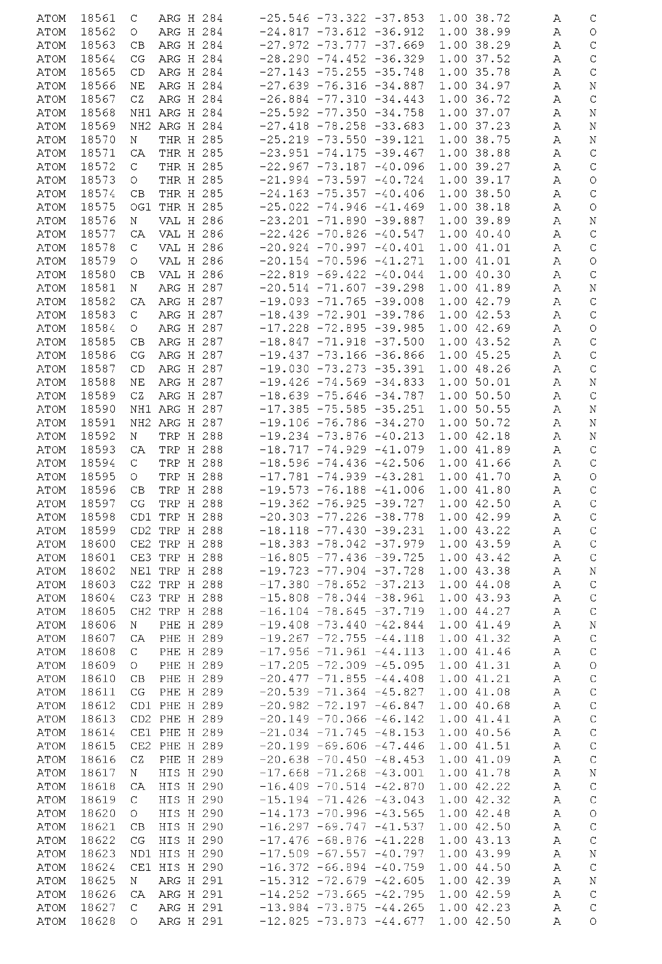

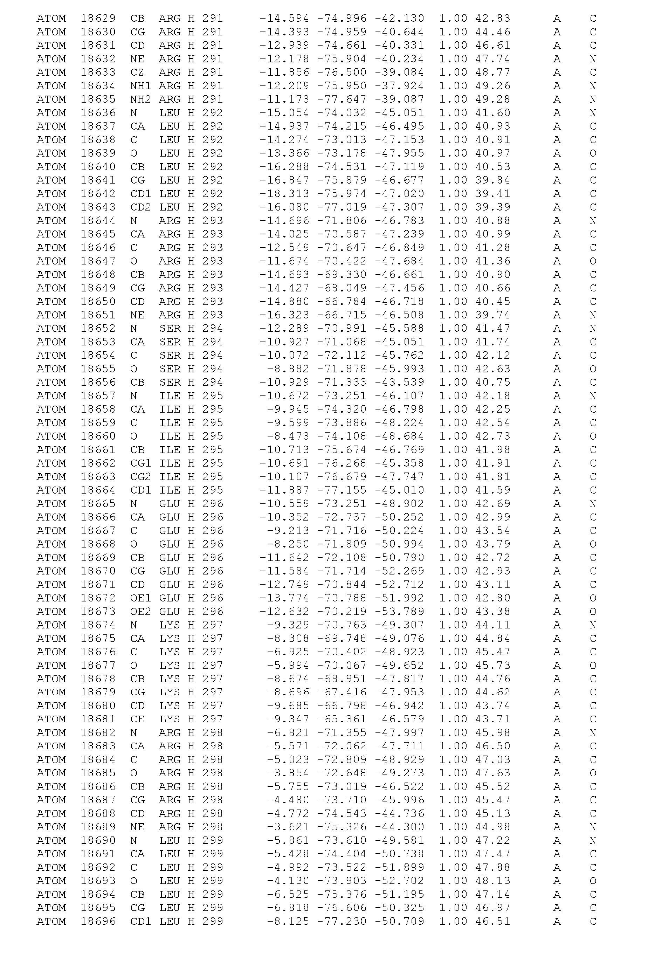

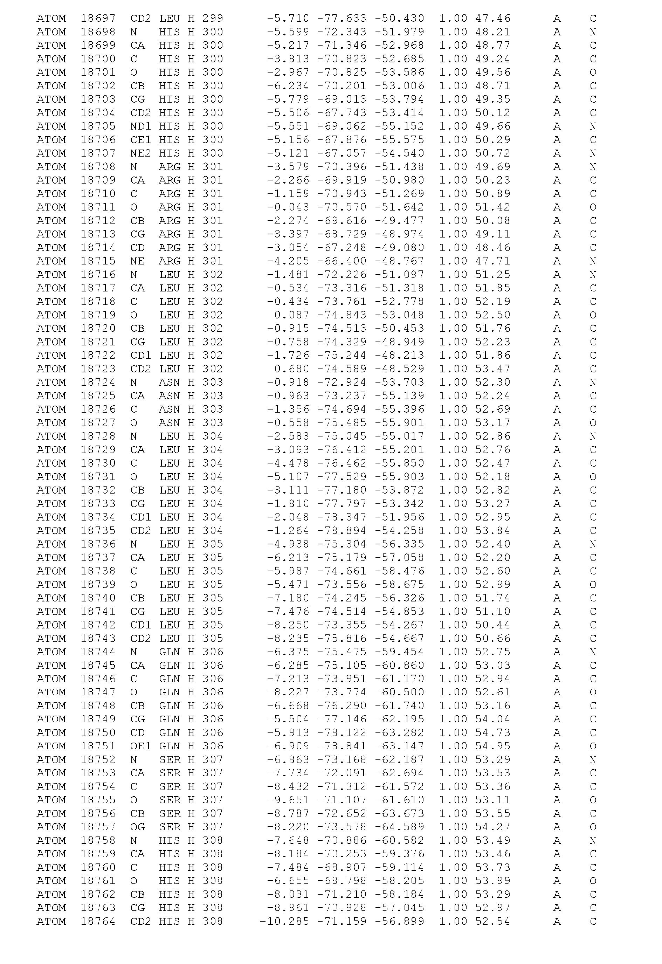

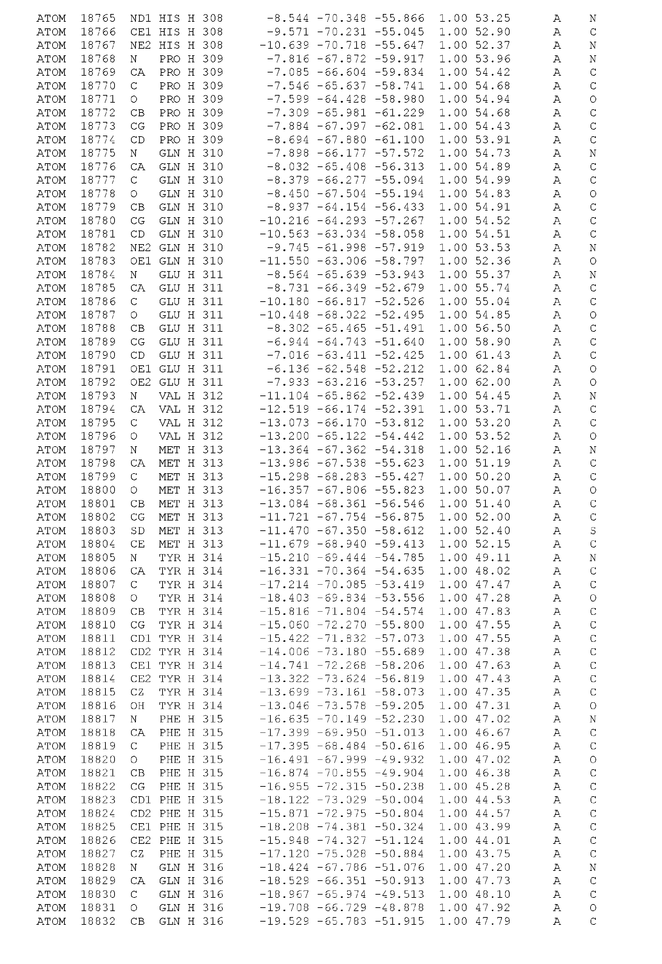

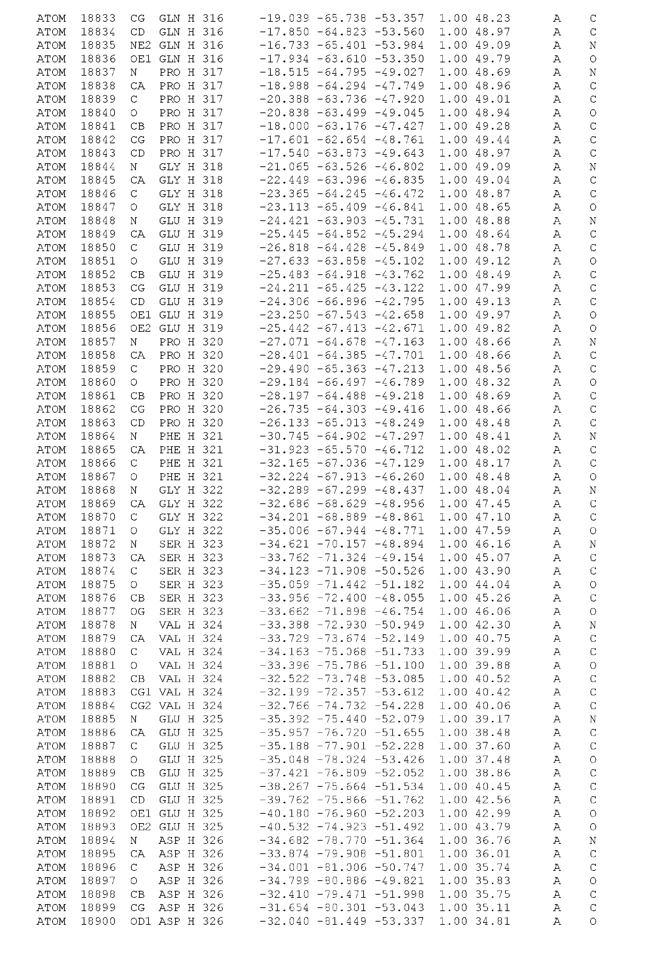

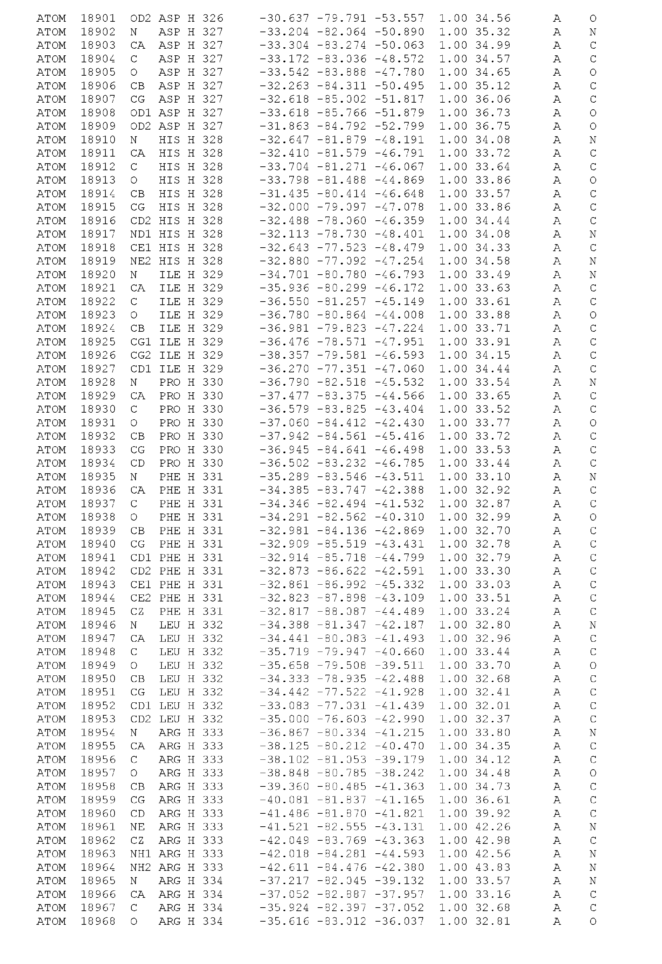

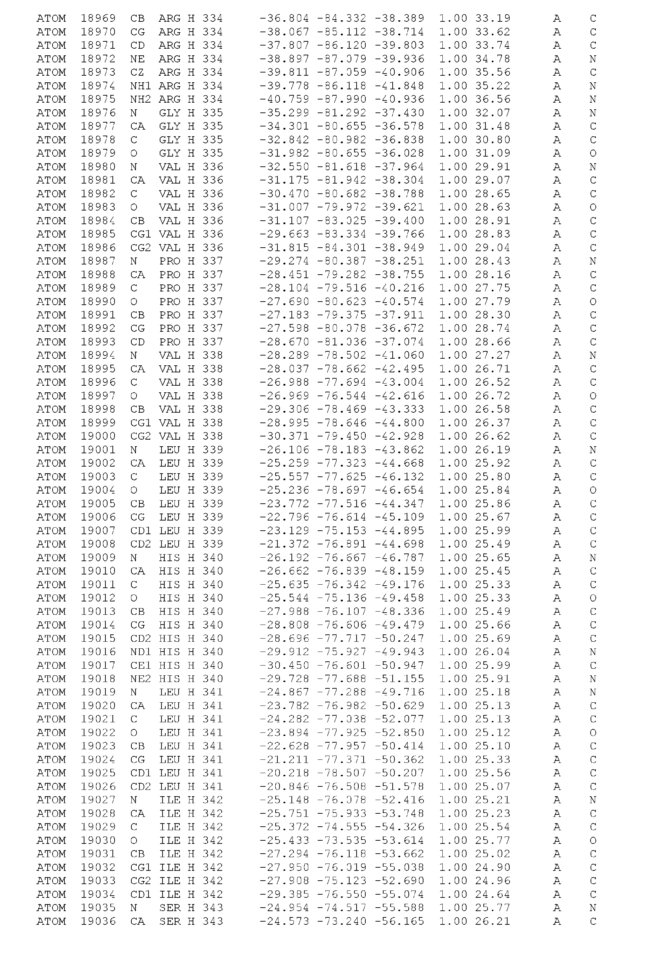

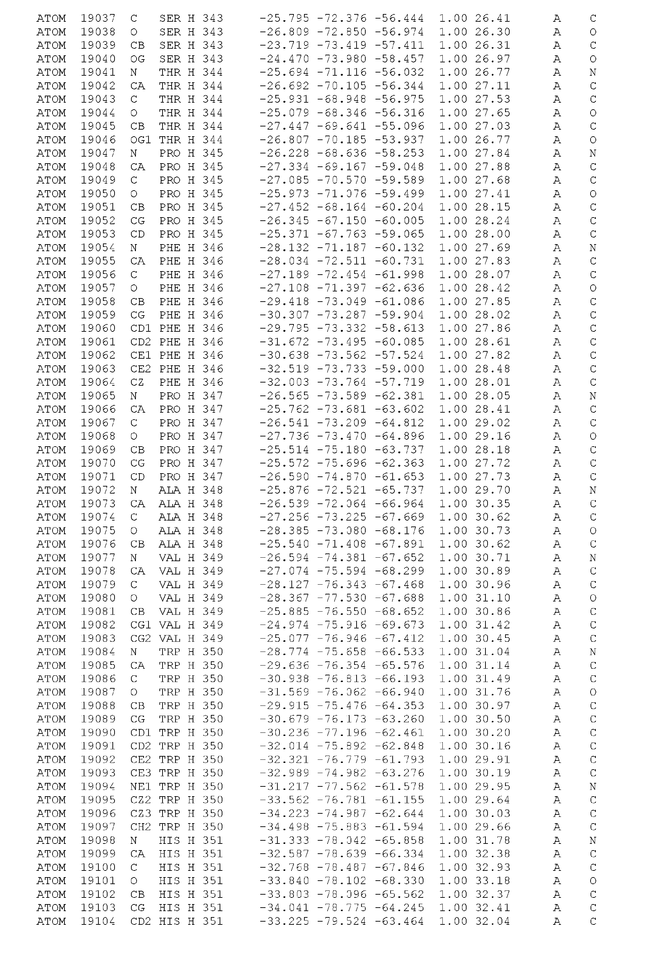

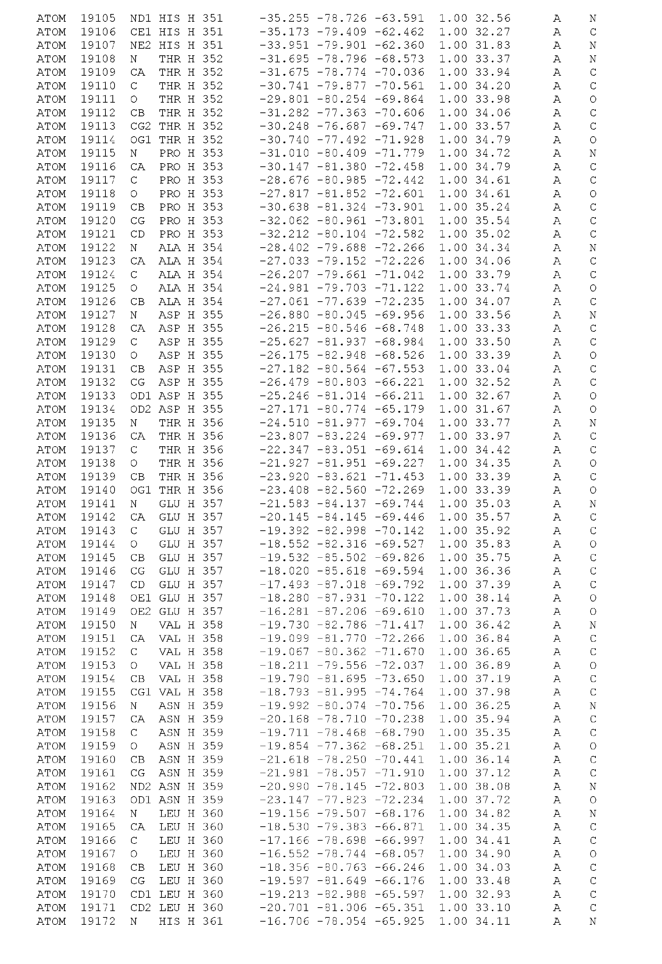

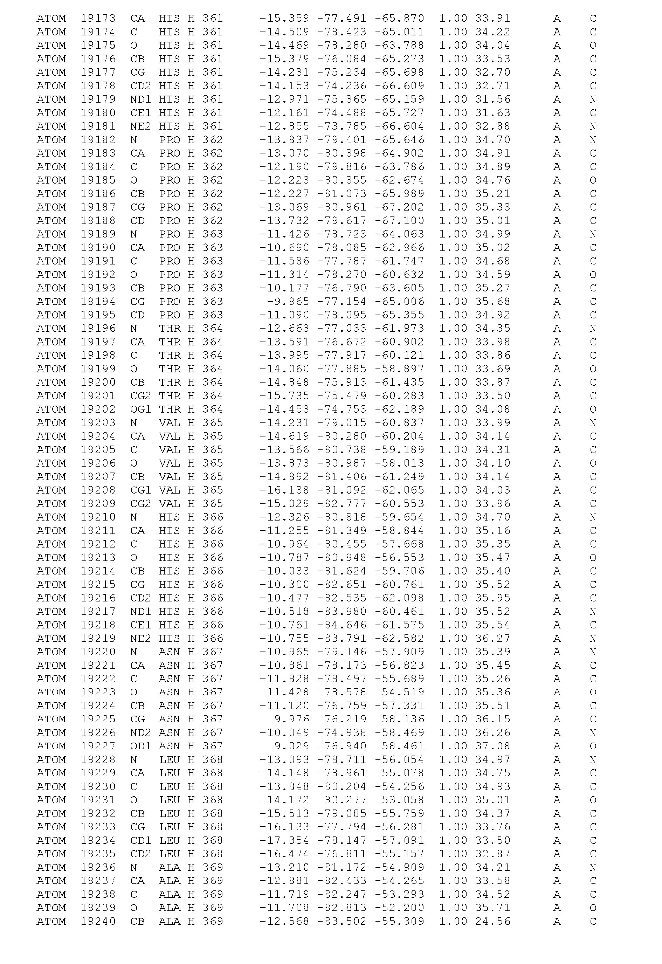

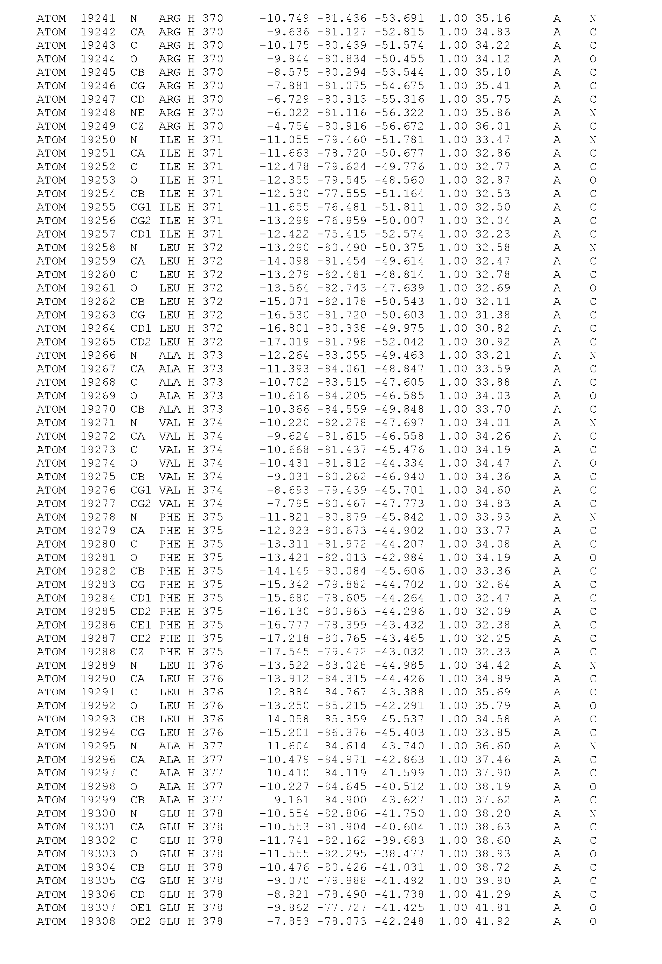

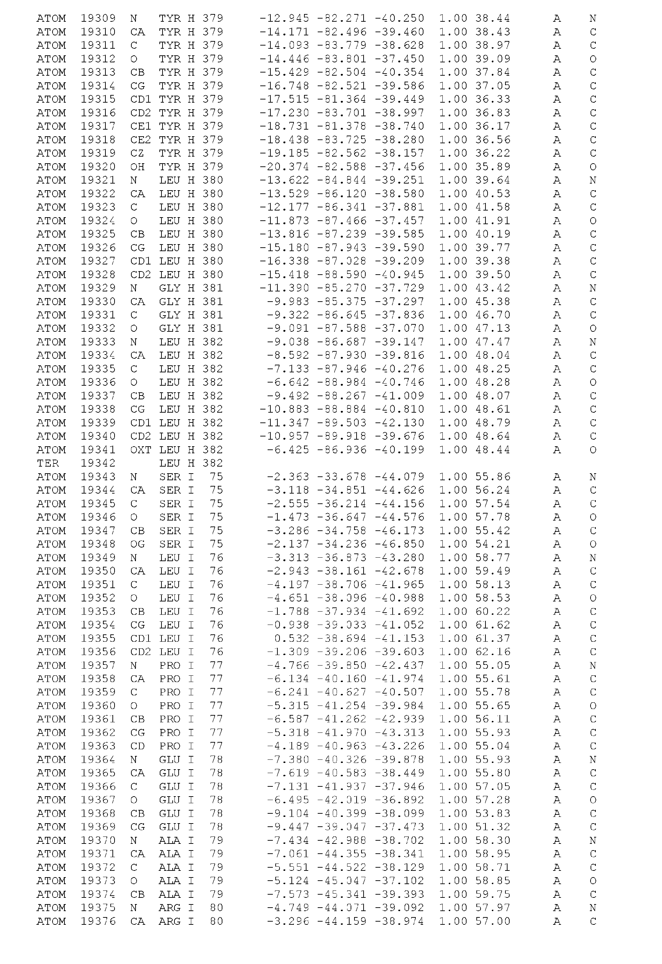

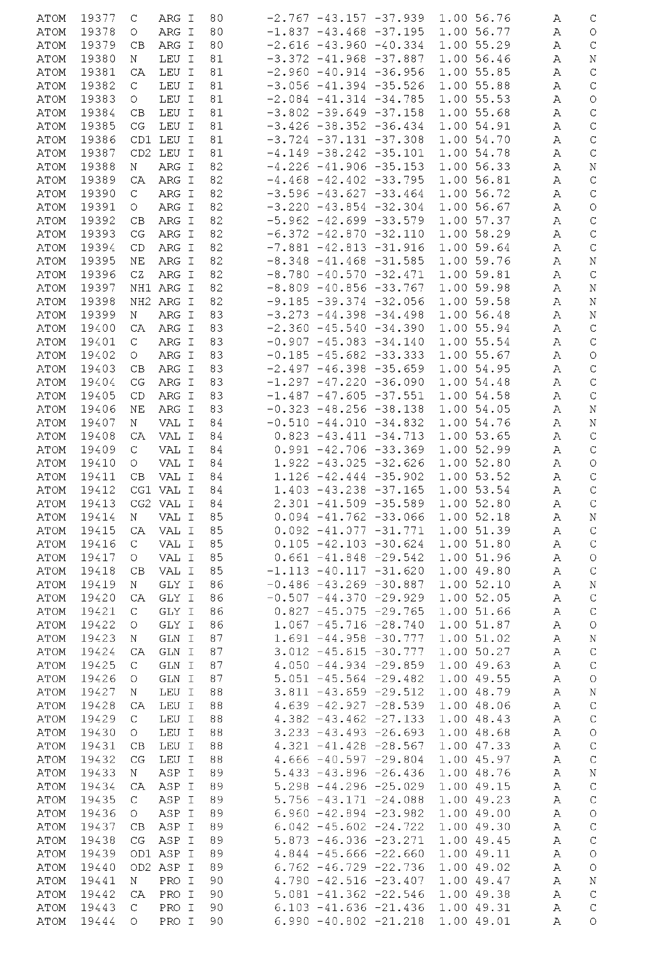

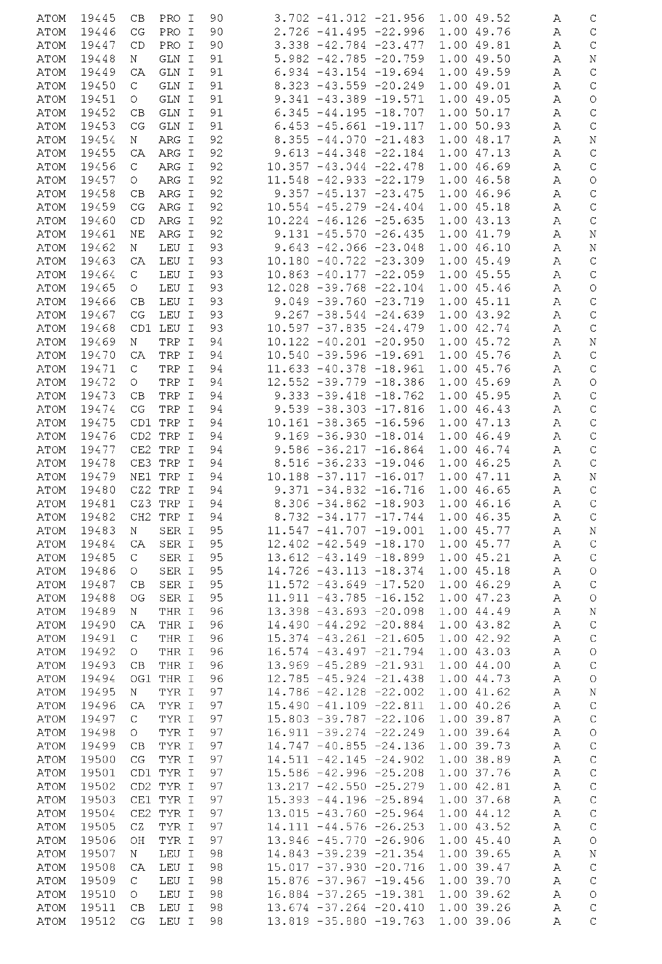

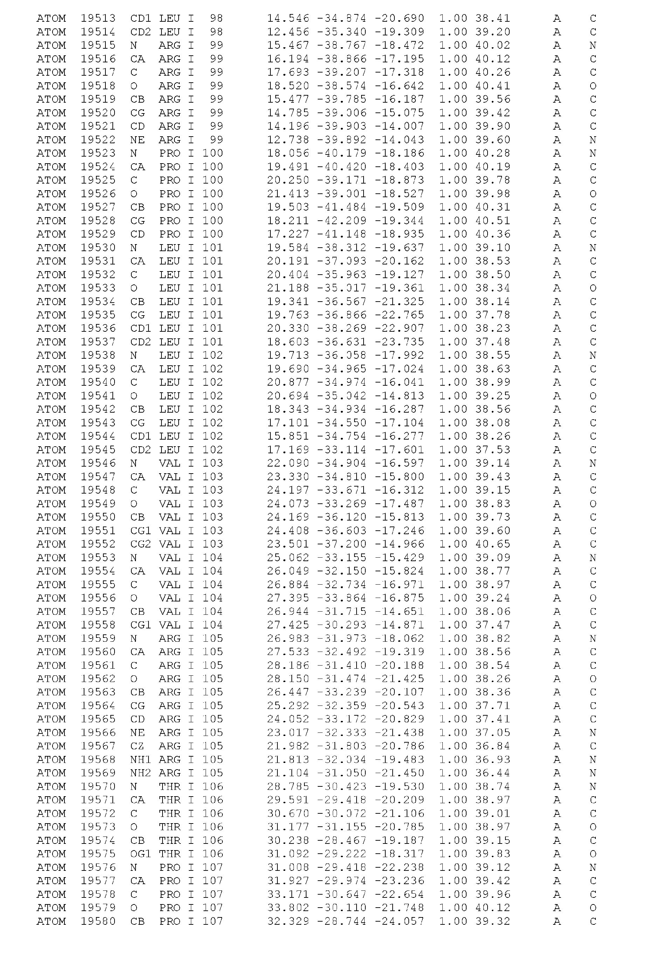

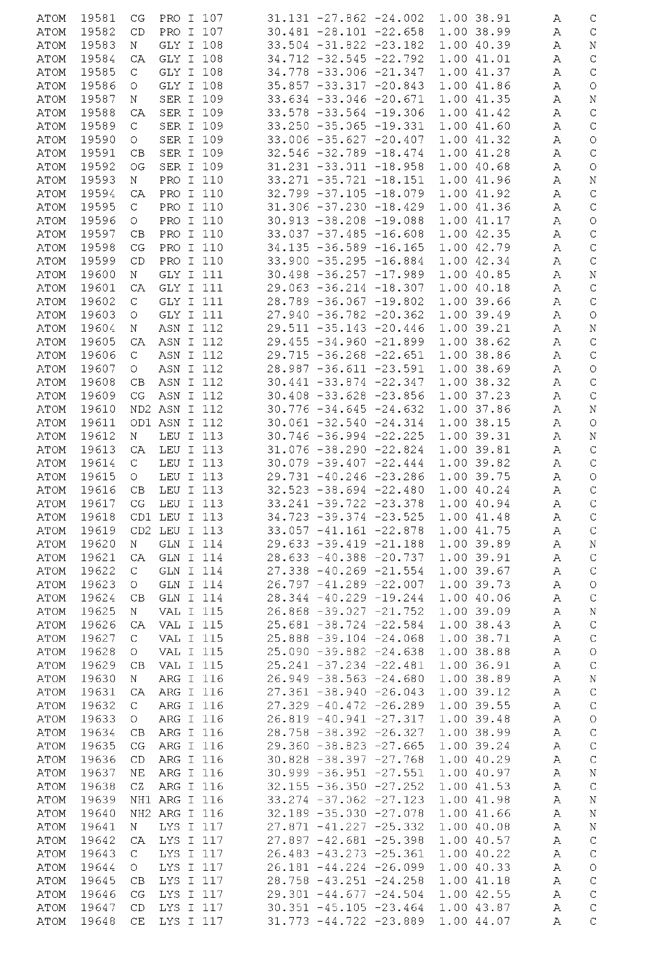

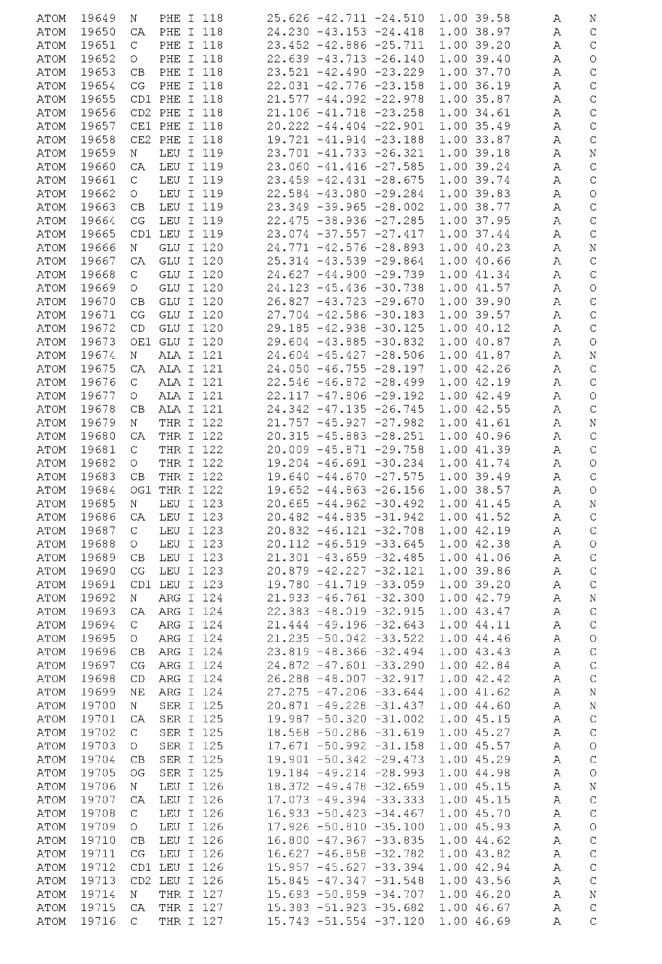

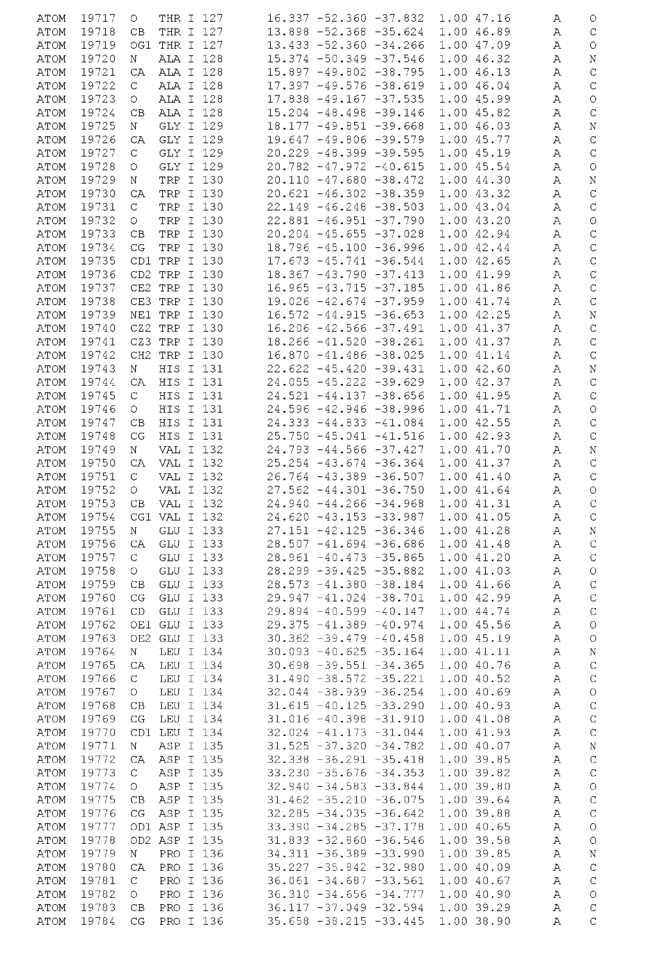

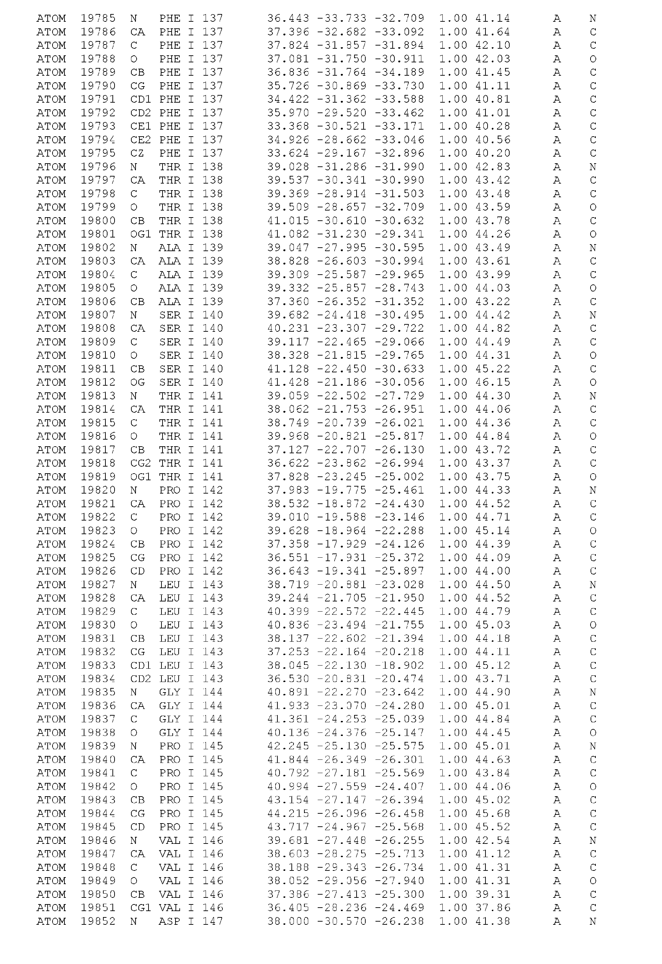

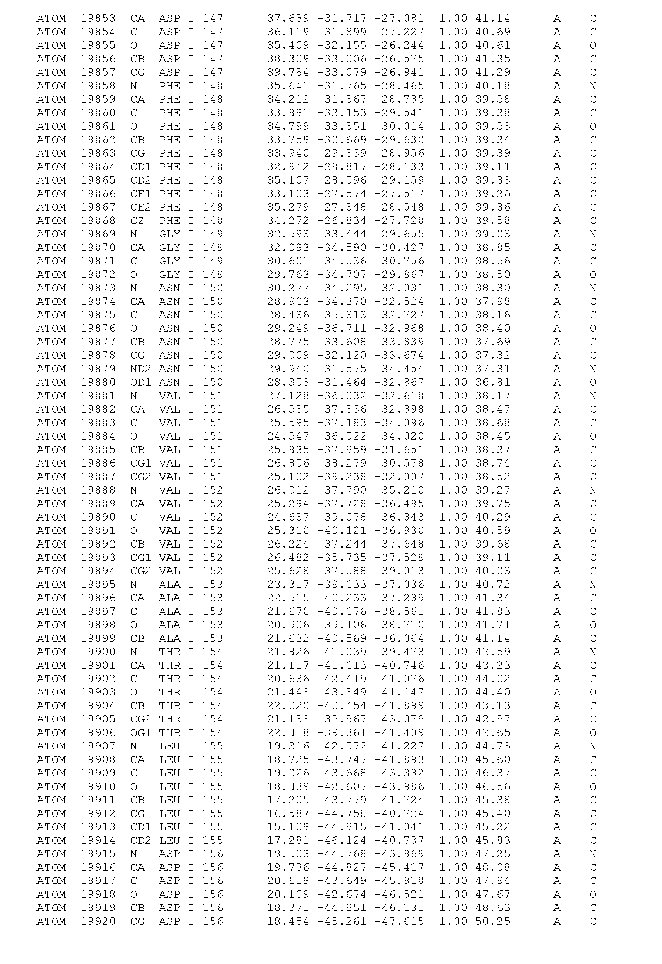

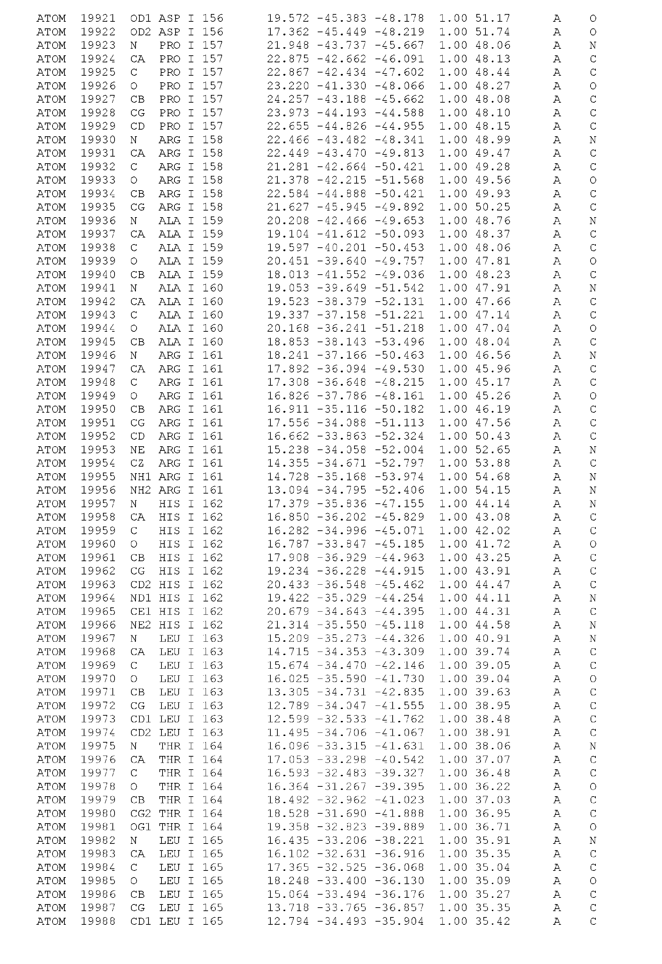

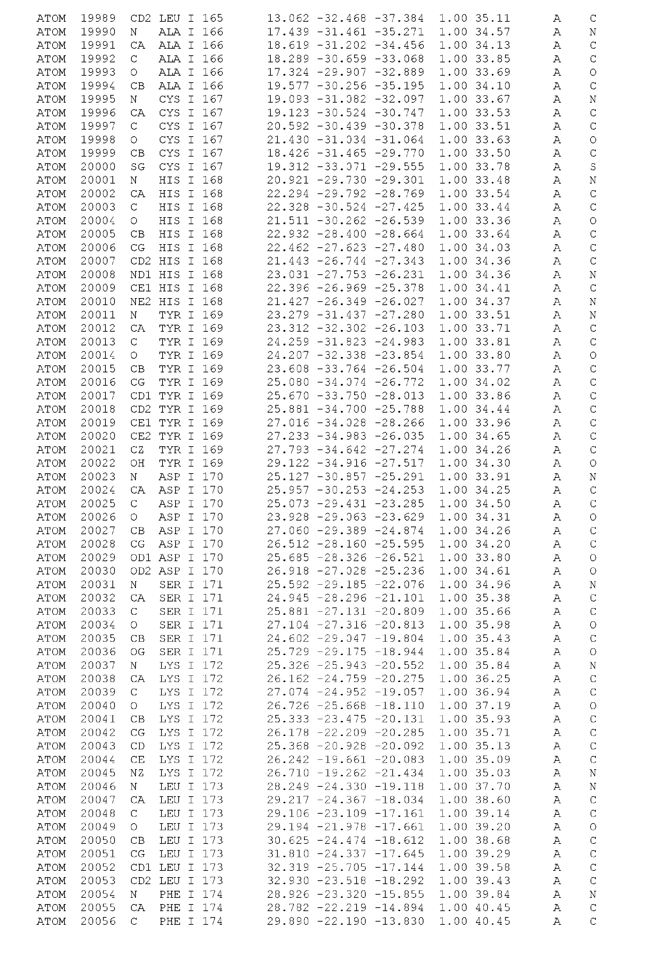

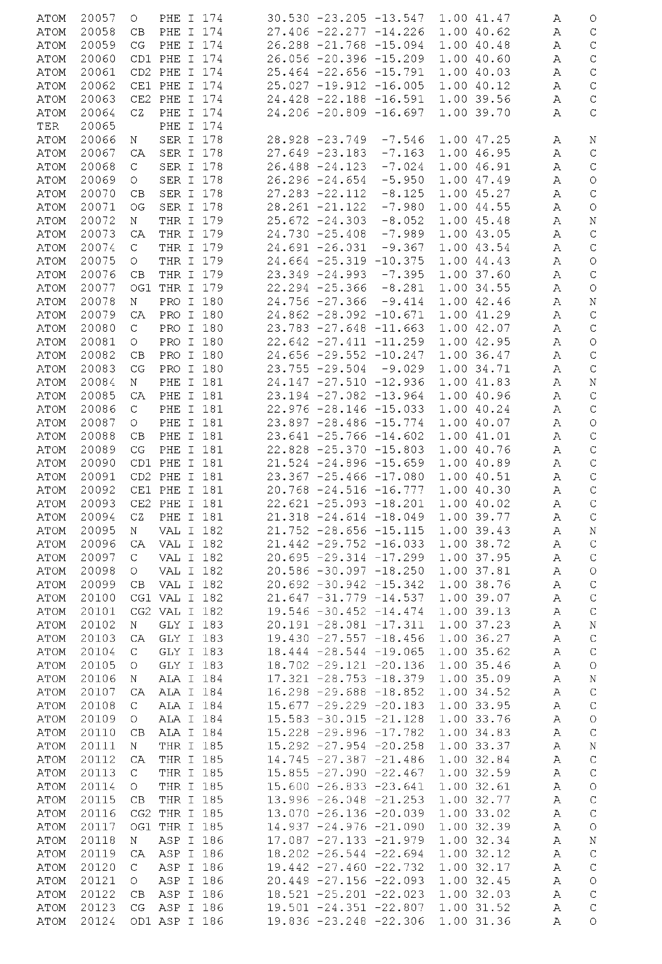

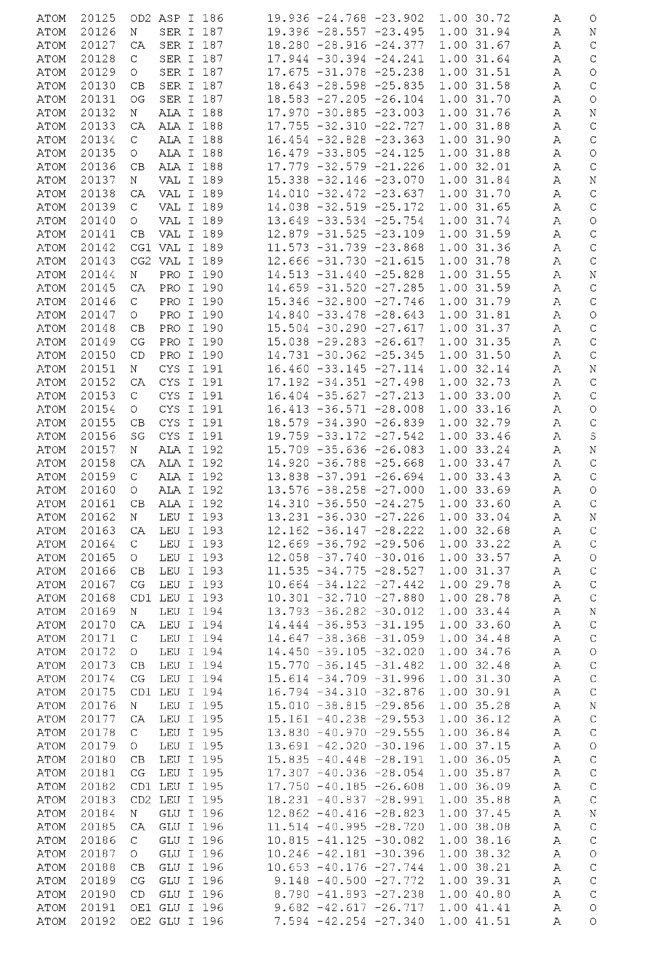

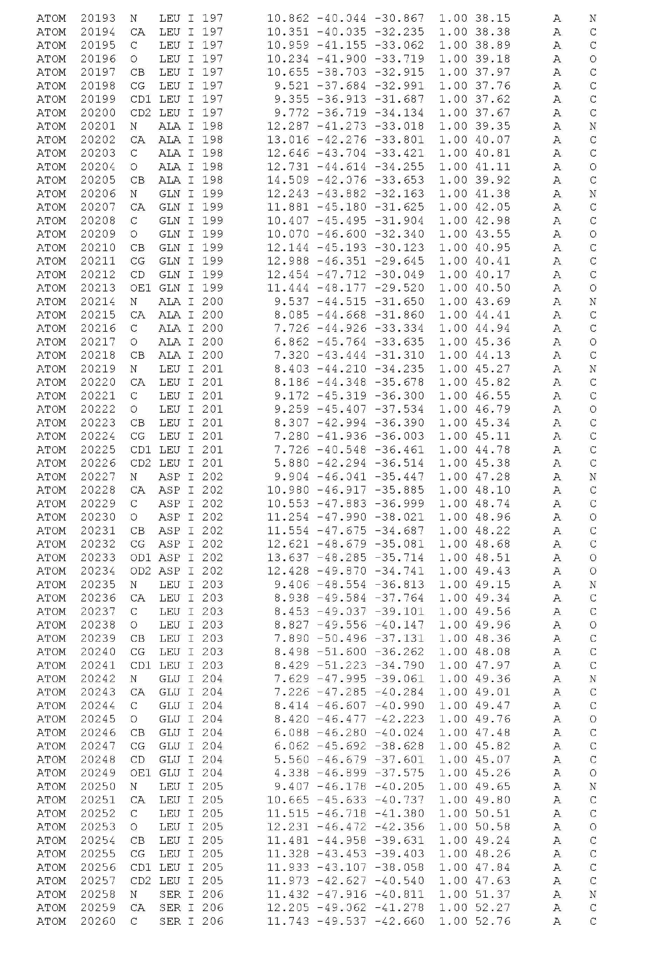

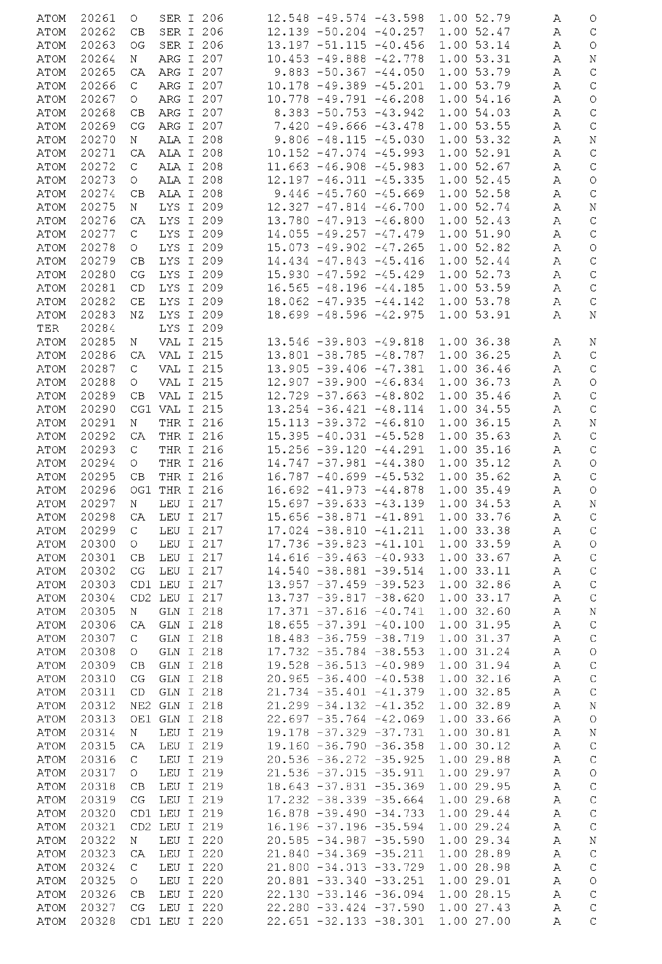

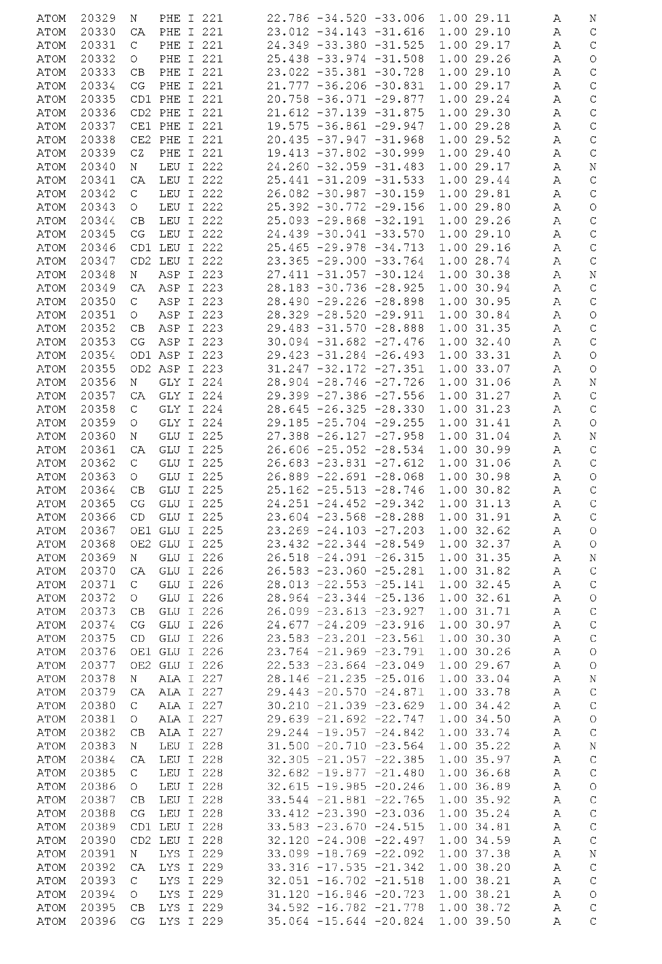

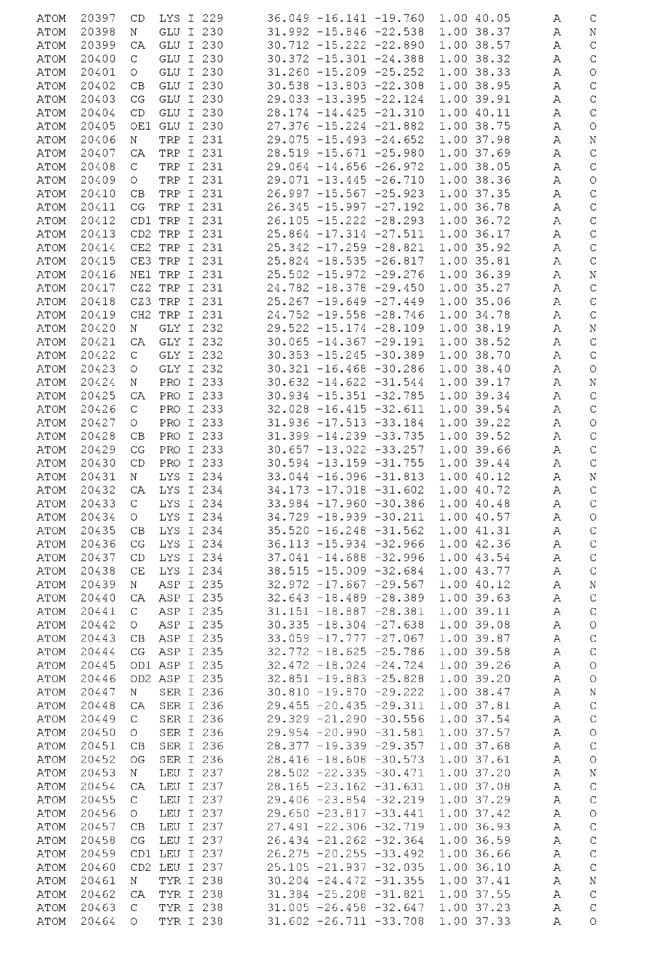

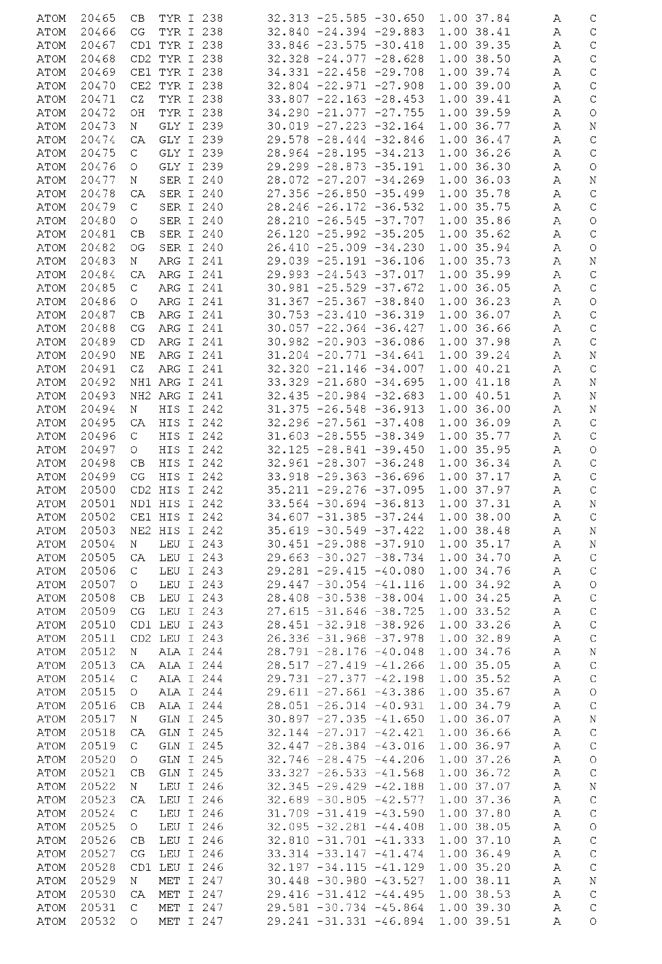

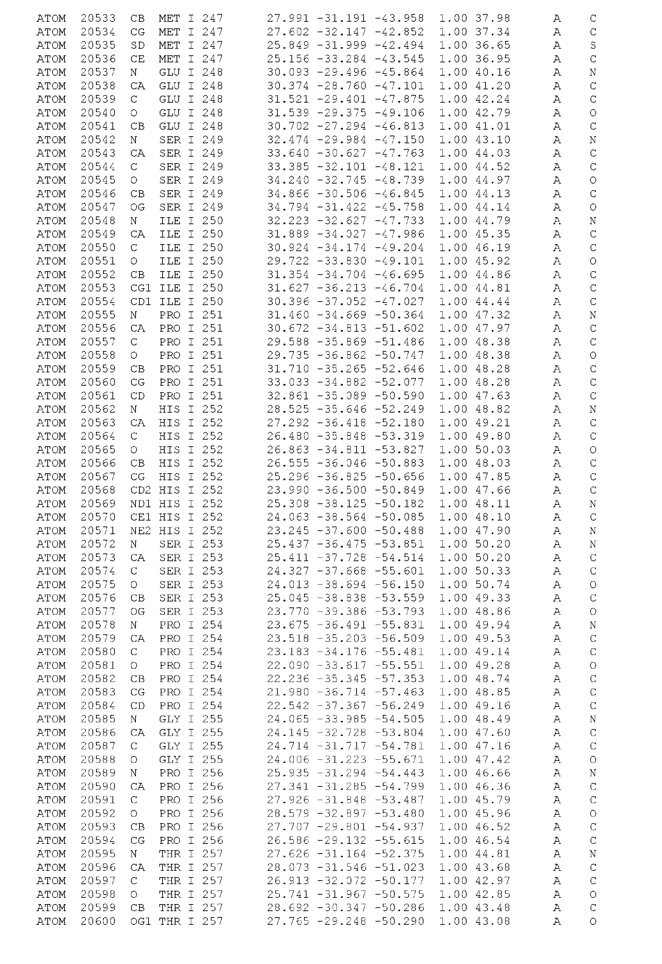

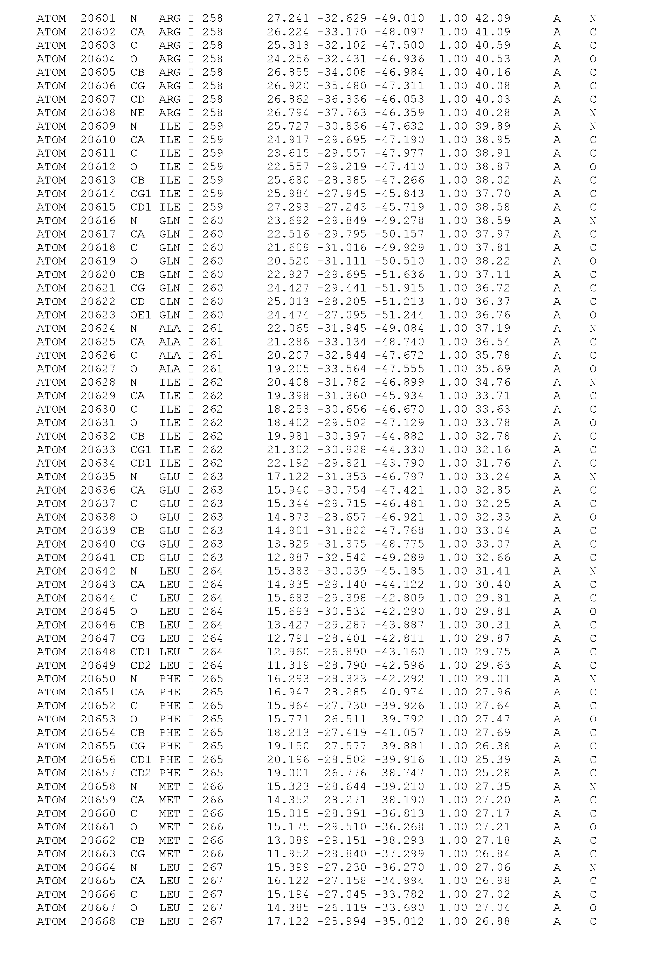

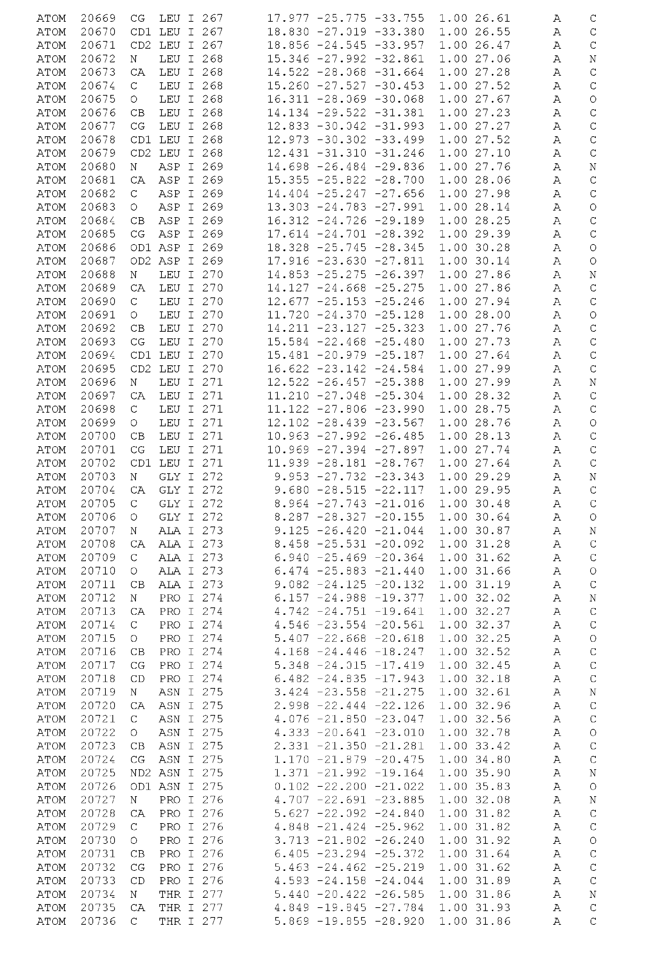

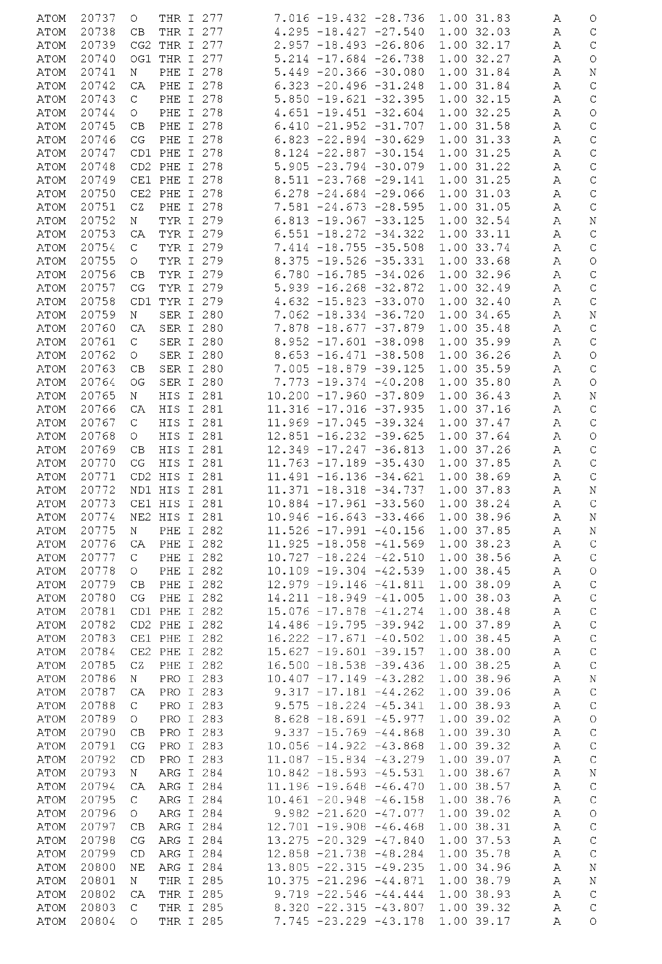

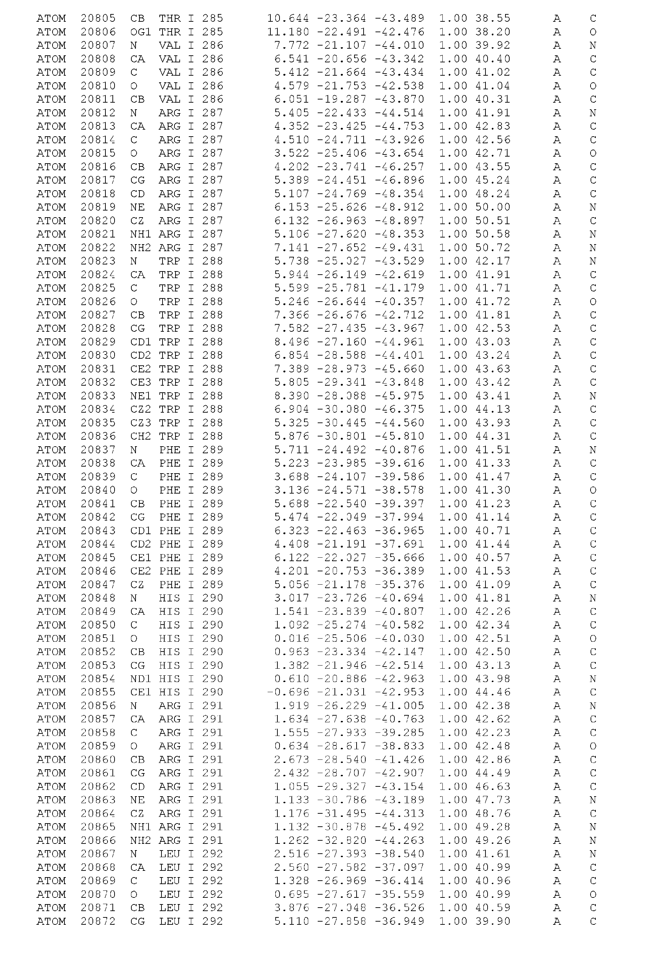

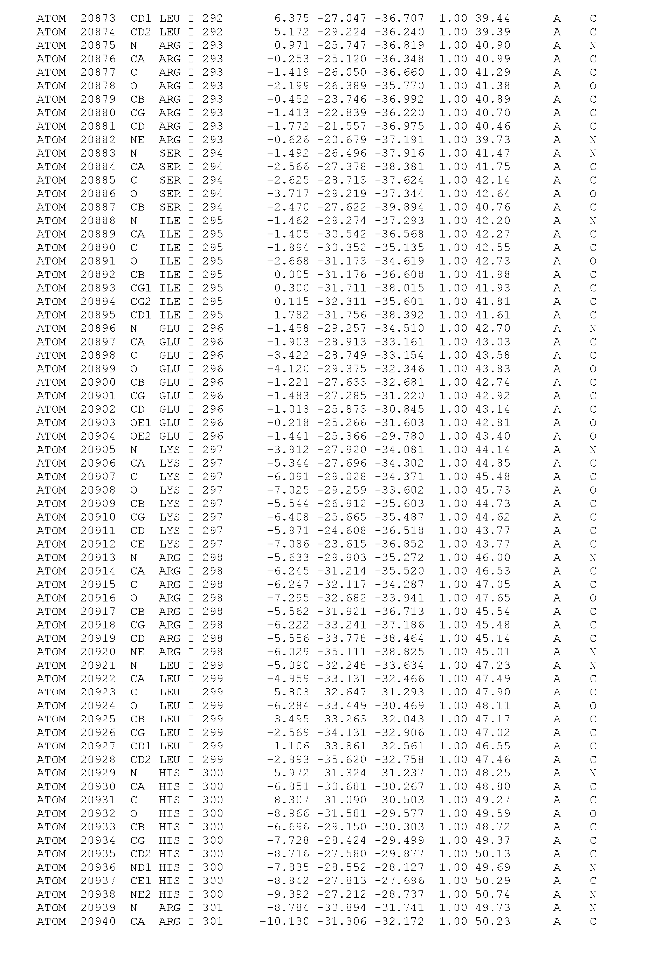

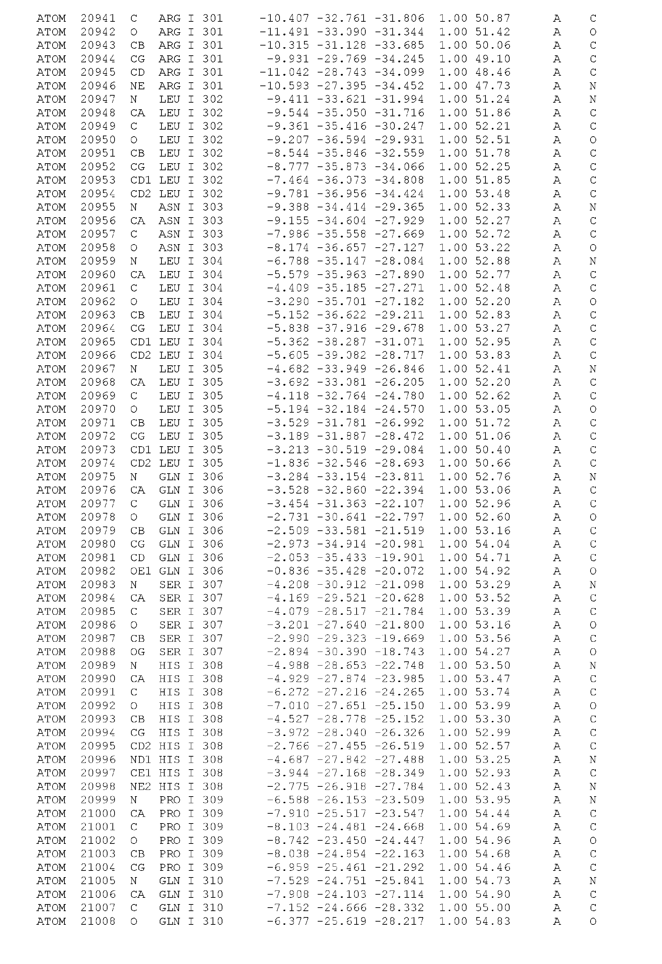

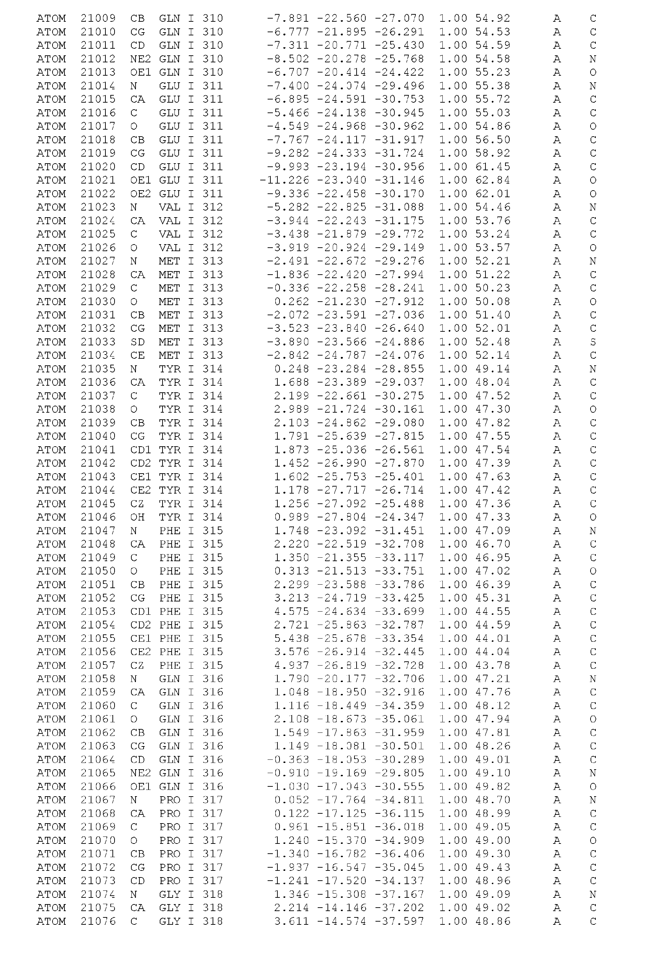

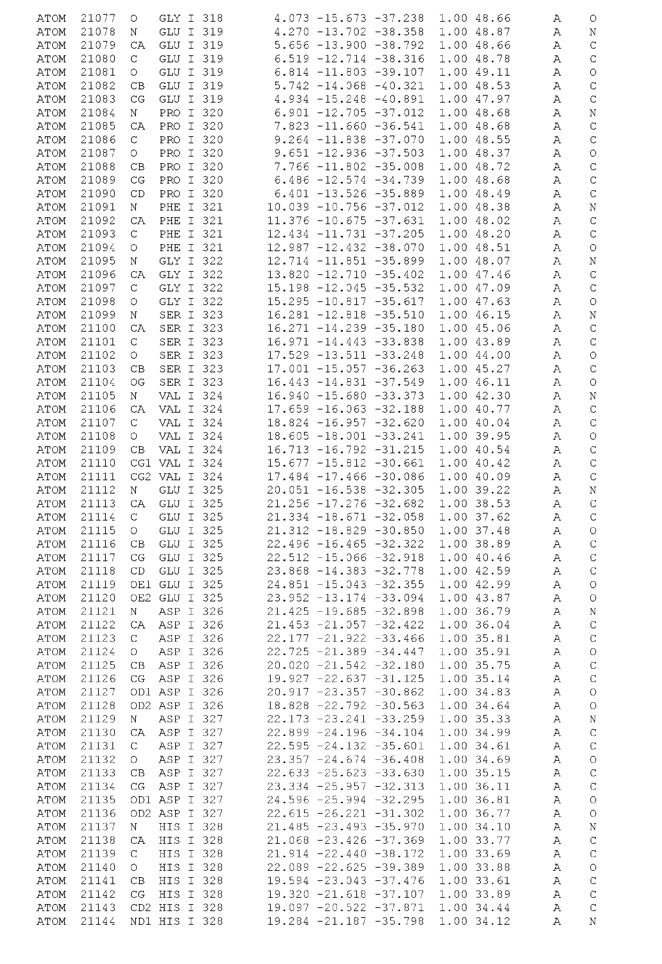

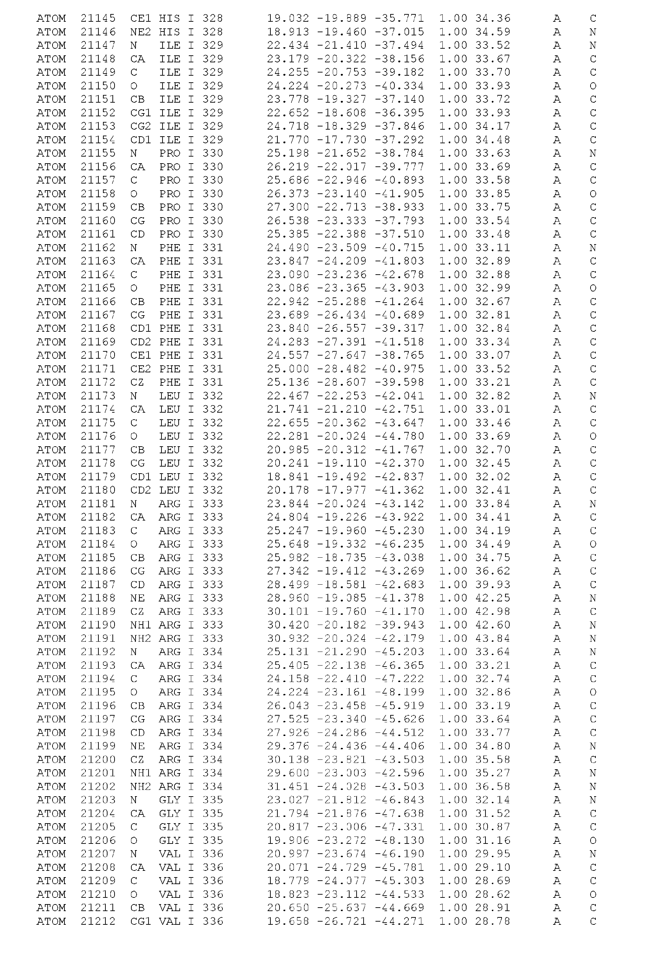

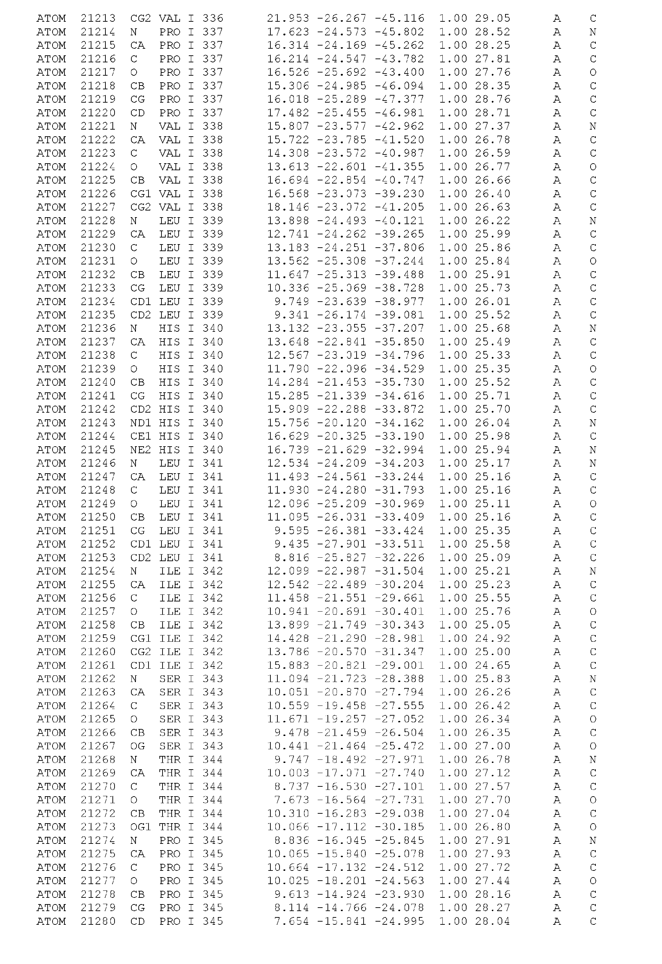

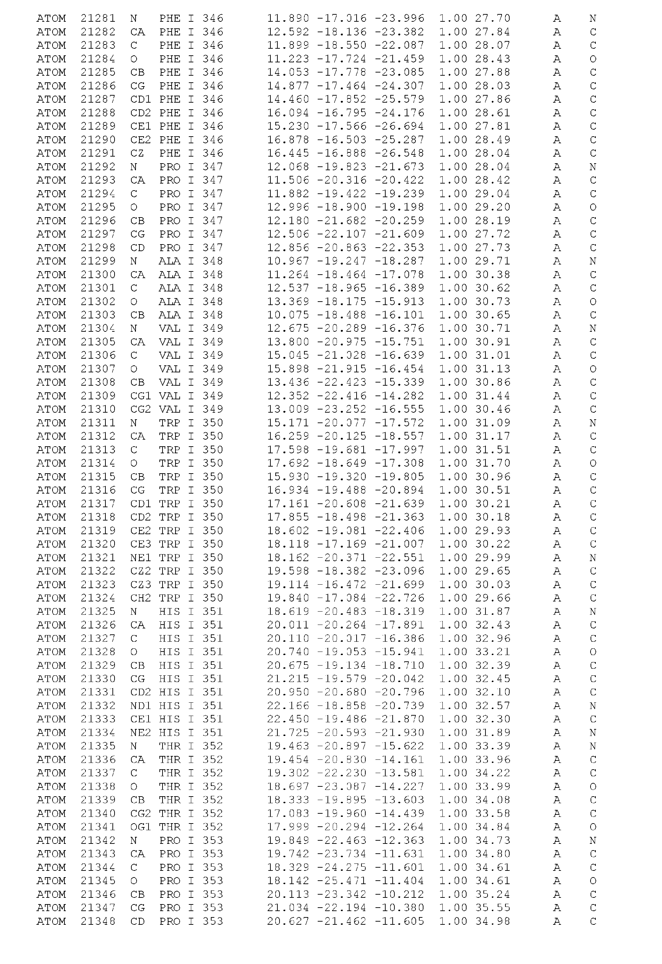

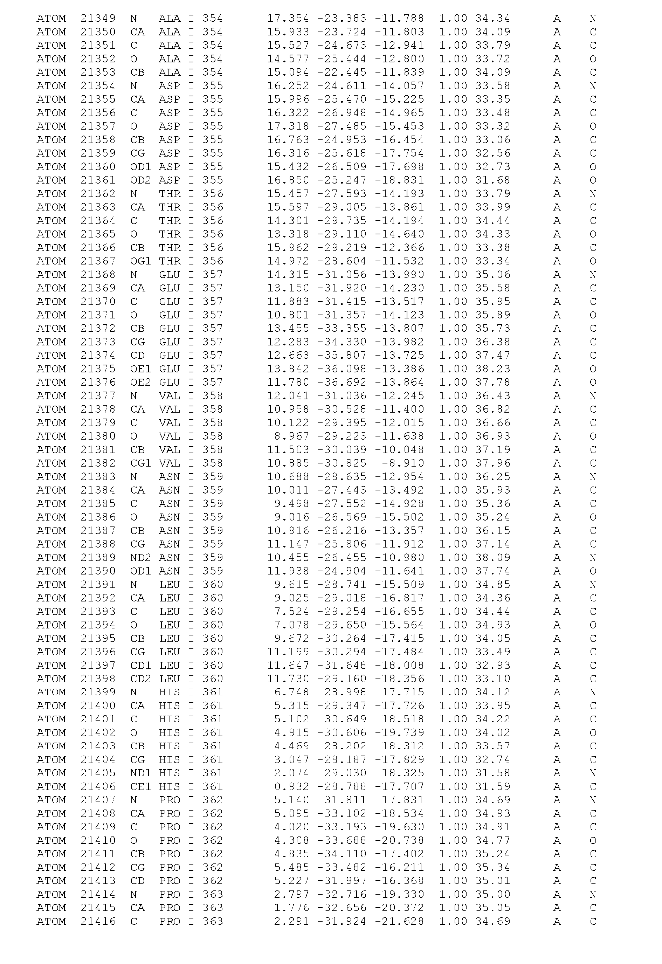

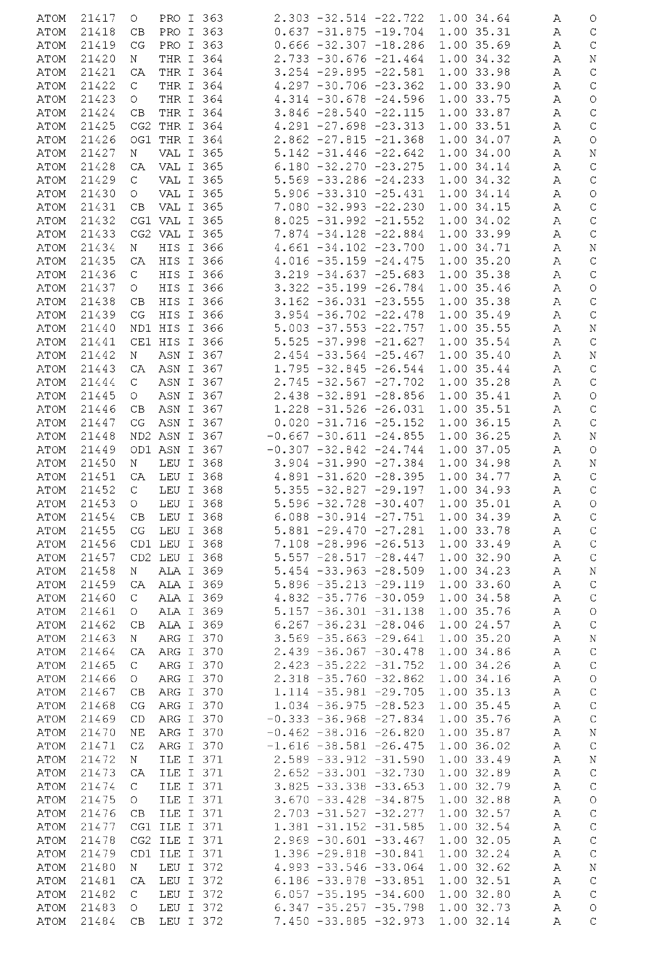

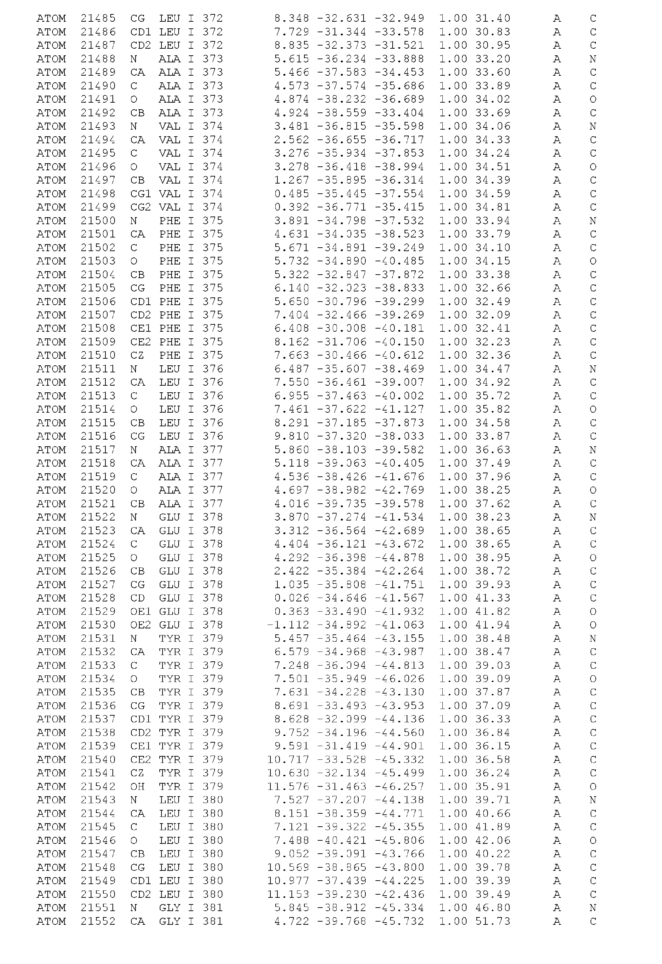

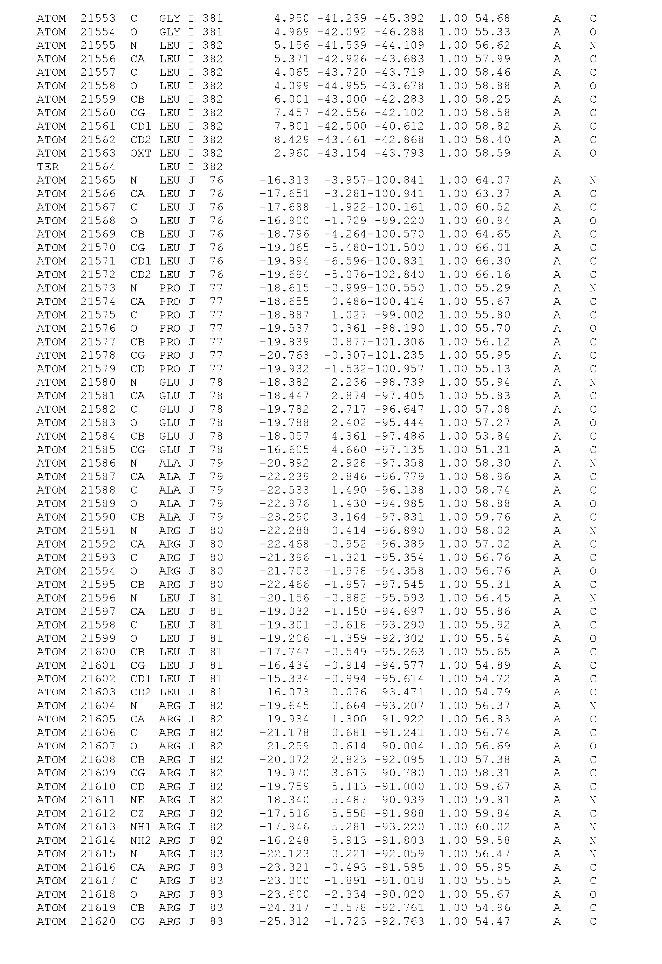

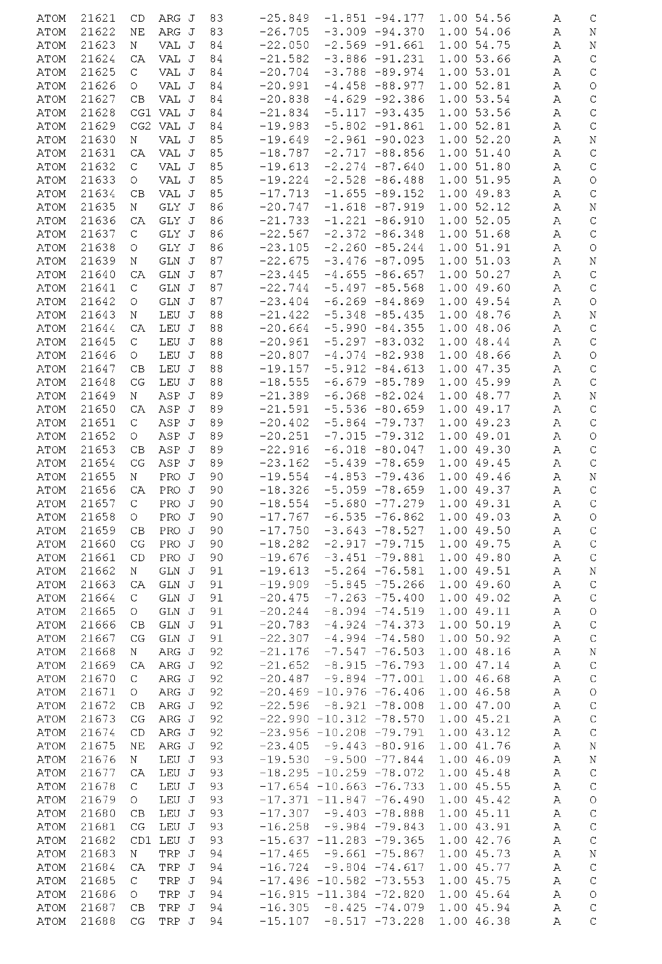

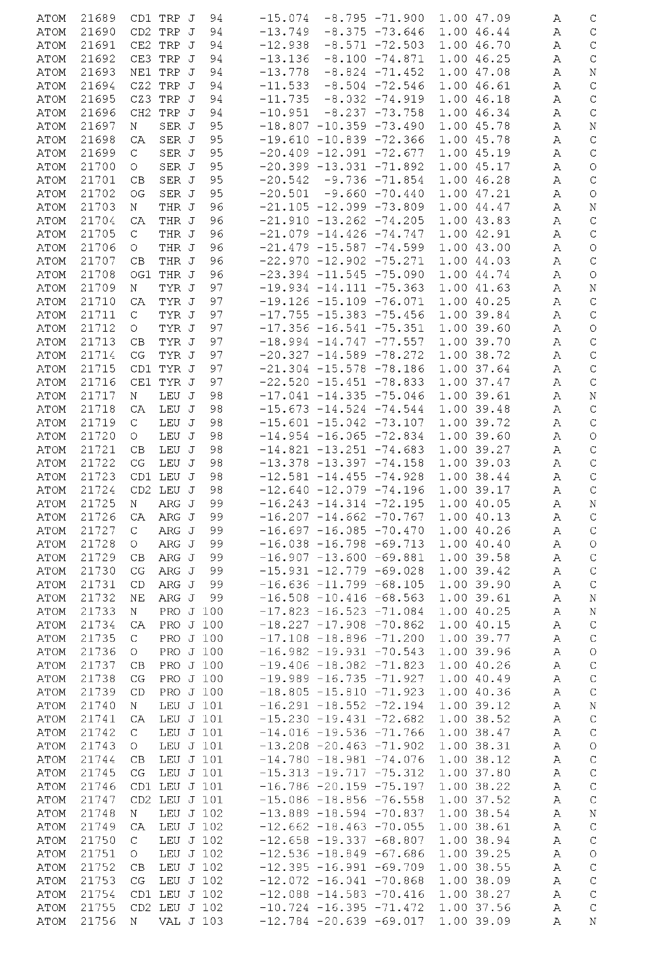

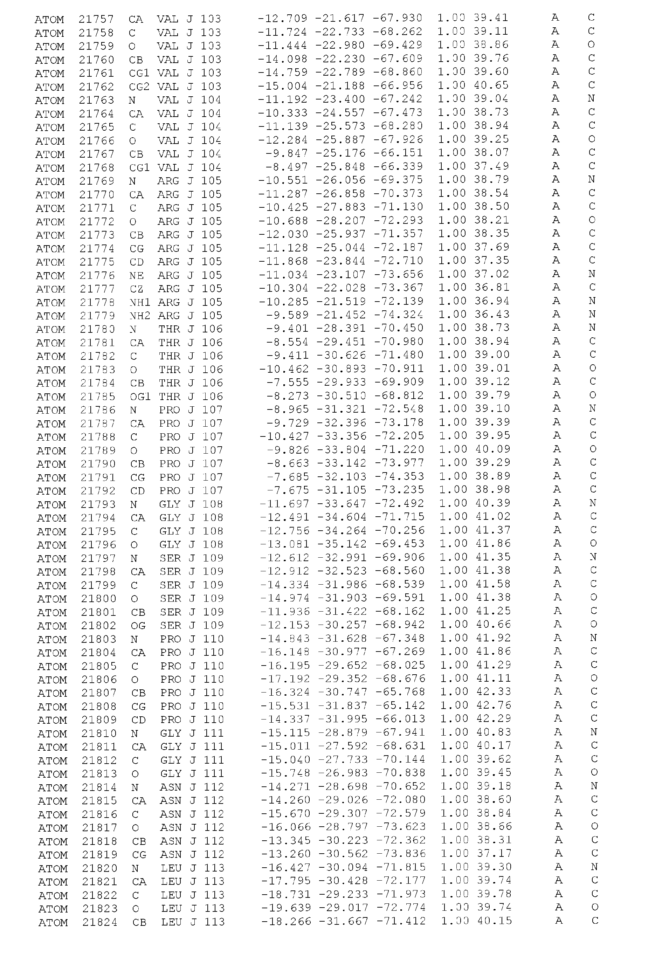

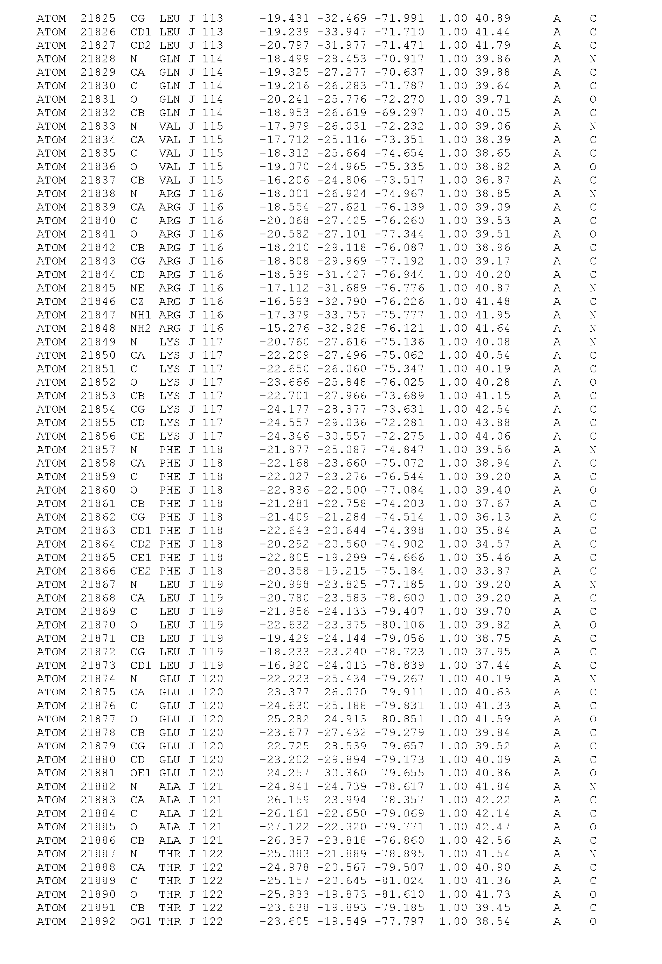

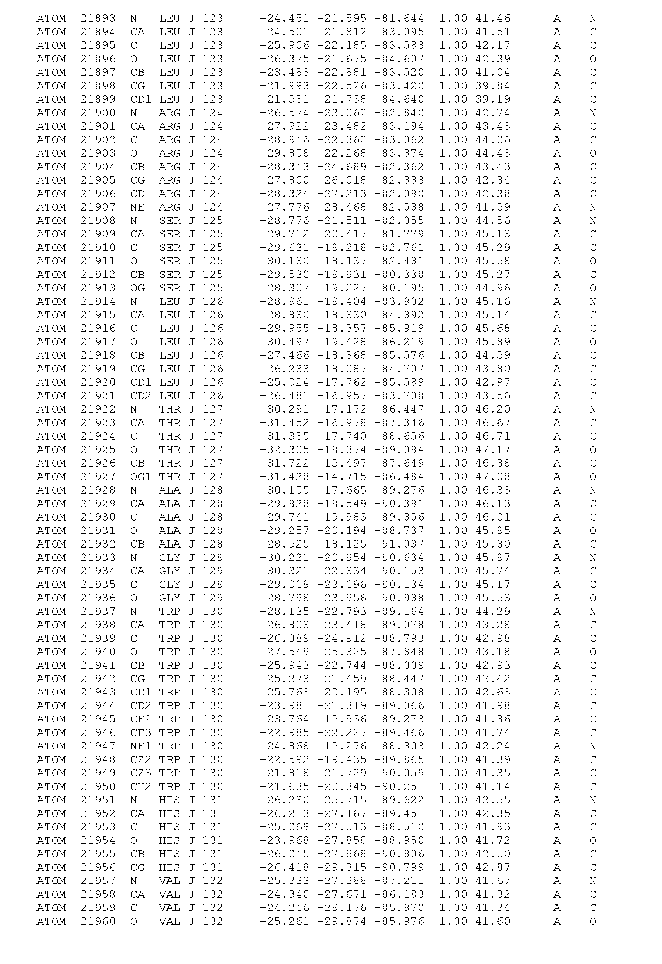

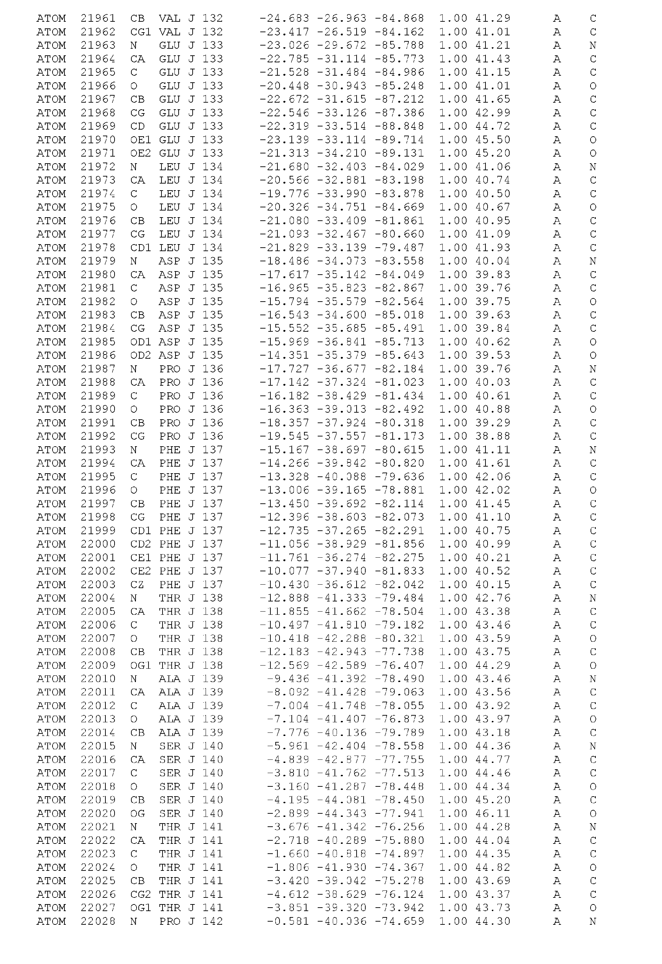

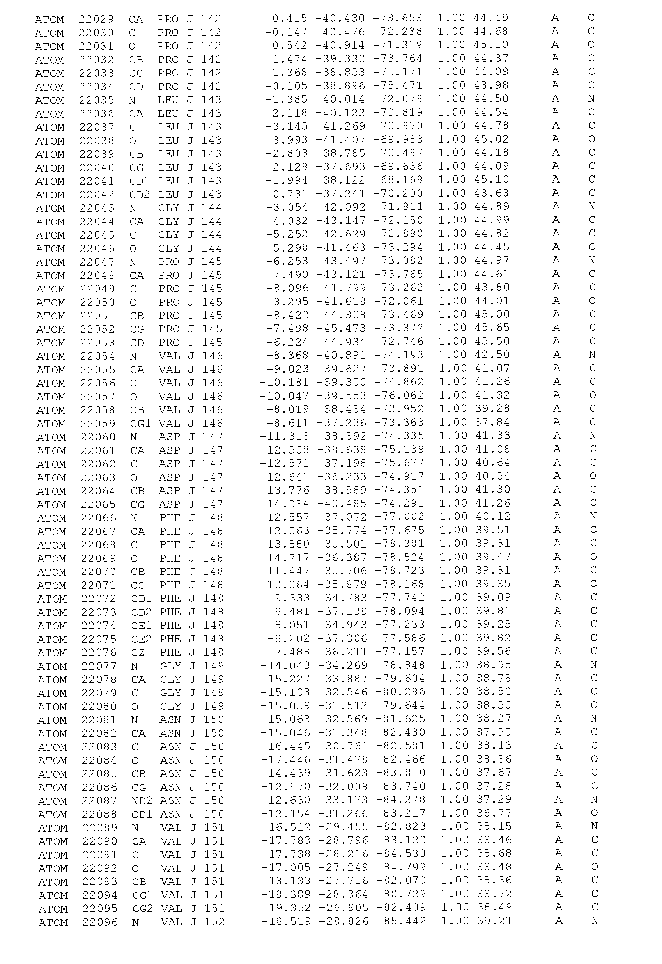

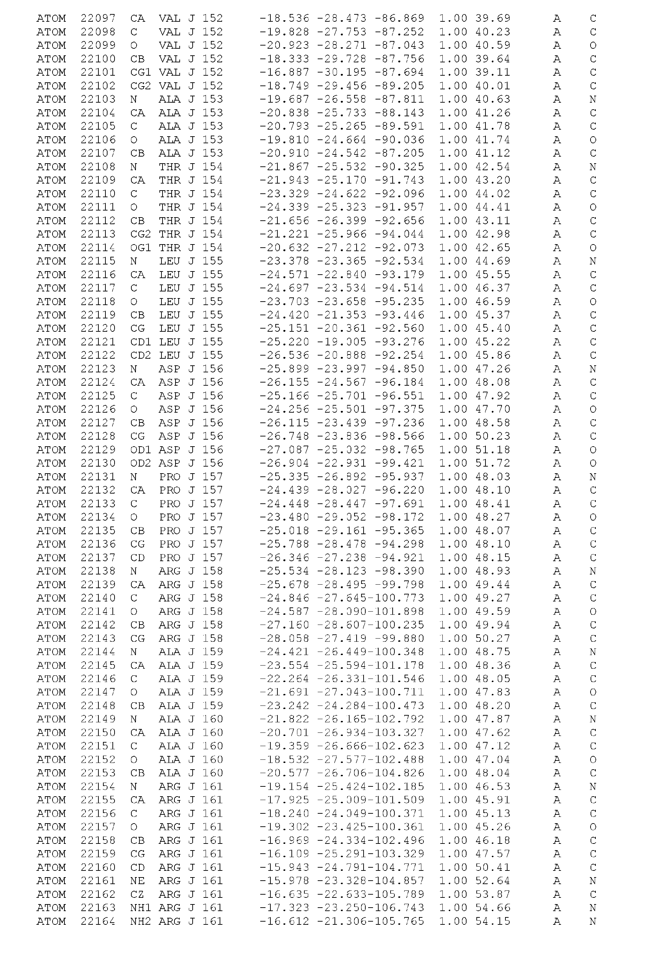

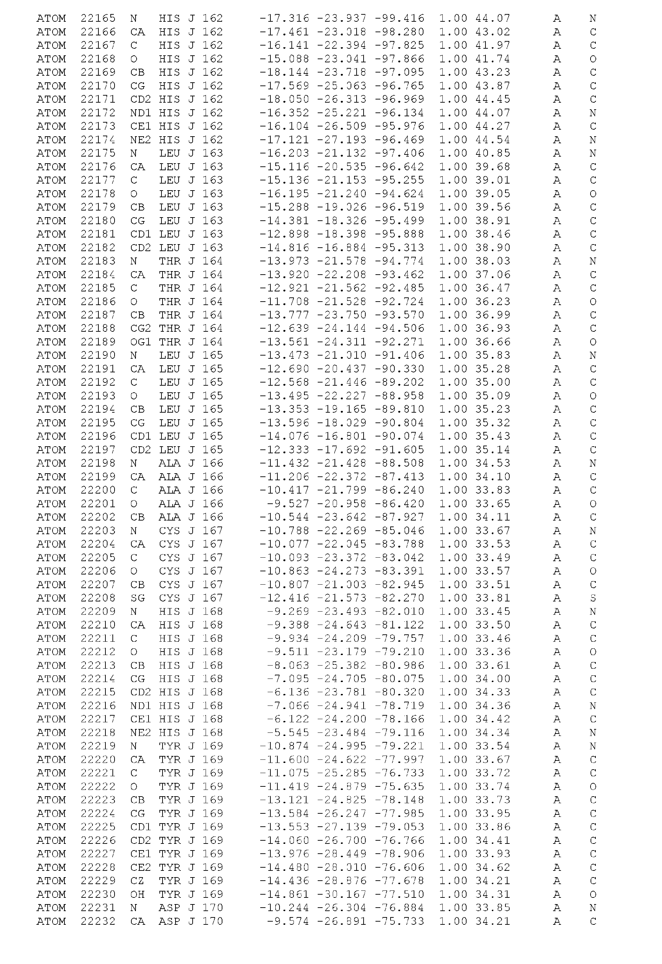

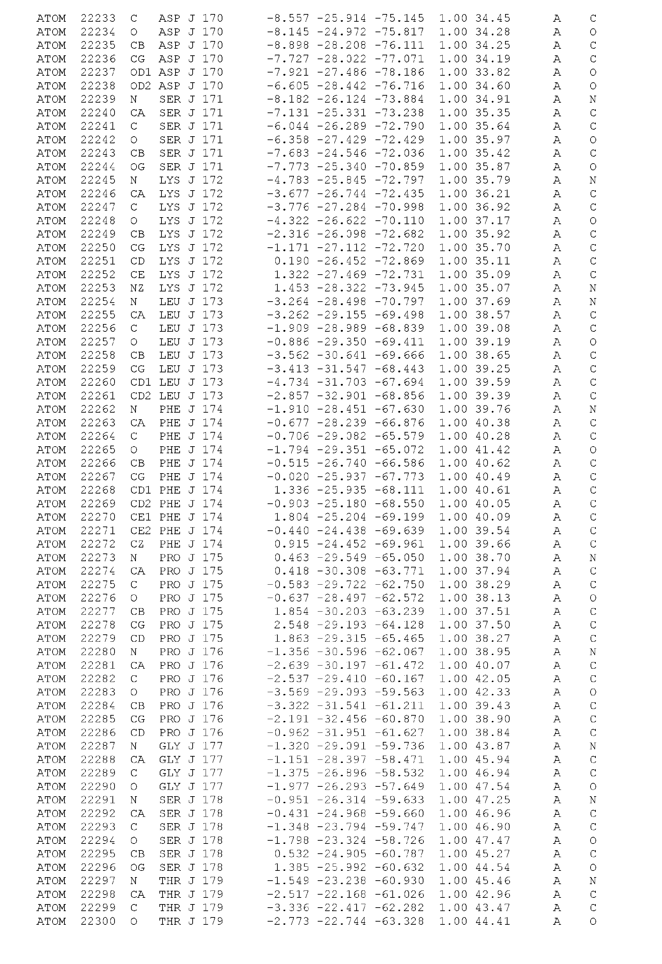

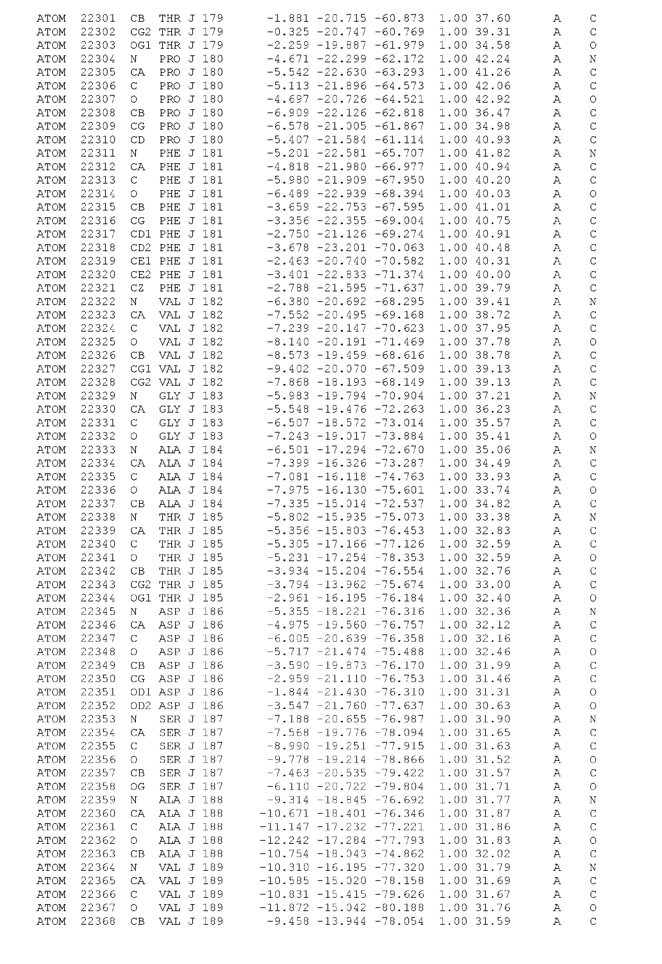

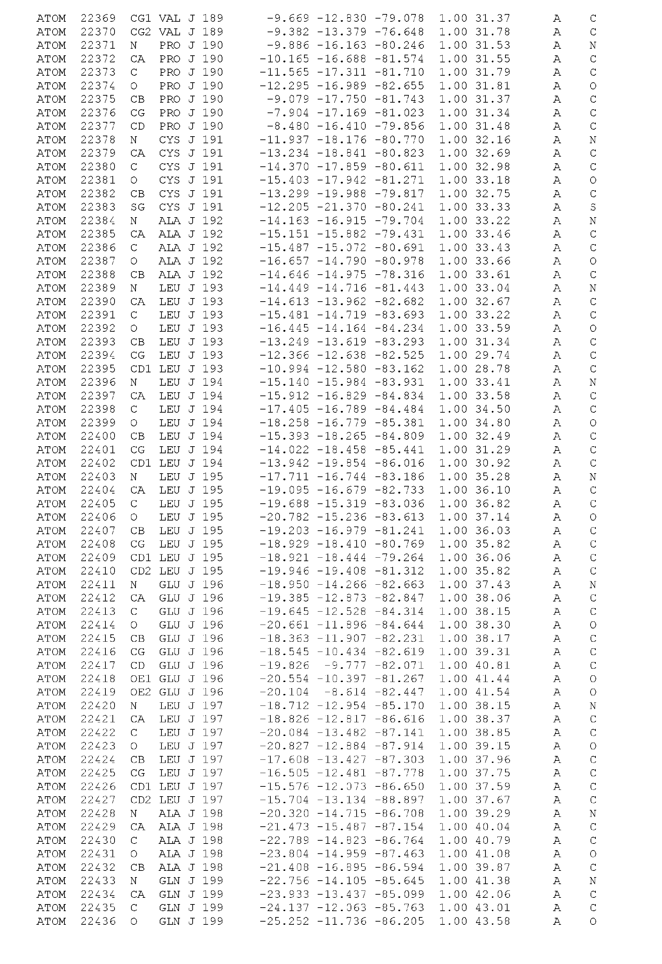

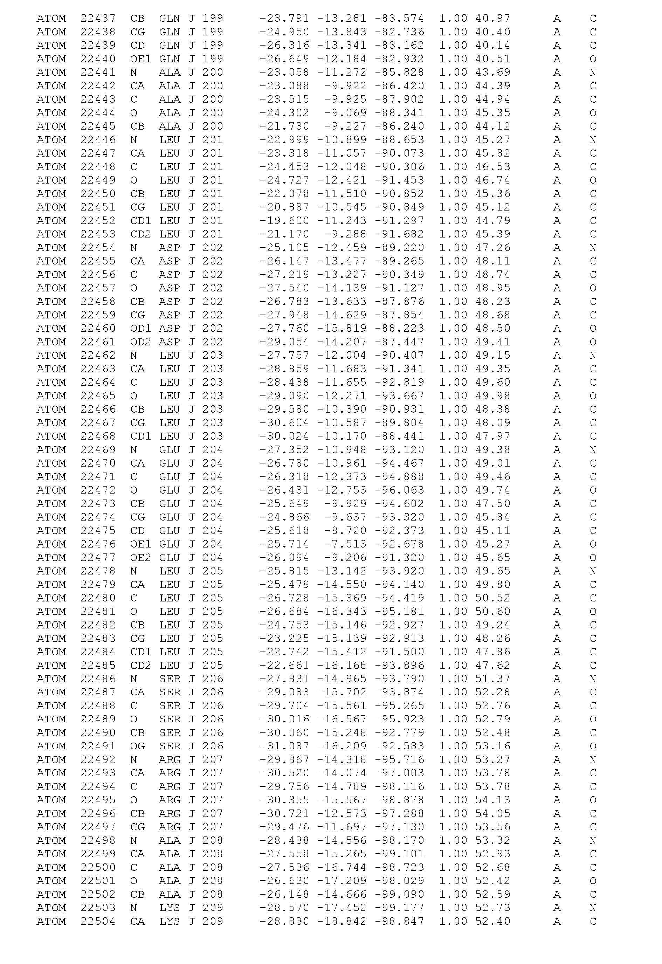

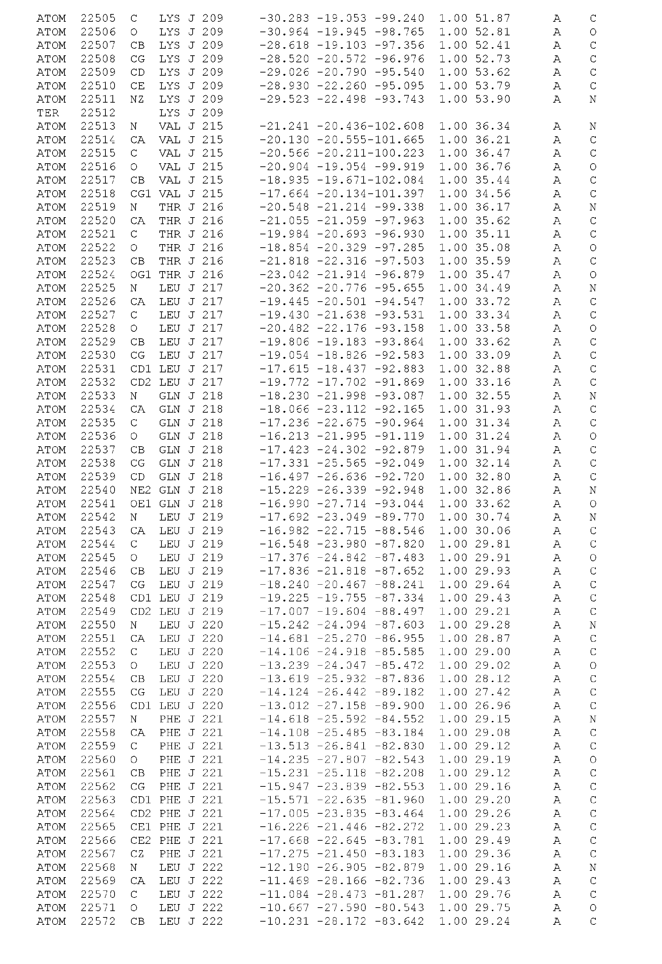

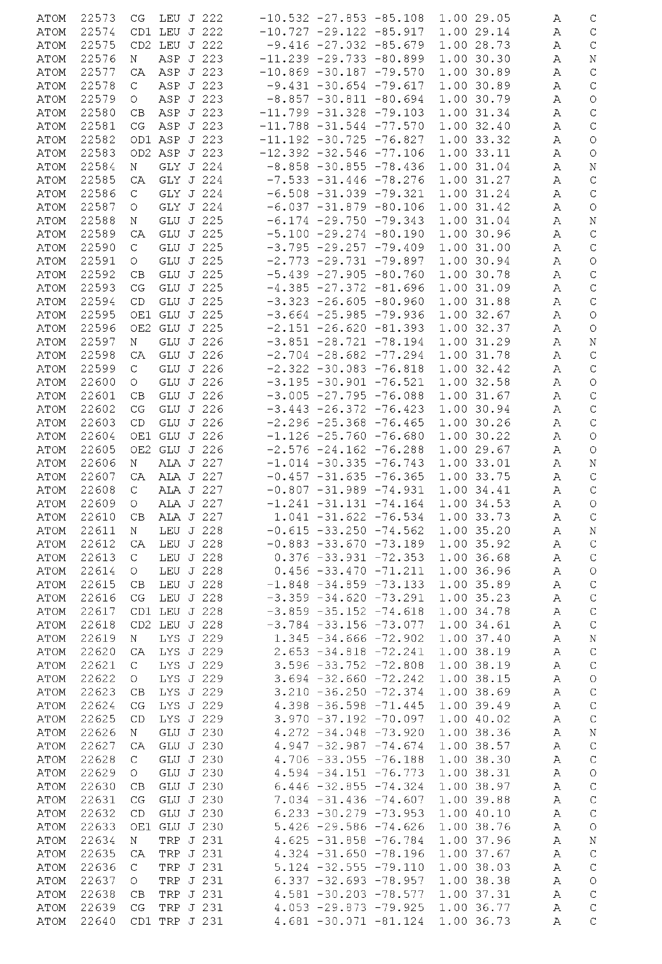

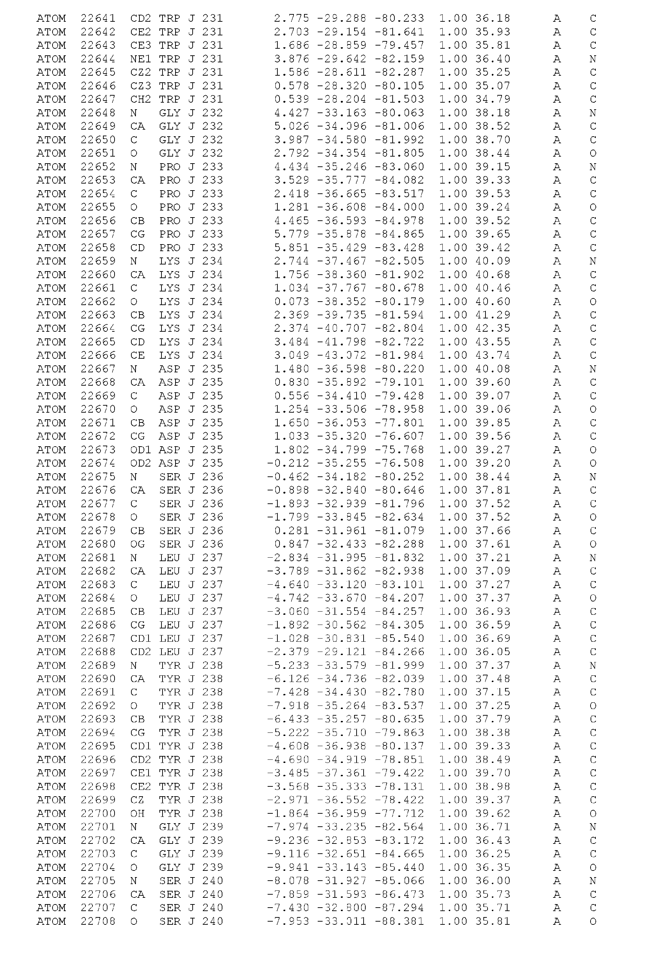

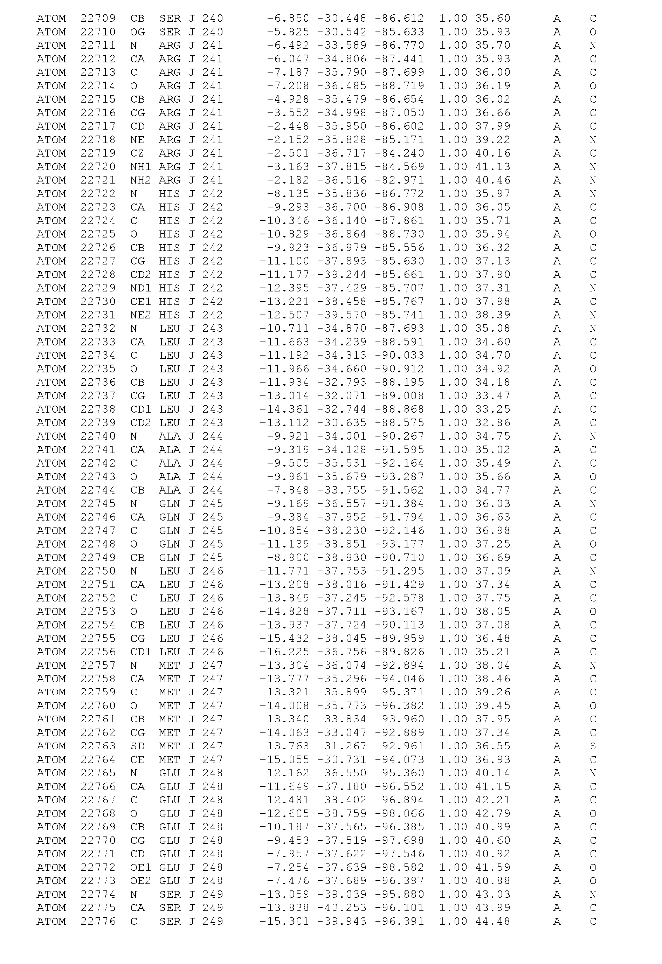

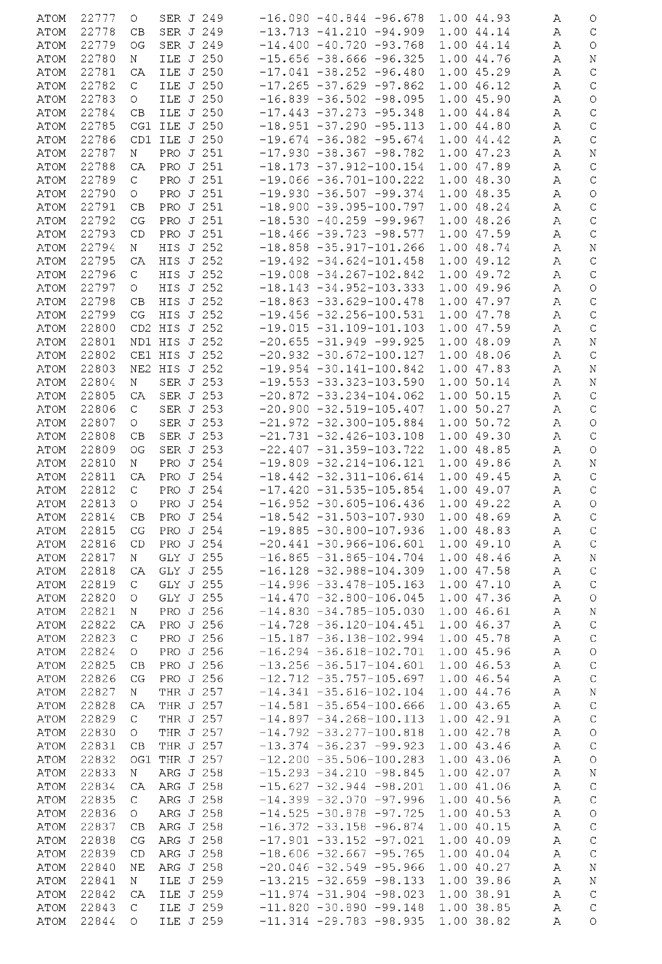

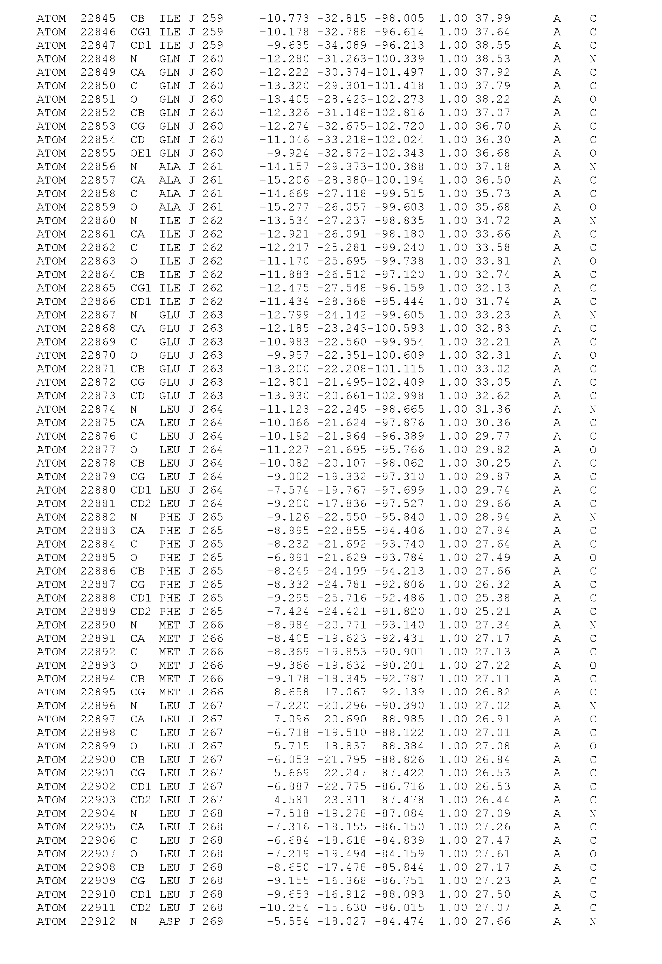

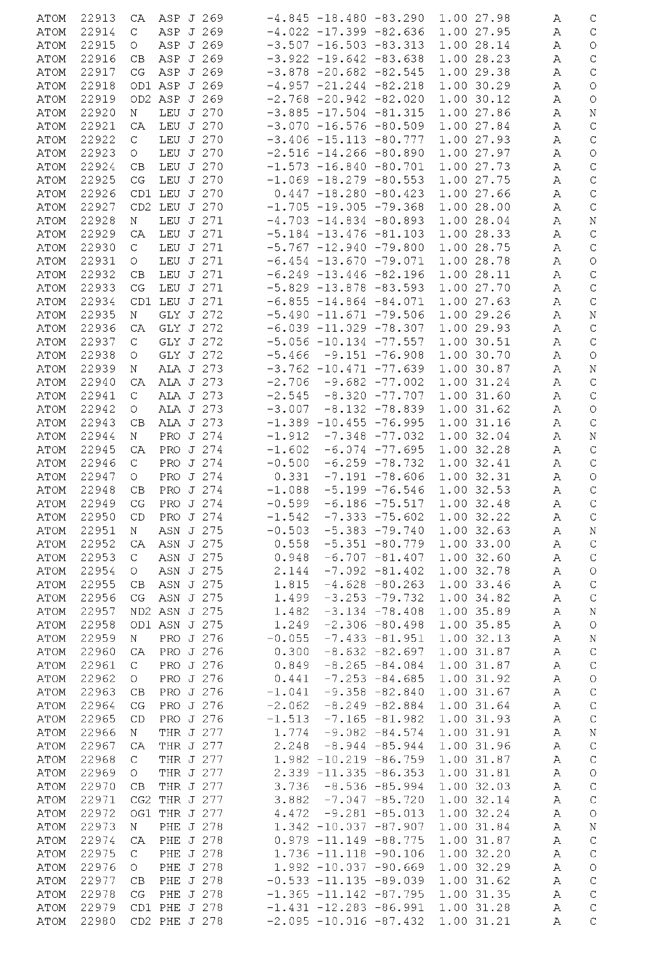

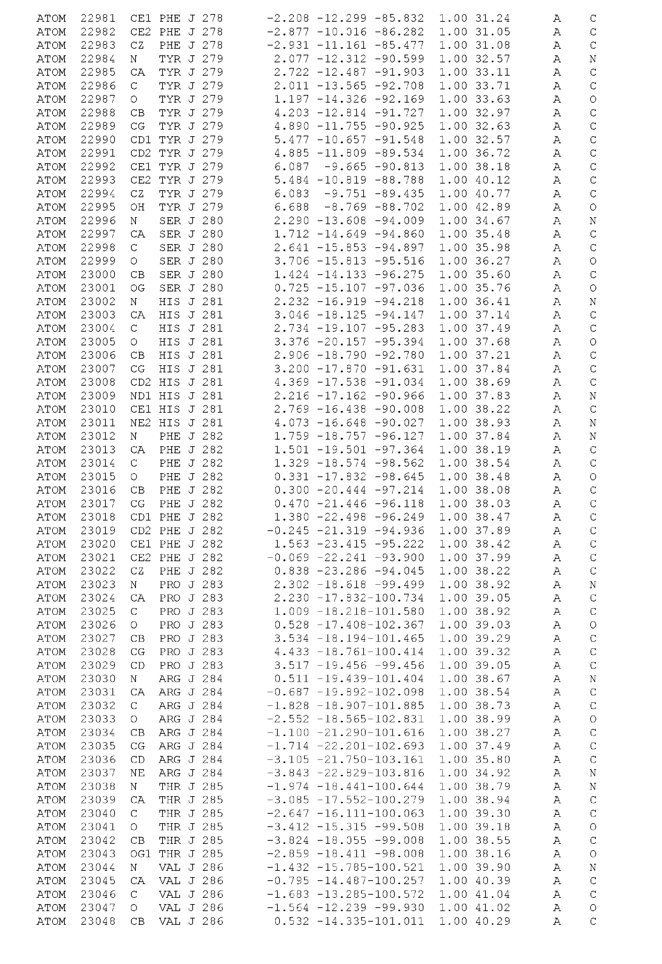

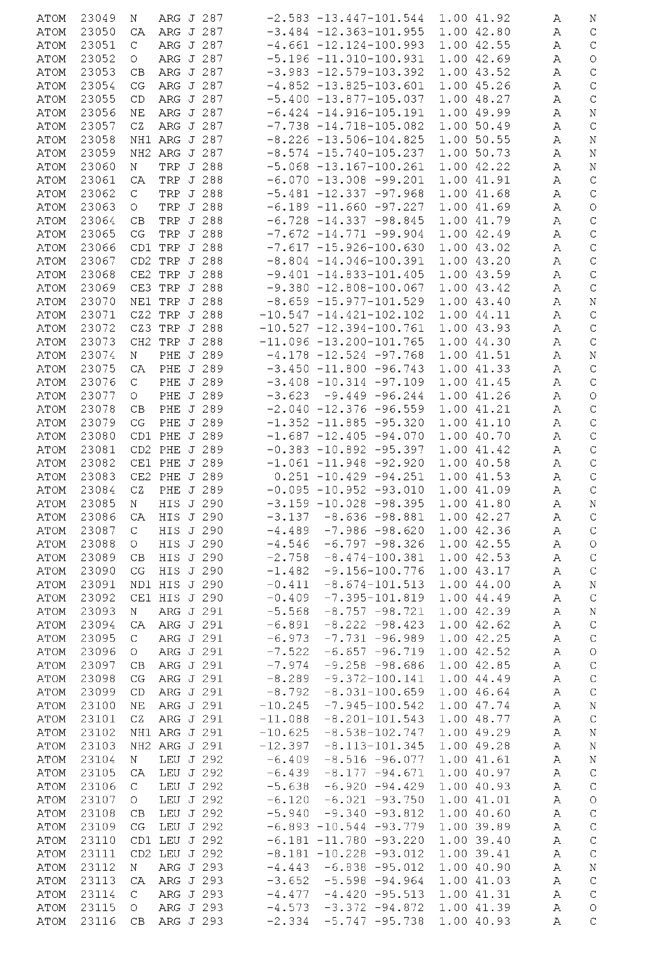

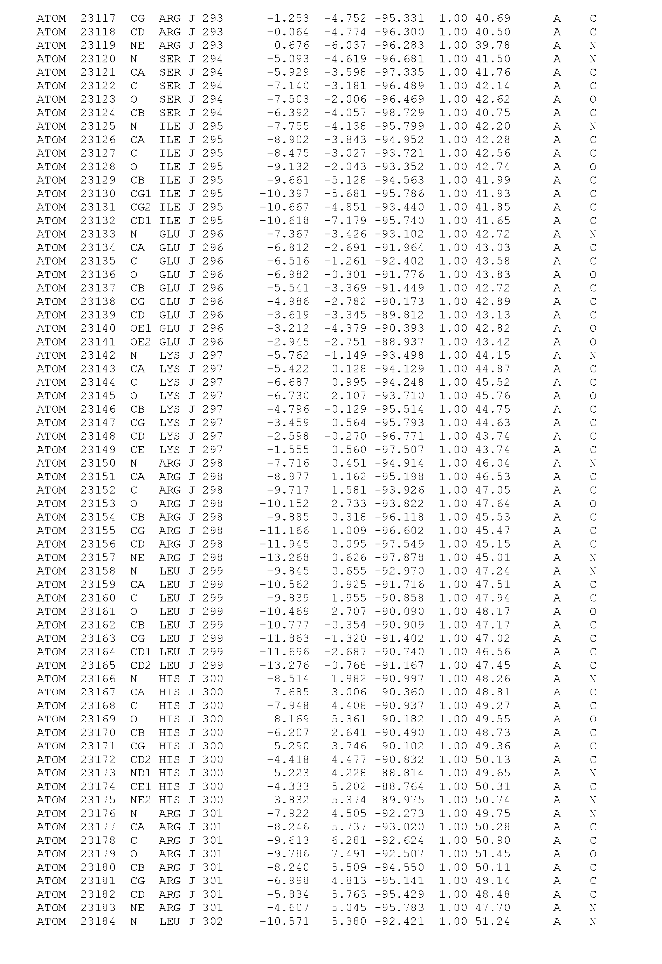

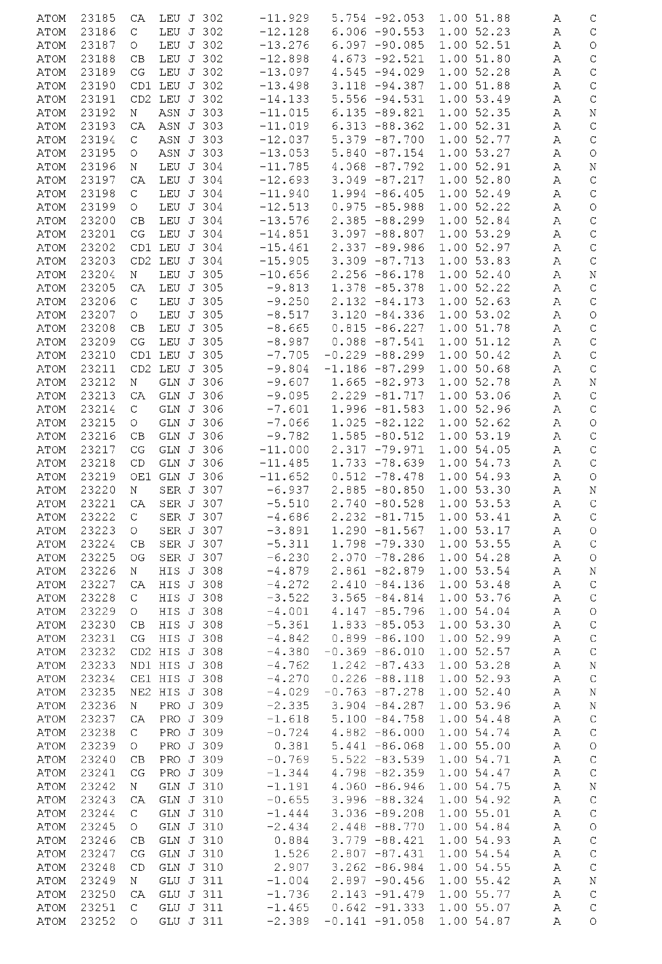

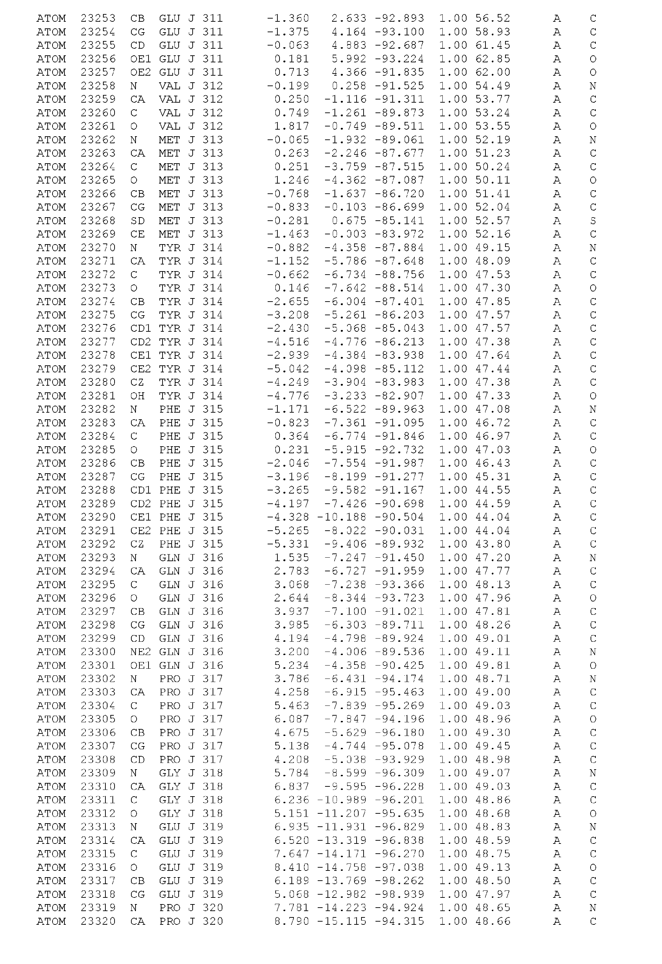

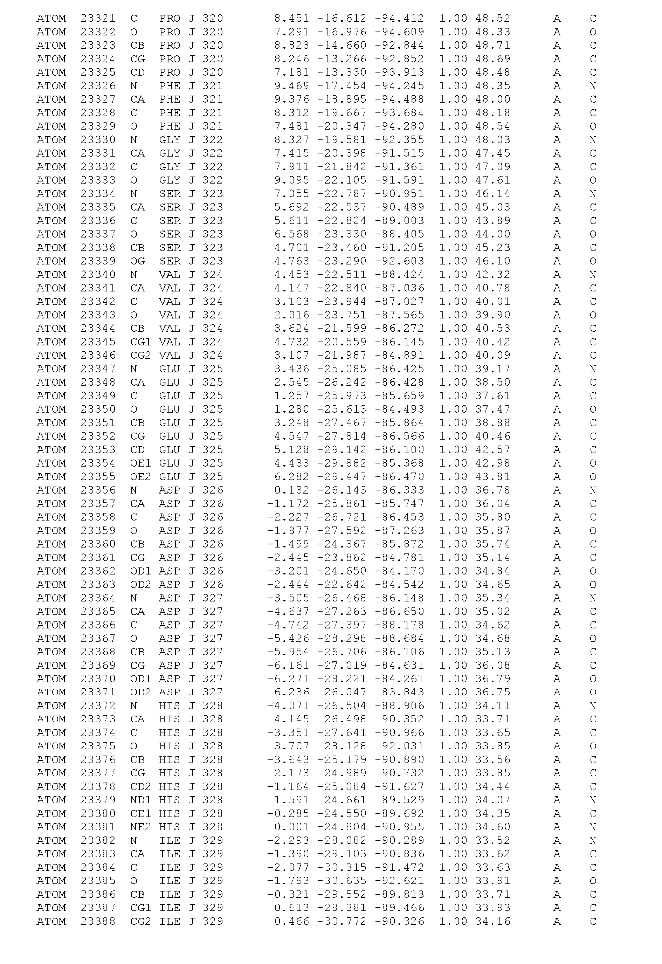

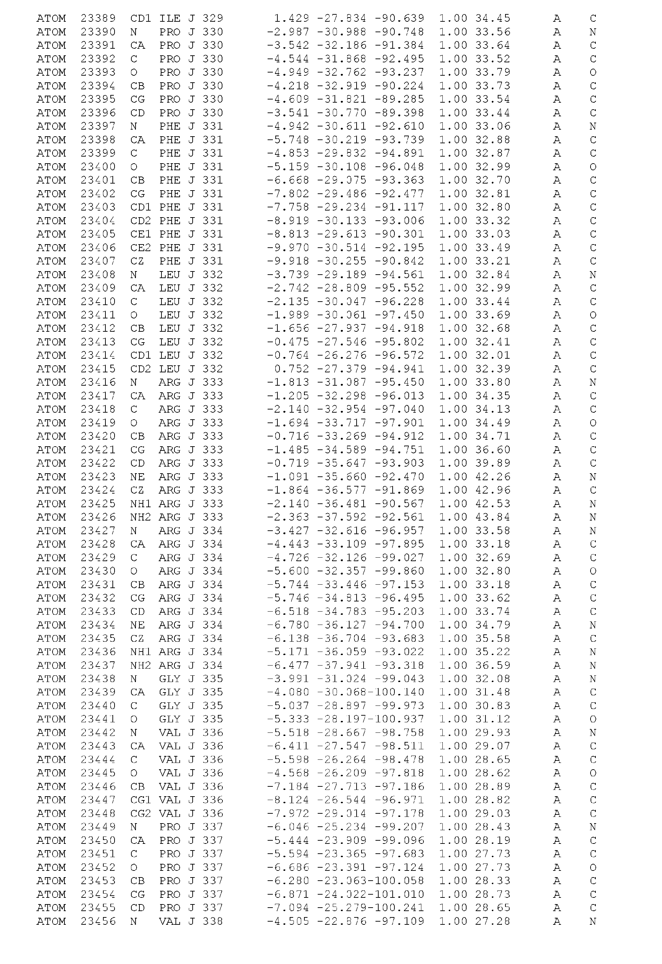

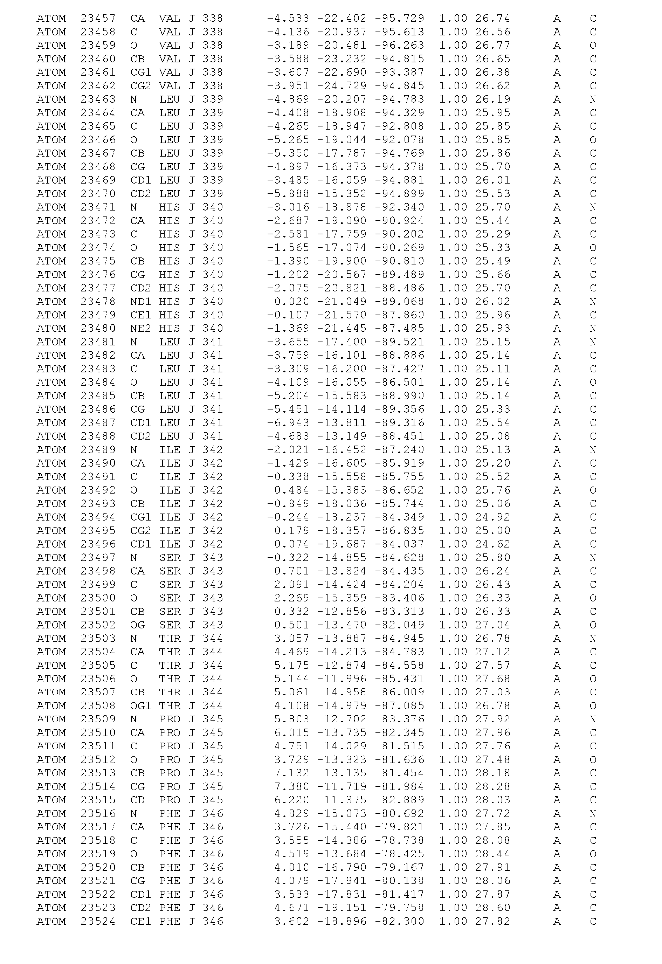

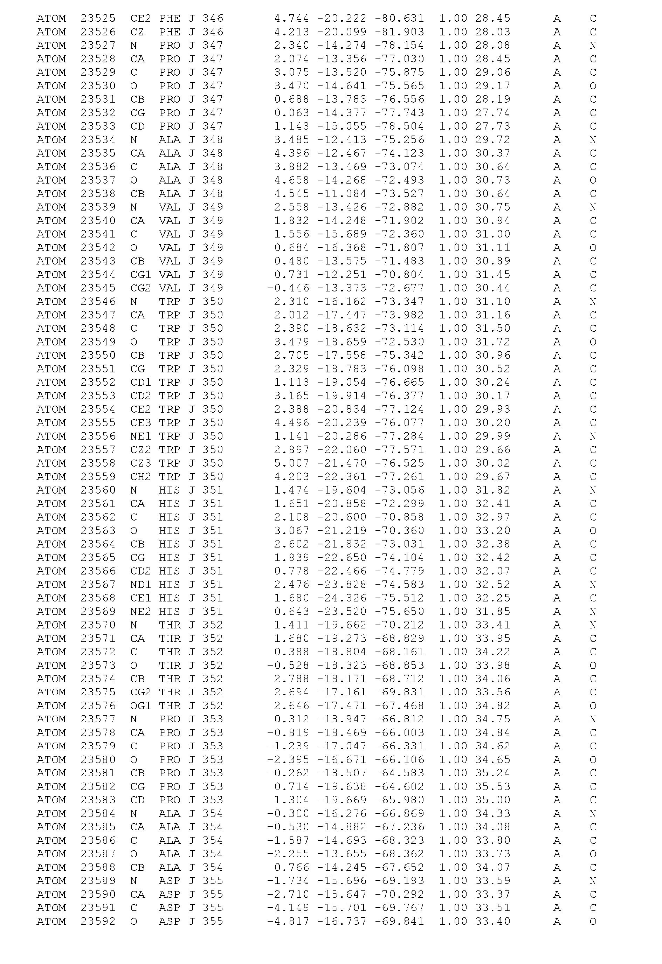

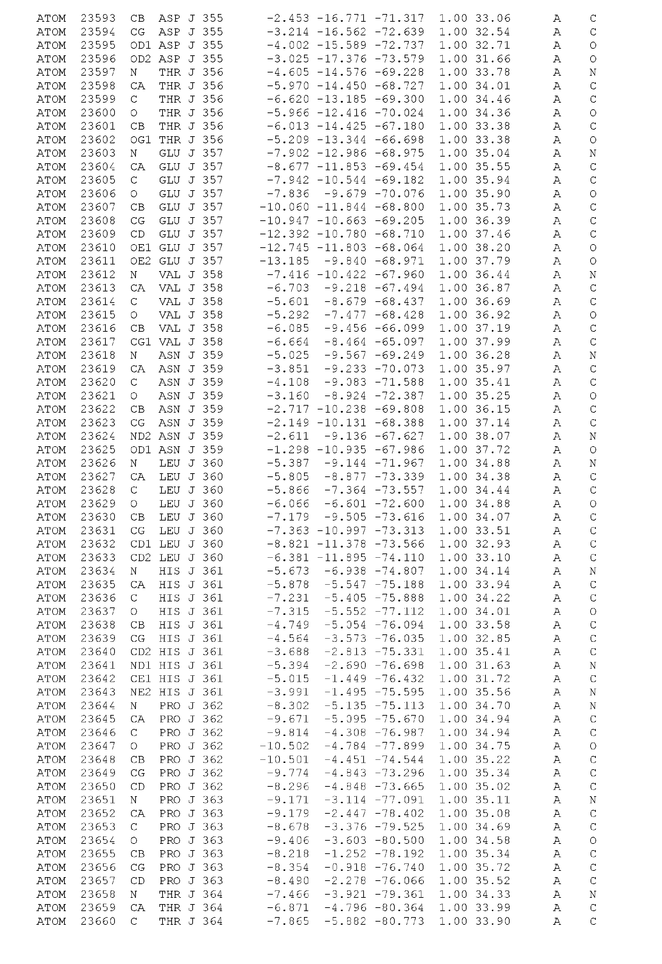

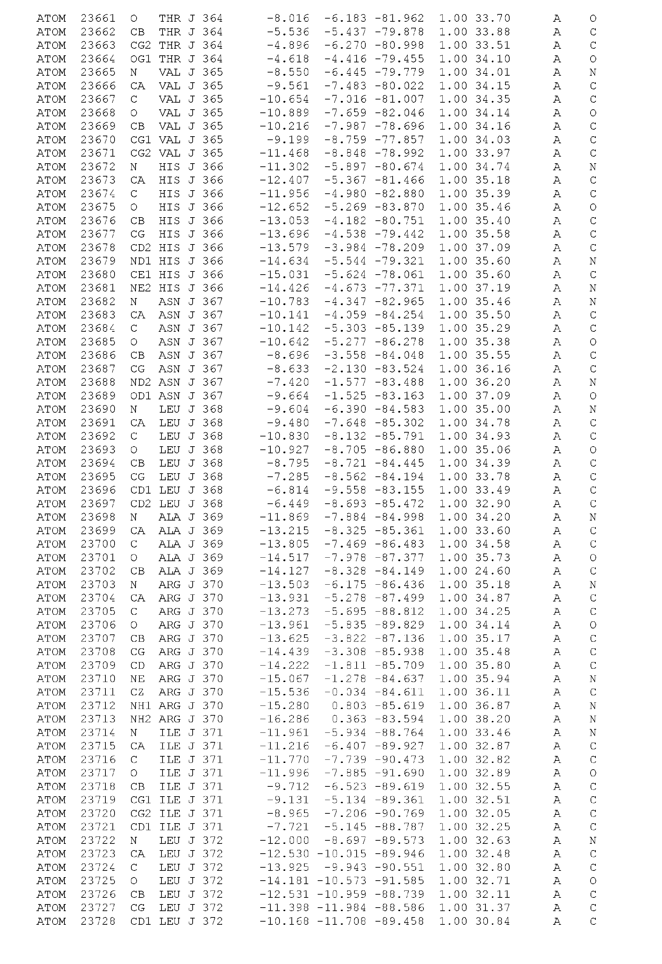

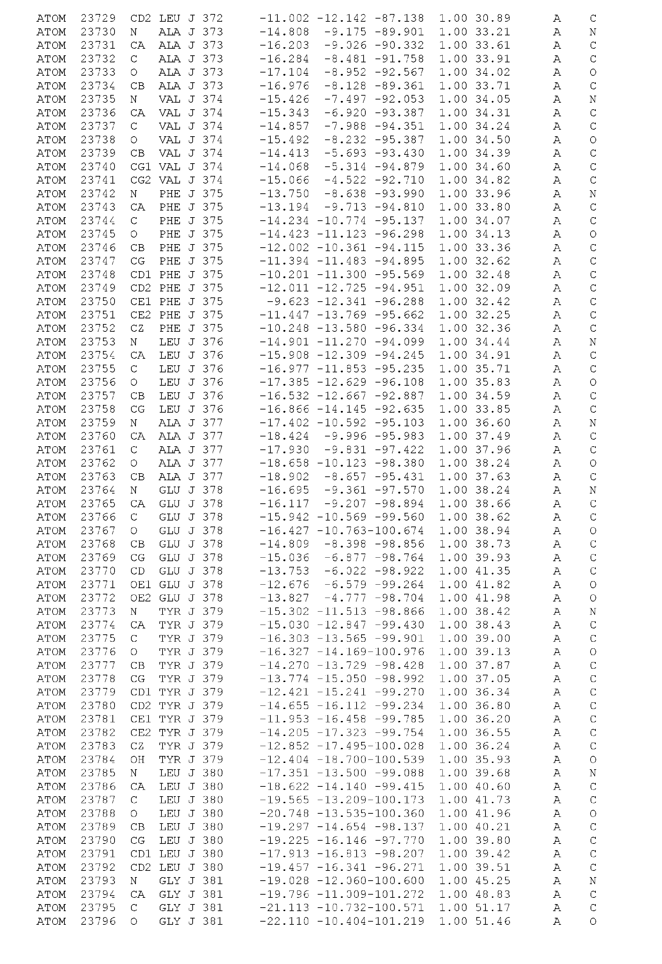

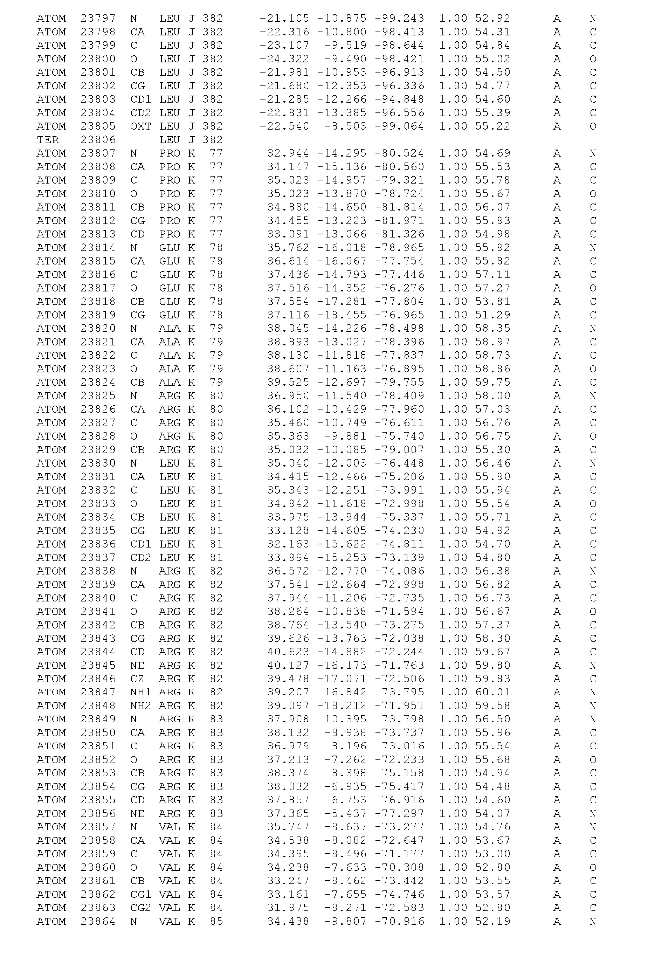

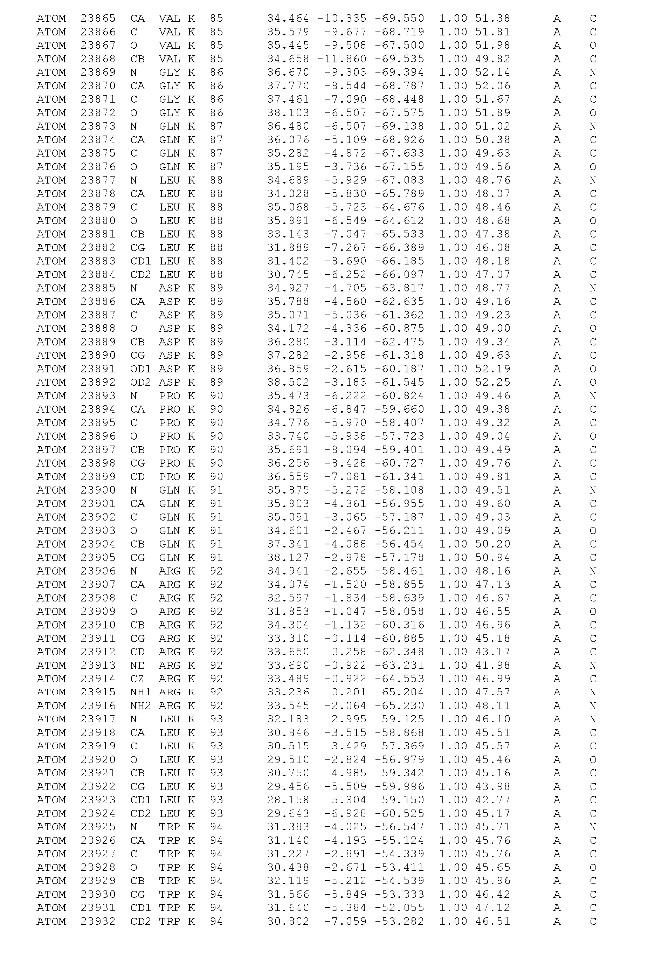

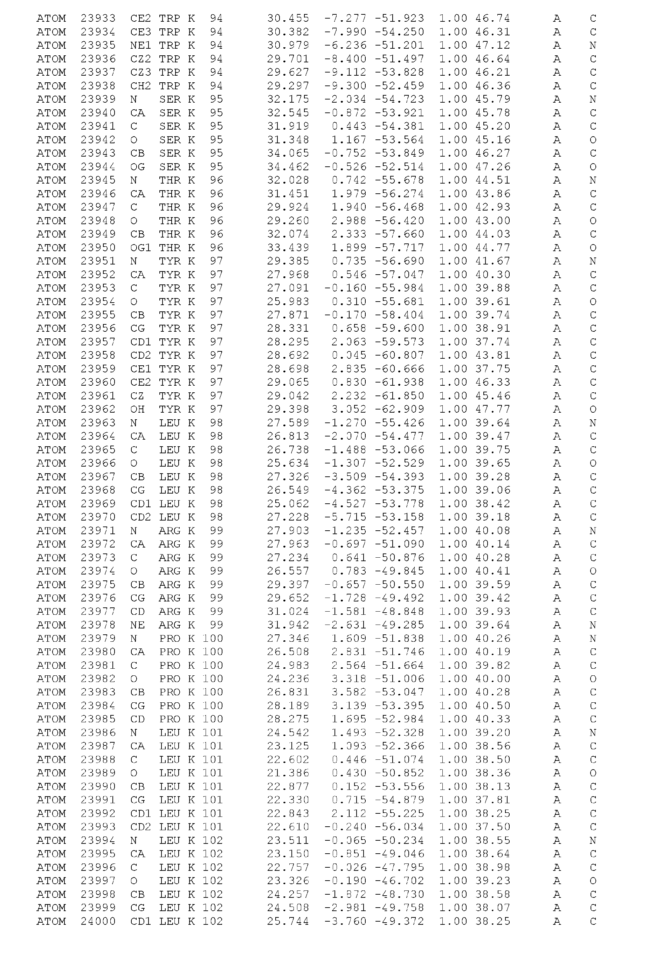

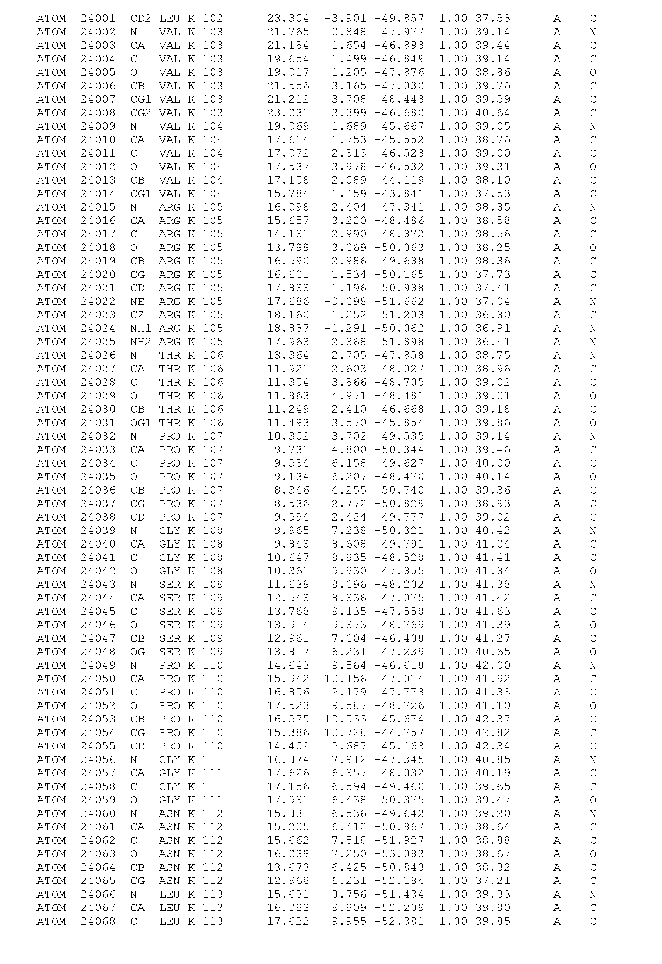

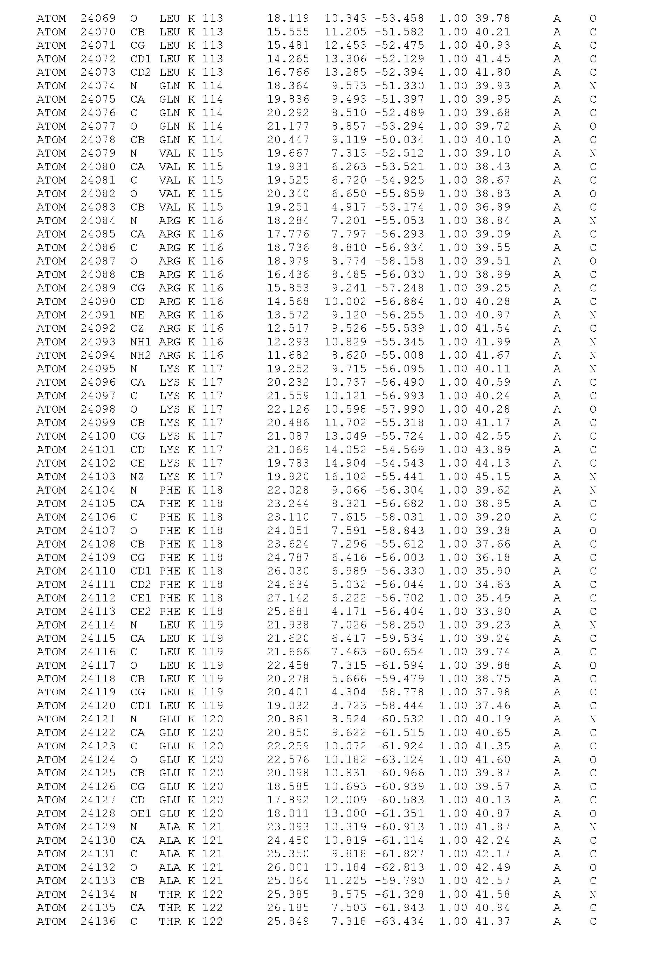

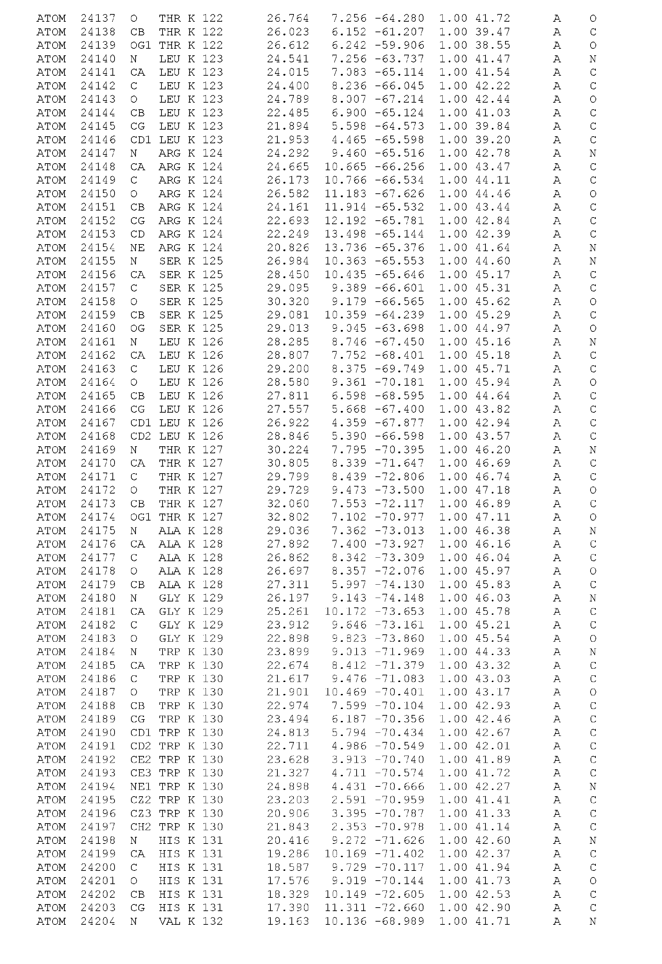

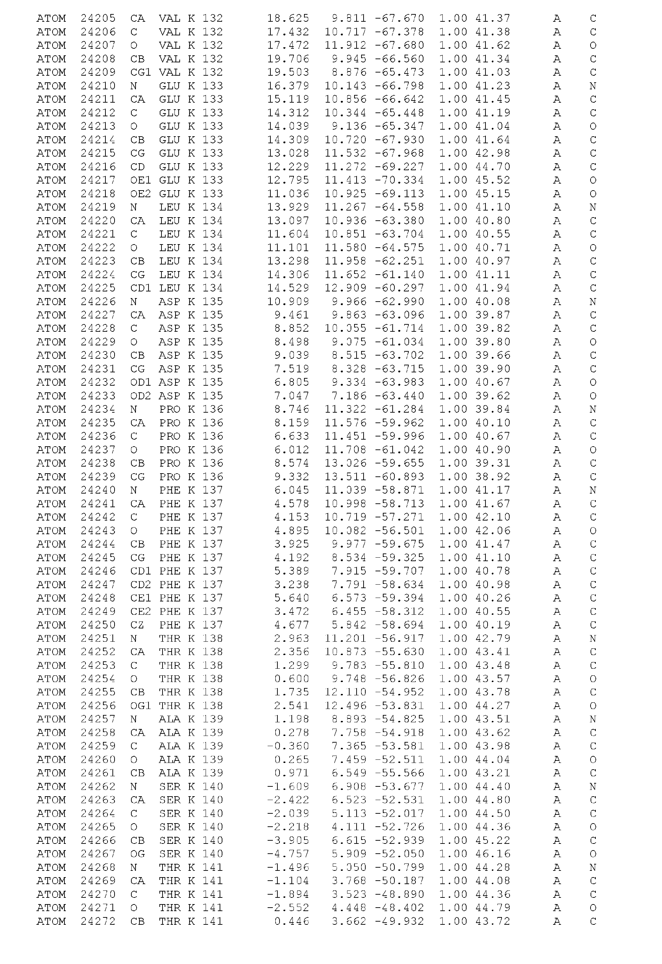

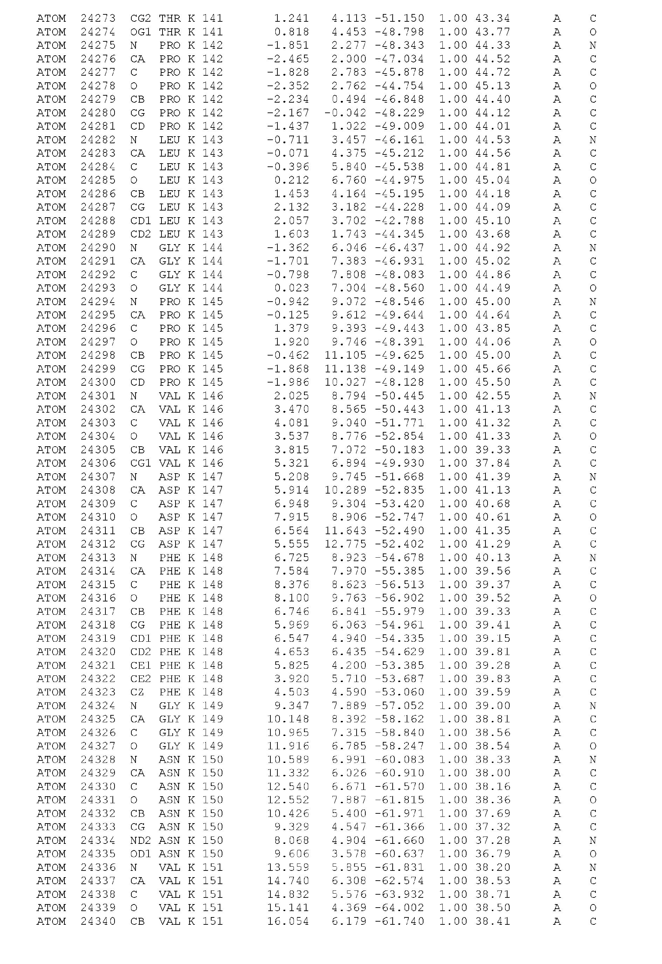

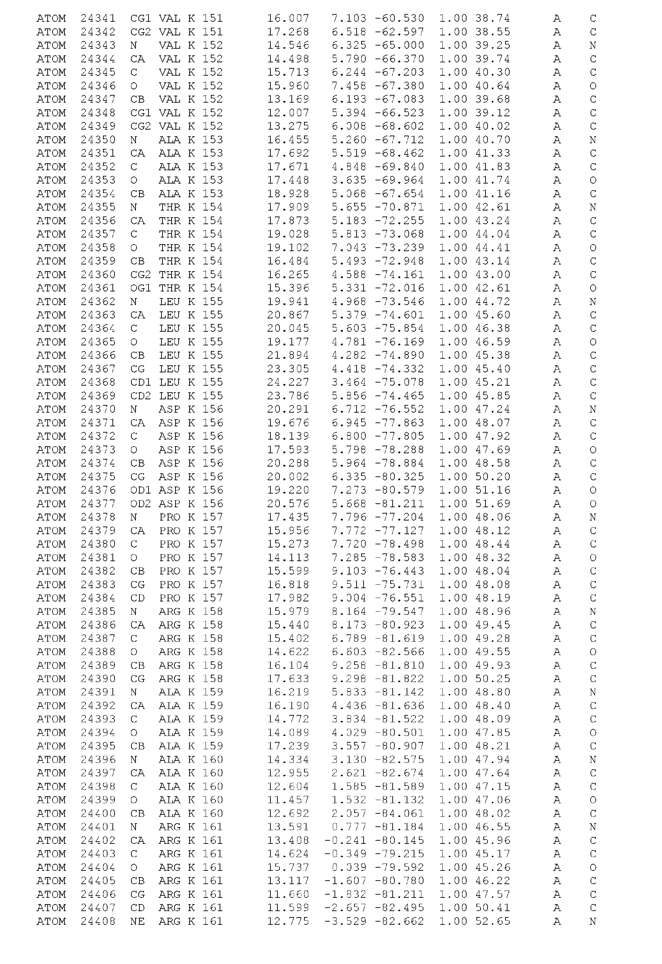

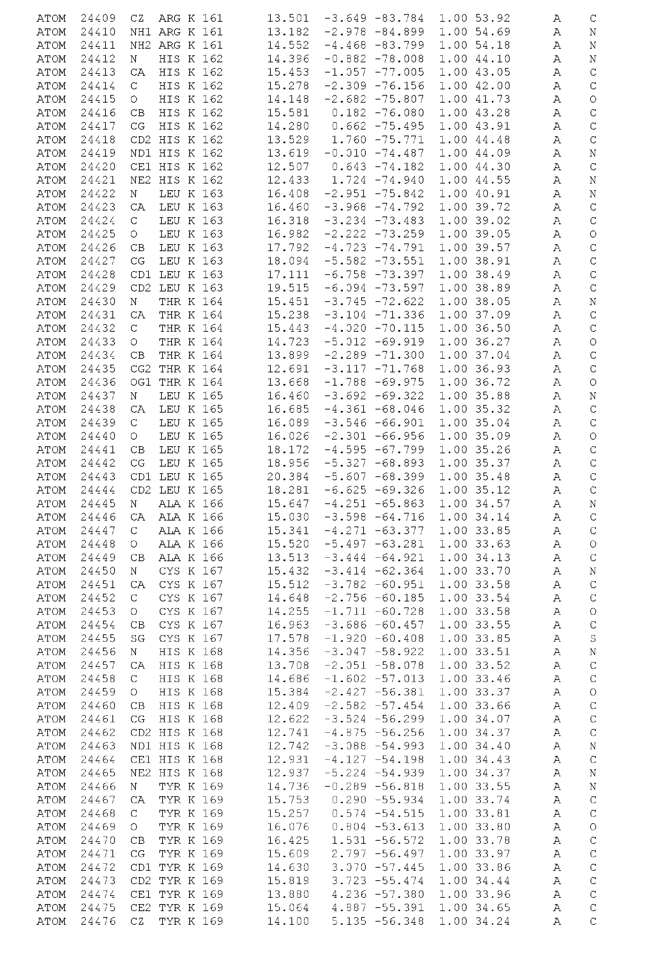

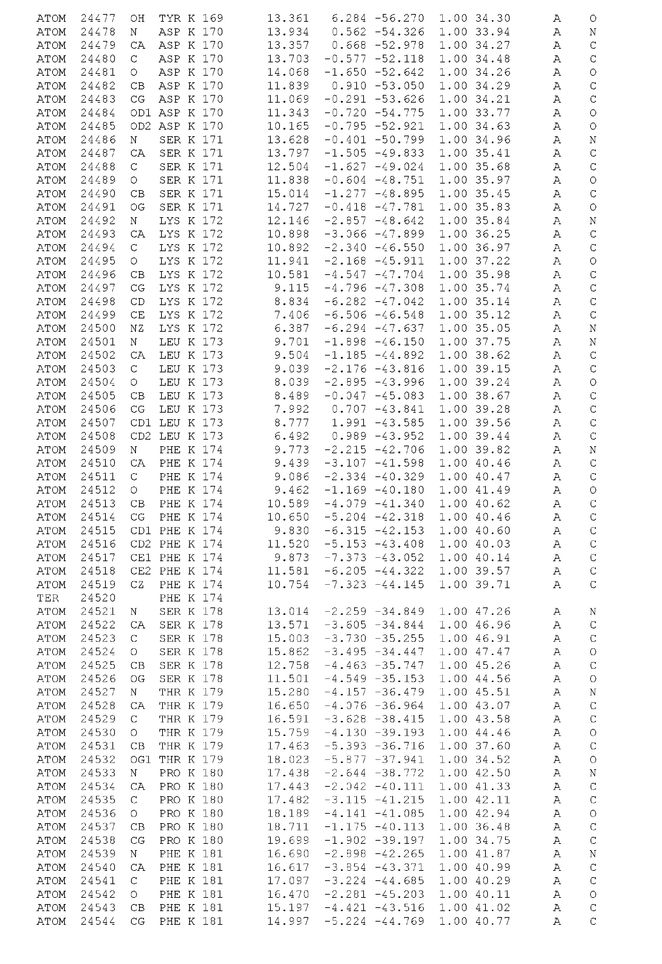

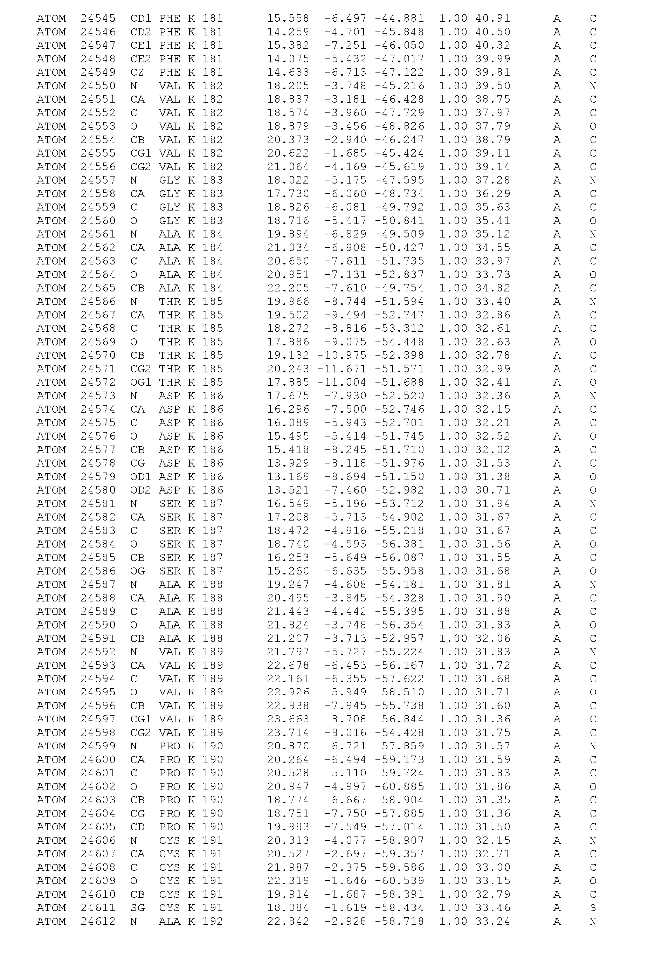

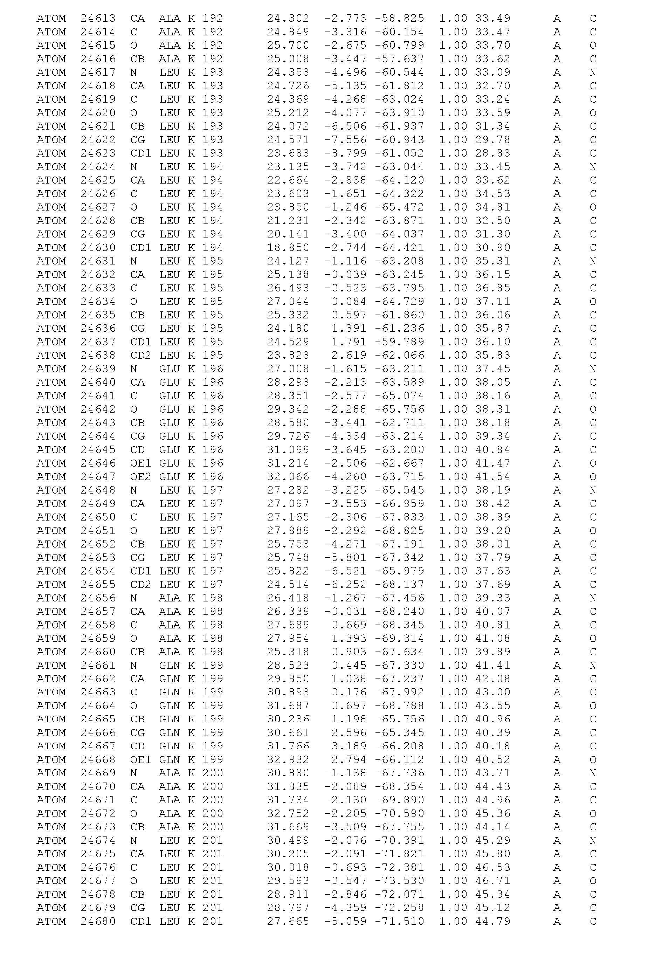

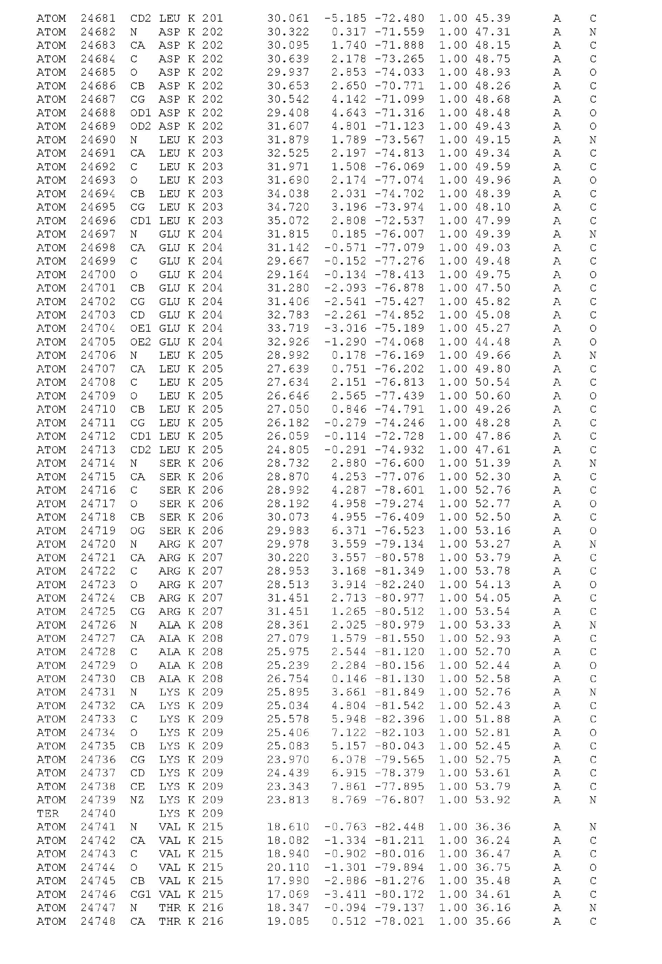

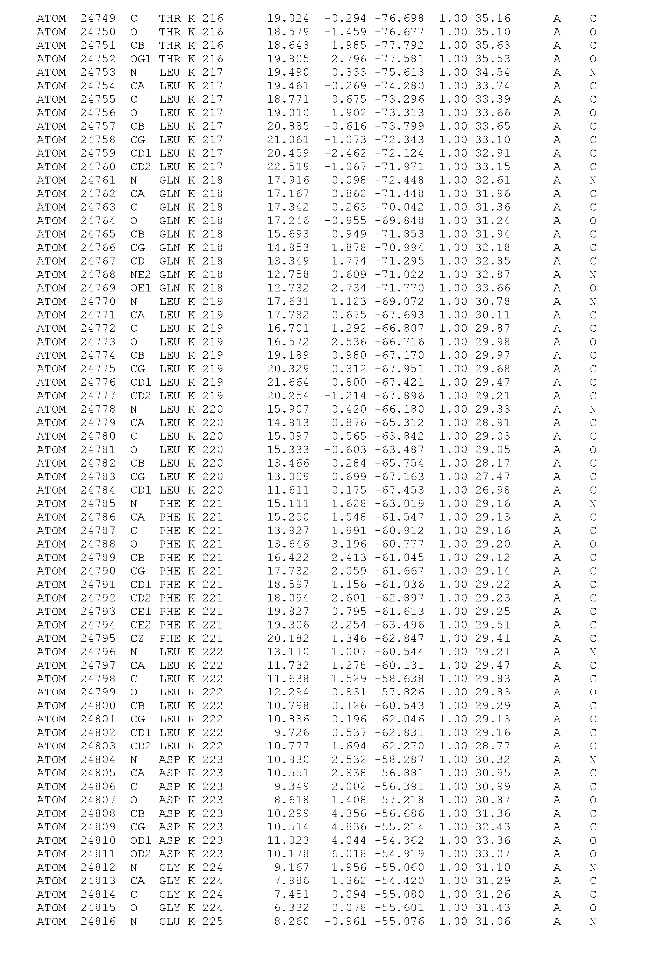

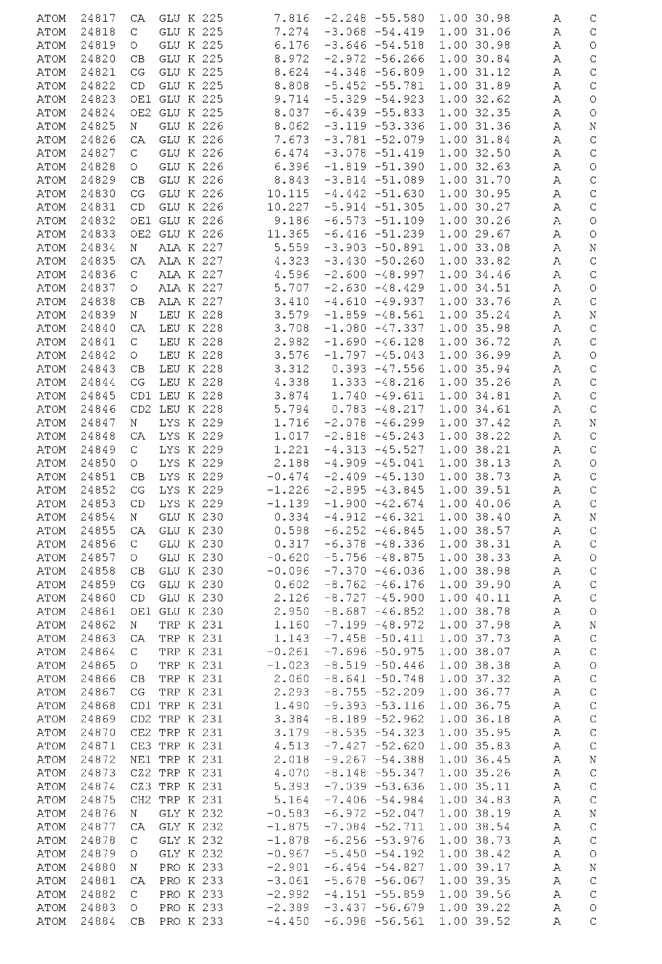

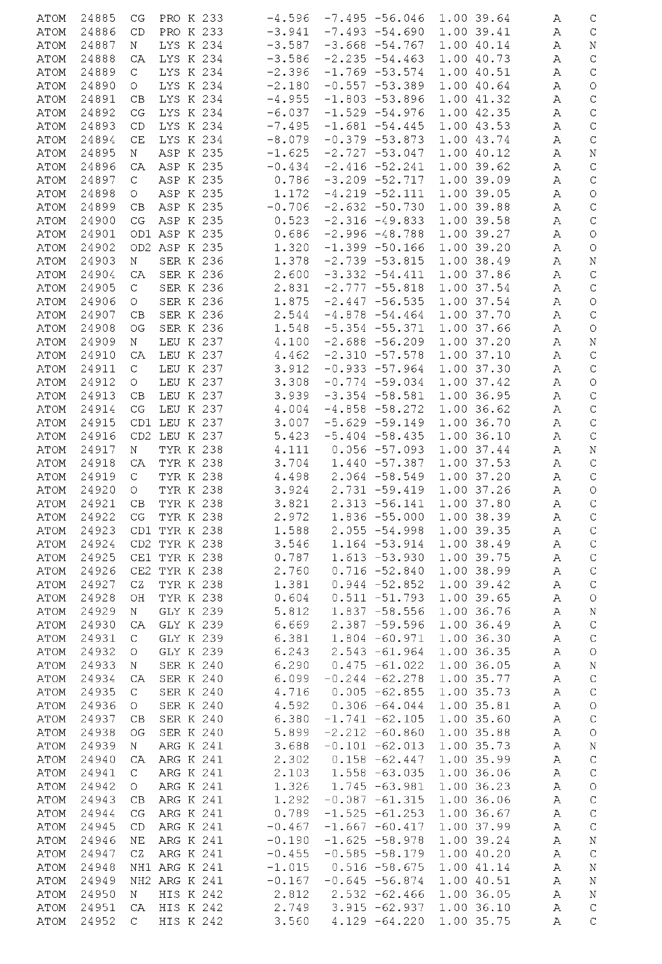

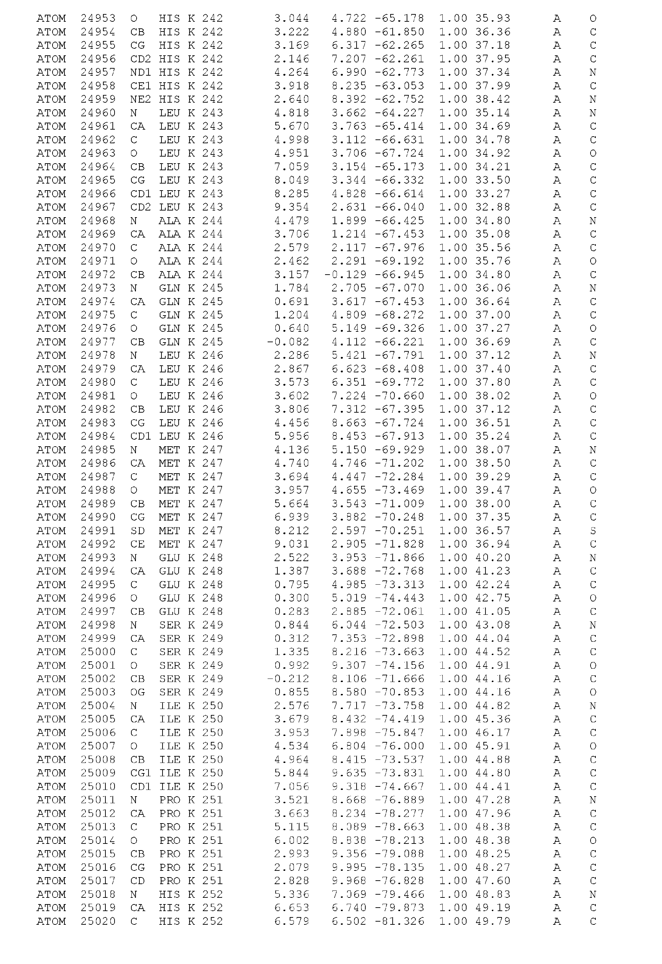

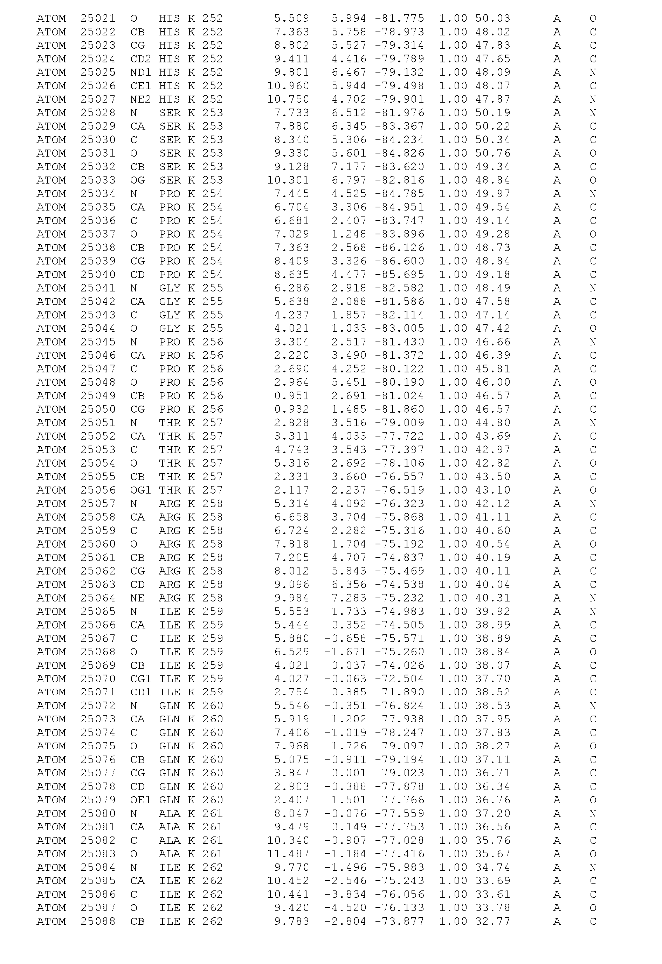

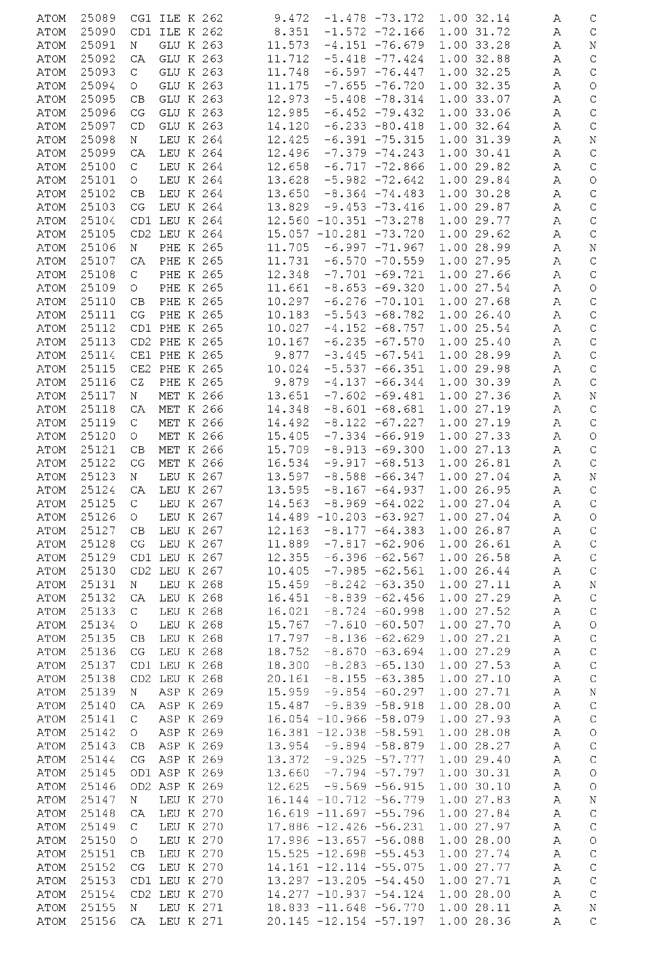

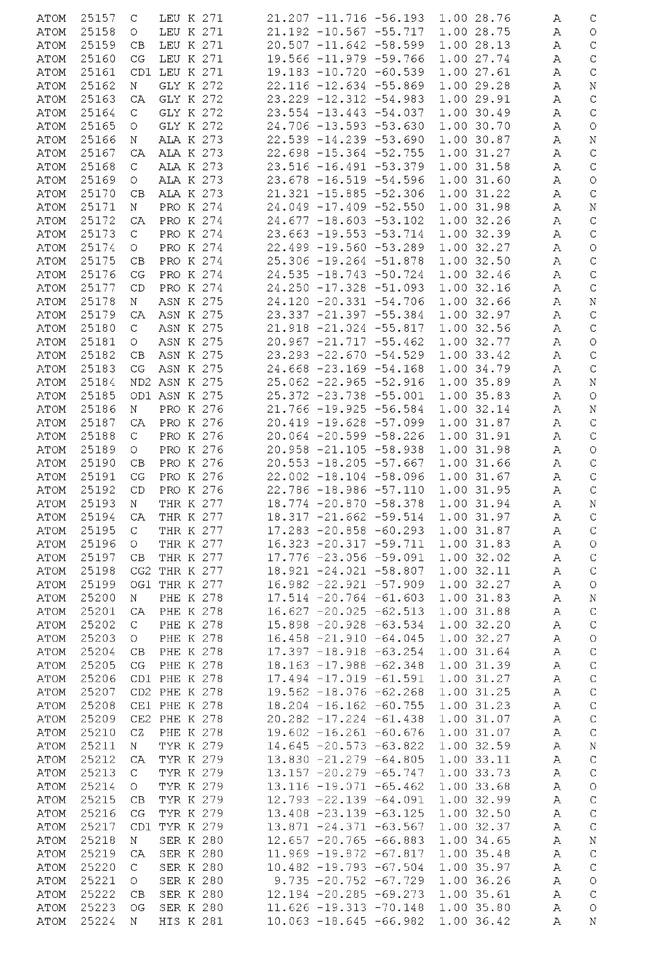

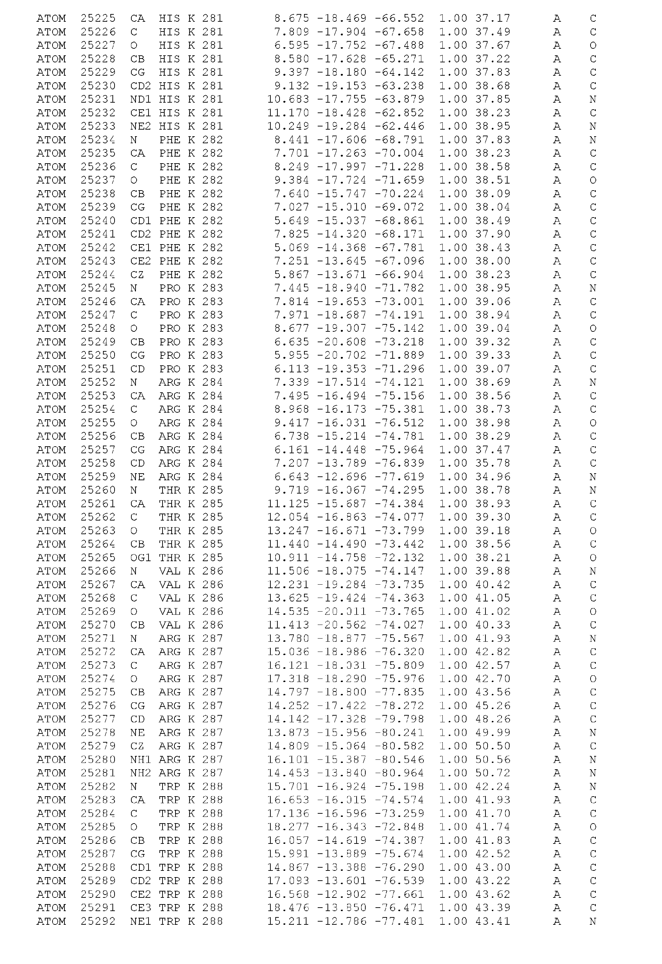

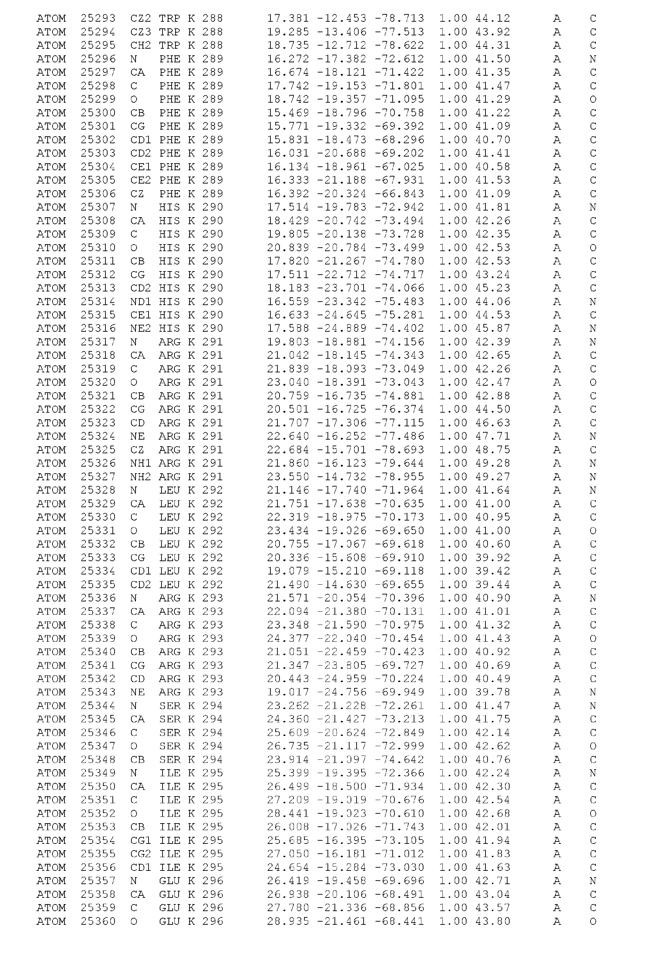

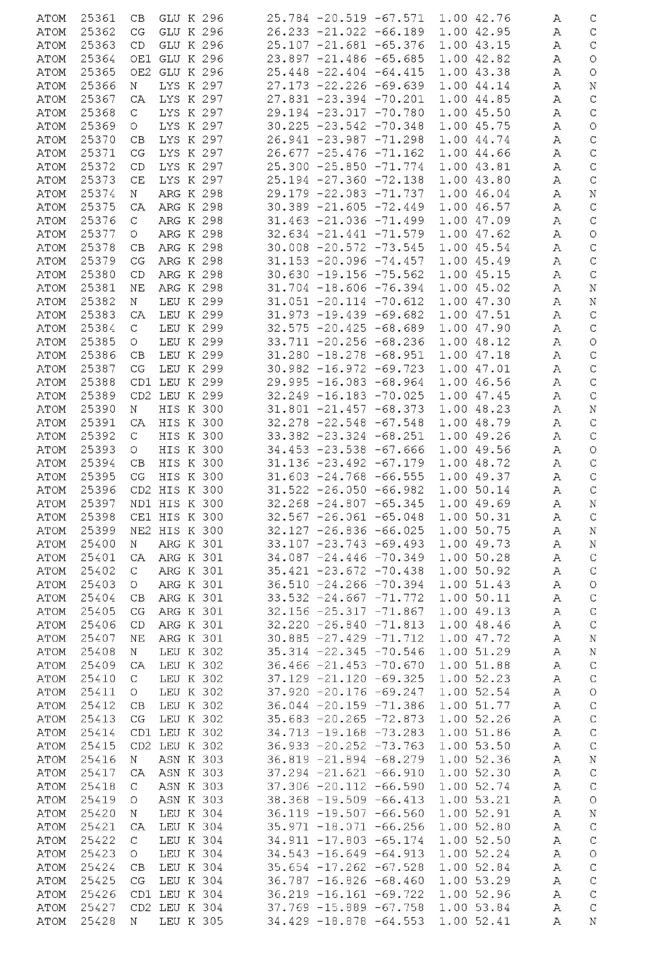

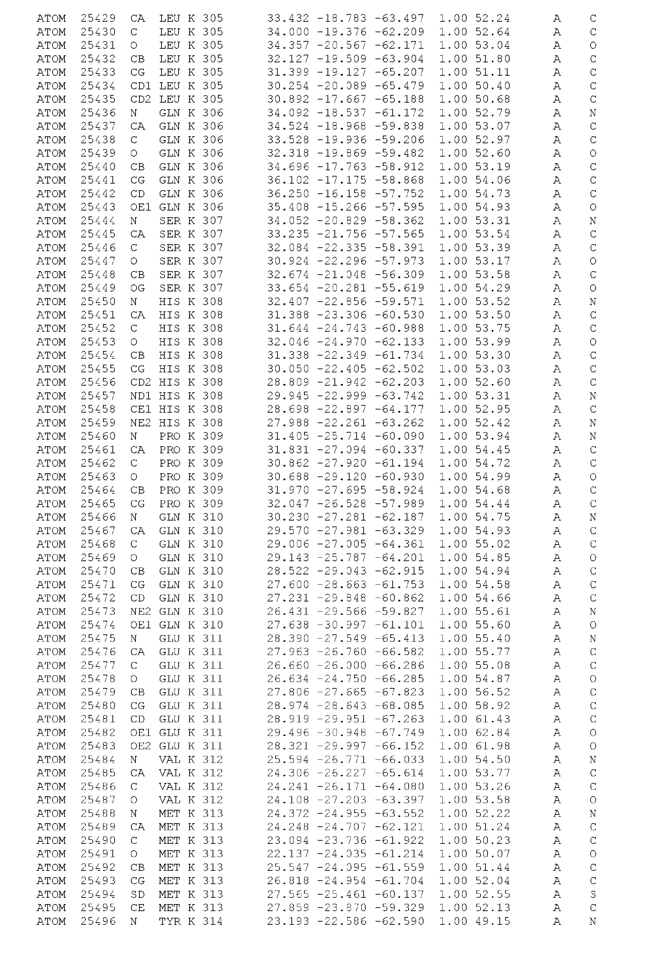

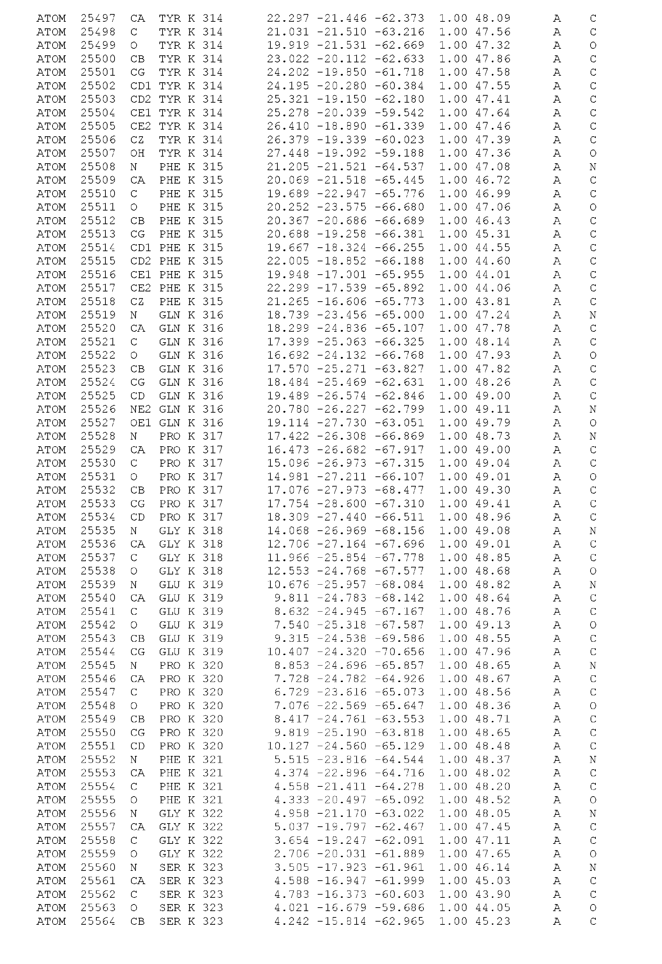

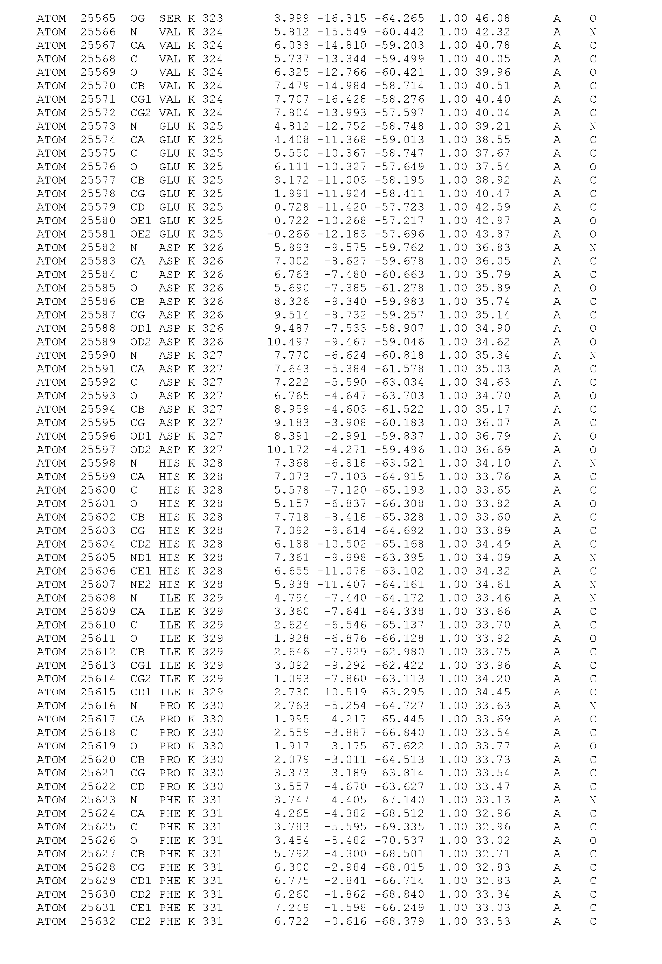

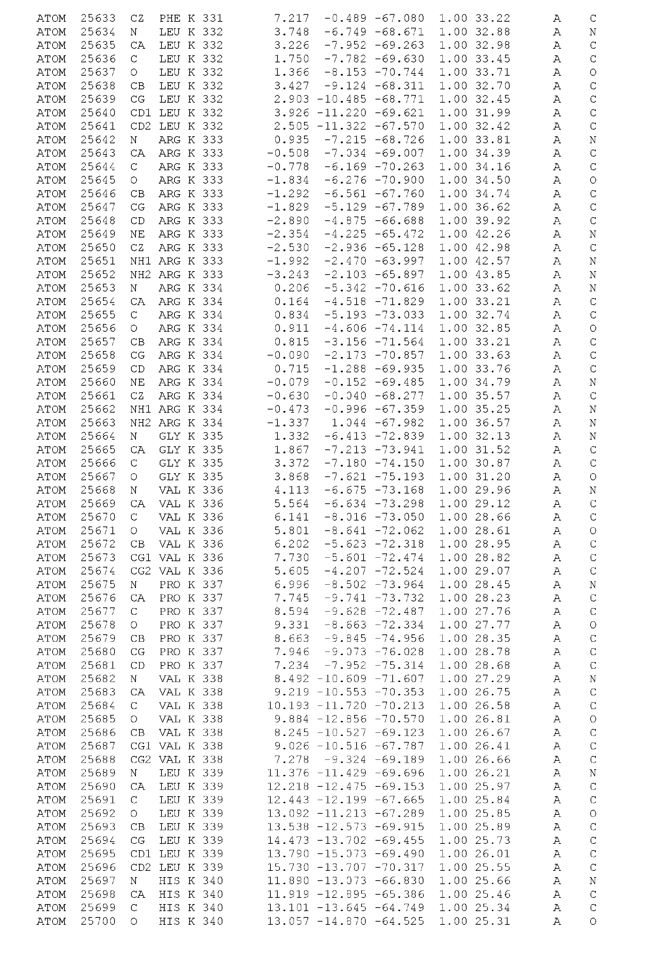

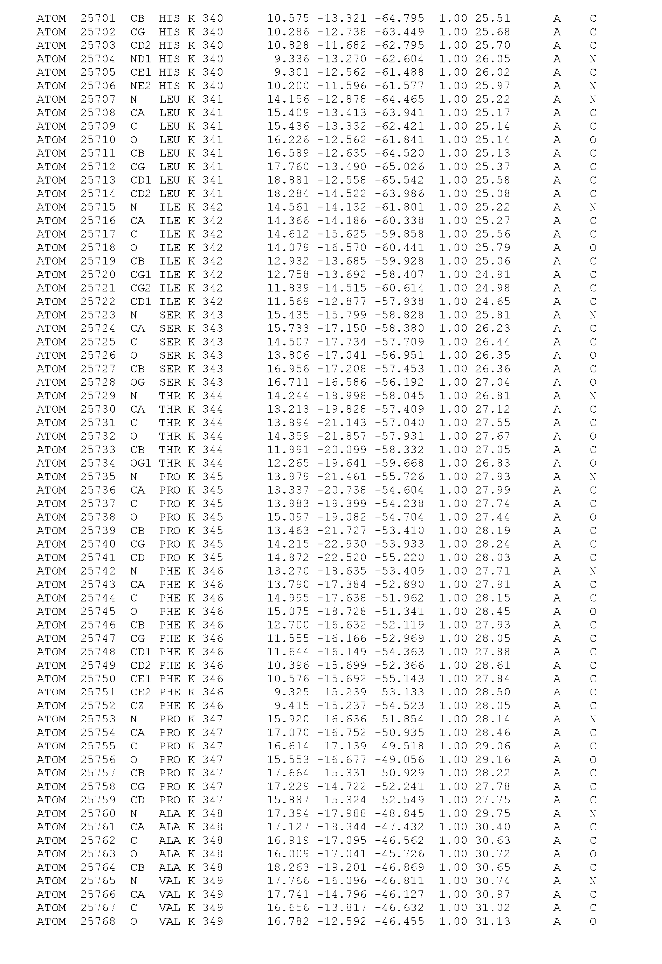

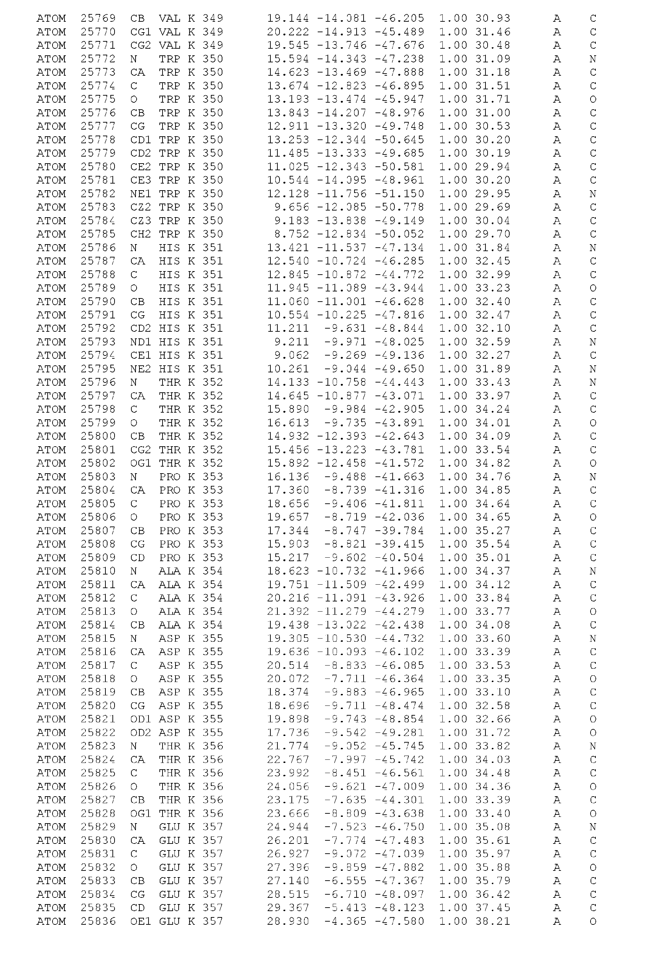

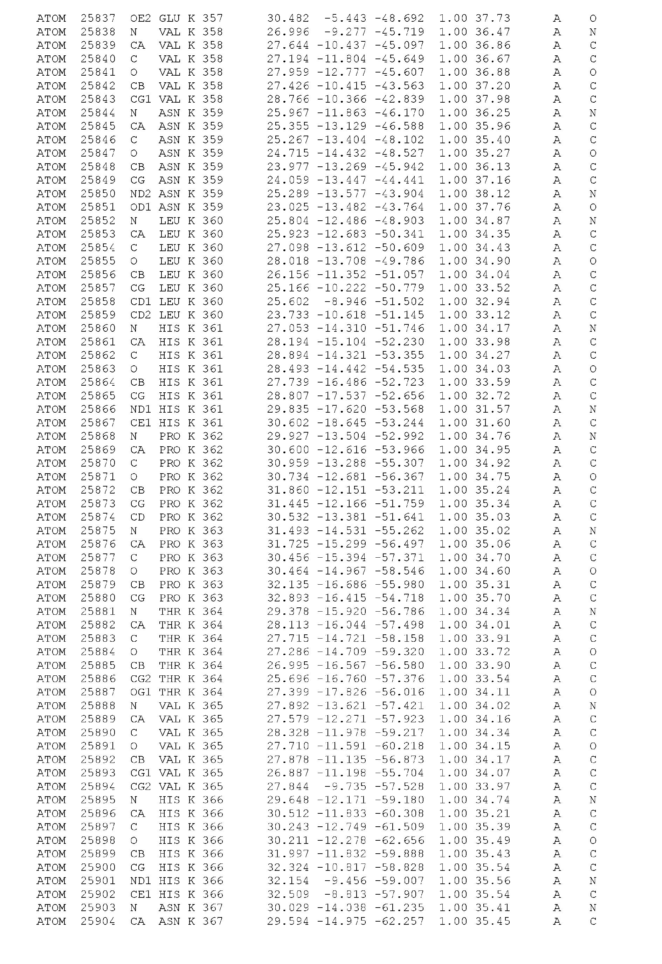

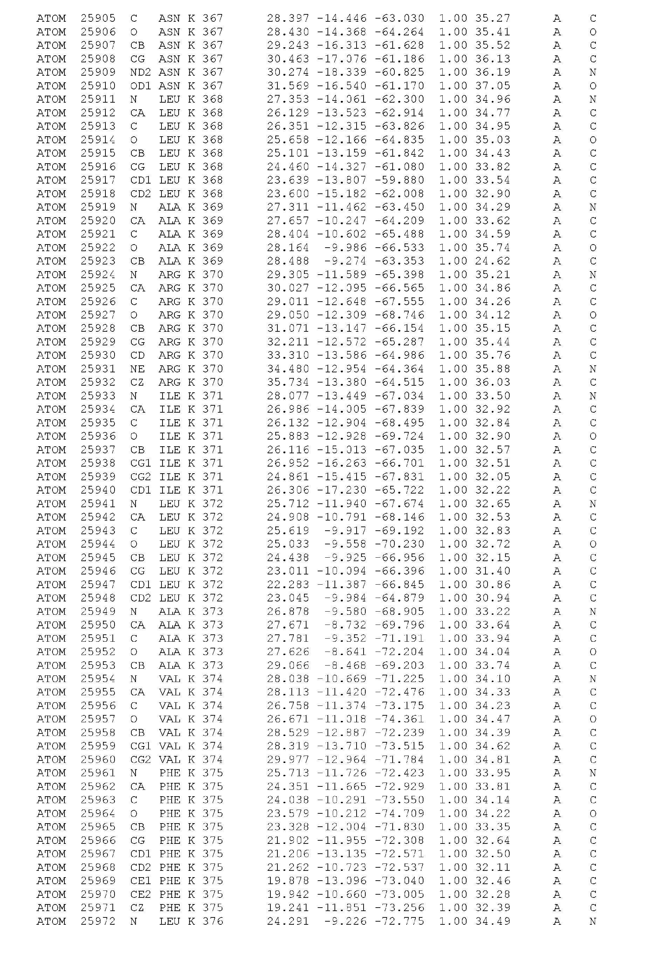

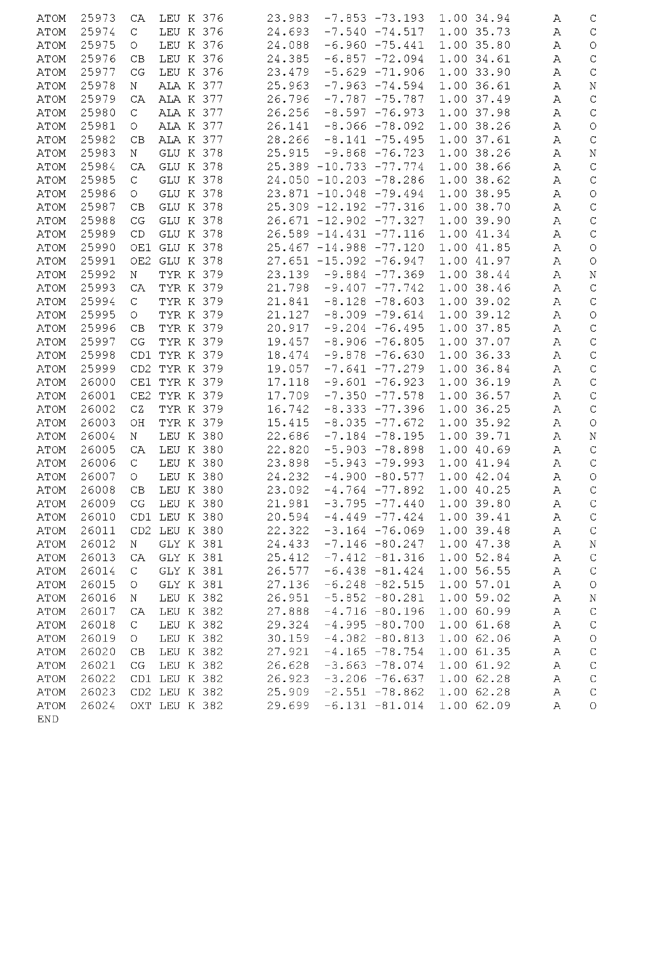

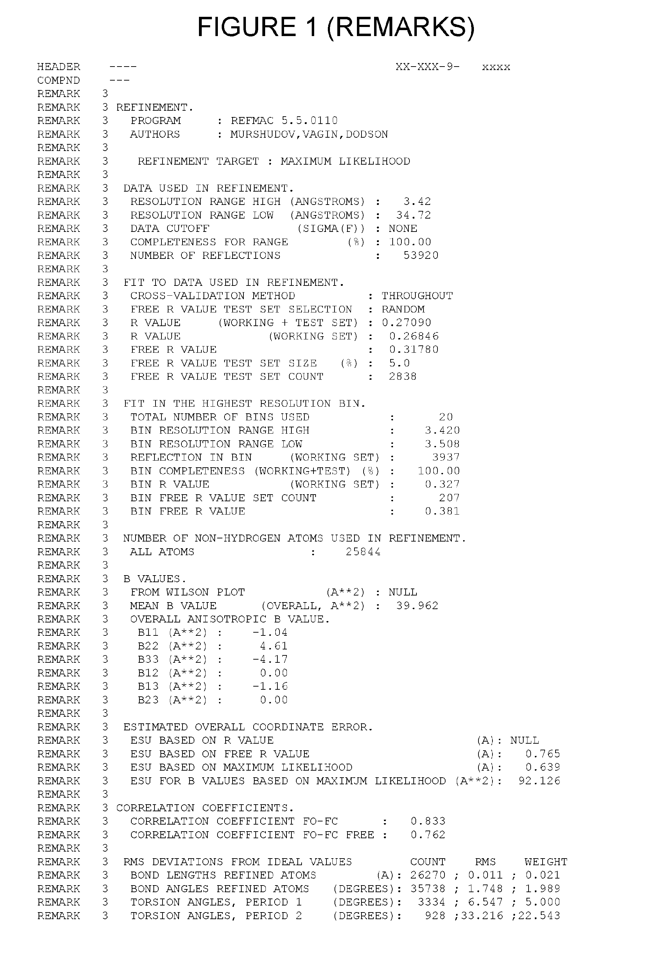

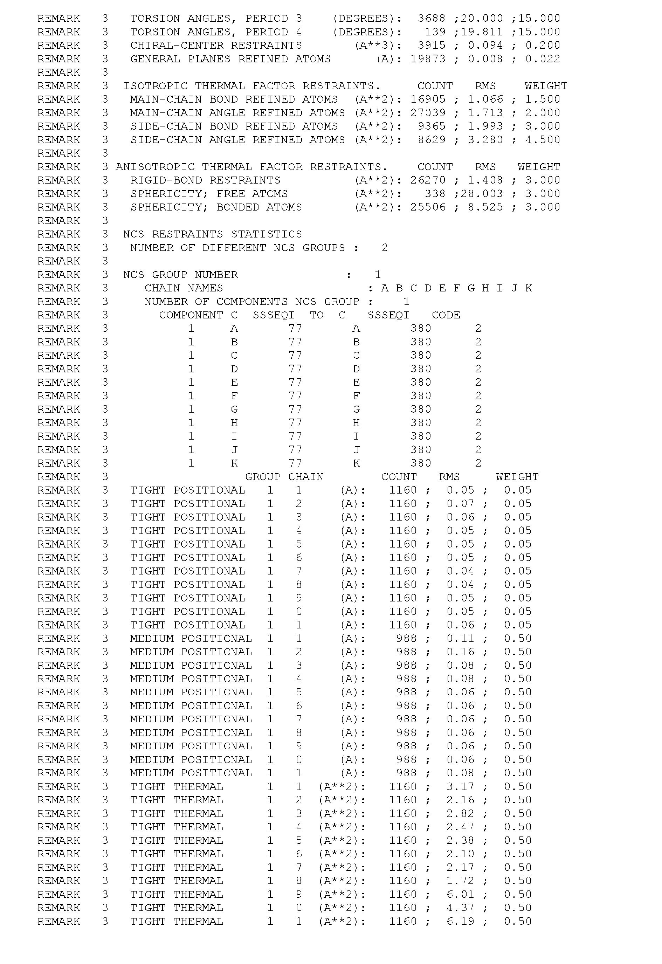

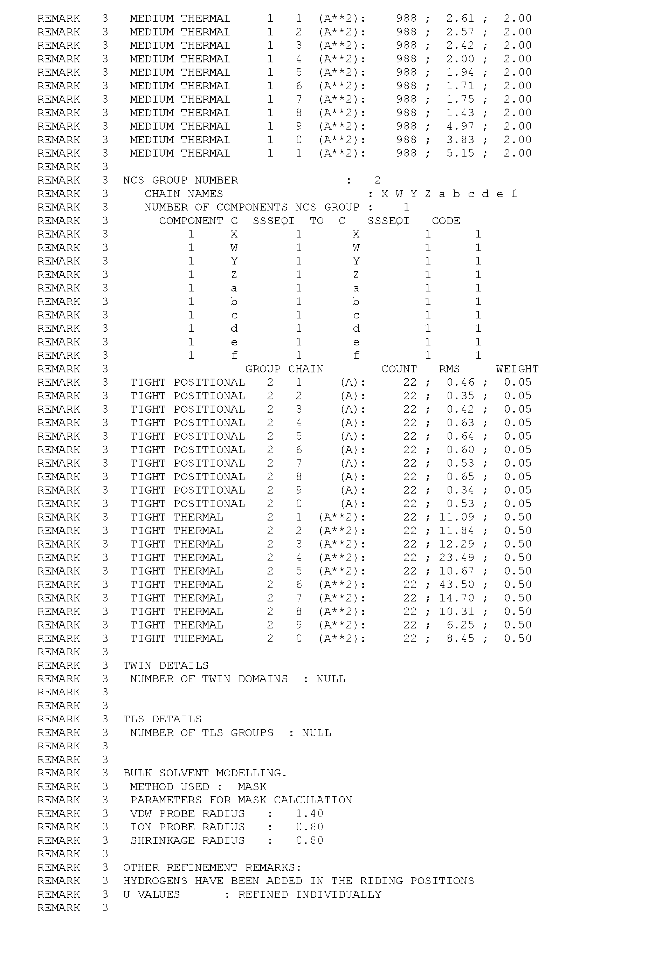

According to a fourth aspect of the invention there is provided a method of identifying an inhibitor of human isoglutaminyl cyclase which comprises the following steps:
(a) generating a 3-dimensional model of human isoglutaminyl cyclase using the structural coordinates described in Figure 1;
(b) analysing the binding pocket provided by residues E226, D186 and H351 of SEQ ID NO: 1 according to the coordinates of Figure 1;
(c) performing computer modeling analysis to identify an inhibitor compound which may associate with the binding pocket of human isoglutaminyl cyclase.

### BRIEF DESCRIPTION OF THE FIGURES

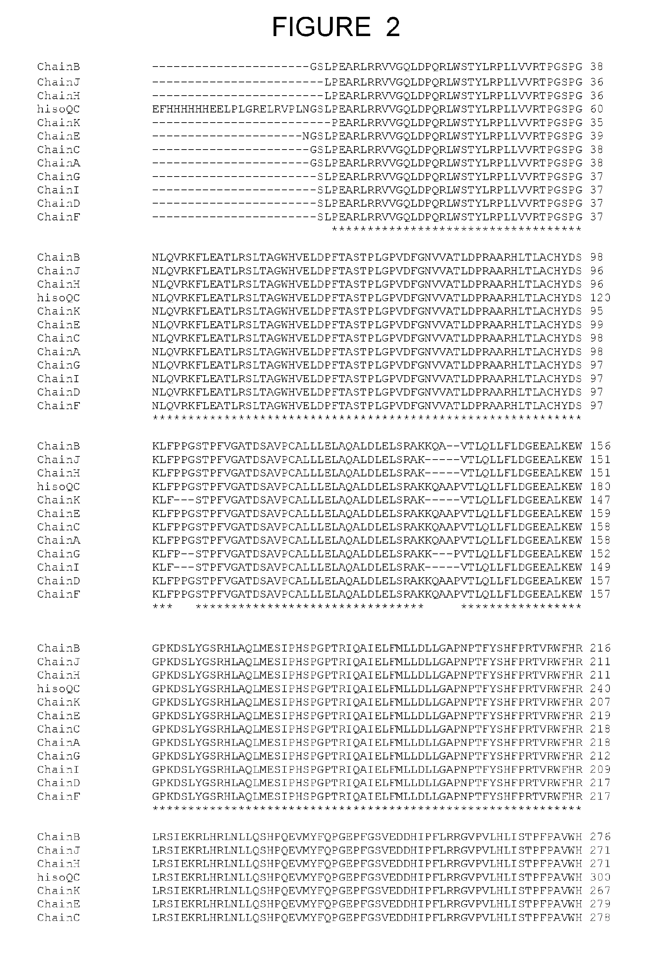

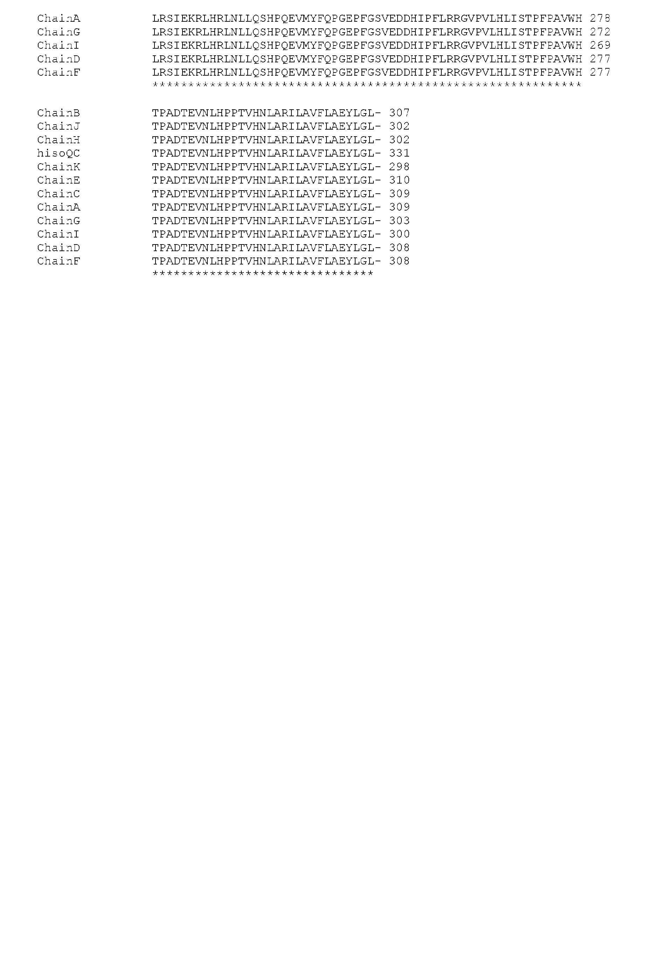

**Figure 1** describes the X-ray coordinates of human isoQC crystallized with 1mM Inhibitor A. The crystal structure contains 11 molecules in the AU (designated chain A to chain K). The sequences of these different chains are as follows:
   chain A (SEQ ID NO: 2): A74-A383
   chain B (SEQ ID NO: 3): G74-L382
   chain C (SEQ ID NO: 4): G74-L382
   chain D (SEQ ID NO: 5): A75-L382
   chain E (SEQ ID NO: 6): A73-L382
   chain F (SEQ ID NO: 7): A75-L382
   chain G (SEQ ID NO: 8): A75-L382
   chain H (SEQ ID NO: 9): L76-L382
   chain I (SEQ ID NO: 10): A75-L382
   chain J (SEQ ID NO: 11): L76-L382
   chain K (SEQ ID NO: 12): P77-L382
**Figure 2** shows the sequence alignment of chains A-K (SEQ ID NOS: 2-12) in comparison with the expressed human isoQC as described herein (SEQ ID NO: 19).
**Figure 3** shows the purification of human isoQC, based on construct YSShisoQCE42I55NC351A N-His, from the medium of a transgenic *P.pastoris* strain. The isoQC was purified by a combination of IMAC (immobilized metal affinity chromatography, lane 3), HIC (hydrophobic interaction chromatography, lane 4) and desalting (lane 5). The glycosylation of the enzyme was evidenced by enzymatic deglycosalytion, which resulted in a shift in migration of the protein (lane 6). Lane 1, protein standard: Lane 2, medium prior to purification.
**Figure 4** shows crystals of human isoglutaminyl cyclase grown in buffer consisting of 0.1M sodium citrate 0.1 M ammonium sulfate pH 6.5 and 13% (w/v) 35k PEG.
**Figure 5** describes a representation of the arrangement of the eleven human isoglutaminyl cyclase molecules contained in the AU with the unit cell as background shown with axes.
**Figure 6** describes a cartoon representation of the obtained three dimensional structure of the human isoQC of (chain A) described by the coordinates shown in Figure 1. The structure is shown in two orthogonal views. The structure clearly comprises β-sheet structures, α-helices and random coiled loops.
**Figure 7** describes a detailed representation of the modeled active site of chain A in complex with an inhibitory molecule. Stick representation of the active site related residues of human isoQC forming complex with Inhibitor A with the catalytic zinc ion shown with a sphere.

### DETAILED DESCRIPTION OF THE INVENTION

The present invention relates to crystals of human isoglutaminyl cyclase, where the crystals are of sufficient quality and size to allow for the determination of the three-dimensional X-ray diffraction structure of isoglutaminyl cyclase to a resolution of about 3.4 angstrom in a P1211 space group. The invention also relates to methods for preparing and crystallizing human isoglutaminyl cyclase. The crystal structure of human isoglutaminyl cyclase, as well as information derived from the crystal structure, can be used to analyze and modify isoglutaminyl cyclase as well as to identify compounds that interact with isoglutaminyl cyclase.

According to a first aspect of the invention there is provided a crystal comprising human isoglutaminyl cyclase having a characterised space group of P1211 and unit cell dimensions of +/- 5% of a = 126.51 Å, b = 109.68 Å, c = 159.53 Å, α = 90.0°, β = 104.9° and γ = 90.0°.

In one embodiment of the first aspect of the invention, the crystal has unit cell dimensions of a = 126.51 Å, b = 109.68 Å, and c = 159.53 Å.

In one embodiment of the first aspect of the invention, the crystal has unit cell dimensions of α = 90.0°, β = 104.9° and γ = 90.0°.

In one embodiment of the first aspect of the invention, the crystal diffracts x-rays for determination of atomic co-ordinates of the crystal to a resolution of between 3.42 Å, and 34.72 Å.

In one embodiment of the first aspect of the invention, the crystal has unit cell dimensions of a = 126.51 Å, b = 109.68 Å and c = 159.53 Å, α = 90.0°, β = 104.9° and γ = 90.0°. This embodiment of the first aspect of the invention relates to the crystal structure of human isoglutaminyl cyclase complexed with an glutaminyl cyclase inhibitor referred to herein as Inhibitor A which has the structure as follows:

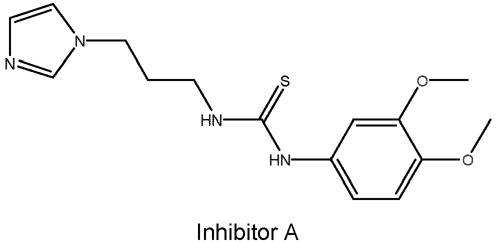

Inhibitor A inhibits both human and mouse QC *in vitro* and moreover, Inhibitor A is soluble in water and therefore useful for crystallization.

It will be appreciated that the crystals within the scope of the invention include both apo crystals and co-crystals. The apo crystals generally comprise substantially pure isoglutaminyl cyclase. The co-crystals generally comprise substantially pure isoglutaminyl cyclase with a binding ligand, such as an isoglutaminyl cyclase inhibitor, bound to isoglutaminyl cyclase. Thus, according to a further aspect of the invention there is provided a co-crystal comprising the crystal as defined herein bound to a binding ligand, such as an isoglutaminyl cyclase inhibitor.

In one embodiment of the first aspect of the invention, the human isoglutaminyl cyclase consists of amino acid residues I73 to L382 of the native human isoQC sequence of SEQ ID NO:1. In an alternative embodiment, the human isoglutaminyl cyclase consists of amino acid residues A74 to A383 of SEQ ID NO: 2. In an alternative embodiment, the human isoglutaminyl cyclase consists of amino acid residues G74 to L382 (SEQ ID NO: 3). In an alternative embodiment, the human isoglutaminyl cyclase consists of amino acid residues G74 to L382 (SEQ ID NO: 4). In an alternative embodiment, the human isoglutaminyl cyclase consists of amino acid A75 to L382 (SEQ ID NO: 5). In an alternative embodiment, the human isoglutaminyl cyclase consists of amino acid residues A73 to L382 (SEQ ID NO: 6). In an alternative embodiment, the human isoglutaminyl cyclase consists of amino acid residues A75 to L382 (SEQ ID NO: 7). In an alternative embodiment, the human isoglutaminyl cyclase consists of amino acid residues A75 to L382 (SEQ ID NO: 8). In an alternative embodiment, the human isoglutaminyl cyclase consists of amino acid residues L76 to L382 (SEQ ID NO: 9). In an alternative embodiment, the human isoglutaminyl cyclase consists of amino acid residues A75 to L382 (SEQ ID NO: 10). In an alternative embodiment, the human isoglutaminyl cyclase consists of amino acid residues L76 to L382 (SEQ ID NO: 11). In an alternative embodiment, the human isoglutaminyl cyclase consists of amino acid residues P77 to L382 (SEQ ID NO: 12).

It will be appreciated that the crystals comprising isoglutaminyl cyclase are not limited to those obtainable from naturally occurring or native isoglutaminyl cyclase. The crystals include mutants that have one or more amino acid insertions, deletions, or substitutions in native isoglutaminyl cyclase. Therefore, mutants of native isoglutaminyl cyclase are obtained by replacing at least one (such as up to 10, e.g. up to 25) amino acid residue in a native isoglutaminyl cyclase with a different amino acid residue, or by adding or deleting amino acid residues within the native protein or at the N- or C-terminus of the native protein, and have substantially the same three-dimensional structure as native isoglutaminyl cyclase from which the mutant is derived.

By having substantially the same three-dimensional structure is meant as having a set of atomic structure coordinates from an apo- or co-crystal that have a root mean square deviation of less than or equal to about 2 Å when superimposed with the atomic structure coordinates of native isoglutaminyl cyclase from which the mutant is derived when at least about 50% to about 100% of the alpha carbon atoms of native isoglutaminyl cyclase are included in the superposition.

In some instances, it may be particularly advantageous or convenient to substitute, delete, and/or add amino acid residues to native isoglutaminyl cyclase in order to provide convenient cloning sites in the cDNA encoding the protein, to aid in protein purification, and the like. Such substitutions, deletions, and/or additions, which do not substantially alter the three dimensional structure of native isoglutaminyl cyclase will be apparent to those skilled in the art.

It should be noted that the mutant polypeptides contemplated herein need not exhibit isoglutaminyl cyclase activity. Indeed, amino acid substitutions, additions, or deletions that interfere with the activity of isoglutaminyl cyclase but which do not significantly alter the three-dimensional structure of isoglutaminyl cyclase are also included. Such polypeptide crystals, or the atomic structure coordinates obtained therefrom, can be used to identify compounds that bind to native isoglutaminyl cyclase and which may affect the activity of the native isoglutaminyl cyclase.

The derivative crystals of the invention generally comprise isoglutaminyl cyclase crystals in non-covalent/covalent association with one or more metal atoms. The polypeptide may correspond to native or mutated isoglutaminyl cyclase. One such example of a suitable metal atom is zinc.

The co-crystals of isoglutaminyl cyclase generally comprise crystals comprising isoglutaminyl cyclase in association with one or more compounds bound to isoglutaminyl cyclase. The association may be covalent or non-covalent. In one embodiment, the compounds bound to isoglutaminyl cyclase comprise isoglutaminyl cyclase inhibitors. Examples of such isoglutaminyl cyclase inhibitors include those described in WO 2008/034891, WO 2008/087197 and WO 2010/026209.

The native and mutated isoglutaminyl cyclase described herein may be isolated from natural sources or produced by methods well known to those skilled in the art of molecular biology. Detailed experimental for the preparation of human isoglutaminyl cyclase is described in Example 1 herein.

The apo, derivative and co-crystals of isoglutaminyl cyclase can be obtained by techniques well-known in the art of protein crystallography, including batch, liquid bridge, dialysis, vapor diffusion, such as hanging drop vapor diffusion, and the like (See for example, McPherson, 1982, Preparation and Analysis of Protein Crystals, John Wiley, NY; McPherson, 1990, Eur. J. Biochem. 189:1-23; Webber, 1991, Adv. Protein Chem. 41:1-36; Crystallization of Nucleic Acids and Proteins, Edited by Ducruix and Giege, Oxford University Press; Protein Crystallization Techniques, Strategies, and Tips, Edited by Bergfors, International University Line, 1999).

In one embodiment, isoglutaminyl cyclase crystals, apo or co-crystals are grown by vapor diffusion, such as hanging drop vapor diffusion.

According to a second aspect of the invention there is provided a method of preparing the crystal of human isoglutaminyl cyclase as described herein, which comprises the steps of:
(a) providing a solution of human isoglutaminyl cyclase, optionally in the presence of a known isoglutaminyl cyclase inhibitor, in a suitable buffer such as 25mM Bis-Tris pH6.8 / 100mM NaCl buffer;
(b) mixing the solution with a crystallization solution comprising 0.1 M sodium citrate, 0.1 M ammonium sulfate and 13% (w/v) 35000 PEG; and
(c) incubating the mixture under conditions to promote hanging drop vapor diffusion for a time sufficient to produce the crystal of human isoglutaminyl cyclase.

According to a further aspect of the invention, there is provided a method of preparing the co-crystal of human isoglutaminyl cyclase bound to a binding ligand, such as a isoglutaminyl cyclase inhibitor, which comprises the steps of:
(a) providing a solution of human isoglutaminyl cyclase in the presence of a binding ligand, such as a isoglutaminyl cyclase inhibitor, in a suitable buffer such as 25mM Bis-Tris pH6.8 / 100mM NaCl buffer;
(b) mixing the solution with a crystallization solution comprising 0.1 M sodium citrate, 0.1 M ammonium sulfate and 13% (w/v) 35000 PEG; and
(c) incubating the mixture under conditions to promote hanging drop vapor diffusion for a time sufficient to produce the co-crystal of human isoglutaminyl cyclase bound to a binding ligand, such as a isoglutaminyl cyclase inhibitor.

According to a further aspect there is provided a crystal or co-crystal comprising human isoglutaminyl cyclase obtainable by the crystallisation method as defined herein.

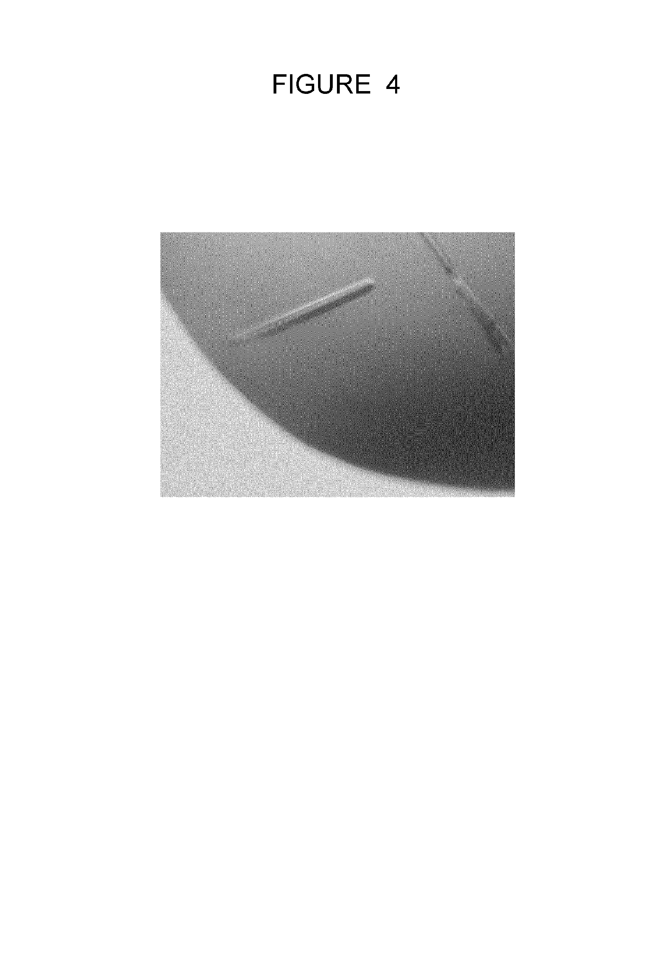

Crystallization of human isoglutaminyl cyclase to produce co-crystals can be carried out as described below and in Example 1. As described, purified human isoglutaminyl cyclase is concentrated to 10 mg/mL in the presence of a suitable buffer, such as 25mM Bis-Tris pH6.8 / 100mM NaCl buffer and 1mM of Inhibitor A. Macroscopic rod crystal forms are grown by hanging drop vapor diffusion at 21 °C by mixing an equal volume of protein solution with a crystallization solution comprising 0.1 M sodium citrate, 0.1 M ammonium sulfate and 13% (w/v) 35000 PEG. Crystals typically appeared 10-15 days after the experiment was initiated and examples of such crystals obtained following this protocol are shown in Figure 4. It will be appreciated that to produce apo-crystals, the ligand is omitted in the above protocol.

In one embodiment, human isoglutaminyl cyclase is deglycosylated prior to crystallisation. Glycosylation is known to result in a significant loss in solubility. As described herein, the best results for deglycosylation were obtained using endoglycosidase H_{f}.

In one embodiment, a solubility enhancing moiety is added prior to crystallisation of human isoglutaminyl cyclase. As described herein, the best results for enhanced solubility were obtained using an acyl-N-glucamid based non-ionic detergent MEGA-8.

The human isoglutaminyl cyclase crystals may be frozen prior to data collection. The crystals can be cryo-protected with for example, either (a) 20-30% saturated glucose present in the crystallization setup, (b) ethanol added to 15-20%, (c) ethylene glycol added to 10-20% and PEG10,000 brought up to 25%, or (d) glycerol added to 15%. In one embodiment of the third aspect of the invention, the crystals are cryo-protected with the addition of glycerol added to 15% (v/v). The crystals can be either briefly immersed in the cryo-protectant or soaked in the cryo-protectant for periods as long as a day. Freezing can be accomplished by immersing the crystal in a bath of liquid nitrogen or by placing the crystal in a stream of nitrogen gas at -180 °C.

As described in Example 1, the crystal structure of human isoglutaminyl cyclase in complex with Inhibitor A was obtained. A summary of the crystal's attributes for human isoglutaminyl cyclase bound to Inhibitor A are listed in Table 2 and the three dimensional structure coordinates for space groups P1211 for human isoglutaminyl cyclase bound to Inhibitor A are shown in Figure 1.

References herein to "coordinates" include references to Cartesian coordinates derived from the mathematical equations related to the patterns obtained on diffraction of a monochromatic beam of X-ray by the atoms of a protein or protein complex in crystal form. The diffraction data are used to calculate an electron density map of the repeating units of the crystal. The electron density maps are then used to establish the positions of the individual atoms of the molecule or molecular complex.

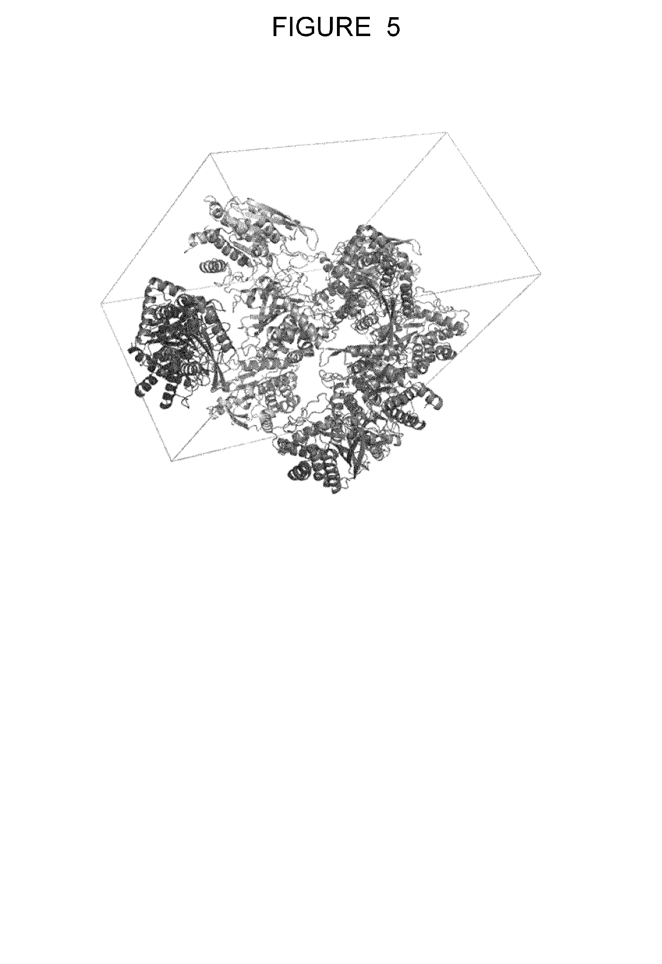

Ribbon and overlay diagrams of human isoglutaminyl cyclase bound to Inhibitor A based upon the coordinates for space groups P1211 are shown in Figure 5. In particular, it was found that the protein comprised a globular α/β hydrolase fold. A central β-sheet was formed with six β-strands all in parallel fashion but not the second. This β-sheet was observed to be surrounded by α-helices in a sandwich manner with two helices in one side and six more α-helices in the opposite face. The protein's structure was completed with a rather large amount of random coiled loops which are belived to build the active site of the enzyme.

References herein to "active site" include references to a specific region (or atom) in a molecular entity that is capable of entering into a stabilising interaction with another molecular entity. In certain embodiments, the term also refers to the reactive parts of a macromolecule that directly participate in its specific combination with another molecule. In an alternative embodiment, a binding site may be comprised or defined by the three dimensional arrangement of one or more amino acid residues within a folded polypeptide. References to "binding pocket" shall be interpreted in an analogous manner to "active site" and it will be appreciated that these terms may be used interchangeably.

This active site of human isoglutaminyl cyclase was found to accommodate a zinc ion which is coordinated by three protein residues, E226, D186 and H351. Thus, in one embodiment of the first aspect of the invention, the crystal comprises a binding pocket provided by residues E226, D186 and H351 of SEQ ID NO: 1 according to the coordinates of Figure 1.

Moreover, the protein shows the presence of a disulfide bridge between residues C¹⁶⁷ and C¹⁹¹. Thus, in one embodiment of the first aspect of the invention, the crystal comprises a disulfide bridge between residues C167 and C191 of SEQ ID NO: 1. The presence of such a disulfide bridge has not previously been reported during crystallisation studies with QC (Wang *et al,* Huang *et al*)*.*

Finally two segments of the polylpeptide chain are not visible in the electron density. The gaps include residues between K¹⁸² and D¹⁹⁰ and between F¹⁴⁶ and N¹⁵⁰.

The present invention is also directed to machine-readable data storage media having data storage material encoded with machine-readable data that comprises structure coordinates for isoglutaminyl cyclase. The present invention is also directed to a machine readable data storage media having data storage material encoded with machine readable data, which, when read by an appropriate machine, can display a three dimensional representation of a structure of isoglutaminyl cyclase.

All or a portion of isoglutaminyl cyclase coordinate data shown in Figure 1, when used in conjunction with a computer programmed with software to translate those coordinates into the three-dimensional structure of isoglutaminyl cyclase may be used for a variety of purposes, especially for purposes relating to drug discovery. Software for generating three-dimensional graphical representations are known and commercially available. The ready use of the coordinate data requires that it be stored in a computer-readable format. Thus, in accordance with the present invention, data capable of being displayed as the three-dimensional structure of isoglutaminyl cyclase and/or portions thereof and/or their structurally similar variants may be stored in a machine-readable storage medium, which is capable of displaying a graphical three-dimensional representation of the structure.

Another embodiment of this invention provides a machine-readable data storage medium, comprising a data storage material encoded with machine readable data which, when used by a machine programmed with instructions for using said data, displays a graphical three-dimensional representation comprising isoglutaminyl cyclase or variant thereof.

Optionally, a computer system is provided in combination with the machine-readable data storage medium provided herein. In one embodiment, the computer system comprises a working memory for storing instructions for processing the machine-readable data; a processing unit coupled to the working memory and to the machine-readable data storage medium, for processing the machine-readable data into the three-dimensional representation; and an output hardware coupled to the processing unit, for receiving the three-dimensional representation.

The three-dimensional crystal structure of the present invention may be used to identify isoglutaminyl cyclase binding sites, be used as a molecular replacement model to solve the structure of unknown crystallized proteins, to design mutants having desirable binding properties, and ultimately, to design, characterize, identify entities capable of binding and inhibiting isoglutaminyl cyclase and other structurally similar proteins as well as other uses that would be recognized by one of ordinary skill in the art. Such entities may be chemical entities or proteins. The term "chemical entity", as used herein, refers to chemical compounds, complexes of at least two chemical compounds, and fragments of such compounds.

The isoglutaminyl cyclase structural coordinates provided herein are useful for screening and identifying drugs that inhibit isoglutaminyl cyclase and other structurally similar proteins. For example, the structure encoded by the data may be computationally evaluated for its ability to **associate with putative substrates or ligands**. **Such compounds that associate** with isoglutaminyl cyclase may inhibit isoglutaminyl cyclase activity, and are potential drug candidates. Additionally or alternatively, the structure encoded by the data may be displayed in a graphical three-dimensional representation on a computer screen. This allows visual inspection of the structure, as well as visual inspection of the structure's association with the compounds.

Thus, according to a fourth aspect of the invention there is provided a method of identifying an inhibitor of human isoglutaminyl cyclase which comprises the following steps:
(a) generating a 3-dimensional model of human isoglutaminyl cyclase using the structural coordinates described in Figure 1;
(b) analysing the binding pocket provided by residues E226, D186 and H351 of SEQ ID NO: 1 according to the coordinates of Figure 1;
(c) performing computer modeling analysis to identify an inhibitor compound which may associate with the binding pocket of human isoglutaminyl cyclase.

A method is also provided for evaluating the potential of an entity to associate with isoglutaminyl cyclase or variant thereof by using all or a portion of the structure coordinates provided in Figure 1 or functional equivalents thereof. A method is also provided for evaluating the potential of an entity to associate with isoglutaminyl cyclase or variant thereof by using structure coordinates similar to all or a portion of the structure coordinates provided in Figure 1 or functional equivalents thereof.

Further described is the step of synthesizing the inhibitor compound and contacting the compound with the binding pocket of isoglutaminyl cyclase to determine the ability of the compound to inhibit isoglutaminyl cyclase.

In one embodiment of the fourth aspect of the invention, the step of performing computer modeling analysis to identify said inhibitor compound comprises identifying said compound from a library of compounds.

In one embodiment of the fourth aspect of the invention, the step of performing computer modeling analysis to identify said inhibitor compound comprises identifying said compound in a database.

In one embodiment of the fourth aspect of the invention, the step of performing computer modeling analysis to identify said inhibitor compound comprises designing the compound from a known isoglutaminyl cyclase inhibitor.

With the structures provided herein, the present invention permits the use of molecular design techniques to identify, select or design potential inhibitors of isoglutaminyl cyclase based on the structure of isoglutaminyl cyclase. Such a predictive model is valuable in light of the high costs associated with the preparation and testing of the many diverse compounds that may possibly bind to isoglutaminyl cyclase.

According to the invention, a potential isoglutaminyl cyclase inhibitor may be evaluated for its ability to bind isoglutaminyl cyclase prior to its actual synthesis and testing. If a proposed entity is predicted to have insufficient interaction or association with the binding pocket, preparation and testing of the entity can be obviated. However, if the computer modeling indicates a strong interaction, the entity may then be obtained and tested for its ability to bind.

A potential inhibitor of isoglutaminyl cyclase may be computationally evaluated using a series of steps in which chemical entities or fragments are screened and selected for their ability to associate with isoglutaminyl cyclase.

One skilled in the art may use one of several methods to screen entities (whether chemical or protein) for their ability to associate with isoglutaminyl cyclase. This process may begin by visual inspection of, for example, isoglutaminyl cyclase on a computer screen based on the isoglutaminyl cyclase structure coordinates in Figure 1 or other coordinates which define a similar shape generated from the machine-readable storage medium. Selected fragments or chemical entities may then be positioned in a variety of orientations, or docked, within that binding pocket as defined above. Docking may be accomplished using software such as Quanta and Sybyl, followed by energy minimization and molecular dynamics with standard molecular mechanics force fields, such as CHARMM and AMBER.

Specialized computer programs may also assist in the process of selecting entities. These include: GRID (Goodford, "A Computational Procedure for Determining Energetically Favorable Binding Sites on Biologically Important Macromolecules", J. Med. Chem., 28, pp. 849-857 (1985)). GRID is available from Oxford University, Oxford, UK; MCSS (Miranker et al., "Functionality. Maps of Binding Sites: A Multiple Copy Simultaneous Search Method." Proteins: Structure, Function and Genetics, 11, pp. 29-34 (1991)). MCSS is available from Molecular Simulations, San Diego, Calif.; AUTODOCK (Goodsell et al., "Automated Docking of Substrates to Proteins by Simulated Annealing", Proteins: Structure, Function, and Genetics, 8, pp. 195-202 (1990)). AUTODOCK is available from Scripps Research Institute, La Jolla, Calif.; & DOCK (Kuntz et al., "A Geometric Approach to Macromolecule-Ligand Interactions", J. Mol. Biol., 161, pp. 269-288 (1982)). DOCK is available from University of California, San Francisco, Calif.

Once suitable entities have been selected, they can be designed or assembled. Assembly may be preceded by visual inspection of the relationship of the fragments to each other on the three-dimensional image displayed on a computer screen in relation to the structure coordinates of isoglutaminyl cyclase. This may then be followed by manual model building using software such as MOE, QUANTA or Sybyl (Tripos Associates, St. Louis, Mo.).

Useful programs to aid one of skill in the art in connecting the individual chemical entities or fragments include: CAVEAT (Bartlett et al., "CAVEAT: A Program to Facilitate the Structure-Derived Design of Biologically Active Molecules", in "Molecular Recognition in Chemical and Biological Problems", Special Pub., Royal Chem. Soc., 78, pp. 182-196 (1989); Lauri and Bartlett, "CAVEAT: a Program to Facilitate the Design of Organic Molecules", J. Comput. Aided Mol. Des., 8, pp. 51-66 (1994)). CAVEAT is available from the University of California, Berkeley, Calif.; 3D Database systems such as ISIS (MDL Information Systems, San Leandro, Calif.). This area is reviewed in Martin, "3D. Database Searching in Drug Design", J. Med. Chem., 35, pp. 2.145-2154 (1992); HOOK (Eisen et al., "HOOK: A Program for Finding Novel Molecular Architectures that Satisfy the Chemical and Steric Requirements of a Macromolecule Binding Site", Proteins: Struct., Funct., Genet., 19, pp. 199-221 (1994). HOOK is available from Molecular Simulations, San Diego, Calif.

Instead of proceeding to build an inhibitor of isoglutaminyl cyclase in a step-wise fashion one fragment or entity at a time as described above, inhibitory or other isoglutaminyl cyclase binding compounds may be designed as a whole or "*de novo*" using either an empty binding site or optionally including some portion(s) of a known inhibitor(s). There are many *de novo* ligand design methods including: LUDI (Bohm, "The Computer Program LUDI: A New Method for the De Novo Design of Enzyme Inhibitors", J. Comp. Aid. Molec. Design, 6, pp. 61-78 (1992)). LUDI is available from Molecular Simulations Incorporated, San Diego, Calif.; LEGEND (Nishibata et al., Tetrahedron, 47, p. 8985 (1991)). LEGEND is available from Molecular Simulations Incorporated, San Diego, Calif.; LEAPFROG (available from Tripos Associates, St. Louis, Mo.); & SPROUT (Gillet et al., "SPROUT: A Program for Structure Generation)", J. Comput. Aided Mol. Design, 7, pp. 127-153 (1993)). SPROUT is available from the University of Leeds, UK.

Other molecular modeling techniques may also be employed in accordance with this invention (See, for example, Cohen et al., "Molecular Modeling Software and Methods for Medicinal Chemistry, J. Med. Chem., 33, pp. 883-894 (1990); See also, Navia and Murcko, "The Use of Structural Information in Drug Design", Current Opinions in Structural Biology, 2, pp. 202-210 (1992); Balbes et al., "A Perspective of Modern Methods in Computer-Aided Drug Design", in Reviews in Computational Chemistry, Vol. 5, Lipkowitz and Boyd, Eds., VCH, New York, pp. 337-380 (1994); See also, Guida, "Software For Structure-Based Drug Design", Curr. Opin. Struct. Biology, 4, pp. 777-781 (1994)).

Once an entity has been designed or selected, for example, by the above methods, the efficiency with which that entity may bind to isoglutaminyl cyclase may be tested and optimized by computational evaluation. For example, an effective isoglutaminyl cyclase inhibitor preferably demonstrates a relatively small difference in energy between its bound and free states (i.e., a small deformation energy of binding). Thus, the most efficient isoglutaminyl cyclase inhibitors should preferably be designed with deformation energy of binding of not greater than about 10 kcal/mole, more preferably, not greater than 7 kcal/mole. Isoglutaminyl cyclase inhibitors may interact with the protein in more than one of multiple conformations that are similar in overall binding energy. In those cases, the deformation energy of binding is taken to be the difference between the energy of the free entity and the average energy of the conformations observed when the inhibitor binds to the protein.

**An entity designed or selected as binding to isoglutaminyl cyclase may be further** computationally optimized so that in its bound state it would preferably lack repulsive electrostatic interaction with the target enzyme and with the surrounding water molecules. Such non-complementary electrostatic interactions include repulsive charge-charge, dipole-dipole and charge-dipole interactions.

Specific computer software is available in the art to evaluate compound deformation energy and electrostatic interactions. Examples of programs designed for such uses include: Gaussian 94, revision C (Frisch, Gaussian, Inc., Pittsburgh, Pa. 1995); AMBER, version 4.1 (Kollman, University of California at San Francisco, 1995); QUANTA/CHARMM (Molecular Simulations, Inc., San Diego, Calif. 1995); Insight II/Discover (Molecular Simulations, Inc., San Diego, Calif. 1995); DelPhi (Molecular Simulations, Inc., San Diego, Calif. 1995); and AMSOL (Quantum Chemistry Program Exchange, Indiana University). These programs may be implemented, for instance, using a Silicon Graphics workstation such as an Indigo.sup.2 with "IMPACT" graphics. Other hardware systems and software packages will be known to those skilled in the art.

Another approach provided by the invention, is the computational screening of small molecule databases for chemical entities or compounds that can bind in whole, or in part, to isoglutaminyl cyclase. In this screening, the quality of fit of such entities to the binding site may be judged either by shape complementarities or by estimated interaction energy (Meng et al., J. Comp. Chem., 13, 505-524 (1992)).

According to a further aspect of the invention there is provided a compound that associates with isoglutaminyl cyclase produced or identified by various methods as described hereinbefore.

The structure coordinates set forth in Figure 1 can also be used to aid in obtaining structural information about another crystallized molecule or molecular complex. This may be achieved by any of a number of well-known techniques, including molecular replacement.

For example, a method is also provided for utilizing molecular replacement to obtain structural information about a protein whose structure is unknown comprising the steps of: generating an X-ray diffraction pattern of a crystal of the protein whose structure is unknown; generating a three-dimensional electron density map of the protein whose structure is unknown from the X-ray diffraction pattern by using at least a portion of the structure coordinates set forth in Figure 1 as a molecular replacement model.

By using molecular replacement, the structure coordinates of isoglutaminyl cyclase provided by the invention (and set forth in Figure 1) can be used to determine the structure of another crystallized molecule or molecular complex more quickly and efficiently than attempting an *ab initio* structure determination. One particular use includes use with other structurally similar proteins. Molecular replacement provides an accurate estimation of the phases for an unknown structure. Phases are a factor in equations used to solve crystal structures that cannot be determined directly. Obtaining accurate values for the phases, by methods other than molecular replacement, is a time-consuming process that involves iterative cycles of approximations and refinements and greatly hinders the solution of crystal structures. However, when the crystal structure of a protein containing at least a homologous portion has been solved, the phases from the known structure provide a satisfactory estimate of the phases for the unknown structure.

Thus, this method involves generating a preliminary model of a molecule or molecular complex whose structure coordinates are unknown, by orienting and positioning the relevant portion of isoglutaminyl cyclase according to Figure 1 within the unit cell of the crystal of the unknown molecule or molecular complex so as best to account for the observed X-ray diffraction pattern of the crystal of the molecule or molecular complex whose structure is unknown. Phases can then be calculated from this model and combined with the observed X-ray diffraction pattern amplitudes to generate an electron density map of the structure whose coordinates are unknown. This, in turn, can be subjected to any well-known model building and structure refinement techniques to provide a final, accurate structure of the unknown crystallized molecule or molecular complex (Lattman, "Use of the Rotation and Translation Functions", in Meth. Enzymol., 115, pp. 55-77 (1985); Rossmann, ed., "The Molecular Replacement Method", Int. Sci. Rev. Ser., No. 13, Gordon & Breach, New York (1972)).

The structure of any portion of any crystallized molecule or molecular complex that is sufficiently homologous to any portion of isoglutaminyl cyclase can be resolved by this method.

In one embodiment, the method of molecular replacement is utilized to obtain structural information about the present invention and any other isoglutaminyl cyclase-like molecule.

The structure coordinates of isoglutaminyl cyclase as provided by the invention are useful in solving the structure of isoglutaminyl cyclase variants that have amino acid substitutions, additions and/or deletions (referred to collectively as " isoglutaminyl cyclase mutants", as compared to naturally occurring isoglutaminyl cyclase). These isoglutaminyl cyclase mutants may optionally be crystallized in co-complex with a ligand, such as an inhibitor or substrate analogue. The crystal structures of a series of such complexes may then be solved by molecular replacement and compared with that of isoglutaminyl cyclase. Potential sites for modification within the various binding sites of the enzyme may thus be identified. This information provides an additional tool for determining the most efficient binding interactions such as, for example, increased hydrophobic interactions, between isoglutaminyl cyclase and a ligand. It is noted that the ligand may be the protein's natural ligand or may be a potential agonist or antagonist of a protein.

All of the complexes referred to above may be studied using well-known X-ray diffraction techniques and may be refined versus 1.5-3.5 A resolution X-ray data to an R value of about 0.22 or less using computer software, such as X-PLOR (Brunger et al., X-PLOR, Version 3.1, A system for X-ray crystallography and NMR, Yale University, (1992)), CNS (Brunger et al., Crystallography & NMR System (CNS), A new software suite for macromolecular structure determination, Acta Cryst. D54: 905-921(1998)), TNT (Tronrud et al., An efficient general-Purpose least-squares refinement program for macromolecular structures, Acta Cryst. A43, 489-501 (1987)), Buster (Bricogne, The Bayesian Statistical Viewpoint on Structure Determination: Basic Concepts and Examples", in Methods in Enzymology, 276A, 361-423. Carter & Sweet, eds. (1997)) and Refmac (Murshudov et al., Refinement of macromolecular structures by the maximum-likelihood method, Acta Cryst D53 :240-255 (1997)) (See, e.g., Blundell & Johnson, supra; Meth. Enzymol., Vol. 114 & 115, Wyckoff et al., eds., Academic Press (1985)). This information may thus be used to optimize known isoglutaminyl cyclase inhibitors, and more importantly, to design new isoglutaminyl cyclase inhibitors.

The structure coordinates described herein may also be used to derive the dihedral angles, phi and psi, that define the conformation of the amino acids in the protein backbone. As will be understood by those skilled in the art, the phi angle refers to the rotation around the bond between the alpha-carbon and the nitrogen, and the psi angle refers to the rotation around the bond between the carbonyl carbon and the alpha-carbon. The subscript "n" identifies the amino acid whose conformation is being described (for a general reference, See Blundell and Johnson, Protein Crystallography, Academic Press, London, 1976).

The invention is further illustrated by the following non-limiting examples:

### Preparation of 1-(3-(1H-Imidazol-1-yl)propyl)-3-(3,4-dimethoxyphenyl)thiourea (Inhibitor A)

4.0 mmol of 3,4-dimethoxyphenyl isothiocyanate and 4.0 mmol of 3-(1*H*-imidazol-1-yl)alkyl-1-amine were dissolved in 10 mL of absolute ethanol. After stirring for 2 h under reflux, the solvent was evaporated and the resulting solid was recrystallized from ethanol.

Yield: 0.66 g (51.3 %); mp: 160.0 - 161.0°C

¹H NMR δ 1.8 - 2.0 (m, 2H), 3.4 - 3.5 (m, 2H), 3.75 (s, 6H), 3.9 - 4.0 (m, 2H), 6.7 - 6.8 (m, 1 H), 6.9 (br m, 2H), 6.95 (s, 1H), 7.15 (s, 1H), 7.55 (br s, 1H), 7.6 (s, 1 H), 9.3 (s, 1 H); MS m/z 321.2 (M+H), 253.3 (M-C₃H₃N₂•)

### Example 1

### Expression, Purification and Crystallisation of Human Isoglutaminyl Cyclase

### (A) Expression and Purification of Human Isoglutaminyl Cyclase in P. pastoris

### Host strains and media

*Escherichia coli* strain DH5α was used for propagation of plasmids and *P. pastoris* strain X-33 was used for the expression of human isoQC in yeast. *E. coli and P. pastoris* strains were grown, transformed and analyzed according to the manufacturer's instructions (Qiagen (DH5α), Invitrogen (X-33)). The media required for *E. coli,* i.e. Luria-Bertani (LB) medium, was prepared according to the manufacturer's recommendations. The media required for *Pichia pastoris,* i.e. BMMY, BMGY, YPD, YPDS and the concentration of the antibiotics, i.e. Zeocin, were prepared as described in the Pichia manual (Invitrogen, catalog. No. K1740-01). The manual also includes all relevant descriptions for the handling of yeast.

### Molecular cloning of plasmid vectors encoding the human isoQC

All cloning procedures were done applying standard molecular biology techniques. For expression in *Pichia pastoris* X-33, the pPiCZαA (Invitrogen) was used. The cDNA of the mature human isoQC starting with codon 42 (counting from methionine II) was fused in frame with the plasmid encoded α-factor, directing the protein into the secretory pathway. After amplification utilizing the primers 5'-ATA TGA ATT CCA TCA CCA TCA CCA TCA CGA GGA GCT GCC GCT GGG CCG G-3' (SEQ ID NO: 13) (sense) and 5'-ATA TAT GCG GCC GCC TAG AGC CCC AGG TAT TCA GC-3' (SEQ ID NO: 14) (antisense), the fragment was inserted into the expression vector employing the restriction sites of *Not*I and *EcoR* I. Mutations were introduced in codons 55 using the primer 5'-CTG CGG GTC CCA TTG AAC GGA AGC CTC CCC GAA-3' (SEQ ID NO: 15) (sense) and 5'- TTC GGG GAG GCT TCC GTT CAA TGG GAC CCG CAG-3' (SEQ ID NO: 16) (antisense) as well as in codon 351 (Cys) using the primer 5'- ACG GTA CAC AAC TTG GCC CGC ATT CTC GCT GTG-3' (SEQ ID NO: 17) (sense) and 5'- CAC AGC GAG AAT GCG GGC CAA GTT GTG TAC CGT-3' (SEQ ID NO: 18) (antisense). The mutagenesis was performed according to standard PCR techniques followed by digestion of the parent DNA using *Dpn*I (quik-change II site-directed mutagenesis kit, Stratagene, Catalog No. 200524).

### Transformation of P. pastoris and Mini-Scale Expression

1-2 µg of plasmid DNA were applied for transformation of competent *P. pastoris* cells by electroporation according to the manufacturer's instructions (BioRad). Selection was done on plates containing 100 µg/ml Zeocin. In order to test the recombinant yeast clones upon isoQC expression, recombinants were grown for 24 h in 10 ml conical tubes containing 2 ml BMGY. Afterwards, the yeast was centrifuged and resuspended in 2 ml BMMY containing 0.5 % methanol. This concentration was maintained by addition of methanol every 24 h for about 72 h. Subsequently, QC activity in the supernatant was determined. Clones that displayed the highest activity were chosen for further experiments and large scale expression.

### Expression and purification of hisoQC in P. pastoris

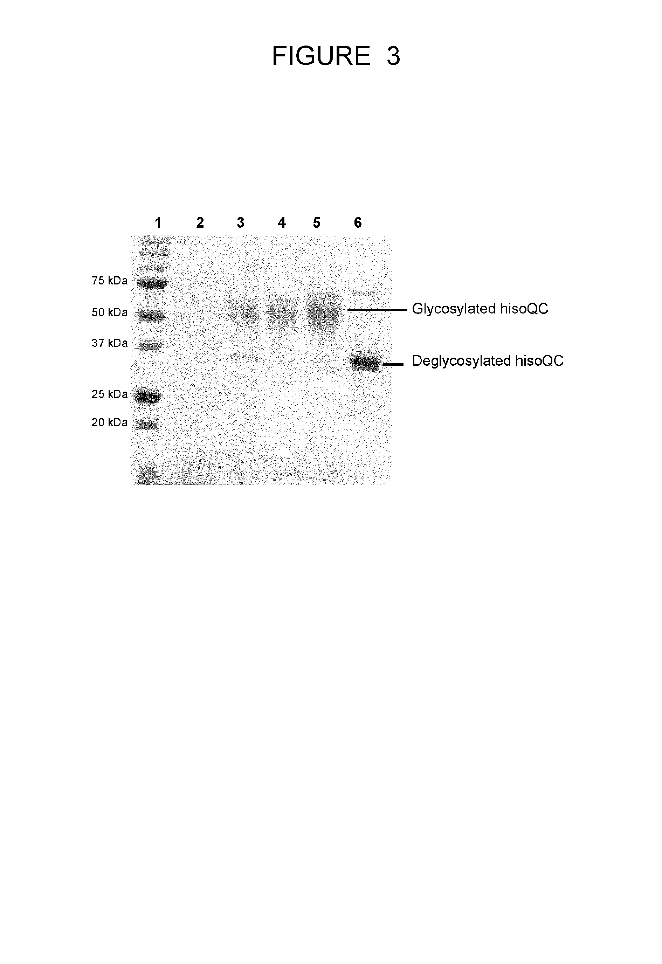

For large scale expression of isoQC in *Pichia pastoris,* the conditions were kept as described in the mini-scale expression, however, the total volume was 8 L. The expression was performed in shake flasks. After expression, cells were separated from the medium by centrigugation (1500xg, 20 min), and the pellet discarded. The pH-value of the supernatant was adjusted to neutrality, centrifuged again and applied for the first purification step. The *hisoQC* protein was purified utilizing a 3-step protocol (Table 1). The purification is illustrated by SDS-PAGE analysis in Figure 3. The yield of purification was 60 %. The apparent protein was inhomogenous glycosylated as evidenced by the migration of *hisoQC* between 50 and 75 kDa (Figure 3). After purification the protein was concentrated to 12 mg/ml using U-Tube^{™} Concentrators (Novagen) with a molecular weight cut off of 10 kDa and stored at -80 °C.

**Table 1**

| **Purification of hisoQC (YSShisoQCE₄₂I55NC351A N-His) following Expression in *P. pastoris.*** | | | |
|---|---|---|---|
| **Purification Step** | **1** | **2** | **3** |
| Method | **Ni²⁺-IMAC** | **HIC** | **GF (Desalting)** |
| Column type (Amersham Biosciences AB, Sweden) | Chelating Sepharose Fast Flow | Butyl Sepharose 4Fast Flow | Sephadex G-25 Fine |
| Column size | d=2,5cm | d=1,6cm | d=2,6cm |
| | l=42cm | l=15,5cm | l=10cm |
| | CV=206cm³ | CV=23cm³ | CV=53cm³ |
| Equilibration | | | |
| Buffer | 50mM NaH₂PO₄ | 30mM NaH₂PO₄ | 50mM Bis-Tris |
| | | 1M (NH₄)₂SO₄ | 100mM NaCl |
| pH | 7,0 | 7,0 | 6,8 |
| Volume | 10CV | 10CV | 10CV |
| Intermediate (Wash) | | | |
| Buffer | 50mM NaH₂PO₄ | 30mM NaH₂PO₄ | |
| | 0,5mM Histidin | 1M (NH₄)₂SO₄ | - |
| pH | 7,0 | 7,0 | |
| Volume | 10CV | 6CV | |
| Elution | | | |
| Buffer | 50mM NaH₂PO₄ | 30mM NaH₂PO₄ | 50mM Bis-Tris |
| | 100mM Histidin | | 100mM NaCl |
| pH | 7,0 | 7,0 | 6,8 |
| Volume | 1,5 CV | 5 CV | 1CV |

### (B) Crystallization of Human Isoglutaminyl Cyclase

### Crystal Growth

Crystals were grown using the hanging drop vapor diffusion technique at room temperature (21°C) in Easyxtal 24-well plates plates (Qiagen). The mother liquor buffer consisted of 0.1 M sodium citrate, 0.1 M ammonium sulfate and 13 % (w/v) 35000 PEG. Protein solution concentrated up to a 10 mg/ml was recombinant human isoglutaminyl cyclase (hisoQC) in the presence of 25mM Bis-Tris pH6.8 / 100mM NaCl Buffer. Before crystallization *hisoQC* was deglycosylated using endoglycosidase H_{f} (New England Biolabs). The removal of the sugar chains resulted in a tremendous decrease of the solubility of *hisoQC.* To keep the protein soluble *hisoQC* was treated with detergent MEGA-8 (Fluka) (final concentration 100 mM). Afterwards the endoglycosidase H_{f} was added (final activity 4.8*10³ units/ml). The reaction was performed over night at room temperature. After deglycosylation Inhibitor A was added (final concentration 1 mM).

The crystallization of *hisoQC* requires homogenous protein. Therefore it was necessary to remove the glycosylation. As mentioned above deglycosylation results in a tremendous loss of solubility. To avoid protein aggregation different agents and detergents were tested on their influence on protein solubility and stability. The acyl-N-glucamid based nonionic detergent MEGA-8 turned out to be a suitable additive. Besides the mediation of solubility of *hisoQC* it had no detectable influence on endoglycosidase H_{f} activity.

A mixture of 1µl mother liquor buffer and 1µl deglycosylated protein solution as described above was set. Crystals appeared usually ten to fifteen days after experiment was initiated and they displayed a macroscopic rod shape (Figure 4).

### Cryosolution

Before x-ray measurements, crystals were rapidly soaked into a new cryo-buffer solution where mother liquor buffer solution was saturated with 15% (vol/vol) glycerol. Immediately crystals were collected from the cryo-buffer solution using a pin nylon loop and they were mounted onto the goniometer and flash frozen at -180°C under a nitrogen stream.

### Data collection

Data collection from a single crystal was undertaken by means of Cu Kα radiation (λ = 1.5418 Å) by using a rotating-anode source (RA Micro 007; Rigaku/MSC, Tokyo, Japan) and CCD detector device (CCD Saturn 944+, Rigaku) with Varimax^{™} Optics (Rigaku) and an AFC-11 goniometer.

Finally, the crystal was demounted and stored in liquid nitrogen for an additional measurement in the beam line Bessx-MX BL14.1 of the synchrotron in Berlin (Bessy). Resolution cut-off was set to an l/sig(l) of 1.9 with a Rmerge of 13.7 achieving finally a 3.42 Angstrom of resolution.

### Data processing

The image reflection intensities were indexed and integrated using the program XDS. Further data processing was carried out using the programs included in the crystallographic suite CCP4. The integrated intensities were scaled and merged using the program Scala. The initial mtz file was analysed by calculating Mathews coefficient. Mathews coefficient showed the highest probability for an asymmetric unit (AU) containing either 11 or 12 molecules with a water content of about 50%.

Moreover, initial phases were subsequently obtained with a molecular replacement approach using Phaser. The crystal structure of human glutaminyl cyclase (hQC) (structure not yet published) was taken as a searching model. Previous to phasing the PDB file of the hQC file was modified with Chainsaw program to replace the original sequence of hQC for the one of hisoQC. This model gave a solution that showed rotational (RFZ) and translational (TFZ)I scores of 4.7 and 29.1 respectively and a LLG score of 4846 indicating the likeliness of a useful solution. The Phaser solution succeeded with an initial model containing 11 molecules in the AU (Figure 5) showing additional well defined positive electron density maps localized on the area of active site related residues

Thus, initial electron densities were obtained. Subsequent manual building of the missing fragments and maximum-likelihood refinement cycles were performed by using the programs COOT and REFMAC5 as well from the CCP4 suite. Initially, model building was eased using an averaged FoFc density map that was used to more accurately model the chain A. Furthermore an Non-crystallographic symmetry (NCS) refinement was performed using chain A as template and applying tight NCS restrains to main chain and side chain residues of chains B ,C, D, E, F, G, H, I, J and K in the residue range from 77 to 380. Thus the model was improved achieving R_{work} and R_{free} of 26.84 and 31.7 respectively

### Results

Table 2 summarizes the statistics of the data set obtained in Berlin synchrotron Bessy with a crystal of the human iso-glutaminyl cyclase and their corresponding statistics for the data processing and model building. Programs used were for data processing XDS and SCALA, for molecular replacement PHASER, for refinement REFMAC5 and for Model Building: Coot (all of them included in the CCP4 suite). Numbers between brackets belong to the outer shell resolution limit.

**Table 2**

| ***Summary of Statistics for Human Isoglutaminyl Cyclase Crystal*** | |
|---|---|
| **Data collection** | |
| Data set collected at | BESSY |
| Space group | P1 21 1 |
| Cell dimensions | |
| *a, b, c* (Å) | 126.51 109.68 159.53 |
| α, β, γ (°) | 90.0 104.9 90.0 |
| Resolution (Å) | 34.72 - 3.42 |
| Rmerge | 13.7 (46.3) |
| I/Sigmal | 6.7 (1.9) |
| Completeness (%) | 83.7 (86.2) |
| Redundancy | 1.8 (1.7) |
| | |

| **Refinement** | |
|---|---|
| Resolution (Å) | 3.42 |
| No. reflections (work/test) | 53920 / 2838 |
| *R*_{work} / *R*_{free} | 26.8 / 31.31 |
| No. atoms | |
| Protein | 26523 |
| Water | 203 |
| *B*-factors (Mean value) | 42.37 |
| R.m.s deviations | |
| Bond lengths (Å) | 0.011 |
| Bond angles (°) | 1.748 |

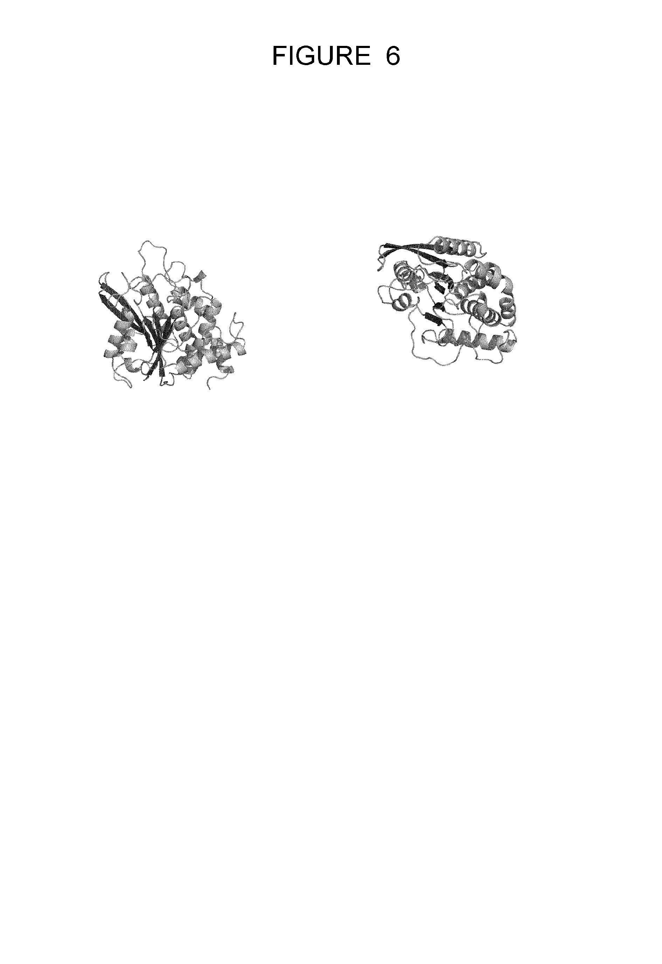

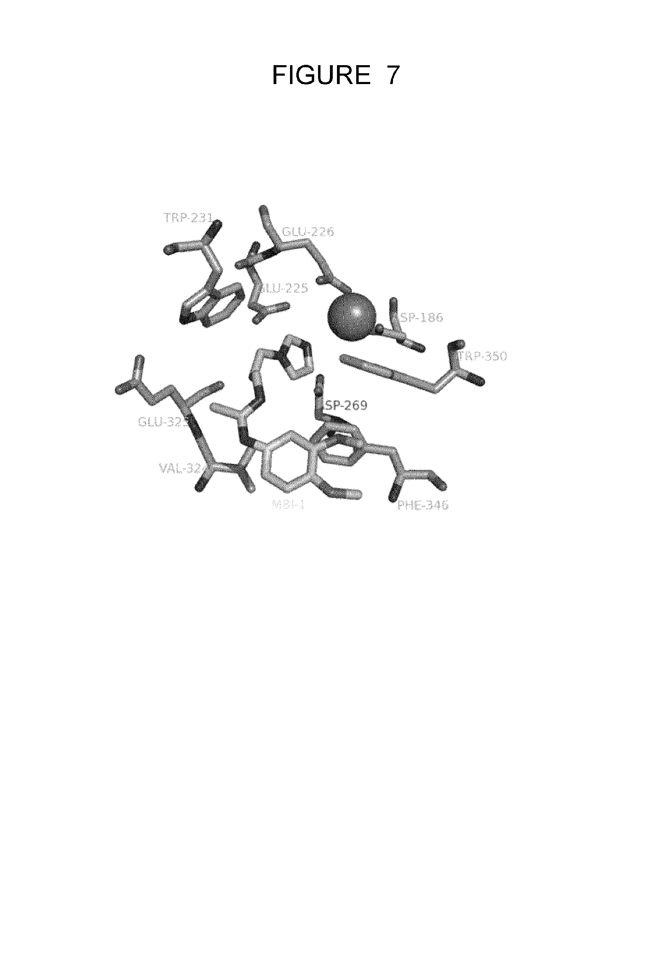

Overall three dimensional structure. For the sake of simplicity the chain A of the asymmetric unit will be described. The model built from the obtained experimental electron density includes 11 molecules of the hisoQC and expands the polypeptide chain from residue 75 to 383. The model includes a zinc ion that is achieving a complex with the Inhibitor A molecule (Figure 7). The protein shows a globular α/β hydrolase fold (Figure 6). A central β-sheet is formed with six β-strands all in parallel fashion but not the second. This β-sheet is surrounded by α-helices in a sandwich manner with two helices in one side and six more α-helices in the opposite face. The protein's structure is completed with a rather large amount of random coiled loops which are building the active site of the enzyme. **This active site is** accommodating a zinc ion which is coordinated by three protein residues, E²²⁶, D¹⁸⁶ and H³⁵¹ and a molecule of Inhibtor A (Figure 7). Moreover the protein shows the presence of a disulfide bridge between residues C¹⁶⁷ and C¹⁹¹. Finally two segments of the polylpeptide chain are not visible in the electron density. The gaps include residues between K¹⁸² and D¹⁹⁰ and between F¹⁴⁶ and N¹⁵⁰.

## Claims

1. A crystal comprising human isoglutaminyl cyclase having a characterised space group of P1211 and unit cell dimensions of +/- 5% of a = 126.51 Å, b = 109.68 Å, c = 159.53 Å, α = 90.0º, β = 104.9º and γ = 90.0º.

2. A crystal as defined in claim 1, which diffracts x-rays for determination of atomic co-ordinates of the crystal to a resolution of between 3.42 Å and 34.72 Å.

3. A crystal as defined in claim 1 or claim 2, wherein the human isoglutaminyl cyclase consists of amino acid residues I73 to L382 of SEQ ID NO:1.

4. A crystal as defined in claim 3, which comprises a binding pocket provided by residues E226, D186 and H351 of SEQ ID NO: 1 according to the coordinates of Figure 1.

5. A crystal as defined in claim 3 or claim 4, which comprises a disulfide bridge between residues C167 and C191 of SEQ ID NO: 1.

6. A co-crystal comprising the crystal as defined in any preceding claims, wherein the isoglutaminyl cyclase is bound to a binding ligand, such as an isoglutaminyl cyclase inhibitor.

7. A method of identifying an inhibitor of human isoglutaminyl cyclase which comprises the following steps:
(a) generating a 3-dimensional model of human isoglutaminyl cyclase using the structural coordinates described in Figure 1;
(b) analysing the binding pocket provided by residues E226, D186 and H351 of SEQ ID NO: 1 according to the coordinates of Figure 1;
(c) performing computer modeling analysis to identify an inhibitor compound which may associate with the binding pocket of human isoglutaminyl cyclase.

8. A method as defined in claim 7 wherein the step of performing computer modeling analysis to identify said inhibitor compound comprises identifying said compound from a library of compounds or identifying said compound in a database or designing the compound from a known human isoglutaminyl cyclase inhibitor.

9. A method of preparing the crystal of human isoglutaminyl cyclase as described in any of claims 1 to 5, which comprises the steps of:
(a) providing a solution of human isoglutaminyl cyclase, optionally in the presence of a known isoglutaminyl cyclase inhibitor, in a suitable buffer such as 25mM Bis-Tris pH6.8 / 100mM NaCl buffer;
(b) mixing the solution with a crystallization solution comprising 0.1 M sodium citrate, 0.1 M ammonium sulfate and 13% (w/v) 35000 PEG; and
(c) incubating the mixture under conditions to promote hanging drop vapor diffusion for a time sufficient to produce the crystal of human isoglutaminyl cyclase.

10. A method of preparing the co-crystal as defined in claim 6, which comprises the steps of:
(a) providing a solution of human isoglutaminyl cyclase in the presence of a binding ligand, such as an isoglutaminyl cyclase inhibitor, in a suitable buffer such as 25mM Bis-Tris pH6.8 / 100mM NaCl buffer;
(b) mixing the solution with a crystallization solution comprising 0.1 M sodium citrate, 0.1 M ammonium sulfate and 13% (w/v) 35000 PEG; and
(c) incubating the mixture under conditions to promote hanging drop vapor diffusion for a time sufficient to produce the co-crystal of human isoglutaminyl cyclase bound to a binding ligand, such as an isoglutaminyl cyclase inhibitor.

## Patentansprüche

1. Ein Kristall, umfassend humane Isoglutaminyl Cyclase, die eine charakterisierte Raumgruppe von P1211 und Abmessungen der Elementarzelle von +/- 5% von a = 126.51 Ä, b = 109.68 Ä, c = 159.53 Å, α = 90.0º, β = 104.9º und γ = 90.0º hat.

2. Ein Kristall wie in Anspruch 1 definiert, der die Röntgenstrahlen zur Bestimmung der Atomkoordinaten des Kristalls zu einer Auflösung zwischen 3.42 Ä und 34.72 Ä beugt.

3. Ein Kristall wie in Anspruch 1 oder Anspruch 2 definiert, wobei die humane Isoglutaminyl Cyclase aus den Aminosäureresten I73 bis L382 der SEQ ID NO:1 besteht.

4. Ein Kristall wie in Anspruch 3 definiert, der eine Bindungstasche umfasst, die von den Resten E226, D186 und H351 der SEQ ID NO: 1 entsprechend der Koordinaten in Figur 1 bereitgestellt wird.

5. Ein Kristall wie in Anspruch 3 oder Anspruch 4 definiert, der eine Disulfidbrücke zwischen den Resten C167 und C191 der SEQ ID NO: 1 umfasst.

6. Ein Co-Kristall umfassend den wie in einem der vorhergehenden Ansprüche definierten Kristall, der an einen Bindungsliganden, wie zum Beispiel an einen Inhibitor der Isoglutaminyl Cyclase, gebunden ist.

7. Ein Verfahren zur Identifizierung eines Inhibitors der humanen Isoglutaminyl Cyclase, welches die folgenden Schritte umfasst:
(a) Erzeugung eines 3-dimensionalen Models der humanen Isoglutaminyl Cyclase unter Verwendung der in Figur 1 beschriebenen Strukturkoordinaten;
(b) Analysieren der durch die Reste E226, D186 und H351 der SEQ ID NO: 1 entsprechend der Koordinaten der Figur 1 bereitgestellten Bindungstasche;
(c) Durchführen einer Analyse mittels Computermodellierung, um eine Inhibitorsubstanz zu identifizieren, die mit der Bindungstasche der humanen Isoglutaminyl Cyclase assoziieren kann.

8. Ein Verfahren wie in Anspruch 7 definiert, wobei der Schritt der Durchführung einer Analyse mittels Computermodellierung zur Identifizierung der besagten Inhibitorsubstanz die Identifizierung der besagten Substanz in einer Substanzbibliothek; oder die Identifizierung der besagten Substanz in einer Datenbank; oder das Design der Substanz nach einem bekannten Inhibitor der humanen Isoglutaminyl Cyclase umfasst.

9. Ein Verfahren zur Herstellung des Kristalls der humanen Isoglutaminyl Cyclase wie in irgendeinem der Ansprüche 1 - 5 beschrieben, welches die Schritte umfasst:
(a) Bereitstellen einer Lösung der humanen Isoglutaminyl Cyclase, wahlweise in Gegenwart eines bekannten Isoglutaminyl Cyclase Inhibitors, in einem geeigneten Puffer, wie zum Beispiel 25mM Bis-Tris pH 6,8 / 100mM NaCl Puffer;
(b) Vermischen der Lösung mit einer Kristallisierungslösung umfassend 0,1 M Natriumcitrat, 0,1 M Ammoniumsulfat und 13% (w/v) 35000 PEG; und
(c) Inkubieren der Mischung unter Bedingungen zur Förderung der Hängetropfendampfdiffusion für eine ausreichende Zeit, um den Kristall der humanen Isoglutaminyl Cyclase herzustellen.

10. Ein Verfahren zur Herstellung eines Ko-Kristalls wie in Anspruch 6 definiert, dass die Schritte umfasst:
(a) Bereitstellen einer Lösung der humanen Isoglutaminyl Cyclase in Gegenwart eines Bindungsliganden, wie zu Beispiel eines Inhibitors der Isoglutaminyl Cyclase, in einem geeigneten Puffer, wie zum Beispiel einem 25mM Bis-Tris pH 6,8 /1 00mM NaCl Puffer;
(b) Vermischen der Lösung mit einer Kristallisierungslösung umfassend 0,1 M Natriumcitrat, 0,1 M Ammoniumsulfat und 13% (w/v) 35000 PEG; und
(c) Inkubieren der Mischung unter Bedingungen zur Förderung der Hängetropfendampfdiffusion für eine ausreichende Zeit, um einen Ko-Kristall der humanen Isoglutaminyl Cyclase, gebunden an einen Bindungsliganden, wie zum Beispiel einen Inhibitor der Isoglutaminyl Cyclase, herzustellen.

## Revendications

1. Cristal comprenant une isoglutaminylcyclase humaine ayant un groupe d'espace caractérisé de P1211 et des dimensions de cellule unitaire de +/- 5 % de a = 126,51 Å, b = 109,68 Å, c = 159,53 Å, α = 90,0°, β = 104,9° et γ = 90,0°.

2. Cristal tel que défini selon la revendication 1, qui diffracte les rayons X pour la détermination des coordonnées atomiques du cristal à une résolution comprise entre 3,42 Å et 34,72 Å.

3. Cristal tel que défini selon la revendication 1 ou la revendication 2, dans lequel l'isoglutaminylcyclase humaine se compose des résidus acides aminés 173 à L382 de la SEQ ID N° 1.

4. Cristal tel que défini selon la revendication 3, qui comprend une poche de liaison fournie par les résidus E226, D186 et H351 de la SEQ ID N° 1 selon les coordonnées de la figure 1.

5. Cristal tel que défini selon la revendication 3 ou la revendication 4, qui comprend un pont disulfure entre les résidus C167 et C191 de la SEQ ID N° 1.

6. Co-cristal comprenant le cristal tel que défini selon l'une quelconque des revendications précédentes, dans lequel l'isoglutaminylcyclase est liée à un ligand de liaison, tel qu'un inhibiteur d'isoglutaminylcyclase.

7. Procédé d'identification d'un inhibiteur d'isoglutaminylcyclase humaine qui comprend les étapes consistant à :
(a) générer un modèle en 3 dimensions d'isoglutaminylcyclase humaine en utilisant les coordonnées structurales décrites sur la figure 1 ;
(b) analyser la poche de liaison fournie par les résidus E226, D186 et H351 de la SEQ ID N° 1 selon les coordonnées de la figure 1 ;
(c) réaliser une analyse de modélisation par ordinateur pour identifier un composé inhibiteur qui peut être associé à la poche de liaison de l'isoglutaminylcyclase humaine.

8. Procédé tel que défini selon la revendication 7, dans lequel l'étape consistant à réaliser une analyse de modélisation par ordinateur pour identifier ledit composé inhibiteur consiste à identifier ledit composé à partir d'une banque de composés ou à identifier ledit composé dans une base de données ou à créer le composé à partir d'un inhibiteur d'isoglutaminylcyclase humaine connu.

9. Procédé de préparation du cristal d'isoglutaminylcyclase humaine tel que décrit selon l'une quelconque des revendications 1 à 5, qui comprend les étapes consistant à :
(a) fournir une solution d'isoglutaminylcyclase humaine, éventuellement en présence d'un inhibiteur d'isoglutaminylcyclase humaine connu, dans un tampon approprié tel qu'un tampon de 25 mM de Bis-Tris pH 6,8 / 100 mM de NaCl ;
(b) mélanger la solution avec une solution de cristallisation comprenant 0,1 M de citrate de sodium, 0,1 M de sulfate d'ammonium et 13 % (m/v) de PEG 35000 ; et
(c) incuber le mélange dans des conditions favorisant la diffusion de vapeur en goutte suspendue pendant un temps suffisant pour produire le cristal d'isoglutaminylcyclase humaine.

10. Procédé de préparation du co-cristal tel que défini selon la revendication 6, qui comprend les étapes consistant à :
(a) fournir une solution d'isoglutaminylcyclase humaine, éventuellement en présence d'un ligand de liaison, tel qu'un inhibiteur d'isoglutaminylcyclase, dans un tampon approprié tel qu'un tampon de 25 mM de Bis-Tris pH 6,8 / 100 mM de NaCl ;
(b) mélanger la solution avec une solution de cristallisation comprenant 0,1 M de citrate de sodium, 0,1 M de sulfate d'ammonium et 13 % (m/v) de PEG 35000 ; et
(c) incuber le mélange dans des conditions favorisant la diffusion de vapeur en goutte suspendue pendant un temps suffisant pour produire le co-cristal d'isoglutaminylcyclase humaine lié à un ligand de liaison, tel qu'un inhibiteur d'isoglutaminylcyclase.
